(19) Europäisches Patentamt
European Patent Office
Office européen des brevets

(11) EP 3 303 618 B1

# (12) EUROPEAN PATENT SPECIFICATION

(45) Date of publication and mention of the grant of the patent:
**25.09.2019 Bulletin 2019/39**

(21) Application number: **16728245.8**

(22) Date of filing: **26.05.2016**

(51) Int Cl.:
***C12Q 1/6886*** *(2018.01)*

(86) International application number:
**PCT/EP2016/061885**

(87) International publication number:
**WO 2016/193109 (08.12.2016 Gazette 2016/49)**

## (54) METHODS OF PROSTATE CANCER PROGNOSIS

VERFAHREN ZUR PROGNOSE VON PROSTATAKREBS

PROCÉDÉS DE PRONOSTIC DU CANCER DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2015 EP 15169782**

(43) Date of publication of application:
**11.04.2018 Bulletin 2018/15**

(73) Proprietor: **Koninklijke Philips N.V.**
**5656 AE Eindhoven (NL)**

(72) Inventors:
- **HOFFMANN, Ralf**
  **5656 AE Eindhoven (NL)**
- **DEN BIEZEN-TIMMERMANS, Eveline**
  **5656 AE Eindhoven (NL)**
- **VAN STRIJP, Dianne Arnoldina Margaretha Wilhelmina**
  **5656 AE Eindhoven (NL)**
- **VAN BRUSSEL, Anne Godefrida Catharina**
  **5656 AE Eindhoven (NL)**
- **ALVES DE INDA, Márcia**
  **5656 AE Eindhoven (NL)**
- **WROBEL, Janneke**
  **5656 AE Eindhoven (NL)**

(74) Representative: **van Velzen, Maaike Mathilde**
**Philips Intellectual Property & Standards**
**High Tech Campus 5**
**5656 AE Eindhoven (NL)**

(56) References cited:
**WO-A1-2010/131194 US-A1- 2013 196 321**

- **MIMOTO REI ET AL: "DYRK2 controls the epithelial-mesenchymal transition in breast cancer by degrading S", CANCER LETTERS, vol. 339, no. 2, 1 October 2013 (2013-10-01), pages 214-225, XP028715017, ISSN: 0304-3835, DOI: 10.1016/J.CANLET.2013.06.005**
- **"Affymetrix GeneChip Human Genome U133 Array Set HG-U133A", GEO,, 11 March 2002 (2002-03-11), XP002355386, [retrieved on 2002-03-11]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 303 618 B1

SEQUENCE LISTING

<110> Koninklijke Philips N.V.

<120> Methods of prostate cancer prognosis

<130> P14144 2014PF00611

<160> 204

<170> PatentIn version 3.5

<210> 1
<211> 7979
<212> DNA
<213> Homo sapiens

<400> 1

cagcagcagg ctcagacctg cttccctgga catttccggg accgtgagcg agggaaccac 60
gttgccctgg attcttgcca gctgtacaaa gttgaccagg aaaatggctc agcagacaag 120
cccggacact ttaacagtac ctgaagtgga taatccgcat tgtccaaacc cgtggctgaa 180
cgaagacctt gtgaaatcct tgcgagaaaa cctgttgcag catgagaagt ccaagacagc 240
gaggaaatcg gtttctccca agctctctcc agtgatctct ccgagaaatt cccccaggct 300
tctgcgcaga atgcttctca gcagcaacat ccccaaacag cggcgtttca cggtggcaca 360
tacatgtttt gatgtggaca atggcacatc tgcgggacgg agtcccttgg atcccatgac 420
cagcccagga tccgggctaa ttctccaagc aaattttgtc cacagtcaac gacgggagtc 480
cttcctgtat cgatccgaca gcgattatga cctctctcca aagtctatgt cccggaactc 540
ctccattgcc agtgatatac acggagatga cttgattgtg actccatttg ctcaggtctt 600
ggccagtctg cgaactgtac gaaacaactt tgctgcatta actaatttgc aagatcgagc 660
acctagcaaa agatcaccca tgtgcaacca accatccatc aacaaagcca ccataacaga 720
ggaggcctac cagaaactgg ccagcgagac cctggaggag ctggactggt gtctggacca 780
gctagagacc ctacagacca ggcactccgt cagtgagatg gcctccaaca agtttaaaag 840
gatgcttaat cgggagctca cccatctctc tgaaatgagt cggtctggaa atcaagtgtc 900
agagtttata tcaaacacat tcttagataa gcaacatgaa gtggaaattc cttctccaac 960
tcagaaggaa aaggagaaaa agaaaagacc aatgtctcag atcagtggag tcaagaaatt 1020
gatgcacagc tctagtctga ctaattcaag tatcccaagg tttggagtta aaactgaaca 1080
agaagatgtc cttgccaagg aactagaaga tgtgaacaaa tggggtcttc atgttttcag 1140
aatagcagag ttgtctggta accggccctt gactgttatc atgcacacca tttttcagga 1200
acgggattta ttaaaaacat ttaaaattcc agtagatact ttaattacat atcttatgac 1260
tctcgaagac cattaccatg ctgatgtggc ctatcacaac aatatccatg ctgcagatgt 1320
tgtccagtct actcatgtgc tattatctac acctgctttg gaggctgtgt ttacagattt 1380

```
ggagattctt gcagcaattt ttgccagtgc aatacatgat gtagatcatc ctggtgtgtc    1440
caatcaattt ctgatcaata caaactctga acttgccttg atgtacaatg attcctcagt    1500
cttagagaac catcatttgg ctgtgggctt taaattgctt caggaagaaa actgtgacat    1560
tttccagaat ttgaccaaaa aacaaagaca atctttaagg aaaatggtca ttgacatcgt    1620
acttgcaaca gatatgtcaa aacacatgaa tctactggct gatttgaaga ctatggttga    1680
aactaagaaa gtgacaagct ctggagttct tcttcttgat aattattccg ataggattca    1740
ggttcttcag aatatggtgc actgtgcaga tctgagcaac ccaacaaagc ctctccagct    1800
gtaccgccag tggacggacc ggataatgga ggagttcttc cgccaaggag accgagagag    1860
ggaacgtggc atggagataa gccccatgtg tgacaagcac aatgcttccg tggaaaaatc    1920
acaggtgggc ttcatagact atattgttca tcccctctgg gagacatggg cagacctcgt    1980
ccaccctgac gcccaggata ttttggacac tttggaggac aatcgtgaat ggtaccagag    2040
cacaatccct cagagcccct ctcctgcacc tgatgaccca gaggagggcc ggcagggtca    2100
aactgagaaa ttccagtttg aactaacttt agaggaagat ggtgagtcag acacggaaaa    2160
ggacagtggc agtcaagtgg aagaagacac tagctgcagt gactccaaga ctctttgtac    2220
tcaagactca gagtctactg aaattcccct tgatgaacag gttgaagagg aggcagtagg    2280
ggaagaagag gaaagccagc ctgaagcctg tgtcatagat gatcgttctc ctgacacgta    2340
acagtgcaaa aactttcatg cctttttttt ttttaagtag aaaaattgtt tccaaagtgc    2400
atgtcacatg ccacaaccac ggtcacacct cactgtcatc tgccaggacg tttgttgaac    2460
aaaactgacc ttgactactc agtccagcgc tcaggaatat cgtaaccagt tttttcacct    2520
ccatgtcatc cgagcaaggt ggacatcttc acgaacagcg tttttaacaa gatttcagct    2580
tggtagagct gacaaagcag ataaaatcta ctccaaatta ttttcaagag agtgtgactc    2640
atcaggcagc ccaaaagttt attggacttg ggtttctat tcctttttat ttgtttgcaa     2700
tattttcaga agaaaggcat tgcacagagt gaacttaatg gacgaagcaa caaatatgtc    2760
aagaacagga catagcacga atctgttacc agtaggagga ggatgagcca cagaaattgc    2820
ataattttct aatttcaagt cttcctgata catgactgaa tagtgtggtt cagtgagctg    2880
cactgacctc tacattttgt atgatatgta aaacagattt tttgtagagc ttacttttat    2940
tattaaatgt attgaggtat tatatttaaa aaaaactatg ttcagaactt catctgccac    3000
tggttatttt tttctaagga gtaacttgca agttttcagt acaaatctgt gctacactgg    3060
ataaaaatct aatttatgaa ttttacttgc accttatagt tcatagcaat taactgattt    3120
gtagtgattc attgtttgtt ttatatacca atgacttcca tattttaaaa gagaaaaaca    3180
actttatgtt gcaggaaacc ctttttgtaa gtctttatta tttactttgc attttgtttc    3240
```

actctttcca gataagcaga gttgctcttc accagtgttt ttcttcatgt gcaaagtgac 3300
tatttgttct ataatacttt tatgtgtgtt atatcaaatg tgtcttaagc ttcatgcaaa 3360
ctcagtcatc agttcgtgtt gtctgaagca agtgggagat atataaatac ccagtagcta 3420
aaatggtcag tctttttag atgttttcct acttagtatc tcctaataac gttttgctgt 3480
gtcactagat gttcatttca caagtgcatg tctttctaat aatccacaca tttcatgctc 3540
taataatcca cacatttcat gctcattttt attgttttta cagccagtta tagtaagaaa 3600
aaggtttttc cccttgtgct gctttataat ttagcgtgtg tctgaacctt atccatgttt 3660
gctagatgag gtcttgtcaa atatatcact accattgtca ccggtgaaaa gaaacaggta 3720
gttaagttag ggttaacatt catttcaacc acgaggttgt atatcatgac tagcttttac 3780
tcttggttta cagagaaaag ttaaacagcc aactaggcag tttttaagaa tattaacaat 3840
atattaacaa acaccaatac aactaatcct atttggtttt aatgatttca ccatgggatt 3900
aagaactata tcaggaacat ccctgagaaa cggttttaag tgtagcaact actcttcctt 3960
aatggacagc cacataacgt gtaggaagtc ctttatcact tatcctcgat ccataagcat 4020
atcttgcaga ggggaactac ttctttaaac acatggaggg aaagaagatg atgccactgg 4080
caccagaggg ttagtactgt gatgcatcct aaaatattta ttatattggt aaaaattctg 4140
gttaaataaa aaattagaga tcactcttgg ctgatttcag caccaggaac tgtattacag 4200
ttttagagat taattcctag tgtttacctg attatagcag ttggcatcat ggggcattta 4260
attctgactt tatccccacg tcagccttaa taaagtcttc tttaccttct ctatgaagac 4320
tttaaagccc aaataatcat ttttcacatt gatattcaag aattgagata gatagaagcc 4380
aaagtgggta tctgacaagt ggaaaatcaa acgtttaaga agaattacaa ctctgaaaag 4440
catttatatg tggaacttct caaggagcct cctggggact ggaaagtaag tcatcagcca 4500
ggcaaatgac tcatgctgaa gagagtcccc atttcagtcc cctgagatct agctgatgct 4560
tagatccttt gaaataaaaa ttatgtcttt ataactctga tcttttacat aaagcagaag 4620
aggaatcaac tagttaattg caaggtttct actctgtttc ctctgtaaag atcagatggt 4680
aatctttcaa ataagaaaaa aataaagacg tatgtttgac caagtagttt cacaagaata 4740
tttgggaact tgtttctttt aattttattt gtccctgagt gaagtctaga aagaaaggta 4800
aagagtctag agtttattcc tctttccaaa acattctcat tcctctcctc cctacactta 4860
gtatttcccc cacagagtgc ctagaatctt aataatgaat aaaataaaaa gcagcaatat 4920
gtcattaaca aatccagacc tgaaagggta aagggtttat aactgcacta ataaagagag 4980
gctctttttt tttcttccag tttgttggtt tttaatggta ccgtgttgta aagataccca 5040
ctaatggaca atcaaattgc agaaaaggct caatatccaa gagacaggga ctaatgcact 5100
gtacaatctg cttatccttg cccttctctc ttgccaaagt gtgcttcaga aatatatact 5160

gctttaaaaa agaataaaag aatatccttt tacaagtggc tttacatttc ctaaaatgcc 5220
ataagaaaat gcaatatctg ggtactgtat ggggaaaaaa atgtccaagt ttgtgtaaaa 5280
ccagtgcatt tcagcttgca agttactgaa cacaataatg ctgttttaat tttgttttat 5340
atcagttaaa attcacaata atgtagatag aacaaattac agacaaggaa agaaaaaact 5400
tgaatgaaat ggattttaca gaaagcttta tgataatttt tgaatgcatt atttattttt 5460
tgtgccatgc attttttttc tcaccaaatg accttacctg taatacagtc ttgtttgtct 5520
gtttacaacc atgtatttat tgcaatgtac atactgtaat gttaattgta aattatctgt 5580
tcttattaaa acatcatccc atgatgggat ggtgttgata tatttggaaa ctcttggtga 5640
gagaatgaat ggtgtgtata catactctgt acatttttct tttctcctgt aatatagtct 5700
tgtcacctta gagcttgttt atggaagatt caagaaaact ataaaatact taaagatata 5760
taaatttaaa aaaacatagc tgcaggtctt tggtcccagg gctgtgcctt aactttaacc 5820
aatattttct tctgttttgc tgcatttgaa aggtaacagt ggagctaggg ctgggcattt 5880
tacatccagg cttttaattg attagaattc tgccaatagg tggattttac aaaaccacag 5940
acaacctctg aaagattctg agaccctttt gagacagaag ctcttaagta cttcttgcca 6000
gggagcagca ctgcatgtgt gatggttgtt tgccatctgt tgatcaggaa ctacttcagc 6060
tacttgcatt tgattatttc cttttttttt ttttttaact cggaaacaca actggggaaa 6120
tatattcttt cccagtgatt ataaacaatc tttttctttt ttttaagtcc ttttggcttc 6180
tagagctcat aggaaaatgg acttgatttg aaattggagc cagagtttac tcgtgttggt 6240
tatctattca tcagcttcct gacatgttaa gagaatacat taaagagaaa atactgtttt 6300
ttaatcctaa aatttttctt ccactaagat aaaccaaatg tccttacata tatgtaaacc 6360
catctattta aacgcaaagg tgggttgatg tcagtttaca tagcagaaag cattcactat 6420
cctctaagat ttgtttctgc aaaactttca ttgctttaga attttaaaat ttcaccttgt 6480
acaatggcca gcccctaaag caggaaacat ttataatgga ttatatggaa acatcctccc 6540
agtacttgcc cagcccttga atcatgtggc ttttcagtga aaggaaagat tctttttcta 6600
ggaaaaatga gcctatttta ttttatttta ttttattttt tgacacaaac tgtagatttt 6660
agcagccctg gcccaaagga atttgattac ttttgtttta aacagtacaa aggggacact 6720
ataattacaa aaacatcctt aactgatttg agttgttttt atttctttgg atatattttc 6780
agagtggtaa attgtgtgtg agaattacaa atgattattc ttttagtggt ttcttagcct 6840
ctcttacagc ccacggggat agtactgtac atcaatacct tcatatgaaa tttttatatg 6900
caatgaaaat aaaagcatgg gttgattctg cctatttatg actcaatctt ttacaaataa 6960
aagattattc attttaaatt atagttcaat cagcatgtct cttaggatac tgaacgtggt 7020

```
tgaaatgaaa ggatagtgac atcataagtt agtactgata ttcataacca aataaagcca     7080
acttgagtaa ttttgctaca ttaaaaatta ccaaaattac ttagatggcc tataagatta     7140
agcatggtgt tttctaagca agctttgaaa ggggccttcc atacttactt aattgaatat     7200
tctgggatat tgaaaattat tcagatactt gacaattatt tttggttacc tactccgcaa     7260
actacaaagt tttaaggact caacaataag ttaatgagac acagtgtttg ctttcatgga     7320
gcttacagtc tggaggggac aaaggcttaa acaatactca tataattata tatgtgatca     7380
gtacaatgaa ggagctcagt ggggtaaata agcaggaacc tgaacttgat ctgttccgga     7440
gggccacaga aggcttcctt gaggccttga gaaagtgatt tgcatctgag ttctgaagga     7500
ttgtaagagg taactaggga aaaagttgac aggaagagga aggggatcca gacaagaaac     7560
atttgcaaag atcttgaggc ataaatgagc ttgagacatc tggagaaact gaggaaaagt     7620
gagagagtag gcagggcctg gagccgcaga gccattgcta accatcctgt gtgagatatc     7680
ccccattctg tagctttatt ctcataaccc tgctcaattt tctttataac acttctcaca     7740
gatttatata cgtgtttgtt tttgttatct gtctctccca ccagaccaca gctccatgag     7800
agcaaggtct ttgcttacca atatatcact agcacttaaa actatgcctg gtacacagta     7860
ggttcttaat atgtgttgaa tatagccatc aaattgatat tggatataat tcaatctgat     7920
aagatatttt gagatattaa agagttttta acttgatacc ataaaaaaaa aaaaaaaaa      7979
```

<210> 2
<211> 745
<212> PRT
<213> Homo sapiens

<400> 2

```
Met Ala Gln Gln Thr Ser Pro Asp Thr Leu Thr Val Pro Glu Val Asp
1               5                   10                  15

Asn Pro His Cys Pro Asn Pro Trp Leu Asn Glu Asp Leu Val Lys Ser
            20                  25                  30

Leu Arg Glu Asn Leu Leu Gln His Glu Lys Ser Lys Thr Ala Arg Lys
        35                  40                  45

Ser Val Ser Pro Lys Leu Ser Pro Val Ile Ser Pro Arg Asn Ser Pro
    50                  55                  60

Arg Leu Leu Arg Arg Met Leu Leu Ser Ser Asn Ile Pro Lys Gln Arg
65                  70                  75                  80

Arg Phe Thr Val Ala His Thr Cys Phe Asp Val Asp Asn Gly Thr Ser
                85                  90                  95
```

```
Ala Gly Arg Ser Pro Leu Asp Pro Met Thr Ser Pro Gly Ser Gly Leu
            100                 105                 110

Ile Leu Gln Ala Asn Phe Val His Ser Gln Arg Arg Glu Ser Phe Leu
        115                 120                 125

Tyr Arg Ser Asp Ser Asp Tyr Asp Leu Ser Pro Lys Ser Met Ser Arg
    130                 135                 140

Asn Ser Ser Ile Ala Ser Asp Ile His Gly Asp Asp Leu Ile Val Thr
145                 150                 155                 160

Pro Phe Ala Gln Val Leu Ala Ser Leu Arg Thr Val Arg Asn Asn Phe
                165                 170                 175

Ala Ala Leu Thr Asn Leu Gln Asp Arg Ala Pro Ser Lys Arg Ser Pro
            180                 185                 190

Met Cys Asn Gln Pro Ser Ile Asn Lys Ala Thr Ile Thr Glu Glu Ala
        195                 200                 205

Tyr Gln Lys Leu Ala Ser Glu Thr Leu Glu Glu Leu Asp Trp Cys Leu
    210                 215                 220

Asp Gln Leu Glu Thr Leu Gln Thr Arg His Ser Val Ser Glu Met Ala
225                 230                 235                 240

Ser Asn Lys Phe Lys Arg Met Leu Asn Arg Glu Leu Thr His Leu Ser
                245                 250                 255

Glu Met Ser Arg Ser Gly Asn Gln Val Ser Glu Phe Ile Ser Asn Thr
            260                 265                 270

Phe Leu Asp Lys Gln His Glu Val Glu Ile Pro Ser Pro Thr Gln Lys
        275                 280                 285

Glu Lys Glu Lys Lys Lys Arg Pro Met Ser Gln Ile Ser Gly Val Lys
    290                 295                 300

Lys Leu Met His Ser Ser Ser Leu Thr Asn Ser Ser Ile Pro Arg Phe
305                 310                 315                 320

Gly Val Lys Thr Glu Gln Glu Asp Val Leu Ala Lys Glu Leu Glu Asp
                325                 330                 335

Val Asn Lys Trp Gly Leu His Val Phe Arg Ile Ala Glu Leu Ser Gly
            340                 345                 350
```

```
Asn Arg Pro Leu Thr Val Ile Met His Thr Ile Phe Gln Glu Arg Asp
        355                 360                 365

Leu Leu Lys Thr Phe Lys Ile Pro Val Asp Thr Leu Ile Thr Tyr Leu
    370                 375                 380

Met Thr Leu Glu Asp His Tyr His Ala Asp Val Ala Tyr His Asn Asn
385                 390                 395                 400

Ile His Ala Ala Asp Val Val Gln Ser Thr His Val Leu Leu Ser Thr
                405                 410                 415

Pro Ala Leu Glu Ala Val Phe Thr Asp Leu Glu Ile Leu Ala Ala Ile
            420                 425                 430

Phe Ala Ser Ala Ile His Asp Val Asp His Pro Gly Val Ser Asn Gln
        435                 440                 445

Phe Leu Ile Asn Thr Asn Ser Glu Leu Ala Leu Met Tyr Asn Asp Ser
    450                 455                 460

Ser Val Leu Glu Asn His His Leu Ala Val Gly Phe Lys Leu Leu Gln
465                 470                 475                 480

Glu Glu Asn Cys Asp Ile Phe Gln Asn Leu Thr Lys Lys Gln Arg Gln
                485                 490                 495

Ser Leu Arg Lys Met Val Ile Asp Ile Val Leu Ala Thr Asp Met Ser
            500                 505                 510

Lys His Met Asn Leu Leu Ala Asp Leu Lys Thr Met Val Glu Thr Lys
        515                 520                 525

Lys Val Thr Ser Ser Gly Val Leu Leu Leu Asp Asn Tyr Ser Asp Arg
    530                 535                 540

Ile Gln Val Leu Gln Asn Met Val His Cys Ala Asp Leu Ser Asn Pro
545                 550                 555                 560

Thr Lys Pro Leu Gln Leu Tyr Arg Gln Trp Thr Asp Arg Ile Met Glu
                565                 570                 575

Glu Phe Phe Arg Gln Gly Asp Arg Glu Arg Glu Arg Gly Met Glu Ile
            580                 585                 590

Ser Pro Met Cys Asp Lys His Asn Ala Ser Val Glu Lys Ser Gln Val
```

```
            595                 600                 605

Gly Phe Ile Asp Tyr Ile Val His Pro Leu Trp Glu Thr Trp Ala Asp
    610                 615                 620

Leu Val His Pro Asp Ala Gln Asp Ile Leu Asp Thr Leu Glu Asp Asn
625                 630                 635                 640

Arg Glu Trp Tyr Gln Ser Thr Ile Pro Gln Ser Pro Ser Pro Ala Pro
                645                 650                 655

Asp Asp Pro Glu Glu Gly Arg Gln Gly Gln Thr Glu Lys Phe Gln Phe
            660                 665                 670

Glu Leu Thr Leu Glu Glu Asp Gly Glu Ser Asp Thr Glu Lys Asp Ser
        675                 680                 685

Gly Ser Gln Val Glu Glu Asp Thr Ser Cys Ser Asp Ser Lys Thr Leu
    690                 695                 700

Cys Thr Gln Asp Ser Glu Ser Thr Glu Ile Pro Leu Asp Glu Gln Val
705                 710                 715                 720

Glu Glu Glu Ala Val Gly Glu Glu Glu Glu Ser Gln Pro Glu Ala Cys
                725                 730                 735

Val Ile Asp Asp Arg Ser Pro Asp Thr
            740                 745
```

<210> 3
<211> 19
<212> DNA
<213> Artificial Sequence

<220>
<223> PDE4D5_forward primer

<400> 3
gcttctcagc agcaacatc 19

<210> 4
<211> 20
<212> DNA
<213> Artificial Sequence

<220>
<223> PDE4D5_reverse primer

<400> 4
tgccattgtc cacatcaaaa 20

<210> 5
<211> 24
<212> DNA
<213> Artificial Sequence

<220>
<223> PDE4D5 probe

<400> 5
acagcggcgt ttcacggtgg caca 24

<210> 6
<211> 8130
<212> DNA
<213> Homo sapiens

<400> 6

agattatagc ccagcgtacg agaagcacga gtcctatagt tggcgtaccc tgaggcctgc 60

cagttcctgc cttaatgcat atgtagtcgt aattgagttc tgacacggcc ttggatgttt 120

ctgtcctaaa tagctgacat tgcatcttca agactgtcat tccagttggc ttttgagtgg 180

atacgtgcag tgagatcatt gacactggaa acactagttc ccattttaat tacttaaaac 240

accacgatga aaagaaatac ctgtgatttg ctttctcgga gcaaaagtgc ctctgaggaa 300

acactacatt ccagtaatga agaggaagac cctttccgcg gaatggaacc ctatcttgtc 360

cggagacttt catgtcgcaa tattcagctt ccccctctcg ccttcagaca gttggaacaa 420

gctgacttga aaagtgaatc agagaacatt caacgaccaa ccagcctccc cctgaagatt 480

ctgccgctga ttgctatcac ttctgcagaa tccagtggtt ttgatgtgga caatggcaca 540

tctgcgggac ggagtccctt ggatcccatg accagcccag gatccgggct aattctccaa 600

gcaaattttg tccacagtca acgacgggag tccttcctgt atcgatccga cagcgattat 660

gacctctctc caaagtctat gtcccggaac tcctccattg ccagtgatat acacggagat 720

gacttgattg tgactccatt tgctcaggtc ttggccagtc tgcgaactgt acgaaacaac 780

tttgctgcat taactaattt gcaagatcga gcacctagca aaagatcacc catgtgcaac 840

caaccatcca tcaacaaagc caccataaca gaggaggcct accagaaact ggccagcgag 900

accctggagg agctggactg gtgtctggac cagctagaga ccctacagac caggcactcc 960

gtcagtgaga tggcctccaa caagtttaaa aggatgctta atcgggagct cacccatctc 1020

tctgaaatga gtcggtctgg aaatcaagtg tcagagttta tatcaaacac attcttagat 1080

aagcaacatg aagtggaaat tccttctcca actcagaagg aaaaggagaa aaagaaaaga 1140

ccaatgtctc agatcagtgg agtcaagaaa ttgatgcaca gctctagtct gactaattca 1200

agtatcccaa ggtttggagt taaaactgaa caagaagatg tccttgccaa ggaactagaa 1260

gatgtgaaca aatggggtct tcatgttttc agaatagcag agttgtctgg taaccggccc 1320

ttgactgtta tcatgcacac cattttcag gaacgggatt tattaaaaac atttaaaatt 1380

```
ccagtagata ctttaattac atatcttatg actctcgaag accattacca tgctgatgtg     1440
gcctatcaca acaatatcca tgctgcagat gttgtccagt ctactcatgt gctattatct     1500
acacctgctt tggaggctgt gtttacagat ttggagattc ttgcagcaat ttttgccagt     1560
gcaatacatg atgtagatca tcctggtgtg tccaatcaat ttctgatcaa tacaaactct     1620
gaacttgcct tgatgtacaa tgattcctca gtcttagaga accatcattt ggctgtgggc     1680
tttaaattgc ttcaggaaga aaactgtgac attttccaga atttgaccaa aaaacaaaga     1740
caatctttaa ggaaaatggt cattgacatc gtacttgcaa cagatatgtc aaaacacatg     1800
aatctactgg ctgatttgaa gactatggtt gaaactaaga aagtgacaag ctctggagtt     1860
cttcttcttg ataattattc cgataggatt caggttcttc agaatatggt gcactgtgca     1920
gatctgagca acccaacaaa gcctctccag ctgtaccgcc agtggacgga ccggataatg     1980
gaggagttct tccgccaagg agaccgagag agggaacgtg gcatggagat aagccccatg     2040
tgtgacaagc acaatgcttc cgtggaaaaa tcacaggtgg gcttcataga ctatattgtt     2100
catcccctct gggagacatg ggcagacctc gtccaccctg acgcccagga tattttggac     2160
actttggagg acaatcgtga atggtaccag agcacaatcc ctcagagccc ctctcctgca     2220
cctgatgacc cagaggaggg ccggcagggt caaactgaga aattccagtt tgaactaact     2280
ttagaggaag atggtgagtc agacacggaa aaggacagtg gcagtcaagt ggaagaagac     2340
actagctgca gtgactccaa gactctttgt actcaagact cagagtctac tgaaattccc     2400
cttgatgaac aggttgaaga ggaggcagta ggggaagaag aggaaagcca gcctgaagcc     2460
tgtgtcatag atgatcgttc tcctgacacg taacagtgca aaaactttca tgcctttttt     2520
ttttttaagt agaaaaattg tttccaaagt gcatgtcaca tgccacaacc acggtcacac     2580
ctcactgtca tctgccagga cgtttgttga acaaaactga ccttgactac tcagtccagc     2640
gctcaggaat atcgtaacca gttttttcac ctccatgtca tccgagcaag gtggacatct     2700
tcacgaacag cgtttttaac aagatttcag cttggtagag ctgacaaagc agataaaatc     2760
tactccaaat tattttcaag agagtgtgac tcatcaggca gcccaaaagt ttattggact     2820
tggggtttct attccttttt atttgtttgc aatattttca gaagaaaggc attgcacaga     2880
gtgaacttaa tggacgaagc aacaaatatg tcaagaacag gacatagcac gaatctgtta     2940
ccagtaggag gaggatgagc cacagaaatt gcataatttt ctaatttcaa gtcttcctga     3000
tacatgactg aatagtgtgg ttcagtgagc tgcactgacc tctacatttt gtatgatatg     3060
taaaacagat tttttgtaga gcttactttt attattaaat gtattgaggt attatattta     3120
aaaaaaacta tgttcagaac ttcatctgcc actggttatt tttttctaag gagtaacttg     3180
caagttttca gtacaaatct gtgctacact ggataaaaat ctaatttatg aattttactt     3240
```

gcaccttata gttcatagca attaactgat ttgtagtgat tcattgtttg ttttatatac 3300
caatgacttc catattttaa aagagaaaaa caactttatg ttgcaggaaa cccttttttgt 3360
aagtctttat tatttacttt gcattttgtt tcactctttc cagataagca gagttgctct 3420
tcaccagtgt ttttcttcat gtgcaaagtg actatttgtt ctataatact tttatgtgtg 3480
ttatatcaaa tgtgtcttaa gcttcatgca aactcagtca tcagttcgtg ttgtctgaag 3540
caagtgggag atatataaat acccagtagc taaaatggtc agtctttttt agatgttttc 3600
ctacttagta tctcctaata acgttttgct gtgtcactag atgttcattt cacaagtgca 3660
tgtctttcta ataatccaca catttcatgc tctaataatc cacacatttc atgctcattt 3720
ttattgtttt tacagccagt tatagtaaga aaaaggtttt tccccttgtg ctgctttata 3780
atttagcgtg tgtctgaacc ttatccatgt ttgctagatg aggtcttgtc aaatatatca 3840
ctaccattgt caccggtgaa aagaaacagg tagttaagtt agggttaaca ttcatttcaa 3900
ccacgaggtt gtatatcatg actagctttt actcttggtt tacagagaaa agttaaacag 3960
ccaactaggc agtttttaag aatattaaca atatattaac aaacaccaat acaactaatc 4020
ctatttggtt ttaatgattt caccatggga ttaagaacta tatcaggaac atccctgaga 4080
aacggtttta agtgtagcaa ctactcttcc ttaatggaca gccacataac gtgtaggaag 4140
tcctttatca cttatcctcg atccataagc atatcttgca gaggggaact acttctttaa 4200
acacatggag ggaaagaaga tgatgccact ggcaccagag ggttagtact gtgatgcatc 4260
ctaaaatatt tattatattg gtaaaaattc tggttaaata aaaaattaga gatcactctt 4320
ggctgatttc agcaccagga actgtattac agttttagag attaattcct agtgtttacc 4380
tgattatagc agttggcatc atggggcatt taattctgac tttatcccca cgtcagcctt 4440
aataaagtct tctttacctt ctctatgaag actttaaagc ccaaataatc atttttcaca 4500
ttgatattca agaattgaga tagatagaag ccaaagtggg tatctgacaa gtggaaaatc 4560
aaacgtttaa gaagaattac aactctgaaa agcatttata tgtggaactt ctcaaggagc 4620
ctcctgggga ctggaaagta agtcatcagc caggcaaatg actcatgctg aagagagtcc 4680
ccatttcagt cccctgagat ctagctgatg cttagatcct ttgaaataaa aattatgtct 4740
ttataactct gatcttttac ataaagcaga agaggaatca actagttaat tgcaaggttt 4800
ctactctgtt tcctctgtaa agatcagatg gtaatctttc aaataagaaa aaaataaaga 4860
cgtatgtttg accaagtagt ttcacaagaa tatttgggaa cttgtttctt ttaattttat 4920
ttgtccctga gtgaagtcta gaaagaaagg taaagagtct agagtttatt cctctttcca 4980
aaacattctc attcctctcc tccctacact tagtatttcc cccacagagt gcctagaatc 5040
ttaataatga ataaaataaa aagcagcaat atgtcattaa caaatccaga cctgaaaggg 5100
taaagggttt ataactgcac taataaagag aggctctttt tttttcttcc agtttgttgg 5160

tttttaatgg taccgtgttg taaagatacc cactaatgga caatcaaatt gcagaaaagg 5220
ctcaatatcc aagagacagg gactaatgca ctgtacaatc tgcttatcct tgcccttctc 5280
tcttgccaaa gtgtgcttca gaaatatata ctgctttaaa aaagaataaa agaatatcct 5340
tttacaagtg gctttacatt tcctaaaatg ccataagaaa atgcaatatc tgggtactgt 5400
atggggaaaa aaatgtccaa gtttgtgtaa aaccagtgca tttcagcttg caagttactg 5460
aacacaataa tgctgtttta attttgtttt atatcagtta aaattcacaa taatgtagat 5520
agaacaaatt acagacaagg aaagaaaaaa cttgaatgaa atggatttta cagaaagctt 5580
tatgataatt tttgaatgca ttatttattt tttgtgccat gcattttttt tctcaccaaa 5640
tgaccttacc tgtaatacag tcttgtttgt ctgtttacaa ccatgtattt attgcaatgt 5700
acatactgta atgttaattg taaattatct gttcttatta aaacatcatc ccatgatggg 5760
atggtgttga tatatttgga aactcttggt gagagaatga atggtgtgta tacatactct 5820
gtacattttt cttttctcct gtaatatagt cttgtcacct tagagcttgt ttatggaaga 5880
ttcaagaaaa ctataaaata cttaaagata tataaattta aaaaaacata gctgcaggtc 5940
tttggtccca gggctgtgcc ttaactttaa ccaatatttt cttctgtttt gctgcatttg 6000
aaaggtaaca gtggagctag ggctgggcat tttacatcca ggcttttaat tgattagaat 6060
tctgccaata ggtggatttt acaaaaccac agacaacctc tgaaagattc tgagaccctt 6120
ttgagacaga agctcttaag tacttcttgc cagggagcag cactgcatgt gtgatggttg 6180
tttgccatct gttgatcagg aactacttca gctacttgca tttgattatt tccttttttt 6240
tttttttaa ctcggaaaca caactgggga aatatattct ttcccagtga ttataaacaa 6300
tctttttctt ttttttaagt ccttttggct tctagagctc ataggaaaat ggacttgatt 6360
tgaaattgga gccagagttt actcgtgttg gttatctatt catcagcttc ctgacatgtt 6420
aagagaatac attaaagaga aaatactgtt ttttaatcct aaaatttttc ttccactaag 6480
ataaaccaaa tgtccttaca tatatgtaaa cccatctatt taaacgcaaa ggtgggttga 6540
tgtcagttta catagcagaa agcattcact atcctctaag atttgtttct gcaaaacttt 6600
cattgcttta gaattttaaa atttcacctt gtacaatggc cagcccctaa agcaggaaac 6660
atttataatg gattatatgg aaacatcctc ccagtacttg cccagccctt gaatcatgtg 6720
gcttttcagt gaaaggaaag attctttttc taggaaaaat gagcctattt tattttattt 6780
tattttattt tttgacacaa actgtagatt ttagcagccc tggcccaaag gaatttgatt 6840
acttttgttt taaacagtac aaaggggaca ctataattac aaaaacatcc ttaactgatt 6900
tgagttgttt ttatttcttt ggatatattt tcagagtggt aaattgtgtg tgagaattac 6960
aaatgattat tcttttagtg gtttcttagc ctctcttaca gcccacgggg atagtactgt 7020

acatcaatac cttcatatga aatttttata tgcaatgaaa ataaaagcat gggttgattc 7080

tgcctattta tgactcaatc ttttacaaat aaaagattat tcattttaaa ttatagttca 7140

atcagcatgt ctcttaggat actgaacgtg gttgaaatga aaggatagtg acatcataag 7200

ttagtactga tattcataac caaataaagc caacttgagt aattttgcta cattaaaaat 7260

taccaaaatt acttagatgg cctataagat taagcatggt gttttctaag caagctttga 7320

aaggggcctt ccatacttac ttaattgaat attctgggat attgaaaatt attcagatac 7380

ttgacaatta tttttggtta cctactccgc aaactacaaa gttttaagga ctcaacaata 7440

agttaatgag acacagtgtt tgctttcatg gagcttacag tctggagggg acaaaggctt 7500

aaacaatact catataatta tatatgtgat cagtacaatg aaggagctca gtggggtaaa 7560

taagcaggaa cctgaacttg atctgttccg gagggccaca gaaggcttcc ttgaggcctt 7620

gagaaagtga tttgcatctg agttctgaag gattgtaaga ggtaactagg gaaaaagttg 7680

acaggaagag gaaggggatc cagacaagaa acatttgcaa agatcttgag gcataaatga 7740

gcttgagaca tctggagaaa ctgaggaaaa gtgagagagt aggcagggcc tggagccgca 7800

gagccattgc taaccatcct gtgtgagata tcccccattc tgtagcttta ttctcataac 7860

cctgctcaat tttctttata acacttctca cagatttata tacgtgtttg tttttgttat 7920

ctgtctctcc caccagacca cagctccatg agagcaaggt ctttgcttac caatatatca 7980

ctagcactta aaactatgcc tggtacacag taggttctta atatgtgttg aatatagcca 8040

tcaaattgat attggatata attcaatctg ataagatatt ttgagatatt aaagagtttt 8100

taacttgata ccataaaaaa aaaaaaaaaa 8130

<210> 7
<211> 748
<212> PRT
<213> Homo sapiens

<400> 7

```
Met Lys Arg Asn Thr Cys Asp Leu Leu Ser Arg Ser Lys Ser Ala Ser
1               5                   10                  15

Glu Glu Thr Leu His Ser Ser Asn Glu Glu Glu Asp Pro Phe Arg Gly
            20                  25                  30

Met Glu Pro Tyr Leu Val Arg Arg Leu Ser Cys Arg Asn Ile Gln Leu
        35                  40                  45

Pro Pro Leu Ala Phe Arg Gln Leu Glu Gln Ala Asp Leu Lys Ser Glu
    50                  55                  60

Ser Glu Asn Ile Gln Arg Pro Thr Ser Leu Pro Leu Lys Ile Leu Pro
```

65 70 75 80

Leu Ile Ala Ile Thr Ser Ala Glu Ser Ser Gly Phe Asp Val Asp Asn
85 90 95

Gly Thr Ser Ala Gly Arg Ser Pro Leu Asp Pro Met Thr Ser Pro Gly
100 105 110

Ser Gly Leu Ile Leu Gln Ala Asn Phe Val His Ser Gln Arg Arg Glu
115 120 125

Ser Phe Leu Tyr Arg Ser Asp Ser Asp Tyr Asp Leu Ser Pro Lys Ser
130 135 140

Met Ser Arg Asn Ser Ser Ile Ala Ser Asp Ile His Gly Asp Asp Leu
145 150 155 160

Ile Val Thr Pro Phe Ala Gln Val Leu Ala Ser Leu Arg Thr Val Arg
165 170 175

Asn Asn Phe Ala Ala Leu Thr Asn Leu Gln Asp Arg Ala Pro Ser Lys
180 185 190

Arg Ser Pro Met Cys Asn Gln Pro Ser Ile Asn Lys Ala Thr Ile Thr
195 200 205

Glu Glu Ala Tyr Gln Lys Leu Ala Ser Glu Thr Leu Glu Glu Leu Asp
210 215 220

Trp Cys Leu Asp Gln Leu Glu Thr Leu Gln Thr Arg His Ser Val Ser
225 230 235 240

Glu Met Ala Ser Asn Lys Phe Lys Arg Met Leu Asn Arg Glu Leu Thr
245 250 255

His Leu Ser Glu Met Ser Arg Ser Gly Asn Gln Val Ser Glu Phe Ile
260 265 270

Ser Asn Thr Phe Leu Asp Lys Gln His Glu Val Glu Ile Pro Ser Pro
275 280 285

Thr Gln Lys Glu Lys Glu Lys Lys Lys Arg Pro Met Ser Gln Ile Ser
290 295 300

Gly Val Lys Lys Leu Met His Ser Ser Ser Leu Thr Asn Ser Ser Ile
305 310 315 320

```
Pro Arg Phe Gly Val Lys Thr Glu Gln Glu Asp Val Leu Ala Lys Glu
                325                 330                 335

Leu Glu Asp Val Asn Lys Trp Gly Leu His Val Phe Arg Ile Ala Glu
            340                 345                 350

Leu Ser Gly Asn Arg Pro Leu Thr Val Ile Met His Thr Ile Phe Gln
        355                 360                 365

Glu Arg Asp Leu Leu Lys Thr Phe Lys Ile Pro Val Asp Thr Leu Ile
    370                 375                 380

Thr Tyr Leu Met Thr Leu Glu Asp His Tyr His Ala Asp Val Ala Tyr
385                 390                 395                 400

His Asn Asn Ile His Ala Ala Asp Val Val Gln Ser Thr His Val Leu
                405                 410                 415

Leu Ser Thr Pro Ala Leu Glu Ala Val Phe Thr Asp Leu Glu Ile Leu
            420                 425                 430

Ala Ala Ile Phe Ala Ser Ala Ile His Asp Val Asp His Pro Gly Val
        435                 440                 445

Ser Asn Gln Phe Leu Ile Asn Thr Asn Ser Glu Leu Ala Leu Met Tyr
    450                 455                 460

Asn Asp Ser Ser Val Leu Glu Asn His His Leu Ala Val Gly Phe Lys
465                 470                 475                 480

Leu Leu Gln Glu Glu Asn Cys Asp Ile Phe Gln Asn Leu Thr Lys Lys
                485                 490                 495

Gln Arg Gln Ser Leu Arg Lys Met Val Ile Asp Ile Val Leu Ala Thr
            500                 505                 510

Asp Met Ser Lys His Met Asn Leu Leu Ala Asp Leu Lys Thr Met Val
        515                 520                 525

Glu Thr Lys Lys Val Thr Ser Ser Gly Val Leu Leu Leu Asp Asn Tyr
    530                 535                 540

Ser Asp Arg Ile Gln Val Leu Gln Asn Met Val His Cys Ala Asp Leu
545                 550                 555                 560

Ser Asn Pro Thr Lys Pro Leu Gln Leu Tyr Arg Gln Trp Thr Asp Arg
                565                 570                 575
```

Ile Met Glu Glu Phe Phe Arg Gln Gly Asp Arg Glu Arg Glu Arg Gly
580 585 590

Met Glu Ile Ser Pro Met Cys Asp Lys His Asn Ala Ser Val Glu Lys
595 600 605

Ser Gln Val Gly Phe Ile Asp Tyr Ile Val His Pro Leu Trp Glu Thr
610 615 620

Trp Ala Asp Leu Val His Pro Asp Ala Gln Asp Ile Leu Asp Thr Leu
625 630 635 640

Glu Asp Asn Arg Glu Trp Tyr Gln Ser Thr Ile Pro Gln Ser Pro Ser
645 650 655

Pro Ala Pro Asp Asp Pro Glu Glu Gly Arg Gln Gly Gln Thr Glu Lys
660 665 670

Phe Gln Phe Glu Leu Thr Leu Glu Glu Asp Gly Glu Ser Asp Thr Glu
675 680 685

Lys Asp Ser Gly Ser Gln Val Glu Glu Asp Thr Ser Cys Ser Asp Ser
690 695 700

Lys Thr Leu Cys Thr Gln Asp Ser Glu Ser Thr Glu Ile Pro Leu Asp
705 710 715 720

Glu Gln Val Glu Glu Glu Ala Val Gly Glu Glu Glu Glu Ser Gln Pro
725 730 735

Glu Ala Cys Val Ile Asp Asp Arg Ser Pro Asp Thr
740 745

<210> 8
<211> 20
<212> DNA
<213> Artificial Sequence

<220>
<223> PDE4D7

<400> 8
gaacattcaa cgaccaacca 20

<210> 9
<211> 20
<212> DNA
<213> Artificial Sequence

<220>

<223> PDE4D7_reverse primer

<400> 9
tgccattgtc cacatcaaaa 20

<210> 10
<211> 27
<212> DNA
<213> Artificial Sequence

<220>
<223> PDE4D7 probe

<400> 10
ctgccgctga ttgctatcac ttctgca 27

<210> 11
<211> 1278
<212> DNA
<213> Homo sapiens

<400> 11

```
ccattggcct gtagattcac ctcccctggg cagggcccca ggacccagga taatatctgt        60
gcctcctgcc cagaaccctc caagcagaca caatggtaag aatggtgcct gtcctgctgt       120
ctctgctgct gcttctgggt cctgctgtcc cccaggagaa ccaagatggt cgttactctc       180
tgacctatat ctacactggg ctgtccaagc atgttgaaga cgtccccgcg tttcaggccc       240
ttggctcact caatgacctc cagttcttta gatacaacag taaagacagg aagtctcagc       300
ccatgggact ctggagacag gtggaaggaa tggaggattg gaagcaggac agccaacttc       360
agaaggccag ggaggacatc tttatggaga ccctgaaaga catcgtggag tattacaacg       420
acagtaacgg gtctcacgta ttgcagggaa ggtttggttg tgagatcgag aataacagaa       480
gcagcggagc attctggaaa tattactatg atggaaagga ctacattgaa ttcaacaaag       540
aaatcccagc ctgggtcccc ttcgacccag cagcccagat aaccaagcag aagtgggagg       600
cagaaccagt ctacgtgcag cgggccaagg cttacctgga ggaggagtgc cctgcgactc       660
tgcggaaata cctgaaatac agcaaaaata tcctggaccg gcaagatcct ccctctgtgg       720
tggtcaccag ccaccaggcc ccaggagaaa agaagaaact gaagtgcctg gcctacgact       780
tctacccagg gaaaattgat gtgcactgga ctcgggccgg cgaggtgcag gagcctgagt       840
tacggggaga tgttcttcac aatggaaatg gcacttacca gtcctgggtg gtggtggcag       900
tgcccccgca ggacacagcc ccctactcct gccacgtgca gcacagcagc ctggcccagc       960
cctcgtggt gccctgggag gccagctagg aagcaagggt tggaggcaat gtgggatctc       1020
agacccagta gctgcccttc ctgcctgatg tgggagctga accacagaaa tcacagtcaa      1080
tggatccaca aggcctgagg agcagtgtgg ggggacagac aggaggtgga tttggagacc      1140
gaagactggg atgcctgtct tgagtagact tggacccaaa aaatcatctc accttgagcc      1200
cacccccacc ccattgtcta atctgtagaa gctaataaat aatcatccct ccttgcctag      1260
cataaaaaaa aaaaaaaa                                                    1278
```

<210> 12
<211> 298
<212> PRT
<213> Homo sapiens

<400> 12

```
Met Val Arg Met Val Pro Val Leu Leu Ser Leu Leu Leu Leu Leu Gly
1               5                   10                  15

Pro Ala Val Pro Gln Glu Asn Gln Asp Gly Arg Tyr Ser Leu Thr Tyr
            20                  25                  30

Ile Tyr Thr Gly Leu Ser Lys His Val Glu Asp Val Pro Ala Phe Gln
        35                  40                  45

Ala Leu Gly Ser Leu Asn Asp Leu Gln Phe Phe Arg Tyr Asn Ser Lys
    50                  55                  60

Asp Arg Lys Ser Gln Pro Met Gly Leu Trp Arg Gln Val Glu Gly Met
65                  70                  75                  80

Glu Asp Trp Lys Gln Asp Ser Gln Leu Gln Lys Ala Arg Glu Asp Ile
                85                  90                  95

Phe Met Glu Thr Leu Lys Asp Ile Val Glu Tyr Tyr Asn Asp Ser Asn
            100                 105                 110

Gly Ser His Val Leu Gln Gly Arg Phe Gly Cys Glu Ile Glu Asn Asn
        115                 120                 125

Arg Ser Ser Gly Ala Phe Trp Lys Tyr Tyr Tyr Asp Gly Lys Asp Tyr
    130                 135                 140

Ile Glu Phe Asn Lys Glu Ile Pro Ala Trp Val Pro Phe Asp Pro Ala
145                 150                 155                 160

Ala Gln Ile Thr Lys Gln Lys Trp Glu Ala Glu Pro Val Tyr Val Gln
                165                 170                 175

Arg Ala Lys Ala Tyr Leu Glu Glu Glu Cys Pro Ala Thr Leu Arg Lys
            180                 185                 190

Tyr Leu Lys Tyr Ser Lys Asn Ile Leu Asp Arg Gln Asp Pro Pro Ser
        195                 200                 205
```

```
Val Val Val Thr Ser His Gln Ala Pro Gly Glu Lys Lys Lys Leu Lys
    210                 215                 220

Cys Leu Ala Tyr Asp Phe Tyr Pro Gly Lys Ile Asp Val His Trp Thr
225                 230                 235                 240

Arg Ala Gly Glu Val Gln Glu Pro Glu Leu Arg Gly Asp Val Leu His
                245                 250                 255

Asn Gly Asn Gly Thr Tyr Gln Ser Trp Val Val Val Ala Val Pro Pro
            260                 265                 270

Gln Asp Thr Ala Pro Tyr Ser Cys His Val Gln His Ser Ser Leu Ala
        275                 280                 285

Gln Pro Leu Val Val Pro Trp Glu Ala Ser
    290                 295
```

<210> 13
<211> 20
<212> DNA
<213> Artificial Sequence

<220>
<223> AZGP1_forward primer

<400> 13
tggagaccct gaaagacatc 20

<210> 14
<211> 20
<212> DNA
<213> Artificial Sequence

<220>
<223> AZGP1_reverse primer

<400> 14
ctcgatctca caaccaaacc 20

<210> 15
<211> 23
<212> DNA
<213> Artificial Sequence

<220>
<223> AZGP1 probe

<400> 15
acgggtctca cgtattgcag gga 23

<210> 16
<211> 2312
<212> DNA

<213> Homo sapiens

<400> 16

ctcctcccgg gcgggataat tgaacggcgc ggccctggcc cagcgttggc tgccgaggct 60
cggccggagc gtggagcccg cgccgctgcc ccaggaccgc gcccgcgcct ttgtccgccg 120
ccgcccaccg cccgtcgccc gccgcccatg gagcgcgccg cgccgtcgcg ccgggtcccg 180
cttccgctgc tgctgctcgg cggccttgcg ctgctggcgg ccggagtgga cgcggatgtc 240
ctcctggagg cctgctgtgc ggacggacac cggatggcca ctcatcagaa ggactgctcg 300
ctgccatatg ctacggaatc caaagaatgc aggatggtgc aggagcagtg ctgccacagc 360
cagctggagg agctgcactg tgccacgggc atcagcctgg ccaacgagca ggaccgctgt 420
gccacgcccc acggtgacaa cgccagcctg gaggccacat ttgtgaagag gtgctgccat 480
tgctgtctgc tggggagggc ggcccaggcc cagggccaga gctgcgagta cagcctcatg 540
gttggctacc agtgtggaca ggtcttccgg gcatgctgtg tcaagagcca ggagaccgga 600
gatttggatg tcgggggcct ccaagaaacg gataagatca ttgaggttga ggaggaacaa 660
gaggacccat atctgaatga ccgctgccga ggaggcgggc cctgcaagca gcagtgccga 720
gacacgggtg acgaggtggt ctgctcctgc ttcgtgggct accagctgct gtctgatggt 780
gtctcctgtg aagatgtcaa tgaatgcatc acgggcagcc acagctgccg gcttggagaa 840
tcctgcatca acacagtggg ctctttccgc tgccagcggg acagcagctg cgggactggc 900
tatgagctca cagaggacaa tagctgcaaa gatattgacg agtgtgagag tggtattcat 960
aactgcctcc ccgattttat ctgtcagaat actctgggat ccttccgctg ccgacccaag 1020
ctacagtgca agagtggctt tatacaagat gctctaggca actgtattga tatcaatgag 1080
tgtttgagta tcagtgcccc gtgccctatc gggcatacat gcatcaacac agagggctcc 1140
tacacgtgcc agaagaacgt gcccaactgt ggccgtggct accatctcaa cgaggaggga 1200
acgcgctgtg ttgatgtgga cgagtgcgcg ccacctgctg agccctgtgg gaagggacat 1260
cgctgcgtga actctcccgg cagtttccgc tgcgaatgca agacgggtta ctattttgac 1320
ggcatcagca ggatgtgtgt cgatgtcaac gagtgccagc gctaccccgg gcgcctgtgt 1380
ggccacaagt gcgagaacac gctgggctcc tacctctgca gctgttccgt gggcttccgg 1440
ctctctgtgg atggcaggtc atgtgaagac atcaatgagt gcagcagcag cccctgtagc 1500
caggagtgtg ccaacgtcta cggctcctac cagtgttact gccggcgagg ctaccagctc 1560
agcgatgtgg atggagtcac ctgtgaagac atcgacgagt gcgccctgcc caccgggggc 1620
cacatctgct cctaccgctg catcaacatc cctggaagct tccagtgcag ctgcccctcg 1680
tctggctaca ggctggcccc caatggccgc aactgccaag acattgatga gtgtgtgact 1740

```
ggcatccaca actgctccat caacgagacc tgcttcaaca tccagggcgg cttccgctgc      1800
ctggccttcg agtgccctga gaactaccgc cgctccgcag ccacccgctg tgagcgcttg      1860
ccttgccatg agaatcggga gtgctccaag ctgcctctga gaataaccta ctaccacctc      1920
tctttcccca ccaacatcca agcgcccgcg gtggttttcc gcatgggccc ctccagtgct      1980
gtccccgggg acagcatgca gctggccatc accggcggca atgaggaggg ctttttcacc      2040
acccggaagg tgagccccca cagtggggtg gtggccctca ccaagcctgt ccccgagccc      2100
agggacttgc tcctgaccgt caagatggat ctctctcgcc acggcaccgt cagctccttt      2160
gtggccaagc ttttcatctt tgtgtctgca gagctctgag cactcgcttc gcgtcgcggg      2220
gtctccctcc tgttgctttc ctaaccctgc cctccggggc gttaataaag tcttagcaag      2280
cgtcccacac agtgaaaaaa aaaaaaaaaa aa                                    2312
```

<210> 17
<211> 2662
<212> DNA
<213> Homo sapiens

<400> 17

ctcctcccgg gcgggataat tgaacggcgc ggccctggcc cagcgttggc tgccgaggct 60

cggccggagc gtggagcccg cgccgctgcc ccaggaccgc gcccgcgcct ttgtccgccg 120

ccgcccaccg cccgtcgccc gccgcccatg gagcgcgccg cgccgtcgcg ccgggtcccg 180

cttccgctgc tgctgctcgg cggccttgcg ctgctggcgg ccggagtgga cgcggatgtc 240

ctcctggagg cctgctgtgc ggacggacac cggatggcca ctcatcagaa ggactgctcg 300

ctgccatatg ctacggaatc caaagaatgc aggatggtgc aggagcagtg ctgccacagc 360

cagctggagg agctgcactg tgccacgggc atcagcctgg ccaacgagca ggaccgctgt 420

gccacgcccc acggtgacaa cgccagcctg gaggccacat ttgtgaagag gtgctgccat 480

tgctgtctgc tggggagggc ggcccaggcc cagggccaga gctgcgagta cagcctcatg 540

gttggctacc agtgtggaca ggtcttccgg gcatgctgtg tcaagagcca ggagaccgga 600

gatttggatg tcgggggcct ccaagaaacg gataagatca ttgaggttga ggaggaacaa 660

gaggacccat atctgaatga ccgctgccga ggaggcgggc cctgcaagca gcagtgccga 720

gacacgggtg acgaggtggt ctgctcctgc ttcgtgggct accagctgct gtctgatggt 780

gtctcctgtg aagatgtcaa tgaatgcatc acgggcagcc acagctgccg gcttggagaa 840

tcctgcatca acacagtggg ctctttccgc tgccagcggg acagcagctg cgggactggc 900

tatgagctca cagaggacaa tagctgcaaa gatattgacg agtgtgagag tggtattcat 960

aactgcctcc ccgattttat ctgtcagaat actctgggat ccttccgctg ccgacccaag 1020

ctacagtgca agagtggctt tatacaagat gctctaggca actgtattga tatcaatgag 1080

tgtttgagta tcagtgcccc gtgccctatc gggcatacat gcatcaacac agagggctcc 1140

tacacgtgcc agaagaacgt gcccaactgt ggccgtggct accatctcaa cgaggaggga 1200

acgcgctgtg ttgatgtgga cgagtgcgcg ccacctgctg agccctgtgg gaagggacat 1260

cgctgcgtga actctcccgg cagtttccgc tgcgaatgca agacgggtta ctattttgac 1320

ggcatcagca ggatgtgtgt cgatgtcaac gagtgccagc gctaccccgg gcgcctgtgt 1380

ggccacaagt gcgagaacac gctgggctcc tacctctgca gctgttccgt gggcttccgg 1440

ctctctgtgg atggcaggtc atgtgaagac atcaatgagt gcagcagcag cccctgtagc 1500

caggagtgtg ccaacgtcta cggctcctac cagtgttact gccggcgagg ctaccagctc 1560

agcgatgtgg atggagtcac ctgtgaagac atcgacgagt gcgccctgcc caccgggggc 1620

cacatctgct cctaccgctg catcaacatc cctggaagct tccagtgcag ctgcccctcg 1680

tctggctaca ggctggcccc caatggccgc aactgccaag acattgatga gtgtgtgact 1740

ggcatccaca actgctccat caacgagacc tgcttcaaca tccagggcgg cttccgctgc 1800

ctggccttcg agtgccctga gaactaccgc cgctccgcag ccacgcagaa atccaagaag 1860

ggaaggcaga acaccccagc gggatcaagt aaagaggact gcagggttct tccatggaag 1920

caggggttgg aggataccca ccttgatgcc tagtgaggaa gatggacctg gacagacagt 1980

cagctccaca ccttgcgctg agcagctgtg attgtgccac gggagcatga gcccttttcc 2040

ccacggccct tgccactgtc tcctggccct ctctctgatc atgccaggtt tgcaccagcc 2100

tcgagtctcc catgttgtag tacattctcc aagatgcagc ccaggagcct ctctgaagga 2160

ccagtctggt tacgatggtc tgagcttcct tagaaccttc catggttgtc ttttcccagc 2220

agatgaagca tagcctcctt ggaatggcat gggaggcctg gcctgatctg gcctctgccc 2280

accctttgag ctgcacctgc cccaccccag ctcatccatg tgcttgtacc ctggccccac 2340

ggggaggctt gcccttccct gaatctgcct tcttgtggct ttagcatgtg ccatgctgtc 2400

cccctgcaga ctgccattct ccttgcagac ttggctcaga agtcacctcc tcagtgcagt 2460

tagcctgagc tcccctggcc ccaggtgcct ccatcagagc atttacccca ttgtgttgtg 2520

gctgttcctt aacgtcccca ctagccaggc tctttgaggg cagggattgt gtctggttaa 2580

tttctgtatt ctctgcaact ttgcaatgtt tggcttgaag aaggagctca gtaaacatct 2640

gaataaacat gcaggttgat gg 2662

<210> 18
<211> 2947
<212> DNA
<213> Homo sapiens

<400> 18

ctcctcccgg gcgggataat tgaacggcgc ggccctggcc cagcgttggc tgccgaggct 60

cggccggagc gtggagcccg cgccgctgcc ccaggaccgc gcccgcgcct ttgtccgccg 120

ccgcccaccg cccgtcgccc gccgcccatg gagcgcgccg cgccgtcgcg ccgggtcccg 180

cttccgctgc tgctgctcgg cggccttgcg ctgctggcgg ccggagtgga cgcggatgtc 240

ctcctggagg cctgctgtgc ggacggacac cggatggcca ctcatcagaa ggactgctcg 300

ctgccatatg ctacggaatc caaagaatgc aggatggtgc aggagcagtg ctgccacagc 360

cagctggagg agctgcactg tgccacgggc atcagcctgg ccaacgagca ggaccgctgt 420

gccacgcccc acggtgacaa cgccagcctg gaggccacat ttgtgaagag gtgctgccat 480

tgctgtctgc tggggagggc ggcccaggcc cagggccaga gctgcgagta cagcctcatg 540

gttggctacc agtgtggaca ggtcttccgg gcatgctgtg tcaagagcca ggagaccgga 600

gatttggatg tcgggggcct ccaagaaacg gataagatca ttgaggttga ggaggaacaa 660

gaggacccat atctgaatga ccgctgccga ggaggcgggc cctgcaagca gcagtgccga 720

gacacgggtg acgaggtggt ctgctcctgc ttcgtgggct accagctgct gtctgatggt 780

gtctcctgtg aagatgtcaa tgaatgcatc acgggcagcc acagctgccg gcttggagaa 840

tcctgcatca acacagtggg ctctttccgc tgccagcggg acagcagctg cgggactggc 900

tatgagctca cagaggacaa tagctgcaaa gatattgacg agtgtgagag tggtattcat 960

aactgcctcc ccgattttat ctgtcagaat actctgggat ccttccgctg ccgacccaag 1020

ctacagtgca agagtggctt tatacaagat gctctaggca actgtattga tatcaatgag 1080

tgtttgagta tcagtgcccc gtgccctatc gggcatacat gcatcaacac agagggctcc 1140

tacacgtgcc agaagaacgt gcccaactgt ggccgtggct accatctcaa cgaggaggga 1200

acgcgctgtg ttgatgtgga cgagtgcgcg ccacctgctg agccctgtgg gaagggacat 1260

cgctgcgtga actctcccgg cagtttccgc tgcgaatgca agacgggtta ctattttgac 1320

ggcatcagca ggatgtgtgt cgatgtcaac gagtgccagc gctaccccgg gcgcctgtgt 1380

ggccacaagt gcgagaacac gctgggctcc tacctctgca gctgttccgt gggcttccgg 1440

ctctctgtgg atggcaggtc atgtgaagac atcaatgagt gcagcagcag cccctgtagc 1500

caggagtgtg ccaacgtcta cggctcctac cagtgttact gccggcgagg ctaccagctc 1560

agcgatgtgg atggagtcac ctgtgaagac atcgacgagt gcgccctgcc caccgggggc 1620

cacatctgct cctaccgctg catcaacatc cctggaagct tccagtgcag ctgcccctcg 1680

tctggctaca ggctggcccc caatggccgc aactgccaag acattgatga gtgtgtgact 1740

ggcatccaca actgctccat caacgagacc tgcttcaaca tccagggcgg cttccgctgc 1800

ctggccttcg agtgccctga gaactaccgc cgctccgcag ccacgctcca gcaggagaag 1860

acagacacgg tccgctgcat caagtcctgc cgccccaacg atgtcacatg cgtgttcgac 1920

cccgtgcaca ccatctccca caccgtcatc tcgctgccta ccttccgcga gttcacccgc 1980

```
cctgaagaga tcatcttcct ccgggccatc acgccaccgc atcctgccag ccaggctaac      2040
atcatcttcg acatcacgga agggaacctg cgggactctt ttgacatcat caagcgttac      2100
atggacggca tgaccgtggg tgtcgtgcgc caggtgcggc ccatcgtggg cccatttcat      2160
gccgtcctga agctggagat gaactatgtg gtcgggggcg tggtctccca ccgaaatgtt      2220
gtcaacgtcc acatcttcgt ctctgagtac tggttctgag ggctggtctg ccgcacagcc      2280
gcaggtgcac ctccaggcca aatcattgct gccagtgact gtggtctgta cttgtttata      2340
ccctcagact tttttaatgt taggtatttg tagcattagg ccaacatgta ttaagctgag      2400
ccagatgaat aagtccatct gatgtatttt cggtgtttaa aaaatgagcc cagttgctca      2460
actgtttggt tgaaaacctt gctcattttt taatgcgaag gctaagtgtc acccccttc       2520
tctgcctctg gctgggcctt gctaagggcc aaggaaagaa agacattttt taggggggcag     2580
ccagtccaaa tgccaaaaga agaccagttc ttgccctgat tgtatgaaat ttgacatttt      2640
ggcacttttt tttttttttt ggccaatcag attttctatg ttctaaggac atggctgctg      2700
tagaatagca cagacgtgga tgataaatta tccccagaag cagcatgaca gaatgcctcg      2760
gggagcactt ggaagggaaa ttgcagttct gttgaaatag aggaaaatcc cttggtaaag      2820
acacagcctg ttaggctcgt gtgggcctcc agtatgttca ccaggggaat ggctgggatt      2880
tctcggcact ctgcatcatc catcttttct tataggtggg aaaataaaca actttgtgat      2940
cctcctg                                                                2947
```

<210> 19
<211> 2485
<212> DNA
<213> Homo sapiens

<400> 19

```
ctcctcccgg gcgggataat tgaacggcgc ggccctggcc cagcgttggc tgccgaggct        60
cggccggagc gtggagcccg cgccgctgcc ccaggaccgc gcccgcgcct ttgtccgccg       120
ccgcccaccg cccgtcgccc gccgcccatg gagcgcgccg cgccgtcgcg ccgggtcccg       180
cttccgctgc tgctgctcgg cggccttgcg ctgctggcgg ccggagtgga cgcggatgtc       240
ctcctggagg cctgctgtgc ggacggacac cggatggcca ctcatcagaa ggactgctcg       300
ctgccatatg ctacggaatc caaagaatgc aggatggtgc aggagcagtg ctgccacagc       360
cagctggagg agctgcactg tgccacgggc atcagcctgg ccaacgagca ggaccgctgt       420
gccacgcccc acggtgacaa cgccagcctg gaggccacat ttgtgaagag gtgctgccat       480
tgctgtctgc tggggagggc ggcccaggcc cagggccaga gctgcgagta cagcctcatg       540
gttggctacc agtgtggaca ggtcttccgg gcatgctgtg tcaagagcca ggagaccgga       600
gatttggatg tcgggggcct ccaagaaacg gataagatca ttgaggttga ggaggaacaa       660
```

gaggacccat atctgaatga ccgctgccga ggaggcgggc cctgcaagca gcagtgccga 720

gacacgggtg acgaggtggt ctgctcctgc ttcgtgggct accagctgct gtctgatggt 780

gtctcctgtg aagatgtcaa tgaatgcatc acgggcagcc acagctgccg gcttggagaa 840

tcctgcatca acacagtggg ctctttccgc tgccagcggg acagcagctg cgggactggc 900

tatgagctca cagaggacaa tagctgcaaa gatattgacg agtgtgagag tggtattcat 960

aactgcctcc ccgattttat ctgtcagaat actctgggat ccttccgctg ccgacccaag 1020

ctacagtgca agagtggctt tatacaagat gctctaggca actgtattga tatcaatgag 1080

tgtttgagta tcagtgcccc gtgccctatc gggcatacat gcatcaacac agagggctcc 1140

tacacgtgcc agaagaacgt gcccaactgt ggccgtggct accatctcaa cgaggaggga 1200

acgcgctgtg ttgatgtgga cgagtgcgcg ccacctgctg agccctgtgg gaagggacat 1260

cgctgcgtga actctcccgg cagtttccgc tgcgaatgca agacgggtta ctattttgac 1320

ggcatcagca ggatgtgtgt cgatgtcaac gagtgccagc gctaccccgg gcgcctgtgt 1380

ggccacaagt gcgagaacac gctgggctcc tacctctgca gctgttccgt gggcttccgg 1440

ctctctgtgg atggcaggtc atgtgaagac atcaatgagt gcagcagcag cccctgtagc 1500

caggagtgtg ccaacgtcta cggctcctac cagtgttact gccggcgagg ctaccagctc 1560

agcgatgtgg atggagtcac ctgtgaagac atcgacgagt gcgccctgcc caccgggggc 1620

cacatctgct cctaccgctg catcaacatc cctggaagct tccagtgcag ctgcccctcg 1680

tctggctaca ggctggcccc caatggccgc aactgccaag acattgatga gtgtgtgact 1740

ggcatccaca actgctccat caacgagacc tgcttcaaca tccagggcgg cttccgctgc 1800

ctggccttcg agtgccctga gaactaccgc cgctccgcag ccacatgatc gtagggaact 1860

ctgcatgagg ccatcggtgc aggctggaga agagaaggca agttggcagg agtggagacc 1920

acgggcattt gagccacttc ctcatgtaac ttaacttgtg ccttcaggac ctgctcaagc 1980

ccgatcacgt atataccact tccatttgat gatggaatgc tgctgttcat gaccaacttt 2040

atggctagat gggtcagaaa gcacccagtt catgataggc agttcaggtc atatggtgac 2100

ttgatgaccc agagtcaaac attcagtttc caccaaagcc cagtaacagg ccaagagctg 2160

tctctcaaaa ggagagtagt tatctgcaga agatggcagg gccttgctcc gaaagcctag 2220

agaccgccac tgtgattcac ctatgggggc ctgccaaagg ctgcagccag catccttatc 2280

tgccactgac acctcaagca acattggatc tgctgggtca tatggcccaa gtggcagagc 2340

aacttgcaca acagcctgga cctgtcatag agctttctcc tgttctggac cccactcaaa 2400

actggcagcc tttcaggtca ctcgataaat gtgctggagt aacactcaaa cgaggaatgt 2460

gttgcctcca aaatccaata ggccc 2485

<210> 20
<211> 566
<212> PRT
<213> Homo sapiens

<400> 20

```
Met Glu Arg Ala Ala Pro Ser Arg Arg Val Pro Leu Pro Leu Leu Leu
1               5                   10                  15

Leu Gly Gly Leu Ala Leu Leu Ala Ala Gly Val Asp Ala Asp Val Leu
            20                  25                  30

Leu Glu Ala Cys Cys Ala Asp Gly His Arg Met Ala Thr His Gln Lys
        35                  40                  45

Asp Cys Ser Leu Pro Tyr Ala Thr Glu Ser Lys Glu Cys Arg Met Val
    50                  55                  60

Gln Glu Gln Cys Cys His Ser Gln Leu Glu Glu Leu His Cys Ala Thr
65                  70                  75                  80

Gly Ile Ser Leu Ala Asn Glu Gln Asp Arg Cys Ala Thr Pro His Gly
                85                  90                  95

Asp Asn Ala Ser Leu Glu Ala Thr Phe Val Lys Arg Cys Cys His Cys
            100                 105                 110

Cys Leu Leu Gly Arg Ala Ala Gln Ala Gln Gly Gln Ser Cys Glu Tyr
        115                 120                 125

Ser Leu Met Val Gly Tyr Gln Cys Gly Gln Val Phe Arg Ala Cys Cys
    130                 135                 140

Val Lys Ser Gln Glu Thr Gly Asp Leu Asp Val Gly Gly Leu Gln Glu
145                 150                 155                 160

Thr Asp Lys Ile Ile Glu Val Glu Glu Glu Gln Glu Asp Pro Tyr Leu
                165                 170                 175

Asn Asp Arg Cys Arg Gly Gly Gly Pro Cys Lys Gln Gln Cys Arg Asp
            180                 185                 190

Thr Gly Asp Glu Val Val Cys Ser Cys Phe Val Gly Tyr Gln Leu Leu
        195                 200                 205

Ser Asp Gly Val Ser Cys Glu Asp Val Asn Glu Cys Ile Thr Gly Ser
    210                 215                 220
```

```
His Ser Cys Arg Leu Gly Glu Ser Cys Ile Asn Thr Val Gly Ser Phe
225                 230                 235                 240

Arg Cys Gln Arg Asp Ser Ser Cys Gly Thr Gly Tyr Glu Leu Thr Glu
                245                 250                 255

Asp Asn Ser Cys Lys Asp Ile Asp Glu Cys Glu Ser Gly Ile His Asn
            260                 265                 270

Cys Leu Pro Asp Phe Ile Cys Gln Asn Thr Leu Gly Ser Phe Arg Cys
        275                 280                 285

Arg Pro Lys Leu Gln Cys Lys Ser Gly Phe Ile Gln Asp Ala Leu Gly
    290                 295                 300

Asn Cys Ile Asp Ile Asn Glu Cys Leu Ser Ile Ser Ala Pro Cys Pro
305                 310                 315                 320

Ile Gly His Thr Cys Ile Asn Thr Glu Gly Ser Tyr Thr Cys Gln Lys
                325                 330                 335

Asn Val Pro Asn Cys Gly Arg Gly Tyr His Leu Asn Glu Glu Gly Thr
            340                 345                 350

Arg Cys Val Asp Val Asp Glu Cys Ala Pro Pro Ala Glu Pro Cys Gly
        355                 360                 365

Lys Gly His Arg Cys Val Asn Ser Pro Gly Ser Phe Arg Cys Glu Cys
    370                 375                 380

Lys Thr Gly Tyr Tyr Phe Asp Gly Ile Ser Arg Met Cys Val Asp Val
385                 390                 395                 400

Asn Glu Cys Gln Arg Tyr Pro Gly Arg Leu Cys Gly His Lys Cys Glu
                405                 410                 415

Asn Thr Leu Gly Ser Tyr Leu Cys Ser Cys Ser Val Gly Phe Arg Leu
            420                 425                 430

Ser Val Asp Gly Arg Ser Cys Glu Asp Ile Asn Glu Cys Ser Ser Ser
        435                 440                 445

Pro Cys Ser Gln Glu Cys Ala Asn Val Tyr Gly Ser Tyr Gln Cys Tyr
    450                 455                 460

Cys Arg Arg Gly Tyr Gln Leu Ser Asp Val Asp Gly Val Thr Cys Glu
465                 470                 475                 480
```

Asp Ile Asp Glu Cys Ala Leu Pro Thr Gly Gly His Ile Cys Ser Tyr
485 490 495

Arg Cys Ile Asn Ile Pro Gly Ser Phe Gln Cys Ser Cys Pro Ser Ser
500 505 510

Gly Tyr Arg Leu Ala Pro Asn Gly Arg Asn Cys Gln Asp Ile Asp Glu
515 520 525

Cys Val Thr Gly Ile His Asn Cys Ser Ile Asn Glu Thr Cys Phe Asn
530 535 540

Ile Gln Gly Gly Phe Arg Cys Leu Ala Phe Glu Cys Pro Glu Asn Tyr
545 550 555 560

Arg Arg Ser Ala Ala Thr
565

<210> 21
<211> 601
<212> PRT
<213> Homo sapiens

<400> 21

Met Glu Arg Ala Ala Pro Ser Arg Arg Val Pro Leu Pro Leu Leu Leu
1 5 10 15

Leu Gly Gly Leu Ala Leu Leu Ala Ala Gly Val Asp Ala Asp Val Leu
20 25 30

Leu Glu Ala Cys Cys Ala Asp Gly His Arg Met Ala Thr His Gln Lys
35 40 45

Asp Cys Ser Leu Pro Tyr Ala Thr Glu Ser Lys Glu Cys Arg Met Val
50 55 60

Gln Glu Gln Cys Cys His Ser Gln Leu Glu Glu Leu His Cys Ala Thr
65 70 75 80

Gly Ile Ser Leu Ala Asn Glu Gln Asp Arg Cys Ala Thr Pro His Gly
85 90 95

Asp Asn Ala Ser Leu Glu Ala Thr Phe Val Lys Arg Cys Cys His Cys
100 105 110

Cys Leu Leu Gly Arg Ala Ala Gln Ala Gln Gly Gln Ser Cys Glu Tyr
115 120 125

Ser Leu Met Val Gly Tyr Gln Cys Gly Gln Val Phe Arg Ala Cys Cys
    130                 135                 140

Val Lys Ser Gln Glu Thr Gly Asp Leu Asp Val Gly Gly Leu Gln Glu
145                 150                 155                 160

Thr Asp Lys Ile Ile Glu Val Glu Glu Glu Gln Glu Asp Pro Tyr Leu
                165                 170                 175

Asn Asp Arg Cys Arg Gly Gly Gly Pro Cys Lys Gln Gln Cys Arg Asp
            180                 185                 190

Thr Gly Asp Glu Val Val Cys Ser Cys Phe Val Gly Tyr Gln Leu Leu
        195                 200                 205

Ser Asp Gly Val Ser Cys Glu Asp Val Asn Glu Cys Ile Thr Gly Ser
    210                 215                 220

His Ser Cys Arg Leu Gly Glu Ser Cys Ile Asn Thr Val Gly Ser Phe
225                 230                 235                 240

Arg Cys Gln Arg Asp Ser Ser Cys Gly Thr Gly Tyr Glu Leu Thr Glu
                245                 250                 255

Asp Asn Ser Cys Lys Asp Ile Asp Glu Cys Glu Ser Gly Ile His Asn
            260                 265                 270

Cys Leu Pro Asp Phe Ile Cys Gln Asn Thr Leu Gly Ser Phe Arg Cys
        275                 280                 285

Arg Pro Lys Leu Gln Cys Lys Ser Gly Phe Ile Gln Asp Ala Leu Gly
    290                 295                 300

Asn Cys Ile Asp Ile Asn Glu Cys Leu Ser Ile Ser Ala Pro Cys Pro
305                 310                 315                 320

Ile Gly His Thr Cys Ile Asn Thr Glu Gly Ser Tyr Thr Cys Gln Lys
                325                 330                 335

Asn Val Pro Asn Cys Gly Arg Gly Tyr His Leu Asn Glu Glu Gly Thr
            340                 345                 350

Arg Cys Val Asp Val Asp Glu Cys Ala Pro Pro Ala Glu Pro Cys Gly
        355                 360                 365

Lys Gly His Arg Cys Val Asn Ser Pro Gly Ser Phe Arg Cys Glu Cys

```
    370                 375                 380

Lys Thr Gly Tyr Tyr Phe Asp Gly Ile Ser Arg Met Cys Val Asp Val
385                 390                 395                 400

Asn Glu Cys Gln Arg Tyr Pro Gly Arg Leu Cys Gly His Lys Cys Glu
                405                 410                 415

Asn Thr Leu Gly Ser Tyr Leu Cys Ser Cys Ser Val Gly Phe Arg Leu
            420                 425                 430

Ser Val Asp Gly Arg Ser Cys Glu Asp Ile Asn Glu Cys Ser Ser Ser
        435                 440                 445

Pro Cys Ser Gln Glu Cys Ala Asn Val Tyr Gly Ser Tyr Gln Cys Tyr
    450                 455                 460

Cys Arg Arg Gly Tyr Gln Leu Ser Asp Val Asp Gly Val Thr Cys Glu
465                 470                 475                 480

Asp Ile Asp Glu Cys Ala Leu Pro Thr Gly Gly His Ile Cys Ser Tyr
                485                 490                 495

Arg Cys Ile Asn Ile Pro Gly Ser Phe Gln Cys Ser Cys Pro Ser Ser
            500                 505                 510

Gly Tyr Arg Leu Ala Pro Asn Gly Arg Asn Cys Gln Asp Ile Asp Glu
        515                 520                 525

Cys Val Thr Gly Ile His Asn Cys Ser Ile Asn Glu Thr Cys Phe Asn
    530                 535                 540

Ile Gln Gly Gly Phe Arg Cys Leu Ala Phe Glu Cys Pro Glu Asn Tyr
545                 550                 555                 560

Arg Arg Ser Ala Ala Thr Gln Lys Ser Lys Lys Gly Arg Gln Asn Thr
                565                 570                 575

Pro Ala Gly Ser Ser Lys Glu Asp Cys Arg Val Leu Pro Trp Lys Gln
            580                 585                 590

Gly Leu Glu Asp Thr His Leu Asp Ala
        595                 600
```

<210> 22
<211> 703
<212> PRT

<213> Homo sapiens

<400> 22

```
Met Glu Arg Ala Ala Pro Ser Arg Arg Val Pro Leu Pro Leu Leu Leu
1               5                   10                  15

Leu Gly Gly Leu Ala Leu Leu Ala Ala Gly Val Asp Ala Asp Val Leu
            20                  25                  30

Leu Glu Ala Cys Cys Ala Asp Gly His Arg Met Ala Thr His Gln Lys
        35                  40                  45

Asp Cys Ser Leu Pro Tyr Ala Thr Glu Ser Lys Glu Cys Arg Met Val
    50                  55                  60

Gln Glu Gln Cys Cys His Ser Gln Leu Glu Glu Leu His Cys Ala Thr
65                  70                  75                  80

Gly Ile Ser Leu Ala Asn Glu Gln Asp Arg Cys Ala Thr Pro His Gly
                85                  90                  95

Asp Asn Ala Ser Leu Glu Ala Thr Phe Val Lys Arg Cys Cys His Cys
            100                 105                 110

Cys Leu Leu Gly Arg Ala Ala Gln Ala Gln Gly Gln Ser Cys Glu Tyr
        115                 120                 125

Ser Leu Met Val Gly Tyr Gln Cys Gly Gln Val Phe Arg Ala Cys Cys
    130                 135                 140

Val Lys Ser Gln Glu Thr Gly Asp Leu Asp Val Gly Gly Leu Gln Glu
145                 150                 155                 160

Thr Asp Lys Ile Ile Glu Val Glu Glu Glu Gln Glu Asp Pro Tyr Leu
                165                 170                 175

Asn Asp Arg Cys Arg Gly Gly Gly Pro Cys Lys Gln Gln Cys Arg Asp
            180                 185                 190

Thr Gly Asp Glu Val Val Cys Ser Cys Phe Val Gly Tyr Gln Leu Leu
        195                 200                 205

Ser Asp Gly Val Ser Cys Glu Asp Val Asn Glu Cys Ile Thr Gly Ser
    210                 215                 220

His Ser Cys Arg Leu Gly Glu Ser Cys Ile Asn Thr Val Gly Ser Phe
225                 230                 235                 240
```

```
Arg Cys Gln Arg Asp Ser Ser Cys Gly Thr Gly Tyr Glu Leu Thr Glu
                245                 250                 255

Asp Asn Ser Cys Lys Asp Ile Asp Glu Cys Glu Ser Gly Ile His Asn
            260                 265                 270

Cys Leu Pro Asp Phe Ile Cys Gln Asn Thr Leu Gly Ser Phe Arg Cys
        275                 280                 285

Arg Pro Lys Leu Gln Cys Lys Ser Gly Phe Ile Gln Asp Ala Leu Gly
    290                 295                 300

Asn Cys Ile Asp Ile Asn Glu Cys Leu Ser Ile Ser Ala Pro Cys Pro
305                 310                 315                 320

Ile Gly His Thr Cys Ile Asn Thr Glu Gly Ser Tyr Thr Cys Gln Lys
                325                 330                 335

Asn Val Pro Asn Cys Gly Arg Gly Tyr His Leu Asn Glu Glu Gly Thr
            340                 345                 350

Arg Cys Val Asp Val Asp Glu Cys Ala Pro Pro Ala Glu Pro Cys Gly
        355                 360                 365

Lys Gly His Arg Cys Val Asn Ser Pro Gly Ser Phe Arg Cys Glu Cys
    370                 375                 380

Lys Thr Gly Tyr Tyr Phe Asp Gly Ile Ser Arg Met Cys Val Asp Val
385                 390                 395                 400

Asn Glu Cys Gln Arg Tyr Pro Gly Arg Leu Cys Gly His Lys Cys Glu
                405                 410                 415

Asn Thr Leu Gly Ser Tyr Leu Cys Ser Cys Ser Val Gly Phe Arg Leu
            420                 425                 430

Ser Val Asp Gly Arg Ser Cys Glu Asp Ile Asn Glu Cys Ser Ser Ser
        435                 440                 445

Pro Cys Ser Gln Glu Cys Ala Asn Val Tyr Gly Ser Tyr Gln Cys Tyr
    450                 455                 460

Cys Arg Arg Gly Tyr Gln Leu Ser Asp Val Asp Gly Val Thr Cys Glu
465                 470                 475                 480

Asp Ile Asp Glu Cys Ala Leu Pro Thr Gly Gly His Ile Cys Ser Tyr
                485                 490                 495
```

Arg Cys Ile Asn Ile Pro Gly Ser Phe Gln Cys Ser Cys Pro Ser Ser
500 505 510

Gly Tyr Arg Leu Ala Pro Asn Gly Arg Asn Cys Gln Asp Ile Asp Glu
515 520 525

Cys Val Thr Gly Ile His Asn Cys Ser Ile Asn Glu Thr Cys Phe Asn
530 535 540

Ile Gln Gly Gly Phe Arg Cys Leu Ala Phe Glu Cys Pro Glu Asn Tyr
545 550 555 560

Arg Arg Ser Ala Ala Thr Leu Gln Gln Glu Lys Thr Asp Thr Val Arg
565 570 575

Cys Ile Lys Ser Cys Arg Pro Asn Asp Val Thr Cys Val Phe Asp Pro
580 585 590

Val His Thr Ile Ser His Thr Val Ile Ser Leu Pro Thr Phe Arg Glu
595 600 605

Phe Thr Arg Pro Glu Glu Ile Ile Phe Leu Arg Ala Ile Thr Pro Pro
610 615 620

His Pro Ala Ser Gln Ala Asn Ile Ile Phe Asp Ile Thr Glu Gly Asn
625 630 635 640

Leu Arg Asp Ser Phe Asp Ile Ile Lys Arg Tyr Met Asp Gly Met Thr
645 650 655

Val Gly Val Val Arg Gln Val Arg Pro Ile Val Gly Pro Phe His Ala
660 665 670

Val Leu Lys Leu Glu Met Asn Tyr Val Val Gly Gly Val Val Ser His
675 680 685

Arg Asn Val Val Asn Val His Ile Phe Val Ser Glu Tyr Trp Phe
690 695 700

<210> 23
<211> 566
<212> PRT
<213> Homo sapiens

<400> 23

Met Glu Arg Ala Ala Pro Ser Arg Arg Val Pro Leu Pro Leu Leu Leu
1 5 10 15

```
Leu Gly Gly Leu Ala Leu Leu Ala Ala Gly Val Asp Ala Asp Val Leu
            20                  25                  30

Leu Glu Ala Cys Cys Ala Asp Gly His Arg Met Ala Thr His Gln Lys
        35                  40                  45

Asp Cys Ser Leu Pro Tyr Ala Thr Glu Ser Lys Glu Cys Arg Met Val
    50                  55                  60

Gln Glu Gln Cys Cys His Ser Gln Leu Glu Glu Leu His Cys Ala Thr
65                  70                  75                  80

Gly Ile Ser Leu Ala Asn Glu Gln Asp Arg Cys Ala Thr Pro His Gly
                85                  90                  95

Asp Asn Ala Ser Leu Glu Ala Thr Phe Val Lys Arg Cys Cys His Cys
            100                 105                 110

Cys Leu Leu Gly Arg Ala Ala Gln Ala Gln Gly Gln Ser Cys Glu Tyr
        115                 120                 125

Ser Leu Met Val Gly Tyr Gln Cys Gly Gln Val Phe Arg Ala Cys Cys
    130                 135                 140

Val Lys Ser Gln Glu Thr Gly Asp Leu Asp Val Gly Gly Leu Gln Glu
145                 150                 155                 160

Thr Asp Lys Ile Ile Glu Val Glu Glu Glu Gln Glu Asp Pro Tyr Leu
                165                 170                 175

Asn Asp Arg Cys Arg Gly Gly Gly Pro Cys Lys Gln Gln Cys Arg Asp
            180                 185                 190

Thr Gly Asp Glu Val Val Cys Ser Cys Phe Val Gly Tyr Gln Leu Leu
        195                 200                 205

Ser Asp Gly Val Ser Cys Glu Asp Val Asn Glu Cys Ile Thr Gly Ser
    210                 215                 220

His Ser Cys Arg Leu Gly Glu Ser Cys Ile Asn Thr Val Gly Ser Phe
225                 230                 235                 240

Arg Cys Gln Arg Asp Ser Ser Cys Gly Thr Gly Tyr Glu Leu Thr Glu
                245                 250                 255

Asp Asn Ser Cys Lys Asp Ile Asp Glu Cys Glu Ser Gly Ile His Asn
            260                 265                 270
```

```
Cys Leu Pro Asp Phe Ile Cys Gln Asn Thr Leu Gly Ser Phe Arg Cys
        275                 280                 285

Arg Pro Lys Leu Gln Cys Lys Ser Gly Phe Ile Gln Asp Ala Leu Gly
    290                 295                 300

Asn Cys Ile Asp Ile Asn Glu Cys Leu Ser Ile Ser Ala Pro Cys Pro
305                 310                 315                 320

Ile Gly His Thr Cys Ile Asn Thr Glu Gly Ser Tyr Thr Cys Gln Lys
                325                 330                 335

Asn Val Pro Asn Cys Gly Arg Gly Tyr His Leu Asn Glu Glu Gly Thr
            340                 345                 350

Arg Cys Val Asp Val Asp Glu Cys Ala Pro Pro Ala Glu Pro Cys Gly
        355                 360                 365

Lys Gly His Arg Cys Val Asn Ser Pro Gly Ser Phe Arg Cys Glu Cys
    370                 375                 380

Lys Thr Gly Tyr Tyr Phe Asp Gly Ile Ser Arg Met Cys Val Asp Val
385                 390                 395                 400

Asn Glu Cys Gln Arg Tyr Pro Gly Arg Leu Cys Gly His Lys Cys Glu
                405                 410                 415

Asn Thr Leu Gly Ser Tyr Leu Cys Ser Cys Ser Val Gly Phe Arg Leu
            420                 425                 430

Ser Val Asp Gly Arg Ser Cys Glu Asp Ile Asn Glu Cys Ser Ser Ser
        435                 440                 445

Pro Cys Ser Gln Glu Cys Ala Asn Val Tyr Gly Ser Tyr Gln Cys Tyr
    450                 455                 460

Cys Arg Arg Gly Tyr Gln Leu Ser Asp Val Asp Gly Val Thr Cys Glu
465                 470                 475                 480

Asp Ile Asp Glu Cys Ala Leu Pro Thr Gly Gly His Ile Cys Ser Tyr
                485                 490                 495

Arg Cys Ile Asn Ile Pro Gly Ser Phe Gln Cys Ser Cys Pro Ser Ser
            500                 505                 510

Gly Tyr Arg Leu Ala Pro Asn Gly Arg Asn Cys Gln Asp Ile Asp Glu
```

```
                515                 520                 525

Cys Val Thr Gly Ile His Asn Cys Ser Ile Asn Glu Thr Cys Phe Asn
    530                 535                 540

Ile Gln Gly Gly Phe Arg Cys Leu Ala Phe Glu Cys Pro Glu Asn Tyr
545                 550                 555                 560

Arg Arg Ser Ala Ala Thr
                565
```

<210> 24
<211> 17
<212> DNA
<213> Artificial Sequence

<220>
<223> FBLN1_forward primer

<400> 24
gcctggaggc cacattt 17

<210> 25
<211> 20
<212> DNA
<213> Artificial Sequence

<220>
<223> FBLN1_reverse primer

<400> 25
ccaaccatga ggctgtactc 20

<210> 26
<211> 21
<212> DNA
<213> Artificial Sequence

<220>
<223> FBLN1 probe

<400> 26
tgctgccatt gctgtctgct g 21

<210> 27
<211> 1797
<212> DNA
<213> Homo sapiens

<400> 27

```
agggacggag gcccgcgctg cccaagagcg ccacgggcgg ggcggggccg gcggcgggct      60
gcgggcgcgg ccggacggga gttccccgga gaaggatcct gcagcccgag tcccgtcctc     120
aggcttcccc aatccagggg actcggcgcc gggacgctgc tatggacgac attttcactc     180
agtgccggga gggcaacgca gtcgccgttc gcctgtggct ggacaacacg gagaacgacc     240
tcaaccaggg ggacgatcat ggcttctccc ccttgcactg ggcctgccga gagggccgct     300
ctgctgtggt tgagatgttg atcatgcggg gggcacggat caatgtaatg aaccgtgggg     360
atgacacccc cctgcatctg gcagccagtc atggacaccg tgatattgta cagaagctat     420
tgcagtacaa ggcagacatc aatgcagtga atgaacacgg gaatgtgccc ctgcactatg     480
cctgtttttg gggccaagat caagtggcag aggacctggt ggcaaatggg gcccttgtca     540
gcatctgtaa caagtatgga gagatgcctg tggacaaagc caaggcaccc ctgagagagc     600
ttctccgaga gcgggcagag aagatgggcc agaatctcaa ccgtattcca tacaaggaca     660
cattctggaa ggggaccacc cgcactcggc cccgaaatgg aaccctgaac aaacactctg     720
gcattgactt caaacagctt aacttcctga cgaagctcaa cgagaatcac tctggagagc     780
tatggaaggg ccgctggcag ggcaatgaca ttgtcgtgaa ggtgctgaag gttcgagact     840
ggagtacaag gaagagcagg gacttcaatg aagagtgtcc ccggctcagg attttctcgc     900
atccaaatgt gctcccagtg ctaggtgcct gccagtctcc acctgctcct catcctactc     960
tcatcacaca ctggatgccg tatggatccc tctacaatgt actacatgaa ggcaccaatt    1020
tcgtcgtgga ccagagccag gctgtgaagt ttgctttgga catggcaagg ggcatggcct    1080
tcctacacac actagagccc ctcatcccac gacatgcact caatagccgt agtgtaatga    1140
ttgatgagga catgactgcc cgaattagca tggctgatgt caagttctct ttccaatgtc    1200
ctggtcgcat gtatgcacct gcctgggtag cccccgaagc tctgcagaag aagcctgaag    1260
acacaaacag acgctcagca gacatgtgga gttttgcagt gcttctgtgg gaactggtga    1320
cacgggaggt accctttgct gacctctcca atatggagat tggaatgaag gtggcattgg    1380
aaggccttcg gcctaccatc ccaccaggta tttcccctca tgtgtgtaag ctcatgaaga    1440
tctgcatgaa tgaagaccct gcaaagcgac ccaaatttga catgattgtg cctatccttg    1500
agaagatgca ggacaagtag gactggaagg tccttgcctg aactccagag gtgtcgggac    1560
atggttgggg gaatgcacct ccccaaagca gcaggcctct ggttgcctcc cccgcctcca    1620
gtcatggtac taccccagcc atggggtcca tccccttccc ccatccctac cactgtggcc    1680
ccaagagggg cgggctcaga gctttgtcac ttgccacatg gtgtctccca acatgggagg    1740
gatcagcccc gcctgtcaca ataaagttta ttatgaaaac aggaaaaaaa aaaaaaa       1797
```

<210> 28

<211> 2098
<212> DNA
<213> Homo sapiens

<400> 28

agcccgagtc ccggtgagtg cgagaggacc cgcgccccct gtcacccctc acttctcctg 60

agtcccaagt aaggaacgga gccaaccctg gggaggaccc ccggtcccct ctcccagagt 120
atacggagcc tgaacctccc cacttccccc aactctgttc gcggataggg tctagttgcc 180
tgctctcgga catccgttca gcagacacta cctcttcgtc acccccctgcc caccctgacc 240
cgcctttacc tcgcgtctag aggacacagc cagggatcat cccgcagccc cgaactcctt 300
cacagacccc cactagccgg ggacgcagct caggcccccct acccccaaca caaacacttc 360
tctcctgtag aggataaagc ttggggttca tcctccttcc ctggatcact ccacagtcct 420
caggcttccc caatccaggg gactcggcgc cgggacgctg ctatggacga cattttcact 480
cagtgccggg agggcaacgc agtcgccgtt cgcctgtggc tggacaacac ggagaacgac 540
ctcaaccagg gggacgatca tggcttctcc cccttgcact gggcctgccg agagggccgc 600
tctgctgtgg ttgagatgtt gatcatgcgg ggggcacgga tcaatgtaat gaaccgtggg 660
gatgacaccc ccctgcatct ggcagccagt catggacacc gtgatattgt acagaagcta 720
ttgcagtaca aggcagacat caatgcagtg aatgaacacg ggaatgtgcc cctgcactat 780
gcctgttttt ggggccaaga tcaagtggca gaggacctgg tggcaaatgg ggcccttgtc 840
agcatctgta acaagtatgg agagatgcct gtggacaaag ccaaggcacc cctgagagag 900
cttctccgag agcgggcaga gaagatgggc cagaatctca accgtattcc atacaaggac 960
acattctgga aggggaccac ccgcactcgg ccccgaaatg gaaccctgaa caaacactct 1020
ggcattgact tcaaacagct taacttcctg acgaagctca acgagaatca ctctggagag 1080
ctatggaagg gccgctggca gggcaatgac attgtcgtga aggtgctgaa ggttcgagac 1140
tggagtacaa ggaagagcag ggacttcaat gaagagtgtc cccggctcag gatttctcg 1200
catccaaatg tgctcccagt gctaggtgcc tgccagtctc cacctgctcc tcatcctact 1260
ctcatcacac actggatgcc gtatggatcc ctctacaatg tactacatga aggcaccaat 1320
ttcgtcgtgg accagagcca ggctgtgaag tttgctttgg acatggcaag gggcatggcc 1380
ttcctacaca cactagagcc cctcatccca cgacatgcac tcaatagccg tagtgtaatg 1440
attgatgagg acatgactgc ccgaattagc atggctgatg tcaagttctc tttccaatgt 1500
cctggtcgca tgtatgcacc tgcctgggta gcccccgaag ctctgcagaa gaagcctgaa 1560
gacacaaaca gacgctcagc agacatgtgg agttttgcag tgcttctgtg ggaactggtg 1620
acacgggagg taccctttgc tgacctctcc aatatggaga ttggaatgaa ggtggcattg 1680
gaaggccttc ggcctaccat cccaccaggt atttcccctc atgtgtgtaa gctcatgaag 1740
atctgcatga atgaagaccc tgcaaagcga cccaaatttg acatgattgt gcctatcctt 1800
gagaagatgc aggacaagta ggactggaag gtccttgcct gaactccaga ggtgtcggga 1860
catggttggg ggaatgcacc tccccaaagc agcaggcctc tggttgcctc cccgcctcc 1920
agtcatggta ctaccccagc catggggtcc atccccttcc cccatcccta ccactgtggc 1980

cccaagaggg gcgggctcag agctttgtca cttgccacat ggtgtctccc aacatgggag 2040

ggatcagccc cgcctgtcac aataaagttt attatgaaaa caggaaaaaa aaaaaaaa 2098

<210> 29
<211> 1660
<212> DNA
<213> Homo sapiens

<400> 29

agggacggag gcccgcgctg cccaagagcg ccacgggcgg ggcggggccg gcggcgggct 60
gcgggcgcgg ccggacggga gttccccgga gaaggatcct gcagcccgag tcccgaggat 120
aaagcttggg gttcatcctc cttccctgga tcactccaca gtcctcaggc ttccccaatc 180
caggggactc ggcgccggga cgctgctatg gacgacattt tcactcagtg ccgggagggc 240
aacgcagtcg ccgttcgcct gtggctggac aacacggaga acgacctcaa ccaggggac 300
gatcatggct tctcccctt gcactgggcc tgccgagagg gccgctctgc tgtggttgag 360
atgttgatca tgcggggggc acggatcaat gtaatgaacc gtggggatga cacccccctg 420
catctggcag ccagtcatgg acaccgtgat attgtacaga agctattgca gtacaaggca 480
gacatcaatg cagtgaatga acacgggaat gtgcccctgc actatgcctg tttttggggc 540
caagatcaag tggcagagag cgggcagaga agatgggcca gaatctcaac cgtattccat 600
acaaggacac attctggaag gggaccaccc gcactcggcc ccctatggaa gggccgctgg 660
cagggcaatg acattgtcgt gaaggtgctg aaggttcgag actggagtac aaggaagagc 720
agggacttca atgaagagtg tccccggctc aggattttct cgcatccaaa tgtgctccca 780
gtgctaggtg cctgccagtc tccacctgct cctcatccta ctctcatcac acactggatg 840
ccgtatggat ccctctacaa tgtactacat gaaggcacca atttcgtcgt ggaccagagc 900
caggctgtga agtttgcttt ggacatggca aggggcatgg ccttcctaca cacactagag 960
cccctcatcc cacgacatgc actcaatagc cgtagtgtaa tgattgatga ggacatgact 1020
gcccgaatta gcatggctga tgtcaagttc tctttccaat gtcctggtcg catgtatgca 1080
cctgcctggg tagcccccga agctctgcag aagaagcctg aagacacaaa cagacgctca 1140
gcagacatgt ggagttttgc agtgcttctg tgggaactgg tgacacggga ggtacccttt 1200
gctgacctct ccaatatgga gattggaatg aaggtggcat tggaaggcct tcggcctacc 1260
atcccaccag gtatttcccc tcatgtgtgt aagctcatga agatctgcat gaatgaagac 1320
cctgcaaagc gacccaaatt tgacatgatt gtgcctatcc ttgagaagat gcaggacaag 1380
taggactgga aggtccttgc ctgaactcca gaggtgtcgg gacatggttg ggggaatgca 1440
cctccccaaa gcagcaggcc tctggttgcc tcccccgcct ccagtcatgg tactacccca 1500
gccatggggt ccatcccctt cccccatccc taccactgtg gccccaagag gggcgggctc 1560
agagctttgt cacttgccac atggtgtctc ccaacatggg agggatcagc cccgcctgtc 1620
acaataaagt ttattatgaa aacaggaaaa aaaaaaaaaa 1660

<210> 30
<211> 1631
<212> DNA
<213> Homo sapiens

<400> 30

```
agggacggag gcccgcgctg cccaagagcg ccacgggcgg ggcggggccg gcggcgggct      60
gcgggcgcgg ccggacggga gttccccgga gaaggatcct gcagcccgag tcccgtcctc     120
aggcttcccc aatccagggg actcggcgcc gggacgctgc tatggacgac attttcactc     180
agtgccggga gggcaacgca gtcgccgttc gcctgtggct ggacaacacg gagaacgacc     240
tcaaccaggg ctattgcagt acaaggcaga catcaatgca gtgaatgaac acgggaatgt     300
gcccctgcac tatgcctgtt tttggggcca agatcaagtg gcagaggacc tggtggcaaa     360
tggggccctt gtcagcatct gtaacaagta tggagagatg cctgtggaca aagccaaggc     420
acccctgaga gagcttctcc gagagcgggc agagaagatg ggccagaatc tcaaccgtat     480
tccatacaag gacacattct ggaaggggac cacccgcact cggccccgaa atggaaccct     540
gaacaaacac tctggcattg acttcaaaca gcttaacttc ctgacgaagc tcaacgagaa     600
tcactctgga gagctatgga agggccgctg gcagggcaat gacattgtcg tgaaggtgct     660
gaaggttcga gactggagta caaggaagag cagggacttc aatgaagagt gtccccggct     720
caggattttc tcgcatccaa atgtgctccc agtgctaggt gcctgccagt ctccacctgc     780
tcctcatcct actctcatca cacactggat gccgtatgga tccctctaca atgtactaca     840
tgaaggcacc aatttcgtcg tggaccagag ccaggctgtg aagtttgctt tggacatggc     900
aaggggcatg gccttcctac acacactaga gcccctcatc ccacgacatg cactcaatag     960
ccgtagtgta atgattgatg aggacatgac tgcccgaatt agcatggctg atgtcaagtt    1020
ctctttccaa tgtcctggtc gcatgtatgc acctgcctgg gtagcccccg aagctctgca    1080
gaagaagcct gaagacacaa acagacgctc agcagacatg tggagttttg cagtgcttct    1140
gtgggaactg gtgacacggg aggtaccctt tgctgacctc tccaatatgg agattggaat    1200
gaaggtggca ttggaaggcc ttcggcctac catcccacca ggtatttccc ctcatgtgtg    1260
taagctcatg aagatctgca tgaatgaaga ccctgcaaag cgacccaaat ttgacatgat    1320
tgtgcctatc cttgagaaga tgcaggacaa gtaggactgg aaggtccttg cctgaactcc    1380
agaggtgtcg ggacatggtt gggggaatgc acctccccaa agcagcaggc ctctggttgc    1440
ctcccccgcc tccagtcatg gtactacccc agccatgggg tccatcccct tcccccatcc    1500
ctaccactgt ggccccaaga ggggcgggct cagagctttg tcacttgcca catggtgtct    1560
cccaacatgg gagggatcag ccccgcctgt cacaataaag tttattatga aaacaggaaa    1620
aaaaaaaaaa a                                                          1631
```

<210> 31
<211> 1843
<212> DNA

<213> Homo sapiens

<400> 31

```
agggacggag gcccgcgctg cccaagagcg ccacgggcgg ggcggggccg gcggcgggct      60
gcgggcgcgg ccggacggga gttccccgga gaaggatcct gcagcccgag tcccgaggat     120
aaagcttggg gttcatcctc cttccctgga tcactccaca gtcctcaggc ttccccaatc     180
caggggactc ggcgccggga cgctgctatg gacgacattt tcactcagtg ccgggagggc     240
aacgcagtcg ccgttcgcct gtggctggac aacacggaga acgacctcaa ccaggggac      300
gatcatggct tctccccctt gcactgggcc tgccgagagg gccgctctgc tgtggttgag     360
atgttgatca tgcgggggc acggatcaat gtaatgaacc gtggggatga cacccccctg      420
catctggcag ccagtcatgg acaccgtgat attgtacaga agctattgca gtacaaggca     480
gacatcaatg cagtgaatga acacgggaat gtgcccctgc actatgcctg tttttggggc     540
caagatcaag tggcagagga cctggtggca aatggggccc ttgtcagcat ctgtaacaag     600
tatggagaga tgcctgtgga caaagccaag gcacccctga gagagcttct ccgagagcgg     660
gcagagaaga tgggccagaa tctcaaccgt attccataca aggacacatt ctggaagggg     720
accacccgca ctcggccccg aaatggaacc ctgaacaaac actctggcat tgacttcaaa     780
cagcttaact tcctgacgaa gctcaacgag aatcactctg gagagctatg gaagggccgc     840
tggcagggca atgacattgt cgtgaaggtg ctgaaggttc gagactggag tacaaggaag     900
agcagggact tcaatgaaga gtgtccccgg ctcaggattt tctcgcatcc aaatgtgctc     960
ccagtgctag gtgcctgcca gtctccacct gctcctcatc ctactctcat cacacactgg    1020
atgccgtatg gatccctcta caatgtacta catgaaggca ccaatttcgt cgtggaccag    1080
agccaggctg tgaagtttgc tttggacatg gcaaggggca tggccttcct acacacacta    1140
gagcccctca tcccacgaca tgcactcaat agccgtagtg taatgattga tgaggacatg    1200
actgcccgaa ttagcatggc tgatgtcaag ttctctttcc aatgtcctgg tcgcatgtat    1260
gcacctgcct gggtagcccc cgaagctctg cagaagaagc ctgaagacac aaacagacgc    1320
tcagcagaca tgtggagttt tgcagtgctt ctgtgggaac tggtgacacg ggaggtaccc    1380
tttgctgacc tctccaatat ggagattgga atgaaggtgg cattggaagg ccttcggcct    1440
accatcccac caggtatttc ccctcatgtg tgtaagctca tgaagatctg catgaatgaa    1500
gaccctgcaa agcgacccaa atttgacatg attgtgccta tccttgagaa gatgcaggac    1560
```

```
aagtaggact ggaaggtcct tgcctgaact ccagaggtgt cgggacatgg ttgggggaat     1620

gcacctcccc aaagcagcag gcctctggtt gcctccccccg cctccagtca tggtactacc     1680

ccagccatgg ggtccatccc cttcccccat ccctaccact gtggccccaa gaggggcggg     1740

ctcagagctt tgtcacttgc cacatggtgt ctcccaacat gggagggatc agccccgcct     1800

gtcacaataa agtttattat gaaaacagga aaaaaaaaaa aaa                       1843
```

<210> 32
<211> 452
<212> PRT
<213> Homo sapiens

<400> 32

```
Met Asp Asp Ile Phe Thr Gln Cys Arg Glu Gly Asn Ala Val Ala Val
1               5                   10                  15

Arg Leu Trp Leu Asp Asn Thr Glu Asn Asp Leu Asn Gln Gly Asp Asp
            20                  25                  30

His Gly Phe Ser Pro Leu His Trp Ala Cys Arg Glu Gly Arg Ser Ala
        35                  40                  45

Val Val Glu Met Leu Ile Met Arg Gly Ala Arg Ile Asn Val Met Asn
    50                  55                  60

Arg Gly Asp Asp Thr Pro Leu His Leu Ala Ala Ser His Gly His Arg
65                  70                  75                  80

Asp Ile Val Gln Lys Leu Leu Gln Tyr Lys Ala Asp Ile Asn Ala Val
                85                  90                  95

Asn Glu His Gly Asn Val Pro Leu His Tyr Ala Cys Phe Trp Gly Gln
            100                 105                 110

Asp Gln Val Ala Glu Asp Leu Val Ala Asn Gly Ala Leu Val Ser Ile
        115                 120                 125

Cys Asn Lys Tyr Gly Glu Met Pro Val Asp Lys Ala Lys Ala Pro Leu
    130                 135                 140

Arg Glu Leu Leu Arg Glu Arg Ala Glu Lys Met Gly Gln Asn Leu Asn
145                 150                 155                 160

Arg Ile Pro Tyr Lys Asp Thr Phe Trp Lys Gly Thr Thr Arg Thr Arg
                165                 170                 175
```

```
Pro Arg Asn Gly Thr Leu Asn Lys His Ser Gly Ile Asp Phe Lys Gln
            180                 185                 190

Leu Asn Phe Leu Thr Lys Leu Asn Glu Asn His Ser Gly Glu Leu Trp
        195                 200                 205

Lys Gly Arg Trp Gln Gly Asn Asp Ile Val Val Lys Val Leu Lys Val
    210                 215                 220

Arg Asp Trp Ser Thr Arg Lys Ser Arg Asp Phe Asn Glu Glu Cys Pro
225                 230                 235                 240

Arg Leu Arg Ile Phe Ser His Pro Asn Val Leu Pro Val Leu Gly Ala
                245                 250                 255

Cys Gln Ser Pro Pro Ala Pro His Pro Thr Leu Ile Thr His Trp Met
            260                 265                 270

Pro Tyr Gly Ser Leu Tyr Asn Val Leu His Glu Gly Thr Asn Phe Val
        275                 280                 285

Val Asp Gln Ser Gln Ala Val Lys Phe Ala Leu Asp Met Ala Arg Gly
    290                 295                 300

Met Ala Phe Leu His Thr Leu Glu Pro Leu Ile Pro Arg His Ala Leu
305                 310                 315                 320

Asn Ser Arg Ser Val Met Ile Asp Glu Asp Met Thr Ala Arg Ile Ser
                325                 330                 335

Met Ala Asp Val Lys Phe Ser Phe Gln Cys Pro Gly Arg Met Tyr Ala
            340                 345                 350

Pro Ala Trp Val Ala Pro Glu Ala Leu Gln Lys Lys Pro Glu Asp Thr
        355                 360                 365

Asn Arg Arg Ser Ala Asp Met Trp Ser Phe Ala Val Leu Leu Trp Glu
    370                 375                 380

Leu Val Thr Arg Glu Val Pro Phe Ala Asp Leu Ser Asn Met Glu Ile
385                 390                 395                 400

Gly Met Lys Val Ala Leu Glu Gly Leu Arg Pro Thr Ile Pro Pro Gly
                405                 410                 415

Ile Ser Pro His Val Cys Lys Leu Met Lys Ile Cys Met Asn Glu Asp
            420                 425                 430
```

Pro Ala Lys Arg Pro Lys Phe Asp Met Ile Val Pro Ile Leu Glu Lys
435 440 445

Met Gln Asp Lys
450

<210> 33
<211> 452
<212> PRT
<213> Homo sapiens

<400> 33

Met Asp Asp Ile Phe Thr Gln Cys Arg Glu Gly Asn Ala Val Ala Val
1 5 10 15

Arg Leu Trp Leu Asp Asn Thr Glu Asn Asp Leu Asn Gln Gly Asp Asp
20 25 30

His Gly Phe Ser Pro Leu His Trp Ala Cys Arg Glu Gly Arg Ser Ala
35 40 45

Val Val Glu Met Leu Ile Met Arg Gly Ala Arg Ile Asn Val Met Asn
50 55 60

Arg Gly Asp Asp Thr Pro Leu His Leu Ala Ala Ser His Gly His Arg
65 70 75 80

Asp Ile Val Gln Lys Leu Leu Gln Tyr Lys Ala Asp Ile Asn Ala Val
85 90 95

Asn Glu His Gly Asn Val Pro Leu His Tyr Ala Cys Phe Trp Gly Gln
100 105 110

Asp Gln Val Ala Glu Asp Leu Val Ala Asn Gly Ala Leu Val Ser Ile
115 120 125

Cys Asn Lys Tyr Gly Glu Met Pro Val Asp Lys Ala Lys Ala Pro Leu
130 135 140

Arg Glu Leu Leu Arg Glu Arg Ala Glu Lys Met Gly Gln Asn Leu Asn
145 150 155 160

Arg Ile Pro Tyr Lys Asp Thr Phe Trp Lys Gly Thr Thr Arg Thr Arg
165 170 175

Pro Arg Asn Gly Thr Leu Asn Lys His Ser Gly Ile Asp Phe Lys Gln
180 185 190

```
Leu Asn Phe Leu Thr Lys Leu Asn Glu Asn His Ser Gly Glu Leu Trp
        195                 200                 205

Lys Gly Arg Trp Gln Gly Asn Asp Ile Val Val Lys Val Leu Lys Val
    210                 215                 220

Arg Asp Trp Ser Thr Arg Lys Ser Arg Asp Phe Asn Glu Glu Cys Pro
225                 230                 235                 240

Arg Leu Arg Ile Phe Ser His Pro Asn Val Leu Pro Val Leu Gly Ala
                245                 250                 255

Cys Gln Ser Pro Pro Ala Pro His Pro Thr Leu Ile Thr His Trp Met
            260                 265                 270

Pro Tyr Gly Ser Leu Tyr Asn Val Leu His Glu Gly Thr Asn Phe Val
        275                 280                 285

Val Asp Gln Ser Gln Ala Val Lys Phe Ala Leu Asp Met Ala Arg Gly
    290                 295                 300

Met Ala Phe Leu His Thr Leu Glu Pro Leu Ile Pro Arg His Ala Leu
305                 310                 315                 320

Asn Ser Arg Ser Val Met Ile Asp Glu Asp Met Thr Ala Arg Ile Ser
                325                 330                 335

Met Ala Asp Val Lys Phe Ser Phe Gln Cys Pro Gly Arg Met Tyr Ala
            340                 345                 350

Pro Ala Trp Val Ala Pro Glu Ala Leu Gln Lys Lys Pro Glu Asp Thr
        355                 360                 365

Asn Arg Arg Ser Ala Asp Met Trp Ser Phe Ala Val Leu Leu Trp Glu
    370                 375                 380

Leu Val Thr Arg Glu Val Pro Phe Ala Asp Leu Ser Asn Met Glu Ile
385                 390                 395                 400

Gly Met Lys Val Ala Leu Glu Gly Leu Arg Pro Thr Ile Pro Pro Gly
                405                 410                 415

Ile Ser Pro His Val Cys Lys Leu Met Lys Ile Cys Met Asn Glu Asp
            420                 425                 430

Pro Ala Lys Arg Pro Lys Phe Asp Met Ile Val Pro Ile Leu Glu Lys
        435                 440                 445
```

Met Gln Asp Lys
450

<210> 34
<211> 391
<212> PRT
<213> Homo sapiens

<400> 34

Met Asp Asp Ile Phe Thr Gln Cys Arg Glu Gly Asn Ala Val Ala Val
1 5 10 15

Arg Leu Trp Leu Asp Asn Thr Glu Asn Asp Leu Asn Gln Gly Asp Asp
20 25 30

His Gly Phe Ser Pro Leu His Trp Ala Cys Arg Glu Gly Arg Ser Ala
35 40 45

Val Val Glu Met Leu Ile Met Arg Gly Ala Arg Ile Asn Val Met Asn
50 55 60

Arg Gly Asp Asp Thr Pro Leu His Leu Ala Ala Ser His Gly His Arg
65 70 75 80

Asp Ile Val Gln Lys Leu Leu Gln Tyr Lys Ala Asp Ile Asn Ala Val
85 90 95

Asn Glu His Gly Asn Val Pro Leu His Tyr Ala Cys Phe Trp Gly Gln
100 105 110

Asp Gln Val Ala Glu Ser Gly Gln Arg Arg Trp Ala Arg Ile Ser Thr
115 120 125

Val Phe His Thr Arg Thr His Ser Gly Arg Gly Pro Pro Ala Leu Gly
130 135 140

Pro Leu Trp Lys Gly Arg Trp Gln Gly Asn Asp Ile Val Val Lys Val
145 150 155 160

Leu Lys Val Arg Asp Trp Ser Thr Arg Lys Ser Arg Asp Phe Asn Glu
165 170 175

Glu Cys Pro Arg Leu Arg Ile Phe Ser His Pro Asn Val Leu Pro Val
180 185 190

Leu Gly Ala Cys Gln Ser Pro Pro Ala Pro His Pro Thr Leu Ile Thr
195 200 205

```
His Trp Met Pro Tyr Gly Ser Leu Tyr Asn Val Leu His Glu Gly Thr
    210                 215                 220

Asn Phe Val Val Asp Gln Ser Gln Ala Val Lys Phe Ala Leu Asp Met
225                 230                 235                 240

Ala Arg Gly Met Ala Phe Leu His Thr Leu Glu Pro Leu Ile Pro Arg
                245                 250                 255

His Ala Leu Asn Ser Arg Ser Val Met Ile Asp Glu Asp Met Thr Ala
            260                 265                 270

Arg Ile Ser Met Ala Asp Val Lys Phe Ser Phe Gln Cys Pro Gly Arg
        275                 280                 285

Met Tyr Ala Pro Ala Trp Val Ala Pro Glu Ala Leu Gln Lys Lys Pro
    290                 295                 300

Glu Asp Thr Asn Arg Arg Ser Ala Asp Met Trp Ser Phe Ala Val Leu
305                 310                 315                 320

Leu Trp Glu Leu Val Thr Arg Glu Val Pro Phe Ala Asp Leu Ser Asn
                325                 330                 335

Met Glu Ile Gly Met Lys Val Ala Leu Glu Gly Leu Arg Pro Thr Ile
            340                 345                 350

Pro Pro Gly Ile Ser Pro His Val Cys Lys Leu Met Lys Ile Cys Met
        355                 360                 365

Asn Glu Asp Pro Ala Lys Arg Pro Lys Phe Asp Met Ile Val Pro Ile
    370                 375                 380

Leu Glu Lys Met Gln Asp Lys
385                 390
```

<210> 35
<211> 318
<212> PRT
<213> Homo sapiens

<400> 35

```
Met Pro Val Asp Lys Ala Lys Ala Pro Leu Arg Glu Leu Leu Arg Glu
1               5                   10                  15

Arg Ala Glu Lys Met Gly Gln Asn Leu Asn Arg Ile Pro Tyr Lys Asp
            20                  25                  30
```

```
Thr Phe Trp Lys Gly Thr Thr Arg Thr Arg Pro Arg Asn Gly Thr Leu
        35                  40                  45

Asn Lys His Ser Gly Ile Asp Phe Lys Gln Leu Asn Phe Leu Thr Lys
    50                  55                  60

Leu Asn Glu Asn His Ser Gly Glu Leu Trp Lys Gly Arg Trp Gln Gly
65                  70                  75                  80

Asn Asp Ile Val Val Lys Val Leu Lys Val Arg Asp Trp Ser Thr Arg
                85                  90                  95

Lys Ser Arg Asp Phe Asn Glu Glu Cys Pro Arg Leu Arg Ile Phe Ser
            100                 105                 110

His Pro Asn Val Leu Pro Val Leu Gly Ala Cys Gln Ser Pro Pro Ala
        115                 120                 125

Pro His Pro Thr Leu Ile Thr His Trp Met Pro Tyr Gly Ser Leu Tyr
    130                 135                 140

Asn Val Leu His Glu Gly Thr Asn Phe Val Val Asp Gln Ser Gln Ala
145                 150                 155                 160

Val Lys Phe Ala Leu Asp Met Ala Arg Gly Met Ala Phe Leu His Thr
                165                 170                 175

Leu Glu Pro Leu Ile Pro Arg His Ala Leu Asn Ser Arg Ser Val Met
            180                 185                 190

Ile Asp Glu Asp Met Thr Ala Arg Ile Ser Met Ala Asp Val Lys Phe
        195                 200                 205

Ser Phe Gln Cys Pro Gly Arg Met Tyr Ala Pro Ala Trp Val Ala Pro
    210                 215                 220

Glu Ala Leu Gln Lys Lys Pro Glu Asp Thr Asn Arg Arg Ser Ala Asp
225                 230                 235                 240

Met Trp Ser Phe Ala Val Leu Leu Trp Glu Leu Val Thr Arg Glu Val
                245                 250                 255

Pro Phe Ala Asp Leu Ser Asn Met Glu Ile Gly Met Lys Val Ala Leu
            260                 265                 270

Glu Gly Leu Arg Pro Thr Ile Pro Pro Gly Ile Ser Pro His Val Cys
```

275 280 285

Lys Leu Met Lys Ile Cys Met Asn Glu Asp Pro Ala Lys Arg Pro Lys
290 295 300

Phe Asp Met Ile Val Pro Ile Leu Glu Lys Met Gln Asp Lys
305 310 315

<210> 36
<211> 452
<212> PRT
<213> Homo sapiens

<400> 36

```
Met Asp Asp Ile Phe Thr Gln Cys Arg Glu Gly Asn Ala Val Ala Val
1               5                   10                  15

Arg Leu Trp Leu Asp Asn Thr Glu Asn Asp Leu Asn Gln Gly Asp Asp
            20                  25                  30

His Gly Phe Ser Pro Leu His Trp Ala Cys Arg Glu Gly Arg Ser Ala
        35                  40                  45

Val Val Glu Met Leu Ile Met Arg Gly Ala Arg Ile Asn Val Met Asn
    50                  55                  60

Arg Gly Asp Asp Thr Pro Leu His Leu Ala Ala Ser His Gly His Arg
65                  70                  75                  80

Asp Ile Val Gln Lys Leu Leu Gln Tyr Lys Ala Asp Ile Asn Ala Val
                85                  90                  95

Asn Glu His Gly Asn Val Pro Leu His Tyr Ala Cys Phe Trp Gly Gln
            100                 105                 110

Asp Gln Val Ala Glu Asp Leu Val Ala Asn Gly Ala Leu Val Ser Ile
        115                 120                 125

Cys Asn Lys Tyr Gly Glu Met Pro Val Asp Lys Ala Lys Ala Pro Leu
    130                 135                 140

Arg Glu Leu Leu Arg Glu Arg Ala Glu Lys Met Gly Gln Asn Leu Asn
145                 150                 155                 160

Arg Ile Pro Tyr Lys Asp Thr Phe Trp Lys Gly Thr Thr Arg Thr Arg
                165                 170                 175

Pro Arg Asn Gly Thr Leu Asn Lys His Ser Gly Ile Asp Phe Lys Gln
```

```
            180                 185                 190

Leu Asn Phe Leu Thr Lys Leu Asn Glu Asn His Ser Gly Glu Leu Trp
        195                 200                 205

Lys Gly Arg Trp Gln Gly Asn Asp Ile Val Val Lys Val Leu Lys Val
    210                 215                 220

Arg Asp Trp Ser Thr Arg Lys Ser Arg Asp Phe Asn Glu Glu Cys Pro
225                 230                 235                 240

Arg Leu Arg Ile Phe Ser His Pro Asn Val Leu Pro Val Leu Gly Ala
                245                 250                 255

Cys Gln Ser Pro Pro Ala Pro His Pro Thr Leu Ile Thr His Trp Met
            260                 265                 270

Pro Tyr Gly Ser Leu Tyr Asn Val Leu His Glu Gly Thr Asn Phe Val
        275                 280                 285

Val Asp Gln Ser Gln Ala Val Lys Phe Ala Leu Asp Met Ala Arg Gly
    290                 295                 300

Met Ala Phe Leu His Thr Leu Glu Pro Leu Ile Pro Arg His Ala Leu
305                 310                 315                 320

Asn Ser Arg Ser Val Met Ile Asp Glu Asp Met Thr Ala Arg Ile Ser
                325                 330                 335

Met Ala Asp Val Lys Phe Ser Phe Gln Cys Pro Gly Arg Met Tyr Ala
            340                 345                 350

Pro Ala Trp Val Ala Pro Glu Ala Leu Gln Lys Lys Pro Glu Asp Thr
        355                 360                 365

Asn Arg Arg Ser Ala Asp Met Trp Ser Phe Ala Val Leu Leu Trp Glu
    370                 375                 380

Leu Val Thr Arg Glu Val Pro Phe Ala Asp Leu Ser Asn Met Glu Ile
385                 390                 395                 400

Gly Met Lys Val Ala Leu Glu Gly Leu Arg Pro Thr Ile Pro Pro Gly
                405                 410                 415

Ile Ser Pro His Val Cys Lys Leu Met Lys Ile Cys Met Asn Glu Asp
            420                 425                 430
```

Pro Ala Lys Arg Pro Lys Phe Asp Met Ile Val Pro Ile Leu Glu Lys
435 440 445

Met Gln Asp Lys
450

<210> 37
<211> 21
<212> DNA
<213> Artificial Sequence

<220>
<223> ILK_forward primer

<400> 37
catccaaatg tgctcccagt g 21

<210> 38
<211> 20
<212> DNA
<213> Artificial Sequence

<220>
<223> ILK_reverse primer

<400> 38
tggctctggt ccacgacgaa 20

<210> 39
<211> 22
<212> DNA
<213> Artificial Sequence

<220>
<223> ILK probe

<400> 39
tgcctgccag tctccacctg ct 22

<210> 40
<211> 1861
<212> DNA
<213> Homo sapiens

<400> 40

```
cactcaaggt gtgcaggcag ctgtgtttgt caggaaggca gaaggagttg gctttgcttt        60
aggggaggag acgaggtccc acaacaccct ctgaagggta tataaggagc cccagcgtgc       120
agcctggcct ggtacctcct gccagcatct cttgggtttg ctgagaactc acgggctcca       180
gctacctggc catgaccacc acatttctgc aaacttcttc ctccaccttt gggggtggct       240
caacccgagg gggttccctc ctggctgggg gaggtggctt tggtgggggg agtctctctg       300
ggggaggtgg aagccgaagt atctcagctt cttctgctag gtttgtctct tcagggtcag       360
gaggaggata tgggggtggc atgagggtct gtggctttgg tggaggggct ggtagtgttt       420
```

tcggtggagg ctttggaggg ggcgttggtg ggggttttgg tggtggcttt ggtggtggcg 480

atggtggtct cctctctggc aatgagaaaa ttaccatgca gaacctcaat gaccgcctgg 540

cctcctacct ggacaaggta cgtgccctgg aggaggccaa tgctgacctg gaggtgaaga 600

tccatgactg gtaccagaag cagaccccaa ccagcccaga atgcgactac agccaatact 660

tcaagaccat tgaagagctc cgggacaaga tcatggccac caccatcgac aactcccggg 720

tcatcctgga gatcgacaat gccaggctgg ctgcggacga cttcaggctc aagtatgaga 780

atgagctggc cctgcgccag ggcgttgagg ctgacatcaa cggcttgcgc cgagtcctgg 840

atgagctgac cctggccagg actgacctgg agatgcagat cgagggcctg aatgaggagc 900

tagcctacct gaagaagaac cacgaagagg agatgaagga gttcagcagc cagctggccg 960

gccaggtcaa tgtggagatg gacgcagcac cgggtgtgga cctgacccgt gtgctggcag 1020

agatgaggga gcagtacgag gccatggcgg agaagaaccg ccgggatgtc gaggcctggt 1080

tcttcagcaa gactgaggag ctgaacaaag aggtggcctc caacacagaa atgatccaga 1140

ccagcaagac ggagatcaca gacctgagac gcacgatgca ggagctggag atcgagctgc 1200

agtcccagct cagcatgaaa gctgggctgg agaactcact ggccgagaca gagtgccgct 1260

atgccacgca gctgcagcag atccaggggc tcattggtgg cctggaggcc cagctgagtg 1320

agctccgatg cgagatggag gctcagaacc aggagtacaa gatgctgctt gacataaaga 1380

cacggctgga gcaggagatc gctacttacc gcagcctgct cgagggccag gatgccaaga 1440

tggctggcat tggcatcagg gaagcctctt caggaggtgg tggtagcagc agcaatttcc 1500

acatcaatgt agaagagtca gtggatggac aggtggtttc ttcccacaag agagaaatct 1560

aagtgtctat tgcaggagaa acgtcccttg ccactcccca ctctcatcag gccaagtgga 1620

ggactggcca gagggcctgc acatgcaaac tccagtccct gccttcagag agctgaaaag 1680

ggtccctcgg tcttttattt cagggctttg catgcgctct attcccctc tgcctctccc 1740

caccttcttt ggagcaagga gatgcagctg tattgtgtaa caagctcatt tgtacagtgt 1800

ctgttcatgt aataaagaat tacttttcct tttgcaaata aaaaaaaaaa aaaaaaaaaa 1860

a 1861

<210> 41
<211> 456
<212> PRT
<213> Homo sapiens

<400> 41

Met Thr Thr Thr Phe Leu Gln Thr Ser Ser Ser Thr Phe Gly Gly Gly
1 5 10 15

```
Ser Thr Arg Gly Gly Ser Leu Leu Ala Gly Gly Gly Gly Phe Gly Gly
            20                  25                  30

Gly Ser Leu Ser Gly Gly Gly Gly Ser Arg Ser Ile Ser Ala Ser Ser
        35                  40                  45

Ala Arg Phe Val Ser Ser Gly Ser Gly Gly Gly Tyr Gly Gly Gly Met
    50                  55                  60

Arg Val Cys Gly Phe Gly Gly Gly Ala Gly Ser Val Phe Gly Gly Gly
65                  70                  75                  80

Phe Gly Gly Gly Val Gly Gly Gly Phe Gly Gly Gly Phe Gly Gly Gly
                85                  90                  95

Asp Gly Gly Leu Leu Ser Gly Asn Glu Lys Ile Thr Met Gln Asn Leu
            100                 105                 110

Asn Asp Arg Leu Ala Ser Tyr Leu Asp Lys Val Arg Ala Leu Glu Glu
        115                 120                 125

Ala Asn Ala Asp Leu Glu Val Lys Ile His Asp Trp Tyr Gln Lys Gln
    130                 135                 140

Thr Pro Thr Ser Pro Glu Cys Asp Tyr Ser Gln Tyr Phe Lys Thr Ile
145                 150                 155                 160

Glu Glu Leu Arg Asp Lys Ile Met Ala Thr Thr Ile Asp Asn Ser Arg
                165                 170                 175

Val Ile Leu Glu Ile Asp Asn Ala Arg Leu Ala Ala Asp Asp Phe Arg
            180                 185                 190

Leu Lys Tyr Glu Asn Glu Leu Ala Leu Arg Gln Gly Val Glu Ala Asp
        195                 200                 205

Ile Asn Gly Leu Arg Arg Val Leu Asp Glu Leu Thr Leu Ala Arg Thr
    210                 215                 220

Asp Leu Glu Met Gln Ile Glu Gly Leu Asn Glu Glu Leu Ala Tyr Leu
225                 230                 235                 240

Lys Lys Asn His Glu Glu Glu Met Lys Glu Phe Ser Ser Gln Leu Ala
                245                 250                 255

Gly Gln Val Asn Val Glu Met Asp Ala Ala Pro Gly Val Asp Leu Thr
            260                 265                 270
```

```
Arg Val Leu Ala Glu Met Arg Glu Gln Tyr Glu Ala Met Ala Glu Lys
        275                 280                 285

Asn Arg Arg Asp Val Glu Ala Trp Phe Phe Ser Lys Thr Glu Glu Leu
    290                 295                 300

Asn Lys Glu Val Ala Ser Asn Thr Glu Met Ile Gln Thr Ser Lys Thr
305                 310                 315                 320

Glu Ile Thr Asp Leu Arg Arg Thr Met Gln Glu Leu Glu Ile Glu Leu
                325                 330                 335

Gln Ser Gln Leu Ser Met Lys Ala Gly Leu Glu Asn Ser Leu Ala Glu
            340                 345                 350

Thr Glu Cys Arg Tyr Ala Thr Gln Leu Gln Gln Ile Gln Gly Leu Ile
        355                 360                 365

Gly Gly Leu Glu Ala Gln Leu Ser Glu Leu Arg Cys Glu Met Glu Ala
    370                 375                 380

Gln Asn Gln Glu Tyr Lys Met Leu Leu Asp Ile Lys Thr Arg Leu Glu
385                 390                 395                 400

Gln Glu Ile Ala Thr Tyr Arg Ser Leu Leu Glu Gly Gln Asp Ala Lys
                405                 410                 415

Met Ala Gly Ile Gly Ile Arg Glu Ala Ser Ser Gly Gly Gly Gly Ser
            420                 425                 430

Ser Ser Asn Phe His Ile Asn Val Glu Glu Ser Val Asp Gly Gln Val
        435                 440                 445

Val Ser Ser His Lys Arg Glu Ile
    450                 455
```

<210> 42
<211> 21
<212> DNA
<213> Artificial Sequence

<220>
<223> KRT15_forward primer

<400> 42
gctagcctac ctgaagaaga a 21

<210> 43
<211> 18
<212> DNA

<213> Artificial Sequence

<220>
<223> KRT15_reverse primer

<400> 43
ccagctggct gctgaact 18

<210> 44
<211> 20
<212> DNA
<213> Artificial Sequence

<220>
<223> KRT15 probe

<400> 44
ccacgaagag gagatgaagg 20

<210> 45
<211> 3123
<212> DNA
<213> Homo sapiens

<400> 45

```
ttggctacat cggacccaga tgactgcctc ctcacttcct ccctcccgat tccgccgcgg      60
cccccaaaga ctctcggggt ggccccttgt ccgcaccgct tggagggagt gtgctctgag     120
ttaagctggt ctcttctggt cctggaaaaa aatgagtatt gacaaggttg ctggatctgc     180
gcagaaaaga aagtgccact taataaaaaa tttagcccgg cagtggtacc gtctgcagag     240
cttgctgccc ttggacgtta gcaggaagcc ttcggggtgc tgtaatcggc gggcagagga     300
gagggaggcc gcggaattaa aaggaacaaa agctagagcg ccatgccaaa cgtccccggc     360
aagacccagt taggcaggag ccgggagtga tgggaaaatg aactagaaca tattggagaa     420
ttgggagaag tctctttggt ttggaaaaaa aaaaaaggaa tcttcagcct agatcacttt     480
cttatccgga ctgggatatt aaatatacga cacatccagg agtttattgg agcgcagact     540
gatggcgcaa aggtacgatg agctgcccca ttacggcggg atggacggag taggggttcc     600
cgcttccatg tacggagacc ctcacgcgcc gcggccgatc cccccggttc accacctgaa     660
ccacgggccg ccgctccacg ccacacagca ctacggcgcg cacgccccgc accccaatgt     720
catgccggcc agtatgggat ccgctgtcaa cgacgccttg aagcgggaca aggacgcgat     780
ctatgggcac ccgttgtttc ctctgttagc tctggtcttt gagaagtgcg agctggcgac     840
ctgcactccc cgggaacctg gagtggctgg cggagacgtc tgctcctccg actccttcaa     900
cgaggacatc gcggtcttcg ccaagcaggt tcgcgccgaa aagccacttt tttcctcaaa     960
tccagagctg gacaatttga tgatacaagc aatacaagta ctaaggtttc atcttttgga    1020
gttagaaaag gtccacgaac tgtgcgataa cttctgccac cgatacatta gctgtttgaa    1080
```

ggggaaaatg cccatcgacc tcgtcattga tgaaagagac ggcagctcca agtcagatca 1140
tgaagaactt tcaggctcct ccacaaatct cgctgaccat aacccttctt cttggcgaga 1200
ccacgatgat gcaacctcaa cccactcagc aggcacccca gggccctcca gtgggggcca 1260
tgcttcccag agcggagaca acagcagtga gcaaggggat ggtttagaca acagtgtagc 1320
ttcacctggt acaggtgacg atgatgatcc ggataaggac aaaaaacgcc agaagaaaag 1380
aggcattttc cccaaagtag caacaaatat catgagagca tggctcttcc agcatctcac 1440
acatccgtac ccttccgaag agcagaagaa acagttagcg caagacacag gacttacaat 1500
tctccaagta aacaactggt ttattaatgc cagaagaaga atagtacagc ccatgattga 1560
ccagtcaaat cgagcagtga gccaaggagc agcatatagt ccagagggtc agcccatggg 1620
gagctttgtg ttggatggtc agcaacacat ggggatccgg cctgcaggtt tgcagagcat 1680
gccaggggac tacgtttctc agggtggtcc tatgggaatg agtatggcac agccaagtta 1740
cactcctccc cagatgaccc cacaccctac tcaattaaga catggacccc caatgcattc 1800
atatttgcca agccatcccc accacccagc catgatgatg cacggaggac cccctaccca 1860
ccctggaatg actatgtcag cacagagccc cacaatgtta aattctgtag atcccaatgt 1920
tggcggacag gttatggaca ttcatgccca atagtataag ggaactcaag ggaaaaggaa 1980
acacacgcaa aaactatttt aagactttct gaactttgac cagatgttga cacttaatat 2040
gaaattccag acagctgtga ttattttta cttttgtcat tttcatcaa gcaacagagg 2100
accaatgcaa caagaacaca aatgtgaaat catgggctga ctgagacaat tctgtccatg 2160
taaagatcct ctggaaaaag actccgagag ttataactac tgtagtataa atataggaac 2220
taagttaaac ttgtacattt ctgttgatca cgccgttatg ttgcctcaaa tagttttaga 2280
agagaaaaaa aaatatatcc ttgttttcca cactatgtgt gttgttccca aaagaatgac 2340
tgttttggtt catcagtgaa ttcaccatcc aggagagact gtggtatata ttttaaacct 2400
gttgggccaa tgagaaaaga accacactgg agatcatgat gaacttttgg ctgaacctca 2460
tcactcgaac tccagcttca agaatgtgtt ttcatgcccg gcctttgttc ctccataaat 2520
gtgtccttta gtttcaaaca gatctttata gttcgtgctt cataagccaa ttcttattat 2580
tatttttggg ggactcttct tcaaagagct tgccaatgaa gatttaaaga cagagcagga 2640
gcttcttcca ggagttctga gccttggttg tggacaaaac aatcttaagt tgggcagctt 2700
tcctcaacac aaaaaaaagt tattaatggt cattgaacca taactaggac tttatcagaa 2760
actcaaagct tgggggataa aaaggagcaa gagaatactg taacaaactt cgtacagagt 2820
tcggtctatt aattgtttca tgttagatat tctatgtgtt tacctcaatt gaaaaaaaaa 2880
agaatgtttt tgctagtatc agatctgctg tggaattggt attgtatgtc catgaattct 2940

```
tcttttctca gcacgtgttc ctcactagaa gaaaatgctg ttacctttaa gctttgtcaa     3000
atttacatta aaatacttgt atgaggactg tgacgttatg ttaaaaaaaa aaaggtgtta     3060
agtcacaaaa agcggtaata aatatttcat tttgatttt ttgttaaaaa aaaaaaaaaa      3120
aaa                                                                   3123
```

<210> 46
<211> 3074
<212> DNA
<213> Homo sapiens

<400> 46

ttggctacat cggacccaga tgactgcctc ctcacttcct ccctcccgat tccgccgcgg 60
cccccaaaga ctctcggggt ggccccttgt ccgcaccgct tggagggagt gtgctctgag 120
ttaagctggt ctcttctggt cctggaaaaa aatgagtatt gacaaggttg ctggatctgc 180
gcagaaaaga aagtgccact taataaaaaa tttagcccgg cagtggtacc gtctgcagag 240
cttgctgccc ttggacgtta gcaggaagcc ttcggggtgc tgtaatcggc gggcagagga 300
gagggaggcc gcggaattaa aaggaacaaa agctagagcg ccatgccaaa cgtccccggc 360
aagacccagt taggcaggag ccgggagtga tgggaaaatg aactagaata cgatgagctg 420
ccccattacg gcgggatgga cggagtaggg gttcccgctt ccatgtacgg agaccctcac 480
gcgccgcggc cgatccccc ggttcaccac ctgaaccacg ggccgccgct ccacgccaca 540
cagcactacg gcgcgcacgc cccgcacccc aatgtcatgc cggccagtat gggatccgct 600
gtcaacgacg ccttgaagcg ggacaaggac gcgatctatg ggcacccgtt gtttcctctg 660
ttagctctgg tctttgagaa gtgcgagctg gcgacctgca ctccccggga acctggagtg 720
gctggcggag acgtctgctc ctccgactcc ttcaacgagg acatcgcggt cttcgccaag 780
caggttcgcg ccgaaaagcc actttttcc tcaaatccag agctggacaa tttgatgata 840
caagcaatac aagtactaag gtttcatctt ttggagttag aaaaggtcca cgaactgtgc 900
gataacttct gccaccgata cattagctgt ttgaagggga aaatgcccat cgacctcgtc 960
attgatgaaa gagacggcag ctccaagtca gatcatgaag aactttcagg ctcctccaca 1020
aatctcgctg accataaccc ttcttcttgg cgagaccacg atgatgcaac ctcaacccac 1080
tcagcaggca ccccagggcc ctccagtggg ggccatgctt cccagagcgg agacaacagc 1140
agtgagcaag ggatggttt agacaacagt gtagcttcac ctggtacagg tgacgatgat 1200
gatccggata aggacaaaaa acgccagaag aaaagaggca ttttccccaa agtagcaaca 1260
aatatcatga gagcatggct cttccagcat ctcacacatc cgtacccttc cgaagagcag 1320
aagaaacagt tagcgcaaga cacaggactt acaattctcc aagtaaacaa ctggtttatt 1380
aatgccagaa gaagaatagt acagcccatg attgaccagt caaatcgagc agtgagccaa 1440

ggagcagcat atagtccaga gggtcagccc atggggagct ttgtgttgga tggtcagcaa 1500

cacatgggga tccggcctgc aggacctatg agtggaatgg gcatgaatat gggcatggat 1560

gggcaatggc actacatgta accttcatca tgtaaagcaa tcgcaaagca agggggaagt 1620

ttgcagagca tgccagggga ctacgtttct cagggtggtc ctatgggaat gagtatggca 1680

cagccaagtt acactcctcc ccagatgacc ccacacccta ctcaattaag acatggaccc 1740

ccaatgcatt catatttgcc aagccatccc caccacccag ccatgatgat gcacggagga 1800

ccccctaccc accctggaat gactatgtca gcacagagcc ccacaatgtt aaattctgta 1860

gatcccaatg ttggcggaca ggttatggac attcatgccc aatagtataa gggaactcaa 1920

gggaaaagga aacacacgca aaaactattt taagactttc tgaactttga ccagatgttg 1980

acacttaata tgaaattcca gacagctgtg attatttttt acttttgtca tttttcatca 2040

agcaacagag gaccaatgca acaagaacac aaatgtgaaa tcatgggctg actgagacaa 2100

ttctgtccat gtaaagatcc tctggaaaaa gactccgaga gttataacta ctgtagtata 2160

aatataggaa ctaagttaaa cttgtacatt tctgttgatc acgccgttat gttgcctcaa 2220

atagttttag aagagaaaaa aaaatatatc cttgttttcc acactatgtg tgttgttccc 2280

aaaagaatga ctgttttggt tcatcagtga attcaccatc caggagagac tgtggtatat 2340

attttaaacc tgttgggcca atgagaaaag aaccacactg gagatcatga tgaacttttg 2400

gctgaacctc atcactcgaa ctccagcttc aagaatgtgt tttcatgccc ggcctttgtt 2460

cctccataaa tgtgtccttt agtttcaaac agatctttat agttcgtgct tcataagcca 2520

attcttatta ttatttttgg gggactcttc ttcaaagagc ttgccaatga agatttaaag 2580

acagagcagg agcttcttcc aggagttctg agccttggtt gtggacaaaa caatcttaag 2640

ttgggcagct ttcctcaaca caaaaaaaag ttattaatgg tcattgaacc ataactagga 2700

ctttatcaga aactcaaagc ttgggggata aaaaggagca agagaatact gtaacaaact 2760

tcgtacagag ttcggtctat taattgtttc atgttagata ttctatgtgt ttacctcaat 2820

tgaaaaaaaa aagaatgttt ttgctagtat cagatctgct gtggaattgg tattgtatgt 2880

ccatgaattc ttcttttctc agcacgtgtt cctcactaga agaaaatgct gttaccttta 2940

agctttgtca aatttacatt aaaatacttg tatgaggact gtgacgttat gttaaaaaaa 3000

aaaaggtgtt aagtcacaaa aagcggtaat aaatatttca tttttgattt tttgttaaaa 3060

aaaaaaaaaa aaaa 3074

<210> 47
<211> 3352
<212> DNA
<213> Homo sapiens

<400> 47

tttaaagaga gaggatcgta ttatagatac cgcgggggca aagctaaaaa aggggggagg 60
ggggaggaaa aaattcaaga agcagaaacc cctcgcggag ttttactgga agaaaaaaac 120
gggtctgaaa gattcctcct cttcatcatc atcaaccatc attcattcac taccttgaca 180
ttccgggctt tgattgacag ctggagtggc aaaaagccat gaaacacgac agttcggtta 240
catgtgggct gctgacgggc cgctcgtaac cttcagttcg gggcttgac aatttttttc 300
ttctttttct tctttttctt ttctttcttt ttttttccaa ctgaggggaa gagaagagaa 360
agagggggaa aggaggaccg aagaggagga ggagggggagg ggggaggagg aggaggtgga 420
ggaggaggag gaagatcagg aggaggagga agaagaggaa aaaagagaaa aagaagaaat 480
atcacagaaa aaaaaattct tcgttgtcta gactgggctt tttttccccc ctaaaaaata 540
gcatattgga gaattgggag aagtctcttt ggtttggaaa aaaaaaaaag gaatcttcag 600
cctagatcac tttcttatcc ggactgggat attaaatata cgacacatcc aggagtttat 660
tggagcgcag actgatggcg caaaggtacg atgagctgcc ccattacggc gggatggacg 720
gagtaggggt tcccgcttcc atgtacggag accctcacgc gccgcggccg atcccccgg 780
ttcaccacct gaaccacggg ccgccgctcc acgccacaca gcactacggc gcgcacgccc 840
cgcaccccaa tgtcatgccg gccagtatgg gatccgctgt caacgacgcc ttgaagcggg 900
acaaggacgc gatctatggg cacccgttgt ttcctctgtt agctctggtc tttgagaagt 960
gcgagctggc gacctgcact ccccgggaac ctggagtggc tggcggagac gtctgctcct 1020
ccgactcctt caacgaggac atcgcggtct tcgccaagca ggttcgcgcc gaaaagccac 1080
tttttttcctc aaatccagag ctggacaatt tgatgataca agcaatacaa gtactaaggt 1140
ttcatctttt ggagttagaa aaggtccacg aactgtgcga taacttctgc caccgataca 1200
ttagctgttt gaaggggaaa atgcccatcg acctcgtcat tgatgaaaga gacggcagct 1260
ccaagtcaga tcatgaagaa ctttcaggct cctccacaaa tctcgctgac cataaccctt 1320
cttcttggcg agaccacgat gatgcaacct caacccactc agcaggcacc ccagggccct 1380
ccagtggggg ccatgcttcc cagagcggag acaacagcag tgagcaaggg gatggtttag 1440
acaacagtgt agcttcacct ggtacaggtg acgatgatga tccggataag gacaaaaaac 1500
gccagaagaa aagaggcatt ttccccaaag tagcaacaaa tatcatgaga gcatggctct 1560
tccagcatct cacacatccg taccccttccg aagagcagaa gaaacagtta gcgcaagaca 1620
caggacttac aattctccaa gtaaacaact ggtttattaa tgccagaaga agaatagtac 1680
agcccatgat tgaccagtca aatcgagcag tgagccaagg agcagcatat agtccagagg 1740
gtcagcccat ggggagcttt gtgttggatg gtcagcaaca catggggatc cggcctgcag 1800
gacctatgag tggaatgggc atgaatatgg gcatggatgg gcaatggcac tacatgtaac 1860
cttcatcatg taaagcaatc gcaaagcaag gggaagttt gcagagcatg ccaggggact 1920

```
acgtttctca gggtggtcct atgggaatga gtatggcaca gccaagttac actcctcccc      1980
agatgacccc acaccctact caattaagac atggaccccc aatgcattca tatttgccaa      2040
gccatcccca ccacccagcc atgatgatgc acggaggacc ccctacccac cctggaatga      2100
ctatgtcagc acagagcccc acaatgttaa attctgtaga tcccaatgtt ggcggacagg      2160
ttatggacat tcatgcccaa tagtataagg gaactcaagg gaaaaggaaa cacacgcaaa      2220
aactatttta agactttctg aactttgacc agatgttgac acttaatatg aaattccaga      2280
cagctgtgat tattttttac ttttgtcatt tttcatcaag caacagagga ccaatgcaac      2340
aagaacacaa atgtgaaatc atgggctgac tgagacaatt ctgtccatgt aaagatcctc      2400
tggaaaaaga ctccgagagt tataactact gtagtataaa tataggaact aagttaaact      2460
tgtacatttc tgttgatcac gccgttatgt tgcctcaaat agttttagaa gagaaaaaaa      2520
aatatatcct tgttttccac actatgtgtg ttgttcccaa aagaatgact gttttggttc      2580
atcagtgaat tcaccatcca ggagagactg tggtatatat tttaaacctg ttgggccaat      2640
gagaaaagaa ccacactgga gatcatgatg aacttttggc tgaacctcat cactcgaact      2700
ccagcttcaa gaatgtgttt tcatgcccgg cctttgttcc tccataaatg tgtcctttag      2760
tttcaaacag atctttatag ttcgtgcttc ataagccaat tcttattatt atttttgggg      2820
gactcttctt caaagagctt gccaatgaag atttaaagac agagcaggag cttcttccag      2880
gagttctgag ccttggttgt ggacaaaaca atcttaagtt gggcagcttt cctcaacaca      2940
aaaaaaagtt attaatggtc attgaaccat aactaggact ttatcagaaa ctcaaagctt      3000
gggggataaa aaggagcaag agaatactgt aacaaacttc gtacagagtt cggtctatta      3060
attgtttcat gttagatatt ctatgtgttt acctcaattg aaaaaaaaaa gaatgttttt      3120
gctagtatca gatctgctgt ggaattggta ttgtatgtcc atgaattctt cttttctcag      3180
cacgtgttcc tcactagaag aaaatgctgt tacctttaag ctttgtcaaa tttacattaa      3240
aatacttgta tgaggactgt gacgttatgt taaaaaaaaa aaggtgttaa gtcacaaaaa      3300
gcggtaataa atatttcatt tttgattttt tgttaaaaaa aaaaaaaaaa aa              3352
```

<210> 48
<211> 3277
<212> DNA
<213> Homo sapiens

<400> 48

```
tttaaagaga gaggatcgta ttatagatac cgcgggggca aagctaaaaa aggggggagg      60
ggggaggaaa aaattcaaga agcagaaacc cctcgcggag ttttactgga agaaaaaaac     120
gggtctgaaa gattcctcct cttcatcatc atcaaccatc attcattcac taccttgaca     180
ttccgggctt tgattgacag ctggagtggc aaaaagccat gaaacacgac agttcggtta     240
```

catgtgggct gctgacgggc cgctcgtaac cttcagttcg ggggcttgac aatttttttc 300
ttctttttct tctttttctt ttctttcttt ttttttccaa ctgaggggaa gagaagagaa 360
agagggggaa aggaggaccg aagaggagga ggaggggagg ggggaggagg aggaggtgga 420
ggaggaggag gaagatcagg aggaggagga agaagaggaa aaaagagaaa aagaagaaat 480
atcacagaaa aaaaaattct tcgttgtcta gactgggctt tttttccccc ctaaaaaata 540
gcatattgga gaattgggag aagtctcttt ggtttggaaa aaaaaaaaag gaatcttcag 600
cctagatcac tttcttatcc ggactgggat attaaatata cgacacatcc aggagtttat 660
tggagcgcag actgatggcg caaaggtacg atgagctgcc ccattacggc gggatggacg 720
gagtaggggt tcccgcttcc atgtacggag accctcacgc gccgcggccg atccccccgg 780
ttcaccacct gaaccacggg ccgccgctcc acgccacaca gcactacggc gcgcacgccc 840
cgcaccccaa tgtcatgccg gccagtatgg gatccgctgt caacgacgcc ttgaagcggg 900
acaaggacgc gatctatggg cacccgttgt ttcctctgtt agctctggtc tttgagaagt 960
gcgagctggc gacctgcact ccccgggaac ctggagtggc tggcggagac gtctgctcct 1020
ccgactcctt caacgaggac atcgcggtct tcgccaagca ggttcgcgcc gaaaagccac 1080
ttttttcctc aaatccagag ctggacaatt tgatgataca agcaatacaa gtactaaggt 1140
ttcatctttt ggagttagaa aaggtccacg aactgtgcga taacttctgc caccgataca 1200
ttagctgttt gaaggggaaa atgcccatcg acctcgtcat tgatgaaaga gacggcagct 1260
ccaagtcaga tcatgaagaa ctttcaggct cctccacaaa tctcgctgac cataaccctt 1320
cttcttggcg agaccacgat gatgcaacct caacccactc agcaggcacc ccagggccct 1380
ccagtggggg ccatgcttcc cagagcggag acaacagcag tgagcaaggg gatggtttag 1440
acaacagtgt agcttcacct ggtacaggtg acgatgatga tccggataag gacaaaaaac 1500
gccagaagaa aagaggcatt ttccccaaag tagcaacaaa tatcatgaga gcatggctct 1560
tccagcatct cacacatccg tacccttccg aagagcagaa gaaacagtta gcgcaagaca 1620
caggacttac aattctccaa gtaaacaact ggtttattaa tgccagaaga agaatagtac 1680
agcccatgat tgaccagtca aatcgagcag gttttcttct tgatccttca gtgagccaag 1740
gagcagcata tagtccagag ggtcagccca tggggagctt tgtgttggat ggtcagcaac 1800
acatggggat ccggcctgca ggtttgcaga gcatgccagg ggactacgtt tctcagggtg 1860
gtcctatggg aatgagtatg gcacagccaa gttacactcc tccccagatg accccacacc 1920
ctactcaatt aagacatgga cccccaatgc attcatattt gccaagccat ccccaccacc 1980
cagccatgat gatgcacgga ggacccccta cccaccctgg aatgactatg tcagcacaga 2040
gccccacaat gttaaattct gtagatccca atgttggcgg acaggttatg gacattcatg 2100

cccaatagta taagggaact caagggaaaa ggaaacacac gcaaaaacta ttttaagact 2160

ttctgaactt tgaccagatg ttgacactta atatgaaatt ccagacagct gtgattattt 2220

tttacttttg tcatttttca tcaagcaaca gaggaccaat gcaacaagaa cacaaatgtg 2280

aaatcatggg ctgactgaga caattctgtc catgtaaaga tcctctggaa aaagactccg 2340

agagttataa ctactgtagt ataaatatag gaactaagtt aaacttgtac atttctgttg 2400

atcacgccgt tatgttgcct caaatagttt tagaagagaa aaaaaaatat atccttgttt 2460

tccacactat gtgtgttgtt cccaaaagaa tgactgtttt ggttcatcag tgaattcacc 2520

atccaggaga gactgtggta tatattttaa acctgttggg ccaatgagaa aagaaccaca 2580

ctggagatca tgatgaactt ttggctgaac ctcatcactc gaactccagc ttcaagaatg 2640

tgttttcatg cccggccttt gttcctccat aaatgtgtcc tttagtttca aacagatctt 2700

tatagttcgt gcttcataag ccaattctta ttattatttt tgggggactc ttcttcaaag 2760

agcttgccaa tgaagattta aagacagagc aggagcttct tccaggagtt ctgagccttg 2820

gttgtggaca aaacaatctt aagttgggca gctttcctca acacaaaaaa aagttattaa 2880

tggtcattga accataacta ggactttatc agaaactcaa agcttggggg ataaaaagga 2940

gcaagagaat actgtaacaa acttcgtaca gagttcggtc tattaattgt ttcatgttag 3000

atattctatg tgtttacctc aattgaaaaa aaaaagaatg tttttgctag tatcagatct 3060

gctgtggaat tggtattgta tgtccatgaa ttcttctttt ctcagcacgt gttcctcact 3120

agaagaaaat gctgttacct ttaagctttg tcaaatttac attaaaatac ttgtatgagg 3180

actgtgacgt tatgttaaaa aaaaaaaggt gttaagtcac aaaaagcggt aataaatatt 3240

tcatttttga ttttttgtta aaaaaaaaaa aaaaaaa 3277

<210> 49
<211> 3256
<212> DNA
<213> Homo sapiens

<400> 49

```
tttaaagaga gaggatcgta ttatagatac cgcgggggca aagctaaaaa agggggggagg        60
ggggaggaaa aaattcaaga agcagaaacc cctcgcggag ttttactgga agaaaaaaac       120
gggtctgaaa gattcctcct cttcatcatc atcaaccatc attcattcac taccttgaca       180
ttccgggctt tgattgacag ctggagtggc aaaaagccat gaaacacgac agttcggtta       240
catgtgggct gctgacgggc cgctcgtaac cttcagttcg ggggcttgac aatttttttc       300
ttcttttct tctttttctt ttctttcttt tttttccaa ctgaggggaa gagaagagaa         360
agaggggaa aggaggaccg aagaggagga ggaggggagg ggggaggagg aggaggtgga        420
ggaggaggag gaagatcagg aggaggagga agaagaggaa aaaagagaaa aagaagaaat       480
```

atcacagaaa aaaaaattct tcgttgtcta gactgggctt tttttccccc ctaaaaaata 540
gcatattgga gaattgggag aagtctcttt ggtttggaaa aaaaaaaaag gaatcttcag 600
cctagatcac tttcttatcc ggactgggat attaaatata cgacacatcc aggagtttat 660
tggagcgcag actgatggcg caaaggtacg atgagctgcc ccattacggc gggatggacg 720
gagtaggggt tcccgcttcc atgtacggag accctcacgc gccgcggccg atcccccgg 780
ttcaccacct gaaccacggg ccgccgctcc acgccacaca gcactacggc gcgcacgccc 840
cgcaccccaa tgtcatgccg gccagtatgg gatccgctgt caacgacgcc ttgaagcggg 900
acaaggacgc gatctatggg cacccgttgt ttcctctgtt agctctggtc tttgagaagt 960
gcgagctggc gacctgcact ccccgggaac ctggagtggc tggcggagac gtctgctcct 1020
ccgactcctt caacgaggac atcgcggtct tcgccaagca ggttcgcgcc gaaaagccac 1080
ttttttcctc aaatccagag ctggacaatt tgatgataca agcaatacaa gtactaaggt 1140
ttcatctttt ggagttagaa aaggtccacg aactgtgcga taacttctgc caccgataca 1200
ttagctgttt gaaggggaaa atgcccatcg acctcgtcat tgatgaaaga gacggcagct 1260
ccaagtcaga tcatgaagaa ctttcaggct cctccacaaa tctcgctgac cataaccctt 1320
cttcttggcg agaccacgat gatgcaacct caacccactc agcaggcacc ccagggccct 1380
ccagtggggg ccatgcttcc cagagcggag acaacagcag tgagcaaggg gatggtttag 1440
acaacagtgt agcttcacct ggtacaggtg acgatgatga tccggataag gacaaaaaac 1500
gccagaagaa aagaggcatt ttccccaaag tagcaacaaa tatcatgaga gcatggctct 1560
tccagcatct cacacatccg taccccttccg aagagcagaa gaaacagtta gcgcaagaca 1620
caggacttac aattctccaa gtaaacaact ggtttattaa tgccagaaga agaatagtac 1680
agcccatgat tgaccagtca aatcgagcag tgagccaagg agcagcatat agtccagagg 1740
gtcagcccat ggggagcttt gtgttggatg gtcagcaaca catggggatc cggcctgcag 1800
gtttgcagag catgccaggg gactacgttt ctcagggtgg tcctatggga atgagtatgg 1860
cacagccaag ttacactcct ccccagatga ccccacaccc tactcaatta agacatggac 1920
ccccaatgca ttcatatttg ccaagccatc cccaccaccc agccatgatg atgcacggag 1980
gacccccctac ccaccctgga atgactatgt cagcacagag ccccacaatg ttaaattctg 2040
tagatcccaa tgttggcgga caggttatgg acattcatgc ccaatagtat aagggaactc 2100
aagggaaaag gaaacacacg caaaaactat tttaagactt tctgaacttt gaccagatgt 2160
tgacacttaa tatgaaattc cagacagctg tgattatttt ttacttttgt catttttcat 2220
caagcaacag aggaccaatg caacaagaac acaaatgtga aatcatgggc tgactgagac 2280
aattctgtcc atgtaaagat cctctggaaa aagactccga gagttataac tactgtagta 2340
taaatatagg aactaagtta aacttgtaca tttctgttga tcacgccgtt atgttgcctc 2400

aaatagtttt agaagagaaa aaaaaatata tccttgtttt ccacactatg tgtgttgttc 2460

ccaaaagaat gactgttttg gttcatcagt gaattcacca tccaggagag actgtggtat 2520

atattttaaa cctgttgggc caatgagaaa agaaccacac tggagatcat gatgaacttt 2580

tggctgaacc tcatcactcg aactccagct tcaagaatgt gttttcatgc ccggcctttg 2640

ttcctccata aatgtgtcct ttagtttcaa acagatcttt atagttcgtg cttcataagc 2700

caattcttat tattattttt gggggactct tcttcaaaga gcttgccaat gaagatttaa 2760

agacagagca ggagcttctt ccaggagttc tgagccttgg ttgtggacaa aacaatctta 2820

agttgggcag ctttcctcaa cacaaaaaaa agttattaat ggtcattgaa ccataactag 2880

gactttatca gaaactcaaa gcttggggga taaaaaggag caagagaata ctgtaacaaa 2940

cttcgtacag agttcggtct attaattgtt tcatgttaga tattctatgt gtttacctca 3000

attgaaaaaa aaaagaatgt ttttgctagt atcagatctg ctgtggaatt ggtattgtat 3060

gtccatgaat tcttcttttc tcagcacgtg ttcctcacta gaagaaaatg ctgttacctt 3120

taagctttgt caaatttaca ttaaaatact tgtatgagga ctgtgacgtt atgttaaaaa 3180

aaaaaaggtg ttaagtcaca aaaagcggta ataaatattt catttttgat tttttgttaa 3240

aaaaaaaaaa aaaaaa 3256

<210> 50
<211> 3373
<212> DNA
<213> Homo sapiens

<400> 50

```
tttaaagaga gaggatcgta ttatagatac cgcgggggca aagctaaaaa aggggggagg      60
ggggaggaaa aaattcaaga agcagaaacc cctcgcggag ttttactgga agaaaaaaac     120
gggtctgaaa gattcctcct cttcatcatc atcaaccatc attcattcac taccttgaca     180
ttccgggctt tgattgacag ctggagtggc aaaaagccat gaaacacgac agttcggtta     240
catgtgggct gctgacgggc cgctcgtaac cttcagttcg ggggcttgac aatttttttc     300
ttctttttct tctttttctt ttctttcttt ttttttccaa ctgaggggaa gagaagagaa     360
agagggggaa aggaggaccg aagaggagga ggaggggagg ggggaggagg aggaggtgga     420
ggaggaggag gaagatcagg aggaggagga agaagaggaa aaaagagaaa aagaagaaat     480
atcacagaaa aaaaaattct tcgttgtcta gactgggctt tttttccccc ctaaaaaata     540
gcatattgga gaattgggag aagtctcttt ggtttggaaa aaaaaaaaag gaatcttcag     600
cctagatcac tttcttatcc ggactgggat attaaatata cgacacatcc aggagtttat     660
tggagcgcag actgatggcg caaaggtacg atgagctgcc ccattacggc gggatggacg     720
gagtaggggt tcccgcttcc atgtacggag accctcacgc gccgcggccg atcccccccgg     780
```

ttcaccacct gaaccacggg ccgccgctcc acgccacaca gcactacggc gcgcacgccc 840
cgcaccccaa tgtcatgccg gccagtatgg gatccgctgt caacgacgcc ttgaagcggg 900
acaaggacgc gatctatggg cacccgttgt ttcctctgtt agctctggtc tttgagaagt 960
gcgagctggc gacctgcact ccccgggaac ctggagtggc tggcggagac gtctgctcct 1020
ccgactcctt caacgaggac atcgcggtct tcgccaagca ggttcgcgcc gaaaagccac 1080
ttttttcctc aaatccagag ctggacaatt tgatgataca agcaatacaa gtactaaggt 1140
ttcatctttt ggagttagaa aaggtccacg aactgtgcga taacttctgc caccgataca 1200
ttagctgttt gaaggggaaa atgcccatcg acctcgtcat tgatgaaaga gacggcagct 1260
ccaagtcaga tcatgaagaa ctttcaggct cctccacaaa tctcgctgac cataaccctt 1320
cttcttggcg agaccacgat gatgcaacct caacccactc agcaggcacc ccagggccct 1380
ccagtggggg ccatgcttcc cagagcggag acaacagcag tgagcaaggg gatggtttag 1440
acaacagtgt agcttcacct ggtacaggtg acgatgatga tccggataag gacaaaaaac 1500
gccagaagaa aagaggcatt ttccccaaag tagcaacaaa tatcatgaga gcatggctct 1560
tccagcatct cacacatccg tacccttccg aagagcagaa gaaacagtta gcgcaagaca 1620
caggacttac aattctccaa gtaaacaact ggtttattaa tgccagaaga agaatagtac 1680
agcccatgat tgaccagtca aatcgagcag gttttcttct tgatccttca gtgagccaag 1740
gagcagcata tagtccagag ggtcagccca tggggagctt tgtgttggat ggtcagcaac 1800
acatggggat ccggcctgca ggacctatga gtggaatggg catgaatatg ggcatggatg 1860
ggcaatggca ctacatgtaa ccttcatcat gtaaagcaat cgcaaagcaa gggggaagtt 1920
tgcagagcat gccaggggac tacgtttctc agggtggtcc tatgggaatg agtatggcac 1980
agccaagtta cactcctccc cagatgaccc cacaccctac tcaattaaga catggacccc 2040
caatgcattc atatttgcca agccatcccc accacccagc catgatgatg cacggaggac 2100
cccctaccca ccctggaatg actatgtcag cacagagccc cacaatgtta aattctgtag 2160
atcccaatgt tggcggacag gttatggaca ttcatgccca atagtataag ggaactcaag 2220
ggaaaaggaa acacacgcaa aaactatttt aagactttct gaactttgac cagatgttga 2280
cacttaatat gaaattccag acagctgtga ttattttta cttttgtcat tttcatcaa 2340
gcaacagagg accaatgcaa caagaacaca aatgtgaaat catgggctga ctgagacaat 2400
tctgtccatg taaagatcct ctggaaaaag actccgagag ttataactac tgtagtataa 2460
atataggaac taagttaaac ttgtacattt ctgttgatca cgccgttatg ttgcctcaaa 2520
tagttttaga agagaaaaaa aaatatatcc ttgttttcca cactatgtgt gttgttccca 2580
aaagaatgac tgttttggtt catcagtgaa ttcaccatcc aggagagact gtggtatata 2640

```
ttttaaacct gttgggccaa tgagaaaaga accacactgg agatcatgat gaacttttgg     2700
ctgaacctca tcactcgaac tccagcttca agaatgtgtt ttcatgcccg gcctttgttc     2760
ctccataaat gtgtccttta gtttcaaaca gatctttata gttcgtgctt cataagccaa     2820
ttcttattat tatttttggg ggactcttct tcaaagagct tgccaatgaa gatttaaaga     2880
cagagcagga gcttcttcca ggagttctga gccttggttg tggacaaaac aatcttaagt     2940
tgggcagctt tcctcaacac aaaaaaaagt tattaatggt cattgaacca taactaggac     3000
tttatcagaa actcaaagct tgggggataa aaaggagcaa gagaatactg taacaaactt     3060
cgtacagagt tcggtctatt aattgtttca tgttagatat tctatgtgtt tacctcaatt     3120
gaaaaaaaaa agaatgtttt tgctagtatc agatctgctg tggaattggt attgtatgtc     3180
catgaattct tcttttctca gcacgtgttc ctcactagaa gaaaatgctg ttacctttaa     3240
gctttgtcaa atttacatta aaatacttgt atgaggactg tgacgttatg ttaaaaaaaa     3300
aaaggtgtta agtcacaaaa agcggtaata aatatttcat ttttgatttt ttgttaaaaa     3360
aaaaaaaaaa aaa                                                        3373
```

<210> 51
<211> 2849
<212> DNA
<213> Homo sapiens

<400> 51

```
tcctcctcct ctcttctctc ttacctccct ccctctctct catctttatc ccttcctctc      60
ttttctgttc gccctcttct ccctgctctt tttccctttc caccccccctc ctctgttctc    120
cctcacctcc tgcgccccct ccccccttccc gggttctgac agtacgatga gctgccccat    180
tacggcggga tggacggagt aggggttccc gcttccatgt acggagaccc tcacgcgccg    240
cggccgatcc ccccggttca ccacctgaac cacgggccgc cgctccacgc cacacagcac    300
tacggcgcgc acgccccgca ccccaatgtc atgccggcca gtatgggatc cgctgtcaac    360
gacgccttga agcgggacaa ggacgcgatc tatgggcacc cgttgtttcc tctgttagct    420
ctggtctttg agaagtgcga gctggcgacc tgcactcccc gggaacctgg agtggctggc    480
ggagacgtct gctcctccga ctccttcaac gaggacatcg cggtcttcgc caagcaggtt    540
cgcgccgaaa agccactttt ttcctcaaat ccagagctgg acaatttgat gatacaagca    600
atacaagtac taaggtttca tcttttggag ttagaaaagg tccacgaact gtgcgataac    660
ttctgccacc gatacattag ctgtttgaag gggaaaatgc ccatcgacct cgtcattgat    720
gaaagagacg gcagctccaa gtcagatcat gaagaacttt caggctcctc cacaaatctc    780
gctgaccata acccttcttc ttggcgagac cacgatgatg caacctcaac ccactcagca    840
ggcaccccag ggccctccag tgggggccat gcttcccaga gcggagacaa cagcagtgag    900
```

caaggggatg gtttagacaa cagtgtagct tcacctggta caggtgacga tgatgatccg 960
gataaggaca aaaaacgcca gaagaaaaga ggcattttcc ccaaagtagc aacaaatatc 1020
atgagagcat ggctcttcca gcatctcaca catccgtacc cttccgaaga gcagaagaaa 1080
cagttagcgc aagacacagg acttacaatt ctccaagtaa acaactggtt tattaatgcc 1140
agaagaagaa tagtacagcc catgattgac cagtcaaatc gagcaggttt tcttcttgat 1200
ccttcagtga gccaaggagc agcatatagt ccagagggtc agcccatggg gagctttgtg 1260
ttggatggtc agcaacacat ggggatccgg cctgcaggac ctatgagtgg aatgggcatg 1320
aatatgggca tggatgggca atggcactac atgtaacctt catcatgtaa agcaatcgca 1380
aagcaagggg gaagtttgca gagcatgcca ggggactacg tttctcaggg tggtcctatg 1440
ggaatgagta tggcacagcc aagttacact cctccccaga tgaccccaca ccctactcaa 1500
ttaagacatg gacccccaat gcattcatat ttgccaagcc atccccacca cccagccatg 1560
atgatgcacg gaggaccccc tacccaccct ggaatgacta tgtcagcaca gagccccaca 1620
atgttaaatt ctgtagatcc caatgttggc ggacaggtta tggacattca tgcccaatag 1680
tataagggaa ctcaagggaa aaggaaacac acgcaaaaac tattttaaga ctttctgaac 1740
tttgaccaga tgttgacact taatatgaaa ttccagacag ctgtgattat tttttacttt 1800
tgtcattttt catcaagcaa cagaggacca atgcaacaag aacacaaatg tgaaatcatg 1860
ggctgactga gacaattctg tccatgtaaa gatcctctgg aaaaagactc cgagagttat 1920
aactactgta gtataaatat aggaactaag ttaaacttgt acatttctgt tgatcacgcc 1980
gttatgttgc ctcaaatagt tttagaagag aaaaaaaaat atatccttgt tttccacact 2040
atgtgtgttg ttcccaaaag aatgactgtt ttggttcatc agtgaattca ccatccagga 2100
gagactgtgg tatatatttt aaacctgttg ggccaatgag aaaagaacca cactggagat 2160
catgatgaac ttttggctga acctcatcac tcgaactcca gcttcaagaa tgtgttttca 2220
tgcccggcct ttgttcctcc ataaatgtgt cctttagttt caaacagatc tttatagttc 2280
gtgcttcata agccaattct tattattatt tttgggggac tcttcttcaa agagcttgcc 2340
aatgaagatt taaagacaga gcaggagctt cttccaggag ttctgagcct tggttgtgga 2400
caaaacaatc ttaagttggg cagctttcct caacacaaaa aaaagttatt aatggtcatt 2460
gaaccataac taggacttta tcagaaactc aaagcttggg ggataaaaag gagcaagaga 2520
atactgtaac aaacttcgta cagagttcgg tctattaatt gtttcatgtt agatattcta 2580
tgtgtttacc tcaattgaaa aaaaaaagaa tgtttttgct agtatcagat ctgctgtgga 2640
attggtattg tatgtccatg aattcttctt ttctcagcac gtgttcctca ctagaagaaa 2700
atgctgttac ctttaagctt tgtcaaattt acattaaaat acttgtatga ggactgtgac 2760
gttatgttaa aaaaaaaaag gtgttaagtc acaaaaagcg gtaataaata tttcattttt 2820

gatttttgt taaaaaaaa aaaaaaaaa 2849

<210> 52
<211> 0
<212> DNA
<213> Homo sapiens

<400> 52
000

<210> 53
<211> 470
<212> PRT
<213> Homo sapiens

<400> 53

```
Met Ala Gln Arg Tyr Asp Glu Leu Pro His Tyr Gly Gly Met Asp Gly
1               5                   10                  15

Val Gly Val Pro Ala Ser Met Tyr Gly Asp Pro His Ala Pro Arg Pro
            20                  25                  30

Ile Pro Pro Val His His Leu Asn His Gly Pro Pro Leu His Ala Thr
        35                  40                  45

Gln His Tyr Gly Ala His Ala Pro His Pro Asn Val Met Pro Ala Ser
    50                  55                  60

Met Gly Ser Ala Val Asn Asp Ala Leu Lys Arg Asp Lys Asp Ala Ile
65                  70                  75                  80

Tyr Gly His Pro Leu Phe Pro Leu Leu Ala Leu Val Phe Glu Lys Cys
                85                  90                  95

Glu Leu Ala Thr Cys Thr Pro Arg Glu Pro Gly Val Ala Gly Gly Asp
            100                 105                 110

Val Cys Ser Ser Asp Ser Phe Asn Glu Asp Ile Ala Val Phe Ala Lys
        115                 120                 125

Gln Val Arg Ala Glu Lys Pro Leu Phe Ser Ser Asn Pro Glu Leu Asp
    130                 135                 140

Asn Leu Met Ile Gln Ala Ile Gln Val Leu Arg Phe His Leu Leu Glu
145                 150                 155                 160

Leu Glu Lys Val His Glu Leu Cys Asp Asn Phe Cys His Arg Tyr Ile
                165                 170                 175
```

Ser Cys Leu Lys Gly Lys Met Pro Ile Asp Leu Val Ile Asp Glu Arg
180 185 190

Asp Gly Ser Ser Lys Ser Asp His Glu Glu Leu Ser Gly Ser Ser Thr
195 200 205

Asn Leu Ala Asp His Asn Pro Ser Ser Trp Arg Asp His Asp Asp Ala
210 215 220

Thr Ser Thr His Ser Ala Gly Thr Pro Gly Pro Ser Ser Gly Gly His
225 230 235 240

Ala Ser Gln Ser Gly Asp Asn Ser Ser Glu Gln Gly Asp Gly Leu Asp
245 250 255

Asn Ser Val Ala Ser Pro Gly Thr Gly Asp Asp Asp Asp Pro Asp Lys
260 265 270

Asp Lys Lys Arg Gln Lys Lys Arg Gly Ile Phe Pro Lys Val Ala Thr
275 280 285

Asn Ile Met Arg Ala Trp Leu Phe Gln His Leu Thr His Pro Tyr Pro
290 295 300

Ser Glu Glu Gln Lys Lys Gln Leu Ala Gln Asp Thr Gly Leu Thr Ile
305 310 315 320

Leu Gln Val Asn Asn Trp Phe Ile Asn Ala Arg Arg Arg Ile Val Gln
325 330 335

Pro Met Ile Asp Gln Ser Asn Arg Ala Val Ser Gln Gly Ala Ala Tyr
340 345 350

Ser Pro Glu Gly Gln Pro Met Gly Ser Phe Val Leu Asp Gly Gln Gln
355 360 365

His Met Gly Ile Arg Pro Ala Gly Leu Gln Ser Met Pro Gly Asp Tyr
370 375 380

Val Ser Gln Gly Gly Pro Met Gly Met Ser Met Ala Gln Pro Ser Tyr
385 390 395 400

Thr Pro Pro Gln Met Thr Pro His Pro Thr Gln Leu Arg His Gly Pro
405 410 415

Pro Met His Ser Tyr Leu Pro Ser His Pro His His Pro Ala Met Met

420 425 430

Met His Gly Gly Pro Pro Thr His Pro Gly Met Thr Met Ser Ala Gln
435 440 445

Ser Pro Thr Met Leu Asn Ser Val Asp Pro Asn Val Gly Gly Gln Val
450 455 460

Met Asp Ile His Ala Gln
465 470

<210> 54
<211> 381
<212> PRT
<213> Homo sapiens

<400> 54

```
Met Asp Gly Val Gly Val Pro Ala Ser Met Tyr Gly Asp Pro His Ala
1               5                   10                  15

Pro Arg Pro Ile Pro Pro Val His His Leu Asn His Gly Pro Pro Leu
            20                  25                  30

His Ala Thr Gln His Tyr Gly Ala His Ala Pro His Pro Asn Val Met
        35                  40                  45

Pro Ala Ser Met Gly Ser Ala Val Asn Asp Ala Leu Lys Arg Asp Lys
    50                  55                  60

Asp Ala Ile Tyr Gly His Pro Leu Phe Pro Leu Leu Ala Leu Val Phe
65                  70                  75                  80

Glu Lys Cys Glu Leu Ala Thr Cys Thr Pro Arg Glu Pro Gly Val Ala
                85                  90                  95

Gly Gly Asp Val Cys Ser Ser Asp Ser Phe Asn Glu Asp Ile Ala Val
            100                 105                 110

Phe Ala Lys Gln Val Arg Ala Glu Lys Pro Leu Phe Ser Ser Asn Pro
        115                 120                 125

Glu Leu Asp Asn Leu Met Ile Gln Ala Ile Gln Val Leu Arg Phe His
    130                 135                 140

Leu Leu Glu Leu Glu Lys Val His Glu Leu Cys Asp Asn Phe Cys His
145                 150                 155                 160

Arg Tyr Ile Ser Cys Leu Lys Gly Lys Met Pro Ile Asp Leu Val Ile
```

165 170 175

Asp Glu Arg Asp Gly Ser Ser Lys Ser Asp His Glu Glu Leu Ser Gly
180 185 190

Ser Ser Thr Asn Leu Ala Asp His Asn Pro Ser Ser Trp Arg Asp His
195 200 205

Asp Asp Ala Thr Ser Thr His Ser Ala Gly Thr Pro Gly Pro Ser Ser
210 215 220

Gly Gly His Ala Ser Gln Ser Gly Asp Asn Ser Ser Glu Gln Gly Asp
225 230 235 240

Gly Leu Asp Asn Ser Val Ala Ser Pro Gly Thr Gly Asp Asp Asp Asp
245 250 255

Pro Asp Lys Asp Lys Lys Arg Gln Lys Lys Arg Gly Ile Phe Pro Lys
260 265 270

Val Ala Thr Asn Ile Met Arg Ala Trp Leu Phe Gln His Leu Thr His
275 280 285

Pro Tyr Pro Ser Glu Glu Gln Lys Lys Gln Leu Ala Gln Asp Thr Gly
290 295 300

Leu Thr Ile Leu Gln Val Asn Asn Trp Phe Ile Asn Ala Arg Arg Arg
305 310 315 320

Ile Val Gln Pro Met Ile Asp Gln Ser Asn Arg Ala Val Ser Gln Gly
325 330 335

Ala Ala Tyr Ser Pro Glu Gly Gln Pro Met Gly Ser Phe Val Leu Asp
340 345 350

Gly Gln Gln His Met Gly Ile Arg Pro Ala Gly Pro Met Ser Gly Met
355 360 365

Gly Met Asn Met Gly Met Asp Gly Gln Trp His Tyr Met
370 375 380

<210> 55
<211> 394
<212> PRT
<213> Homo sapiens

<400> 55

Met Ala Gln Arg Tyr Asp Glu Leu Pro His Tyr Gly Gly Met Asp Gly

```
1               5                   10                  15

Val Gly Val Pro Ala Ser Met Tyr Gly Asp Pro His Ala Pro Arg Pro
            20                  25                  30

Ile Pro Pro Val His His Leu Asn His Gly Pro Pro Leu His Ala Thr
        35                  40                  45

Gln His Tyr Gly Ala His Ala Pro His Pro Asn Val Met Pro Ala Ser
    50                  55                  60

Met Gly Ser Ala Val Asn Asp Ala Leu Lys Arg Asp Lys Asp Ala Ile
65                  70                  75                  80

Tyr Gly His Pro Leu Phe Pro Leu Leu Ala Leu Val Phe Glu Lys Cys
                85                  90                  95

Glu Leu Ala Thr Cys Thr Pro Arg Glu Pro Gly Val Ala Gly Gly Asp
            100                 105                 110

Val Cys Ser Ser Asp Ser Phe Asn Glu Asp Ile Ala Val Phe Ala Lys
        115                 120                 125

Gln Val Arg Ala Glu Lys Pro Leu Phe Ser Ser Asn Pro Glu Leu Asp
    130                 135                 140

Asn Leu Met Ile Gln Ala Ile Gln Val Leu Arg Phe His Leu Leu Glu
145                 150                 155                 160

Leu Glu Lys Val His Glu Leu Cys Asp Asn Phe Cys His Arg Tyr Ile
                165                 170                 175

Ser Cys Leu Lys Gly Lys Met Pro Ile Asp Leu Val Ile Asp Glu Arg
            180                 185                 190

Asp Gly Ser Ser Lys Ser Asp His Glu Glu Leu Ser Gly Ser Ser Thr
        195                 200                 205

Asn Leu Ala Asp His Asn Pro Ser Ser Trp Arg Asp His Asp Asp Ala
    210                 215                 220

Thr Ser Thr His Ser Ala Gly Thr Pro Gly Pro Ser Ser Gly Gly His
225                 230                 235                 240

Ala Ser Gln Ser Gly Asp Asn Ser Ser Glu Gln Gly Asp Gly Leu Asp
                245                 250                 255
```

Asn Ser Val Ala Ser Pro Gly Thr Gly Asp Asp Asp Asp Pro Asp Lys
260 265 270

Asp Lys Lys Arg Gln Lys Lys Arg Gly Ile Phe Pro Lys Val Ala Thr
275 280 285

Asn Ile Met Arg Ala Trp Leu Phe Gln His Leu Thr His Pro Tyr Pro
290 295 300

Ser Glu Glu Gln Lys Lys Gln Leu Ala Gln Asp Thr Gly Leu Thr Ile
305 310 315 320

Leu Gln Val Asn Asn Trp Phe Ile Asn Ala Arg Arg Arg Ile Val Gln
325 330 335

Pro Met Ile Asp Gln Ser Asn Arg Ala Val Ser Gln Gly Ala Ala Tyr
340 345 350

Ser Pro Glu Gly Gln Pro Met Gly Ser Phe Val Leu Asp Gly Gln Gln
355 360 365

His Met Gly Ile Arg Pro Ala Gly Pro Met Ser Gly Met Gly Met Asn
370 375 380

Met Gly Met Asp Gly Gln Trp His Tyr Met
385 390

<210> 56
<211> 477
<212> PRT
<213> Homo sapiens

<400> 56

Met Ala Gln Arg Tyr Asp Glu Leu Pro His Tyr Gly Gly Met Asp Gly
1 5 10 15

Val Gly Val Pro Ala Ser Met Tyr Gly Asp Pro His Ala Pro Arg Pro
20 25 30

Ile Pro Pro Val His His Leu Asn His Gly Pro Pro Leu His Ala Thr
35 40 45

Gln His Tyr Gly Ala His Ala Pro His Pro Asn Val Met Pro Ala Ser
50 55 60

Met Gly Ser Ala Val Asn Asp Ala Leu Lys Arg Asp Lys Asp Ala Ile
65 70 75 80

```
Tyr Gly His Pro Leu Phe Pro Leu Leu Ala Leu Val Phe Glu Lys Cys
                85                  90                  95

Glu Leu Ala Thr Cys Thr Pro Arg Glu Pro Gly Val Ala Gly Gly Asp
            100                 105                 110

Val Cys Ser Ser Asp Ser Phe Asn Glu Asp Ile Ala Val Phe Ala Lys
        115                 120                 125

Gln Val Arg Ala Glu Lys Pro Leu Phe Ser Ser Asn Pro Glu Leu Asp
    130                 135                 140

Asn Leu Met Ile Gln Ala Ile Gln Val Leu Arg Phe His Leu Leu Glu
145                 150                 155                 160

Leu Glu Lys Val His Glu Leu Cys Asp Asn Phe Cys His Arg Tyr Ile
                165                 170                 175

Ser Cys Leu Lys Gly Lys Met Pro Ile Asp Leu Val Ile Asp Glu Arg
            180                 185                 190

Asp Gly Ser Ser Lys Ser Asp His Glu Glu Leu Ser Gly Ser Ser Thr
        195                 200                 205

Asn Leu Ala Asp His Asn Pro Ser Ser Trp Arg Asp His Asp Asp Ala
    210                 215                 220

Thr Ser Thr His Ser Ala Gly Thr Pro Gly Pro Ser Ser Gly Gly His
225                 230                 235                 240

Ala Ser Gln Ser Gly Asp Asn Ser Ser Glu Gln Gly Asp Gly Leu Asp
                245                 250                 255

Asn Ser Val Ala Ser Pro Gly Thr Gly Asp Asp Asp Asp Pro Asp Lys
            260                 265                 270

Asp Lys Lys Arg Gln Lys Lys Arg Gly Ile Phe Pro Lys Val Ala Thr
        275                 280                 285

Asn Ile Met Arg Ala Trp Leu Phe Gln His Leu Thr His Pro Tyr Pro
    290                 295                 300

Ser Glu Glu Gln Lys Lys Gln Leu Ala Gln Asp Thr Gly Leu Thr Ile
305                 310                 315                 320

Leu Gln Val Asn Asn Trp Phe Ile Asn Ala Arg Arg Arg Ile Val Gln
                325                 330                 335
```

Pro Met Ile Asp Gln Ser Asn Arg Ala Gly Phe Leu Leu Asp Pro Ser
340 345 350

Val Ser Gln Gly Ala Ala Tyr Ser Pro Glu Gly Gln Pro Met Gly Ser
355 360 365

Phe Val Leu Asp Gly Gln Gln His Met Gly Ile Arg Pro Ala Gly Leu
370 375 380

Gln Ser Met Pro Gly Asp Tyr Val Ser Gln Gly Gly Pro Met Gly Met
385 390 395 400

Ser Met Ala Gln Pro Ser Tyr Thr Pro Pro Gln Met Thr Pro His Pro
405 410 415

Thr Gln Leu Arg His Gly Pro Pro Met His Ser Tyr Leu Pro Ser His
420 425 430

Pro His His Pro Ala Met Met Met His Gly Gly Pro Pro Thr His Pro
435 440 445

Gly Met Thr Met Ser Ala Gln Ser Pro Thr Met Leu Asn Ser Val Asp
450 455 460

Pro Asn Val Gly Gly Gln Val Met Asp Ile His Ala Gln
465 470 475

<210> 57
<211> 470
<212> PRT
<213> Homo sapiens

<400> 57

Met Ala Gln Arg Tyr Asp Glu Leu Pro His Tyr Gly Gly Met Asp Gly
1 5 10 15

Val Gly Val Pro Ala Ser Met Tyr Gly Asp Pro His Ala Pro Arg Pro
20 25 30

Ile Pro Pro Val His His Leu Asn His Gly Pro Pro Leu His Ala Thr
35 40 45

Gln His Tyr Gly Ala His Ala Pro His Pro Asn Val Met Pro Ala Ser
50 55 60

Met Gly Ser Ala Val Asn Asp Ala Leu Lys Arg Asp Lys Asp Ala Ile
65 70 75 80

```
Tyr Gly His Pro Leu Phe Pro Leu Leu Ala Leu Val Phe Glu Lys Cys
                85                  90                  95

Glu Leu Ala Thr Cys Thr Pro Arg Glu Pro Gly Val Ala Gly Gly Asp
            100                 105                 110

Val Cys Ser Ser Asp Ser Phe Asn Glu Asp Ile Ala Val Phe Ala Lys
        115                 120                 125

Gln Val Arg Ala Glu Lys Pro Leu Phe Ser Ser Asn Pro Glu Leu Asp
    130                 135                 140

Asn Leu Met Ile Gln Ala Ile Gln Val Leu Arg Phe His Leu Leu Glu
145                 150                 155                 160

Leu Glu Lys Val His Glu Leu Cys Asp Asn Phe Cys His Arg Tyr Ile
                165                 170                 175

Ser Cys Leu Lys Gly Lys Met Pro Ile Asp Leu Val Ile Asp Glu Arg
            180                 185                 190

Asp Gly Ser Ser Lys Ser Asp His Glu Glu Leu Ser Gly Ser Ser Thr
        195                 200                 205

Asn Leu Ala Asp His Asn Pro Ser Ser Trp Arg Asp His Asp Asp Ala
    210                 215                 220

Thr Ser Thr His Ser Ala Gly Thr Pro Gly Pro Ser Ser Gly Gly His
225                 230                 235                 240

Ala Ser Gln Ser Gly Asp Asn Ser Ser Glu Gln Gly Asp Gly Leu Asp
                245                 250                 255

Asn Ser Val Ala Ser Pro Gly Thr Gly Asp Asp Asp Asp Pro Asp Lys
            260                 265                 270

Asp Lys Lys Arg Gln Lys Lys Arg Gly Ile Phe Pro Lys Val Ala Thr
        275                 280                 285

Asn Ile Met Arg Ala Trp Leu Phe Gln His Leu Thr His Pro Tyr Pro
    290                 295                 300

Ser Glu Glu Gln Lys Lys Gln Leu Ala Gln Asp Thr Gly Leu Thr Ile
305                 310                 315                 320

Leu Gln Val Asn Asn Trp Phe Ile Asn Ala Arg Arg Arg Ile Val Gln
                325                 330                 335
```

Pro Met Ile Asp Gln Ser Asn Arg Ala Val Ser Gln Gly Ala Ala Tyr
340 345 350

Ser Pro Glu Gly Gln Pro Met Gly Ser Phe Val Leu Asp Gly Gln Gln
355 360 365

His Met Gly Ile Arg Pro Ala Gly Leu Gln Ser Met Pro Gly Asp Tyr
370 375 380

Val Ser Gln Gly Gly Pro Met Gly Met Ser Met Ala Gln Pro Ser Tyr
385 390 395 400

Thr Pro Pro Gln Met Thr Pro His Pro Thr Gln Leu Arg His Gly Pro
405 410 415

Pro Met His Ser Tyr Leu Pro Ser His Pro His His Pro Ala Met Met
420 425 430

Met His Gly Gly Pro Pro Thr His Pro Gly Met Thr Met Ser Ala Gln
435 440 445

Ser Pro Thr Met Leu Asn Ser Val Asp Pro Asn Val Gly Gly Gln Val
450 455 460

Met Asp Ile His Ala Gln
465 470

<210> 58
<211> 401
<212> PRT
<213> Homo sapiens

<400> 58

Met Ala Gln Arg Tyr Asp Glu Leu Pro His Tyr Gly Gly Met Asp Gly
1 5 10 15

Val Gly Val Pro Ala Ser Met Tyr Gly Asp Pro His Ala Pro Arg Pro
20 25 30

Ile Pro Pro Val His His Leu Asn His Gly Pro Pro Leu His Ala Thr
35 40 45

Gln His Tyr Gly Ala His Ala Pro His Pro Asn Val Met Pro Ala Ser
50 55 60

Met Gly Ser Ala Val Asn Asp Ala Leu Lys Arg Asp Lys Asp Ala Ile
65 70 75 80

Tyr Gly His Pro Leu Phe Pro Leu Leu Ala Leu Val Phe Glu Lys Cys
85 90 95

Glu Leu Ala Thr Cys Thr Pro Arg Glu Pro Gly Val Ala Gly Gly Asp
100 105 110

Val Cys Ser Ser Asp Ser Phe Asn Glu Asp Ile Ala Val Phe Ala Lys
115 120 125

Gln Val Arg Ala Glu Lys Pro Leu Phe Ser Ser Asn Pro Glu Leu Asp
130 135 140

Asn Leu Met Ile Gln Ala Ile Gln Val Leu Arg Phe His Leu Leu Glu
145 150 155 160

Leu Glu Lys Val His Glu Leu Cys Asp Asn Phe Cys His Arg Tyr Ile
165 170 175

Ser Cys Leu Lys Gly Lys Met Pro Ile Asp Leu Val Ile Asp Glu Arg
180 185 190

Asp Gly Ser Ser Lys Ser Asp His Glu Glu Leu Ser Gly Ser Ser Thr
195 200 205

Asn Leu Ala Asp His Asn Pro Ser Ser Trp Arg Asp His Asp Asp Ala
210 215 220

Thr Ser Thr His Ser Ala Gly Thr Pro Gly Pro Ser Ser Gly Gly His
225 230 235 240

Ala Ser Gln Ser Gly Asp Asn Ser Ser Glu Gln Gly Asp Gly Leu Asp
245 250 255

Asn Ser Val Ala Ser Pro Gly Thr Gly Asp Asp Asp Asp Pro Asp Lys
260 265 270

Asp Lys Lys Arg Gln Lys Lys Arg Gly Ile Phe Pro Lys Val Ala Thr
275 280 285

Asn Ile Met Arg Ala Trp Leu Phe Gln His Leu Thr His Pro Tyr Pro
290 295 300

Ser Glu Glu Gln Lys Lys Gln Leu Ala Gln Asp Thr Gly Leu Thr Ile
305 310 315 320

Leu Gln Val Asn Asn Trp Phe Ile Asn Ala Arg Arg Arg Ile Val Gln

```
                    325                 330                 335

Pro Met Ile Asp Gln Ser Asn Arg Ala Gly Phe Leu Leu Asp Pro Ser
            340                 345                 350

Val Ser Gln Gly Ala Ala Tyr Ser Pro Glu Gly Gln Pro Met Gly Ser
        355                 360                 365

Phe Val Leu Asp Gly Gln Gln His Met Gly Ile Arg Pro Ala Gly Pro
    370                 375                 380

Met Ser Gly Met Gly Met Asn Met Gly Met Asp Gly Gln Trp His Tyr
385                 390                 395                 400

Met
```

<210> 59
<211> 388
<212> PRT
<213> Homo sapiens

<400> 59

```
Met Asp Gly Val Gly Val Pro Ala Ser Met Tyr Gly Asp Pro His Ala
1               5                   10                  15

Pro Arg Pro Ile Pro Pro Val His His Leu Asn His Gly Pro Pro Leu
            20                  25                  30

His Ala Thr Gln His Tyr Gly Ala His Ala Pro His Pro Asn Val Met
        35                  40                  45

Pro Ala Ser Met Gly Ser Ala Val Asn Asp Ala Leu Lys Arg Asp Lys
    50                  55                  60

Asp Ala Ile Tyr Gly His Pro Leu Phe Pro Leu Leu Ala Leu Val Phe
65                  70                  75                  80

Glu Lys Cys Glu Leu Ala Thr Cys Thr Pro Arg Glu Pro Gly Val Ala
                85                  90                  95

Gly Gly Asp Val Cys Ser Ser Asp Ser Phe Asn Glu Asp Ile Ala Val
            100                 105                 110

Phe Ala Lys Gln Val Arg Ala Glu Lys Pro Leu Phe Ser Ser Asn Pro
        115                 120                 125

Glu Leu Asp Asn Leu Met Ile Gln Ala Ile Gln Val Leu Arg Phe His
```

130 135 140

Leu Leu Glu Leu Glu Lys Val His Glu Leu Cys Asp Asn Phe Cys His
145 150 155 160

Arg Tyr Ile Ser Cys Leu Lys Gly Lys Met Pro Ile Asp Leu Val Ile
165 170 175

Asp Glu Arg Asp Gly Ser Ser Lys Ser Asp His Glu Glu Leu Ser Gly
180 185 190

Ser Ser Thr Asn Leu Ala Asp His Asn Pro Ser Ser Trp Arg Asp His
195 200 205

Asp Asp Ala Thr Ser Thr His Ser Ala Gly Thr Pro Gly Pro Ser Ser
210 215 220

Gly Gly His Ala Ser Gln Ser Gly Asp Asn Ser Ser Glu Gln Gly Asp
225 230 235 240

Gly Leu Asp Asn Ser Val Ala Ser Pro Gly Thr Gly Asp Asp Asp Asp
245 250 255

Pro Asp Lys Asp Lys Lys Arg Gln Lys Lys Arg Gly Ile Phe Pro Lys
260 265 270

Val Ala Thr Asn Ile Met Arg Ala Trp Leu Phe Gln His Leu Thr His
275 280 285

Pro Tyr Pro Ser Glu Glu Gln Lys Lys Gln Leu Ala Gln Asp Thr Gly
290 295 300

Leu Thr Ile Leu Gln Val Asn Asn Trp Phe Ile Asn Ala Arg Arg Arg
305 310 315 320

Ile Val Gln Pro Met Ile Asp Gln Ser Asn Arg Ala Gly Phe Leu Leu
325 330 335

Asp Pro Ser Val Ser Gln Gly Ala Ala Tyr Ser Pro Glu Gly Gln Pro
340 345 350

Met Gly Ser Phe Val Leu Asp Gly Gln Gln His Met Gly Ile Arg Pro
355 360 365

Ala Gly Pro Met Ser Gly Met Gly Met Asn Met Gly Met Asp Gly Gln
370 375 380

Trp His Tyr Met
385

<210> 60
<211> 306
<212> PRT
<213> Homo sapiens

<400> 60

Met Ala Leu Val Phe Glu Lys Cys Glu Leu Ala Thr Cys Thr Pro Arg
1 5 10 15

Glu Pro Gly Val Ala Gly Gly Asp Val Cys Ser Ser Asp Ser Phe Asn
20 25 30

Glu Asp Ile Ala Val Phe Ala Lys Gln Val Arg Ala Glu Lys Pro Leu
35 40 45

Phe Ser Ser Asn Pro Glu Leu Asp Asn Leu Met Ile Gln Ala Ile Gln
50 55 60

Val Leu Arg Phe His Leu Leu Glu Leu Glu Lys Val His Glu Leu Cys
65 70 75 80

Asp Asn Phe Cys His Arg Tyr Ile Ser Cys Leu Lys Gly Lys Met Pro
85 90 95

Ile Asp Leu Val Ile Asp Glu Arg Asp Gly Ser Ser Lys Ser Asp His
100 105 110

Glu Glu Leu Ser Gly Ser Ser Thr Asn Leu Ala Asp His Asn Pro Ser
115 120 125

Ser Trp Arg Asp His Asp Asp Ala Thr Ser Thr His Ser Ala Gly Thr
130 135 140

Pro Gly Pro Ser Ser Gly Gly His Ala Ser Gln Ser Gly Asp Asn Ser
145 150 155 160

Ser Glu Gln Gly Asp Gly Leu Asp Asn Ser Val Ala Ser Pro Gly Thr
165 170 175

Gly Asp Asp Asp Asp Pro Asp Lys Asp Lys Lys Arg Gln Lys Lys Arg
180 185 190

Gly Ile Phe Pro Lys Val Ala Thr Asn Ile Met Arg Ala Trp Leu Phe
195 200 205

Gln His Leu Thr His Pro Tyr Pro Ser Glu Glu Gln Lys Lys Gln Leu
210 215 220

Ala Gln Asp Thr Gly Leu Thr Ile Leu Gln Val Asn Asn Trp Phe Ile
225 230 235 240

Asn Ala Arg Arg Arg Ile Val Gln Pro Met Ile Asp Gln Ser Asn Arg
245 250 255

Ala Val Ser Gln Gly Ala Ala Tyr Ser Pro Glu Gly Gln Pro Met Gly
260 265 270

Ser Phe Val Leu Asp Gly Gln Gln His Met Gly Ile Arg Pro Ala Gly
275 280 285

Pro Met Ser Gly Met Gly Met Asn Met Gly Met Asp Gly Gln Trp His
290 295 300

Tyr Met
305

<210> 61
<211> 20
<212> DNA
<213> Artificial Sequence

<220>
<223> MEIS2_forward primer

<400> 61
tcaaatccag agctggacaa 20

<210> 62
<211> 23
<212> DNA
<213> Artificial Sequence

<220>
<223> MEIS2_reverse primer

<400> 62
cccttcaaac agctaatgta tcg 23

<210> 63
<211> 22
<212> DNA
<213> Artificial Sequence

<220>
<223> MEIS2 probe

<400> 63
aaaggtccac gaactgtgcg at 22

<210> 64
<211> 3930
<212> DNA
<213> Homo sapiens

<400> 64

```
agaggcagta taaaagcact gggtttctcc ccaactgctt gtcacaccga cctgcaccat      60
ctctcgcctg cctgtggggt ttctgtcaac tagtcgtgga gggaaggaga ctctttaaag     120
aataacatct tattgtggcc atgccagaac ccactaagaa agaggaaaat gaagtgccag     180
ccccagcccc acccccggaa gaaccaagta aagagaagga ggccggaact acaccagcaa     240
aagactggac ccttgtcgaa actcctcctg ggaggaaca agccaagcag aatgccaact      300
cccagctgtc catcttgttc attgaaaaac ctcaaggagg aacagtgaaa gttggtgaag     360
atatcacctt catagccaaa gtcaaggctg aagatcttct gagaaaaccc actatcaaat     420
ggttcaaagg aaaatggatg gacctggcca gcaaagccgg gaagcacctt cagctgaagg     480
aaacctttga gaggcacagt cgggtgtaca catttgagat gcagatcatc aaggccaaag     540
ataactttgc aggaaattac agatgcgagg tcacctataa ggataagttt gacagctgtt     600
catttgatct tgaagtgcac gaatctactg ggactactcc aaacattgac atcagatctg     660
ctttcaagag aagtggagaa ggtcaagagg atgcaggaga acttgacttt agtggtctcc     720
tgaaacgtag ggaggtgaag cagcaggagg aagaacccca ggtggacgta tgggagttgc     780
tgaagaacgc gaaacccagt gagtacgaga agatcgcctt ccagtatgga atcaccgacc     840
tgcgcggcat gctcaagcga ctcaagcgca tgcgcagaga ggagaagaag agcgcagctt     900
ttgcaaaaat tcttgatcct gcatatcagg ttgacaaagg aggcagagtg aggtttgttg     960
tggagctggc agatccaaag ttggaggtga aatggtataa aaatggtcaa gaaattcgac    1020
ccagtaccaa atacatcttt gaacacaaag gatgccagag aatcctgttt atcaataact    1080
gtcagatgac agatgattca gagtattatg tgacagccgg tgatgagaaa tgttccactg    1140
agctcttcgt aagagagcct ccaattatgg tgaccaaaca gctggaagat acaactgctt    1200
attgtgggga gagagtggaa ttagaatgtg aggtgtctga agatgatgcc aatgtaaaat    1260
ggtttaagaa tggtgaagag attatccctg gtccaaaatc aagataccga attagagttg    1320
agggtaaaaa acacatcttg atcatagagg gagcaacaaa ggctgatgct gcagaatatt    1380
cagtaatgac aacaggagga caatcatctg ctaaacttag tgttgacttg aaacctctga    1440
agattttgac acctctgact gatcagactg taaatcttgg aaaagaaatc tgcctgaagt    1500
gtgaaatctc tgaaaacata ccaggaaaat ggactaaaaa tggcctacct gttcaggaga    1560
gtgaccgtct aaaggtggtt cacaagggaa ggatccacaa gttagtgata gccaatgccc    1620
tcactgaaga tgaaggtgat tatgtatttg cacctgatgc ctacaatgtt actctgcctg    1680
```

ccaaagttca tgttattgat cctcctaaga tcatcctgga tggtcttgat gctgacaaca 1740
cagtgacagt gattgcagga aacaagcttc gtcttgagat ccccatcagc ggagaaccac 1800
ctcctaaagc catgtggagc cggggagata aggctattat ggaaggcagt ggccggataa 1860
gaacagaatc ttaccctgat agcagcactc tggtcattga tatagctgaa agagatgact 1920
ctggtgttta ccacatcaat ctgaaaaacg aagctggaga ggcacatgca agcatcaagg 1980
ttaaagttgt ggacttccct gatcctccag tggcaccgac tgtgacagag gtgggagatg 2040
actggtgtat catgaactgg gagcctcctg cctacgacgg aggctctcca atcctaggat 2100
attttattga gaggaagaag aaacaaagct ccaggtggat gaggctgaat tttgatctct 2160
gcaaagaaac aacttttgag cccaagaaga tgattgaagg tgtggcctat gaggtccgca 2220
tctttgcagt caatgccatt ggcatctcca agcccagtat gccctccagg ccttttgttc 2280
ctttggctgt aacaagccct cctactcttc tgactgtgga ctctgtcact gacacgactg 2340
tcacgatgag gtggcgcccc ccagaccaca ttggtgcagc aggtttagat ggctatgtgc 2400
tagagtattg ctttgaagga agtacatcag caaaacagtc tgatgaaaat ggggaggctg 2460
cctatgatct gccagctgag gactggatag ttgcaaacaa agatctgatt gacaagacga 2520
agttcaccat cacaggtctg ccaacagatg caaagatctt tgtgcgtgtg aaggctgtta 2580
atgcagctgg tgccagcgag cccaagtact attctcagcc cattctcgtg aaggaaatca 2640
tagaacctcc aaagattcgc attccaagac acctgaagca aacctatatc cgcagagttg 2700
gagaagctgt caatctggtt ataccttcc agggaaaacc aagaccagaa ttaacttgga 2760
agaaggatgg tgcagaaatt gataagaatc aaataaacat tcgcaactct gagactgata 2820
caatcatatt tattagaaaa gcagagagga gccactctgg gaaatatgat ctgcaagtca 2880
aagtggacaa attcgtggag accgcatcaa ttgacatcca gatcattgac cgtccaggtc 2940
caccccaaat tgtgaagatt gaggatgtct ggggagaaaa tgtcgctctc acatggactc 3000
caccaaagga tgatggaaat gctgctatca caggctatac cattcagaag gctgacaaga 3060
agagcatgga atggtttact gtcattgagc attatcatcg aaccagtgcc accattactg 3120
aattggtcat agggaatgaa tattacttcc gggtcttttc tgaaaacatg tgtggcctca 3180
gtgaggatgc caccatgact aaagagagtg cagtgatcgc cagggatggt aaaatctaca 3240
aaaatccagt gtatgaagac tttgatttct cagaggcacc catgtttact cagcctttgg 3300
ttaacaccta tgccatagct ggttacaatg ccaccctaaa ctgcagtgtg agaggaaatc 3360
ctaagcctaa aataacctgg atgaaaaaca aagttgctat tgtggatgat ccaagataca 3420
ggatgttcag caaccaggga gtctgtaccc tggaaattcg caagcccagc ccctatgatg 3480
gaggcactta ctgctgcaaa gcagtcaatg accttgggac agtggagatt gaatgcaaac 3540
tggaggtgaa agtgatatat caaggagtaa ataccccctgg acaaccagtc ttcctggagg 3600

```
ggcagcaaca ggtgggctct ccttctgcag actcctcttg caaggcgtac ctccaaacat      3660
aattgattcg tatctgcgag acttacactc aagcaatcct gaggaatact gagggagggc      3720
ctggctactg tctctctgca ctctgctgct ttgaaatctg gttgaaatga gaaaaagcat      3780
tttctgtttt cccaccaggc ccccaagtgt ggtctttttc tttcctccta atgttgaaga      3840
gaaaaaaaaa aaaaaaagtt tgcccagatt gcttaattaa aaattgcaaa caaaatctca      3900
tttgaagtca aaaaaaaaaa aaaaaaaaaa                                       3930
```

<210> 65
<211> 3919
<212> DNA
<213> Homo sapiens

<400> 65

agaggcagta taaaagcact gggtttctcc ccaactgctt gtcacaccga cctgcaccat 60
ctctcgcctg cctgtggggt ttctgtcaac tagtcgtgga gggaaggaga ctctttaaag 120
aataacatct tattgtggcc atgccagaac ccactaagaa agaggaaaat gaagtgccag 180
ccccagcccc acccccggaa gaaccaagta aagagaagga ggccggaact acaccagcaa 240
aagactggac ccttgtcgaa actcctcctg gggaggaaca agccaagcag aatgccaact 300
cccagctgtc catcttgttc attgaaaaac ctcaaggagg aacagtgaaa gttggtgaag 360
atatcacctt catagccaaa gtcaaggctg aagatcttct gagaaaaccc actatcaaat 420
ggttcaaagg aaaatggatg gacctggcca gcaaagccgg gaagcacctt cagctgaagg 480
aaacctttga gaggcacagt cgggtgtaca catttgagat gcagatcatc aaggccaaag 540
ataactttgc aggaaattac agatgcgagg tcacctataa ggataagttt gacagctgtt 600
catttgatct tgaagtgcac gaatctactg ggactactcc aaacattgac atcagatctg 660
ctttcaagag aagtggagaa ggtcaagagg atgcaggaga acttgacttt agtggtctcc 720
tgaaacgtag ggaggtgaag cagcaggagg aagaacccca ggtggacgta tgggagttgc 780
tgaagaacgc gaaacccagt gagtacgaga agatcgcctt ccagtatgga atcaccgacc 840
tgcgcggcat gctcaagcga ctcaagcgca tgcgcagaga ggagaagaag agcgcagctt 900
ttgcaaaaat tcttgatcct gcatatcagg ttgacaaagg aggcagagtg aggtttgttg 960
tggagctggc agatccaaag ttggaggtga aatggtataa aaatggtcaa gaaattcgac 1020
ccagtaccaa atacatcttt gaacacaaag gatgccagag aatcctgttt atcaataact 1080
gtcagatgac agatgattca gagtattatg tgacagccgg tgatgagaaa tgttccactg 1140
agctcttcgt aagagagcct ccaattatgg tgaccaaaca gctggaagat acaactgctt 1200
attgtgggga gagagtggaa ttagaatgtg aggtgtctga agatgatgcc aatgtaaaat 1260
ggtttaagaa tggtgaagag attatccctg gtccaaaatc aagataccga attagagttg 1320

agggtaaaaa acacatcttg atcatagagg gagcaacaaa ggctgatgct gcagaatatt 1380
cagtaatgac aacaggagga caatcatctg ctaaacttag tgttgacttg aaacctctga 1440
agattttgac acctctgact gatcagactg taaatcttgg aaaagaaatc tgcctgaagt 1500
gtgaaatctc tgaaaacata ccaggaaaat ggactaaaaa tggcctacct gttcaggaga 1560
gtgaccgtct aaaggtggtt cacaagggaa ggatccacaa gttagtgata gccaatgccc 1620
tcactgaaga tgaaggtgat tatgtatttg cacctgatgc ctacaatgtt actctgcctg 1680
ccaaagttca tgttattgat cctcctaaga tcatcctgga tggtcttgat gctgacaaca 1740
cagtgacagt gattgcagga aacaagcttc gtcttgagat ccccatcagc ggagaaccac 1800
ctcctaaagc catgtggagc cggggagata aggctattat ggaaggcagt ggccggataa 1860
gaacagaatc ttaccctgat agcagcactc tggtcattga tatagctgaa agagatgact 1920
ctggtgttta ccacatcaat ctgaaaaacg aagctggaga ggcacatgca agcatcaagg 1980
ttaaagttgt ggacttccct gatcctccag tggcaccgac tgtgacagag gtgggagatg 2040
actggtgtat catgaactgg gagcctcctg cctacgacgg aggctctcca atcctaggat 2100
attttattga gaggaagaag aaacaaagct ccaggtggat gaggctgaat tttgatctct 2160
gcaaagaaac aacttttgag cccaagaaga tgattgaagg tgtggcctat gaggtccgca 2220
tctttgcagt caatgccatt ggcatctcca agcccagtat gccctccagg ccttttgttc 2280
ctttggctgt aacaagccct cctactcttc tgactgtgga ctctgtcact gacacgactg 2340
tcacgatgag gtggcgcccc ccagaccaca ttggtgcagc aggtttagat ggctatgtgc 2400
tagagtattg ctttgaagga actgaggact ggatagttgc aaacaaagat ctgattgaca 2460
agacgaagtt caccatcaca ggtctgccaa cagatgcaaa gatctttgtg cgtgtgaagg 2520
ctgttaatgc agctggtgcc agcgagccca agtactattc tcagcccatt ctcgtgaagg 2580
aaatcataga acctccaaag attcgcattc caagacacct gaagcaaacc tatatccgca 2640
gagttggaga agctgtcaat ctggttatac ctttccaggg aaaaccaaga ccagaattaa 2700
cttggaagaa ggatggtgca gaaattgata agaatcaaat aaacattcgc aactctgaga 2760
ctgatacaat catatttatt agaaaagcag agaggagcca ctctgggaaa tatgatctgc 2820
aagtcaaagt ggacaaattc gtggagaccg catcaattga catccagatc attgaccgtc 2880
caggtccacc ccaaattgtg aagattgagg atgtctgggg agaaaatgtc gctctcacat 2940
ggactccacc aaaggatgat ggaaatgctg ctatcacagg ctataccatt cagaaggctg 3000
acaagaagag catggaatgg tttactgtca ttgagcatta tcatcgaacc agtgccacca 3060
ttactgaatt ggtcataggg aatgaatatt acttccgggt cttttctgaa aacatgtgtg 3120
gcctcagtga ggatgccacc atgactaaag agagtgcagt gatcgccagg gatggtaaaa 3180

```
tctacaaaaa tccagtgtat gaagactttg atttctcaga ggcacccatg tttactcagc      3240
ctttggttaa cacctatgcc atagctggtt acaatgccac cctaaactgc agtgtgagag      3300
gaaatcctaa gcctaaaata acctggatga aaaacaaagt tgctattgtg gatgatccaa      3360
gatacaggat gttcagcaac cagggagtct gtaccctgga aattcgcaag cccagcccct      3420
atgatggagg cacttactgc tgcaaagcag tcaatgacct tgggacagtg gagattgaat      3480
gcaaactgga ggtgaaagtg atatatcaag gagtaaatac ccctggacaa ccagtcttcc      3540
tggaggggca gcaacagtca ttgcacaata aggatttttg aatgtataat atcatctaag      3600
gtgggctctc cttctgcaga ctcctcttgc aaggcgtacc tccaaacata attgattcgt      3660
atctgcgaga cttacactca agcaatcctg aggaatactg agggagggcc tggctactgt      3720
ctctctgcac tctgctgctt tgaaatctgg ttgaaatgag aaaaagcatt ttctgttttc      3780
ccaccaggcc cccaagtgtg gtctttttct ttcctcctaa tgttgaagag aaaaaaaaaa      3840
aaaaaagttt gcccagattg cttaattaaa aattgcaaac aaaatctcat ttgaagtcaa      3900
aaaaaaaaaa aaaaaaaaa                                                   3919
```

<210> 66
<211> 3883
<212> DNA
<213> Homo sapiens

<400> 66

```
agaggcagta taaaagcact gggtttctcc ccaactgctt gtcacaccga cctgcaccat    60
ctctcgcctg cctgtggggt ttctgtcaac tagtcgtgga gggaaggaga ctctttaaag   120
aataacatct tattgtggcc atgccagaac ccactaagaa agaggaacca agtaaagaga   180
aggaggccgg aactacacca gcaaaagact ggacccttgt cgaaactcct cctggggagg   240
aacaagccaa gcagaatgcc aactcccagc tgtccatctt gttcattgaa aaacctcaag   300
gaggaacagt gaaagttggt gaagatatca ccttcatagc caaagtcaag gctgaagatc   360
ttctgagaaa acccactatc aaatggttca aggaaaatg gatggacctg gccagcaaag   420
ccgggaagca ccttcagctg aaggaaacct ttgagaggca cagtcgggtg tacacatttg   480
agatgcagat catcaaggcc aaagataact ttgcaggaaa ttacagatgc gaggtcacct   540
ataaggataa gtttgacagc tgttcatttg atcttgaagt gcacgaatct actgggacta   600
ctccaaacat tgacatcaga tctgctttca agagaagtgg agaaggtcaa gaggatgcag   660
gagaacttga ctttagtggt ctcctgaaac gtagggaggt gaagcagcag gaggaagaac   720
cccaggtgga cgtatgggag ttgctgaaga acgcgaaacc cagtgagtac gagaagatcg   780
ccttccagta tggaatcacc gacctgcgcg gcatgctcaa gcgactcaag cgcatgcgca   840
gagaggagaa gaagagcgca gcttttgcaa aaattcttga tcctgcatat caggttgaca   900
```

aaggaggcag agtgaggttt gttgtggagc tggcagatcc aaagttggag gtgaaatggt 960
ataaaaatgg tcaagaaatt cgacccagta ccaaatacat ctttgaacac aaaggatgcc 1020
agagaatcct gtttatcaat aactgtcaga tgacagatga ttcagagtat tatgtgacag 1080
ccggtgatga gaaatgttcc actgagctct tcgtaagaga gcctccaatt atggtgacca 1140
aacagctgga agatacaact gcttattgtg gggagagagt ggaattagaa tgtgaggtgt 1200
ctgaagatga tgccaatgta aaatggttta agaatggtga agagattatc cctggtccaa 1260
aatcaagata ccgaattaga gttgagggta aaaaacacat cttgatcata gagggagcaa 1320
caaaggctga tgctgcagaa tattcagtaa tgacaacagg aggacaatca tctgctaaac 1380
ttagtgttga cttgaaacct ctgaagattt tgacacctct gactgatcag actgtaaatc 1440
ttggaaaaga aatctgcctg aagtgtgaaa tctctgaaaa cataccagga aaatggacta 1500
aaaatggcct acctgttcag gagagtgacc gtctaaaggt ggttcacaag ggaaggatcc 1560
acaagttagt gatagccaat gccctcactg aagatgaagg tgattatgta tttgcacctg 1620
atgcctacaa tgttactctg cctgccaaag ttcatgttat tgatcctcct aagatcatcc 1680
tggatggtct tgatgctgac aacacagtga cagtgattgc aggaaacaag cttcgtcttg 1740
agatccccat cagcggagaa ccacctccta aagccatgtg gagccgggga gataaggcta 1800
ttatggaagg cagtggccgg ataagaacag aatcttaccc tgatagcagc actctggtca 1860
ttgatatagc tgaaagagat gactctggtg tttaccacat caatctgaaa aacgaagctg 1920
gagaggcaca tgcaagcatc aaggttaaag ttgtggactt ccctgatcct ccagtggcac 1980
cgactgtgac agaggtggga gatgactggt gtatcatgaa ctgggagcct cctgcctacg 2040
acggaggctc tccaatccta ggatatttta ttgagaggaa gaagaaacaa agctccaggt 2100
ggatgaggct gaattttgat ctctgcaaag aaacaacttt tgagcccaag aagatgattg 2160
aaggtgtggc ctatgaggtc cgcatctttg cagtcaatgc cattggcatc tccaagccca 2220
gtatgccctc caggcctttt gttcctttgg ctgtaacaag ccctcctact cttctgactg 2280
tggactctgt cactgacacg actgtcacga tgaggtggcg ccccccagac cacattggtg 2340
cagcaggttt agatggctat gtgctagagt attgctttga aggaactgag gactggatag 2400
ttgcaaacaa agatctgatt gacaagacga agttcaccat cacaggtctg ccaacagatg 2460
caaagatctt tgtgcgtgtg aaggctgtta atgcagctgg tgccagcgag cccaagtact 2520
attctcagcc cattctcgtg aaggaaatca tagaacctcc aaagattcgc attccaagac 2580
acctgaagca aacctatatc cgcagagttg gagaagctgt caatctggtt ataccttttcc 2640
agggaaaacc aagaccagaa ttaacttgga agaaggatgg tgcagaaatt gataagaatc 2700
aaataaacat tcgcaactct gagactgata caatcatatt tattagaaaa gcagagagga 2760
gccactctgg gaaatatgat ctgcaagtca aagtggacaa attcgtggag accgcatcaa 2820

```
ttgacatcca gatcattgac cgtccaggtc caccccaaat tgtgaagatt gaggatgtct      2880
ggggagaaaa tgtcgctctc acatggactc caccaaagga tgatggaaat gctgctatca      2940
caggctatac cattcagaag gctgacaaga agagcatgga atggtttact gtcattgagc      3000
attatcatcg aaccagtgcc accattactg aattggtcat agggaatgaa tattacttcc      3060
gggtcttttc tgaaaacatg tgtggcctca gtgaggatgc caccatgact aaagagagtg      3120
cagtgatcgc cagggatggt aaaatctaca aaaatccagt gtatgaagac tttgatttct      3180
cagaggcacc catgtttact cagcctttgg ttaacaccta tgccatagct ggttacaatg      3240
ccaccctaaa ctgcagtgtg agaggaaatc ctaagcctaa aataacctgg atgaaaaaca      3300
aagttgctat tgtggatgat ccaagataca ggatgttcag caaccaggga gtctgtaccc      3360
tggaaattcg caagcccagc ccctatgatg gaggcactta ctgctgcaaa gcagtcaatg      3420
accttgggac agtggagatt gaatgcaaac tggaggtgaa agtgatatat caaggagtaa      3480
atacccctgg acaaccagtc ttcctggagg ggcagcaaca gtcattgcac aataaggatt      3540
tttgaatgta taatatcatc taaggtgggc tctccttctg cagactcctc ttgcaaggcg      3600
tacctccaaa cataattgat tcgtatctgc gagacttaca ctcaagcaat cctgaggaat      3660
actgagggag ggcctggcta ctgtctctct gcactctgct gctttgaaat ctggttgaaa      3720
tgagaaaaag cattttctgt tttcccacca ggcccccaag tgtggtcttt ttctttcctc      3780
ctaatgttga agagaaaaaa aaaaaaaaaa gtttgcccag attgcttaat taaaaattgc      3840
aaacaaaatc tcatttgaag tcaaaaaaaa aaaaaaaaaa aaa                        3883
```

<210> 67
<211> 3862
<212> DNA
<213> Homo sapiens

<400> 67

```
agaggcagta taaaagcact gggtttctcc ccaactgctt gtcacaccga cctgcaccat      60
ctctcgcctg cctgtggggt ttctgtcaac tagtcgtgga gggaaggaga ctctttaaag     120
aataacatct tattgtggcc atgccagaac ccactaagaa agaggaaaat gaagtgccag     180
ccccagcccc acccccggaa gaaccaagta aagagaagga ggccggaact acaccagcaa     240
aagactggac ccttgtcgaa actcctcctg gggaggaaca agccaagcag aatgccaact     300
cccagctgtc catcttgttc attgaaaaac ctcaaggagg aacagtgaaa gttggtgaag     360
atatcacctt catagccaaa gtcaaggctg aagatcttct gagaaaaccc actatcaaat     420
ggttcaaagg aaaatggatg gacctggcca gcaaagccgg gaagcacctt cagctgaagg     480
aaacctttga gaggcacagt cgggtgtaca catttgagat gcagatcatc aaggccaaag     540
ataactttgc aggaaattac agatgcgagg tcacctataa ggataagttt gacagctgtt     600
```

```
catttgatct tgaagtgcac gaatctactg ggactactcc aaacattgac atcagatctg     660
ctttcaagag aagggaggtg aagcagcagg aggaagaacc ccaggtggac gtatgggagt     720
tgctgaagaa cgcgaaaccc agtgagtacg agaagatcgc cttccagtat ggaatcaccg     780
acctgcgcgg catgctcaag cgactcaagc gcatgcgcag agaggagaag aagagcgcag     840
cttttgcaaa aattcttgat cctgcatatc aggttgacaa aggaggcaga gtgaggtttg     900
ttgtggagct ggcagatcca aagttggagg tgaaatggta taaaaatggt caagaaattc     960
gacccagtac caaatacatc tttgaacaca aaggatgcca gagaatcctg tttatcaata    1020
actgtcagat gacagatgat tcagagtatt atgtgacagc cggtgatgag aaatgttcca    1080
ctgagctctt cgtaagagag cctccaatta tggtgaccaa acagctggaa gatacaactg    1140
cttattgtgg ggagagagtg gaattagaat gtgaggtgtc tgaagatgat gccaatgtaa    1200
aatggtttaa gaatggtgaa gagattatcc ctggtccaaa atcaagatac cgaattagag    1260
ttgagggtaa aaaacacatc ttgatcatag agggagcaac aaaggctgat gctgcagaat    1320
attcagtaat gacaacagga ggacaatcat ctgctaaact tagtgttgac ttgaaacctc    1380
tgaagatttt gacacctctg actgatcaga ctgtaaatct tggaaaagaa atctgcctga    1440
agtgtgaaat ctctgaaaac ataccaggaa aatggactaa aaatggccta cctgttcagg    1500
agagtgaccg tctaaaggtg gttcacaagg gaaggatcca caagttagtg atagccaatg    1560
ccctcactga agatgaaggt gattatgtat ttgcacctga tgcctacaat gttactctgc    1620
ctgccaaagt tcatgttatt gatcctccta agatcatcct ggatggtctt gatgctgaca    1680
acacagtgac agtgattgca ggaaacaagc ttcgtcttga gatcccccatc agcggagaac    1740
cacctcctaa agccatgtgg agccggggag ataaggctat tatggaaggc agtggccgga    1800
taagaacaga atcttaccct gatagcagca ctctggtcat tgatatagct gaaagagatg    1860
actctggtgt ttaccacatc aatctgaaaa acgaagctgg agaggcacat gcaagcatca    1920
aggttaaagt tgtggacttc cctgatcctc cagtggcacc gactgtgaca gaggtgggag    1980
atgactggtg tatcatgaac tgggagcctc ctgcctacga cggaggctct ccaatcctag    2040
gatattttat tgagaggaag aagaaacaaa gctccaggtg gatgaggctg aattttgatc    2100
tctgcaaaga aacaactttt gagcccaaga agatgattga aggtgtggcc tatgaggtcc    2160
gcatctttgc agtcaatgcc attggcatct ccaagcccag tatgccctcc aggccttttg    2220
ttcctttggc tgtaacaagc cctcctactc ttctgactgt ggactctgtc actgacacga    2280
ctgtcacgat gaggtggcgc cccccagacc acattggtgc agcaggttta gatggctatg    2340
tgctagagta ttgctttgaa ggaactgagg actggatagt tgcaaacaaa gatctgattg    2400
acaagacgaa gttcaccatc acaggtctgc caacagatgc aaagatcttt gtgcgtgtga    2460
```

aggctgttaa tgcagctggt gccagcgagc ccaagtacta ttctcagccc attctcgtga 2520

aggaaatcat agaacctcca aagattcgca ttccaagaca cctgaagcaa acctatatcc 2580

gcagagttgg agaagctgtc aatctggtta tacctttcca gggaaaacca agaccagaat 2640

taacttggaa gaaggatggt gcagaaattg ataagaatca aataaacatt cgcaactctg 2700

agactgatac aatcatattt attagaaaag cagagaggag ccactctggg aaatatgatc 2760

tgcaagtcaa agtggacaaa ttcgtggaga ccgcatcaat tgacatccag atcattgacc 2820

gtccaggtcc accccaaatt gtgaagattg aggatgtctg gggagaaaat gtcgctctca 2880

catggactcc accaaaggat gatggaaatg ctgctatcac aggctatacc attcagaagg 2940

ctgacaagaa gagcatggaa tggtttactg tcattgagca ttatcatcga accagtgcca 3000

ccattactga attggtcata gggaatgaat attacttccg ggtcttttct gaaaacatgt 3060

gtggcctcag tgaggatgcc accatgacta aagagagtgc agtgatcgcc agggatggta 3120

aaatctacaa aaatccagtg tatgaagact ttgatttctc agaggcaccc atgtttactc 3180

agcctttggt taacacctat gccatagctg gttacaatgc caccctaaac tgcagtgtga 3240

gaggaaatcc taagcctaaa ataacctgga tgaaaaacaa agttgctatt gtggatgatc 3300

caagatacag gatgttcagc aaccagggag tctgtaccct ggaaattcgc aagcccagcc 3360

cctatgatgg aggcacttac tgctgcaaag cagtcaatga ccttgggaca gtggagattg 3420

aatgcaaact ggaggtgaaa gtgatatatc aaggagtaaa taccccctgga caaccagtct 3480

tcctggaggg gcagcaacag tcattgcaca ataaggattt ttgaatgtat aatatcatct 3540

aaggtgggct ctccttctgc agactcctct tgcaaggcgt acctccaaac ataattgatt 3600

cgtatctgcg agacttacac tcaagcaatc ctgaggaata ctgagggagg gcctggctac 3660

tgtctctctg cactctgctg ctttgaaatc tggttgaaat gagaaaaagc attttctgtt 3720

ttcccaccag gcccccaagt gtggtctttt tctttcctcc taatgttgaa gagaaaaaaa 3780

aaaaaaaaag tttgcccaga ttgcttaatt aaaaattgca aacaaaatct catttgaagt 3840

caaaaaaaaa aaaaaaaaaa aa 3862

<210> 68
<211> 3841
<212> DNA
<213> Homo sapiens

<400> 68

```
agaggcagta taaaagcact gggtttctcc ccaactgctt gtcacaccga cctgcaccat      60
ctctcgcctg cctgtggggt ttctgtcaac tagtcgtgga gggaaggaga ctctttaaag     120
aataacatct tattgtggcc atgccagaac ccactaagaa agaggactgg acccttgtcg     180
aaactcctcc tggggaggaa caagccaagc agaatgccaa ctcccagctg tccatcttgt     240
```

tcattgaaaa acctcaagga ggaacagtga aagttggtga agatatcacc ttcatagcca 300
aagtcaaggc tgaagatctt ctgagaaaac ccactatcaa atggttcaaa ggaaaatgga 360
tggacctggc cagcaaagcc gggaagcacc ttcagctgaa ggaaaccttt gagaggcaca 420
gtcgggtgta cacatttgag atgcagatca tcaaggccaa agataacttt gcaggaaatt 480
acagatgcga ggtcacctat aaggataagt ttgacagctg ttcatttgat cttgaagtgc 540
acgaatctac tgggactact ccaaacattg acatcagatc tgctttcaag agaagtggag 600
aaggtcaaga ggatgcagga gaacttgact ttagtggtct cctgaaacgt agggaggtga 660
agcagcagga ggaagaaccc caggtggacg tatgggagtt gctgaagaac gcgaaaccca 720
gtgagtacga gaagatcgcc ttccagtatg gaatcaccga cctgcgcggc atgctcaagc 780
gactcaagcg catgcgcaga gaggagaaga agagcgcagc ttttgcaaaa attcttgatc 840
ctgcatatca ggttgacaaa ggaggcagag tgaggtttgt tgtggagctg gcagatccaa 900
agttggaggt gaaatggtat aaaaatggtc aagaaattcg acccagtacc aaatacatct 960
ttgaacacaa aggatgccag agaatcctgt ttatcaataa ctgtcagatg acagatgatt 1020
cagagtatta tgtgacagcc ggtgatgaga aatgttccac tgagctcttc gtaagagagc 1080
ctccaattat ggtgaccaaa cagctggaag atacaactgc ttattgtggg gagagagtgg 1140
aattagaatg tgaggtgtct gaagatgatg ccaatgtaaa atggtttaag aatggtgaag 1200
agattatccc tggtccaaaa tcaagatacc gaattagagt tgagggtaaa aaacacatct 1260
tgatcataga gggagcaaca aaggctgatg ctgcagaata ttcagtaatg acaacaggag 1320
gacaatcatc tgctaaactt agtgttgact tgaaacctct gaagattttg acacctctga 1380
ctgatcagac tgtaaatctt ggaaaagaaa tctgcctgaa gtgtgaaatc tctgaaaaca 1440
taccaggaaa atggactaaa aatggcctac ctgttcagga gagtgaccgt ctaaaggtgg 1500
ttcacaaggg aaggatccac aagttagtga tagccaatgc cctcactgaa gatgaaggtg 1560
attatgtatt tgcacctgat gcctacaatg ttactctgcc tgccaaagtt catgttattg 1620
atcctcctaa gatcatcctg gatggtcttg atgctgacaa cacagtgaca gtgattgcag 1680
gaaacaagct tcgtcttgag atccccatca gcggagaacc acctcctaaa gccatgtgga 1740
gccggggaga taaggctatt atggaaggca gtggccggat aagaacagaa tcttaccctg 1800
atagcagcac tctggtcatt gatatagctg aaagagatga ctctggtgtt taccacatca 1860
atctgaaaaa cgaagctgga gaggcacatg caagcatcaa ggttaaagtt gtggacttcc 1920
ctgatcctcc agtggcaccg actgtgacag aggtgggaga tgactggtgt atcatgaact 1980
gggagcctcc tgcctacgac ggaggctctc caatcctagg atattttatt gagaggaaga 2040
agaaacaaag ctccaggtgg atgaggctga attttgatct ctgcaaagaa acaacttttg 2100
agcccaagaa gatgattgaa ggtgtggcct atgaggtccg catctttgca gtcaatgcca 2160

ttggcatctc caagcccagt atgccctcca ggccttttgt tcctttggct gtaacaagcc 2220

ctcctactct tctgactgtg gactctgtca ctgacacgac tgtcacgatg aggtggcgcc 2280

ccccagacca cattggtgca gcaggtttag atggctatgt gctagagtat tgctttgaag 2340

gaactgagga ctggatagtt gcaaacaaag atctgattga caagacgaag ttcaccatca 2400

caggtctgcc aacagatgca aagatctttg tgcgtgtgaa ggctgttaat gcagctggtg 2460

ccagcgagcc caagtactat tctcagccca ttctcgtgaa ggaaatcata gaacctccaa 2520

agattcgcat tccaagacac ctgaagcaaa cctatatccg cagagttgga gaagctgtca 2580

atctggttat acctttccag ggaaaaccaa gaccagaatt aacttggaag aaggatggtg 2640

cagaaattga taagaatcaa ataaacattc gcaactctga gactgataca atcatattta 2700

ttagaaaagc agagaggagc cactctggga aatatgatct gcaagtcaaa gtggacaaat 2760

tcgtggagac cgcatcaatt gacatccaga tcattgaccg tccaggtcca ccccaaattg 2820

tgaagattga ggatgtctgg ggagaaaatg tcgctctcac atggactcca ccaaaggatg 2880

atggaaatgc tgctatcaca ggctatacca ttcagaaggc tgacaagaag agcatggaat 2940

ggtttactgt cattgagcat tatcatcgaa ccagtgccac cattactgaa ttggtcatag 3000

ggaatgaata ttacttccgg gtcttttctg aaaacatgtg tggcctcagt gaggatgcca 3060

ccatgactaa agagagtgca gtgatcgcca gggatggtaa aatctacaaa aatccagtgt 3120

atgaagactt tgatttctca gaggcaccca tgtttactca gcctttggtt aacacctatg 3180

ccatagctgg ttacaatgcc accctaaact gcagtgtgag aggaaatcct aagcctaaaa 3240

taacctggat gaaaaacaaa gttgctattg tggatgatcc aagatacagg atgttcagca 3300

accagggagt ctgtaccctg gaaattcgca agcccagccc ctatgatgga ggcacttact 3360

gctgcaaagc agtcaatgac cttgggacag tggagattga atgcaaactg gaggtgaaag 3420

tgatatatca aggagtaaat acccctggac aaccagtctt cctggagggg cagcaacagt 3480

cattgcacaa taaggatttt tgaatgtata atatcatcta aggtgggctc tccttctgca 3540

gactcctctt gcaaggcgta cctccaaaca taattgattc gtatctgcga gacttacact 3600

caagcaatcc tgaggaatac tgagggaggg cctggctact gtctctctgc actctgctgc 3660

tttgaaatct ggttgaaatg agaaaaagca ttttctgttt tcccaccagg cccccaagtg 3720

tggtcttttt ctttcctcct aatgttgaag agaaaaaaaa aaaaaaaagt ttgcccagat 3780

tgcttaatta aaaattgcaa acaaaatctc atttgaagtc aaaaaaaaaa aaaaaaaaaa 3840

a 3841

<210> 69
<211> 3778
<212> DNA

<213> Homo sapiens

<400> 69

```
agaggcagta taaaagcact gggtttctcc ccaactgctt gtcacaccga cctgcaccat      60
ctctcgcctg cctgtggggt ttctgtcaac tagtcgtgga gggaaggaga ctctttaaag     120
aataacatct tattgtggcc atgccagaac ccactaagaa agaggatgaa gaggaagtct     180
ccccgcctag cgccttgcct ccagactgga cccttgtcga aactcctcct ggggaggaac     240
aagccaagca gaatgccaac tcccagctgt ccatcttgtt cattgaaaaa cctcaaggag     300
gaacagtgaa agttggtgaa gatatcacct tcatagccaa agtcaaggct gaagatcttc     360
tgagaaaacc cactatcaaa tggttcaaag gaaaatggat ggacctggcc agcaaagccg     420
ggaagcacct tcagctgaag gaaacctttg agaggcacag tcgggtgtac acatttgaga     480
tgcagatcat caaggccaaa gataactttg caggaaatta cagatgcgag gtcacctata     540
aggataagtt tgacagctgt tcatttgatc ttgaagtgca cgaatctact gggactactc     600
caaacattga catcagatct gctttcaaga gaagtggaga aggtcaagag gatgcaggag     660
aacttgactt tagtggtctc ctgaaacgta gggaggtgaa gcagcaggag gaagaacccc     720
aggtggacgt atgggagttg ctgaagaacg cgaaacccag tgagtacgag aagatcgcct     780
tccagtatgg aatcaccgac ctgcgcggca tgctcaagcg actcaagcgc atgcgcagag     840
aggagaagaa gagcgcagct tttgcaaaaa ttcttgatcc tgcatatcag gttgacaaag     900
gaggcagagt gaggtttgtt gtggagctgg cagatccaaa gttggaggtg aaatggtata     960
aaaatggtca agaaattcga cccagtacca aatacatctt tgaacacaaa ggatgccaga    1020
gaatcctgtt tatcaataac tgtcagatga cagatgattc agagtattat gtgacagccg    1080
gtgatgagaa atgttccact gagctcttcg taagagagcc tccaattatg gtgaccaaac    1140
agctggaaga tacaactgct tattgtgggg agagagtgga attagaatgt gaggtgtctg    1200
aagatgatgc caatgtaaaa tggtttaaga atggtgaaga gattatccct ggtccaaaat    1260
caagataccg aattagagtt gagggtaaaa aacacatctt gatcatagag ggagcaacaa    1320
aggctgatgc tgcagaatat tcagtaatga caacaggagg acaatcatct gctaaactta    1380
gtgttgactt gaaacctctg aagattttga cacctctgac tgatcagact gtaaatcttg    1440
gaaaagaaat ctgcctgaag tgtgaaatct ctgaaaacat accaggaaaa tggactaaaa    1500
atggcctacc tgttcaggag agtgaccgtc taaaggtggt tcacaaggga aggatccaca    1560
agttagtgat agccaatgcc ctcactgaag atgaaggtga ttatgtattt gcacctgatg    1620
cctacaatgt tactctgcct gccaaagttc atgttattga tcctcctaag atcatcctgg    1680
atggtcttga tgctgacaac acagtgacag tgattgcagg aaacaagctt cgtcttgaga    1740
tccccatcag cggagaacca cctcctaaag ccatgtggag ccggggagat aaggctatta    1800
```

tggaaggcag tggccggata agaacagaat cttaccctga tagcagcact ctggtcattg 1860
atatagctga aagagatgac tctggtgttt accacatcaa tctgaaaaac gaagctggag 1920
aggcacatgc aagcatcaag gttaaagttg tggacttccc tgatcctcca gtggcaccga 1980
ctgtgacaga ggtgggagat gactggtgta tcatgaactg ggagcctcct gcctacgacg 2040
gaggctctcc aatcctagga tattttattg agaggaagaa gaaacaaagc tccaggtgga 2100
tgaggctgaa ttttgatctc tgcaaagaaa caacttttga gcccaagaag atgattgaag 2160
gtgtggccta tgaggtccgc atctttgcag tcaatgccat tggcatctcc aagcccagta 2220
tgccctccag gccttttgtt cctttggctg taacaagccc tcctactctt ctgactgtgg 2280
actctgtcac tgacacgact gtcacgatga ggtggcgccc cccagaccac attggtgcag 2340
caggtttaga tggctatgtg ctagagtatt gctttgaagg aactgaggac tggatagttg 2400
caaacaaaga tctgattgac aagacgaagt tcaccatcac aggtctgcca acagatgcaa 2460
agatctttgt gcgtgtgaag gctgttaatg cagctggtgc cagcgagccc aagtactatt 2520
ctcagcccat tctcgtgaag gaaatcatag aacctccaaa gattcgcatt ccaagacacc 2580
tgaagcaaac ctatatccgc agagttggag aagctgtcaa tctggttata cctttccagg 2640
gaaaaccaag accagaatta acttggaaga aggatggtgc agaaattgat aagaatcaaa 2700
taaacattcg caactctgag actgatacaa tcatatttat tagaaaagca gagaggagcc 2760
actctgggaa atatgatctg caagtcaaag tggacaaatt cgtggagacc gcatcaattg 2820
acatccagat cattgaccgt ccaggtccac cccaaattgt gaagattgag gatgtctggg 2880
gagaaaatgt cgctctcaca tggactccac caaaggatga tggaaatgct gctatcacag 2940
gctataccat tcagaaggct gacaagaaga gcatggaatg gtttactgtc attgagcatt 3000
atcatcgaac cagtgccacc attactgaat tggtcatagg gaatgaatat tacttccggg 3060
tcttttctga aaacatgtgt ggcctcagtg aggatgccac catgactaaa gagagtgcag 3120
tgatcgccag ggatggtaaa atctacaaaa atccagtgta tgaagacttt gatttctcag 3180
aggcacccat gtttactcag cctttggtta acacctatgc catagctggt tacaatgcca 3240
ccctaaactg cagtgtgaga ggaaatccta agcctaaaat aacctggatg aaaaacaaag 3300
ttgctattgt ggatgatcca agatacagga tgttcagcaa ccagggagtc tgtaccctgg 3360
aaattcgcaa gcccagcccc tatgatggag gcacttactg ctgcaaagca gtcaatgacc 3420
ttgggacagt ggagattgaa tgcaaactgg aggtgaaagg tgggctctcc ttctgcagac 3480
tcctcttgca aggcgtacct ccaaacataa ttgattcgta tctgcgagac ttacactcaa 3540
gcaatcctga ggaatactga gggagggcct ggctactgtc tctctgcact ctgctgcttt 3600
gaaatctggt tgaaatgaga aaaagcattt tctgttttcc caccaggccc ccaagtgtgg 3660
tctttttctt tcctcctaat gttgaagaga aaaaaaaaaa aaaaagtttg cccagattgc 3720

ttaattaaaa attgcaaaca aaatctcatt tgaagtcaaa aaaaaaaaaa aaaaaaaa 3778

<210> 70
<211> 3994
<212> DNA
<213> Homo sapiens

<400> 70

agaggcagta taaaagcact gggtttctcc ccaactgctt gtcacaccga cctgcaccat 60
ctctcgcctg cctgtggggt ttctgtcaac tagtcgtgga gggaaggaga ctctttaaag 120
aataacatct tattgtggcc atgccagaac ccactaagaa agaggaaaat gaagtgccag 180
ccccagcccc acccccggaa gaaccaagta aagagaagga ggccggaact acaccagcaa 240
aagatgaaga ggaagtctcc ccgcctagcg ccttgcctcc aggtttgggt agtcgggccc 300
tggagagaaa agattcagac tggacccttg tcgaaactcc tcctggggag gaacaagcca 360
agcagaatgc caactcccag ctgtccatct tgttcattga aaaacctcaa ggaggaacag 420
tgaaagttgg tgaagatatc accttcatag ccaaagtcaa ggctgaagat cttctgagaa 480
aacccactat caaatggttc aaaggaaaat ggatggacct ggccagcaaa gccgggaagc 540
accttcagct gaaggaaacc tttgagaggc acagtcgggt gtacacattt gagatgcaga 600
tcatcaaggc caaagataac tttgcaggaa attacagatg cgaggtcacc tataaggata 660
agtttgacag ctgttcattt gatcttgaag tgcacgaatc tactgggact actccaaaca 720
ttgacatcag atctgctttc aagagaagtg gagaaggtca agaggatgca ggagaacttg 780
actttagtgg tctcctgaaa cgtagggagg tgaagcagca ggaggaagaa ccccaggtgg 840
acgtatggga gttgctgaag aacgcgaaac ccagtgagta cgagaagatc gccttccagt 900
atggaatcac cgacctgcgc ggcatgctca agcgactcaa gcgcatgcgc agagaggaga 960
agaagagcgc agcttttgca aaaattcttg atcctgcata tcaggttgac aaaggaggca 1020
gagtgaggtt tgttgtggag ctggcagatc caaagttgga ggtgaaatgg tataaaaatg 1080
gtcaagaaat tcgacccagt accaaataca tctttgaaca caaaggatgc cagagaatcc 1140
tgtttatcaa taactgtcag atgacagatg attcagagta ttatgtgaca gccggtgatg 1200
agaaatgttc cactgagctc ttcgtaagag agcctccaat tatggtgacc aaacagctgg 1260
aagatacaac tgcttattgt ggggagagag tggaattaga atgtgaggtg tctgaagatg 1320
atgccaatgt aaaatggttt aagaatggtg aagagattat ccctggtcca aaatcaagat 1380
accgaattag agttgagggt aaaaaacaca tcttgatcat agagggagca acaaaggctg 1440
atgctgcaga atattcagta atgacaacag gaggacaatc atctgctaaa cttagtgttg 1500
acttgaaacc tctgaagatt ttgacacctc tgactgatca gactgtaaat cttggaaaag 1560
aaatctgcct gaagtgtgaa atctctgaaa acataccagg aaaatggact aaaaatggcc 1620

tacctgttca ggagagtgac cgtctaaagg tggttcacaa gggaaggatc cacaagttag 1680

tgatagccaa tgccctcact gaagatgaag gtgattatgt atttgcacct gatgcctaca 1740

atgttactct gcctgccaaa gttcatgtta ttgatcctcc taagatcatc ctggatggtc 1800

ttgatgctga caacacagtg acagtgattg caggaaacaa gcttcgtctt gagatcccca 1860

tcagcggaga accacctcct aaagccatgt ggagccgggg agataaggct attatggaag 1920

gcagtggccg gataagaaca gaatcttacc ctgatagcag cactctggtc attgatatag 1980

ctgaaagaga tgactctggt gtttaccaca tcaatctgaa aaacgaagct ggagaggcac 2040

atgcaagcat caaggttaaa gttgtggact tccctgatcc tccagtggca ccgactgtga 2100

cagaggtggg agatgactgg tgtatcatga actgggagcc tcctgcctac gacggaggct 2160

ctccaatcct aggatatttt attgagagga agaagaaaca aagctccagg tggatgaggc 2220

tgaattttga tctctgcaaa gaaacaactt ttgagcccaa gaagatgatt gaaggtgtgg 2280

cctatgaggt ccgcatcttt gcagtcaatg ccattggcat ctccaagccc agtatgccct 2340

ccaggccttt tgttcctttg gctgtaacaa gccctcctac tcttctgact gtggactctg 2400

tcactgacac gactgtcacg atgaggtggc gcccccccaga ccacattggt gcagcaggtt 2460

tagatggcta tgtgctagag tattgctttg aaggaactga ggactggata gttgcaaaca 2520

aagatctgat tgacaagacg aagttcacca tcacaggtct gccaacagat gcaaagatct 2580

ttgtgcgtgt gaaggctgtt aatgcagctg gtgccagcga gcccaagtac tattctcagc 2640

ccattctcgt gaaggaaatc atagaacctc caaagattcg cattccaaga cacctgaagc 2700

aaacctatat ccgcagagtt ggagaagctg tcaatctggt tatacctttc cagggaaaac 2760

caagaccaga attaacttgg aagaaggatg gtgcagaaat tgataagaat caaataaaca 2820

ttcgcaactc tgagactgat acaatcatat ttattagaaa agcagagagg agccactctg 2880

ggaaatatga tctgcaagtc aaagtggaca aattcgtgga gaccgcatca attgacatcc 2940

agatcattga ccgtccaggt ccaccccaaa ttgtgaagat tgaggatgtc tggggagaaa 3000

atgtcgctct cacatggact ccaccaaagg atgatggaaa tgctgctatc acaggctata 3060

ccattcagaa ggctgacaag aagagcatgg aatggtttac tgtcattgag cattatcatc 3120

gaaccagtgc caccattact gaattggtca tagggaatga atattacttc cgggtctttt 3180

ctgaaaacat gtgtggcctc agtgaggatg ccaccatgac taaagagagt gcagtgatcg 3240

ccagggatgg taaaatctac aaaaatccag tgtatgaaga ctttgatttc tcagaggcac 3300

ccatgtttac tcagcctttg gttaacacct atgccatagc tggttacaat gccaccctaa 3360

actgcagtgt gagaggaaat cctaagccta aaataacctg gatgaaaaac aaagttgcta 3420

ttgtggatga tccaagatac aggatgttca gcaaccaggg agtctgtacc ctggaaattc 3480

gcaagcccag cccctatgat ggaggcactt actgctgcaa agcagtcaat gaccttggga 3540

cagtggagat tgaatgcaaa ctggaggtga aagtgatata tcaaggagta aataccccctg 3600

gacaaccagt cttcctggag gggcagcaac agtcattgca caataaggat ttttgaatgt 3660

ataatatcat ctaaggtggg ctctccttct gcagactcct cttgcaaggc gtacctccaa 3720

acataattga ttcgtatctg cgagacttac actcaagcaa tcctgaggaa tactgaggga 3780

gggcctggct actgtctctc tgcactctgc tgctttgaaa tctggttgaa atgagaaaaa 3840

gcattttctg ttttcccacc aggcccccaa gtgtggtctt tttctttcct cctaatgttg 3900

aagagaaaaa aaaaaaaaaa agtttgccca gattgcttaa ttaaaaattg caaacaaaat 3960

ctcatttgaa gtcaaaaaaa aaaaaaaaaa aaaa 3994

<210> 71
<211> 3935
<212> DNA
<213> Homo sapiens

<400> 71

agaggcagta taaaagcact gggtttctcc ccaactgctt gtcacaccga cctgcaccat 60
ctctcgcctg cctgtggggt ttctgtcaac tagtcgtgga gggaaggaga ctctttaaag 120
aataacatct tattgtggcc atgccagaac ccactaagaa agaggaaaat gaagtgccag 180
ccccagcccc acccccggaa gaaccaagta aagagaagga ggccggaact acaccagcaa 240
aagatgaaga ggaagtctcc ccgcctagcg ccttgcctcc aggtttgggt agtcgggccc 300
tggagagaaa agattcagac tggacccttg tcgaaactcc tcctggggag gaacaagcca 360
agcagaatgc caactcccag ctgtccatct tgttcattga aaaacctcaa ggaggaacag 420
tgaaagttgg tgaagatatc accttcatag ccaaagtcaa ggctgaagat cttctgagaa 480
aacccactat caaatggttc aaaggaaaat ggatggacct ggccagcaaa gccgggaagc 540
accttcagct gaaggaaacc tttgagaggc acagtcgggt gtacacattt gagatgcaga 600
tcatcaaggc caaagataac tttgcaggaa attacagatg cgaggtcacc tataaggata 660
agtttgacag ctgttcattt gatcttgaag tgcacgaatc tactgggact actccaaaca 720
ttgacatcag atctgctttc aagagaagtg gagaaggtca agaggatgca ggagaacttg 780
actttagtgg tctcctgaaa cgtagggagg tgaagcagca ggaggaagaa ccccaggtgg 840
acgtatggga gttgctgaag aacgcgaaac ccagtgagta cgagaagatc gccttccagt 900
atggaatcac cgacctgcgc ggcatgctca agcgactcaa gcgcatgcgc agagaggaga 960
agaagagcgc agcttttgca aaaattcttg atcctgcata tcaggttgac aaaggaggca 1020
gagtgaggtt tgttgtggag ctggcagatc caaagttgga ggtgaaatgg tataaaaatg 1080
gtcaagaaat tcgacccagt accaaataca tctttgaaca caaaggatgc cagagaatcc 1140

tgtttatcaa taactgtcag atgacagatg attcagagta ttatgtgaca gccggtgatg 1200
agaaatgttc cactgagctc ttcgtaagag agcctccaat tatggtgacc aaacagctgg 1260
aagatacaac tgcttattgt ggggagagag tggaattaga atgtgaggtg tctgaagatg 1320
atgccaatgt aaaatggttt aagaatggtg aagagattat ccctggtcca aaatcaagat 1380
accgaattag agttgagggt aaaaaacaca tcttgatcat agagggagca acaaaggctg 1440
atgctgcaga atattcagta atgacaacag gaggacaatc atctgctaaa cttagtgttg 1500
acttgaaacc tctgaagatt ttgacacctc tgactgatca gactgtaaat cttggaaaag 1560
aaatctgcct gaagtgtgaa atctctgaaa acataccagg aaaatggact aaaaatggcc 1620
tacctgttca ggagagtgac cgtctaaagg tggttcacaa gggaaggatc cacaagttag 1680
tgatagccaa tgccctcact gaagatgaag gtgattatgt atttgcacct gatgcctaca 1740
atgttactct gcctgccaaa gttcatgtta ttgatcctcc taagatcatc ctggatggtc 1800
ttgatgctga caacacagtg acagtgattg caggaaacaa gcttcgtctt gagatcccca 1860
tcagcggaga accacctcct aaagccatgt ggagccgggg agataaggct attatggaag 1920
gcagtggccg gataagaaca gaatcttacc ctgatagcag cactctggtc attgatatag 1980
ctgaaagaga tgactctggt gtttaccaca tcaatctgaa aaacgaagct ggagaggcac 2040
atgcaagcat caaggttaaa gttgtggact tccctgatcc tccagtggca ccgactgtga 2100
cagaggtggg agatgactgg tgtatcatga actgggagcc tcctgcctac gacggaggct 2160
ctccaatcct aggatatttt attgagagga agaagaaaca aagctccagg tggatgaggc 2220
tgaattttga tctctgcaaa gaaacaactt ttgagcccaa gaagatgatt gaaggtgtgg 2280
cctatgaggt ccgcatcttt gcagtcaatg ccattggcat ctccaagccc agtatgccct 2340
ccaggccttt tgttcctttg gctgtaacaa gccctcctac tcttctgact gtggactctg 2400
tcactgacac gactgtcacg atgaggtggc gcccccccaga ccacattggt gcagcaggtt 2460
tagatggcta tgtgctagag tattgctttg aaggaactga ggactggata gttgcaaaca 2520
aagatctgat tgacaagacg aagttcacca tcacaggtct gccaacagat gcaaagatct 2580
ttgtgcgtgt gaaggctgtt aatgcagctg gtgccagcga gcccaagtac tattctcagc 2640
ccattctcgt gaaggaaatc atagaacctc caaagattcg cattccaaga cacctgaagc 2700
aaacctatat ccgcagagtt ggagaagctg tcaatctggt tatacctttc cagggaaaac 2760
caagaccaga attaacttgg aagaaggatg gtgcagaaat tgataagaat caaataaaca 2820
ttcgcaactc tgagactgat acaatcatat ttattagaaa agcagagagg agccactctg 2880
ggaaatatga tctgcaagtc aaagtggaca aattcgtgga gaccgcatca attgacatcc 2940
agatcattga ccgtccaggt ccaccccaaa ttgtgaagat tgaggatgtc tggggagaaa 3000
atgtcgctct cacatggact ccaccaaagg atgatggaaa tgctgctatc acaggctata 3060

ccattcagaa ggctgacaag aagagcatgg aatggtttac tgtcattgag cattatcatc 3120
gaaccagtgc caccattact gaattggtca tagggaatga atattacttc cgggtctttt 3180
ctgaaaacat gtgtggcctc agtgaggatg ccaccatgac taaagagagt gcagtgatcg 3240
ccagggatgg taaaatctac aaaaatccag tgtatgaaga ctttgatttc tcagaggcac 3300
ccatgtttac tcagcctttg gttaacacct atgccatagc tggttacaat gccaccctaa 3360
actgcagtgt gagaggaaat cctaagccta aaataacctg gatgaaaaac aaagttgcta 3420
ttgtggatga tccaagatac aggatgttca gcaaccaggg agtctgtacc ctggaaattc 3480
gcaagcccag cccctatgat ggaggcactt actgctgcaa agcagtcaat gaccttggga 3540
cagtggagat tgaatgcaaa ctggaggtga aagtcattgc acaataagga tttttgaatg 3600
tataatatca tctaaggtgg gctctccttc tgcagactcc tcttgcaagg cgtacctcca 3660
aacataattg attcgtatct gcgagactta cactcaagca atcctgagga atactgaggg 3720
agggcctggc tactgtctct ctgcactctg ctgctttgaa atctggttga aatgagaaaa 3780
agcattttct gttttcccac caggccccca agtgtggtct tttctttcc tcctaatgtt 3840
gaagagaaaa aaaaaaaaaa aagtttgccc agattgctta attaaaaatt gcaaacaaaa 3900
tctcatttga agtcaaaaaa aaaaaaaaaa aaaaa 3935

<210> 72
<211> 3914
<212> DNA
<213> Homo sapiens

<400> 72

```
agaggcagta taaaagcact gggtttctcc ccaactgctt gtcacaccga cctgcaccat        60
ctctcgcctg cctgtggggt ttctgtcaac tagtcgtgga gggaaggaga ctctttaaag       120
aataacatct tattgtggcc atgccagaac ccactaagaa agaggaaaat gaagtgccag       180
ccccagcccc acccccggaa gaaccaagta aagagaagga ggccggaact acaccagcaa       240
aagactggac ccttgtcgaa actcctcctg ggaggaaca agccaagcag aatgccaact       300
cccagctgtc catcttgttc attgaaaaac ctcaaggagg aacagtgaaa gttggtgaag       360
atatcacctt catagccaaa gtcaaggctg aagatcttct gagaaaaccc actatcaaat       420
ggttcaaagg aaaatggatg gacctggcca gcaaagccgg gaagcacctt cagctgaagg       480
aaacctttga gaggcacagt cgggtgtaca catttgagat gcagatcatc aaggccaaag       540
ataactttgc aggaaattac agatgcgagg tcacctataa ggataagttt gacagctgtt       600
catttgatct tgaagtgcac gaatctactg ggactactcc aaacattgac atcagatctg       660
ctttcaagag aagtggagaa ggtcaagagg atgcaggaga acttgacttt agtggtctcc       720
tgaaacgtag ggaggtgaag cagcaggagg aagaacccca ggtggacgta tgggagttgc       780
```

tgaagaacgc gaaacccagt gagtacgaga agatcgcctt ccagtatgga atcaccgacc 840
tgcgcggcat gctcaagcga ctcaagcgca tgcgcagaga ggagaagaag agcgcagctt 900
ttgcaaaaat tcttgatcct gcatatcagg ttgacaaagg aggcagagtg aggtttgttg 960
tggagctggc agatccaaag ttggaggtga aatggtataa aaatggtcaa gaaattcgac 1020
ccagtaccaa atacatcttt gaacacaaag gatgccagag aatcctgttt atcaataact 1080
gtcagatgac agatgattca gagtattatg tgacagccgg tgatgagaaa tgttccactg 1140
agctcttcgt aagagagcct ccaattatgg tgaccaaaca gctggaagat acaactgctt 1200
attgtgggga gagagtggaa ttagaatgtg aggtgtctga agatgatgcc aatgtaaaat 1260
ggtttaagaa tggtgaagag attatccctg gtccaaaatc aagataccga attagagttg 1320
agggtaaaaa acacatcttg atcatagagg gagcaacaaa ggctgatgct gcagaatatt 1380
cagtaatgac aacaggagga caatcatctg ctaaacttag tgttgacttg aaacctctga 1440
agattttgac acctctgact gatcagactg taaatcttgg aaaagaaatc tgcctgaagt 1500
gtgaaatctc tgaaaacata ccaggaaaat ggactaaaaa tggcctacct gttcaggaga 1560
gtgaccgtct aaaggtggtt cacaagggaa ggatccacaa gttagtgata gccaatgccc 1620
tcactgaaga tgaaggtgat tatgtatttg cacctgatgc ctacaatgtt actctgcctg 1680
ccaaagttca tgttattgat cctcctaaga tcatcctgga tggtcttgat gctgacaaca 1740
cagtgacagt gattgcagga aacaagcttc gtcttgagat ccccatcagc ggagaaccac 1800
ctcctaaagc catgtggagc cggggagata aggctattat ggaaggcagt ggccggataa 1860
gaacagaatc ttaccctgat agcagcactc tggtcattga tatagctgaa agagatgact 1920
ctggtgttta ccacatcaat ctgaaaaacg aagctggaga ggcacatgca agcatcaagg 1980
ttaaagttgt ggacttccct gatcctccag tggcaccgac tgtgacagag gtgggagatg 2040
actggtgtat catgaactgg gagcctcctg cctacgacgg aggctctcca atcctaggat 2100
attttattga gaggaagaag aaacaaagct ccaggtggat gaggctgaat tttgatctct 2160
gcaaagaaac aacttttgag cccaagaaga tgattgaagg tgtggcctat gaggtccgca 2220
tctttgcagt caatgccatt ggcatctcca agcccagtat gccctccagg ccttttgttc 2280
ctttggctgt aacaagccct cctactcttc tgactgtgga ctctgtcact gacacgactg 2340
tcacgatgag gtggcgcccc ccagaccaca ttggtgcagc aggtttagat ggctatgtgc 2400
tagagtattg ctttgaagga agtacatcag caaaacagtc tgatgaaaat ggggaggctg 2460
cctatgatct gccagctgag gactggatag ttgcaaacaa agatctgatt gacaagacga 2520
agttcaccat cacaggtctg ccaacagatg caaagatctt tgtgcgtgtg aaggctgtta 2580
atgcagctgg tgccagcgag cccaagtact attctcagcc cattctcgtg aaggaaatca 2640

tagaacctcc aaagattcgc attccaagac acctgaagca aacctatatc cgcagagttg 2700

gagaagctgt caatctggtt atacctttcc agggaaaacc aagaccagaa ttaacttgga 2760

agaaggatgg tgcagaaatt gataagaatc aaataaacat tcgcaactct gagactgata 2820

caatcatatt tattagaaaa gcagagagga gccactctgg gaaatatgat ctgcaagtca 2880

aagtggacaa attcgtggag accgcatcaa ttgacatcca gatcattgac cgtccaggtc 2940

caccccaaat tgtgaagatt gaggatgtct ggggagaaaa tgtcgctctc acatggactc 3000

caccaaagga tgatggaaat gctgctatca caggctatac cattcagaag gctgacaaga 3060

agagcatgga atggtttact gtcattgagc attatcatcg aaccagtgcc accattactg 3120

aattggtcat agggaatgaa tattacttcc gggtcttttc tgaaaacatg tgtggcctca 3180

gtgaggatgc caccatgact aaagagagtg cagtgatcgc cagggatggt aaaatctaca 3240

aaaatccagt gtatgaagac tttgatttct cagaggcacc catgtttact cagcctttgg 3300

ttaacaccta tgccatagct ggttacaatg ccaccctaaa ctgcagtgtg agaggaaatc 3360

ctaagcctaa aataacctgg atgaaaaaca aagttgctat tgtggatgat ccaagataca 3420

ggatgttcag caaccaggga gtctgtaccc tggaaattcg caagcccagc ccctatgatg 3480

gaggcactta ctgctgcaaa gcagtcaatg accttgggac agtggagatt gaatgcaaac 3540

tggaggtgaa agtcattgca caataaggat tttgaatgt ataatatcat ctaaggtggg 3600

ctctccttct gcagactcct cttgcaaggc gtacctccaa acataattga ttcgtatctg 3660

cgagacttac actcaagcaa tcctgaggaa tactgaggga gggcctggct actgtctctc 3720

tgcactctgc tgctttgaaa tctggttgaa atgagaaaaa gcattttctg ttttcccacc 3780

aggcccccaa gtgtggtctt tttctttcct cctaatgttg aagagaaaaa aaaaaaaaaa 3840

agtttgccca gattgcttaa ttaaaaattg caaacaaaat ctcatttgaa gtcaaaaaaa 3900

aaaaaaaaaa aaaa 3914

<210> 73
<211> 3860
<212> DNA
<213> Homo sapiens

<400> 73

```
agaggcagta taaaagcact gggtttctcc ccaactgctt gtcacaccga cctgcaccat      60
ctctcgcctg cctgtggggt ttctgtcaac tagtcgtgga gggaaggaga ctctttaaag     120
aataacatct tattgtggcc atgccagaac ccactaagaa agaggaaaat gaagtgccag     180
ccccagcccc acccccggaa gaaccaagta aagagaagga ggccggaact acaccagcaa     240
aagactggac ccttgtcgaa actcctcctg ggagggaaca agccaagcag aatgccaact     300
cccagctgtc catcttgttc attgaaaaac ctcaaggagg aacagtgaaa gttggtgaag     360
```

atatcacctt catagccaaa gtcaaggctg aagatcttct gagaaaaccc actatcaaat 420

ggttcaaagg aaaatggatg gacctggcca gcaaagccgg gaagcacctt cagctgaagg 480

aaacctttga gaggcacagt cgggtgtaca catttgagat gcagatcatc aaggccaaag 540

ataactttgc aggaaattac agatgcgagg tcacctataa ggataagttt gacagctgtt 600

catttgatct tgaagtgcac gaatctactg ggactactcc aaacattgac atcagatctg 660

ctttcaagag aagtggagaa ggtcaagagg atgcaggaga acttgacttt agtggtctcc 720

tgaaacgtag ggaggtgaag cagcaggagg aagaacccca ggtggacgta tgggagttgc 780

tgaagaacgc gaaacccagt gagtacgaga agatcgcctt ccagtatgga atcaccgacc 840

tgcgcggcat gctcaagcga ctcaagcgca tgcgcagaga ggagaagaag agcgcagctt 900

ttgcaaaaat tcttgatcct gcatatcagg ttgacaaagg aggcagagtg aggtttgttg 960

tggagctggc agatccaaag ttggaggtga aatggtataa aaatggtcaa gaaattcgac 1020

ccagtaccaa atacatcttt gaacacaaag gatgccagag aatcctgttt atcaataact 1080

gtcagatgac agatgattca gagtattatg tgacagccgg tgatgagaaa tgttccactg 1140

agctcttcgt aagagagcct ccaattatgg tgaccaaaca gctggaagat acaactgctt 1200

attgtgggga gagagtggaa ttagaatgtg aggtgtctga agatgatgcc aatgtaaaat 1260

ggtttaagaa tggtgaagag attatccctg gtccaaaatc aagataccga attagagttg 1320

agggtaaaaa acacatcttg atcatagagg gagcaacaaa ggctgatgct gcagaatatt 1380

cagtaatgac aacaggagga caatcatctg ctaaacttag tgttgacttg aaacctctga 1440

agattttgac acctctgact gatcagactg taaatcttgg aaaagaaatc tgcctgaagt 1500

gtgaaatctc tgaaaacata ccaggaaaat ggactaaaaa tggcctacct gttcaggaga 1560

gtgaccgtct aaaggtggtt cacaagggaa ggatccacaa gttagtgata gccaatgccc 1620

tcactgaaga tgaaggtgat tatgtatttg cacctgatgc ctacaatgtt actctgcctg 1680

ccaaagttca tgttattgat cctcctaaga tcatcctgga tggtcttgat gctgacaaca 1740

cagtgacagt gattgcagga aacaagcttc gtcttgagat ccccatcagc ggagaaccac 1800

ctcctaaagc catgtggagc cggggagata aggctattat ggaaggcagt ggccggataa 1860

gaacagaatc ttaccctgat agcagcactc tggtcattga tatagctgaa agagatgact 1920

ctggtgttta ccacatcaat ctgaaaaacg aagctggaga ggcacatgca agcatcaagg 1980

ttaaagttgt ggacttccct gatcctccag tggcaccgac tgtgacagag gtgggagatg 2040

actggtgtat catgaactgg gagcctcctg cctacgacgg aggctctcca atcctaggat 2100

attttattga gaggaagaag aaacaaagct ccaggtggat gaggctgaat tttgatctct 2160

gcaaagaaac aacttttgag cccaagaaga tgattgaagg tgtggcctat gaggtccgca 2220

tctttgcagt caatgccatt ggcatctcca agcccagtat gccctccagg ccttttgttc 2280

ctttggctgt aacaagccct cctactcttc tgactgtgga ctctgtcact gacacgactg 2340
tcacgatgag gtggcgcccc ccagaccaca ttggtgcagc aggtttagat ggctatgtgc 2400
tagagtattg ctttgaagga actgaggact ggatagttgc aaacaaagat ctgattgaca 2460
agacgaagtt caccatcaca ggtctgccaa cagatgcaaa gatctttgtg cgtgtgaagg 2520
ctgttaatgc agctggtgcc agcgagccca agtactattc tcagcccatt ctcgtgaagg 2580
aaatcataga acctccaaag attcgcattc caagacacct gaagcaaacc tatatccgca 2640
gagttggaga agctgtcaat ctggttatac ctttccaggg aaaaccaaga ccagaattaa 2700
cttggaagaa ggatggtgca gaaattgata agaatcaaat aaacattcgc aactctgaga 2760
ctgatacaat catatttatt agaaaagcag agaggagcca ctctgggaaa tatgatctgc 2820
aagtcaaagt ggacaaattc gtggagaccg catcaattga catccagatc attgaccgtc 2880
caggtccacc ccaaattgtg aagattgagg atgtctgggg agaaaatgtc gctctcacat 2940
ggactccacc aaaggatgat ggaaatgctg ctatcacagg ctataccatt cagaaggctg 3000
acaagaagag catggaatgg tttactgtca ttgagcatta tcatcgaacc agtgccacca 3060
ttactgaatt ggtcataggg aatgaatatt acttccgggt cttttctgaa aacatgtgtg 3120
gcctcagtga ggatgccacc atgactaaag agagtgcagt gatcgccagg gatggtaaaa 3180
tctacaaaaa tccagtgtat gaagactttg atttctcaga ggcacccatg tttactcagc 3240
ctttggttaa cacctatgcc atagctggtt acaatgccac cctaaactgc agtgtgagag 3300
gaaatcctaa gcctaaaata acctggatga aaaacaaagt tgctattgtg gatgatccaa 3360
gatacaggat gttcagcaac cagggagtct gtaccctgga aattcgcaag cccagcccct 3420
atgatggagg cacttactgc tgcaaagcag tcaatgacct tgggacagtg gagattgaat 3480
gcaaactgga ggtgaaagtc attgcacaat aaggattttt gaatgtataa tatcatctaa 3540
ggtgggctct ccttctgcag actcctcttg caaggcgtac ctccaaacat aattgattcg 3600
tatctgcgag acttacactc aagcaatcct gaggaatact gagggagggc ctggctactg 3660
tctctctgca ctctgctgct ttgaaatctg gttgaaatga gaaaaagcat tttctgtttt 3720
cccaccaggc ccccaagtgt ggtctttttc tttcctccta atgttgaaga gaaaaaaaaa 3780
aaaaaaagtt tgcccagatt gcttaattaa aaattgcaaa caaaatctca tttgaagtca 3840
aaaaaaaaaa aaaaaaaaaa 3860

<210> 74
<211> 1173
<212> PRT
<213> Homo sapiens

<400> 74

```
Met Pro Glu Pro Thr Lys Lys Glu Glu Asn Glu Val Pro Ala Pro Ala
1               5                   10                  15

Pro Pro Pro Glu Glu Pro Ser Lys Glu Lys Glu Ala Gly Thr Thr Pro
            20                  25                  30

Ala Lys Asp Trp Thr Leu Val Glu Thr Pro Pro Gly Glu Glu Gln Ala
        35                  40                  45

Lys Gln Asn Ala Asn Ser Gln Leu Ser Ile Leu Phe Ile Glu Lys Pro
    50                  55                  60

Gln Gly Gly Thr Val Lys Val Gly Glu Asp Ile Thr Phe Ile Ala Lys
65                  70                  75                  80

Val Lys Ala Glu Asp Leu Leu Arg Lys Pro Thr Ile Lys Trp Phe Lys
                85                  90                  95

Gly Lys Trp Met Asp Leu Ala Ser Lys Ala Gly Lys His Leu Gln Leu
            100                 105                 110

Lys Glu Thr Phe Glu Arg His Ser Arg Val Tyr Thr Phe Glu Met Gln
        115                 120                 125

Ile Ile Lys Ala Lys Asp Asn Phe Ala Gly Asn Tyr Arg Cys Glu Val
    130                 135                 140

Thr Tyr Lys Asp Lys Phe Asp Ser Cys Ser Phe Asp Leu Glu Val His
145                 150                 155                 160

Glu Ser Thr Gly Thr Thr Pro Asn Ile Asp Ile Arg Ser Ala Phe Lys
                165                 170                 175

Arg Ser Gly Glu Gly Gln Glu Asp Ala Gly Glu Leu Asp Phe Ser Gly
            180                 185                 190

Leu Leu Lys Arg Arg Glu Val Lys Gln Gln Glu Glu Glu Pro Gln Val
        195                 200                 205

Asp Val Trp Glu Leu Leu Lys Asn Ala Lys Pro Ser Glu Tyr Glu Lys
    210                 215                 220

Ile Ala Phe Gln Tyr Gly Ile Thr Asp Leu Arg Gly Met Leu Lys Arg
225                 230                 235                 240

Leu Lys Arg Met Arg Arg Glu Glu Lys Lys Ser Ala Ala Phe Ala Lys
                245                 250                 255
```

```
Ile Leu Asp Pro Ala Tyr Gln Val Asp Lys Gly Gly Arg Val Arg Phe
            260                 265                 270

Val Val Glu Leu Ala Asp Pro Lys Leu Glu Val Lys Trp Tyr Lys Asn
        275                 280                 285

Gly Gln Glu Ile Arg Pro Ser Thr Lys Tyr Ile Phe Glu His Lys Gly
    290                 295                 300

Cys Gln Arg Ile Leu Phe Ile Asn Asn Cys Gln Met Thr Asp Asp Ser
305                 310                 315                 320

Glu Tyr Tyr Val Thr Ala Gly Asp Glu Lys Cys Ser Thr Glu Leu Phe
                325                 330                 335

Val Arg Glu Pro Pro Ile Met Val Thr Lys Gln Leu Glu Asp Thr Thr
            340                 345                 350

Ala Tyr Cys Gly Glu Arg Val Glu Leu Glu Cys Glu Val Ser Glu Asp
        355                 360                 365

Asp Ala Asn Val Lys Trp Phe Lys Asn Gly Glu Glu Ile Ile Pro Gly
    370                 375                 380

Pro Lys Ser Arg Tyr Arg Ile Arg Val Glu Gly Lys Lys His Ile Leu
385                 390                 395                 400

Ile Ile Glu Gly Ala Thr Lys Ala Asp Ala Ala Glu Tyr Ser Val Met
                405                 410                 415

Thr Thr Gly Gly Gln Ser Ser Ala Lys Leu Ser Val Asp Leu Lys Pro
            420                 425                 430

Leu Lys Ile Leu Thr Pro Leu Thr Asp Gln Thr Val Asn Leu Gly Lys
        435                 440                 445

Glu Ile Cys Leu Lys Cys Glu Ile Ser Glu Asn Ile Pro Gly Lys Trp
    450                 455                 460

Thr Lys Asn Gly Leu Pro Val Gln Glu Ser Asp Arg Leu Lys Val Val
465                 470                 475                 480

His Lys Gly Arg Ile His Lys Leu Val Ile Ala Asn Ala Leu Thr Glu
                485                 490                 495

Asp Glu Gly Asp Tyr Val Phe Ala Pro Asp Ala Tyr Asn Val Thr Leu
            500                 505                 510
```

```
Pro Ala Lys Val His Val Ile Asp Pro Pro Lys Ile Ile Leu Asp Gly
        515                 520                 525

Leu Asp Ala Asp Asn Thr Val Thr Val Ile Ala Gly Asn Lys Leu Arg
    530                 535                 540

Leu Glu Ile Pro Ile Ser Gly Glu Pro Pro Pro Lys Ala Met Trp Ser
545                 550                 555                 560

Arg Gly Asp Lys Ala Ile Met Glu Gly Ser Gly Arg Ile Arg Thr Glu
                565                 570                 575

Ser Tyr Pro Asp Ser Ser Thr Leu Val Ile Asp Ile Ala Glu Arg Asp
            580                 585                 590

Asp Ser Gly Val Tyr His Ile Asn Leu Lys Asn Glu Ala Gly Glu Ala
        595                 600                 605

His Ala Ser Ile Lys Val Lys Val Val Asp Phe Pro Asp Pro Pro Val
    610                 615                 620

Ala Pro Thr Val Thr Glu Val Gly Asp Asp Trp Cys Ile Met Asn Trp
625                 630                 635                 640

Glu Pro Pro Ala Tyr Asp Gly Gly Ser Pro Ile Leu Gly Tyr Phe Ile
                645                 650                 655

Glu Arg Lys Lys Lys Gln Ser Ser Arg Trp Met Arg Leu Asn Phe Asp
            660                 665                 670

Leu Cys Lys Glu Thr Thr Phe Glu Pro Lys Lys Met Ile Glu Gly Val
        675                 680                 685

Ala Tyr Glu Val Arg Ile Phe Ala Val Asn Ala Ile Gly Ile Ser Lys
    690                 695                 700

Pro Ser Met Pro Ser Arg Pro Phe Val Pro Leu Ala Val Thr Ser Pro
705                 710                 715                 720

Pro Thr Leu Leu Thr Val Asp Ser Val Thr Asp Thr Thr Val Thr Met
                725                 730                 735

Arg Trp Arg Pro Pro Asp His Ile Gly Ala Ala Gly Leu Asp Gly Tyr
            740                 745                 750

Val Leu Glu Tyr Cys Phe Glu Gly Ser Thr Ser Ala Lys Gln Ser Asp
```

755 760 765

Glu Asn Gly Glu Ala Ala Tyr Asp Leu Pro Ala Glu Asp Trp Ile Val
770 775 780

Ala Asn Lys Asp Leu Ile Asp Lys Thr Lys Phe Thr Ile Thr Gly Leu
785 790 795 800

Pro Thr Asp Ala Lys Ile Phe Val Arg Val Lys Ala Val Asn Ala Ala
805 810 815

Gly Ala Ser Glu Pro Lys Tyr Tyr Ser Gln Pro Ile Leu Val Lys Glu
820 825 830

Ile Ile Glu Pro Pro Lys Ile Arg Ile Pro Arg His Leu Lys Gln Thr
835 840 845

Tyr Ile Arg Arg Val Gly Glu Ala Val Asn Leu Val Ile Pro Phe Gln
850 855 860

Gly Lys Pro Arg Pro Glu Leu Thr Trp Lys Lys Asp Gly Ala Glu Ile
865 870 875 880

Asp Lys Asn Gln Ile Asn Ile Arg Asn Ser Glu Thr Asp Thr Ile Ile
885 890 895

Phe Ile Arg Lys Ala Glu Arg Ser His Ser Gly Lys Tyr Asp Leu Gln
900 905 910

Val Lys Val Asp Lys Phe Val Glu Thr Ala Ser Ile Asp Ile Gln Ile
915 920 925

Ile Asp Arg Pro Gly Pro Pro Gln Ile Val Lys Ile Glu Asp Val Trp
930 935 940

Gly Glu Asn Val Ala Leu Thr Trp Thr Pro Pro Lys Asp Asp Gly Asn
945 950 955 960

Ala Ala Ile Thr Gly Tyr Thr Ile Gln Lys Ala Asp Lys Lys Ser Met
965 970 975

Glu Trp Phe Thr Val Ile Glu His Tyr His Arg Thr Ser Ala Thr Ile
980 985 990

Thr Glu Leu Val Ile Gly Asn Glu Tyr Tyr Phe Arg Val Phe Ser Glu
995 1000 1005

Asn Met Cys Gly Leu Ser Glu Asp Ala Thr Met Thr Lys Glu Ser
1010 1015 1020

Ala Val Ile Ala Arg Asp Gly Lys Ile Tyr Lys Asn Pro Val Tyr
1025 1030 1035

Glu Asp Phe Asp Phe Ser Glu Ala Pro Met Phe Thr Gln Pro Leu
1040 1045 1050

Val Asn Thr Tyr Ala Ile Ala Gly Tyr Asn Ala Thr Leu Asn Cys
1055 1060 1065

Ser Val Arg Gly Asn Pro Lys Pro Lys Ile Thr Trp Met Lys Asn
1070 1075 1080

Lys Val Ala Ile Val Asp Asp Pro Arg Tyr Arg Met Phe Ser Asn
1085 1090 1095

Gln Gly Val Cys Thr Leu Glu Ile Arg Lys Pro Ser Pro Tyr Asp
1100 1105 1110

Gly Gly Thr Tyr Cys Cys Lys Ala Val Asn Asp Leu Gly Thr Val
1115 1120 1125

Glu Ile Glu Cys Lys Leu Glu Val Lys Val Ile Tyr Gln Gly Val
1130 1135 1140

Asn Thr Pro Gly Gln Pro Val Phe Leu Glu Gly Gln Gln Gln Val
1145 1150 1155

Gly Ser Pro Ser Ala Asp Ser Ser Cys Lys Ala Tyr Leu Gln Thr
1160 1165 1170

<210> 75
<211> 1146
<212> PRT
<213> Homo sapiens

<400> 75

Met Pro Glu Pro Thr Lys Lys Glu Glu Asn Glu Val Pro Ala Pro Ala
1 5 10 15

Pro Pro Pro Glu Glu Pro Ser Lys Glu Lys Glu Ala Gly Thr Thr Pro
20 25 30

Ala Lys Asp Trp Thr Leu Val Glu Thr Pro Pro Gly Glu Glu Gln Ala
35 40 45

```
Lys Gln Asn Ala Asn Ser Gln Leu Ser Ile Leu Phe Ile Glu Lys Pro
    50                  55                  60

Gln Gly Gly Thr Val Lys Val Gly Glu Asp Ile Thr Phe Ile Ala Lys
65                  70                  75                  80

Val Lys Ala Glu Asp Leu Leu Arg Lys Pro Thr Ile Lys Trp Phe Lys
                85                  90                  95

Gly Lys Trp Met Asp Leu Ala Ser Lys Ala Gly Lys His Leu Gln Leu
            100                 105                 110

Lys Glu Thr Phe Glu Arg His Ser Arg Val Tyr Thr Phe Glu Met Gln
        115                 120                 125

Ile Ile Lys Ala Lys Asp Asn Phe Ala Gly Asn Tyr Arg Cys Glu Val
    130                 135                 140

Thr Tyr Lys Asp Lys Phe Asp Ser Cys Ser Phe Asp Leu Glu Val His
145                 150                 155                 160

Glu Ser Thr Gly Thr Thr Pro Asn Ile Asp Ile Arg Ser Ala Phe Lys
                165                 170                 175

Arg Ser Gly Glu Gly Gln Glu Asp Ala Gly Glu Leu Asp Phe Ser Gly
            180                 185                 190

Leu Leu Lys Arg Arg Glu Val Lys Gln Gln Glu Glu Glu Pro Gln Val
        195                 200                 205

Asp Val Trp Glu Leu Leu Lys Asn Ala Lys Pro Ser Glu Tyr Glu Lys
    210                 215                 220

Ile Ala Phe Gln Tyr Gly Ile Thr Asp Leu Arg Gly Met Leu Lys Arg
225                 230                 235                 240

Leu Lys Arg Met Arg Arg Glu Glu Lys Lys Ser Ala Ala Phe Ala Lys
                245                 250                 255

Ile Leu Asp Pro Ala Tyr Gln Val Asp Lys Gly Gly Arg Val Arg Phe
            260                 265                 270

Val Val Glu Leu Ala Asp Pro Lys Leu Glu Val Lys Trp Tyr Lys Asn
        275                 280                 285

Gly Gln Glu Ile Arg Pro Ser Thr Lys Tyr Ile Phe Glu His Lys Gly
    290                 295                 300
```

Cys Gln Arg Ile Leu Phe Ile Asn Asn Cys Gln Met Thr Asp Asp Ser
305 310 315 320

Glu Tyr Tyr Val Thr Ala Gly Asp Glu Lys Cys Ser Thr Glu Leu Phe
325 330 335

Val Arg Glu Pro Pro Ile Met Val Thr Lys Gln Leu Glu Asp Thr Thr
340 345 350

Ala Tyr Cys Gly Glu Arg Val Glu Leu Glu Cys Glu Val Ser Glu Asp
355 360 365

Asp Ala Asn Val Lys Trp Phe Lys Asn Gly Glu Glu Ile Ile Pro Gly
370 375 380

Pro Lys Ser Arg Tyr Arg Ile Arg Val Glu Gly Lys Lys His Ile Leu
385 390 395 400

Ile Ile Glu Gly Ala Thr Lys Ala Asp Ala Ala Glu Tyr Ser Val Met
405 410 415

Thr Thr Gly Gly Gln Ser Ser Ala Lys Leu Ser Val Asp Leu Lys Pro
420 425 430

Leu Lys Ile Leu Thr Pro Leu Thr Asp Gln Thr Val Asn Leu Gly Lys
435 440 445

Glu Ile Cys Leu Lys Cys Glu Ile Ser Glu Asn Ile Pro Gly Lys Trp
450 455 460

Thr Lys Asn Gly Leu Pro Val Gln Glu Ser Asp Arg Leu Lys Val Val
465 470 475 480

His Lys Gly Arg Ile His Lys Leu Val Ile Ala Asn Ala Leu Thr Glu
485 490 495

Asp Glu Gly Asp Tyr Val Phe Ala Pro Asp Ala Tyr Asn Val Thr Leu
500 505 510

Pro Ala Lys Val His Val Ile Asp Pro Pro Lys Ile Ile Leu Asp Gly
515 520 525

Leu Asp Ala Asp Asn Thr Val Thr Val Ile Ala Gly Asn Lys Leu Arg
530 535 540

Leu Glu Ile Pro Ile Ser Gly Glu Pro Pro Pro Lys Ala Met Trp Ser
545 550 555 560

Arg Gly Asp Lys Ala Ile Met Glu Gly Ser Gly Arg Ile Arg Thr Glu
565 570 575

Ser Tyr Pro Asp Ser Ser Thr Leu Val Ile Asp Ile Ala Glu Arg Asp
580 585 590

Asp Ser Gly Val Tyr His Ile Asn Leu Lys Asn Glu Ala Gly Glu Ala
595 600 605

His Ala Ser Ile Lys Val Lys Val Val Asp Phe Pro Asp Pro Pro Val
610 615 620

Ala Pro Thr Val Thr Glu Val Gly Asp Asp Trp Cys Ile Met Asn Trp
625 630 635 640

Glu Pro Pro Ala Tyr Asp Gly Gly Ser Pro Ile Leu Gly Tyr Phe Ile
645 650 655

Glu Arg Lys Lys Lys Gln Ser Ser Arg Trp Met Arg Leu Asn Phe Asp
660 665 670

Leu Cys Lys Glu Thr Thr Phe Glu Pro Lys Lys Met Ile Glu Gly Val
675 680 685

Ala Tyr Glu Val Arg Ile Phe Ala Val Asn Ala Ile Gly Ile Ser Lys
690 695 700

Pro Ser Met Pro Ser Arg Pro Phe Val Pro Leu Ala Val Thr Ser Pro
705 710 715 720

Pro Thr Leu Leu Thr Val Asp Ser Val Thr Asp Thr Thr Val Thr Met
725 730 735

Arg Trp Arg Pro Pro Asp His Ile Gly Ala Ala Gly Leu Asp Gly Tyr
740 745 750

Val Leu Glu Tyr Cys Phe Glu Gly Thr Glu Asp Trp Ile Val Ala Asn
755 760 765

Lys Asp Leu Ile Asp Lys Thr Lys Phe Thr Ile Thr Gly Leu Pro Thr
770 775 780

Asp Ala Lys Ile Phe Val Arg Val Lys Ala Val Asn Ala Ala Gly Ala
785 790 795 800

Ser Glu Pro Lys Tyr Tyr Ser Gln Pro Ile Leu Val Lys Glu Ile Ile

805 810 815

Glu Pro Pro Lys Ile Arg Ile Pro Arg His Leu Lys Gln Thr Tyr Ile
820 825 830

Arg Arg Val Gly Glu Ala Val Asn Leu Val Ile Pro Phe Gln Gly Lys
835 840 845

Pro Arg Pro Glu Leu Thr Trp Lys Lys Asp Gly Ala Glu Ile Asp Lys
850 855 860

Asn Gln Ile Asn Ile Arg Asn Ser Glu Thr Asp Thr Ile Ile Phe Ile
865 870 875 880

Arg Lys Ala Glu Arg Ser His Ser Gly Lys Tyr Asp Leu Gln Val Lys
885 890 895

Val Asp Lys Phe Val Glu Thr Ala Ser Ile Asp Ile Gln Ile Ile Asp
900 905 910

Arg Pro Gly Pro Pro Gln Ile Val Lys Ile Glu Asp Val Trp Gly Glu
915 920 925

Asn Val Ala Leu Thr Trp Thr Pro Pro Lys Asp Asp Gly Asn Ala Ala
930 935 940

Ile Thr Gly Tyr Thr Ile Gln Lys Ala Asp Lys Lys Ser Met Glu Trp
945 950 955 960

Phe Thr Val Ile Glu His Tyr His Arg Thr Ser Ala Thr Ile Thr Glu
965 970 975

Leu Val Ile Gly Asn Glu Tyr Tyr Phe Arg Val Phe Ser Glu Asn Met
980 985 990

Cys Gly Leu Ser Glu Asp Ala Thr Met Thr Lys Glu Ser Ala Val Ile
995 1000 1005

Ala Arg Asp Gly Lys Ile Tyr Lys Asn Pro Val Tyr Glu Asp Phe
1010 1015 1020

Asp Phe Ser Glu Ala Pro Met Phe Thr Gln Pro Leu Val Asn Thr
1025 1030 1035

Tyr Ala Ile Ala Gly Tyr Asn Ala Thr Leu Asn Cys Ser Val Arg
1040 1045 1050

```
Gly Asn  Pro Lys Pro Lys Ile  Thr Trp Met Lys Asn  Lys Val Ala
    1055                 1060                 1065

Ile Val  Asp Asp Pro Arg Tyr  Arg Met Phe Ser Asn  Gln Gly Val
    1070                 1075                 1080

Cys Thr  Leu Glu Ile Arg Lys  Pro Ser Pro Tyr Asp  Gly Gly Thr
    1085                 1090                 1095

Tyr Cys  Cys Lys Ala Val Asn  Asp Leu Gly Thr Val  Glu Ile Glu
    1100                 1105                 1110

Cys Lys  Leu Glu Val Lys Val  Ile Tyr Gln Gly Val  Asn Thr Pro
    1115                 1120                 1125

Gly Gln  Pro Val Phe Leu Glu  Gly Gln Gln Gln Ser  Leu His Asn
    1130                 1135                 1140

Lys Asp  Phe
    1145
```

<210> 76
<211> 1134
<212> PRT
<213> Homo sapiens

<400> 76

```
Met Pro Glu Pro Thr Lys Lys Glu Glu Pro Ser Lys Glu Lys Glu Ala
1               5                   10                  15

Gly Thr Thr Pro Ala Lys Asp Trp Thr Leu Val Glu Thr Pro Pro Gly
            20                  25                  30

Glu Glu Gln Ala Lys Gln Asn Ala Asn Ser Gln Leu Ser Ile Leu Phe
        35                  40                  45

Ile Glu Lys Pro Gln Gly Gly Thr Val Lys Val Gly Glu Asp Ile Thr
    50                  55                  60

Phe Ile Ala Lys Val Lys Ala Glu Asp Leu Leu Arg Lys Pro Thr Ile
65                  70                  75                  80

Lys Trp Phe Lys Gly Lys Trp Met Asp Leu Ala Ser Lys Ala Gly Lys
                85                  90                  95

His Leu Gln Leu Lys Glu Thr Phe Glu Arg His Ser Arg Val Tyr Thr
            100                 105                 110
```

Phe Glu Met Gln Ile Ile Lys Ala Lys Asp Asn Phe Ala Gly Asn Tyr
115 120 125

Arg Cys Glu Val Thr Tyr Lys Asp Lys Phe Asp Ser Cys Ser Phe Asp
130 135 140

Leu Glu Val His Glu Ser Thr Gly Thr Thr Pro Asn Ile Asp Ile Arg
145 150 155 160

Ser Ala Phe Lys Arg Ser Gly Glu Gly Gln Glu Asp Ala Gly Glu Leu
165 170 175

Asp Phe Ser Gly Leu Leu Lys Arg Arg Glu Val Lys Gln Gln Glu Glu
180 185 190

Glu Pro Gln Val Asp Val Trp Glu Leu Leu Lys Asn Ala Lys Pro Ser
195 200 205

Glu Tyr Glu Lys Ile Ala Phe Gln Tyr Gly Ile Thr Asp Leu Arg Gly
210 215 220

Met Leu Lys Arg Leu Lys Arg Met Arg Arg Glu Glu Lys Lys Ser Ala
225 230 235 240

Ala Phe Ala Lys Ile Leu Asp Pro Ala Tyr Gln Val Asp Lys Gly Gly
245 250 255

Arg Val Arg Phe Val Val Glu Leu Ala Asp Pro Lys Leu Glu Val Lys
260 265 270

Trp Tyr Lys Asn Gly Gln Glu Ile Arg Pro Ser Thr Lys Tyr Ile Phe
275 280 285

Glu His Lys Gly Cys Gln Arg Ile Leu Phe Ile Asn Asn Cys Gln Met
290 295 300

Thr Asp Asp Ser Glu Tyr Tyr Val Thr Ala Gly Asp Glu Lys Cys Ser
305 310 315 320

Thr Glu Leu Phe Val Arg Glu Pro Pro Ile Met Val Thr Lys Gln Leu
325 330 335

Glu Asp Thr Thr Ala Tyr Cys Gly Glu Arg Val Glu Leu Glu Cys Glu
340 345 350

Val Ser Glu Asp Asp Ala Asn Val Lys Trp Phe Lys Asn Gly Glu Glu
355 360 365

```
Ile Ile Pro Gly Pro Lys Ser Arg Tyr Arg Ile Arg Val Glu Gly Lys
    370                 375                 380

Lys His Ile Leu Ile Ile Glu Gly Ala Thr Lys Ala Asp Ala Ala Glu
385                 390                 395                 400

Tyr Ser Val Met Thr Thr Gly Gly Gln Ser Ser Ala Lys Leu Ser Val
                405                 410                 415

Asp Leu Lys Pro Leu Lys Ile Leu Thr Pro Leu Thr Asp Gln Thr Val
            420                 425                 430

Asn Leu Gly Lys Glu Ile Cys Leu Lys Cys Glu Ile Ser Glu Asn Ile
        435                 440                 445

Pro Gly Lys Trp Thr Lys Asn Gly Leu Pro Val Gln Glu Ser Asp Arg
    450                 455                 460

Leu Lys Val Val His Lys Gly Arg Ile His Lys Leu Val Ile Ala Asn
465                 470                 475                 480

Ala Leu Thr Glu Asp Glu Gly Asp Tyr Val Phe Ala Pro Asp Ala Tyr
                485                 490                 495

Asn Val Thr Leu Pro Ala Lys Val His Val Ile Asp Pro Pro Lys Ile
            500                 505                 510

Ile Leu Asp Gly Leu Asp Ala Asp Asn Thr Val Thr Val Ile Ala Gly
        515                 520                 525

Asn Lys Leu Arg Leu Glu Ile Pro Ile Ser Gly Glu Pro Pro Pro Lys
    530                 535                 540

Ala Met Trp Ser Arg Gly Asp Lys Ala Ile Met Glu Gly Ser Gly Arg
545                 550                 555                 560

Ile Arg Thr Glu Ser Tyr Pro Asp Ser Ser Thr Leu Val Ile Asp Ile
                565                 570                 575

Ala Glu Arg Asp Asp Ser Gly Val Tyr His Ile Asn Leu Lys Asn Glu
            580                 585                 590

Ala Gly Glu Ala His Ala Ser Ile Lys Val Lys Val Val Asp Phe Pro
        595                 600                 605

Asp Pro Pro Val Ala Pro Thr Val Thr Glu Val Gly Asp Asp Trp Cys
    610                 615                 620
```

Ile Met Asn Trp Glu Pro Pro Ala Tyr Asp Gly Gly Ser Pro Ile Leu
625 630 635 640

Gly Tyr Phe Ile Glu Arg Lys Lys Lys Gln Ser Ser Arg Trp Met Arg
645 650 655

Leu Asn Phe Asp Leu Cys Lys Glu Thr Thr Phe Glu Pro Lys Lys Met
660 665 670

Ile Glu Gly Val Ala Tyr Glu Val Arg Ile Phe Ala Val Asn Ala Ile
675 680 685

Gly Ile Ser Lys Pro Ser Met Pro Ser Arg Pro Phe Val Pro Leu Ala
690 695 700

Val Thr Ser Pro Pro Thr Leu Leu Thr Val Asp Ser Val Thr Asp Thr
705 710 715 720

Thr Val Thr Met Arg Trp Arg Pro Pro Asp His Ile Gly Ala Ala Gly
725 730 735

Leu Asp Gly Tyr Val Leu Glu Tyr Cys Phe Glu Gly Thr Glu Asp Trp
740 745 750

Ile Val Ala Asn Lys Asp Leu Ile Asp Lys Thr Lys Phe Thr Ile Thr
755 760 765

Gly Leu Pro Thr Asp Ala Lys Ile Phe Val Arg Val Lys Ala Val Asn
770 775 780

Ala Ala Gly Ala Ser Glu Pro Lys Tyr Tyr Ser Gln Pro Ile Leu Val
785 790 795 800

Lys Glu Ile Ile Glu Pro Pro Lys Ile Arg Ile Pro Arg His Leu Lys
805 810 815

Gln Thr Tyr Ile Arg Arg Val Gly Glu Ala Val Asn Leu Val Ile Pro
820 825 830

Phe Gln Gly Lys Pro Arg Pro Glu Leu Thr Trp Lys Lys Asp Gly Ala
835 840 845

Glu Ile Asp Lys Asn Gln Ile Asn Ile Arg Asn Ser Glu Thr Asp Thr
850 855 860

Ile Ile Phe Ile Arg Lys Ala Glu Arg Ser His Ser Gly Lys Tyr Asp

865 870 875 880

Leu Gln Val Lys Val Asp Lys Phe Val Glu Thr Ala Ser Ile Asp Ile
885 890 895

Gln Ile Ile Asp Arg Pro Gly Pro Pro Gln Ile Val Lys Ile Glu Asp
900 905 910

Val Trp Gly Glu Asn Val Ala Leu Thr Trp Thr Pro Pro Lys Asp Asp
915 920 925

Gly Asn Ala Ala Ile Thr Gly Tyr Thr Ile Gln Lys Ala Asp Lys Lys
930 935 940

Ser Met Glu Trp Phe Thr Val Ile Glu His Tyr His Arg Thr Ser Ala
945 950 955 960

Thr Ile Thr Glu Leu Val Ile Gly Asn Glu Tyr Tyr Phe Arg Val Phe
965 970 975

Ser Glu Asn Met Cys Gly Leu Ser Glu Asp Ala Thr Met Thr Lys Glu
980 985 990

Ser Ala Val Ile Ala Arg Asp Gly Lys Ile Tyr Lys Asn Pro Val Tyr
995 1000 1005

Glu Asp Phe Asp Phe Ser Glu Ala Pro Met Phe Thr Gln Pro Leu
1010 1015 1020

Val Asn Thr Tyr Ala Ile Ala Gly Tyr Asn Ala Thr Leu Asn Cys
1025 1030 1035

Ser Val Arg Gly Asn Pro Lys Pro Lys Ile Thr Trp Met Lys Asn
1040 1045 1050

Lys Val Ala Ile Val Asp Asp Pro Arg Tyr Arg Met Phe Ser Asn
1055 1060 1065

Gln Gly Val Cys Thr Leu Glu Ile Arg Lys Pro Ser Pro Tyr Asp
1070 1075 1080

Gly Gly Thr Tyr Cys Cys Lys Ala Val Asn Asp Leu Gly Thr Val
1085 1090 1095

Glu Ile Glu Cys Lys Leu Glu Val Lys Val Ile Tyr Gln Gly Val
1100 1105 1110

```
Asn Thr  Pro Gly Gln Pro Val  Phe Leu Glu Gly Gln  Gln Gln Ser
    1115                 1120                 1125

Leu His  Asn Lys Asp Phe
    1130
```

<210> 77
<211> 1127
<212> PRT
<213> Homo sapiens

<400> 77

```
Met Pro Glu Pro Thr Lys Lys Glu Glu Asn Glu Val Pro Ala Pro Ala
1               5                   10                  15

Pro Pro Pro Glu Glu Pro Ser Lys Glu Lys Glu Ala Gly Thr Thr Pro
            20                  25                  30

Ala Lys Asp Trp Thr Leu Val Glu Thr Pro Pro Gly Glu Glu Gln Ala
        35                  40                  45

Lys Gln Asn Ala Asn Ser Gln Leu Ser Ile Leu Phe Ile Glu Lys Pro
    50                  55                  60

Gln Gly Gly Thr Val Lys Val Gly Glu Asp Ile Thr Phe Ile Ala Lys
65                  70                  75                  80

Val Lys Ala Glu Asp Leu Leu Arg Lys Pro Thr Ile Lys Trp Phe Lys
                85                  90                  95

Gly Lys Trp Met Asp Leu Ala Ser Lys Ala Gly Lys His Leu Gln Leu
            100                 105                 110

Lys Glu Thr Phe Glu Arg His Ser Arg Val Tyr Thr Phe Glu Met Gln
        115                 120                 125

Ile Ile Lys Ala Lys Asp Asn Phe Ala Gly Asn Tyr Arg Cys Glu Val
    130                 135                 140

Thr Tyr Lys Asp Lys Phe Asp Ser Cys Ser Phe Asp Leu Glu Val His
145                 150                 155                 160

Glu Ser Thr Gly Thr Thr Pro Asn Ile Asp Ile Arg Ser Ala Phe Lys
                165                 170                 175

Arg Arg Glu Val Lys Gln Gln Glu Glu Glu Pro Gln Val Asp Val Trp
            180                 185                 190
```

Glu Leu Leu Lys Asn Ala Lys Pro Ser Glu Tyr Glu Lys Ile Ala Phe
195 200 205

Gln Tyr Gly Ile Thr Asp Leu Arg Gly Met Leu Lys Arg Leu Lys Arg
210 215 220

Met Arg Arg Glu Glu Lys Lys Ser Ala Ala Phe Ala Lys Ile Leu Asp
225 230 235 240

Pro Ala Tyr Gln Val Asp Lys Gly Gly Arg Val Arg Phe Val Val Glu
245 250 255

Leu Ala Asp Pro Lys Leu Glu Val Lys Trp Tyr Lys Asn Gly Gln Glu
260 265 270

Ile Arg Pro Ser Thr Lys Tyr Ile Phe Glu His Lys Gly Cys Gln Arg
275 280 285

Ile Leu Phe Ile Asn Asn Cys Gln Met Thr Asp Asp Ser Glu Tyr Tyr
290 295 300

Val Thr Ala Gly Asp Glu Lys Cys Ser Thr Glu Leu Phe Val Arg Glu
305 310 315 320

Pro Pro Ile Met Val Thr Lys Gln Leu Glu Asp Thr Thr Ala Tyr Cys
325 330 335

Gly Glu Arg Val Glu Leu Glu Cys Glu Val Ser Glu Asp Asp Ala Asn
340 345 350

Val Lys Trp Phe Lys Asn Gly Glu Glu Ile Ile Pro Gly Pro Lys Ser
355 360 365

Arg Tyr Arg Ile Arg Val Glu Gly Lys Lys His Ile Leu Ile Ile Glu
370 375 380

Gly Ala Thr Lys Ala Asp Ala Ala Glu Tyr Ser Val Met Thr Thr Gly
385 390 395 400

Gly Gln Ser Ser Ala Lys Leu Ser Val Asp Leu Lys Pro Leu Lys Ile
405 410 415

Leu Thr Pro Leu Thr Asp Gln Thr Val Asn Leu Gly Lys Glu Ile Cys
420 425 430

Leu Lys Cys Glu Ile Ser Glu Asn Ile Pro Gly Lys Trp Thr Lys Asn
435 440 445

Gly Leu Pro Val Gln Glu Ser Asp Arg Leu Lys Val Val His Lys Gly
450 455 460

Arg Ile His Lys Leu Val Ile Ala Asn Ala Leu Thr Glu Asp Glu Gly
465 470 475 480

Asp Tyr Val Phe Ala Pro Asp Ala Tyr Asn Val Thr Leu Pro Ala Lys
485 490 495

Val His Val Ile Asp Pro Pro Lys Ile Ile Leu Asp Gly Leu Asp Ala
500 505 510

Asp Asn Thr Val Thr Val Ile Ala Gly Asn Lys Leu Arg Leu Glu Ile
515 520 525

Pro Ile Ser Gly Glu Pro Pro Pro Lys Ala Met Trp Ser Arg Gly Asp
530 535 540

Lys Ala Ile Met Glu Gly Ser Gly Arg Ile Arg Thr Glu Ser Tyr Pro
545 550 555 560

Asp Ser Ser Thr Leu Val Ile Asp Ile Ala Glu Arg Asp Asp Ser Gly
565 570 575

Val Tyr His Ile Asn Leu Lys Asn Glu Ala Gly Glu Ala His Ala Ser
580 585 590

Ile Lys Val Lys Val Val Asp Phe Pro Asp Pro Pro Val Ala Pro Thr
595 600 605

Val Thr Glu Val Gly Asp Asp Trp Cys Ile Met Asn Trp Glu Pro Pro
610 615 620

Ala Tyr Asp Gly Gly Ser Pro Ile Leu Gly Tyr Phe Ile Glu Arg Lys
625 630 635 640

Lys Lys Gln Ser Ser Arg Trp Met Arg Leu Asn Phe Asp Leu Cys Lys
645 650 655

Glu Thr Thr Phe Glu Pro Lys Lys Met Ile Glu Gly Val Ala Tyr Glu
660 665 670

Val Arg Ile Phe Ala Val Asn Ala Ile Gly Ile Ser Lys Pro Ser Met
675 680 685

Pro Ser Arg Pro Phe Val Pro Leu Ala Val Thr Ser Pro Pro Thr Leu
690 695 700

```
Leu Thr Val Asp Ser Val Thr Asp Thr Thr Val Thr Met Arg Trp Arg
705                 710                 715                 720

Pro Pro Asp His Ile Gly Ala Ala Gly Leu Asp Gly Tyr Val Leu Glu
                725                 730                 735

Tyr Cys Phe Glu Gly Thr Glu Asp Trp Ile Val Ala Asn Lys Asp Leu
            740                 745                 750

Ile Asp Lys Thr Lys Phe Thr Ile Thr Gly Leu Pro Thr Asp Ala Lys
        755                 760                 765

Ile Phe Val Arg Val Lys Ala Val Asn Ala Ala Gly Ala Ser Glu Pro
    770                 775                 780

Lys Tyr Tyr Ser Gln Pro Ile Leu Val Lys Glu Ile Ile Glu Pro Pro
785                 790                 795                 800

Lys Ile Arg Ile Pro Arg His Leu Lys Gln Thr Tyr Ile Arg Arg Val
                805                 810                 815

Gly Glu Ala Val Asn Leu Val Ile Pro Phe Gln Gly Lys Pro Arg Pro
            820                 825                 830

Glu Leu Thr Trp Lys Lys Asp Gly Ala Glu Ile Asp Lys Asn Gln Ile
        835                 840                 845

Asn Ile Arg Asn Ser Glu Thr Asp Thr Ile Ile Phe Ile Arg Lys Ala
    850                 855                 860

Glu Arg Ser His Ser Gly Lys Tyr Asp Leu Gln Val Lys Val Asp Lys
865                 870                 875                 880

Phe Val Glu Thr Ala Ser Ile Asp Ile Gln Ile Ile Asp Arg Pro Gly
                885                 890                 895

Pro Pro Gln Ile Val Lys Ile Glu Asp Val Trp Gly Glu Asn Val Ala
            900                 905                 910

Leu Thr Trp Thr Pro Pro Lys Asp Asp Gly Asn Ala Ala Ile Thr Gly
        915                 920                 925

Tyr Thr Ile Gln Lys Ala Asp Lys Lys Ser Met Glu Trp Phe Thr Val
    930                 935                 940

Ile Glu His Tyr His Arg Thr Ser Ala Thr Ile Thr Glu Leu Val Ile
```

945 950 955 960

Gly Asn Glu Tyr Tyr Phe Arg Val Phe Ser Glu Asn Met Cys Gly Leu
965 970 975

Ser Glu Asp Ala Thr Met Thr Lys Glu Ser Ala Val Ile Ala Arg Asp
980 985 990

Gly Lys Ile Tyr Lys Asn Pro Val Tyr Glu Asp Phe Asp Phe Ser Glu
995 1000 1005

Ala Pro Met Phe Thr Gln Pro Leu Val Asn Thr Tyr Ala Ile Ala
1010 1015 1020

Gly Tyr Asn Ala Thr Leu Asn Cys Ser Val Arg Gly Asn Pro Lys
1025 1030 1035

Pro Lys Ile Thr Trp Met Lys Asn Lys Val Ala Ile Val Asp Asp
1040 1045 1050

Pro Arg Tyr Arg Met Phe Ser Asn Gln Gly Val Cys Thr Leu Glu
1055 1060 1065

Ile Arg Lys Pro Ser Pro Tyr Asp Gly Gly Thr Tyr Cys Cys Lys
1070 1075 1080

Ala Val Asn Asp Leu Gly Thr Val Glu Ile Glu Cys Lys Leu Glu
1085 1090 1095

Val Lys Val Ile Tyr Gln Gly Val Asn Thr Pro Gly Gln Pro Val
1100 1105 1110

Phe Leu Glu Gly Gln Gln Gln Ser Leu His Asn Lys Asp Phe
1115 1120 1125

<210> 78
<211> 1120
<212> PRT
<213> Homo sapiens

<400> 78

```
Met Pro Glu Pro Thr Lys Lys Glu Asp Trp Thr Leu Val Glu Thr Pro
1               5                   10                  15

Pro Gly Glu Glu Gln Ala Lys Gln Asn Ala Asn Ser Gln Leu Ser Ile
            20                  25                  30

Leu Phe Ile Glu Lys Pro Gln Gly Gly Thr Val Lys Val Gly Glu Asp
```

```
        35                  40                  45

Ile Thr Phe Ile Ala Lys Val Lys Ala Glu Asp Leu Leu Arg Lys Pro
    50                  55                  60

Thr Ile Lys Trp Phe Lys Gly Lys Trp Met Asp Leu Ala Ser Lys Ala
65                  70                  75                  80

Gly Lys His Leu Gln Leu Lys Glu Thr Phe Glu Arg His Ser Arg Val
                85                  90                  95

Tyr Thr Phe Glu Met Gln Ile Ile Lys Ala Lys Asp Asn Phe Ala Gly
            100                 105                 110

Asn Tyr Arg Cys Glu Val Thr Tyr Lys Asp Lys Phe Asp Ser Cys Ser
        115                 120                 125

Phe Asp Leu Glu Val His Glu Ser Thr Gly Thr Thr Pro Asn Ile Asp
    130                 135                 140

Ile Arg Ser Ala Phe Lys Arg Ser Gly Glu Gly Gln Glu Asp Ala Gly
145                 150                 155                 160

Glu Leu Asp Phe Ser Gly Leu Leu Lys Arg Arg Glu Val Lys Gln Gln
                165                 170                 175

Glu Glu Glu Pro Gln Val Asp Val Trp Glu Leu Leu Lys Asn Ala Lys
            180                 185                 190

Pro Ser Glu Tyr Glu Lys Ile Ala Phe Gln Tyr Gly Ile Thr Asp Leu
        195                 200                 205

Arg Gly Met Leu Lys Arg Leu Lys Arg Met Arg Arg Glu Glu Lys Lys
    210                 215                 220

Ser Ala Ala Phe Ala Lys Ile Leu Asp Pro Ala Tyr Gln Val Asp Lys
225                 230                 235                 240

Gly Gly Arg Val Arg Phe Val Val Glu Leu Ala Asp Pro Lys Leu Glu
                245                 250                 255

Val Lys Trp Tyr Lys Asn Gly Gln Glu Ile Arg Pro Ser Thr Lys Tyr
            260                 265                 270

Ile Phe Glu His Lys Gly Cys Gln Arg Ile Leu Phe Ile Asn Asn Cys
        275                 280                 285
```

```
Gln Met Thr Asp Asp Ser Glu Tyr Tyr Val Thr Ala Gly Asp Glu Lys
    290                 295                 300

Cys Ser Thr Glu Leu Phe Val Arg Glu Pro Pro Ile Met Val Thr Lys
305                 310                 315                 320

Gln Leu Glu Asp Thr Thr Ala Tyr Cys Gly Glu Arg Val Glu Leu Glu
                325                 330                 335

Cys Glu Val Ser Glu Asp Asp Ala Asn Val Lys Trp Phe Lys Asn Gly
            340                 345                 350

Glu Glu Ile Ile Pro Gly Pro Lys Ser Arg Tyr Arg Ile Arg Val Glu
        355                 360                 365

Gly Lys Lys His Ile Leu Ile Ile Glu Gly Ala Thr Lys Ala Asp Ala
    370                 375                 380

Ala Glu Tyr Ser Val Met Thr Thr Gly Gly Gln Ser Ser Ala Lys Leu
385                 390                 395                 400

Ser Val Asp Leu Lys Pro Leu Lys Ile Leu Thr Pro Leu Thr Asp Gln
                405                 410                 415

Thr Val Asn Leu Gly Lys Glu Ile Cys Leu Lys Cys Glu Ile Ser Glu
            420                 425                 430

Asn Ile Pro Gly Lys Trp Thr Lys Asn Gly Leu Pro Val Gln Glu Ser
        435                 440                 445

Asp Arg Leu Lys Val Val His Lys Gly Arg Ile His Lys Leu Val Ile
    450                 455                 460

Ala Asn Ala Leu Thr Glu Asp Glu Gly Asp Tyr Val Phe Ala Pro Asp
465                 470                 475                 480

Ala Tyr Asn Val Thr Leu Pro Ala Lys Val His Val Ile Asp Pro Pro
                485                 490                 495

Lys Ile Ile Leu Asp Gly Leu Asp Ala Asp Asn Thr Val Thr Val Ile
            500                 505                 510

Ala Gly Asn Lys Leu Arg Leu Glu Ile Pro Ile Ser Gly Glu Pro Pro
        515                 520                 525

Pro Lys Ala Met Trp Ser Arg Gly Asp Lys Ala Ile Met Glu Gly Ser
    530                 535                 540
```

Gly Arg Ile Arg Thr Glu Ser Tyr Pro Asp Ser Ser Thr Leu Val Ile
545 550 555 560

Asp Ile Ala Glu Arg Asp Asp Ser Gly Val Tyr His Ile Asn Leu Lys
565 570 575

Asn Glu Ala Gly Glu Ala His Ala Ser Ile Lys Val Lys Val Val Asp
580 585 590

Phe Pro Asp Pro Pro Val Ala Pro Thr Val Thr Glu Val Gly Asp Asp
595 600 605

Trp Cys Ile Met Asn Trp Glu Pro Pro Ala Tyr Asp Gly Gly Ser Pro
610 615 620

Ile Leu Gly Tyr Phe Ile Glu Arg Lys Lys Lys Gln Ser Ser Arg Trp
625 630 635 640

Met Arg Leu Asn Phe Asp Leu Cys Lys Glu Thr Thr Phe Glu Pro Lys
645 650 655

Lys Met Ile Glu Gly Val Ala Tyr Glu Val Arg Ile Phe Ala Val Asn
660 665 670

Ala Ile Gly Ile Ser Lys Pro Ser Met Pro Ser Arg Pro Phe Val Pro
675 680 685

Leu Ala Val Thr Ser Pro Pro Thr Leu Leu Thr Val Asp Ser Val Thr
690 695 700

Asp Thr Thr Val Thr Met Arg Trp Arg Pro Pro Asp His Ile Gly Ala
705 710 715 720

Ala Gly Leu Asp Gly Tyr Val Leu Glu Tyr Cys Phe Glu Gly Thr Glu
725 730 735

Asp Trp Ile Val Ala Asn Lys Asp Leu Ile Asp Lys Thr Lys Phe Thr
740 745 750

Ile Thr Gly Leu Pro Thr Asp Ala Lys Ile Phe Val Arg Val Lys Ala
755 760 765

Val Asn Ala Ala Gly Ala Ser Glu Pro Lys Tyr Tyr Ser Gln Pro Ile
770 775 780

Leu Val Lys Glu Ile Ile Glu Pro Pro Lys Ile Arg Ile Pro Arg His
785 790 795 800

Leu Lys Gln Thr Tyr Ile Arg Arg Val Gly Glu Ala Val Asn Leu Val
805 810 815

Ile Pro Phe Gln Gly Lys Pro Arg Pro Glu Leu Thr Trp Lys Lys Asp
820 825 830

Gly Ala Glu Ile Asp Lys Asn Gln Ile Asn Ile Arg Asn Ser Glu Thr
835 840 845

Asp Thr Ile Ile Phe Ile Arg Lys Ala Glu Arg Ser His Ser Gly Lys
850 855 860

Tyr Asp Leu Gln Val Lys Val Asp Lys Phe Val Glu Thr Ala Ser Ile
865 870 875 880

Asp Ile Gln Ile Ile Asp Arg Pro Gly Pro Pro Gln Ile Val Lys Ile
885 890 895

Glu Asp Val Trp Gly Glu Asn Val Ala Leu Thr Trp Thr Pro Pro Lys
900 905 910

Asp Asp Gly Asn Ala Ala Ile Thr Gly Tyr Thr Ile Gln Lys Ala Asp
915 920 925

Lys Lys Ser Met Glu Trp Phe Thr Val Ile Glu His Tyr His Arg Thr
930 935 940

Ser Ala Thr Ile Thr Glu Leu Val Ile Gly Asn Glu Tyr Tyr Phe Arg
945 950 955 960

Val Phe Ser Glu Asn Met Cys Gly Leu Ser Glu Asp Ala Thr Met Thr
965 970 975

Lys Glu Ser Ala Val Ile Ala Arg Asp Gly Lys Ile Tyr Lys Asn Pro
980 985 990

Val Tyr Glu Asp Phe Asp Phe Ser Glu Ala Pro Met Phe Thr Gln Pro
995 1000 1005

Leu Val Asn Thr Tyr Ala Ile Ala Gly Tyr Asn Ala Thr Leu Asn
1010 1015 1020

Cys Ser Val Arg Gly Asn Pro Lys Pro Lys Ile Thr Trp Met Lys
1025 1030 1035

Asn Lys Val Ala Ile Val Asp Asp Pro Arg Tyr Arg Met Phe Ser

```
            1040                1045                1050

Asn Gln  Gly Val Cys Thr Leu  Glu Ile Arg Lys Pro  Ser Pro Tyr
    1055                1060                1065

Asp Gly  Gly Thr Tyr Cys Cys  Lys Ala Val Asn Asp  Leu Gly Thr
    1070                1075                1080

Val Glu  Ile Glu Cys Lys Leu  Glu Val Lys Val Ile  Tyr Gln Gly
    1085                1090                1095

Val Asn  Thr Pro Gly Gln Pro  Val Phe Leu Glu Gly  Gln Gln Gln
    1100                1105                1110

Ser Leu  His Asn Lys Asp Phe
    1115                1120
```

<210> 79
<211> 1139
<212> PRT
<213> Homo sapiens

<400> 79

```
Met Pro Glu Pro Thr Lys Lys Glu Asp Glu Glu Glu Val Ser Pro Pro
1               5                   10                  15

Ser Ala Leu Pro Pro Asp Trp Thr Leu Val Glu Thr Pro Pro Gly Glu
            20                  25                  30

Glu Gln Ala Lys Gln Asn Ala Asn Ser Gln Leu Ser Ile Leu Phe Ile
        35                  40                  45

Glu Lys Pro Gln Gly Gly Thr Val Lys Val Gly Glu Asp Ile Thr Phe
    50                  55                  60

Ile Ala Lys Val Lys Ala Glu Asp Leu Leu Arg Lys Pro Thr Ile Lys
65                  70                  75                  80

Trp Phe Lys Gly Lys Trp Met Asp Leu Ala Ser Lys Ala Gly Lys His
                85                  90                  95

Leu Gln Leu Lys Glu Thr Phe Glu Arg His Ser Arg Val Tyr Thr Phe
            100                 105                 110

Glu Met Gln Ile Ile Lys Ala Lys Asp Asn Phe Ala Gly Asn Tyr Arg
        115                 120                 125

Cys Glu Val Thr Tyr Lys Asp Lys Phe Asp Ser Cys Ser Phe Asp Leu
```

130 135 140

Glu Val His Glu Ser Thr Gly Thr Thr Pro Asn Ile Asp Ile Arg Ser
145 150 155 160

Ala Phe Lys Arg Ser Gly Glu Gly Gln Glu Asp Ala Gly Glu Leu Asp
165 170 175

Phe Ser Gly Leu Leu Lys Arg Arg Glu Val Lys Gln Gln Glu Glu Glu
180 185 190

Pro Gln Val Asp Val Trp Glu Leu Leu Lys Asn Ala Lys Pro Ser Glu
195 200 205

Tyr Glu Lys Ile Ala Phe Gln Tyr Gly Ile Thr Asp Leu Arg Gly Met
210 215 220

Leu Lys Arg Leu Lys Arg Met Arg Arg Glu Glu Lys Lys Ser Ala Ala
225 230 235 240

Phe Ala Lys Ile Leu Asp Pro Ala Tyr Gln Val Asp Lys Gly Gly Arg
245 250 255

Val Arg Phe Val Val Glu Leu Ala Asp Pro Lys Leu Glu Val Lys Trp
260 265 270

Tyr Lys Asn Gly Gln Glu Ile Arg Pro Ser Thr Lys Tyr Ile Phe Glu
275 280 285

His Lys Gly Cys Gln Arg Ile Leu Phe Ile Asn Asn Cys Gln Met Thr
290 295 300

Asp Asp Ser Glu Tyr Tyr Val Thr Ala Gly Asp Glu Lys Cys Ser Thr
305 310 315 320

Glu Leu Phe Val Arg Glu Pro Pro Ile Met Val Thr Lys Gln Leu Glu
325 330 335

Asp Thr Thr Ala Tyr Cys Gly Glu Arg Val Glu Leu Glu Cys Glu Val
340 345 350

Ser Glu Asp Asp Ala Asn Val Lys Trp Phe Lys Asn Gly Glu Glu Ile
355 360 365

Ile Pro Gly Pro Lys Ser Arg Tyr Arg Ile Arg Val Glu Gly Lys Lys
370 375 380

```
His Ile Leu Ile Ile Glu Gly Ala Thr Lys Ala Asp Ala Ala Glu Tyr
385                 390                 395                 400

Ser Val Met Thr Thr Gly Gly Gln Ser Ser Ala Lys Leu Ser Val Asp
                405                 410                 415

Leu Lys Pro Leu Lys Ile Leu Thr Pro Leu Thr Asp Gln Thr Val Asn
            420                 425                 430

Leu Gly Lys Glu Ile Cys Leu Lys Cys Glu Ile Ser Glu Asn Ile Pro
        435                 440                 445

Gly Lys Trp Thr Lys Asn Gly Leu Pro Val Gln Glu Ser Asp Arg Leu
    450                 455                 460

Lys Val Val His Lys Gly Arg Ile His Lys Leu Val Ile Ala Asn Ala
465                 470                 475                 480

Leu Thr Glu Asp Glu Gly Asp Tyr Val Phe Ala Pro Asp Ala Tyr Asn
                485                 490                 495

Val Thr Leu Pro Ala Lys Val His Val Ile Asp Pro Pro Lys Ile Ile
            500                 505                 510

Leu Asp Gly Leu Asp Ala Asp Asn Thr Val Thr Val Ile Ala Gly Asn
        515                 520                 525

Lys Leu Arg Leu Glu Ile Pro Ile Ser Gly Glu Pro Pro Pro Lys Ala
    530                 535                 540

Met Trp Ser Arg Gly Asp Lys Ala Ile Met Glu Gly Ser Gly Arg Ile
545                 550                 555                 560

Arg Thr Glu Ser Tyr Pro Asp Ser Ser Thr Leu Val Ile Asp Ile Ala
                565                 570                 575

Glu Arg Asp Asp Ser Gly Val Tyr His Ile Asn Leu Lys Asn Glu Ala
            580                 585                 590

Gly Glu Ala His Ala Ser Ile Lys Val Lys Val Val Asp Phe Pro Asp
        595                 600                 605

Pro Pro Val Ala Pro Thr Val Thr Glu Val Gly Asp Asp Trp Cys Ile
    610                 615                 620

Met Asn Trp Glu Pro Pro Ala Tyr Asp Gly Gly Ser Pro Ile Leu Gly
625                 630                 635                 640
```

```
Tyr Phe Ile Glu Arg Lys Lys Lys Gln Ser Ser Arg Trp Met Arg Leu
                645                 650                 655

Asn Phe Asp Leu Cys Lys Glu Thr Thr Phe Glu Pro Lys Lys Met Ile
            660                 665                 670

Glu Gly Val Ala Tyr Glu Val Arg Ile Phe Ala Val Asn Ala Ile Gly
        675                 680                 685

Ile Ser Lys Pro Ser Met Pro Ser Arg Pro Phe Val Pro Leu Ala Val
    690                 695                 700

Thr Ser Pro Pro Thr Leu Leu Thr Val Asp Ser Val Thr Asp Thr Thr
705                 710                 715                 720

Val Thr Met Arg Trp Arg Pro Pro Asp His Ile Gly Ala Ala Gly Leu
                725                 730                 735

Asp Gly Tyr Val Leu Glu Tyr Cys Phe Glu Gly Thr Glu Asp Trp Ile
            740                 745                 750

Val Ala Asn Lys Asp Leu Ile Asp Lys Thr Lys Phe Thr Ile Thr Gly
        755                 760                 765

Leu Pro Thr Asp Ala Lys Ile Phe Val Arg Val Lys Ala Val Asn Ala
    770                 775                 780

Ala Gly Ala Ser Glu Pro Lys Tyr Tyr Ser Gln Pro Ile Leu Val Lys
785                 790                 795                 800

Glu Ile Ile Glu Pro Pro Lys Ile Arg Ile Pro Arg His Leu Lys Gln
                805                 810                 815

Thr Tyr Ile Arg Arg Val Gly Glu Ala Val Asn Leu Val Ile Pro Phe
            820                 825                 830

Gln Gly Lys Pro Arg Pro Glu Leu Thr Trp Lys Lys Asp Gly Ala Glu
        835                 840                 845

Ile Asp Lys Asn Gln Ile Asn Ile Arg Asn Ser Glu Thr Asp Thr Ile
    850                 855                 860

Ile Phe Ile Arg Lys Ala Glu Arg Ser His Ser Gly Lys Tyr Asp Leu
865                 870                 875                 880

Gln Val Lys Val Asp Lys Phe Val Glu Thr Ala Ser Ile Asp Ile Gln
                885                 890                 895
```

```
Ile Ile Asp Arg Pro Gly Pro Pro Gln Ile Val Lys Ile Glu Asp Val
            900                 905                 910

Trp Gly Glu Asn Val Ala Leu Thr Trp Thr Pro Pro Lys Asp Asp Gly
        915                 920                 925

Asn Ala Ala Ile Thr Gly Tyr Thr Ile Gln Lys Ala Asp Lys Lys Ser
    930                 935                 940

Met Glu Trp Phe Thr Val Ile Glu His Tyr His Arg Thr Ser Ala Thr
945                 950                 955                 960

Ile Thr Glu Leu Val Ile Gly Asn Glu Tyr Tyr Phe Arg Val Phe Ser
                965                 970                 975

Glu Asn Met Cys Gly Leu Ser Glu Asp Ala Thr Met Thr Lys Glu Ser
            980                 985                 990

Ala Val Ile Ala Arg Asp Gly Lys  Ile Tyr Lys Asn Pro  Val Tyr Glu
        995                 1000                 1005

Asp Phe  Asp Phe Ser Glu Ala  Pro Met Phe Thr Gln  Pro Leu Val
    1010                 1015                 1020

Asn Thr  Tyr Ala Ile Ala Gly  Tyr Asn Ala Thr Leu  Asn Cys Ser
    1025                 1030                 1035

Val Arg  Gly Asn Pro Lys Pro  Lys Ile Thr Trp Met  Lys Asn Lys
    1040                 1045                 1050

Val Ala  Ile Val Asp Asp Pro  Arg Tyr Arg Met Phe  Ser Asn Gln
    1055                 1060                 1065

Gly Val  Cys Thr Leu Glu Ile  Arg Lys Pro Ser Pro  Tyr Asp Gly
    1070                 1075                 1080

Gly Thr  Tyr Cys Cys Lys Ala  Val Asn Asp Leu Gly  Thr Val Glu
    1085                 1090                 1095

Ile Glu  Cys Lys Leu Glu Val  Lys Gly Gly Leu Ser  Phe Cys Arg
    1100                 1105                 1110

Leu Leu  Leu Gln Gly Val Pro  Pro Asn Ile Ile Asp  Ser Tyr Leu
    1115                 1120                 1125

Arg Asp  Leu His Ser Ser Asn  Pro Glu Glu Tyr
```

1130 1135

<210> 80
<211> 1171
<212> PRT
<213> Homo sapiens

<400> 80

```
Met Pro Glu Pro Thr Lys Lys Glu Glu Asn Glu Val Pro Ala Pro Ala
1               5                   10                  15

Pro Pro Pro Glu Glu Pro Ser Lys Glu Lys Glu Ala Gly Thr Thr Pro
            20                  25                  30

Ala Lys Asp Glu Glu Glu Val Ser Pro Pro Ser Ala Leu Pro Pro Gly
        35                  40                  45

Leu Gly Ser Arg Ala Leu Glu Arg Lys Asp Ser Asp Trp Thr Leu Val
    50                  55                  60

Glu Thr Pro Pro Gly Glu Glu Gln Ala Lys Gln Asn Ala Asn Ser Gln
65                  70                  75                  80

Leu Ser Ile Leu Phe Ile Glu Lys Pro Gln Gly Gly Thr Val Lys Val
                85                  90                  95

Gly Glu Asp Ile Thr Phe Ile Ala Lys Val Lys Ala Glu Asp Leu Leu
            100                 105                 110

Arg Lys Pro Thr Ile Lys Trp Phe Lys Gly Lys Trp Met Asp Leu Ala
        115                 120                 125

Ser Lys Ala Gly Lys His Leu Gln Leu Lys Glu Thr Phe Glu Arg His
    130                 135                 140

Ser Arg Val Tyr Thr Phe Glu Met Gln Ile Ile Lys Ala Lys Asp Asn
145                 150                 155                 160

Phe Ala Gly Asn Tyr Arg Cys Glu Val Thr Tyr Lys Asp Lys Phe Asp
                165                 170                 175

Ser Cys Ser Phe Asp Leu Glu Val His Glu Ser Thr Gly Thr Thr Pro
            180                 185                 190

Asn Ile Asp Ile Arg Ser Ala Phe Lys Arg Ser Gly Glu Gly Gln Glu
        195                 200                 205

Asp Ala Gly Glu Leu Asp Phe Ser Gly Leu Leu Lys Arg Arg Glu Val
```

```
    210                 215                 220

Lys Gln Gln Glu Glu Glu Pro Gln Val Asp Val Trp Glu Leu Leu Lys
225                 230                 235                 240

Asn Ala Lys Pro Ser Glu Tyr Glu Lys Ile Ala Phe Gln Tyr Gly Ile
                245                 250                 255

Thr Asp Leu Arg Gly Met Leu Lys Arg Leu Lys Arg Met Arg Arg Glu
            260                 265                 270

Glu Lys Lys Ser Ala Ala Phe Ala Lys Ile Leu Asp Pro Ala Tyr Gln
        275                 280                 285

Val Asp Lys Gly Gly Arg Val Arg Phe Val Val Glu Leu Ala Asp Pro
    290                 295                 300

Lys Leu Glu Val Lys Trp Tyr Lys Asn Gly Gln Glu Ile Arg Pro Ser
305                 310                 315                 320

Thr Lys Tyr Ile Phe Glu His Lys Gly Cys Gln Arg Ile Leu Phe Ile
                325                 330                 335

Asn Asn Cys Gln Met Thr Asp Asp Ser Glu Tyr Tyr Val Thr Ala Gly
            340                 345                 350

Asp Glu Lys Cys Ser Thr Glu Leu Phe Val Arg Glu Pro Pro Ile Met
        355                 360                 365

Val Thr Lys Gln Leu Glu Asp Thr Thr Ala Tyr Cys Gly Glu Arg Val
    370                 375                 380

Glu Leu Glu Cys Glu Val Ser Glu Asp Asp Ala Asn Val Lys Trp Phe
385                 390                 395                 400

Lys Asn Gly Glu Glu Ile Ile Pro Gly Pro Lys Ser Arg Tyr Arg Ile
                405                 410                 415

Arg Val Glu Gly Lys Lys His Ile Leu Ile Ile Glu Gly Ala Thr Lys
            420                 425                 430

Ala Asp Ala Ala Glu Tyr Ser Val Met Thr Thr Gly Gly Gln Ser Ser
        435                 440                 445

Ala Lys Leu Ser Val Asp Leu Lys Pro Leu Lys Ile Leu Thr Pro Leu
    450                 455                 460
```

```
Thr Asp Gln Thr Val Asn Leu Gly Lys Glu Ile Cys Leu Lys Cys Glu
465                 470                 475                 480

Ile Ser Glu Asn Ile Pro Gly Lys Trp Thr Lys Asn Gly Leu Pro Val
                485                 490                 495

Gln Glu Ser Asp Arg Leu Lys Val Val His Lys Gly Arg Ile His Lys
            500                 505                 510

Leu Val Ile Ala Asn Ala Leu Thr Glu Asp Glu Gly Asp Tyr Val Phe
        515                 520                 525

Ala Pro Asp Ala Tyr Asn Val Thr Leu Pro Ala Lys Val His Val Ile
    530                 535                 540

Asp Pro Pro Lys Ile Ile Leu Asp Gly Leu Asp Ala Asp Asn Thr Val
545                 550                 555                 560

Thr Val Ile Ala Gly Asn Lys Leu Arg Leu Glu Ile Pro Ile Ser Gly
                565                 570                 575

Glu Pro Pro Pro Lys Ala Met Trp Ser Arg Gly Asp Lys Ala Ile Met
            580                 585                 590

Glu Gly Ser Gly Arg Ile Arg Thr Glu Ser Tyr Pro Asp Ser Ser Thr
        595                 600                 605

Leu Val Ile Asp Ile Ala Glu Arg Asp Asp Ser Gly Val Tyr His Ile
    610                 615                 620

Asn Leu Lys Asn Glu Ala Gly Glu Ala His Ala Ser Ile Lys Val Lys
625                 630                 635                 640

Val Val Asp Phe Pro Asp Pro Pro Val Ala Pro Thr Val Thr Glu Val
                645                 650                 655

Gly Asp Asp Trp Cys Ile Met Asn Trp Glu Pro Pro Ala Tyr Asp Gly
            660                 665                 670

Gly Ser Pro Ile Leu Gly Tyr Phe Ile Glu Arg Lys Lys Lys Gln Ser
        675                 680                 685

Ser Arg Trp Met Arg Leu Asn Phe Asp Leu Cys Lys Glu Thr Thr Phe
    690                 695                 700

Glu Pro Lys Lys Met Ile Glu Gly Val Ala Tyr Glu Val Arg Ile Phe
705                 710                 715                 720
```

Ala Val Asn Ala Ile Gly Ile Ser Lys Pro Ser Met Pro Ser Arg Pro
725 730 735

Phe Val Pro Leu Ala Val Thr Ser Pro Pro Thr Leu Leu Thr Val Asp
740 745 750

Ser Val Thr Asp Thr Thr Val Thr Met Arg Trp Arg Pro Pro Asp His
755 760 765

Ile Gly Ala Ala Gly Leu Asp Gly Tyr Val Leu Glu Tyr Cys Phe Glu
770 775 780

Gly Thr Glu Asp Trp Ile Val Ala Asn Lys Asp Leu Ile Asp Lys Thr
785 790 795 800

Lys Phe Thr Ile Thr Gly Leu Pro Thr Asp Ala Lys Ile Phe Val Arg
805 810 815

Val Lys Ala Val Asn Ala Ala Gly Ala Ser Glu Pro Lys Tyr Tyr Ser
820 825 830

Gln Pro Ile Leu Val Lys Glu Ile Ile Glu Pro Pro Lys Ile Arg Ile
835 840 845

Pro Arg His Leu Lys Gln Thr Tyr Ile Arg Arg Val Gly Glu Ala Val
850 855 860

Asn Leu Val Ile Pro Phe Gln Gly Lys Pro Arg Pro Glu Leu Thr Trp
865 870 875 880

Lys Lys Asp Gly Ala Glu Ile Asp Lys Asn Gln Ile Asn Ile Arg Asn
885 890 895

Ser Glu Thr Asp Thr Ile Ile Phe Ile Arg Lys Ala Glu Arg Ser His
900 905 910

Ser Gly Lys Tyr Asp Leu Gln Val Lys Val Asp Lys Phe Val Glu Thr
915 920 925

Ala Ser Ile Asp Ile Gln Ile Ile Asp Arg Pro Gly Pro Pro Gln Ile
930 935 940

Val Lys Ile Glu Asp Val Trp Gly Glu Asn Val Ala Leu Thr Trp Thr
945 950 955 960

Pro Pro Lys Asp Asp Gly Asn Ala Ala Ile Thr Gly Tyr Thr Ile Gln
965 970 975

Lys Ala Asp Lys Lys Ser Met Glu Trp Phe Thr Val Ile Glu His Tyr
980 985 990

His Arg Thr Ser Ala Thr Ile Thr Glu Leu Val Ile Gly Asn Glu Tyr
995 1000 1005

Tyr Phe Arg Val Phe Ser Glu Asn Met Cys Gly Leu Ser Glu Asp
1010 1015 1020

Ala Thr Met Thr Lys Glu Ser Ala Val Ile Ala Arg Asp Gly Lys
1025 1030 1035

Ile Tyr Lys Asn Pro Val Tyr Glu Asp Phe Asp Phe Ser Glu Ala
1040 1045 1050

Pro Met Phe Thr Gln Pro Leu Val Asn Thr Tyr Ala Ile Ala Gly
1055 1060 1065

Tyr Asn Ala Thr Leu Asn Cys Ser Val Arg Gly Asn Pro Lys Pro
1070 1075 1080

Lys Ile Thr Trp Met Lys Asn Lys Val Ala Ile Val Asp Asp Pro
1085 1090 1095

Arg Tyr Arg Met Phe Ser Asn Gln Gly Val Cys Thr Leu Glu Ile
1100 1105 1110

Arg Lys Pro Ser Pro Tyr Asp Gly Gly Thr Tyr Cys Cys Lys Ala
1115 1120 1125

Val Asn Asp Leu Gly Thr Val Glu Ile Glu Cys Lys Leu Glu Val
1130 1135 1140

Lys Val Ile Tyr Gln Gly Val Asn Thr Pro Gly Gln Pro Val Phe
1145 1150 1155

Leu Glu Gly Gln Gln Gln Ser Leu His Asn Lys Asp Phe
1160 1165 1170

<210> 81
<211> 1148
<212> PRT
<213> Homo sapiens

<400> 81

Met Pro Glu Pro Thr Lys Lys Glu Glu Asn Glu Val Pro Ala Pro Ala
1 5 10 15

```
Pro Pro Pro Glu Glu Pro Ser Lys Glu Lys Glu Ala Gly Thr Thr Pro
            20                  25                  30

Ala Lys Asp Glu Glu Glu Val Ser Pro Pro Ser Ala Leu Pro Pro Gly
        35                  40                  45

Leu Gly Ser Arg Ala Leu Glu Arg Lys Asp Ser Asp Trp Thr Leu Val
    50                  55                  60

Glu Thr Pro Pro Gly Glu Glu Gln Ala Lys Gln Asn Ala Asn Ser Gln
65                  70                  75                  80

Leu Ser Ile Leu Phe Ile Glu Lys Pro Gln Gly Gly Thr Val Lys Val
                85                  90                  95

Gly Glu Asp Ile Thr Phe Ile Ala Lys Val Lys Ala Glu Asp Leu Leu
            100                 105                 110

Arg Lys Pro Thr Ile Lys Trp Phe Lys Gly Lys Trp Met Asp Leu Ala
        115                 120                 125

Ser Lys Ala Gly Lys His Leu Gln Leu Lys Glu Thr Phe Glu Arg His
    130                 135                 140

Ser Arg Val Tyr Thr Phe Glu Met Gln Ile Ile Lys Ala Lys Asp Asn
145                 150                 155                 160

Phe Ala Gly Asn Tyr Arg Cys Glu Val Thr Tyr Lys Asp Lys Phe Asp
                165                 170                 175

Ser Cys Ser Phe Asp Leu Glu Val His Glu Ser Thr Gly Thr Thr Pro
            180                 185                 190

Asn Ile Asp Ile Arg Ser Ala Phe Lys Arg Ser Gly Glu Gly Gln Glu
        195                 200                 205

Asp Ala Gly Glu Leu Asp Phe Ser Gly Leu Leu Lys Arg Arg Glu Val
    210                 215                 220

Lys Gln Gln Glu Glu Glu Pro Gln Val Asp Val Trp Glu Leu Leu Lys
225                 230                 235                 240

Asn Ala Lys Pro Ser Glu Tyr Glu Lys Ile Ala Phe Gln Tyr Gly Ile
                245                 250                 255

Thr Asp Leu Arg Gly Met Leu Lys Arg Leu Lys Arg Met Arg Arg Glu
```

260 265 270

Glu Lys Lys Ser Ala Ala Phe Ala Lys Ile Leu Asp Pro Ala Tyr Gln
275 280 285

Val Asp Lys Gly Gly Arg Val Arg Phe Val Val Glu Leu Ala Asp Pro
290 295 300

Lys Leu Glu Val Lys Trp Tyr Lys Asn Gly Gln Glu Ile Arg Pro Ser
305 310 315 320

Thr Lys Tyr Ile Phe Glu His Lys Gly Cys Gln Arg Ile Leu Phe Ile
325 330 335

Asn Asn Cys Gln Met Thr Asp Asp Ser Glu Tyr Tyr Val Thr Ala Gly
340 345 350

Asp Glu Lys Cys Ser Thr Glu Leu Phe Val Arg Glu Pro Pro Ile Met
355 360 365

Val Thr Lys Gln Leu Glu Asp Thr Thr Ala Tyr Cys Gly Glu Arg Val
370 375 380

Glu Leu Glu Cys Glu Val Ser Glu Asp Asp Ala Asn Val Lys Trp Phe
385 390 395 400

Lys Asn Gly Glu Glu Ile Ile Pro Gly Pro Lys Ser Arg Tyr Arg Ile
405 410 415

Arg Val Glu Gly Lys Lys His Ile Leu Ile Ile Glu Gly Ala Thr Lys
420 425 430

Ala Asp Ala Ala Glu Tyr Ser Val Met Thr Thr Gly Gly Gln Ser Ser
435 440 445

Ala Lys Leu Ser Val Asp Leu Lys Pro Leu Lys Ile Leu Thr Pro Leu
450 455 460

Thr Asp Gln Thr Val Asn Leu Gly Lys Glu Ile Cys Leu Lys Cys Glu
465 470 475 480

Ile Ser Glu Asn Ile Pro Gly Lys Trp Thr Lys Asn Gly Leu Pro Val
485 490 495

Gln Glu Ser Asp Arg Leu Lys Val Val His Lys Gly Arg Ile His Lys
500 505 510

Leu Val Ile Ala Asn Ala Leu Thr Glu Asp Glu Gly Asp Tyr Val Phe
515 520 525

Ala Pro Asp Ala Tyr Asn Val Thr Leu Pro Ala Lys Val His Val Ile
530 535 540

Asp Pro Pro Lys Ile Ile Leu Asp Gly Leu Asp Ala Asp Asn Thr Val
545 550 555 560

Thr Val Ile Ala Gly Asn Lys Leu Arg Leu Glu Ile Pro Ile Ser Gly
565 570 575

Glu Pro Pro Pro Lys Ala Met Trp Ser Arg Gly Asp Lys Ala Ile Met
580 585 590

Glu Gly Ser Gly Arg Ile Arg Thr Glu Ser Tyr Pro Asp Ser Ser Thr
595 600 605

Leu Val Ile Asp Ile Ala Glu Arg Asp Asp Ser Gly Val Tyr His Ile
610 615 620

Asn Leu Lys Asn Glu Ala Gly Glu Ala His Ala Ser Ile Lys Val Lys
625 630 635 640

Val Val Asp Phe Pro Asp Pro Pro Val Ala Pro Thr Val Thr Glu Val
645 650 655

Gly Asp Asp Trp Cys Ile Met Asn Trp Glu Pro Pro Ala Tyr Asp Gly
660 665 670

Gly Ser Pro Ile Leu Gly Tyr Phe Ile Glu Arg Lys Lys Lys Gln Ser
675 680 685

Ser Arg Trp Met Arg Leu Asn Phe Asp Leu Cys Lys Glu Thr Thr Phe
690 695 700

Glu Pro Lys Lys Met Ile Glu Gly Val Ala Tyr Glu Val Arg Ile Phe
705 710 715 720

Ala Val Asn Ala Ile Gly Ile Ser Lys Pro Ser Met Pro Ser Arg Pro
725 730 735

Phe Val Pro Leu Ala Val Thr Ser Pro Pro Thr Leu Leu Thr Val Asp
740 745 750

Ser Val Thr Asp Thr Thr Val Thr Met Arg Trp Arg Pro Pro Asp His
755 760 765

```
Ile Gly Ala Ala Gly Leu Asp Gly Tyr Val Leu Glu Tyr Cys Phe Glu
    770                 775                 780

Gly Thr Glu Asp Trp Ile Val Ala Asn Lys Asp Leu Ile Asp Lys Thr
785                 790                 795                 800

Lys Phe Thr Ile Thr Gly Leu Pro Thr Asp Ala Lys Ile Phe Val Arg
                805                 810                 815

Val Lys Ala Val Asn Ala Ala Gly Ala Ser Glu Pro Lys Tyr Tyr Ser
            820                 825                 830

Gln Pro Ile Leu Val Lys Glu Ile Ile Glu Pro Pro Lys Ile Arg Ile
        835                 840                 845

Pro Arg His Leu Lys Gln Thr Tyr Ile Arg Arg Val Gly Glu Ala Val
    850                 855                 860

Asn Leu Val Ile Pro Phe Gln Gly Lys Pro Arg Pro Glu Leu Thr Trp
865                 870                 875                 880

Lys Lys Asp Gly Ala Glu Ile Asp Lys Asn Gln Ile Asn Ile Arg Asn
                885                 890                 895

Ser Glu Thr Asp Thr Ile Ile Phe Ile Arg Lys Ala Glu Arg Ser His
            900                 905                 910

Ser Gly Lys Tyr Asp Leu Gln Val Lys Val Asp Lys Phe Val Glu Thr
        915                 920                 925

Ala Ser Ile Asp Ile Gln Ile Ile Asp Arg Pro Gly Pro Pro Gln Ile
    930                 935                 940

Val Lys Ile Glu Asp Val Trp Gly Glu Asn Val Ala Leu Thr Trp Thr
945                 950                 955                 960

Pro Pro Lys Asp Asp Gly Asn Ala Ala Ile Thr Gly Tyr Thr Ile Gln
                965                 970                 975

Lys Ala Asp Lys Lys Ser Met Glu Trp Phe Thr Val Ile Glu His Tyr
            980                 985                 990

His Arg Thr Ser Ala Thr Ile Thr  Glu Leu Val Ile Gly  Asn Glu Tyr
        995                 1000                1005

Tyr Phe  Arg Val Phe Ser Glu  Asn Met Cys Gly Leu  Ser Glu Asp
    1010                1015                1020
```

Ala Thr Met Thr Lys Glu Ser Ala Val Ile Ala Arg Asp Gly Lys
1025 1030 1035

Ile Tyr Lys Asn Pro Val Tyr Glu Asp Phe Asp Phe Ser Glu Ala
1040 1045 1050

Pro Met Phe Thr Gln Pro Leu Val Asn Thr Tyr Ala Ile Ala Gly
1055 1060 1065

Tyr Asn Ala Thr Leu Asn Cys Ser Val Arg Gly Asn Pro Lys Pro
1070 1075 1080

Lys Ile Thr Trp Met Lys Asn Lys Val Ala Ile Val Asp Asp Pro
1085 1090 1095

Arg Tyr Arg Met Phe Ser Asn Gln Gly Val Cys Thr Leu Glu Ile
1100 1105 1110

Arg Lys Pro Ser Pro Tyr Asp Gly Gly Thr Tyr Cys Cys Lys Ala
1115 1120 1125

Val Asn Asp Leu Gly Thr Val Glu Ile Glu Cys Lys Leu Glu Val
1130 1135 1140

Lys Val Ile Ala Gln
1145

<210> 82
<211> 1141
<212> PRT
<213> Homo sapiens

<400> 82

Met Pro Glu Pro Thr Lys Lys Glu Glu Asn Glu Val Pro Ala Pro Ala
1 5 10 15

Pro Pro Pro Glu Glu Pro Ser Lys Glu Lys Glu Ala Gly Thr Thr Pro
20 25 30

Ala Lys Asp Trp Thr Leu Val Glu Thr Pro Pro Gly Glu Glu Gln Ala
35 40 45

Lys Gln Asn Ala Asn Ser Gln Leu Ser Ile Leu Phe Ile Glu Lys Pro
50 55 60

Gln Gly Gly Thr Val Lys Val Gly Glu Asp Ile Thr Phe Ile Ala Lys
65 70 75 80

```
Val Lys Ala Glu Asp Leu Leu Arg Lys Pro Thr Ile Lys Trp Phe Lys
                85                  90                  95

Gly Lys Trp Met Asp Leu Ala Ser Lys Ala Gly Lys His Leu Gln Leu
            100                 105                 110

Lys Glu Thr Phe Glu Arg His Ser Arg Val Tyr Thr Phe Glu Met Gln
        115                 120                 125

Ile Ile Lys Ala Lys Asp Asn Phe Ala Gly Asn Tyr Arg Cys Glu Val
    130                 135                 140

Thr Tyr Lys Asp Lys Phe Asp Ser Cys Ser Phe Asp Leu Glu Val His
145                 150                 155                 160

Glu Ser Thr Gly Thr Thr Pro Asn Ile Asp Ile Arg Ser Ala Phe Lys
                165                 170                 175

Arg Ser Gly Glu Gly Gln Glu Asp Ala Gly Glu Leu Asp Phe Ser Gly
            180                 185                 190

Leu Leu Lys Arg Arg Glu Val Lys Gln Gln Glu Glu Glu Pro Gln Val
        195                 200                 205

Asp Val Trp Glu Leu Leu Lys Asn Ala Lys Pro Ser Glu Tyr Glu Lys
    210                 215                 220

Ile Ala Phe Gln Tyr Gly Ile Thr Asp Leu Arg Gly Met Leu Lys Arg
225                 230                 235                 240

Leu Lys Arg Met Arg Arg Glu Glu Lys Lys Ser Ala Ala Phe Ala Lys
                245                 250                 255

Ile Leu Asp Pro Ala Tyr Gln Val Asp Lys Gly Gly Arg Val Arg Phe
            260                 265                 270

Val Val Glu Leu Ala Asp Pro Lys Leu Glu Val Lys Trp Tyr Lys Asn
        275                 280                 285

Gly Gln Glu Ile Arg Pro Ser Thr Lys Tyr Ile Phe Glu His Lys Gly
    290                 295                 300

Cys Gln Arg Ile Leu Phe Ile Asn Asn Cys Gln Met Thr Asp Asp Ser
305                 310                 315                 320

Glu Tyr Tyr Val Thr Ala Gly Asp Glu Lys Cys Ser Thr Glu Leu Phe
```

325 330 335

Val Arg Glu Pro Pro Ile Met Val Thr Lys Gln Leu Glu Asp Thr Thr
340 345 350

Ala Tyr Cys Gly Glu Arg Val Glu Leu Glu Cys Glu Val Ser Glu Asp
355 360 365

Asp Ala Asn Val Lys Trp Phe Lys Asn Gly Glu Glu Ile Ile Pro Gly
370 375 380

Pro Lys Ser Arg Tyr Arg Ile Arg Val Glu Gly Lys Lys His Ile Leu
385 390 395 400

Ile Ile Glu Gly Ala Thr Lys Ala Asp Ala Ala Glu Tyr Ser Val Met
405 410 415

Thr Thr Gly Gly Gln Ser Ser Ala Lys Leu Ser Val Asp Leu Lys Pro
420 425 430

Leu Lys Ile Leu Thr Pro Leu Thr Asp Gln Thr Val Asn Leu Gly Lys
435 440 445

Glu Ile Cys Leu Lys Cys Glu Ile Ser Glu Asn Ile Pro Gly Lys Trp
450 455 460

Thr Lys Asn Gly Leu Pro Val Gln Glu Ser Asp Arg Leu Lys Val Val
465 470 475 480

His Lys Gly Arg Ile His Lys Leu Val Ile Ala Asn Ala Leu Thr Glu
485 490 495

Asp Glu Gly Asp Tyr Val Phe Ala Pro Asp Ala Tyr Asn Val Thr Leu
500 505 510

Pro Ala Lys Val His Val Ile Asp Pro Pro Lys Ile Ile Leu Asp Gly
515 520 525

Leu Asp Ala Asp Asn Thr Val Thr Val Ile Ala Gly Asn Lys Leu Arg
530 535 540

Leu Glu Ile Pro Ile Ser Gly Glu Pro Pro Pro Lys Ala Met Trp Ser
545 550 555 560

Arg Gly Asp Lys Ala Ile Met Glu Gly Ser Gly Arg Ile Arg Thr Glu
565 570 575

```
Ser Tyr Pro Asp Ser Ser Thr Leu Val Ile Asp Ile Ala Glu Arg Asp
            580                 585                 590

Asp Ser Gly Val Tyr His Ile Asn Leu Lys Asn Glu Ala Gly Glu Ala
        595                 600                 605

His Ala Ser Ile Lys Val Lys Val Val Asp Phe Pro Asp Pro Pro Val
    610                 615                 620

Ala Pro Thr Val Thr Glu Val Gly Asp Asp Trp Cys Ile Met Asn Trp
625                 630                 635                 640

Glu Pro Pro Ala Tyr Asp Gly Gly Ser Pro Ile Leu Gly Tyr Phe Ile
                645                 650                 655

Glu Arg Lys Lys Lys Gln Ser Ser Arg Trp Met Arg Leu Asn Phe Asp
            660                 665                 670

Leu Cys Lys Glu Thr Thr Phe Glu Pro Lys Lys Met Ile Glu Gly Val
        675                 680                 685

Ala Tyr Glu Val Arg Ile Phe Ala Val Asn Ala Ile Gly Ile Ser Lys
    690                 695                 700

Pro Ser Met Pro Ser Arg Pro Phe Val Pro Leu Ala Val Thr Ser Pro
705                 710                 715                 720

Pro Thr Leu Leu Thr Val Asp Ser Val Thr Asp Thr Thr Val Thr Met
                725                 730                 735

Arg Trp Arg Pro Pro Asp His Ile Gly Ala Ala Gly Leu Asp Gly Tyr
            740                 745                 750

Val Leu Glu Tyr Cys Phe Glu Gly Ser Thr Ser Ala Lys Gln Ser Asp
        755                 760                 765

Glu Asn Gly Glu Ala Ala Tyr Asp Leu Pro Ala Glu Asp Trp Ile Val
    770                 775                 780

Ala Asn Lys Asp Leu Ile Asp Lys Thr Lys Phe Thr Ile Thr Gly Leu
785                 790                 795                 800

Pro Thr Asp Ala Lys Ile Phe Val Arg Val Lys Ala Val Asn Ala Ala
                805                 810                 815

Gly Ala Ser Glu Pro Lys Tyr Tyr Ser Gln Pro Ile Leu Val Lys Glu
            820                 825                 830
```

```
Ile Ile Glu Pro Pro Lys Ile Arg Ile Pro Arg His Leu Lys Gln Thr
        835                 840                 845

Tyr Ile Arg Arg Val Gly Glu Ala Val Asn Leu Val Ile Pro Phe Gln
    850                 855                 860

Gly Lys Pro Arg Pro Glu Leu Thr Trp Lys Lys Asp Gly Ala Glu Ile
865                 870                 875                 880

Asp Lys Asn Gln Ile Asn Ile Arg Asn Ser Glu Thr Asp Thr Ile Ile
                885                 890                 895

Phe Ile Arg Lys Ala Glu Arg Ser His Ser Gly Lys Tyr Asp Leu Gln
            900                 905                 910

Val Lys Val Asp Lys Phe Val Glu Thr Ala Ser Ile Asp Ile Gln Ile
        915                 920                 925

Ile Asp Arg Pro Gly Pro Pro Gln Ile Val Lys Ile Glu Asp Val Trp
    930                 935                 940

Gly Glu Asn Val Ala Leu Thr Trp Thr Pro Pro Lys Asp Asp Gly Asn
945                 950                 955                 960

Ala Ala Ile Thr Gly Tyr Thr Ile Gln Lys Ala Asp Lys Lys Ser Met
                965                 970                 975

Glu Trp Phe Thr Val Ile Glu His Tyr His Arg Thr Ser Ala Thr Ile
            980                 985                 990

Thr Glu Leu Val Ile Gly Asn Glu  Tyr Tyr Phe Arg Val  Phe Ser Glu
        995                 1000                1005

Asn Met  Cys Gly Leu Ser Glu  Asp Ala Thr Met Thr  Lys Glu Ser
    1010                 1015                 1020

Ala Val  Ile Ala Arg Asp Gly  Lys Ile Tyr Lys Asn  Pro Val Tyr
    1025                 1030                 1035

Glu Asp  Phe Asp Phe Ser Glu  Ala Pro Met Phe Thr  Gln Pro Leu
    1040                 1045                 1050

Val Asn  Thr Tyr Ala Ile Ala  Gly Tyr Asn Ala Thr  Leu Asn Cys
    1055                 1060                 1065

Ser Val  Arg Gly Asn Pro Lys  Pro Lys Ile Thr Trp  Met Lys Asn
    1070                 1075                 1080
```

Lys Val Ala Ile Val Asp Asp Pro Arg Tyr Arg Met Phe Ser Asn
1085 1090 1095

Gln Gly Val Cys Thr Leu Glu Ile Arg Lys Pro Ser Pro Tyr Asp
1100 1105 1110

Gly Gly Thr Tyr Cys Cys Lys Ala Val Asn Asp Leu Gly Thr Val
1115 1120 1125

Glu Ile Glu Cys Lys Leu Glu Val Lys Val Ile Ala Gln
1130 1135 1140

<210> 83
<211> 1123
<212> PRT
<213> Homo sapiens

<400> 83

Met Pro Glu Pro Thr Lys Lys Glu Glu Asn Glu Val Pro Ala Pro Ala
1 5 10 15

Pro Pro Pro Glu Glu Pro Ser Lys Glu Lys Glu Ala Gly Thr Thr Pro
20 25 30

Ala Lys Asp Trp Thr Leu Val Glu Thr Pro Pro Gly Glu Glu Gln Ala
35 40 45

Lys Gln Asn Ala Asn Ser Gln Leu Ser Ile Leu Phe Ile Glu Lys Pro
50 55 60

Gln Gly Gly Thr Val Lys Val Gly Glu Asp Ile Thr Phe Ile Ala Lys
65 70 75 80

Val Lys Ala Glu Asp Leu Leu Arg Lys Pro Thr Ile Lys Trp Phe Lys
85 90 95

Gly Lys Trp Met Asp Leu Ala Ser Lys Ala Gly Lys His Leu Gln Leu
100 105 110

Lys Glu Thr Phe Glu Arg His Ser Arg Val Tyr Thr Phe Glu Met Gln
115 120 125

Ile Ile Lys Ala Lys Asp Asn Phe Ala Gly Asn Tyr Arg Cys Glu Val
130 135 140

Thr Tyr Lys Asp Lys Phe Asp Ser Cys Ser Phe Asp Leu Glu Val His
145 150 155 160

```
Glu Ser Thr Gly Thr Thr Pro Asn Ile Asp Ile Arg Ser Ala Phe Lys
                165                 170                 175

Arg Ser Gly Glu Gly Gln Glu Asp Ala Gly Glu Leu Asp Phe Ser Gly
            180                 185                 190

Leu Leu Lys Arg Arg Glu Val Lys Gln Gln Glu Glu Glu Pro Gln Val
        195                 200                 205

Asp Val Trp Glu Leu Leu Lys Asn Ala Lys Pro Ser Glu Tyr Glu Lys
    210                 215                 220

Ile Ala Phe Gln Tyr Gly Ile Thr Asp Leu Arg Gly Met Leu Lys Arg
225                 230                 235                 240

Leu Lys Arg Met Arg Arg Glu Glu Lys Lys Ser Ala Ala Phe Ala Lys
                245                 250                 255

Ile Leu Asp Pro Ala Tyr Gln Val Asp Lys Gly Gly Arg Val Arg Phe
            260                 265                 270

Val Val Glu Leu Ala Asp Pro Lys Leu Glu Val Lys Trp Tyr Lys Asn
        275                 280                 285

Gly Gln Glu Ile Arg Pro Ser Thr Lys Tyr Ile Phe Glu His Lys Gly
    290                 295                 300

Cys Gln Arg Ile Leu Phe Ile Asn Asn Cys Gln Met Thr Asp Asp Ser
305                 310                 315                 320

Glu Tyr Tyr Val Thr Ala Gly Asp Glu Lys Cys Ser Thr Glu Leu Phe
                325                 330                 335

Val Arg Glu Pro Pro Ile Met Val Thr Lys Gln Leu Glu Asp Thr Thr
            340                 345                 350

Ala Tyr Cys Gly Glu Arg Val Glu Leu Glu Cys Glu Val Ser Glu Asp
        355                 360                 365

Asp Ala Asn Val Lys Trp Phe Lys Asn Gly Glu Glu Ile Ile Pro Gly
    370                 375                 380

Pro Lys Ser Arg Tyr Arg Ile Arg Val Glu Gly Lys Lys His Ile Leu
385                 390                 395                 400

Ile Ile Glu Gly Ala Thr Lys Ala Asp Ala Ala Glu Tyr Ser Val Met
```

```
                405                 410                 415

Thr Thr Gly Gly Gln Ser Ser Ala Lys Leu Ser Val Asp Leu Lys Pro
            420                 425                 430

Leu Lys Ile Leu Thr Pro Leu Thr Asp Gln Thr Val Asn Leu Gly Lys
        435                 440                 445

Glu Ile Cys Leu Lys Cys Glu Ile Ser Glu Asn Ile Pro Gly Lys Trp
    450                 455                 460

Thr Lys Asn Gly Leu Pro Val Gln Glu Ser Asp Arg Leu Lys Val Val
465                 470                 475                 480

His Lys Gly Arg Ile His Lys Leu Val Ile Ala Asn Ala Leu Thr Glu
                485                 490                 495

Asp Glu Gly Asp Tyr Val Phe Ala Pro Asp Ala Tyr Asn Val Thr Leu
            500                 505                 510

Pro Ala Lys Val His Val Ile Asp Pro Pro Lys Ile Ile Leu Asp Gly
        515                 520                 525

Leu Asp Ala Asp Asn Thr Val Thr Val Ile Ala Gly Asn Lys Leu Arg
    530                 535                 540

Leu Glu Ile Pro Ile Ser Gly Glu Pro Pro Pro Lys Ala Met Trp Ser
545                 550                 555                 560

Arg Gly Asp Lys Ala Ile Met Glu Gly Ser Gly Arg Ile Arg Thr Glu
                565                 570                 575

Ser Tyr Pro Asp Ser Ser Thr Leu Val Ile Asp Ile Ala Glu Arg Asp
            580                 585                 590

Asp Ser Gly Val Tyr His Ile Asn Leu Lys Asn Glu Ala Gly Glu Ala
        595                 600                 605

His Ala Ser Ile Lys Val Lys Val Val Asp Phe Pro Asp Pro Pro Val
    610                 615                 620

Ala Pro Thr Val Thr Glu Val Gly Asp Asp Trp Cys Ile Met Asn Trp
625                 630                 635                 640

Glu Pro Pro Ala Tyr Asp Gly Gly Ser Pro Ile Leu Gly Tyr Phe Ile
                645                 650                 655
```

```
Glu Arg Lys Lys Lys Gln Ser Ser Arg Trp Met Arg Leu Asn Phe Asp
            660                 665                 670

Leu Cys Lys Glu Thr Thr Phe Glu Pro Lys Lys Met Ile Glu Gly Val
        675                 680                 685

Ala Tyr Glu Val Arg Ile Phe Ala Val Asn Ala Ile Gly Ile Ser Lys
    690                 695                 700

Pro Ser Met Pro Ser Arg Pro Phe Val Pro Leu Ala Val Thr Ser Pro
705                 710                 715                 720

Pro Thr Leu Leu Thr Val Asp Ser Val Thr Asp Thr Thr Val Thr Met
                725                 730                 735

Arg Trp Arg Pro Pro Asp His Ile Gly Ala Ala Gly Leu Asp Gly Tyr
            740                 745                 750

Val Leu Glu Tyr Cys Phe Glu Gly Thr Glu Asp Trp Ile Val Ala Asn
        755                 760                 765

Lys Asp Leu Ile Asp Lys Thr Lys Phe Thr Ile Thr Gly Leu Pro Thr
    770                 775                 780

Asp Ala Lys Ile Phe Val Arg Val Lys Ala Val Asn Ala Ala Gly Ala
785                 790                 795                 800

Ser Glu Pro Lys Tyr Tyr Ser Gln Pro Ile Leu Val Lys Glu Ile Ile
                805                 810                 815

Glu Pro Pro Lys Ile Arg Ile Pro Arg His Leu Lys Gln Thr Tyr Ile
            820                 825                 830

Arg Arg Val Gly Glu Ala Val Asn Leu Val Ile Pro Phe Gln Gly Lys
        835                 840                 845

Pro Arg Pro Glu Leu Thr Trp Lys Lys Asp Gly Ala Glu Ile Asp Lys
    850                 855                 860

Asn Gln Ile Asn Ile Arg Asn Ser Glu Thr Asp Thr Ile Ile Phe Ile
865                 870                 875                 880

Arg Lys Ala Glu Arg Ser His Ser Gly Lys Tyr Asp Leu Gln Val Lys
                885                 890                 895

Val Asp Lys Phe Val Glu Thr Ala Ser Ile Asp Ile Gln Ile Ile Asp
            900                 905                 910
```

```
Arg Pro Gly Pro Pro Gln Ile Val Lys Ile Glu Asp Val Trp Gly Glu
        915                 920                 925

Asn Val Ala Leu Thr Trp Thr Pro Pro Lys Asp Asp Gly Asn Ala Ala
    930                 935                 940

Ile Thr Gly Tyr Thr Ile Gln Lys Ala Asp Lys Lys Ser Met Glu Trp
945                 950                 955                 960

Phe Thr Val Ile Glu His Tyr His Arg Thr Ser Ala Thr Ile Thr Glu
                965                 970                 975

Leu Val Ile Gly Asn Glu Tyr Tyr Phe Arg Val Phe Ser Glu Asn Met
            980                 985                 990

Cys Gly Leu Ser Glu Asp Ala Thr  Met Thr Lys Glu Ser  Ala Val Ile
        995                 1000                1005

Ala Arg  Asp Gly Lys Ile Tyr  Lys Asn Pro Val Tyr  Glu Asp Phe
    1010                1015                1020

Asp Phe  Ser Glu Ala Pro Met  Phe Thr Gln Pro Leu  Val Asn Thr
    1025                1030                1035

Tyr Ala  Ile Ala Gly Tyr Asn  Ala Thr Leu Asn Cys  Ser Val Arg
    1040                1045                1050

Gly Asn  Pro Lys Pro Lys Ile  Thr Trp Met Lys Asn  Lys Val Ala
    1055                1060                1065

Ile Val  Asp Asp Pro Arg Tyr  Arg Met Phe Ser Asn  Gln Gly Val
    1070                1075                1080

Cys Thr  Leu Glu Ile Arg Lys  Pro Ser Pro Tyr Asp  Gly Gly Thr
    1085                1090                1095

Tyr Cys  Cys Lys Ala Val Asn  Asp Leu Gly Thr Val  Glu Ile Glu
    1100                1105                1110

Cys Lys  Leu Glu Val Lys Val  Ile Ala Gln
    1115                1120
```

<210> 84
<211> 17
<212> DNA
<213> Artificial Sequence

<220>

<223> MYBPC1_forward primer

<400> 84
tcccagctgt ccatctt 17

<210> 85
<211> 20
<212> DNA
<213> Artificial Sequence

<220>
<223> MYCBPC1

<400> 85
cagccttgac tttggctatg 20

<210> 86
<211> 26
<212> DNA
<213> Artificial Sequence

<220>
<223> MYCBPC1_reverse primer

<400> 86
caaggaggaa cagtgaaagt tggtga 26

<210> 87
<211> 563
<212> DNA
<213> Homo sapiens

<400> 87

```
gccacttctc ttcccttcat tcttcgccag gctctctgct gactcaagtt cttcagttca     60
cgatcttcta gttgcagcga tgagtgcacg agtgagatca agatccagag gaagaggaga    120
tggtcaggag gctcccgatg tggttgcatt cgtggctccc ggtgaatctc agcaagagga    180
accaccaact gacaatcagg atattgaacc tggacaagag agagaaggaa cacctccgat    240
cgaagaacgt aaagtagaag gtgattgcca ggaaatggat ctggaaaaga ctcggagtga    300
gcgtggagat ggctctgatg taaaagagaa gactccacct aatcctaagc atgctaagac    360
taaagaagca ggagatgggc agccataagt taaaaagaag acaagctgaa gctacacaca    420
tggctgatgt cacattgaaa atgtgactga aaatttgaaa attctctcaa taaagtttga    480
gttttctctg aagaagtcat gcgtgtcttt tgtaaaattt attcctagga aattgacact    540
attgtaaatg gtatctttaa aaa                                            563
```

<210> 88
<211> 102
<212> PRT
<213> Homo sapiens

<400> 88

```
Met Ser Ala Arg Val Arg Ser Arg Ser Arg Gly Arg Gly Asp Gly Gln
1               5                   10                  15

Glu Ala Pro Asp Val Val Ala Phe Val Ala Pro Gly Glu Ser Gln Gln
            20                  25                  30

Glu Glu Pro Pro Thr Asp Asn Gln Asp Ile Glu Pro Gly Gln Glu Arg
        35                  40                  45

Glu Gly Thr Pro Pro Ile Glu Glu Arg Lys Val Glu Gly Asp Cys Gln
    50                  55                  60

Glu Met Asp Leu Glu Lys Thr Arg Ser Glu Arg Gly Asp Gly Ser Asp
65                  70                  75                  80

Val Lys Glu Lys Thr Pro Pro Asn Pro Lys His Ala Lys Thr Lys Glu
                85                  90                  95

Ala Gly Asp Gly Gln Pro
            100
```

<210> 89
<211> 17
<212> DNA
<213> Artificial Sequence

<220>
<223> PAGE4_forward primer

<400> 89
gaaggaacac ctccgat 17

<210> 90
<211> 20
<212> DNA
<213> Artificial Sequence

<220>
<223> PAG1_reverse primer

<400> 90
catctccacg ctcactccga 20

<210> 91
<211> 25
<212> DNA
<213> Artificial Sequence

<220>
<223> PAGE1 probe

<400> 91
aggtgattgc caggaaatgg atctg 25

<210> 92
<211> 2446
<212> DNA
<213> Homo sapiens

<400> 92

tccataaagg ggttgcgggg gccgcgctct cttctgggag ggcagcggcc accggcgagg 60

aacacggcgc gatgcaggtt cagtgccagc agagcccagt gctggcaggc agcgccactt 120

tggtcgccct tggggcactg gccttgtacg tcgcgaagcc ctccggctac gggaagcaca 180

cggagagcct gaagccggcg gctacccgcc tgccagcccg cgccgcctgg ttcctgcagg 240

agctgccttc cttcgcggtg cccgcgggga tcctcgcccg gcagcccctc tccctcttcg 300

ggccacctgg gacggtactt ctgggcctct tctgcctaca ttacttccac aggacatttg 360

tgtactcact gctcaatcga gggaggcctt atccagctat actcattctc agaggcactg 420

ccttctgcac tggaaatgga gtccttcaag gctactatct gatttactgt gctgaatacc 480

ctgatgggtg gtacacagac atacggttta gcttgggtgt cttcttattt attttgggaa 540

tgggaataaa cattcatagt gactatatat tgcgccagct caggaagcct ggagaaatca 600

gctacaggat tccacaaggt ggcttgttta cgtatgtttc tggagccaat ttcctcggtg 660

agatcattga atggatcggc tatgccctgg ccacttggtc cctcccagca cttgcatttg 720

catttttctc actttgtttc cttgggctgc gagcttttca ccaccatagg ttctacctca 780

agatgtttga ggactacccc aaatctcgga aagcccttat tccattcatc ttttaaagga 840

accaaattaa aaaggagcag agctcccaca atgctgatga aaactgtcaa gctgctgaaa 900

ctgtaatttt catgatataa tagtcccgta tatatgtaat agtaggtctc ctggcgttct 960

gccagctggc ctggggattc tgagtggtgt ctgcttagag tttactccta cccttccagg 1020

gacccctatc ctgatcccca actgaagctt caaaaagcca cttttccaaa tggcgacagt 1080

tgcttcttag ctattgctct gagaaagtac aaacttctcc tatgtctttc accgggcaat 1140

ccaagtacat gtggcttcat acccactccc tgtcaatgca ggacaactct gtaatcaaga 1200

attttttgac ttgaaggcag tacttataga ccttattaaa ggtatgcatt ttatacatgt 1260

aacagagtag cagaaattta aactctgaag ccacaaagac ccagagcaaa cccactccca 1320

aatgaaaacc ccagtcatgg cttccttttt cttggttaat taggaaagat gagaaattat 1380

taggtagacc ttgaatacag gagccctctc ctcatagtgc tgaaaagata ctgatgcatt 1440

gacctcattt caaatttgtg cagtgtctta gttgatgagt gcctctgttt tccagaagat 1500

ttcacaatcc ccggaaaact ggtatggcta ttcttgaagg ccaggtttta ataaccacaa 1560

acaaaaaggc atgaacctgg gtggcttatg agagagtaga gaacaacatg accctggatg 1620

gctactaaga ggatagagaa cagttttaca atagacattg caaactctca tgtttttgga 1680

aactagtggc aatatccaaa taatgagtag tgtaaaacaa agagaattaa tgatgaggtt 1740

acatgctgct tgcctccacc agatgtccac aacaatatga agtacagcag aagccccaag 1800

caactttcct ttcctggagc ttcttccttg tagttctcag gacctgttca agaaggtgtc 1860

tcctaggggc agcctgaatg cctccctcaa aggacctgca ggcagagact gaaaattgca 1920

gacagagggg cacgtctggg cagaaaacct gttttgtttg gctcagacat atagtttttt 1980

ttttttttac aaagtttcaa aaacttaaaa atcaggagat tccttcataa aactctagca 2040

ttctagtttc atttaaaaag ttggaggatc tgaacataca gagcccacat ttccacacca 2100

gaactggaac tacgtagcta gtaagcattt gagtttgcaa actcttgtga aggggtcacc 2160

ccagcatgag tgctgagata tggactctct aaggaagggg ccgaacgctt gtaattggaa 2220

tacatggaaa tatttgtctt ctcaggccta tgtttgcgga atgcattgtc aatatttagc 2280

aaactgtttt gacaaatgag caccagtggt actaagcaca gaaactcact atataagtca 2340

cataggaaac ttgaaaggtc tgaggatgat gtagattact gaaaaatgca aattgcaatc 2400

atataaataa gtgtttttgt tgttcattaa atacctttaa atcatg 2446

<210> 93
<211> 254
<212> PRT
<213> Homo sapiens

<400> 93

Met Gln Val Gln Cys Gln Gln Ser Pro Val Leu Ala Gly Ser Ala Thr
1               5                   10                  15

Leu Val Ala Leu Gly Ala Leu Ala Leu Tyr Val Ala Lys Pro Ser Gly
            20                  25                  30

Tyr Gly Lys His Thr Glu Ser Leu Lys Pro Ala Ala Thr Arg Leu Pro
        35                  40                  45

Ala Arg Ala Ala Trp Phe Leu Gln Glu Leu Pro Ser Phe Ala Val Pro
    50                  55                  60

Ala Gly Ile Leu Ala Arg Gln Pro Leu Ser Leu Phe Gly Pro Pro Gly
65                  70                  75                  80

Thr Val Leu Leu Gly Leu Phe Cys Leu His Tyr Phe His Arg Thr Phe
                85                  90                  95

Val Tyr Ser Leu Leu Asn Arg Gly Arg Pro Tyr Pro Ala Ile Leu Ile
            100                 105                 110

Leu Arg Gly Thr Ala Phe Cys Thr Gly Asn Gly Val Leu Gln Gly Tyr
115 120 125

Tyr Leu Ile Tyr Cys Ala Glu Tyr Pro Asp Gly Trp Tyr Thr Asp Ile
130 135 140

Arg Phe Ser Leu Gly Val Phe Leu Phe Ile Leu Gly Met Gly Ile Asn
145 150 155 160

Ile His Ser Asp Tyr Ile Leu Arg Gln Leu Arg Lys Pro Gly Glu Ile
165 170 175

Ser Tyr Arg Ile Pro Gln Gly Gly Leu Phe Thr Tyr Val Ser Gly Ala
180 185 190

Asn Phe Leu Gly Glu Ile Ile Glu Trp Ile Gly Tyr Ala Leu Ala Thr
195 200 205

Trp Ser Leu Pro Ala Leu Ala Phe Ala Phe Phe Ser Leu Cys Phe Leu
210 215 220

Gly Leu Arg Ala Phe His His His Arg Phe Tyr Leu Lys Met Phe Glu
225 230 235 240

Asp Tyr Pro Lys Ser Arg Lys Ala Leu Ile Pro Phe Ile Phe
245 250

<210> 94
<211> 21
<212> DNA
<213> Artificial Sequence

<220>
<223> SRD5A2_forward primer

<400> 94
ggcctcttct gcctacatta c 21

<210> 95
<211> 24
<212> DNA
<213> Artificial Sequence

<220>
<223> SRD5A2_reverse primer

<400> 95
cagtgcctct gagaatgagt atag 24

<210> 96
<211> 22
<212> DNA

<213> Artificial Sequence

<220>
<223> SRD5A2

<400> 96
ctcactgctc aatcgaggga gg 22

<210> 97
<211> 5927
<212> DNA
<213> Homo sapiens

<400> 97

```
tcgtcggagc agacgggagt ttctcctcgg ggtcggagca ggaggcacgc ggagtgtgag      60
gccacgcatg agcggacgct aaccccctcc ccagccacaa agagtctaca tgtctagggt     120
ctagacatgt tcagctttgt ggacctccgg ctcctgctcc tcttagcggc caccgccctc     180
ctgacgcacg gccaagagga aggccaagtc gagggccaag acgaagacat cccaccaatc     240
acctgcgtac agaacggcct caggtaccat gaccgagacg tgtggaaacc cgagccctgc     300
cggatctgcg tctgcgacaa cggcaaggtg ttgtgcgatg acgtgatctg tgacgagacc     360
aagaactgcc ccggcgccga agtccccgag ggcgagtgct gtcccgtctg ccccgacggc     420
tcagagtcac ccaccgacca agaaaccacc ggcgtcgagg gacccaaggg agacactggc     480
ccccgaggcc caaggggacc cgcaggcccc cctggccgag atggcatccc tggacagcct     540
ggacttcccg gacccccegg acccccegga cctcccggac cccctggcct cggaggaaac     600
tttgctcccc agctgtctta tggctatgat gagaaatcaa ccggaggaat ttccgtgcct     660
ggccccatgg gtccctctgg tcctcgtggt ctccctggcc cccctggtgc acctggtccc     720
caaggcttcc aaggtccccc tggtgagcct ggcgagcctg gagcttcagg tcccatgggt     780
ccccgaggtc ccccaggtcc ccctggaaag aatggagatg atggggaagc tggaaaacct     840
ggtcgtcctg gtgagcgtgg gcctcctggg cctcagggtg ctcgaggatt gcccggaaca     900
gctggcctcc ctggaatgaa gggacacaga ggtttcagtg gtttggatgg tgccaaggga     960
gatgctggtc ctgctggtcc taagggtgag cctggcagcc ctggtgaaaa tggagctcct    1020
ggtcagatgg gcccccgtgg cctgcctggt gagagaggtc gccctggagc ccctggccct    1080
gctggtgctc gtggaaatga tggtgctact ggtgctgccg ggccccctgg tcccaccggc    1140
cccgctggtc ctcctggctt ccctggtgct gttggtgcta agggtgaagc tggtccccaa    1200
gggccccgag gctctgaagg tccccagggt gtgcgtggtg agcctggccc ccctggccct    1260
gctggtgctg ctggccctgc tggaaacect ggtgctgatg gacagcctgg tgctaaaggt    1320
gccaatggtg ctcctggtat tgctggtgct cctggcttcc ctggtgcccg aggcccctct    1380
ggaccccagg gccccggcgg ccctcctggt cccaagggta acagcggtga acctggtgct    1440
```

cctggcagca aaggagacac tggtgctaag ggagagcctg gccctgttgg tgttcaagga 1500
ccccctggcc ctgctggaga ggaaggaaag cgaggagctc gaggtgaacc cggacccact 1560
ggcctgcccg gaccccctgg cgagcgtggt ggacctggta gccgtggttt ccctggcgca 1620
gatggtgttg ctggtcccaa gggtcccgct ggtgaacgtg gttctcctgg ccctgctggc 1680
cccaaaggat ctcctggtga agctggtcgt cccggtgaag ctggtctgcc tggtgccaag 1740
ggtctgactg gaagccctgg cagccctggt cctgatggca aaactggccc ccctggtccc 1800
gccggtcaag atggtcgccc cggaccccca ggcccacctg gtgcccgtgg tcaggctggt 1860
gtgatgggat tccctggacc taaaggtgct gctggagagc ccggcaaggc tggagagcga 1920
ggtgttcccg gaccccctgg cgctgtcggt cctgctggca aagatggaga ggctggagct 1980
cagggacccc ctggccctgc tggtcccgct ggcgagagag gtgaacaagg ccctgctggc 2040
tcccccggat tccagggtct ccctggtcct gctggtcctc caggtgaagc aggcaaacct 2100
ggtgaacagg gtgttcctgg agaccttggc gcccctggcc cctctggagc aagaggcgag 2160
agaggtttcc ctggcgagcg tggtgtgcaa ggtccccctg gtcctgctgg tccccgaggg 2220
gccaacggtg ctcccggcaa cgatggtgct aagggtgatg ctggtgcccc tggagctccc 2280
ggtagccagg gcgcccctgg ccttcaggga atgcctggtg aacgtggtgc agctggtctt 2340
ccagggccta agggtgacag aggtgatgct ggtcccaaag gtgctgatgg ctctcctggc 2400
aaagatggcg tccgtggtct gactggcccc attggtcctc ctggccctgc tggtgcccct 2460
ggtgacaagg gtgaaagtgg tcccagcggc cctgctggtc ccactggagc tcgtggtgcc 2520
cccggagacc gtggtgagcc tggtcccccc ggccctgctg gctttgctgg ccccccctggt 2580
gctgacggcc aacctggtgc taaaggcgaa cctggtgatg ctggtgctaa aggcgatgct 2640
ggtccccctg gccctgccgg acccgctgga ccccctggcc ccattggtaa tgttggtgct 2700
cctggagcca aaggtgctcg cggcagcgct ggtccccctg gtgctactgg tttccctggt 2760
gctgctggcc gagtcggtcc tcctggcccc tctggaaatg ctggaccccc tggccctcct 2820
ggtcctgctg gcaaagaagg cggcaaaggt ccccgtggtg agactggccc tgctggacgt 2880
cctggtgaag ttggtccccc tggtccccct ggccctgctg gcgagaaagg atcccctggt 2940
gctgatggtc ctgctggtgc tcctggtact cccgggcctc aaggtattgc tggacagcgt 3000
ggtgtggtcg gcctgcctgg tcagagagga gagagaggct tccctggtct tcctggcccc 3060
tctggtgaac ctggcaaaca aggtccctct ggagcaagtg gtgaacgtgg tccccctggt 3120
cccatgggcc cccctggatt ggctggaccc cctggtgaat ctggacgtga gggggctcct 3180
ggtgccgaag gttcccctgg acgagacggt tctcctggcg ccaagggtga ccgtggtgag 3240
accggccccg ctggaccccc tggtgctcct ggtgctcctg gtgcccctgg ccccgttggc 3300
cctgctggca agagtggtga tcgtggtgag actggtcctg ctggtcccgc cggtcctgtc 3360

ggccctgttg gcgcccgtgg ccccgccgga ccccaaggcc cccgtggtga caagggtgag 3420

acaggcgaac agggcgacag aggcataaag ggtcaccgtg gcttctctgg cctccagggt 3480

cccccctggcc ctcctggctc tcctggtgaa caaggtccct ctggagcctc tggtcctgct 3540

ggtccccgag gtccccctgg ctctgctggt gctcctggca aagatggact caacggtctc 3600

cctggcccca ttgggccccc tggtcctcgc ggtcgcactg gtgatgctgg tcctgttggt 3660

ccccccggcc ctcctggacc tcctggtccc cctggtcctc ccagcgctgg tttcgacttc 3720

agcttcctgc cccagccacc tcaagagaag gctcacgatg gtggccgcta ctaccgggct 3780

gatgatgcca atgtggttcg tgaccgtgac ctcgaggtgg acaccaccct caagagcctg 3840

agccagcaga tcgagaacat ccggagccca gagggcagcc gcaagaaccc cgcccgcacc 3900

tgccgtgacc tcaagatgtg ccactctgac tggaagagtg gagagtactg gattgacccc 3960

aaccaaggct gcaacctgga tgccatcaaa gtcttctgca acatggagac tggtgagacc 4020

tgcgtgtacc ccactcagcc cagtgtggcc cagaagaact ggtacatcag caagaacccc 4080

aaggacaaga ggcatgtctg gttcggcgag agcatgaccg atggattcca gttcgagtat 4140

ggcggccagg gctccgaccc tgccgatgtg gccatccagc tgaccttcct gcgcctgatg 4200

tccaccgagg cctcccagaa catcacctac cactgcaaga acagcgtggc ctacatggac 4260

cagcagactg gcaacctcaa gaaggccctg ctcctccagg gctccaacga gatcgagatc 4320

cgcgccgagg gcaacagccg cttcacctac agcgtcactg tcgatggctg cacgagtcac 4380

accggagcct ggggcaagac agtgattgaa tacaaaacca ccaagacctc ccgcctgccc 4440

atcatcgatg tggccccctt ggacgttggt gccccagacc aggaattcgg cttcgacgtt 4500

ggccctgtct gcttcctgta aactccctcc atcccaacct ggctccctcc cacccaacca 4560

actttccccc caacccggaa acagacaagc aacccaaact gaaccccctc aaaagccaaa 4620

aaatgggaga caatttcaca tggactttgg aaaatatttt tttcctttgc attcatctct 4680

caaacttagt ttttatcttt gaccaaccga acatgaccaa aaaccaaaag tgcattcaac 4740

cttaccaaaa aaaaaaaaaa aaaaagaata aataaataac tttttaaaaa aggaagcttg 4800

gtccacttgc ttgaagaccc atgcgggggt aagtcccttt ctgcccgttg ggcttatgaa 4860

accccaatgc tgccctttct gctcctttct ccacaccccc cttggggcct cccctccact 4920

ccttcccaaa tctgtctccc cagaagacac aggaaacaat gtattgtctg cccagcaatc 4980

aaaggcaatg ctcaaacacc caagtggccc ccaccctcag cccgctcctg cccgcccagc 5040

acccccaggc cctgggggac ctggggttct cagactgcca aagaagcctt gccatctggc 5100

gctcccatgg ctcttgcaac atctcccctt cgtttttgag gggtcatgc cgggggagcc 5160

accagcccct cactgggttc ggaggagagt caggaagggc cacgacaaag cagaaacatc 5220

```
ggatttgggg aacgcgtgtc aatcccttgt gccgcagggc tgggcgggag agactgttct      5280
gttccttgtg taactgtgtt gctgaaagac tacctcgttc ttgtcttgat gtgtcaccgg      5340
ggcaactgcc tgggggcggg gatgggggca gggtggaagc ggctccccat tttataccaa      5400
aggtgctaca tctatgtgat gggtggggtg gggagggaat cactggtgct atagaaattg      5460
agatgccccc ccaggccagc aaatgttcct ttttgttcaa agtctatttt tattccttga      5520
tatttttctt tttttttttt tttttttgtg gatggggact tgtgaatttt tctaaaggtg      5580
ctatttaaca tgggaggaga gcgtgtgcgg ctccagccca gcccgctgct cactttccac      5640
cctctctcca cctgcctctg gcttctcagg cctctgctct ccgacctctc tcctctgaaa      5700
ccctcctcca cagctgcagc ccatcctccc ggctccctcc tagtctgtcc tgcgtcctct      5760
gtccccgggt ttcagagaca acttcccaaa gcacaaagca gtttttcccc ctaggggtgg      5820
gaggaagcaa aagactctgt acctattttg tatgtgtata ataatttgag atgtttttaa      5880
ttattttgat tgctggaata aagcatgtgg aaatgaccca aacataa                     5927
```

<210> 98
<211> 1464
<212> PRT
<213> Homo sapiens

<400> 98

```
Met Phe Ser Phe Val Asp Leu Arg Leu Leu Leu Leu Leu Ala Ala Thr
1               5                   10                  15

Ala Leu Leu Thr His Gly Gln Glu Glu Gly Gln Val Glu Gly Gln Asp
            20                  25                  30

Glu Asp Ile Pro Pro Ile Thr Cys Val Gln Asn Gly Leu Arg Tyr His
        35                  40                  45

Asp Arg Asp Val Trp Lys Pro Glu Pro Cys Arg Ile Cys Val Cys Asp
    50                  55                  60

Asn Gly Lys Val Leu Cys Asp Asp Val Ile Cys Asp Glu Thr Lys Asn
65                  70                  75                  80

Cys Pro Gly Ala Glu Val Pro Glu Gly Glu Cys Cys Pro Val Cys Pro
                85                  90                  95

Asp Gly Ser Glu Ser Pro Thr Asp Gln Glu Thr Thr Gly Val Glu Gly
            100                 105                 110

Pro Lys Gly Asp Thr Gly Pro Arg Gly Pro Arg Gly Pro Ala Gly Pro
        115                 120                 125
```

```
Pro Gly Arg Asp Gly Ile Pro Gly Gln Pro Gly Leu Pro Gly Pro Pro
    130                 135                 140

Gly Pro Pro Gly Pro Pro Gly Pro Pro Gly Leu Gly Gly Asn Phe Ala
145                 150                 155                 160

Pro Gln Leu Ser Tyr Gly Tyr Asp Glu Lys Ser Thr Gly Gly Ile Ser
                165                 170                 175

Val Pro Gly Pro Met Gly Pro Ser Gly Pro Arg Gly Leu Pro Gly Pro
            180                 185                 190

Pro Gly Ala Pro Gly Pro Gln Gly Phe Gln Gly Pro Pro Gly Glu Pro
        195                 200                 205

Gly Glu Pro Gly Ala Ser Gly Pro Met Gly Pro Arg Gly Pro Pro Gly
    210                 215                 220

Pro Pro Gly Lys Asn Gly Asp Asp Gly Glu Ala Gly Lys Pro Gly Arg
225                 230                 235                 240

Pro Gly Glu Arg Gly Pro Pro Gly Pro Gln Gly Ala Arg Gly Leu Pro
                245                 250                 255

Gly Thr Ala Gly Leu Pro Gly Met Lys Gly His Arg Gly Phe Ser Gly
            260                 265                 270

Leu Asp Gly Ala Lys Gly Asp Ala Gly Pro Ala Gly Pro Lys Gly Glu
        275                 280                 285

Pro Gly Ser Pro Gly Glu Asn Gly Ala Pro Gly Gln Met Gly Pro Arg
    290                 295                 300

Gly Leu Pro Gly Glu Arg Gly Arg Pro Gly Ala Pro Gly Pro Ala Gly
305                 310                 315                 320

Ala Arg Gly Asn Asp Gly Ala Thr Gly Ala Ala Gly Pro Pro Gly Pro
                325                 330                 335

Thr Gly Pro Ala Gly Pro Pro Gly Phe Pro Gly Ala Val Gly Ala Lys
            340                 345                 350

Gly Glu Ala Gly Pro Gln Gly Pro Arg Gly Ser Glu Gly Pro Gln Gly
        355                 360                 365

Val Arg Gly Glu Pro Gly Pro Pro Gly Pro Ala Gly Ala Ala Gly Pro
    370                 375                 380
```

```
Ala Gly Asn Pro Gly Ala Asp Gly Gln Pro Gly Ala Lys Gly Ala Asn
385                 390                 395                 400

Gly Ala Pro Gly Ile Ala Gly Ala Pro Gly Phe Pro Gly Ala Arg Gly
                405                 410                 415

Pro Ser Gly Pro Gln Gly Pro Gly Gly Pro Pro Gly Pro Lys Gly Asn
            420                 425                 430

Ser Gly Glu Pro Gly Ala Pro Gly Ser Lys Gly Asp Thr Gly Ala Lys
        435                 440                 445

Gly Glu Pro Gly Pro Val Gly Val Gln Gly Pro Pro Gly Pro Ala Gly
    450                 455                 460

Glu Glu Gly Lys Arg Gly Ala Arg Gly Glu Pro Gly Pro Thr Gly Leu
465                 470                 475                 480

Pro Gly Pro Pro Gly Glu Arg Gly Gly Pro Gly Ser Arg Gly Phe Pro
                485                 490                 495

Gly Ala Asp Gly Val Ala Gly Pro Lys Gly Pro Ala Gly Glu Arg Gly
            500                 505                 510

Ser Pro Gly Pro Ala Gly Pro Lys Gly Ser Pro Gly Glu Ala Gly Arg
        515                 520                 525

Pro Gly Glu Ala Gly Leu Pro Gly Ala Lys Gly Leu Thr Gly Ser Pro
    530                 535                 540

Gly Ser Pro Gly Pro Asp Gly Lys Thr Gly Pro Pro Gly Pro Ala Gly
545                 550                 555                 560

Gln Asp Gly Arg Pro Gly Pro Pro Gly Pro Pro Gly Ala Arg Gly Gln
                565                 570                 575

Ala Gly Val Met Gly Phe Pro Gly Pro Lys Gly Ala Ala Gly Glu Pro
            580                 585                 590

Gly Lys Ala Gly Glu Arg Gly Val Pro Gly Pro Pro Gly Ala Val Gly
        595                 600                 605

Pro Ala Gly Lys Asp Gly Glu Ala Gly Ala Gln Gly Pro Pro Gly Pro
    610                 615                 620

Ala Gly Pro Ala Gly Glu Arg Gly Glu Gln Gly Pro Ala Gly Ser Pro
```

625 630 635 640

Gly Phe Gln Gly Leu Pro Gly Pro Ala Gly Pro Pro Gly Glu Ala Gly
645 650 655

Lys Pro Gly Glu Gln Gly Val Pro Gly Asp Leu Gly Ala Pro Gly Pro
660 665 670

Ser Gly Ala Arg Gly Glu Arg Gly Phe Pro Gly Glu Arg Gly Val Gln
675 680 685

Gly Pro Pro Gly Pro Ala Gly Pro Arg Gly Ala Asn Gly Ala Pro Gly
690 695 700

Asn Asp Gly Ala Lys Gly Asp Ala Gly Ala Pro Gly Ala Pro Gly Ser
705 710 715 720

Gln Gly Ala Pro Gly Leu Gln Gly Met Pro Gly Glu Arg Gly Ala Ala
725 730 735

Gly Leu Pro Gly Pro Lys Gly Asp Arg Gly Asp Ala Gly Pro Lys Gly
740 745 750

Ala Asp Gly Ser Pro Gly Lys Asp Gly Val Arg Gly Leu Thr Gly Pro
755 760 765

Ile Gly Pro Pro Gly Pro Ala Gly Ala Pro Gly Asp Lys Gly Glu Ser
770 775 780

Gly Pro Ser Gly Pro Ala Gly Pro Thr Gly Ala Arg Gly Ala Pro Gly
785 790 795 800

Asp Arg Gly Glu Pro Gly Pro Pro Gly Pro Ala Gly Phe Ala Gly Pro
805 810 815

Pro Gly Ala Asp Gly Gln Pro Gly Ala Lys Gly Glu Pro Gly Asp Ala
820 825 830

Gly Ala Lys Gly Asp Ala Gly Pro Pro Gly Pro Ala Gly Pro Ala Gly
835 840 845

Pro Pro Gly Pro Ile Gly Asn Val Gly Ala Pro Gly Ala Lys Gly Ala
850 855 860

Arg Gly Ser Ala Gly Pro Pro Gly Ala Thr Gly Phe Pro Gly Ala Ala
865 870 875 880

Gly Arg Val Gly Pro Pro Gly Pro Ser Gly Asn Ala Gly Pro Pro Gly
885 890 895

Pro Pro Gly Pro Ala Gly Lys Glu Gly Gly Lys Gly Pro Arg Gly Glu
900 905 910

Thr Gly Pro Ala Gly Arg Pro Gly Glu Val Gly Pro Pro Gly Pro Pro
915 920 925

Gly Pro Ala Gly Glu Lys Gly Ser Pro Gly Ala Asp Gly Pro Ala Gly
930 935 940

Ala Pro Gly Thr Pro Gly Pro Gln Gly Ile Ala Gly Gln Arg Gly Val
945 950 955 960

Val Gly Leu Pro Gly Gln Arg Gly Glu Arg Gly Phe Pro Gly Leu Pro
965 970 975

Gly Pro Ser Gly Glu Pro Gly Lys Gln Gly Pro Ser Gly Ala Ser Gly
980 985 990

Glu Arg Gly Pro Pro Gly Pro Met Gly Pro Pro Gly Leu Ala Gly Pro
995 1000 1005

Pro Gly Glu Ser Gly Arg Glu Gly Ala Pro Gly Ala Glu Gly Ser
1010 1015 1020

Pro Gly Arg Asp Gly Ser Pro Gly Ala Lys Gly Asp Arg Gly Glu
1025 1030 1035

Thr Gly Pro Ala Gly Pro Pro Gly Ala Pro Gly Ala Pro Gly Ala
1040 1045 1050

Pro Gly Pro Val Gly Pro Ala Gly Lys Ser Gly Asp Arg Gly Glu
1055 1060 1065

Thr Gly Pro Ala Gly Pro Ala Gly Pro Val Gly Pro Val Gly Ala
1070 1075 1080

Arg Gly Pro Ala Gly Pro Gln Gly Pro Arg Gly Asp Lys Gly Glu
1085 1090 1095

Thr Gly Glu Gln Gly Asp Arg Gly Ile Lys Gly His Arg Gly Phe
1100 1105 1110

Ser Gly Leu Gln Gly Pro Pro Gly Pro Pro Gly Ser Pro Gly Glu
1115 1120 1125

```
Gln Gly  Pro Ser Gly Ala Ser  Gly Pro Ala Gly Pro  Arg Gly Pro
    1130                 1135                 1140

Pro Gly  Ser Ala Gly Ala Pro  Gly Lys Asp Gly Leu  Asn Gly Leu
    1145                 1150                 1155

Pro Gly  Pro Ile Gly Pro Pro  Gly Pro Arg Gly Arg  Thr Gly Asp
    1160                 1165                 1170

Ala Gly  Pro Val Gly Pro Pro  Gly Pro Pro Gly Pro  Pro Gly Pro
    1175                 1180                 1185

Pro Gly  Pro Pro Ser Ala Gly  Phe Asp Phe Ser Phe  Leu Pro Gln
    1190                 1195                 1200

Pro Pro  Gln Glu Lys Ala His  Asp Gly Gly Arg Tyr  Tyr Arg Ala
    1205                 1210                 1215

Asp Asp  Ala Asn Val Val Arg  Asp Arg Asp Leu Glu  Val Asp Thr
    1220                 1225                 1230

Thr Leu  Lys Ser Leu Ser Gln  Gln Ile Glu Asn Ile  Arg Ser Pro
    1235                 1240                 1245

Glu Gly  Ser Arg Lys Asn Pro  Ala Arg Thr Cys Arg  Asp Leu Lys
    1250                 1255                 1260

Met Cys  His Ser Asp Trp Lys  Ser Gly Glu Tyr Trp  Ile Asp Pro
    1265                 1270                 1275

Asn Gln  Gly Cys Asn Leu Asp  Ala Ile Lys Val Phe  Cys Asn Met
    1280                 1285                 1290

Glu Thr  Gly Glu Thr Cys Val  Tyr Pro Thr Gln Pro  Ser Val Ala
    1295                 1300                 1305

Gln Lys  Asn Trp Tyr Ile Ser  Lys Asn Pro Lys Asp  Lys Arg His
    1310                 1315                 1320

Val Trp  Phe Gly Glu Ser Met  Thr Asp Gly Phe Gln  Phe Glu Tyr
    1325                 1330                 1335

Gly Gly  Gln Gly Ser Asp Pro  Ala Asp Val Ala Ile  Gln Leu Thr
    1340                 1345                 1350

Phe Leu  Arg Leu Met Ser Thr  Glu Ala Ser Gln Asn  Ile Thr Tyr
    1355                 1360                 1365
```

```
His Cys Lys Asn Ser Val Ala Tyr Met Asp Gln Gln Thr Gly Asn
    1370                1375                1380

Leu Lys Lys Ala Leu Leu Leu Gln Gly Ser Asn Glu Ile Glu Ile
    1385                1390                1395

Arg Ala Glu Gly Asn Ser Arg Phe Thr Tyr Ser Val Thr Val Asp
    1400                1405                1410

Gly Cys Thr Ser His Thr Gly Ala Trp Gly Lys Thr Val Ile Glu
    1415                1420                1425

Tyr Lys Thr Thr Lys Thr Ser Arg Leu Pro Ile Ile Asp Val Ala
    1430                1435                1440

Pro Leu Asp Val Gly Ala Pro Asp Gln Glu Phe Gly Phe Asp Val
    1445                1450                1455

Gly Pro Val Cys Phe Leu
    1460
```

<210> 99
<211> 20
<212> DNA
<213> Artificial Sequence

<220>
<223> COL1A1_forward primer

<400> 99
gaagacatcc caccaatcac 20

<210> 100
<211> 18
<212> DNA
<213> Artificial Sequence

<220>
<223> COL1A1_reverse primer

<400> 100
cgggtttcca cacgtctc 18

<210> 101
<211> 24
<212> DNA
<213> Artificial Sequence

<220>
<223> COL1A1 probe

<400> 101
ctgcgtacag aacggcctca ggta 24

<210> 102
<211> 5490
<212> DNA
<213> Homo sapiens

<400> 102

```
ggctgagttt tatgacgggc ccggtgctga agggcaggga acaacttgat ggtgctactt      60
tgaactgctt ttcttttctc ctttttgcac aaagagtctc atgtctgata tttagacatg     120
atgagctttg tgcaaaaggg gagctggcta cttctcgctc tgcttcatcc cactattatt     180
ttggcacaac aggaagctgt tgaaggagga tgttcccatc ttggtcagtc ctatgcggat     240
agagatgtct ggaagccaga accatgccaa atatgtgtct gtgactcagg atccgttctc     300
tgcgatgaca taatatgtga cgatcaagaa ttagactgcc ccaacccaga aattccattt     360
ggagaatgtt gtgcagtttg cccacagcct ccaactgctc ctactcgccc tcctaatggt     420
caaggacctc aaggccccaa gggagatcca ggccctcctg gtattcctgg gagaaatggt     480
gaccctggta ttccaggaca accagggtcc cctggttctc ctggcccccc tggaatctgt     540
gaatcatgcc ctactggtcc tcagaactat tctccccagt atgattcata tgatgtcaag     600
tctggagtag cagtaggagg actcgcaggc tatcctggac cagctggccc cccaggccct     660
cccggtcccc ctggtacatc tggtcatcct ggttcccctg gatctccagg ataccaagga     720
ccccctggtg aacctgggca agctggtcct tcaggccctc caggacctcc tggtgctata     780
ggtccatctg gtcctgctgg aaaagatgga gaatcaggta gacccggacg acctggagag     840
cgaggattgc ctggacctcc aggtatcaaa ggtccagctg ggatacctgg attccctggt     900
atgaaaggac acagaggctt cgatggacga aatggagaaa agggtgaaac aggtgctcct     960
ggattaaagg gtgaaaatgg tcttccaggc gaaaatggag ctcctggacc catgggtcca    1020
agaggggctc ctggtgagcg aggacggcca ggacttcctg gggctgcagg tgctcggggt    1080
aatgacggtg ctcgaggcag tgatggtcaa ccaggccctc ctggtcctcc tggaactgcc    1140
ggattccctg gatcccctgg tgctaagggt gaagttggac ctgcagggtc tcctggttca    1200
aatggtgccc ctggacaaag aggagaacct ggacctcagg gacacgctgg tgctcaaggt    1260
cctcctggcc ctcctgggat taatggtagt cctggtggta aaggcgaaat gggtcccgct    1320
ggcattcctg gagctcctgg actgatggga gcccggggtc ctccaggacc agccggtgct    1380
aatggtgctc ctggactgcg aggtggtgca ggtgagcctg gtaagaatgg tgccaaagga    1440
gagcccggac cacgtggtga acgcggtgag gctggtattc caggtgttcc aggagctaaa    1500
ggcgaagatg gcaaggatgg atcacctgga gaacctggtg caaatgggct tccaggagct    1560
gcaggagaaa ggggtgcccc tgggttccga ggacctgctg gaccaaatgg catcccagga    1620
```

gaaaagggtc ctgctggaga gcgtggtgct ccaggccctg cagggcccag aggagctgct 1680
ggagaacctg gcagagatgg cgtccctgga ggtccaggaa tgaggggcat gcccggaagt 1740
ccaggaggac caggaagtga tgggaaacca gggcctcccg gaagtcaagg agaaagtggt 1800
cgaccaggtc ctcctgggcc atctggtccc cgaggtcagc ctggtgtcat gggcttcccc 1860
ggtcctaaag gaaatgatgg tgctcctggt aagaatggag aacgaggtgg ccctggagga 1920
cctggccctc agggtcctcc tggaaagaat ggtgaaactg gacctcaggg acccccaggg 1980
cctactgggc ctggtggtga caaaggagac acaggacccc ctggtccaca aggattacaa 2040
ggcttgcctg gtacaggtgg tcctccagga gaaaatggaa aacctgggga accaggtcca 2100
aagggtgatg ccggtgcacc tggagctcca ggaggcaagg gtgatgctgg tgcccctggt 2160
gaacgtggac ctcctggatt ggcaggggcc ccaggactta gaggtggagc tggtccccct 2220
ggtcccgaag gaggaaaggg tgctgctggt cctcctgggc cacctggtgc tgctggtact 2280
cctggtctgc aaggaatgcc tggagaaaga ggaggtcttg gaagtcctgg tccaaagggt 2340
gacaagggtg aaccaggcgg tccaggtgct gatggtgtcc cagggaaaga tggcccaagg 2400
ggtcctactg gtcctattgg tcctcctggc ccagctggcc agcctggaga taagggtgaa 2460
ggtggtgccc ccggacttcc aggtatagct ggacctcgtg gtagccctgg tgagagaggt 2520
gaaactggcc ctccaggacc tgctggtttc cctggtgctc ctggacagaa tggtgaacct 2580
ggtggtaaag gagaaagagg ggctccgggt gagaaaggtg aaggaggccc tcctggagtt 2640
gcaggacccc ctggaggttc tggacctgct ggtcctcctg gtccccaagg tgtcaaaggt 2700
gaacgtggca gtcctggtgg acctggtgct gctggcttcc ctggtgctcg tggtcttcct 2760
ggtcctcctg gtagtaatgg taacccagga cccccaggtc ccagcggttc tccaggcaag 2820
gatgggcccc caggtcctgc gggtaacact ggtgctcctg gcagccctgg agtgtctgga 2880
ccaaaaggtg atgctggcca accaggagag aagggatcgc ctggtgccca gggcccacca 2940
ggagctccag gcccacttgg gattgctggg atcactggag cacggggtct tgcaggacca 3000
ccaggcatgc caggtcctag gggaagccct ggccctcagg gtgtcaaggg tgaaagtggg 3060
aaaccaggag ctaacggtct cagtggagaa cgtggtcccc ctggacccca gggtcttcct 3120
ggtctggctg gtacagctgg tgaacctgga agagatggaa accctggatc agatggtctt 3180
ccaggccgag atggatctcc tggtggcaag ggtgatcgtg gtgaaaatgg ctctcctggt 3240
gcccctggcg ctcctggtca tccaggccca cctggtcctg tcggtccagc tggaaagagt 3300
ggtgacagag gagaaagtgg ccctgctggc cctgctggtg ctcccggtcc tgctggttcc 3360
cgaggtgctc ctggtcctca aggcccacgt ggtgacaaag gtgaaacagg tgaacgtgga 3420
gctgctggca tcaaaggaca tcgaggattc cctggtaatc caggtgcccc aggttctcca 3480
ggccctgctg gtcagcaggg tgcaatcggc agtccaggac ctgcaggccc cagaggacct 3540

gttggaccca gtggacctcc tggcaaagat ggaaccagtg gacatccagg tcccattgga 3600
ccaccagggc ctcgaggtaa cagaggtgaa agaggatctg agggctcccc aggccaccca 3660
gggcaaccag gccctcctgg acctcctggt gcccctggtc cttgctgtgg tggtgttgga 3720
gccgctgcca ttgctgggat tggaggtgaa aaagctggcg gttttgcccc gtattatgga 3780
gatgaaccaa tggatttcaa aatcaacacc gatgagatta tgacttcact caagtctgtt 3840
aatggacaaa tagaaagcct cattagtcct gatggttctc gtaaaaaccc cgctagaaac 3900
tgcagagacc tgaaattctg ccatcctgaa ctcaagagtg gagaatactg ggttgaccct 3960
aaccaaggat gcaaattgga tgctatcaag gtattctgta atatggaaac tggggaaaca 4020
tgcataagtg ccaatccttt gaatgttcca cggaaacact ggtggacaga ttctagtgct 4080
gagaagaaac acgtttggtt tggagagtcc atggatggtg gttttcagtt tagctacggc 4140
aatcctgaac ttcctgaaga tgtccttgat gtgcagctgg cattccttcg acttctctcc 4200
agccgagctt cccagaacat cacatatcac tgcaaaaata gcattgcata catggatcag 4260
gccagtggaa atgtaaagaa ggccctgaag ctgatggggt caaatgaagg tgaattcaag 4320
gctgaaggaa atagcaaatt cacctacaca gttctggagg atggttgcac gaaacacact 4380
ggggaatgga gcaaaacagt ctttgaatat cgaacacgca aggctgtgag actacctatt 4440
gtagatattg caccctatga cattggtggt cctgatcaag aatttggtgt ggacgttggc 4500
cctgtttgct ttttataaac caaactctat ctgaaatccc aacaaaaaaa atttaactcc 4560
atatgtgttc ctcttgttct aatcttgtca accagtgcaa gtgaccgaca aaattccagt 4620
tatttatttc caaaatgttt ggaaacagta taatttgaca aagaaaaatg atacttctct 4680
ttttttgctg ttccaccaaa tacaattcaa atgctttttg ttttattttt ttaccaattc 4740
caatttcaaa atgtctcaat ggtgctataa taaataaact tcaacactct ttatgataac 4800
aacactgtgt tatattcttt gaatcctagc ccatctgcag agcaatgact gtgctcacca 4860
gtaaaagata acctttcttt ctgaaatagt caaatacgaa attagaaaag ccctccctat 4920
tttaactacc tcaactggtc agaaacacag attgtattct atgagtccca gaagatgaaa 4980
aaaattttat acgttgataa aacttataaa tttcattgat taatctcctg gaagattggt 5040
ttaaaaagaa aagtgtaatg caagaattta aagaaatatt tttaaagcca caattatttt 5100
aatattggat atcaactgct tgtaaaggtg ctcctctttt ttcttgtcat tgctggtcaa 5160
gattactaat atttgggaag gctttaaaga cgcatgttat ggtgctaatg tactttcact 5220
tttaaactct agatcagaat tgttgacttg cattcagaac ataaatgcac aaaatctgta 5280
catgtctccc atcagaaaga ttcattggca tgccacaggg gattctcctc cttcatcctg 5340
taaaggtcaa caataaaaac caaattatgg ggctgctttt gtcacactag catagagaat 5400

gtgttgaaat ttaactttgt aagcttgtat gtggttgttg atcttttttt tccttacaga 5460

cacccataat aaaatatcat attaaaattc 5490

<210> 103
<211> 1466
<212> PRT
<213> Homo sapiens

<400> 103

Met Met Ser Phe Val Gln Lys Gly Ser Trp Leu Leu Leu Ala Leu Leu
1 5 10 15

His Pro Thr Ile Ile Leu Ala Gln Gln Glu Ala Val Glu Gly Gly Cys
20 25 30

Ser His Leu Gly Gln Ser Tyr Ala Asp Arg Asp Val Trp Lys Pro Glu
35 40 45

Pro Cys Gln Ile Cys Val Cys Asp Ser Gly Ser Val Leu Cys Asp Asp
50 55 60

Ile Ile Cys Asp Asp Gln Glu Leu Asp Cys Pro Asn Pro Glu Ile Pro
65 70 75 80

Phe Gly Glu Cys Cys Ala Val Cys Pro Gln Pro Pro Thr Ala Pro Thr
85 90 95

Arg Pro Pro Asn Gly Gln Gly Pro Gln Gly Pro Lys Gly Asp Pro Gly
100 105 110

Pro Pro Gly Ile Pro Gly Arg Asn Gly Asp Pro Gly Ile Pro Gly Gln
115 120 125

Pro Gly Ser Pro Gly Ser Pro Gly Pro Pro Gly Ile Cys Glu Ser Cys
130 135 140

Pro Thr Gly Pro Gln Asn Tyr Ser Pro Gln Tyr Asp Ser Tyr Asp Val
145 150 155 160

Lys Ser Gly Val Ala Val Gly Gly Leu Ala Gly Tyr Pro Gly Pro Ala
165 170 175

Gly Pro Pro Gly Pro Pro Gly Pro Pro Gly Thr Ser Gly His Pro Gly
180 185 190

Ser Pro Gly Ser Pro Gly Tyr Gln Gly Pro Pro Gly Glu Pro Gly Gln
195 200 205

Ala Gly Pro Ser Gly Pro Pro Gly Pro Pro Gly Ala Ile Gly Pro Ser
210 215 220

Gly Pro Ala Gly Lys Asp Gly Glu Ser Gly Arg Pro Gly Arg Pro Gly
225 230 235 240

Glu Arg Gly Leu Pro Gly Pro Pro Gly Ile Lys Gly Pro Ala Gly Ile
245 250 255

Pro Gly Phe Pro Gly Met Lys Gly His Arg Gly Phe Asp Gly Arg Asn
260 265 270

Gly Glu Lys Gly Glu Thr Gly Ala Pro Gly Leu Lys Gly Glu Asn Gly
275 280 285

Leu Pro Gly Glu Asn Gly Ala Pro Gly Pro Met Gly Pro Arg Gly Ala
290 295 300

Pro Gly Glu Arg Gly Arg Pro Gly Leu Pro Gly Ala Ala Gly Ala Arg
305 310 315 320

Gly Asn Asp Gly Ala Arg Gly Ser Asp Gly Gln Pro Gly Pro Pro Gly
325 330 335

Pro Pro Gly Thr Ala Gly Phe Pro Gly Ser Pro Gly Ala Lys Gly Glu
340 345 350

Val Gly Pro Ala Gly Ser Pro Gly Ser Asn Gly Ala Pro Gly Gln Arg
355 360 365

Gly Glu Pro Gly Pro Gln Gly His Ala Gly Ala Gln Gly Pro Pro Gly
370 375 380

Pro Pro Gly Ile Asn Gly Ser Pro Gly Gly Lys Gly Glu Met Gly Pro
385 390 395 400

Ala Gly Ile Pro Gly Ala Pro Gly Leu Met Gly Ala Arg Gly Pro Pro
405 410 415

Gly Pro Ala Gly Ala Asn Gly Ala Pro Gly Leu Arg Gly Gly Ala Gly
420 425 430

Glu Pro Gly Lys Asn Gly Ala Lys Gly Glu Pro Gly Pro Arg Gly Glu
435 440 445

Arg Gly Glu Ala Gly Ile Pro Gly Val Pro Gly Ala Lys Gly Glu Asp
450 455 460

Gly Lys Asp Gly Ser Pro Gly Glu Pro Gly Ala Asn Gly Leu Pro Gly
465 470 475 480

Ala Ala Gly Glu Arg Gly Ala Pro Gly Phe Arg Gly Pro Ala Gly Pro
485 490 495

Asn Gly Ile Pro Gly Glu Lys Gly Pro Ala Gly Glu Arg Gly Ala Pro
500 505 510

Gly Pro Ala Gly Pro Arg Gly Ala Ala Gly Glu Pro Gly Arg Asp Gly
515 520 525

Val Pro Gly Gly Pro Gly Met Arg Gly Met Pro Gly Ser Pro Gly Gly
530 535 540

Pro Gly Ser Asp Gly Lys Pro Gly Pro Pro Gly Ser Gln Gly Glu Ser
545 550 555 560

Gly Arg Pro Gly Pro Pro Gly Pro Ser Gly Pro Arg Gly Gln Pro Gly
565 570 575

Val Met Gly Phe Pro Gly Pro Lys Gly Asn Asp Gly Ala Pro Gly Lys
580 585 590

Asn Gly Glu Arg Gly Gly Pro Gly Gly Pro Gly Pro Gln Gly Pro Pro
595 600 605

Gly Lys Asn Gly Glu Thr Gly Pro Gln Gly Pro Pro Gly Pro Thr Gly
610 615 620

Pro Gly Gly Asp Lys Gly Asp Thr Gly Pro Pro Gly Pro Gln Gly Leu
625 630 635 640

Gln Gly Leu Pro Gly Thr Gly Gly Pro Pro Gly Glu Asn Gly Lys Pro
645 650 655

Gly Glu Pro Gly Pro Lys Gly Asp Ala Gly Ala Pro Gly Ala Pro Gly
660 665 670

Gly Lys Gly Asp Ala Gly Ala Pro Gly Glu Arg Gly Pro Pro Gly Leu
675 680 685

Ala Gly Ala Pro Gly Leu Arg Gly Gly Ala Gly Pro Pro Gly Pro Glu
690 695 700

Gly Gly Lys Gly Ala Ala Gly Pro Pro Gly Pro Pro Gly Ala Ala Gly

705 710 715 720

Thr Pro Gly Leu Gln Gly Met Pro Gly Glu Arg Gly Gly Leu Gly Ser
725 730 735

Pro Gly Pro Lys Gly Asp Lys Gly Glu Pro Gly Gly Pro Gly Ala Asp
740 745 750

Gly Val Pro Gly Lys Asp Gly Pro Arg Gly Pro Thr Gly Pro Ile Gly
755 760 765

Pro Pro Gly Pro Ala Gly Gln Pro Gly Asp Lys Gly Glu Gly Gly Ala
770 775 780

Pro Gly Leu Pro Gly Ile Ala Gly Pro Arg Gly Ser Pro Gly Glu Arg
785 790 795 800

Gly Glu Thr Gly Pro Pro Gly Pro Ala Gly Phe Pro Gly Ala Pro Gly
805 810 815

Gln Asn Gly Glu Pro Gly Gly Lys Gly Glu Arg Gly Ala Pro Gly Glu
820 825 830

Lys Gly Glu Gly Gly Pro Pro Gly Val Ala Gly Pro Pro Gly Gly Ser
835 840 845

Gly Pro Ala Gly Pro Pro Gly Pro Gln Gly Val Lys Gly Glu Arg Gly
850 855 860

Ser Pro Gly Gly Pro Gly Ala Ala Gly Phe Pro Gly Ala Arg Gly Leu
865 870 875 880

Pro Gly Pro Pro Gly Ser Asn Gly Asn Pro Gly Pro Pro Gly Pro Ser
885 890 895

Gly Ser Pro Gly Lys Asp Gly Pro Pro Gly Pro Ala Gly Asn Thr Gly
900 905 910

Ala Pro Gly Ser Pro Gly Val Ser Gly Pro Lys Gly Asp Ala Gly Gln
915 920 925

Pro Gly Glu Lys Gly Ser Pro Gly Ala Gln Gly Pro Pro Gly Ala Pro
930 935 940

Gly Pro Leu Gly Ile Ala Gly Ile Thr Gly Ala Arg Gly Leu Ala Gly
945 950 955 960

```
Pro Pro Gly Met Pro Gly Pro Arg Gly Ser Pro Gly Pro Gln Gly Val
                965                 970                 975

Lys Gly Glu Ser Gly Lys Pro Gly Ala Asn Gly Leu Ser Gly Glu Arg
            980                 985                 990

Gly Pro Pro Gly Pro Gln Gly Leu  Pro Gly Leu Ala Gly  Thr Ala Gly
        995                 1000                 1005

Glu Pro  Gly Arg Asp Gly Asn  Pro Gly Ser Asp Gly  Leu Pro Gly
    1010                 1015                 1020

Arg Asp  Gly Ser Pro Gly Gly  Lys Gly Asp Arg Gly  Glu Asn Gly
    1025                 1030                 1035

Ser Pro  Gly Ala Pro Gly Ala  Pro Gly His Pro Gly  Pro Pro Gly
    1040                 1045                 1050

Pro Val  Gly Pro Ala Gly Lys  Ser Gly Asp Arg Gly  Glu Ser Gly
    1055                 1060                 1065

Pro Ala  Gly Pro Ala Gly Ala  Pro Gly Pro Ala Gly  Ser Arg Gly
    1070                 1075                 1080

Ala Pro  Gly Pro Gln Gly Pro  Arg Gly Asp Lys Gly  Glu Thr Gly
    1085                 1090                 1095

Glu Arg  Gly Ala Ala Gly Ile  Lys Gly His Arg Gly  Phe Pro Gly
    1100                 1105                 1110

Asn Pro  Gly Ala Pro Gly Ser  Pro Gly Pro Ala Gly  Gln Gln Gly
    1115                 1120                 1125

Ala Ile  Gly Ser Pro Gly Pro  Ala Gly Pro Arg Gly  Pro Val Gly
    1130                 1135                 1140

Pro Ser  Gly Pro Pro Gly Lys  Asp Gly Thr Ser Gly  His Pro Gly
    1145                 1150                 1155

Pro Ile  Gly Pro Pro Gly Pro  Arg Gly Asn Arg Gly  Glu Arg Gly
    1160                 1165                 1170

Ser Glu  Gly Ser Pro Gly His  Pro Gly Gln Pro Gly  Pro Pro Gly
    1175                 1180                 1185

Pro Pro  Gly Ala Pro Gly Pro  Cys Cys Gly Gly Val  Gly Ala Ala
    1190                 1195                 1200
```

Ala Ile Ala Gly Ile Gly Gly Glu Lys Ala Gly Gly Phe Ala Pro
1205 1210 1215

Tyr Tyr Gly Asp Glu Pro Met Asp Phe Lys Ile Asn Thr Asp Glu
1220 1225 1230

Ile Met Thr Ser Leu Lys Ser Val Asn Gly Gln Ile Glu Ser Leu
1235 1240 1245

Ile Ser Pro Asp Gly Ser Arg Lys Asn Pro Ala Arg Asn Cys Arg
1250 1255 1260

Asp Leu Lys Phe Cys His Pro Glu Leu Lys Ser Gly Glu Tyr Trp
1265 1270 1275

Val Asp Pro Asn Gln Gly Cys Lys Leu Asp Ala Ile Lys Val Phe
1280 1285 1290

Cys Asn Met Glu Thr Gly Glu Thr Cys Ile Ser Ala Asn Pro Leu
1295 1300 1305

Asn Val Pro Arg Lys His Trp Trp Thr Asp Ser Ser Ala Glu Lys
1310 1315 1320

Lys His Val Trp Phe Gly Glu Ser Met Asp Gly Gly Phe Gln Phe
1325 1330 1335

Ser Tyr Gly Asn Pro Glu Leu Pro Glu Asp Val Leu Asp Val Gln
1340 1345 1350

Leu Ala Phe Leu Arg Leu Leu Ser Ser Arg Ala Ser Gln Asn Ile
1355 1360 1365

Thr Tyr His Cys Lys Asn Ser Ile Ala Tyr Met Asp Gln Ala Ser
1370 1375 1380

Gly Asn Val Lys Lys Ala Leu Lys Leu Met Gly Ser Asn Glu Gly
1385 1390 1395

Glu Phe Lys Ala Glu Gly Asn Ser Lys Phe Thr Tyr Thr Val Leu
1400 1405 1410

Glu Asp Gly Cys Thr Lys His Thr Gly Glu Trp Ser Lys Thr Val
1415 1420 1425

Phe Glu Tyr Arg Thr Arg Lys Ala Val Arg Leu Pro Ile Val Asp
1430 1435 1440

Ile Ala Pro Tyr Asp Ile Gly Gly Pro Asp Gln Glu Phe Gly Val
1445 1450 1455

Asp Val Gly Pro Val Cys Phe Leu
1460 1465

<210> 104
<211> 17
<212> DNA
<213> Artificial Sequence

<220>
<223> COL3A1

<400> 104
gaggacggcc aggactt 17

<210> 105
<211> 19
<212> DNA
<213> Artificial Sequence

<220>
<223> COL3A1

<400> 105
ggtccaactt cacccttag 19

<210> 106
<211> 21
<212> DNA
<213> Artificial Sequence

<220>
<223> COL3A1 probe

<400> 106
agtgatggtc aaccaggccc t 21

<210> 107
<211> 6930
<212> DNA
<213> Homo sapiens

<400> 107

gaccgttgct tggcagacac tggatggtta tgagcctgaa caagctgaaa aggggcagga 60

aaagaagtgg aggcagcatt cttcctattt aaagctgcat cgcttgaaaa aagttttcgc 120

agactgtgct ggagctggtg ctgaaaaagg gggtttgcag aggctgccct ggggctggtg 180

ctgaaagaag agcccacagc tgacttcatg gtgctacaat aacctcagaa tctacttttc 240

actctcagga gaacccacat gtctaatatt tagacatgat ggcaaactgg gcggaagcaa 300

gacctctcct cattcttatt gttttattag ggcaatttgt ctcaataaaa gcccaggaag 360

aagacgagga tgaaggatat ggtgaagaaa tagcctgcac tcagaatggc cagatgtact 420

taaacaggga catttggaaa cctgcccctt gtcagatctg tgtctgtgac aatggagcca 480

ttctctgtga caagatagaa tgccaggatg tgctggactg tgccgaccct gtaacgcccc 540

ctggggaatg ctgtcctgtc tgttcacaaa cacctggagg tggcaataca aattttggta 600

gaggaagaaa gggacaaaag ggagaaccag gattagtgcc tgttgtaaca ggcatacgtg 660

gtcgtccagg accggcagga cctccaggat cacagggacc aagaggagag cgagggccaa 720

aaggaagacc tggccctcgt ggacctcagg gaattgatgg agaaccaggt gttcctggtc 780

aacctggtgc tccaggacct cctggacatc cgtcccaccc aggacccgat ggcttgagca 840

ggccgttttc agctcaaatg gctgggttgg atgaaaaatc tggacttggg agtcaagtag 900

gactaatgcc tggctctgtg ggtcctgttg gcccaagggg accacagggt ttacaaggac 960

agcaaggtgg tgcaggacct acaggacctc ctggtgaacc tggtgatcct ggaccaatgg 1020

gtccgattgg ttcacgtgga ccagagggcc ctcctggtaa acctggggaa gatggtgaac 1080

ctggcagaaa tggaaatcct ggtgaagtgg gatttgcagg atctccggga gctcgtggat 1140

ttcctggggc tcctggtctt ccaggtctga agggtcaccg aggacacaaa ggtcttgaag 1200

gccctaaagg tgaagttgga gcacctggtt ccaagggtga agctggcccc actggtccaa 1260

tgggtgccat gggtcctctg ggtccgaggg gaatgccagg agagagaggg agacttgggc 1320

cacagggtgc tcctggacaa cgaggtgcac atggtatgcc tggaaaacct ggaccaatgg 1380

gtcctcttgg gataccaggc tcttctggtt ttccaggaaa tcctggaatg aagggagaag 1440

caggtcctac aggggcgcga ggccctgaag gtcctcaggg gcagagaggt gaaactgggc 1500

cccaggtcc agttggctct ccaggtcttc ctggtgcaat aggaactgat ggtactcctg 1560

gtgccaaagg cccaacgggc tctccgggta cctctggtcc tcctggctca gcagggcctc 1620

ctggatctcc aggacctcag ggtagcactg gtcctcaggg aattcgaggc caaccgggtg 1680

atccaggagt tccaggtttc aaaggagaag ctggcccaaa aggggaacca gggccacatg 1740

gtattcaggg tccgataggc ccacccggtg aagaaggcaa aagaggtccc agaggtgacc 1800

caggaacagt tggtcctcca gggccagtgg gagaaagggg tgctcctggc aatcgtggtt 1860

ttccaggctc tgatggttta cctgggccaa agggtgctca aggagaacgg ggtcctgtag 1920

gttcttcagg acccaaagga agccaggggg atccaggacg tccaggggaa cctgggcttc 1980

caggtgctcg ggtttgaca ggaaatcctg gtgttcaagg tcctgaagga aaacttggac 2040

ctttgggtgc gccaggggaa gatggccgtc caggtcctcc aggctccata ggaatcagag 2100

ggcagcccgg gagcatgggc cttccaggcc ccaaaggtag cagtggtgac cctgggaaac 2160

ctggagaagc aggaaatgct ggagttcctg ggcagagggg agctcctgga aaagatggtg 2220

aagttggtcc ttctggtcct gtgggcccgc cgggtctagc tggtgaaaga ggagaacaag 2280
gacctccagg ccccacaggt tttcaggggc ttcctggtcc tccagggcct cctggagaag 2340
gtggaaaacc aggtgatcaa ggtgttcctg gagatcccgg agcagttggc ccgttaggac 2400
ctagaggaga acgaggaaat cctggggaaa gaggagaacc tgggataact ggactccctg 2460
gtgagaaggg aatggctgga ggacatggtc ctgatggccc aaaaggcagt ccaggtccat 2520
ctgggacccc tggagataca ggcccaccag gtcttcaagg tatgccggga gaaagaggaa 2580
ttgcaggaac tcctggcccc aagggtgaca gaggtggcat aggagaaaaa ggtgctgaag 2640
gcacagctgg aaatgatggt gcaagaggtc ttccaggtcc tttgggccct ccaggtccgg 2700
caggtcctac tggagaaaag ggtgaacctg gtcctcgagg tttagttggc cctcctggct 2760
cccggggcaa tcctggttct cgaggtgaaa atgggccaac tggagctgtt ggttttgccg 2820
gaccccaggg tcctgacgga cagcctggag taaaaggtga acctggagag ccaggacaga 2880
agggagatgc tggttctcct ggaccacaag gtttagcagg atcccctggc cctcatggtc 2940
ctaatggtgt tcctggacta aaaggtggtc gaggaaccca aggtccgcct ggtgctacag 3000
gatttcctgg ttctgcgggc agagttggac ctccaggccc tgctggagct ccaggacctg 3060
cgggacccct aggggaaccc gggaaggagg gacctccagg tcttcgtggg gaccctggct 3120
ctcatgggcg tgtgggagat cgaggaccag ctggcccccc tggtggccca ggagacaaag 3180
gggacccagg agaagatggg caacctggtc cagatggccc ccctggtcca gctggaacga 3240
ccgggcagag aggaattgtt ggcatgcctg ggcaacgtgg agagagaggc atgcccggcc 3300
taccaggccc agcgggaaca ccaggaaaag taggaccaac tggtgcaaca ggagataaag 3360
gtccacctgg acctgtgggg cccccaggct ccaatggtcc tgtaggggaa cctggaccag 3420
aaggtccagc tggcaatgat ggtaccccag gacgggatgg tgctgttgga gaacgtggtg 3480
atcgtggaga ccctgggcct gcaggtctgc caggctctca gggtgcccct ggaactcctg 3540
gccctgtggg tgctccagga gatgcaggac aaagaggaga tccgggttct cggggtccta 3600
taggaccacc tggtcgagct gggaaacgtg gattacctgg accccaagga cctcgtggtg 3660
acaaaggtga tcatggagac cgaggcgaca gaggtcagaa gggccacaga ggctttactg 3720
gtcttcaggg tcttcctggc cctcctggtc caaatggtga acaaggaagt gctggaatcc 3780
ctggaccatt tggcccaaga ggtcctccag gcccagttgg tccttcaggt aaagaaggaa 3840
accctgggcc acttgggcca attggacctc caggtgtacg aggcagtgta ggagaagcag 3900
gacctgaggg ccctcctggt gagcctggcc cacctggccc tccgggtccc cctggccacc 3960
ttacagctgc tcttggggat atcatggggc actatgatga aagcatgcca gatccacttc 4020
ctgagtttac tgaagatcag gcggctcctg atgacaaaaa caaaacggac ccaggggttc 4080
atgctaccct gaagtcactc agtagtcaga ttgaaaccat gcgcagcccc gatggctcga 4140

aaaagcaccc agcccgcacg tgtgatgacc taaagctttg ccattccgca aagcagagtg 4200
gtgaatactg gattgatcct aaccaaggat ctgttgaaga tgcaatcaaa gtttactgca 4260
acatggaaac aggagaaaca tgtatttcag caaacccatc cagtgtacca cgtaaaacct 4320
ggtgggccag taaatctcct gacaataaac ctgtttggta tggtcttgat atgaacagag 4380
ggtctcagtt cgcttatgga gaccaccaat cacctaatac agccattact cagatgactt 4440
ttttgcgcct tttatcaaaa gaagcctccc agaacatcac ttacatctgt aaaaacagtg 4500
taggatacat ggacgatcaa gctaagaacc tcaaaaaagc tgtggttctc aaaggggcaa 4560
atgacttaga tatcaaagca gagggaaata ttagattccg gtatatcgtt cttcaagaca 4620
cttgctctaa gcggaatgga aatgtgggca agactgtctt tgaatataga acacagaatg 4680
tggcacgctt gcccatcata gatcttgctc ctgtggatgt tggcggcaca gaccaggaat 4740
tcggcgttga aattgggcca gtttgttttg tgtaaagtaa gccaagacac atcgacaatg 4800
agcaccacca tcaatgacca ccgccattca caagaacttt gactgtttga agttgatcct 4860
gagactcttg aagtaatggc tgatcctgca tcagcattgt atatatggtc ttaagtgcct 4920
ggcctcctta tccttcagaa tatttatttt acttacaatc ctcaagtttt aattgatttt 4980
aaatattttt caatacaaca gtttaggttt aagatgacca atgacaatga ccacctttgc 5040
agaaagtaaa ctgattgaat aaataaatct ccgttttctt caatttattt cagtgtaatg 5100
aaaaagttgc ttagtattta tgaggaaatt cttcttcctg gcaggtagct taaagagtgg 5160
ggtatataga gccacaacac atgtttattt tgcttggctg cagttgaaaa atagaaatta 5220
gtgccctttt gtgacctctc attccaagat tgtcaattaa aaatgagttt aaaatgttta 5280
acttgtgatc gagacctaca tgcatgtctt gatattgtgt aactataata gagactcttt 5340
aaggagaatc ttaaaaaaaa aaaaacgttt ctcactgtct taaatagaat ttttaaatag 5400
tatatattca gtggcatttt ggagaacaaa gtgaatttac ttcgacttct taaatttttg 5460
taaaagacta taagtttaga catctttctc attcaaattt aaagatatct ttctcctctt 5520
gatcaatcta tcaatattga tagaagtcac actagtatat accatttaat acatttacac 5580
tttcttattt aagaagatat tgaatgcaaa ataattgaca tatagaactt tacaaacata 5640
tgtccaagga ctctaaattg agactcttcc acatgtacaa tctcatcatc ctgaagccta 5700
taatgaagaa aaagatctag aaactgagtt gtggagctga ctctaatcaa atgtgatgat 5760
tggaattaga ccatttggcc tttgaacttt cataggaaaa atgacccaac atttcttagc 5820
atgagctacc tcatctctag aagctgggat ggacttacta ttcttgttta tattttagat 5880
actgaaaggt gctatgcttc tgttattatt ccaagactgg agataggcag ggctaaaaag 5940
gtattattat ttttccttta atgatggtgc taaaattctt cctataaaat tccttaaaaa 6000

```
taaagatggt ttaatcacta ccattgtgaa aacataactg ttagacttcc cgtttctgaa    6060
agaaagagca tcgttccaat gcttgttcac tgttcctctg tcatactgta tctggaatgc    6120
tttgtaatac ttgcatgctt cttagaccag aacatgtagg tccccttgtg tctcaatact    6180
tttttttct taattgcatt tgttggctct attttaattt ttttctttta aaataaacag    6240
ctgggaccat cccaaaagac aagccatgca tacaactttg gtcatgtatc tctgcaaagc    6300
atcaaattaa atgcacgctt ttgtcatgtc agtggttttt gttttgtgaa attcctttga    6360
ccatattaga tctatttcat ttccaatagt gaaaaggaga tgtggtggta tactttgttt    6420
gccatttgtt taaaagatac aacggatacc ttctatcatg tatgtactgg cttataaatg    6480
aaaatctatc tacaacatta cccacaaagg caacatgaca ccaattatca ctgcctctgc    6540
ccttaaaaat gtcagagtag tattattgat aaaaagggca agcaatagat ttttcatgac    6600
tgaataaact gtaataataa aacatatgtc tcaaagtgta tcacatatga atttagccta    6660
attgttttca gtttcattct caatatttag tttacaacat cattttcccc taaactggtt    6720
atattttgac ctgtatatct taaatttgag tatttatatg cctaaataca tgtgtgagtt    6780
ttgtttgact tccaagtcca aactataaga ttatataagt tcatatagat gaatcagaaa    6840
tatgtggtaa tactattaag tcacaaacac taacaatttc caactataga aataacagtt    6900
cttatttgga ttttgggaat gctaccaata                                     6930
```

<210> 108
<211> 1499
<212> PRT
<213> Homo sapiens

<400> 108

```
Met Met Ala Asn Trp Ala Glu Ala Arg Pro Leu Leu Ile Leu Ile Val
1               5                   10                  15

Leu Leu Gly Gln Phe Val Ser Ile Lys Ala Gln Glu Glu Asp Glu Asp
            20                  25                  30

Glu Gly Tyr Gly Glu Glu Ile Ala Cys Thr Gln Asn Gly Gln Met Tyr
        35                  40                  45

Leu Asn Arg Asp Ile Trp Lys Pro Ala Pro Cys Gln Ile Cys Val Cys
    50                  55                  60

Asp Asn Gly Ala Ile Leu Cys Asp Lys Ile Glu Cys Gln Asp Val Leu
65                  70                  75                  80

Asp Cys Ala Asp Pro Val Thr Pro Pro Gly Glu Cys Cys Pro Val Cys
                85                  90                  95
```

Ser Gln Thr Pro Gly Gly Gly Asn Thr Asn Phe Gly Arg Gly Arg Lys
            100                 105                 110

Gly Gln Lys Gly Glu Pro Gly Leu Val Pro Val Val Thr Gly Ile Arg
        115                 120                 125

Gly Arg Pro Gly Pro Ala Gly Pro Pro Gly Ser Gln Gly Pro Arg Gly
    130                 135                 140

Glu Arg Gly Pro Lys Gly Arg Pro Gly Pro Arg Gly Pro Gln Gly Ile
145                 150                 155                 160

Asp Gly Glu Pro Gly Val Pro Gly Gln Pro Gly Ala Pro Gly Pro Pro
                165                 170                 175

Gly His Pro Ser His Pro Gly Pro Asp Gly Leu Ser Arg Pro Phe Ser
            180                 185                 190

Ala Gln Met Ala Gly Leu Asp Glu Lys Ser Gly Leu Gly Ser Gln Val
        195                 200                 205

Gly Leu Met Pro Gly Ser Val Gly Pro Val Gly Pro Arg Gly Pro Gln
    210                 215                 220

Gly Leu Gln Gly Gln Gln Gly Gly Ala Gly Pro Thr Gly Pro Pro Gly
225                 230                 235                 240

Glu Pro Gly Asp Pro Gly Pro Met Gly Pro Ile Gly Ser Arg Gly Pro
                245                 250                 255

Glu Gly Pro Pro Gly Lys Pro Gly Glu Asp Gly Glu Pro Gly Arg Asn
            260                 265                 270

Gly Asn Pro Gly Glu Val Gly Phe Ala Gly Ser Pro Gly Ala Arg Gly
        275                 280                 285

Phe Pro Gly Ala Pro Gly Leu Pro Gly Leu Lys Gly His Arg Gly His
    290                 295                 300

Lys Gly Leu Glu Gly Pro Lys Gly Glu Val Gly Ala Pro Gly Ser Lys
305                 310                 315                 320

Gly Glu Ala Gly Pro Thr Gly Pro Met Gly Ala Met Gly Pro Leu Gly
                325                 330                 335

Pro Arg Gly Met Pro Gly Glu Arg Gly Arg Leu Gly Pro Gln Gly Ala
            340                 345                 350

Pro Gly Gln Arg Gly Ala His Gly Met Pro Gly Lys Pro Gly Pro Met
355 360 365

Gly Pro Leu Gly Ile Pro Gly Ser Ser Gly Phe Pro Gly Asn Pro Gly
370 375 380

Met Lys Gly Glu Ala Gly Pro Thr Gly Ala Arg Gly Pro Glu Gly Pro
385 390 395 400

Gln Gly Gln Arg Gly Glu Thr Gly Pro Pro Gly Pro Val Gly Ser Pro
405 410 415

Gly Leu Pro Gly Ala Ile Gly Thr Asp Gly Thr Pro Gly Ala Lys Gly
420 425 430

Pro Thr Gly Ser Pro Gly Thr Ser Gly Pro Pro Gly Ser Ala Gly Pro
435 440 445

Pro Gly Ser Pro Gly Pro Gln Gly Ser Thr Gly Pro Gln Gly Ile Arg
450 455 460

Gly Gln Pro Gly Asp Pro Gly Val Pro Gly Phe Lys Gly Glu Ala Gly
465 470 475 480

Pro Lys Gly Glu Pro Gly Pro His Gly Ile Gln Gly Pro Ile Gly Pro
485 490 495

Pro Gly Glu Glu Gly Lys Arg Gly Pro Arg Gly Asp Pro Gly Thr Val
500 505 510

Gly Pro Pro Gly Pro Val Gly Glu Arg Gly Ala Pro Gly Asn Arg Gly
515 520 525

Phe Pro Gly Ser Asp Gly Leu Pro Gly Pro Lys Gly Ala Gln Gly Glu
530 535 540

Arg Gly Pro Val Gly Ser Ser Gly Pro Lys Gly Ser Gln Gly Asp Pro
545 550 555 560

Gly Arg Pro Gly Glu Pro Gly Leu Pro Gly Ala Arg Gly Leu Thr Gly
565 570 575

Asn Pro Gly Val Gln Gly Pro Glu Gly Lys Leu Gly Pro Leu Gly Ala
580 585 590

Pro Gly Glu Asp Gly Arg Pro Gly Pro Pro Gly Ser Ile Gly Ile Arg

595 600 605

Gly Gln Pro Gly Ser Met Gly Leu Pro Gly Pro Lys Gly Ser Ser Gly
610 615 620

Asp Pro Gly Lys Pro Gly Glu Ala Gly Asn Ala Gly Val Pro Gly Gln
625 630 635 640

Arg Gly Ala Pro Gly Lys Asp Gly Glu Val Gly Pro Ser Gly Pro Val
645 650 655

Gly Pro Pro Gly Leu Ala Gly Glu Arg Gly Glu Gln Gly Pro Pro Gly
660 665 670

Pro Thr Gly Phe Gln Gly Leu Pro Gly Pro Pro Gly Pro Pro Gly Glu
675 680 685

Gly Gly Lys Pro Gly Asp Gln Gly Val Pro Gly Asp Pro Gly Ala Val
690 695 700

Gly Pro Leu Gly Pro Arg Gly Glu Arg Gly Asn Pro Gly Glu Arg Gly
705 710 715 720

Glu Pro Gly Ile Thr Gly Leu Pro Gly Glu Lys Gly Met Ala Gly Gly
725 730 735

His Gly Pro Asp Gly Pro Lys Gly Ser Pro Gly Pro Ser Gly Thr Pro
740 745 750

Gly Asp Thr Gly Pro Pro Gly Leu Gln Gly Met Pro Gly Glu Arg Gly
755 760 765

Ile Ala Gly Thr Pro Gly Pro Lys Gly Asp Arg Gly Gly Ile Gly Glu
770 775 780

Lys Gly Ala Glu Gly Thr Ala Gly Asn Asp Gly Ala Arg Gly Leu Pro
785 790 795 800

Gly Pro Leu Gly Pro Pro Gly Pro Ala Gly Pro Thr Gly Glu Lys Gly
805 810 815

Glu Pro Gly Pro Arg Gly Leu Val Gly Pro Pro Gly Ser Arg Gly Asn
820 825 830

Pro Gly Ser Arg Gly Glu Asn Gly Pro Thr Gly Ala Val Gly Phe Ala
835 840 845

```
Gly Pro Gln Gly Pro Asp Gly Gln Pro Gly Val Lys Gly Glu Pro Gly
    850                 855                 860

Glu Pro Gly Gln Lys Gly Asp Ala Gly Ser Pro Gly Pro Gln Gly Leu
865                 870                 875                 880

Ala Gly Ser Pro Gly Pro His Gly Pro Asn Gly Val Pro Gly Leu Lys
                885                 890                 895

Gly Gly Arg Gly Thr Gln Gly Pro Pro Gly Ala Thr Gly Phe Pro Gly
            900                 905                 910

Ser Ala Gly Arg Val Gly Pro Pro Gly Pro Ala Gly Ala Pro Gly Pro
        915                 920                 925

Ala Gly Pro Leu Gly Glu Pro Gly Lys Glu Gly Pro Pro Gly Leu Arg
    930                 935                 940

Gly Asp Pro Gly Ser His Gly Arg Val Gly Asp Arg Gly Pro Ala Gly
945                 950                 955                 960

Pro Pro Gly Gly Pro Gly Asp Lys Gly Asp Pro Gly Glu Asp Gly Gln
                965                 970                 975

Pro Gly Pro Asp Gly Pro Pro Gly Pro Ala Gly Thr Thr Gly Gln Arg
            980                 985                 990

Gly Ile Val Gly Met Pro Gly Gln  Arg Gly Glu Arg Gly  Met Pro Gly
        995                 1000                 1005

Leu Pro  Gly Pro Ala Gly Thr  Pro Gly Lys Val Gly  Pro Thr Gly
    1010                 1015                 1020

Ala Thr  Gly Asp Lys Gly Pro  Pro Gly Pro Val Gly  Pro Pro Gly
    1025                 1030                 1035

Ser Asn  Gly Pro Val Gly Glu  Pro Gly Pro Glu Gly  Pro Ala Gly
    1040                 1045                 1050

Asn Asp  Gly Thr Pro Gly Arg  Asp Gly Ala Val Gly  Glu Arg Gly
    1055                 1060                 1065

Asp Arg  Gly Asp Pro Gly Pro  Ala Gly Leu Pro Gly  Ser Gln Gly
    1070                 1075                 1080

Ala Pro  Gly Thr Pro Gly Pro  Val Gly Ala Pro Gly  Asp Ala Gly
    1085                 1090                 1095
```

Gln Arg Gly Asp Pro Gly Ser Arg Gly Pro Ile Gly Pro Pro Gly
1100 1105 1110

Arg Ala Gly Lys Arg Gly Leu Pro Gly Pro Gln Gly Pro Arg Gly
1115 1120 1125

Asp Lys Gly Asp His Gly Asp Arg Gly Asp Arg Gly Gln Lys Gly
1130 1135 1140

His Arg Gly Phe Thr Gly Leu Gln Gly Leu Pro Gly Pro Pro Gly
1145 1150 1155

Pro Asn Gly Glu Gln Gly Ser Ala Gly Ile Pro Gly Pro Phe Gly
1160 1165 1170

Pro Arg Gly Pro Pro Gly Pro Val Gly Pro Ser Gly Lys Glu Gly
1175 1180 1185

Asn Pro Gly Pro Leu Gly Pro Ile Gly Pro Pro Gly Val Arg Gly
1190 1195 1200

Ser Val Gly Glu Ala Gly Pro Glu Gly Pro Pro Gly Glu Pro Gly
1205 1210 1215

Pro Pro Gly Pro Pro Gly Pro Pro Gly His Leu Thr Ala Ala Leu
1220 1225 1230

Gly Asp Ile Met Gly His Tyr Asp Glu Ser Met Pro Asp Pro Leu
1235 1240 1245

Pro Glu Phe Thr Glu Asp Gln Ala Ala Pro Asp Asp Lys Asn Lys
1250 1255 1260

Thr Asp Pro Gly Val His Ala Thr Leu Lys Ser Leu Ser Ser Gln
1265 1270 1275

Ile Glu Thr Met Arg Ser Pro Asp Gly Ser Lys Lys His Pro Ala
1280 1285 1290

Arg Thr Cys Asp Asp Leu Lys Leu Cys His Ser Ala Lys Gln Ser
1295 1300 1305

Gly Glu Tyr Trp Ile Asp Pro Asn Gln Gly Ser Val Glu Asp Ala
1310 1315 1320

Ile Lys Val Tyr Cys Asn Met Glu Thr Gly Glu Thr Cys Ile Ser
1325 1330 1335

```
Ala Asn Pro Ser Ser Val Pro Arg Lys Thr Trp Trp Ala Ser Lys
    1340                1345                1350

Ser Pro Asp Asn Lys Pro Val Trp Tyr Gly Leu Asp Met Asn Arg
    1355                1360                1365

Gly Ser Gln Phe Ala Tyr Gly Asp His Gln Ser Pro Asn Thr Ala
    1370                1375                1380

Ile Thr Gln Met Thr Phe Leu Arg Leu Leu Ser Lys Glu Ala Ser
    1385                1390                1395

Gln Asn Ile Thr Tyr Ile Cys Lys Asn Ser Val Gly Tyr Met Asp
    1400                1405                1410

Asp Gln Ala Lys Asn Leu Lys Lys Ala Val Val Leu Lys Gly Ala
    1415                1420                1425

Asn Asp Leu Asp Ile Lys Ala Glu Gly Asn Ile Arg Phe Arg Tyr
    1430                1435                1440

Ile Val Leu Gln Asp Thr Cys Ser Lys Arg Asn Gly Asn Val Gly
    1445                1450                1455

Lys Thr Val Phe Glu Tyr Arg Thr Gln Asn Val Ala Arg Leu Pro
    1460                1465                1470

Ile Ile Asp Leu Ala Pro Val Asp Val Gly Gly Thr Asp Gln Glu
    1475                1480                1485

Phe Gly Val Glu Ile Gly Pro Val Cys Phe Val
    1490                1495
```

<210> 109
<211> 20
<212> DNA
<213> Artificial Sequence

<220>
<223> COL5A2

<400> 109
aagaggagaa caaggacctc 20

<210> 110
<211> 20
<212> DNA
<213> Artificial Sequence

<220>

<223> COL5A2_reverse primer

<400> 110
atctccagga acaccttgat 20

<210> 111
<211> 22
<212> DNA
<213> Artificial Sequence

<220>
<223> COL5A2_probe

<400> 111
tttccacctt ctccaggagg cc 22

<210> 112
<211> 2175
<212> DNA
<213> Homo sapiens

<400> 112

```
agtacagtat aaaacttcac agtgccaata ccatgaagag gagctcagac agctcttacc      60
acatgataca agagccggct ggtggaagag tggggaccag aaagagaatt tgctgaagag     120
gagaaggaaa aaaaaacac caaaaaaaa aataaaaaa tccacacaca caaaaaaacc     180
tgcgcgtgag gggggaggaa aagcagggcc ttttaaaaag gcaatcacaa caacttttgc     240
tgccaggatg cccttgcttt ggctgagagg atttctgttg gcaagttgct ggattatagt     300
gaggagttcc cccaccccag gatccgaggg gcacagcgcg gcccccgact gtccgtcctg     360
tgcgctggcc gccctcccaa aggatgtacc caactctcag ccagagatgg tggaggccgt     420
caagaagcac attttaaaca tgctgcactt gaagaagaga cccgatgtca cccagccggt     480
acccaaggcg gcgcttctga acgcgatcag aaagcttcat gtgggcaaag tcggggagaa     540
cgggtatgtg gagatagagg atgacattgg aaggagggca gaaatgaatg aacttatgga     600
gcagacctcg gagatcatca cgtttgccga gtcaggaaca gccaggaaga cgctgcactt     660
cgagatttcc aaggaaggca gtgacctgtc agtggtggag cgtgcagaag tctggctctt     720
cctaaaagtc cccaaggcca acaggaccag gaccaaagtc accatccgcc tcttccagca     780
gcagaagcac ccgcagggca gcttggacac aggggaagag gccgaggaag tgggcttaaa     840
gggggagagg agtgaactgt tgctctctga aaaagtagta gacgctcgga agagcacctg     900
gcatgtcttc cctgtctcca gcagcatcca gcggttgctg gaccagggca agagctccct     960
ggacgttcgg attgcctgtg agcagtgcca ggagagtggc gccagcttgg ttctcctggg    1020
caagaagaag aagaaagaag aggaggggga agggaaaaag aagggcggag gtgaaggtgg    1080
ggcaggagca gatgaggaaa aggagcagtc gcacagacct ttcctcatgc tgcaggcccg    1140
```

gcagtctgaa gaccaccctc atcgccggcg tcggcggggc ttggagtgtg atggcaaggt 1200

caacatctgc tgtaagaaac agttctttgt cagtttcaag gacatcggct ggaatgactg 1260

gatcattgct ccctctggct atcatgccaa ctactgcgag ggtgagtgcc cgagccatat 1320

agcaggcacg tccgggtcct cactgtcctt ccactcaaca gtcatcaacc actaccgcat 1380

gcgggggccat agcccctttg ccaacctcaa atcgtgctgt gtgcccacca agctgagacc 1440

catgtccatg ttgtactatg atgatggtca aaacatcatc aaaaaggaca ttcagaacat 1500

gatcgtggag gagtgtgggt gctcatagag ttgcccagcc caggggggaaa gggagcaaga 1560

gttgtccaga gaagacagtg gcaaaatgaa gaaattttta aggtttctga gttaaccaga 1620

aaaatagaaa ttaaaaacaa aacaaaaaaa aaaacaaaaa aaaacaaaag taaattaaaa 1680

acaaaacctg atgaaacaga tgaaggaaga tgtggaaaaa atccttagcc agggctcaga 1740

gatgaagcag tgaaagagac aggaattggg agggaaaggg agaatggtgt accctttatt 1800

tcttctgaaa tcacactgat gacatcagtt gtttaaacgg ggtattgtcc tttccccccct 1860

tgaggttccc ttgtgagcct tgaatcaacc aatctagtct gcagtagtgt ggactagaac 1920

aacccaaata gcatctagaa agccatgagt ttgaaagggc ccatcacagg cactttccta 1980

cccaattacc caggtcataa ggtatgtctg tgtgacactt atctctgtgt atatcagcat 2040

acacacacac acacacacac acacacacac acacaggcat ttccacacat tacatatata 2100

cacatactgg taaaagaaca atcgtgtgca ggtggtcaca cttccttttt ctgtaccact 2160

tttgcaacaa aacaa 2175

<210> 113
<211> 426
<212> PRT
<213> Homo sapiens

<400> 113

Met Pro Leu Leu Trp Leu Arg Gly Phe Leu Leu Ala Ser Cys Trp Ile
1 5 10 15

Ile Val Arg Ser Ser Pro Thr Pro Gly Ser Glu Gly His Ser Ala Ala
20 25 30

Pro Asp Cys Pro Ser Cys Ala Leu Ala Ala Leu Pro Lys Asp Val Pro
35 40 45

Asn Ser Gln Pro Glu Met Val Glu Ala Val Lys Lys His Ile Leu Asn
50 55 60

Met Leu His Leu Lys Lys Arg Pro Asp Val Thr Gln Pro Val Pro Lys
65 70 75 80

```
Ala Ala Leu Leu Asn Ala Ile Arg Lys Leu His Val Gly Lys Val Gly
                85                  90                  95

Glu Asn Gly Tyr Val Glu Ile Glu Asp Asp Ile Gly Arg Arg Ala Glu
            100                 105                 110

Met Asn Glu Leu Met Glu Gln Thr Ser Glu Ile Ile Thr Phe Ala Glu
        115                 120                 125

Ser Gly Thr Ala Arg Lys Thr Leu His Phe Glu Ile Ser Lys Glu Gly
    130                 135                 140

Ser Asp Leu Ser Val Val Glu Arg Ala Glu Val Trp Leu Phe Leu Lys
145                 150                 155                 160

Val Pro Lys Ala Asn Arg Thr Arg Thr Lys Val Thr Ile Arg Leu Phe
                165                 170                 175

Gln Gln Gln Lys His Pro Gln Gly Ser Leu Asp Thr Gly Glu Glu Ala
            180                 185                 190

Glu Glu Val Gly Leu Lys Gly Glu Arg Ser Glu Leu Leu Leu Ser Glu
        195                 200                 205

Lys Val Val Asp Ala Arg Lys Ser Thr Trp His Val Phe Pro Val Ser
    210                 215                 220

Ser Ser Ile Gln Arg Leu Leu Asp Gln Gly Lys Ser Ser Leu Asp Val
225                 230                 235                 240

Arg Ile Ala Cys Glu Gln Cys Gln Glu Ser Gly Ala Ser Leu Val Leu
                245                 250                 255

Leu Gly Lys Lys Lys Lys Lys Glu Glu Glu Gly Glu Gly Lys Lys Lys
            260                 265                 270

Gly Gly Gly Glu Gly Gly Ala Gly Ala Asp Glu Glu Lys Glu Gln Ser
        275                 280                 285

His Arg Pro Phe Leu Met Leu Gln Ala Arg Gln Ser Glu Asp His Pro
    290                 295                 300

His Arg Arg Arg Arg Arg Gly Leu Glu Cys Asp Gly Lys Val Asn Ile
305                 310                 315                 320

Cys Cys Lys Lys Gln Phe Phe Val Ser Phe Lys Asp Ile Gly Trp Asn
                325                 330                 335
```

Asp Trp Ile Ile Ala Pro Ser Gly Tyr His Ala Asn Tyr Cys Glu Gly
340 345 350

Glu Cys Pro Ser His Ile Ala Gly Thr Ser Gly Ser Ser Leu Ser Phe
355 360 365

His Ser Thr Val Ile Asn His Tyr Arg Met Arg Gly His Ser Pro Phe
370 375 380

Ala Asn Leu Lys Ser Cys Cys Val Pro Thr Lys Leu Arg Pro Met Ser
385 390 395 400

Met Leu Tyr Tyr Asp Asp Gly Gln Asn Ile Ile Lys Lys Asp Ile Gln
405 410 415

Asn Met Ile Val Glu Glu Cys Gly Cys Ser
420 425

<210> 114
<211> 20
<212> DNA
<213> Artificial Sequence

<220>
<223> INHBA_forward primer

<400> 114
cctcggagat catcacgttt 20

<210> 115
<211> 21
<212> DNA
<213> Artificial Sequence

<220>
<223> INHBA_reverse primer

<400> 115
cactgccttc cttggaaatc t 21

<210> 116
<211> 22
<212> DNA
<213> Artificial Sequence

<220>
<223> INHBA probe

<400> 116
cagccaggaa gacgctgcac tt 22

<210> 117
<211> 5898
<212> DNA

<213> Homo sapiens

<400> 117

```
aaaagtgagt ccctgccttc cctctctccg tctggctcct cccaggcctg tctggcaggg      60
gccggggtgc aggaggagga gacggcatcc agtacagagg ggctggactt ggacccctgc     120
agcagccctg cacaggagaa gcggcatata aagccgcgct gcccgggagc cgctcggcca     180
cgtccaccgg agcatcctgc actgcagggc cggtctctcg ctccagcaga gcctgcgcct     240
ttctgactcg gtccggaaca ctgaaaccag tcatcactgc atctttttgg caaaccagga     300
gctcagctgc aggaggcagg atggtctgga ggctggtcct gctggctctg tgggtgtggc     360
ccagcacgca agctggtcac caggacaaag acacgacctt cgaccttttc agtatcagca     420
acatcaaccg caagaccatt ggcgccaagc agttccgcgg gcccgacccc ggcgtgccgg     480
cttaccgctt cgtgcgcttt gactacatcc caccggtgaa cgcagatgac ctcagcaaga     540
tcaccaagat catgcggcag aaggagggct tcttcctcac ggcccagctc aagcaggacg     600
gcaagtccag gggcacgctg ttggctctgg agggccccgg tctctcccag aggcagttcg     660
agatcgtctc caacggcccc gcggacacgc tggatctcac ctactggatt gacggcaccc     720
ggcatgtggt ctccctggag gacgtcggcc tggctgactc gcagtggaag aacgtcaccg     780
tgcaggtggc tggcgagacc tacagcttgc acgtgggctg cgacctcata gacagcttcg     840
ctctggacga gcccttctac gagcacctgc aggcggaaaa gagccggatg tacgtggcca     900
aaggctctgc cagagagagt cacttcaggg gtttgcttca gaacgtccac ctagtgtttg     960
aaaactctgt ggaagatatt ctaagcaaga agggttgcca gcaaggccag ggagctgaga    1020
tcaacgccat cagtgagaac acagagacgc tgcgcctggg tccgcatgtc accaccgagt    1080
acgtgggccc cagctcggag aggaggcccg aggtgtgcga acgctcgtgc gaggagctgg    1140
gaaacatggt ccaggagctc tcggggctcc acgtcctcgt gaaccagctc agcgagaacc    1200
tcaagagagt gtcgaatgat aaccagtttc tctgggagct cattggtggc cctcctaaga    1260
caaggaacat gtcagcttgc tggcaggatg gccggttctt tgcggaaaat gaaacgtggg    1320
tggtggacag ctgcaccacg tgtacctgca agaaatttaa aaccatttgc caccaaatca    1380
cctgcccgcc tgcaacctgc gccagtccat cctttgtgga aggcgaatgc tgcccttcct    1440
gcctccactc ggtggacggt gaggagggct ggtctccgtg ggcagagtgg acccagtgct    1500
ccgtgacgtg tggctctggg acccagcaga gaggccggtc ctgtgacgtc accagcaaca    1560
cctgcttggg gccctccatc cagacacggg cttgcagtct gagcaagtgt gacacccgca    1620
tccggcagga cggcggctgg agccactggt caccttggtc ttcatgctct gtgacctgtg    1680
gagttggcaa tatcacacgc atccgtctct gcaactcccc agtgccccag atgggggggca   1740
```

agaattgcaa agggagtggc cgggagacca aagcctgcca gggcgcccca tgcccaatcg 1800
atggccgctg gagcccctgg tccccgtggt cggcctgcac tgtcacctgt gccggtggga 1860
tccgggagcg cacccgggtc tgcaacagcc ctgagcctca gtacggaggg aaggcctgcg 1920
tgggggatgt gcaggagcgt cagatgtgca acaagaggag ctgccccgtg gatggctgtt 1980
tatccaaccc ctgcttcccg ggagcccagt gcagcagctt ccccgatggg tcctggtcat 2040
gcggctcctg ccctgtgggc ttcttgggca atggcaccca ctgtgaggac ctggacgagt 2100
gtgccctggt ccccgacatc tgcttctcca ccagcaaggt gcctcgctgt gtcaacactc 2160
agcctggctt ccactgcctg ccctgcccgc cccgatacag agggaaccag cccgtcgggg 2220
tcggcctgga agcagccaag acggaaaagc aagtgtgtga gcccgaaaac ccatgcaagg 2280
acaagacaca caactgccac aagcacgcgg agtgcatcta cctgggccac ttcagcgacc 2340
ccatgtacaa gtgcgagtgc cagacaggct acgcgggcga cgggctcatc tgcggggagg 2400
actcggacct ggacggctgg cccaacctca atctggtctg cgccaccaac gccacctacc 2460
actgcatcaa ggataactgc cccatctgc caaattctgg gcaggaagac tttgacaagg 2520
acgggattgg cgatgcctgt gatgatgacg atgacaatga cggtgtgacc gatgagaagg 2580
acaactgcca gctcctcttc aatccccgcc aggctgacta tgacaaggat gaggttgggg 2640
accgctgtga caactgccct tacgtgcaca accctgccca gatcgacaca gacaacaatg 2700
gagagggtga cgcctgctcc gtggacattg atggggacga tgtcttcaat gaacgagaca 2760
attgtcccta cgtctacaac actgaccaga gggacacgga tggtgacggt gtgggggatc 2820
actgtgacaa ctgcccctg gtgcacaacc ctgaccagac cgacgtggac aatgaccttg 2880
ttggggacca gtgtgacaac aacgaggaca tagatgacga cggccaccag aacaaccagg 2940
acaactgccc ctacatctcc aacgccaacc aggctgacca tgacagagac ggccagggcg 3000
acgcctgtga ccctgatgat gacaacgatg gcgtccccga tgacagggac aactgccggc 3060
ttgtgttcaa cccagaccag gaggacttgg acggtgatgg acggggtgat atttgtaaag 3120
atgattttga caatgacaac atcccagata ttgatgatgt gtgtcctgaa aacaatgcca 3180
tcagtgagac agacttcagg aacttccaga tggtcccctt ggatcccaaa gggaccaccc 3240
aaattgatcc caactgggtc attcgccatc aaggcaagga gctggttcag acagccaact 3300
cggaccccgg catcgctgta ggttttgacg agtttgggtc tgtggacttc agtggcacat 3360
tctacgtaaa cactgaccgg gacgacgact atgccggctt cgtctttggt taccagtcaa 3420
gcagccgctt ctatgtggtg atgtggaagc aggtgacgca gacctactgg gaggaccagc 3480
ccacgcgggc ctatggctac tccggcgtgt ccctcaaggt ggtgaactcc accacgggga 3540
cgggcgagca cctgaggaac gcgctgtggc acacggggaa cacgccgggg caggtgcgaa 3600
ccttatggca cgaccccagg aacattggct ggaaggacta cacggcctat aggtggcacc 3660

```
tgactcacag gcccaagact ggctacatca gagtcttagt gcatgaagga aaacaggtca    3720
tggcagactc aggacctatc tatgaccaaa cctacgctgg cgggcggctg ggtctatttg    3780
tcttctctca agaaatggtc tatttctcag acctcaagta cgaatgcaga gatatttaaa    3840
caagatttgc tgcatttccg gcaatgccct gtgcatgcca tggtccctag acacctcagt    3900
tcattgtggt ccttgtggct tctctctcta gcagcacctc ctgtcccttg accttaactc    3960
tgatggttct tcacctcctg ccagcaaccc caaacccaag tgccttcaga ggataaatat    4020
caatggaact cagagatgaa catctaaccc actagaggaa accagtttgg tgatatatga    4080
gactttatgt ggagtgaaaa ttgggcatgc cattacattg ctttttcttg tttgtttaaa    4140
aagaatgacg tttacatata aaatgtaatt acttattgta tttatgtgta tatggagttg    4200
aagggaatac tgtgcataag ccattatgat aaattaagca tgaaaaatat tgctgaacta    4260
cttttggtgc ttaaagttgt cactattctt gaattagagt tgctctacaa tgacacacaa    4320
atcccattaa ataaattata aacaagggtc aattcaaatt tgaagtaatg ttttagtaag    4380
gagagattag aagacaacag gcatagcaaa tgacataagc taccgattaa ctaatcggaa    4440
catgtaaaac agttacaaaa ataaacgaac tctcctcttg tcctacaatg aaagccctca    4500
tgtgcagtag agatgcagtt tcatcaaaga acaaacatcc ttgcaaatgg gtgtgacgcg    4560
gttccagatg tggatttggc aaaacctcat ttaagtaaaa ggttagcaga gcaaagtgcg    4620
gtgctttagc tgctgcttgt gccgctgtgg cgtcggggag gctcctgcct gagcttcctt    4680
ccccagcttt gctgcctgag aggaaccaga gcagacgcac aggccggaaa aggcgcatct    4740
aacgcgtatc taggctttgg taactgcgga caagttgctt ttacctgatt tgatgataca    4800
tttcattaag gttccagtta taaatatttt gttaatattt attaagtgac tatagaatgc    4860
aactccattt accagtaact tattttaaat atgcctagta acacatatgt agtataattt    4920
ctagaaacaa acatctaata agtatataat cctgtgaaaa tatgaggctt gataatatta    4980
ggttgtcacg atgaagcatg ctagaagctg taacagaata catagagaat aatgaggagt    5040
ttatgatgga accttaaata tataatgttg ccagcgattt tagttcaata tttgttactg    5100
ttatctatct gctgtatatg gaattctttt aattcaaacg ctgaaaagaa tcagcattta    5160
gtcttgccag gcacacccaa taatcagtca tgtgtaatat gcacaagttt gtttttgttt    5220
ttgttttttt tgttggttgg tttgtttttt tgctttaagt tgcatgatct ttctgcagga    5280
aatagtcact catcccactc cacataaggg gtttagtaag agaagtctgt ctgtctgatg    5340
atggataggg ggcaaatctt tttccccttt ctgttaatag tcatcacatt tctatgccaa    5400
acaggaacaa tccataactt tagtcttaat gtacacattg cattttgata aaattaattt    5460
tgttgtttcc tttgaggttg atcgttgtgt tgttgttttg ctgcactttt tacttttttg    5520
```

```
cgtgtggagc tgtattcccg agaccaacga agcgttggga tacttcatta aatgtagcga      5580
ctgtcaacag cgtgcaggtt ttctgtttct gtgttgtggg gtcaaccgta caatggtgtg      5640
ggagtgacga tgatgtgaat atttagaatg taccatattt tttgtaaatt atttatgttt      5700
ttctaaacaa atttatcgta taggttgatg aaacgtcatg tgttttgcca aagactgtaa      5760
atatttattt atgtgttcac atggtcaaaa tttcaccact gaaaccctgc acttagctag      5820
aacctcattt ttaaagatta acaacaggaa ataaattgta aaaaaggttt tctatacatg      5880
aaaaaaaaaa aaaaaaaa                                                    5898
```

<210> 118
<211> 1172
<212> PRT
<213> Homo sapiens

<400> 118

Met Val Trp Arg Leu Val Leu Leu Ala Leu Trp Val Trp Pro Ser Thr
1               5                   10                  15

Gln Ala Gly His Gln Asp Lys Asp Thr Thr Phe Asp Leu Phe Ser Ile
            20                  25                  30

Ser Asn Ile Asn Arg Lys Thr Ile Gly Ala Lys Gln Phe Arg Gly Pro
        35                  40                  45

Asp Pro Gly Val Pro Ala Tyr Arg Phe Val Arg Phe Asp Tyr Ile Pro
    50                  55                  60

Pro Val Asn Ala Asp Asp Leu Ser Lys Ile Thr Lys Ile Met Arg Gln
65                  70                  75                  80

Lys Glu Gly Phe Phe Leu Thr Ala Gln Leu Lys Gln Asp Gly Lys Ser
                85                  90                  95

Arg Gly Thr Leu Leu Ala Leu Glu Gly Pro Gly Leu Ser Gln Arg Gln
            100                 105                 110

Phe Glu Ile Val Ser Asn Gly Pro Ala Asp Thr Leu Asp Leu Thr Tyr
        115                 120                 125

Trp Ile Asp Gly Thr Arg His Val Val Ser Leu Glu Asp Val Gly Leu
    130                 135                 140

Ala Asp Ser Gln Trp Lys Asn Val Thr Val Gln Val Ala Gly Glu Thr
145                 150                 155                 160

Tyr Ser Leu His Val Gly Cys Asp Leu Ile Asp Ser Phe Ala Leu Asp

```
                165                 170                 175

Glu Pro Phe Tyr Glu His Leu Gln Ala Glu Lys Ser Arg Met Tyr Val
            180                 185                 190

Ala Lys Gly Ser Ala Arg Glu Ser His Phe Arg Gly Leu Leu Gln Asn
        195                 200                 205

Val His Leu Val Phe Glu Asn Ser Val Glu Asp Ile Leu Ser Lys Lys
    210                 215                 220

Gly Cys Gln Gln Gly Gln Gly Ala Glu Ile Asn Ala Ile Ser Glu Asn
225                 230                 235                 240

Thr Glu Thr Leu Arg Leu Gly Pro His Val Thr Thr Glu Tyr Val Gly
                245                 250                 255

Pro Ser Ser Glu Arg Arg Pro Glu Val Cys Glu Arg Ser Cys Glu Glu
            260                 265                 270

Leu Gly Asn Met Val Gln Glu Leu Ser Gly Leu His Val Leu Val Asn
        275                 280                 285

Gln Leu Ser Glu Asn Leu Lys Arg Val Ser Asn Asp Asn Gln Phe Leu
    290                 295                 300

Trp Glu Leu Ile Gly Gly Pro Pro Lys Thr Arg Asn Met Ser Ala Cys
305                 310                 315                 320

Trp Gln Asp Gly Arg Phe Phe Ala Glu Asn Glu Thr Trp Val Val Asp
                325                 330                 335

Ser Cys Thr Thr Cys Thr Cys Lys Lys Phe Lys Thr Ile Cys His Gln
            340                 345                 350

Ile Thr Cys Pro Pro Ala Thr Cys Ala Ser Pro Ser Phe Val Glu Gly
        355                 360                 365

Glu Cys Cys Pro Ser Cys Leu His Ser Val Asp Gly Glu Glu Gly Trp
    370                 375                 380

Ser Pro Trp Ala Glu Trp Thr Gln Cys Ser Val Thr Cys Gly Ser Gly
385                 390                 395                 400

Thr Gln Gln Arg Gly Arg Ser Cys Asp Val Thr Ser Asn Thr Cys Leu
                405                 410                 415
```

```
Gly Pro Ser Ile Gln Thr Arg Ala Cys Ser Leu Ser Lys Cys Asp Thr
            420                 425                 430

Arg Ile Arg Gln Asp Gly Gly Trp Ser His Trp Ser Pro Trp Ser Ser
        435                 440                 445

Cys Ser Val Thr Cys Gly Val Gly Asn Ile Thr Arg Ile Arg Leu Cys
    450                 455                 460

Asn Ser Pro Val Pro Gln Met Gly Gly Lys Asn Cys Lys Gly Ser Gly
465                 470                 475                 480

Arg Glu Thr Lys Ala Cys Gln Gly Ala Pro Cys Pro Ile Asp Gly Arg
                485                 490                 495

Trp Ser Pro Trp Ser Pro Trp Ser Ala Cys Thr Val Thr Cys Ala Gly
            500                 505                 510

Gly Ile Arg Glu Arg Thr Arg Val Cys Asn Ser Pro Glu Pro Gln Tyr
        515                 520                 525

Gly Gly Lys Ala Cys Val Gly Asp Val Gln Glu Arg Gln Met Cys Asn
    530                 535                 540

Lys Arg Ser Cys Pro Val Asp Gly Cys Leu Ser Asn Pro Cys Phe Pro
545                 550                 555                 560

Gly Ala Gln Cys Ser Ser Phe Pro Asp Gly Ser Trp Ser Cys Gly Ser
                565                 570                 575

Cys Pro Val Gly Phe Leu Gly Asn Gly Thr His Cys Glu Asp Leu Asp
            580                 585                 590

Glu Cys Ala Leu Val Pro Asp Ile Cys Phe Ser Thr Ser Lys Val Pro
        595                 600                 605

Arg Cys Val Asn Thr Gln Pro Gly Phe His Cys Leu Pro Cys Pro Pro
    610                 615                 620

Arg Tyr Arg Gly Asn Gln Pro Val Gly Val Gly Leu Glu Ala Ala Lys
625                 630                 635                 640

Thr Glu Lys Gln Val Cys Glu Pro Glu Asn Pro Cys Lys Asp Lys Thr
                645                 650                 655

His Asn Cys His Lys His Ala Glu Cys Ile Tyr Leu Gly His Phe Ser
            660                 665                 670
```

Asp Pro Met Tyr Lys Cys Glu Cys Gln Thr Gly Tyr Ala Gly Asp Gly
675 680 685

Leu Ile Cys Gly Glu Asp Ser Asp Leu Asp Gly Trp Pro Asn Leu Asn
690 695 700

Leu Val Cys Ala Thr Asn Ala Thr Tyr His Cys Ile Lys Asp Asn Cys
705 710 715 720

Pro His Leu Pro Asn Ser Gly Gln Glu Asp Phe Asp Lys Asp Gly Ile
725 730 735

Gly Asp Ala Cys Asp Asp Asp Asp Asp Asn Asp Gly Val Thr Asp Glu
740 745 750

Lys Asp Asn Cys Gln Leu Leu Phe Asn Pro Arg Gln Ala Asp Tyr Asp
755 760 765

Lys Asp Glu Val Gly Asp Arg Cys Asp Asn Cys Pro Tyr Val His Asn
770 775 780

Pro Ala Gln Ile Asp Thr Asp Asn Asn Gly Glu Gly Asp Ala Cys Ser
785 790 795 800

Val Asp Ile Asp Gly Asp Asp Val Phe Asn Glu Arg Asp Asn Cys Pro
805 810 815

Tyr Val Tyr Asn Thr Asp Gln Arg Asp Thr Asp Gly Asp Gly Val Gly
820 825 830

Asp His Cys Asp Asn Cys Pro Leu Val His Asn Pro Asp Gln Thr Asp
835 840 845

Val Asp Asn Asp Leu Val Gly Asp Gln Cys Asp Asn Asn Glu Asp Ile
850 855 860

Asp Asp Asp Gly His Gln Asn Asn Gln Asp Asn Cys Pro Tyr Ile Ser
865 870 875 880

Asn Ala Asn Gln Ala Asp His Asp Arg Asp Gly Gln Gly Asp Ala Cys
885 890 895

Asp Pro Asp Asp Asp Asn Asp Gly Val Pro Asp Asp Arg Asp Asn Cys
900 905 910

Arg Leu Val Phe Asn Pro Asp Gln Glu Asp Leu Asp Gly Asp Gly Arg
915 920 925

Gly Asp Ile Cys Lys Asp Asp Phe Asp Asn Asp Asn Ile Pro Asp Ile
930 935 940

Asp Asp Val Cys Pro Glu Asn Asn Ala Ile Ser Glu Thr Asp Phe Arg
945 950 955 960

Asn Phe Gln Met Val Pro Leu Asp Pro Lys Gly Thr Thr Gln Ile Asp
965 970 975

Pro Asn Trp Val Ile Arg His Gln Gly Lys Glu Leu Val Gln Thr Ala
980 985 990

Asn Ser Asp Pro Gly Ile Ala Val Gly Phe Asp Glu Phe Gly Ser Val
995 1000 1005

Asp Phe Ser Gly Thr Phe Tyr Val Asn Thr Asp Arg Asp Asp Asp
1010 1015 1020

Tyr Ala Gly Phe Val Phe Gly Tyr Gln Ser Ser Ser Arg Phe Tyr
1025 1030 1035

Val Val Met Trp Lys Gln Val Thr Gln Thr Tyr Trp Glu Asp Gln
1040 1045 1050

Pro Thr Arg Ala Tyr Gly Tyr Ser Gly Val Ser Leu Lys Val Val
1055 1060 1065

Asn Ser Thr Thr Gly Thr Gly Glu His Leu Arg Asn Ala Leu Trp
1070 1075 1080

His Thr Gly Asn Thr Pro Gly Gln Val Arg Thr Leu Trp His Asp
1085 1090 1095

Pro Arg Asn Ile Gly Trp Lys Asp Tyr Thr Ala Tyr Arg Trp His
1100 1105 1110

Leu Thr His Arg Pro Lys Thr Gly Tyr Ile Arg Val Leu Val His
1115 1120 1125

Glu Gly Lys Gln Val Met Ala Asp Ser Gly Pro Ile Tyr Asp Gln
1130 1135 1140

Thr Tyr Ala Gly Gly Arg Leu Gly Leu Phe Val Phe Ser Gln Glu
1145 1150 1155

Met Val Tyr Phe Ser Asp Leu Lys Tyr Glu Cys Arg Asp Ile

1160 1165 1170

<210> 119
<211> 17
<212> DNA
<213> Artificial Sequence

<220>
<223> THBS2_forward primer

<400> 119
cagcacgcaa gctggtc 17

<210> 120
<211> 20
<212> DNA
<213> Artificial Sequence

<220>
<223> THBS2_reverse primer

<400> 120
tgcggttgat gttgctgata 20

<210> 121
<211> 25
<212> DNA
<213> Artificial Sequence

<220>
<223> THBS2 probe

<400> 121
accaggacaa agacacgacc ttcga 25

<210> 122
<211> 4193
<212> DNA
<213> Homo sapiens

<400> 122

```
cttcttctcg ctgagtctcc tcctcggctc tgacggtaca gtgatataat gatgatgggt        60
gtcacaaccc gcatttgaac ttgcaggcga gctgccccga gcctttctgg ggaagaactc       120
caggcgtgcg gacgcaacag ccgagaacat taggtgttgt ggacaggagc tgggaccaag       180
atcttcggcc agccccgcat cctcccgcat cttccagcac cgtcccgcac cctccgcatc       240
cttccccggg ccaccacgct tcctatgtga cccgcctggg caacgccgaa cccagtcgcg       300
cagcgctgca gtgaattttc cccccaaact gcaataagcc gccttccaag gccaagatgt       360
tcataaatat aaagagcatc ttatggatgt gttcaacctt aatagtaacc catgcgctac       420
ataaagtcaa agtgggaaaa agcccaccgg tgaggggctc cctctctgga aaagtcagcc       480
taccttgtca tttttcaacg atgcctactt tgccacccag ttacaacacc agtgaatttc       540
```

tccgcatcaa atggtctaag attgaagtgg acaaaaatgg aaaagatttg aaagagacta 600
ctgtccttgt ggcccaaaat ggaaatatca agattggtca ggactacaaa gggagagtgt 660
ctgtgcccac acatcccgag gctgtgggcg atgcctccct cactgtggtc aagctgctgg 720
caagtgatgc gggtctttac cgctgtgacg tcatgtacgg gattgaagac acacaagaca 780
cggtgtcact gactgtggat ggggttgtgt ttcactacag ggcggcaacc agcaggtaca 840
cactgaattt tgaggctgct cagaaggctt gtttggacgt tggggcagtc atagcaactc 900
cagagcagct ctttgctgcc tatgaagatg gatttgagca gtgtgacgca ggctggctgg 960
ctgatcagac tgtcagatat cccatccggg ctcccagagt aggctgttat ggagataaga 1020
tgggaaaggc aggagtcagg acttatggat tccgttctcc ccaggaaact tacgatgtgt 1080
attgttatgt ggatcatctg gatggtgatg tgttccacct cactgtcccc agtaaattca 1140
ccttcgagga ggctgcaaaa gagtgtgaaa accaggatgc caggctggca acagtggggg 1200
aactccaggc ggcatggagg aacggctttg accagtgcga ttacgggtgg ctgtcggatg 1260
ccagcgtgcg ccaccctgtg actgtggcca gggcccagtg tggaggtggt ctacttgggg 1320
tgagaaccct gtatcgtttt gagaaccaga caggcttccc tcccccctgat agcagatttg 1380
atgcctactg ctttaaacga cctgatcgct gcaaaatgaa cccgtgcctt aacggaggca 1440
cctgttatcc tactgaaact tcctacgtat gcacctgtgt gccaggatac agcggagacc 1500
agtgtgaact tgattttgat gaatgtcact ctaatccctg tcgtaatgga gccacttgtg 1560
ttgatggttt taacacattc aggtgcctct gccttccaag ttatgttggt gcactttgtg 1620
agcaagatac cgagacatgt gactatggct ggcacaaatt ccaagggcag tgctacaaat 1680
actttgccca tcgacgcaca tgggatgcag ctgaacggga atgccgtctg cagggtgccc 1740
atctcacaag catcctgtct cacgaagaac aaatgtttgt taatcgtgtg ggccatgatt 1800
atcagtggat aggcctcaat gacaagatgt ttgagcatga cttccgttgg actgatggca 1860
gcacactgca atacgagaat tggagaccca accagccaga cagcttcttt tctgctggag 1920
aagactgtgt tgtaatcatt tggcatgaga atggccagtg gaatgatgtt ccctgcaatt 1980
accatctcac ctatacgtgc aagaaaggaa cagtcgcttg cggccagccc cctgttgtag 2040
aaaatgccaa gacctttgga aagatgaaac ctcgttatga aatcaactcc ctgattagat 2100
accactgcaa agatggtttc attcaacgtc accttccaac tatccggtgc ttaggaaatg 2160
gaagatgggc tatacctaaa attacctgca tgaacccatc tgcataccaa aggacttatt 2220
ctatgaaata ctttaaaaat tcctcatcag caaaggacaa ttcaataaat acatccaaac 2280
atgatcatcg ttggagccgg aggtggcagg agtcgaggcg ctgatcccta aaatggcgaa 2340
catgtgtttt catcatttca gccaaagtcc taacttcctg tgcctttcct atcacctcga 2400
gaagtaatta tcagttggtt tggatttttg gaccaccgtt cagtcatttt gggttgccgt 2460

gctcccaaaa cattttaaat gaaagtattg gcattcaaaa agacagcaga caaaatgaaa 2520

gaaaatgaga gcagaaagta agcatttcca gcctatctaa tttctttagt tttctatttg 2580

cctccagtgc agtccatttc ctaatgtata ccagcctact gtactattta aaatgctcaa 2640

tttcagcacc gatggccatg taaataagat gatttaatgt tgattttaat cctgtatata 2700

aaataaaaag tcacaatgag tttgggcata tttaatgatg attatggagc cttagaggtc 2760

tttaatcatt ggttcggctg cttttatgta gtttaggctg gaaatggttt cacttgctct 2820

ttgactgtca gcaagactga agatggcttt tcctggacag ctagaaaaca caaaatcttg 2880

taggtcattg cacctatctc agccataggt gcagtttgct tctacatgat gctaaaggct 2940

gcgaatggga tcctgatgga actaaggact ccaatgtcga actcttcttt gctgcattcc 3000

tttttcttca cttacaagaa aggcctgaat ggaggacttt tctgtaacca ggaacatttt 3060

ttaggggtca aagtgctaat aattaactca accaggtcta ctttttaatg gctttcataa 3120

cactaactca taaggttacc gatcaatgca tttcatacgg atatagacct agggctctgg 3180

agggtggggg attgttaaaa cacatgcaaa aaaaaaaaaa aaaaaaaaaa aagaaatttt 3240

gtatatataa ccattttaat cttttataaa gttttgaatg ttcatgtatg aatgctgcag 3300

ctgtgaagca tacataaata aatgaagtaa gccatactga tttaatttat tggatgttat 3360

tttccctaag acctgaaaat gaacatagta tgctagttat ttttcagtgt tagcctttta 3420

ctttcctcac acaatttgga atcatataat ataggtactt tgtccctgat taaataatgt 3480

gacggataga atgcatcaag tgtttattat gaaaagagtg gaaaagtata tagcttttag 3540

caaaaggtgt ttgcccattc taagaaatga gcgaatatat agaaatagtg tgggcatttc 3600

ttcctgttag gtggagtgta tgtgttgaca tttctcccca tctcttccca ctctgttttc 3660

tccccattat ttgaataaag tgactgctga agatgacttt gaatccttat ccacttaatt 3720

taatgtttaa agaaaaacct gtaatggaaa gtaagactcc ttccctaatt tcagtttaga 3780

gcaacttgaa gaagagtaga caaaaaataa aatgcacata gaaaaagaga aaaagggcac 3840

aaagggattg gcccaatatt gattcttttt ttataaaacc tcctttggct tagaaggaat 3900

gactctagct acaataatac acagtatgtt taagcaggtt cccttggttg ttgcattaaa 3960

tgtaatccac ctttaggtat tttagagcac agaacaacac tgtgttgatc tagtaggttt 4020

ctatttttcc tttctcttta caatgcacat aatactttcc tgtatttata tcataacgtg 4080

tatagtgtaa aatgtgaatg actttttttg tgaatgaaaa tctaaaatct ttgtaacttt 4140

ttatatctgc ttttgtttca ccaaagaaac ctaaaatcct tcttttacta cac 4193

<210> 123
<211> 9455
<212> DNA

<213> Homo sapiens

<400> 123

cttcttctcg ctgagtctcc tcctcggctc tgacggtaca gtgatataat gatgatgggt 60
gtcacaaccc gcatttgaac ttgcaggcga gctgccccga gcctttctgg ggaagaactc 120
caggcgtgcg gacgcaacag ccgagaacat taggtgttgt ggacaggagc tgggaccaag 180
atcttcggcc agccccgcat cctcccgcat cttccagcac cgtcccgcac cctccgcatc 240
cttccccggg ccaccacgct tcctatgtga cccgcctggg caacgccgaa cccagtcgcg 300
cagcgctgca gtgaattttc cccccaaact gcaataagcc gccttccaag gccaagatgt 360
tcataaatat aaagagcatc ttatggatgt gttcaacctt aatagtaacc catgcgctac 420
ataaagtcaa agtgggaaaa agcccaccgg tgaggggctc cctctctgga aaagtcagcc 480
taccttgtca tttttcaacg atgcctactt tgccacccag ttacaacacc agtgaatttc 540
tccgcatcaa atggtctaag attgaagtgg acaaaaatgg aaaagatttg aaagagacta 600
ctgtccttgt ggcccaaaat ggaaatatca agattggtca ggactacaaa gggagagtgt 660
ctgtgcccac acatcccgag gctgtgggcg atgcctccct cactgtggtc aagctgctgg 720
caagtgatgc gggtctttac cgctgtgacg tcatgtacgg gattgaagac acacaagaca 780
cggtgtcact gactgtggat ggggttgtgt ttcactacag ggcggcaacc agcaggtaca 840
cactgaattt tgaggctgct cagaaggctt gtttggacgt tggggcagtc atagcaactc 900
cagagcagct ctttgctgcc tatgaagatg gatttgagca gtgtgacgca ggctggctgg 960
ctgatcagac tgtcagatat cccatccggg ctcccagagt aggctgttat ggagataaga 1020
tgggaaaggc aggagtcagg acttatggat tccgttctcc ccaggaaact tacgatgtgt 1080
attgttatgt ggatcatctg gatggtgatg tgttccacct cactgtcccc agtaaattca 1140
ccttcgagga ggctgcaaaa gagtgtgaaa accaggatgc caggctggca acagtggggg 1200
aactccaggc ggcatggagg aacggctttg accagtgcga ttacgggtgg ctgtcggatg 1260
ccagcgtgcg ccaccctgtg actgtggcca gggcccagtg tggaggtggt ctacttgggg 1320
tgagaaccct gtatcgtttt gagaaccaga caggcttccc tccccctgat agcagatttg 1380
atgcctactg ctttaaacgt cgaatgagtg atttgagtgt aattggtcat ccaatagatt 1440
cagaatctaa agaagatgaa ccttgtagtg aagaaacaga tccagtgcat gatctaatgg 1500
ctgaaatttt acctgaattc cctgacataa ttgaaataga cctataccac agtgaagaaa 1560
atgaagaaga agaagaagag tgtgcaaatg ctactgatgt gacaaccacc ccatctgtgc 1620
agtacataaa tgggaagcat ctcgttacca ctgtgcccaa ggacccagaa gctgcagaag 1680
ctaggcgtgg ccagtttgaa agtgttgcac cttctcagaa tttctcggac agctctgaaa 1740
gtgatactca tccatttgta atagccaaaa cggaattgtc tactgctgtg caacctaatg 1800

aatctacaga aacaactgag tctcttgaag ttacatggaa gcctgagact taccctgaaa 1860
catcagaaca tttttcaggt ggtgagcctg atgttttccc cacagtccca ttccatgagg 1920
aatttgaaag tggaacagcc aaaaaagggg cagaatcagt cacagagaga gatactgaag 1980
ttggtcatca ggcacatgaa catactgaac ctgtatctct gtttcctgaa gagtcttcag 2040
gagagattgc cattgaccaa gaatctcaga aaatagcctt tgcaagggct acagaagtaa 2100
catttggtga agaggtagaa aaaagtactt ctgtcacata cactcccact atagttccaa 2160
gttctgcatc agcatatgtt tcagaggaag aagcagttac cctaatagga aatccttggc 2220
cagatgacct gttgtctacc aaagaaagct gggtagaagc aactcctaga caagttgtag 2280
agctctcagg gagttcttcg attccaatta cagaaggctc tggagaagca gaagaagatg 2340
aagatacaat gttcaccatg gtaactgatt tatcacagag aaatactact gatacactca 2400
ttactttaga cactagcagg ataatcacag aaagcttttt tgaggttcct gcaaccacca 2460
tttatccagt ttctgaacaa ccttctgcaa aagtggtgcc taccaagttt gtaagtgaaa 2520
cagacacttc tgagtggatt tccagtacca ctgttgagga aaagaaaagg aaggaggagg 2580
agggaactac aggtacggct tctacatttg aggtatattc atctacacag agatcggatc 2640
aattaatttt accctttgaa ttagaaagtc caaatgtagc tacatctagt gattcaggta 2700
ccaggaaaag ttttatgtcc ttgacaacac caacacagtc tgaaagggaa atgacagatt 2760
ctactcctgt ctttacagaa acaaatacat tagaaaattt gggggcacag accactgagc 2820
acagcagtat ccatcaacct ggggttcagg aagggctgac cactctccca cgtagtcctg 2880
cctctgtctt tatggagcag ggctctggag aagctgctgc cgacccagaa accaccactg 2940
tttcttcatt ttcattaaac gtagagtatg caattcaagc cgaaaaggaa gtagctggca 3000
ctttgtctcc gcatgtggaa actacattct ccactgagcc aacaggactg gttttgagta 3060
cagtaatgga cagagtagtt gctgaaaata taacccaaac atccagggaa atagtgattt 3120
cagagcgatt aggagaacca aattatgggg cagaaataag gggcttttcc acaggttttc 3180
ctttggagga agatttcagt ggtgacttta gagaatactc aacagtgtct catcccatag 3240
caaaagaaga aacggtaatg atggaaggct ctggagatgc agcatttagg gacacccaga 3300
cttcaccatc tacagtacct acttcagttc acatcagtca catatctgac tcagaaggac 3360
ccagtagcac catggtcagc acttcagcct tcccctggga agagtttaca tcctcagctg 3420
agggctcagg tgagcaactg gtcacagtca gcagctctgt tgttccagtg cttcccagtg 3480
ctgtgcaaaa gttttctggt acagcttcct ccattatcga cgaaggattg ggagaagtgg 3540
gtactgtcaa tgaaattgat agaagatcca ccattttacc aacagcagaa gtggaaggta 3600
cgaaagctcc agtagagaag gaggaagtaa aggtcagtgg cacagtttca acaaactttc 3660
cccaaactat agagccagcc aaattatggt ctaggcaaga agtcaaccct gtaagacaag 3720

aaattgaaag tgaaacaaca tcagaggaac aaattcaaga agaaaagtca tttgaatccc 3780

ctcaaaactc tcctgcaaca gaacaaacaa tctttgattc acagacattt actgaaactg 3840

aactcaaaac cacagattat tctgtactaa caacaaagaa aacttacagt gatgataaag 3900

aaatgaagga ggaagacact tctttagtta acatgtctac tccagatcca gatgcaaatg 3960

gcttggaatc ttacacaact ctccctgaag ctactgaaaa gtcacatttt ttcttagcta 4020

ctgcattagt aactgaatct ataccagctg aacatgtagt cacagattca ccaatcaaaa 4080

aggaagaaag tacaaaacat tttccgaaag gcatgagacc aacaattcaa gagtcagata 4140

ctgagctctt attctctgga ctgggatcag gagaagaagt tttacctact ctaccaacag 4200

agtcagtgaa ttttactgaa gtggaacaaa tcaataacac attatatccc cacacttctc 4260

aagtggaaag tacctcaagt gacaaaattg aagactttaa cagaatggaa aatgtggcaa 4320

aagaagttgg accactcgta tctcaaacag acatctttga aggtagtggg tcagtaacca 4380

gcacaacatt aatagaaatt ttaagtgaca ctggagcaga aggacccacg gtggcacctc 4440

tccctttctc cacggacatc ggacatcctc aaaatcagac tgtcaggtgg gcagaagaaa 4500

tccagactag tagaccacaa accataactg aacaagactc taacaagaat tcttcaacag 4560

cagaaattaa cgaaacaaca acctcatcta ctgattttct ggctagagct tatggttttg 4620

aaatggccaa agaatttgtt acatcagcac caaaaccatc tgacttgtat tatgaacctt 4680

ctggagaagg atctggagaa gtggatattg ttgattcatt tcacacttct gcaactactc 4740

aggcaaccag acaagaaagc agcaccacat ttgtttctga tgggtccctg gaaaaacatc 4800

ctgaggtgcc aagcgctaaa gctgttactg ctgatggatt cccaacagtt tcagtgatgc 4860

tgcctcttca ttcagagcag aacaaaagct cccctgatcc aactagcaca ctgtcaaata 4920

cagtgtcata tgagaggtcc acagacggta gtttccaaga ccgtttcagg gaattcgagg 4980

attccacctt aaaacctaac agaaaaaaac ccactgaaaa tattatcata gacctggaca 5040

aagaggacaa ggatttaata ttgacaatta cagagagtac catccttgaa attctacctg 5100

agctgacatc ggataaaaat actatcatag atattgatca tactaaacct gtgtatgaag 5160

acattcttgg aatgcaaaca gatatagata cagaggtacc atcagaacca catgacagta 5220

atgatgaaag taatgatgac agcactcaag ttcaagagat ctatgaggca gctgtcaacc 5280

tttctttaac tgaggaaaca tttgagggct ctgctgatgt tctggctagc tacactcagg 5340

caacacatga tgaatcaatg acttatgaag atagaagcca actagatcac atgggctttc 5400

acttcacaac tgggatccct gctcctagca cagaaacaga attagacgtt ttacttccca 5460

cggcaacatc cctgccaatt cctcgtaagt ctgccacagt tattccagag attgaaggaa 5520

taaaagctga agcaaaagcc ctggatgaca tgtttgaatc aagcactttg tctgatggtc 5580

aagctattgc agaccaaagt gaaataatac caacattggg ccaatttgaa aggactcagg 5640

aggagtatga agacaaaaaa catgctggtc cttcttttca gccagaattc tcttcaggag 5700

ctgaggaggc attagtagac catactccct atctaagtat tgctactacc caccttatgg 5760

atcagagtgt aacagaggtg cctgatgtga tggaaggatc caatcccccca tattacactg 5820

atacaacatt agcagtttca acatttgcga agttgtcttc tcagacacca tcatctcccc 5880

tcactatcta ctcaggcagt gaagcctctg gacacacaga gatcccccag cccagtgctc 5940

tgccaggaat agacgtcggc tcatctgtaa tgtccccaca ggattctttt aaggaaattc 6000

atgtaaatat tgaagcgact ttcaaaccat caagtgagga ataccttcac ataactgagc 6060

ctccctcttt atctcctgac acaaaattag aaccttcaga agatgatggt aaacctgagt 6120

tattagaaga aatggaagct tctcccacag aacttattgc tgtggaagga actgagattc 6180

tccaagattt ccaaaacaaa accgatggtc aagtttctgg agaagcaatc aagatgtttc 6240

ccaccattaa aacacctgag gctggaactg ttattacaac tgccgatgaa attgaattag 6300

aaggtgctac acagtggcca cactctactt ctgcttctgc cacctatggg gtcgaggcag 6360

gtgtggtgcc ttggctaagt ccacagactt ctgagaggcc cacgctttct tcttctccag 6420

aaataaaccc tgaaactcaa gcagctttaa tcagagggca ggattccacg atagcagcat 6480

cagaacagca agtggcagcg agaattcttg attccaatga tcaggcaaca gtaaaccctg 6540

tggaatttaa tactgaggtt gcaacaccac cattttccct tctggagact tctaatgaaa 6600

cagatttcct gattggcatt aatgaagagt cagtggaagg cacggcaatc tatttaccag 6660

gacctgatcg ctgcaaaatg aacccgtgcc ttaacggagg cacctgttat cctactgaaa 6720

cttcctacgt atgcacctgt gtgccaggat acagcggaga ccagtgtgaa cttgattttg 6780

atgaatgtca ctctaatccc tgtcgtaatg gagccacttg tgttgatggt tttaacacat 6840

tcaggtgcct ctgccttcca agttatgttg gtgcactttg tgagcaagat accgagacat 6900

gtgactatgg ctggcacaaa ttccaagggc agtgctacaa atactttgcc catcgacgca 6960

catgggatgc agctgaacgg gaatgccgtc tgcagggtgc ccatctcaca agcatcctgt 7020

ctcacgaaga acaaatgttt gttaatcgtg tgggccatga ttatcagtgg ataggcctca 7080

atgacaagat gtttgagcat gacttccgtt ggactgatgg cagcacactg caatacgaga 7140

attggagacc caaccagcca gacagcttct tttctgctgg agaagactgt gttgtaatca 7200

tttggcatga gaatggccag tggaatgatg ttccctgcaa ttaccatctc acctatacgt 7260

gcaagaaagg aacagtcgct tgcggccagc cccctgttgt agaaaatgcc aagacctttg 7320

gaaagatgaa acctcgttat gaaatcaact ccctgattag ataccactgc aaagatggtt 7380

tcattcaacg tcaccttcca actatccggt gcttaggaaa tggaagatgg gctataccta 7440

aaattacctg catgaaccca tctgcatacc aaaggactta ttctatgaaa tactttaaaa 7500

attcctcatc agcaaaggac aattcaataa atacatccaa acatgatcat cgttggagcc 7560
ggaggtggca ggagtcgagg cgctgatccc taaaatggcg aacatgtgtt ttcatcattt 7620
cagccaaagt cctaacttcc tgtgcctttc ctatcacctc gagaagtaat tatcagttgg 7680
tttggatttt tggaccaccg ttcagtcatt ttgggttgcc gtgctcccaa aacattttaa 7740
atgaaagtat tggcattcaa aaagacagca gacaaaatga aagaaaatga gagcagaaag 7800
taagcatttc cagcctatct aatttcttta gttttctatt tgcctccagt gcagtccatt 7860
tcctaatgta taccagccta ctgtactatt taaaatgctc aatttcagca ccgatggcca 7920
tgtaaataag atgatttaat gttgatttta atcctgtata taaaataaaa agtcacaatg 7980
agtttgggca tatttaatga tgattatgga gccttagagg tctttaatca ttggttcggc 8040
tgcttttatg tagtttaggc tggaaatggt ttcacttgct ctttgactgt cagcaagact 8100
gaagatggct tttcctggac agctagaaaa cacaaaatct tgtaggtcat tgcacctatc 8160
tcagccatag gtgcagtttg cttctacatg atgctaaagg ctgcgaatgg gatcctgatg 8220
gaactaagga ctccaatgtc gaactcttct ttgctgcatt cctttttctt cacttacaag 8280
aaaggcctga atggaggact tttctgtaac caggaacatt ttttaggggt caaagtgcta 8340
ataattaact caaccaggtc tactttttaa tggctttcat aacactaact cataaggtta 8400
ccgatcaatg catttcatac ggatatagac ctagggctct ggagggtggg ggattgttaa 8460
aacacatgca aaaaaaaaaa aaaaaaaaaa aaaagaaatt ttgtatatat aaccatttta 8520
atcttttata aagttttgaa tgttcatgta tgaatgctgc agctgtgaag catacataaa 8580
taaatgaagt aagccatact gatttaattt attggatgtt attttcccta agacctgaaa 8640
atgaacatag tatgctagtt atttttcagt gttagccttt tactttcctc acacaatttg 8700
gaatcatata atataggtac tttgtccctg attaaataat gtgacggata gaatgcatca 8760
agtgtttatt atgaaaagag tggaaaagta tatagctttt agcaaaaggt gtttgcccat 8820
tctaagaaat gagcgaatat atagaaatag tgtgggcatt tcttcctgtt aggtggagtg 8880
tatgtgttga catttctccc catctcttcc cactctgttt tctccccatt atttgaataa 8940
agtgactgct gaagatgact ttgaatcctt atccacttaa tttaatgttt aaagaaaaac 9000
ctgtaatgga aagtaagact ccttccctaa tttcagttta gagcaacttg aagaagagta 9060
gacaaaaaat aaaatgcaca tagaaaaaga gaaaaagggc acaaagggat tggcccaata 9120
ttgattcttt ttttataaaa cctcctttgg cttagaagga atgactctag ctacaataat 9180
acacagtatg tttaagcagg ttcccttggt tgttgcatta aatgtaatcc acctttaggt 9240
attttagagc acagaacaac actgtgttga tctagtaggt ttctattttt cctttctctt 9300
tacaatgcac ataatacttt cctgtattta tatcataacg tgtatagtgt aaaatgtgaa 9360

tgactttttt tgtgaatgaa aatctaaaat ctttgtaact ttttatatct gcttttgttt 9420

caccaaagaa acctaaaatc cttcttttac tacac 9455

<210> 124
<211> 7154
<212> DNA
<213> Homo sapiens

<400> 124

```
cttcttctcg ctgagtctcc tcctcggctc tgacggtaca gtgatataat gatgatgggt      60
gtcacaaccc gcatttgaac ttgcaggcga gctgccccga gcctttctgg ggaagaactc     120
caggcgtgcg gacgcaacag ccgagaacat taggtgttgt ggacaggagc tgggaccaag     180
atcttcggcc agccccgcat cctcccgcat cttccagcac cgtcccgcac cctccgcatc     240
cttccccggg ccaccacgct tcctatgtga cccgcctggg caacgccgaa cccagtcgcg     300
cagcgctgca gtgaattttc cccccaaact gcaataagcc gccttccaag gccaagatgt     360
tcataaatat aaagagcatc ttatggatgt gttcaacctt aatagtaacc catgcgctac     420
ataaagtcaa agtgggaaaa agcccaccgg tgaggggctc cctctctgga aaagtcagcc     480
taccttgtca tttttcaacg atgcctactt tgccacccag ttacaacacc agtgaatttc     540
tccgcatcaa atggtctaag attgaagtgg acaaaaatgg aaaagatttg aaagagacta     600
ctgtccttgt ggcccaaaat ggaaatatca agattggtca ggactacaaa gggagagtgt     660
ctgtgcccac acatcccgag gctgtgggcg atgcctccct cactgtggtc aagctgctgg     720
caagtgatgc gggtctttac cgctgtgacg tcatgtacgg gattgaagac acacaagaca     780
cggtgtcact gactgtggat ggggttgtgt ttcactacag ggcggcaacc agcaggtaca     840
cactgaattt tgaggctgct cagaaggctt gtttggacgt tggggcagtc atagcaactc     900
cagagcagct ctttgctgcc tatgaagatg gatttgagca gtgtgacgca ggctggctgg     960
ctgatcagac tgtcagatat cccatccggg ctcccagagt aggctgttat ggagataaga    1020
tgggaaaggc aggagtcagg acttatggat tccgttctcc ccaggaaact tacgatgtgt    1080
attgttatgt ggatcatctg gatggtgatg tgttccacct cactgtcccc agtaaattca    1140
ccttcgagga ggctgcaaaa gagtgtgaaa accaggatgc caggctggca acagtggggg    1200
aactccaggc ggcatggagg aacggctttg accagtgcga ttacgggtgg ctgtcggatg    1260
ccagcgtgcg ccaccctgtg actgtggcca gggcccagtg tggaggtggt ctacttgggg    1320
tgagaaccct gtatcgtttt gagaaccaga caggcttccc tcccccctgat agcagatttg    1380
atgcctactg ctttaaacct aaagaggcta caaccatcga tttgagtatc ctcgcagaaa    1440
ctgcatcacc cagtttatcc aaagaaccac aaatggtttc tgatagaact acaccaatca    1500
tccctttagt tgatgaatta cctgtcattc caacagagtt ccctcccgtg ggaaatattg    1560
```

```
tcagttttga acagaaagcc acagtccaac ctcaggctat cacagatagt ttagccacca     1620
aattacccac acctactggc agtaccaaga agccctggga tatggatgac tactcacctt     1680
ctgcttcagg acctcttgga aagctagaca tatcagaaat taaggaagaa gtgctccaga     1740
gtacaactgg cgtctctcat tatgctacgg attcatggga tggtgtcgtg gaagataaac     1800
aaacacaaga atcggttaca cagattgaac aaatagaagt gggtcctttg gtaacatcta     1860
tggaaatctt aaagcacatt ccttccaagg aattccctgt aactgaaaca ccattggtaa     1920
ctgcaagaat gatcctggaa tccaaaactg aaaagaaaat ggtaagcact gtttctgaat     1980
tggtaaccac aggtcactat ggattcacct tgggagaaga ggatgatgaa gacagaacac     2040
ttacagttgg atctgatgag agcaccttga tctttgacca aattcctgaa gtcattacgg     2100
tgtcaaagac ttcagaagac accatccaca ctcatttaga agacttggag tcagtctcag     2160
catccacaac tgtttcccct ttaattatgc ctgataataa tggatcatcc atggatgact     2220
gggaagagag acaaactagt ggtaggataa cggaagagtt tcttggcaaa tatctgtcta     2280
ctacaccttt tccatcacag catcgtacag aaatagaatt gtttccttat tctggtgata     2340
aaatattagt agagggaatt tccacagtta tttatccttc tctacaaaca gaaatgacac     2400
atagaagaga aagaacagaa acactaatac cagagatgag aacagatact tatacagatg     2460
aaatacaaga agagatcact aaaagtccat ttatgggaaa aacagaagaa gaagtcttct     2520
ctgggatgaa actctctaca tctctctcag agccaattca tgttacagag tcttctgtgg     2580
aaatgaccaa gtcttttgat ttcccaacat tgataacaaa gttaagtgca gagccaacag     2640
aagtaagaga tatggaggaa gactttacag caactccagg tactacaaaa tatgatgaaa     2700
atattacaac agtgcttttg gcccatggta ctttaagtgt tgaagcagcc actgtatcaa     2760
aatggtcatg ggatgaagat aatacaacat ccaagccttt agagtctaca gaaccttcag     2820
cctcttcaaa attgcccccct gccttactca caactgtggg gatgaatgga aaggataaag     2880
acatcccaag tttcactgaa gatggagcag atgaatttac tcttattcca gatagtactc     2940
aaaagcagtt agaggaggtt actgatgaag acatagcagc ccatggaaaa ttcacaatta     3000
gatttcagcc aactacatca actggtattg cagaaaagtc aactttgaga gattctacaa     3060
ctgaagaaaa agttccacct atcacaagca ctgaaggcca agtttatgca accatggaag     3120
gaagtgcttt gggtgaagta gaagatgtgg acctctctaa gccagtatct actgttcccc     3180
aatttgcaca cacttcagag gtggaaggat tagcatttgt tagttatagt agcacccaag     3240
agcctactac ttatgtagac tcttcccata ccattcctct ttctgtaatt cccaagacag     3300
actggggagt gttagtacct tctgttccat cagaagatga agttctaggt gaaccctctc     3360
aagacatact tgtcattgat cagactcgcc ttgaagcgac tatttctcca gaaactatga     3420
gaacaacaaa aatcacagag ggaacaactc aggaagaatt cccttggaaa gaacagactg     3480
```

cagagaaacc agttcctgct ctcagttcta cagcttggac tcccaaggag gcagtaacac 3540
cactggatga acaagagggc gatggatcag catatacagt ctctgaagat gaattgttga 3600
caggttctga gagggtccca gttttagaaa caactccagt tggaaaaatt gatcacagtg 3660
tgtcttatcc accaggtgct gtaactgagc acaaagtgaa aacagatgaa gtggtaacac 3720
taacaccacg cattgggcca aaagtatctt taagtccagg gcctgaacaa aaatatgaaa 3780
cagaaggtag tagtacaaca ggatttacat catctttgag tccttttagt acccacatta 3840
cccagcttat ggaagaaacc actactgaga aaacatccct agaggatatt gatttaggct 3900
caggattatt tgaaaagccc aaagccacag aactcataga attttcaaca atcaaagtca 3960
cagttccaag tgatattacc actgccttca gttcagtaga cagacttcac acaacttcag 4020
cattcaagcc atcttccgcg atcactaaga aaccacctct catcgacagg gaacctggtg 4080
aagaaacaac cagtgacatg gtaatcattg gagaatcaac atctcatgtt cctcccacta 4140
cccttgaaga tattgtagcc aaggaaacag aaaccgatat tgatagagag tatttcacga 4200
cttcaagtcc tcctgctaca cagccaacaa gaccacccac tgtggaagac aaagaggcct 4260
ttggacctca ggcgctttct acgccacagc cccagcaag cacaaaattt caccctgaca 4320
ttaatgttta tattattgag gtcagagaaa ataagacagg acctgatcgc tgcaaaatga 4380
acccgtgcct taacggaggc acctgttatc ctactgaaac ttcctacgta tgcacctgtg 4440
tgccaggata cagcggagac cagtgtgaac ttgattttga tgaatgtcac tctaatccct 4500
gtcgtaatgg agccacttgt gttgatggtt ttaacacatt caggtgcctc tgccttccaa 4560
gttatgttgg tgcactttgt gagcaagata ccgagacatg tgactatggc tggcacaaat 4620
tccaagggca gtgctacaaa tactttgccc atcgacgcac atgggatgca gctgaacggg 4680
aatgccgtct gcagggtgcc catctcacaa gcatcctgtc tcacgaagaa caaatgtttg 4740
ttaatcgtgt gggccatgat tatcagtgga taggcctcaa tgacaagatg tttgagcatg 4800
acttccgttg gactgatggc agcacactgc aatacgagaa ttggagaccc aaccagccag 4860
acagcttctt ttctgctgga gaagactgtg ttgtaatcat ttggcatgag aatggccagt 4920
ggaatgatgt tccctgcaat taccatctca cctatacgtg caagaaagga acagtcgctt 4980
gcggccagcc ccctgttgta gaaaatgcca agacctttgg aaagatgaaa cctcgttatg 5040
aaatcaactc cctgattaga taccactgca aagatggttt cattcaacgt caccttccaa 5100
ctatccggtg cttaggaaat ggaagatggg ctatacctaa aattacctgc atgaacccat 5160
ctgcatacca aaggacttat tctatgaaat actttaaaaa ttcctcatca gcaaaggaca 5220
attcaataaa tacatccaaa catgatcatc gttggagccg gaggtggcag gagtcgaggc 5280
gctgatccct aaaatggcga acatgtgttt tcatcatttc agccaaagtc ctaacttcct 5340

gtgcctttcc tatcacctcg agaagtaatt atcagttggt ttggattttt ggaccaccgt 5400

tcagtcattt tgggttgccg tgctcccaaa acattttaaa tgaaagtatt ggcattcaaa 5460

aagacagcag acaaaatgaa agaaaatgag agcagaaagt aagcatttcc agcctatcta 5520

atttctttag ttttctattt gcctccagtg cagtccattt cctaatgtat accagcctac 5580

tgtactattt aaaatgctca atttcagcac cgatggccat gtaaataaga tgatttaatg 5640

ttgattttaa tcctgtatat aaaataaaaa gtcacaatga gtttgggcat atttaatgat 5700

gattatggag ccttagaggt ctttaatcat tggttcggct gcttttatgt agtttaggct 5760

ggaaatggtt tcacttgctc tttgactgtc agcaagactg aagatggctt ttcctggaca 5820

gctagaaaac acaaaatctt gtaggtcatt gcacctatct cagccatagg tgcagtttgc 5880

ttctacatga tgctaaaggc tgcgaatggg atcctgatgg aactaaggac tccaatgtcg 5940

aactcttctt tgctgcattc ctttttcttc acttacaaga aaggcctgaa tggaggactt 6000

ttctgtaacc aggaacattt tttaggggtc aaagtgctaa taattaactc aaccaggtct 6060

actttttaat ggctttcata acactaactc ataaggttac cgatcaatgc atttcatacg 6120

gatatagacc tagggctctg gagggtgggg gattgttaaa acacatgcaa aaaaaaaaaa 6180

aaaaaaaaaa aaagaaattt tgtatatata accattttaa tcttttataa agttttgaat 6240

gttcatgtat gaatgctgca gctgtgaagc atacataaat aaatgaagta agccatactg 6300

atttaattta ttggatgtta ttttccctaa gacctgaaaa tgaacatagt atgctagtta 6360

tttttcagtg ttagcctttt actttcctca cacaatttgg aatcatataa tataggtact 6420

ttgtccctga ttaaataatg tgacggatag aatgcatcaa gtgtttatta tgaaaagagt 6480

ggaaaagtat atagctttta gcaaaaggtg tttgcccatt ctaagaaatg agcgaatata 6540

tagaaatagt gtgggcattt cttcctgtta ggtggagtgt atgtgttgac atttctcccc 6600

atctcttccc actctgtttt ctccccatta tttgaataaa gtgactgctg aagatgactt 6660

tgaatcctta tccacttaat ttaatgttta aagaaaaacc tgtaatggaa agtaagactc 6720

cttccctaat ttcagtttag agcaacttga agaagagtag acaaaaaata aaatgcacat 6780

agaaaaagag aaaaagggca caaagggatt ggcccaatat tgattctttt tttataaaac 6840

ctcctttggc ttagaaggaa tgactctagc tacaataata cacagtatgt ttaagcaggt 6900

tcccttggtt gttgcattaa atgtaatcca cctttaggta ttttagagca cagaacaaca 6960

ctgtgttgat ctagtaggtt tctatttttc ctttctcttt acaatgcaca taatactttc 7020

ctgtatttat atcataacgt gtatagtgta aaatgtgaat gacttttttt gtgaatgaaa 7080

atctaaaatc tttgtaactt tttatatctg cttttgtttc accaaagaaa cctaaaatcc 7140

ttcttttact acac 7154

<210> 125
<211> 12416
<212> DNA
<213> Homo sapiens

<400> 125

cttcttctcg ctgagtctcc tcctcggctc tgacggtaca gtgatataat gatgatgggt 60
gtcacaaccc gcatttgaac ttgcaggcga gctgccccga gcctttctgg ggaagaactc 120
caggcgtgcg gacgcaacag ccgagaacat taggtgttgt ggacaggagc tgggaccaag 180
atcttcggcc agccccgcat cctcccgcat cttccagcac cgtcccgcac cctccgcatc 240
cttccccggg ccaccacgct tcctatgtga cccgcctggg caacgccgaa cccagtcgcg 300
cagcgctgca gtgaattttc cccccaaact gcaataagcc gccttccaag gccaagatgt 360
tcataaatat aaagagcatc ttatggatgt gttcaacctt aatagtaacc catgcgctac 420
ataaagtcaa agtgggaaaa agcccaccgg tgaggggctc cctctctgga aaagtcagcc 480
taccttgtca tttttcaacg atgcctactt tgccacccag ttacaacacc agtgaatttc 540
tccgcatcaa atggtctaag attgaagtgg acaaaaatgg aaaagatttg aaagagacta 600
ctgtccttgt ggcccaaaat ggaaatatca agattggtca ggactacaaa gggagagtgt 660
ctgtgcccac acatcccgag gctgtgggcg atgcctccct cactgtggtc aagctgctgg 720
caagtgatgc gggtctttac cgctgtgacg tcatgtacgg gattgaagac acacaagaca 780
cggtgtcact gactgtggat ggggttgtgt ttcactacag ggcggcaacc agcaggtaca 840
cactgaattt tgaggctgct cagaaggctt gtttggacgt tggggcagtc atagcaactc 900
cagagcagct ctttgctgcc tatgaagatg gatttgagca gtgtgacgca ggctggctgg 960
ctgatcagac tgtcagatat cccatccggg ctcccagagt aggctgttat ggagataaga 1020
tgggaaaggc aggagtcagg acttatggat tccgttctcc ccaggaaact tacgatgtgt 1080
attgttatgt ggatcatctg gatggtgatg tgttccacct cactgtcccc agtaaattca 1140
ccttcgagga ggctgcaaaa gagtgtgaaa accaggatgc caggctggca acagtggggg 1200
aactccaggc ggcatggagg aacggctttg accagtgcga ttacgggtgg ctgtcggatg 1260
ccagcgtgcg ccaccctgtg actgtggcca gggcccagtg tggaggtggt ctacttgggg 1320
tgagaaccct gtatcgtttt gagaaccaga caggcttccc tccccctgat agcagatttg 1380
atgcctactg ctttaaacct aaagaggcta caaccatcga tttgagtatc ctcgcagaaa 1440
ctgcatcacc cagtttatcc aaagaaccac aaatggtttc tgatagaact acaccaatca 1500
tccctttagt tgatgaatta cctgtcattc caacagagtt ccctcccgtg ggaaatattg 1560
tcagttttga acagaaagcc acagtccaac ctcaggctat cacagatagt ttagccacca 1620
aattacccac acctactggc agtaccaaga agccctggga tatggatgac tactcacctt 1680
ctgcttcagg acctcttgga aagctagaca tatcagaaat taaggaagaa gtgctccaga 1740

gtacaactgg cgtctctcat tatgctacgg attcatggga tggtgtcgtg gaagataaac 1800
aaacacaaga atcggttaca cagattgaac aaatagaagt gggtcctttg gtaacatcta 1860
tggaaatctt aaagcacatt ccttccaagg aattccctgt aactgaaaca ccattggtaa 1920
ctgcaagaat gatcctggaa tccaaaactg aaaagaaaat ggtaagcact gtttctgaat 1980
tggtaaccac aggtcactat ggattcacct tgggagaaga ggatgatgaa gacagaacac 2040
ttacagttgg atctgatgag agcaccttga tctttgacca aattcctgaa gtcattacgg 2100
tgtcaaagac ttcagaagac accatccaca ctcatttaga agacttggag tcagtctcag 2160
catccacaac tgtttcccct ttaattatgc ctgataataa tggatcatcc atggatgact 2220
gggaagagag acaaactagt ggtaggataa cggaagagtt tcttggcaaa tatctgtcta 2280
ctacaccttt tccatcacag catcgtacag aaatagaatt gtttccttat tctggtgata 2340
aaatattagt agagggaatt tccacagtta tttatccttc tctacaaaca gaaatgacac 2400
atagaagaga aagaacagaa acactaatac cagagatgag aacagatact tatacagatg 2460
aaatacaaga agagatcact aaaagtccat ttatgggaaa aacagaagaa gaagtcttct 2520
ctgggatgaa actctctaca tctctctcag agccaattca tgttacagag tcttctgtgg 2580
aaatgaccaa gtcttttgat ttcccaacat tgataacaaa gttaagtgca gagccaacag 2640
aagtaagaga tatggaggaa gactttacag caactccagg tactacaaaa tatgatgaaa 2700
atattacaac agtgcttttg gcccatggta ctttaagtgt tgaagcagcc actgtatcaa 2760
aatggtcatg ggatgaagat aatacaacat ccaagccttt agagtctaca gaaccttcag 2820
cctcttcaaa attgccccct gccttactca caactgtggg gatgaatgga aaggataaag 2880
acatcccaag tttcactgaa gatggagcag atgaatttac tcttattcca gatagtactc 2940
aaaagcagtt agaggaggtt actgatgaag acatagcagc ccatggaaaa ttcacaatta 3000
gatttcagcc aactacatca actggtattg cagaaaagtc aactttgaga gattctacaa 3060
ctgaagaaaa agttccacct atcacaagca ctgaaggcca agtttatgca accatggaag 3120
gaagtgcttt gggtgaagta gaagatgtgg acctctctaa gccagtatct actgttcccc 3180
aatttgcaca cacttcagag gtggaaggat tagcatttgt tagttatagt agcacccaag 3240
agcctactac ttatgtagac tcttcccata ccattcctct ttctgtaatt cccaagacag 3300
actggggagt gttagtacct tctgttccat cagaagatga agttctaggt gaaccctctc 3360
aagacatact tgtcattgat cagactcgcc ttgaagcgac tatttctcca gaaactatga 3420
gaacaacaaa aatcacagag ggaacaactc aggaagaatt cccttggaaa gaacagactg 3480
cagagaaacc agttcctgct ctcagttcta cagcttggac tcccaaggag gcagtaacac 3540
cactggatga acaagagggc gatggatcag catatacagt ctctgaagat gaattgttga 3600

caggttctga gagggtccca gttttagaaa caactccagt tggaaaaatt gatcacagtg 3660
tgtcttatcc accaggtgct gtaactgagc acaaagtgaa aacagatgaa gtggtaacac 3720
taacaccacg cattgggcca aaagtatctt taagtccagg gcctgaacaa aaatatgaaa 3780
cagaaggtag tagtacaaca ggatttacat catctttgag tccttttagt acccacatta 3840
cccagcttat ggaagaaacc actactgaga aaacatccct agaggatatt gatttaggct 3900
caggattatt tgaaaagccc aaagccacag aactcataga attttcaaca atcaaagtca 3960
cagttccaag tgatattacc actgccttca gttcagtaga cagacttcac acaacttcag 4020
cattcaagcc atcttccgcg atcactaaga aaccacctct catcgacagg gaacctggtg 4080
aagaaacaac cagtgacatg gtaatcattg gagaatcaac atctcatgtt cctcccacta 4140
cccttgaaga tattgtagcc aaggaaacag aaaccgatat tgatagagag tatttcacga 4200
cttcaagtcc tcctgctaca cagccaacaa gaccacccac tgtggaagac aaagaggcct 4260
ttggacctca ggcgctttct acgccacagc cccagcaag cacaaaattt caccctgaca 4320
ttaatgttta tattattgag gtcagagaaa ataagacagg tcgaatgagt gatttgagtg 4380
taattggtca tccaatagat tcagaatcta aagaagatga accttgtagt gaagaaacag 4440
atccagtgca tgatctaatg gctgaaattt tacctgaatt ccctgacata attgaaatag 4500
acctatacca cagtgaagaa aatgaagaag aagaagaaga gtgtgcaaat gctactgatg 4560
tgacaaccac cccatctgtg cagtacataa atgggaagca tctcgttacc actgtgccca 4620
aggacccaga agctgcagaa gctaggcgtg gccagtttga aagtgttgca ccttctcaga 4680
atttctcgga cagctctgaa agtgatactc atccatttgt aatagccaaa acggaattgt 4740
ctactgctgt gcaacctaat gaatctacag aaacaactga gtctcttgaa gttacatgga 4800
agcctgagac ttaccctgaa acatcagaac atttttcagg tggtgagcct gatgttttcc 4860
ccacagtccc attccatgag gaatttgaaa gtggaacagc caaaaaaggg gcagaatcag 4920
tcacagagag agatactgaa gttggtcatc aggcacatga acatactgaa cctgtatctc 4980
tgtttcctga agagtcttca ggagagattg ccattgacca agaatctcag aaaatagcct 5040
ttgcaagggc tacagaagta acatttggtg aagaggtaga aaaaagtact tctgtcacat 5100
acactcccac tatagttcca agttctgcat cagcatatgt ttcagaggaa gaagcagtta 5160
ccctaatagg aaatccttgg ccagatgacc tgttgtctac caaagaaagc tgggtagaag 5220
caactcctag acaagttgta gagctctcag ggagttcttc gattccaatt acagaaggct 5280
ctggagaagc agaagaagat gaagatacaa tgttcaccat ggtaactgat ttatcacaga 5340
gaaatactac tgatacactc attactttag acactagcag gataatcaca gaaagctttt 5400
ttgaggttcc tgcaaccacc atttatccag tttctgaaca accttctgca aaagtggtgc 5460
ctaccaagtt tgtaagtgaa acagacactt ctgagtggat ttccagtacc actgttgagg 5520

aaaagaaaag gaaggaggag gagggaacta caggtacggc ttctacattt gaggtatatt 5580
catctacaca gagatcggat caattaattt taccctttga attagaaagt ccaaatgtag 5640
ctacatctag tgattcaggt accaggaaaa gttttatgtc cttgacaaca ccaacacagt 5700
ctgaaaggga aatgacagat tctactcctg tctttacaga aacaaataca ttagaaaatt 5760
tgggggcaca gaccactgag cacagcagta tccatcaacc tggggttcag gaagggctga 5820
ccactctccc acgtagtcct gcctctgtct ttatggagca gggctctgga gaagctgctg 5880
ccgacccaga aaccaccact gtttcttcat tttcattaaa cgtagagtat gcaattcaag 5940
ccgaaaagga agtagctggc actttgtctc cgcatgtgga aactacattc tccactgagc 6000
caacaggact ggttttgagt acagtaatgg acagagtagt tgctgaaaat ataacccaaa 6060
catccaggga aatagtgatt tcagagcgat taggagaacc aaattatggg gcagaaataa 6120
ggggcttttc cacaggtttt cctttggagg aagatttcag tggtgacttt agagaatact 6180
caacagtgtc tcatcccata gcaaaagaag aaacggtaat gatggaaggc tctggagatg 6240
cagcatttag ggacacccag acttcaccat ctacagtacc tacttcagtt cacatcagtc 6300
acatatctga ctcagaagga cccagtagca ccatggtcag cacttcagcc ttcccctggg 6360
aagagtttac atcctcagct gagggctcag gtgagcaact ggtcacagtc agcagctctg 6420
ttgttccagt gcttcccagt gctgtgcaaa agttttctgg tacagcttcc tccattatcg 6480
acgaaggatt gggagaagtg ggtactgtca atgaaattga tagaagatcc accattttac 6540
caacagcaga agtggaaggt acgaaagctc cagtagagaa ggaggaagta aaggtcagtg 6600
gcacagtttc aacaaacttt ccccaaacta tagagccagc caaattatgg tctaggcaag 6660
aagtcaaccc tgtaagacaa gaaattgaaa gtgaaacaac atcagaggaa caaattcaag 6720
aagaaaagtc atttgaatcc cctcaaaact ctcctgcaac agaacaaaca atctttgatt 6780
cacagacatt tactgaaact gaactcaaaa ccacagatta ttctgtacta acaacaaaga 6840
aaacttacag tgatgataaa gaaatgaagg aggaagacac ttctttagtt aacatgtcta 6900
ctccagatcc agatgcaaat ggcttggaat cttacacaac tctccctgaa gctactgaaa 6960
agtcacattt tttcttagct actgcattag taactgaatc tataccagct gaacatgtag 7020
tcacagattc accaatcaaa aaggaagaaa gtacaaaaca ttttccgaaa ggcatgagac 7080
caacaattca agagtcagat actgagctct tattctctgg actgggatca ggagaagaag 7140
ttttacctac tctaccaaca gagtcagtga attttactga agtggaacaa atcaataaca 7200
cattatatcc ccacacttct caagtggaaa gtacctcaag tgacaaaatt gaagacttta 7260
acagaatgga aaatgtggca aaagaagttg gaccactcgt atctcaaaca gacatctttg 7320
aaggtagtgg gtcagtaacc agcacaacat taatagaaat tttaagtgac actggagcag 7380

aaggacccac ggtggcacct ctccctttct ccacggacat cggacatcct caaaatcaga 7440
ctgtcaggtg ggcagaagaa atccagacta gtagaccaca aaccataact gaacaagact 7500
ctaacaagaa ttcttcaaca gcagaaatta acgaaacaac aacctcatct actgattttc 7560
tggctagagc ttatggtttt gaaatggcca aagaatttgt tacatcagca ccaaaaccat 7620
ctgacttgta ttatgaacct tctggagaag gatctggaga agtggatatt gttgattcat 7680
ttcacacttc tgcaactact caggcaacca gacaagaaag cagcaccaca tttgtttctg 7740
atgggtccct ggaaaaacat cctgaggtgc caagcgctaa agctgttact gctgatggat 7800
tcccaacagt ttcagtgatg ctgcctcttc attcagagca gaacaaaagc tcccctgatc 7860
caactagcac actgtcaaat acagtgtcat atgagaggtc cacagacggt agtttccaag 7920
accgtttcag ggaattcgag gattccacct taaaacctaa cagaaaaaaa cccactgaaa 7980
atattatcat agacctggac aaagaggaca aggatttaat attgacaatt acagagagta 8040
ccatccttga aattctacct gagctgacat cggataaaaa tactatcata gatattgatc 8100
atactaaacc tgtgtatgaa gacattcttg gaatgcaaac agatatagat acagaggtac 8160
catcagaacc acatgacagt aatgatgaaa gtaatgatga cagcactcaa gttcaagaga 8220
tctatgaggc agctgtcaac ctttctttaa ctgaggaaac atttgagggc tctgctgatg 8280
ttctggctag ctacactcag gcaacacatg atgaatcaat gacttatgaa gatagaagcc 8340
aactagatca catgggcttt cacttcacaa ctgggatccc tgctcctagc acagaaacag 8400
aattagacgt tttacttccc acggcaacat ccctgccaat tcctcgtaag tctgccacag 8460
ttattccaga gattgaagga ataaaagctg aagcaaaagc cctggatgac atgtttgaat 8520
caagcacttt gtctgatggt caagctattg cagaccaaag tgaaataata ccaacattgg 8580
gccaatttga aaggactcag gaggagtatg aagacaaaaa acatgctggt ccttcttttc 8640
agccagaatt ctcttcagga gctgaggagg cattagtaga ccatactccc tatctaagta 8700
ttgctactac ccaccttatg gatcagagtg taacagaggt gcctgatgtg atggaaggat 8760
ccaatccccc atattacact gatacaacat tagcagtttc aacatttgcg aagttgtctt 8820
ctcagacacc atcatctccc ctcactatct actcaggcag tgaagcctct ggacacacag 8880
agatccccca gcccagtgct ctgccaggaa tagacgtcgg ctcatctgta atgtccccac 8940
aggattcttt taaggaaatt catgtaaata ttgaagcgac tttcaaacca tcaagtgagg 9000
aataccttca cataactgag cctccctctt tatctcctga cacaaaatta gaaccttcag 9060
aagatgatgg taaacctgag ttattagaag aaatggaagc ttctcccaca gaacttattg 9120
ctgtggaagg aactgagatt ctccaagatt tccaaaacaa aaccgatggt caagtttctg 9180
gagaagcaat caagatgttt cccaccatta aaacacctga ggctggaact gttattacaa 9240
ctgccgatga aattgaatta gaaggtgcta cacagtggcc acactctact tctgcttctg 9300

ccacctatgg ggtcgaggca ggtgtggtgc cttggctaag tccacagact tctgagaggc 9360
ccacgctttc ttcttctcca gaaataaacc ctgaaactca agcagcttta atcagagggc 9420
aggattccac gatagcagca tcagaacagc aagtggcagc gagaattctt gattccaatg 9480
atcaggcaac agtaaaccct gtggaattta atactgaggt tgcaacacca ccattttccc 9540
ttctggagac ttctaatgaa acagatttcc tgattggcat taatgaagag tcagtggaag 9600
gcacggcaat ctatttacca ggacctgatc gctgcaaaat gaacccgtgc cttaacggag 9660
gcacctgtta tcctactgaa acttcctacg tatgcacctg tgtgccagga tacagcggag 9720
accagtgtga acttgatttt gatgaatgtc actctaatcc ctgtcgtaat ggagccactt 9780
gtgttgatgg ttttaacaca ttcaggtgcc tctgccttcc aagttatgtt ggtgcacttt 9840
gtgagcaaga taccgagaca tgtgactatg gctggcacaa attccaaggg cagtgctaca 9900
aatactttgc ccatcgacgc acatgggatg cagctgaacg ggaatgccgt ctgcagggtg 9960
cccatctcac aagcatcctg tctcacgaag aacaaatgtt tgttaatcgt gtgggccatg 10020
attatcagtg gataggcctc aatgacaaga tgtttgagca tgacttccgt tggactgatg 10080
gcagcacact gcaatacgag aattggagac ccaaccagcc agacagcttc ttttctgctg 10140
gagaagactg tgttgtaatc atttggcatg agaatggcca gtggaatgat gttccctgca 10200
attaccatct cacctatacg tgcaagaaag gaacagtcgc ttgcggccag ccccctgttg 10260
tagaaaatgc caagaccttt ggaaagatga aacctcgtta tgaaatcaac tccctgatta 10320
gataccactg caaagatggt ttcattcaac gtcaccttcc aactatccgg tgcttaggaa 10380
atggaagatg ggctatacct aaaattacct gcatgaaccc atctgcatac caaaggactt 10440
attctatgaa atactttaaa aattcctcat cagcaaagga caattcaata aatacatcca 10500
aacatgatca tcgttggagc cggaggtggc aggagtcgag gcgctgatcc ctaaaatggc 10560
gaacatgtgt tttcatcatt tcagccaaag tcctaacttc ctgtgccttt cctatcacct 10620
cgagaagtaa ttatcagttg gtttggattt ttggaccacc gttcagtcat tttgggttgc 10680
cgtgctccca aaacatttta aatgaaagta ttggcattca aaaagacagc agacaaaatg 10740
aaagaaaatg agagcagaaa gtaagcattt ccagcctatc taatttcttt agttttctat 10800
ttgcctccag tgcagtccat ttcctaatgt ataccagcct actgtactat ttaaaatgct 10860
caatttcagc accgatggcc atgtaaataa gatgatttaa tgttgatttt aatcctgtat 10920
ataaaataaa aagtcacaat gagtttgggc atatttaatg atgattatgg agccttagag 10980
gtctttaatc attggttcgg ctgcttttat gtagtttagg ctggaaatgg tttcacttgc 11040
tctttgactg tcagcaagac tgaagatggc ttttcctgga cagctagaaa acacaaaatc 11100
ttgtaggtca ttgcacctat ctcagccata ggtgcagttt gcttctacat gatgctaaag 11160

```
gctgcgaatg ggatcctgat ggaactaagg actccaatgt cgaactcttc tttgctgcat     11220
tcctttttct tcacttacaa gaaaggcctg aatggaggac ttttctgtaa ccaggaacat     11280
tttttagggg tcaaagtgct aataattaac tcaaccaggt ctacttttta atggctttca     11340
taacactaac tcataaggtt accgatcaat gcatttcata cggatataga cctagggctc     11400
tggagggtgg gggattgtta aaacacatgc aaaaaaaaaa aaaaaaaaaa aaaaagaaat     11460
tttgtatata taaccatttt aatcttttat aaagttttga atgttcatgt atgaatgctg     11520
cagctgtgaa gcatacataa ataaatgaag taagccatac tgatttaatt tattggatgt     11580
tattttccct aagacctgaa aatgaacata gtatgctagt tatttttcag tgttagcctt     11640
ttactttcct cacacaattt ggaatcatat aatataggta ctttgtccct gattaaataa     11700
tgtgacggat agaatgcatc aagtgtttat tatgaaaaga gtggaaaagt atatagcttt     11760
tagcaaaagg tgtttgccca ttctaagaaa tgagcgaata tatagaaata gtgtgggcat     11820
ttcttcctgt taggtggagt gtatgtgttg acatttctcc ccatctcttc ccactctgtt     11880
ttctccccat tatttgaata aagtgactgc tgaagatgac tttgaatcct tatccactta     11940
atttaatgtt taaagaaaaa cctgtaatgg aaagtaagac tccttcccta atttcagttt     12000
agagcaactt gaagaagagt agacaaaaaa taaaatgcac atagaaaaag agaaaaaggg     12060
cacaaaggga ttggcccaat attgattctt tttttataaa acctcctttg gcttagaagg     12120
aatgactcta gctacaataa tacacagtat gtttaagcag gttcccttgg ttgttgcatt     12180
aaatgtaatc cacctttagg tattttagag cacagaacaa cactgtgttg atctagtagg     12240
tttctatttt tcctttctct ttacaatgca cataatactt tcctgtattt atatcataac     12300
gtgtatagtg taaaatgtga atgacttttt ttgtgaatga aaatctaaaa tctttgtaac     12360
tttttatatc tgcttttgtt tcaccaaaga aacctaaaat ccttctttta ctacac         12416
```

<210> 126
<211> 655
<212> PRT
<213> Homo sapiens

<400> 126

```
Met Phe Ile Asn Ile Lys Ser Ile Leu Trp Met Cys Ser Thr Leu Ile
1               5                   10                  15

Val Thr His Ala Leu His Lys Val Lys Val Gly Lys Ser Pro Pro Val
            20                  25                  30

Arg Gly Ser Leu Ser Gly Lys Val Ser Leu Pro Cys His Phe Ser Thr
        35                  40                  45

Met Pro Thr Leu Pro Pro Ser Tyr Asn Thr Ser Glu Phe Leu Arg Ile
```

```
    50                  55                  60

Lys Trp Ser Lys Ile Glu Val Asp Lys Asn Gly Lys Asp Leu Lys Glu
65                  70                  75                  80

Thr Thr Val Leu Val Ala Gln Asn Gly Asn Ile Lys Ile Gly Gln Asp
                85                  90                  95

Tyr Lys Gly Arg Val Ser Val Pro Thr His Pro Glu Ala Val Gly Asp
            100                 105                 110

Ala Ser Leu Thr Val Val Lys Leu Leu Ala Ser Asp Ala Gly Leu Tyr
        115                 120                 125

Arg Cys Asp Val Met Tyr Gly Ile Glu Asp Thr Gln Asp Thr Val Ser
    130                 135                 140

Leu Thr Val Asp Gly Val Val Phe His Tyr Arg Ala Ala Thr Ser Arg
145                 150                 155                 160

Tyr Thr Leu Asn Phe Glu Ala Ala Gln Lys Ala Cys Leu Asp Val Gly
                165                 170                 175

Ala Val Ile Ala Thr Pro Glu Gln Leu Phe Ala Ala Tyr Glu Asp Gly
            180                 185                 190

Phe Glu Gln Cys Asp Ala Gly Trp Leu Ala Asp Gln Thr Val Arg Tyr
        195                 200                 205

Pro Ile Arg Ala Pro Arg Val Gly Cys Tyr Gly Asp Lys Met Gly Lys
    210                 215                 220

Ala Gly Val Arg Thr Tyr Gly Phe Arg Ser Pro Gln Glu Thr Tyr Asp
225                 230                 235                 240

Val Tyr Cys Tyr Val Asp His Leu Asp Gly Asp Val Phe His Leu Thr
                245                 250                 255

Val Pro Ser Lys Phe Thr Phe Glu Glu Ala Ala Lys Glu Cys Glu Asn
            260                 265                 270

Gln Asp Ala Arg Leu Ala Thr Val Gly Glu Leu Gln Ala Ala Trp Arg
        275                 280                 285

Asn Gly Phe Asp Gln Cys Asp Tyr Gly Trp Leu Ser Asp Ala Ser Val
    290                 295                 300
```

```
Arg His Pro Val Thr Val Ala Arg Ala Gln Cys Gly Gly Gly Leu Leu
305                 310                 315                 320

Gly Val Arg Thr Leu Tyr Arg Phe Glu Asn Gln Thr Gly Phe Pro Pro
                325                 330                 335

Pro Asp Ser Arg Phe Asp Ala Tyr Cys Phe Lys Arg Pro Asp Arg Cys
            340                 345                 350

Lys Met Asn Pro Cys Leu Asn Gly Gly Thr Cys Tyr Pro Thr Glu Thr
        355                 360                 365

Ser Tyr Val Cys Thr Cys Val Pro Gly Tyr Ser Gly Asp Gln Cys Glu
    370                 375                 380

Leu Asp Phe Asp Glu Cys His Ser Asn Pro Cys Arg Asn Gly Ala Thr
385                 390                 395                 400

Cys Val Asp Gly Phe Asn Thr Phe Arg Cys Leu Cys Leu Pro Ser Tyr
                405                 410                 415

Val Gly Ala Leu Cys Glu Gln Asp Thr Glu Thr Cys Asp Tyr Gly Trp
            420                 425                 430

His Lys Phe Gln Gly Gln Cys Tyr Lys Tyr Phe Ala His Arg Arg Thr
        435                 440                 445

Trp Asp Ala Ala Glu Arg Glu Cys Arg Leu Gln Gly Ala His Leu Thr
    450                 455                 460

Ser Ile Leu Ser His Glu Glu Gln Met Phe Val Asn Arg Val Gly His
465                 470                 475                 480

Asp Tyr Gln Trp Ile Gly Leu Asn Asp Lys Met Phe Glu His Asp Phe
                485                 490                 495

Arg Trp Thr Asp Gly Ser Thr Leu Gln Tyr Glu Asn Trp Arg Pro Asn
            500                 505                 510

Gln Pro Asp Ser Phe Phe Ser Ala Gly Glu Asp Cys Val Val Ile Ile
        515                 520                 525

Trp His Glu Asn Gly Gln Trp Asn Asp Val Pro Cys Asn Tyr His Leu
    530                 535                 540

Thr Tyr Thr Cys Lys Lys Gly Thr Val Ala Cys Gly Gln Pro Pro Val
545                 550                 555                 560
```

```
Val Glu Asn Ala Lys Thr Phe Gly Lys Met Lys Pro Arg Tyr Glu Ile
                565                 570                 575

Asn Ser Leu Ile Arg Tyr His Cys Lys Asp Gly Phe Ile Gln Arg His
            580                 585                 590

Leu Pro Thr Ile Arg Cys Leu Gly Asn Gly Arg Trp Ala Ile Pro Lys
        595                 600                 605

Ile Thr Cys Met Asn Pro Ser Ala Tyr Gln Arg Thr Tyr Ser Met Lys
    610                 615                 620

Tyr Phe Lys Asn Ser Ser Ser Ala Lys Asp Asn Ser Ile Asn Thr Ser
625                 630                 635                 640

Lys His Asp His Arg Trp Ser Arg Arg Trp Gln Glu Ser Arg Arg
                645                 650                 655
```

<210> 127
<211> 2409
<212> PRT
<213> Homo sapiens

<400> 127

```
Met Phe Ile Asn Ile Lys Ser Ile Leu Trp Met Cys Ser Thr Leu Ile
1               5                   10                  15

Val Thr His Ala Leu His Lys Val Lys Val Gly Lys Ser Pro Pro Val
            20                  25                  30

Arg Gly Ser Leu Ser Gly Lys Val Ser Leu Pro Cys His Phe Ser Thr
        35                  40                  45

Met Pro Thr Leu Pro Pro Ser Tyr Asn Thr Ser Glu Phe Leu Arg Ile
    50                  55                  60

Lys Trp Ser Lys Ile Glu Val Asp Lys Asn Gly Lys Asp Leu Lys Glu
65                  70                  75                  80

Thr Thr Val Leu Val Ala Gln Asn Gly Asn Ile Lys Ile Gly Gln Asp
                85                  90                  95

Tyr Lys Gly Arg Val Ser Val Pro Thr His Pro Glu Ala Val Gly Asp
            100                 105                 110

Ala Ser Leu Thr Val Val Lys Leu Leu Ala Ser Asp Ala Gly Leu Tyr
        115                 120                 125
```

Arg Cys Asp Val Met Tyr Gly Ile Glu Asp Thr Gln Asp Thr Val Ser
130 135 140

Leu Thr Val Asp Gly Val Val Phe His Tyr Arg Ala Ala Thr Ser Arg
145 150 155 160

Tyr Thr Leu Asn Phe Glu Ala Ala Gln Lys Ala Cys Leu Asp Val Gly
165 170 175

Ala Val Ile Ala Thr Pro Glu Gln Leu Phe Ala Ala Tyr Glu Asp Gly
180 185 190

Phe Glu Gln Cys Asp Ala Gly Trp Leu Ala Asp Gln Thr Val Arg Tyr
195 200 205

Pro Ile Arg Ala Pro Arg Val Gly Cys Tyr Gly Asp Lys Met Gly Lys
210 215 220

Ala Gly Val Arg Thr Tyr Gly Phe Arg Ser Pro Gln Glu Thr Tyr Asp
225 230 235 240

Val Tyr Cys Tyr Val Asp His Leu Asp Gly Asp Val Phe His Leu Thr
245 250 255

Val Pro Ser Lys Phe Thr Phe Glu Glu Ala Ala Lys Glu Cys Glu Asn
260 265 270

Gln Asp Ala Arg Leu Ala Thr Val Gly Glu Leu Gln Ala Ala Trp Arg
275 280 285

Asn Gly Phe Asp Gln Cys Asp Tyr Gly Trp Leu Ser Asp Ala Ser Val
290 295 300

Arg His Pro Val Thr Val Ala Arg Ala Gln Cys Gly Gly Gly Leu Leu
305 310 315 320

Gly Val Arg Thr Leu Tyr Arg Phe Glu Asn Gln Thr Gly Phe Pro Pro
325 330 335

Pro Asp Ser Arg Phe Asp Ala Tyr Cys Phe Lys Arg Arg Met Ser Asp
340 345 350

Leu Ser Val Ile Gly His Pro Ile Asp Ser Glu Ser Lys Glu Asp Glu
355 360 365

Pro Cys Ser Glu Glu Thr Asp Pro Val His Asp Leu Met Ala Glu Ile
370 375 380

```
Leu Pro Glu Phe Pro Asp Ile Ile Glu Ile Asp Leu Tyr His Ser Glu
385                 390                 395                 400

Glu Asn Glu Glu Glu Glu Glu Glu Cys Ala Asn Ala Thr Asp Val Thr
                405                 410                 415

Thr Thr Pro Ser Val Gln Tyr Ile Asn Gly Lys His Leu Val Thr Thr
            420                 425                 430

Val Pro Lys Asp Pro Glu Ala Ala Glu Ala Arg Arg Gly Gln Phe Glu
        435                 440                 445

Ser Val Ala Pro Ser Gln Asn Phe Ser Asp Ser Ser Glu Ser Asp Thr
    450                 455                 460

His Pro Phe Val Ile Ala Lys Thr Glu Leu Ser Thr Ala Val Gln Pro
465                 470                 475                 480

Asn Glu Ser Thr Glu Thr Thr Glu Ser Leu Glu Val Thr Trp Lys Pro
                485                 490                 495

Glu Thr Tyr Pro Glu Thr Ser Glu His Phe Ser Gly Gly Glu Pro Asp
            500                 505                 510

Val Phe Pro Thr Val Pro Phe His Glu Glu Phe Glu Ser Gly Thr Ala
        515                 520                 525

Lys Lys Gly Ala Glu Ser Val Thr Glu Arg Asp Thr Glu Val Gly His
    530                 535                 540

Gln Ala His Glu His Thr Glu Pro Val Ser Leu Phe Pro Glu Glu Ser
545                 550                 555                 560

Ser Gly Glu Ile Ala Ile Asp Gln Glu Ser Gln Lys Ile Ala Phe Ala
                565                 570                 575

Arg Ala Thr Glu Val Thr Phe Gly Glu Glu Val Glu Lys Ser Thr Ser
            580                 585                 590

Val Thr Tyr Thr Pro Thr Ile Val Pro Ser Ser Ala Ser Ala Tyr Val
        595                 600                 605

Ser Glu Glu Glu Ala Val Thr Leu Ile Gly Asn Pro Trp Pro Asp Asp
    610                 615                 620

Leu Leu Ser Thr Lys Glu Ser Trp Val Glu Ala Thr Pro Arg Gln Val
```

625 630 635 640

Val Glu Leu Ser Gly Ser Ser Ser Ile Pro Ile Thr Glu Gly Ser Gly
645 650 655

Glu Ala Glu Glu Asp Glu Asp Thr Met Phe Thr Met Val Thr Asp Leu
660 665 670

Ser Gln Arg Asn Thr Thr Asp Thr Leu Ile Thr Leu Asp Thr Ser Arg
675 680 685

Ile Ile Thr Glu Ser Phe Phe Glu Val Pro Ala Thr Thr Ile Tyr Pro
690 695 700

Val Ser Glu Gln Pro Ser Ala Lys Val Val Pro Thr Lys Phe Val Ser
705 710 715 720

Glu Thr Asp Thr Ser Glu Trp Ile Ser Ser Thr Thr Val Glu Glu Lys
725 730 735

Lys Arg Lys Glu Glu Glu Gly Thr Thr Gly Thr Ala Ser Thr Phe Glu
740 745 750

Val Tyr Ser Ser Thr Gln Arg Ser Asp Gln Leu Ile Leu Pro Phe Glu
755 760 765

Leu Glu Ser Pro Asn Val Ala Thr Ser Ser Asp Ser Gly Thr Arg Lys
770 775 780

Ser Phe Met Ser Leu Thr Thr Pro Thr Gln Ser Glu Arg Glu Met Thr
785 790 795 800

Asp Ser Thr Pro Val Phe Thr Glu Thr Asn Thr Leu Glu Asn Leu Gly
805 810 815

Ala Gln Thr Thr Glu His Ser Ser Ile His Gln Pro Gly Val Gln Glu
820 825 830

Gly Leu Thr Thr Leu Pro Arg Ser Pro Ala Ser Val Phe Met Glu Gln
835 840 845

Gly Ser Gly Glu Ala Ala Ala Asp Pro Glu Thr Thr Thr Val Ser Ser
850 855 860

Phe Ser Leu Asn Val Glu Tyr Ala Ile Gln Ala Glu Lys Glu Val Ala
865 870 875 880

```
Gly Thr Leu Ser Pro His Val Glu Thr Thr Phe Ser Thr Glu Pro Thr
                885                 890                 895

Gly Leu Val Leu Ser Thr Val Met Asp Arg Val Val Ala Glu Asn Ile
            900                 905                 910

Thr Gln Thr Ser Arg Glu Ile Val Ile Ser Glu Arg Leu Gly Glu Pro
        915                 920                 925

Asn Tyr Gly Ala Glu Ile Arg Gly Phe Ser Thr Gly Phe Pro Leu Glu
    930                 935                 940

Glu Asp Phe Ser Gly Asp Phe Arg Glu Tyr Ser Thr Val Ser His Pro
945                 950                 955                 960

Ile Ala Lys Glu Glu Thr Val Met Met Glu Gly Ser Gly Asp Ala Ala
                965                 970                 975

Phe Arg Asp Thr Gln Thr Ser Pro Ser Thr Val Pro Thr Ser Val His
            980                 985                 990

Ile Ser His Ile Ser Asp Ser Glu  Gly Pro Ser Ser Thr  Met Val Ser
        995                 1000                1005

Thr Ser  Ala Phe Pro Trp Glu  Glu Phe Thr Ser Ser  Ala Glu Gly
    1010                 1015                 1020

Ser Gly  Glu Gln Leu Val Thr  Val Ser Ser Ser Val  Val Pro Val
    1025                 1030                 1035

Leu Pro  Ser Ala Val Gln Lys  Phe Ser Gly Thr Ala  Ser Ser Ile
    1040                 1045                 1050

Ile Asp  Glu Gly Leu Gly Glu  Val Gly Thr Val Asn  Glu Ile Asp
    1055                 1060                 1065

Arg Arg  Ser Thr Ile Leu Pro  Thr Ala Glu Val Glu  Gly Thr Lys
    1070                 1075                 1080

Ala Pro  Val Glu Lys Glu Glu  Val Lys Val Ser Gly  Thr Val Ser
    1085                 1090                 1095

Thr Asn  Phe Pro Gln Thr Ile  Glu Pro Ala Lys Leu  Trp Ser Arg
    1100                 1105                 1110

Gln Glu  Val Asn Pro Val Arg  Gln Glu Ile Glu Ser  Glu Thr Thr
    1115                 1120                 1125
```

```
Ser Glu  Glu Gln Ile Gln Glu  Glu Lys Ser Phe Glu  Ser Pro Gln
    1130                 1135                 1140

Asn Ser  Pro Ala Thr Glu Gln  Thr Ile Phe Asp Ser  Gln Thr Phe
    1145                 1150                 1155

Thr Glu  Thr Glu Leu Lys Thr  Thr Asp Tyr Ser Val  Leu Thr Thr
    1160                 1165                 1170

Lys Lys  Thr Tyr Ser Asp Asp  Lys Glu Met Lys Glu  Glu Asp Thr
    1175                 1180                 1185

Ser Leu  Val Asn Met Ser Thr  Pro Asp Pro Asp Ala  Asn Gly Leu
    1190                 1195                 1200

Glu Ser  Tyr Thr Thr Leu Pro  Glu Ala Thr Glu Lys  Ser His Phe
    1205                 1210                 1215

Phe Leu  Ala Thr Ala Leu Val  Thr Glu Ser Ile Pro  Ala Glu His
    1220                 1225                 1230

Val Val  Thr Asp Ser Pro Ile  Lys Lys Glu Glu Ser  Thr Lys His
    1235                 1240                 1245

Phe Pro  Lys Gly Met Arg Pro  Thr Ile Gln Glu Ser  Asp Thr Glu
    1250                 1255                 1260

Leu Leu  Phe Ser Gly Leu Gly  Ser Gly Glu Glu Val  Leu Pro Thr
    1265                 1270                 1275

Leu Pro  Thr Glu Ser Val Asn  Phe Thr Glu Val Glu  Gln Ile Asn
    1280                 1285                 1290

Asn Thr  Leu Tyr Pro His Thr  Ser Gln Val Glu Ser  Thr Ser Ser
    1295                 1300                 1305

Asp Lys  Ile Glu Asp Phe Asn  Arg Met Glu Asn Val  Ala Lys Glu
    1310                 1315                 1320

Val Gly  Pro Leu Val Ser Gln  Thr Asp Ile Phe Glu  Gly Ser Gly
    1325                 1330                 1335

Ser Val  Thr Ser Thr Thr Leu  Ile Glu Ile Leu Ser  Asp Thr Gly
    1340                 1345                 1350

Ala Glu  Gly Pro Thr Val Ala  Pro Leu Pro Phe Ser  Thr Asp Ile
    1355                 1360                 1365
```

Gly His Pro Gln Asn Gln Thr Val Arg Trp Ala Glu Glu Ile Gln
1370 1375 1380

Thr Ser Arg Pro Gln Thr Ile Thr Glu Gln Asp Ser Asn Lys Asn
1385 1390 1395

Ser Ser Thr Ala Glu Ile Asn Glu Thr Thr Thr Ser Ser Thr Asp
1400 1405 1410

Phe Leu Ala Arg Ala Tyr Gly Phe Glu Met Ala Lys Glu Phe Val
1415 1420 1425

Thr Ser Ala Pro Lys Pro Ser Asp Leu Tyr Tyr Glu Pro Ser Gly
1430 1435 1440

Glu Gly Ser Gly Glu Val Asp Ile Val Asp Ser Phe His Thr Ser
1445 1450 1455

Ala Thr Thr Gln Ala Thr Arg Gln Glu Ser Ser Thr Thr Phe Val
1460 1465 1470

Ser Asp Gly Ser Leu Glu Lys His Pro Glu Val Pro Ser Ala Lys
1475 1480 1485

Ala Val Thr Ala Asp Gly Phe Pro Thr Val Ser Val Met Leu Pro
1490 1495 1500

Leu His Ser Glu Gln Asn Lys Ser Ser Pro Asp Pro Thr Ser Thr
1505 1510 1515

Leu Ser Asn Thr Val Ser Tyr Glu Arg Ser Thr Asp Gly Ser Phe
1520 1525 1530

Gln Asp Arg Phe Arg Glu Phe Glu Asp Ser Thr Leu Lys Pro Asn
1535 1540 1545

Arg Lys Lys Pro Thr Glu Asn Ile Ile Ile Asp Leu Asp Lys Glu
1550 1555 1560

Asp Lys Asp Leu Ile Leu Thr Ile Thr Glu Ser Thr Ile Leu Glu
1565 1570 1575

Ile Leu Pro Glu Leu Thr Ser Asp Lys Asn Thr Ile Ile Asp Ile
1580 1585 1590

Asp His Thr Lys Pro Val Tyr Glu Asp Ile Leu Gly Met Gln Thr

1595 1600 1605

Asp Ile Asp Thr Glu Val Pro Ser Glu Pro His Asp Ser Asn Asp
1610 1615 1620

Glu Ser Asn Asp Asp Ser Thr Gln Val Gln Glu Ile Tyr Glu Ala
1625 1630 1635

Ala Val Asn Leu Ser Leu Thr Glu Glu Thr Phe Glu Gly Ser Ala
1640 1645 1650

Asp Val Leu Ala Ser Tyr Thr Gln Ala Thr His Asp Glu Ser Met
1655 1660 1665

Thr Tyr Glu Asp Arg Ser Gln Leu Asp His Met Gly Phe His Phe
1670 1675 1680

Thr Thr Gly Ile Pro Ala Pro Ser Thr Glu Thr Glu Leu Asp Val
1685 1690 1695

Leu Leu Pro Thr Ala Thr Ser Leu Pro Ile Pro Arg Lys Ser Ala
1700 1705 1710

Thr Val Ile Pro Glu Ile Glu Gly Ile Lys Ala Glu Ala Lys Ala
1715 1720 1725

Leu Asp Asp Met Phe Glu Ser Ser Thr Leu Ser Asp Gly Gln Ala
1730 1735 1740

Ile Ala Asp Gln Ser Glu Ile Ile Pro Thr Leu Gly Gln Phe Glu
1745 1750 1755

Arg Thr Gln Glu Glu Tyr Glu Asp Lys Lys His Ala Gly Pro Ser
1760 1765 1770

Phe Gln Pro Glu Phe Ser Ser Gly Ala Glu Glu Ala Leu Val Asp
1775 1780 1785

His Thr Pro Tyr Leu Ser Ile Ala Thr Thr His Leu Met Asp Gln
1790 1795 1800

Ser Val Thr Glu Val Pro Asp Val Met Glu Gly Ser Asn Pro Pro
1805 1810 1815

Tyr Tyr Thr Asp Thr Thr Leu Ala Val Ser Thr Phe Ala Lys Leu
1820 1825 1830

Ser Ser Gln Thr Pro Ser Ser Pro Leu Thr Ile Tyr Ser Gly Ser
1835 1840 1845

Glu Ala Ser Gly His Thr Glu Ile Pro Gln Pro Ser Ala Leu Pro
1850 1855 1860

Gly Ile Asp Val Gly Ser Ser Val Met Ser Pro Gln Asp Ser Phe
1865 1870 1875

Lys Glu Ile His Val Asn Ile Glu Ala Thr Phe Lys Pro Ser Ser
1880 1885 1890

Glu Glu Tyr Leu His Ile Thr Glu Pro Pro Ser Leu Ser Pro Asp
1895 1900 1905

Thr Lys Leu Glu Pro Ser Glu Asp Asp Gly Lys Pro Glu Leu Leu
1910 1915 1920

Glu Glu Met Glu Ala Ser Pro Thr Glu Leu Ile Ala Val Glu Gly
1925 1930 1935

Thr Glu Ile Leu Gln Asp Phe Gln Asn Lys Thr Asp Gly Gln Val
1940 1945 1950

Ser Gly Glu Ala Ile Lys Met Phe Pro Thr Ile Lys Thr Pro Glu
1955 1960 1965

Ala Gly Thr Val Ile Thr Thr Ala Asp Glu Ile Glu Leu Glu Gly
1970 1975 1980

Ala Thr Gln Trp Pro His Ser Thr Ser Ala Ser Ala Thr Tyr Gly
1985 1990 1995

Val Glu Ala Gly Val Val Pro Trp Leu Ser Pro Gln Thr Ser Glu
2000 2005 2010

Arg Pro Thr Leu Ser Ser Ser Pro Glu Ile Asn Pro Glu Thr Gln
2015 2020 2025

Ala Ala Leu Ile Arg Gly Gln Asp Ser Thr Ile Ala Ala Ser Glu
2030 2035 2040

Gln Gln Val Ala Ala Arg Ile Leu Asp Ser Asn Asp Gln Ala Thr
2045 2050 2055

Val Asn Pro Val Glu Phe Asn Thr Glu Val Ala Thr Pro Pro Phe
2060 2065 2070

```
Ser Leu  Leu Glu Thr Ser Asn  Glu Thr Asp Phe Leu  Ile Gly Ile
    2075                 2080                 2085

Asn Glu  Glu Ser Val Glu Gly  Thr Ala Ile Tyr Leu  Pro Gly Pro
    2090                 2095                 2100

Asp Arg  Cys Lys Met Asn Pro  Cys Leu Asn Gly Gly  Thr Cys Tyr
    2105                 2110                 2115

Pro Thr  Glu Thr Ser Tyr Val  Cys Thr Cys Val Pro  Gly Tyr Ser
    2120                 2125                 2130

Gly Asp  Gln Cys Glu Leu Asp  Phe Asp Glu Cys His  Ser Asn Pro
    2135                 2140                 2145

Cys Arg  Asn Gly Ala Thr Cys  Val Asp Gly Phe Asn  Thr Phe Arg
    2150                 2155                 2160

Cys Leu  Cys Leu Pro Ser Tyr  Val Gly Ala Leu Cys  Glu Gln Asp
    2165                 2170                 2175

Thr Glu  Thr Cys Asp Tyr Gly  Trp His Lys Phe Gln  Gly Gln Cys
    2180                 2185                 2190

Tyr Lys  Tyr Phe Ala His Arg  Arg Thr Trp Asp Ala  Ala Glu Arg
    2195                 2200                 2205

Glu Cys  Arg Leu Gln Gly Ala  His Leu Thr Ser Ile  Leu Ser His
    2210                 2215                 2220

Glu Glu  Gln Met Phe Val Asn  Arg Val Gly His Asp  Tyr Gln Trp
    2225                 2230                 2235

Ile Gly  Leu Asn Asp Lys Met  Phe Glu His Asp Phe  Arg Trp Thr
    2240                 2245                 2250

Asp Gly  Ser Thr Leu Gln Tyr  Glu Asn Trp Arg Pro  Asn Gln Pro
    2255                 2260                 2265

Asp Ser  Phe Phe Ser Ala Gly  Glu Asp Cys Val Val  Ile Ile Trp
    2270                 2275                 2280

His Glu  Asn Gly Gln Trp Asn  Asp Val Pro Cys Asn  Tyr His Leu
    2285                 2290                 2295

Thr Tyr  Thr Cys Lys Lys Gly  Thr Val Ala Cys Gly  Gln Pro Pro
    2300                 2305                 2310
```

Val Val Glu Asn Ala Lys Thr Phe Gly Lys Met Lys Pro Arg Tyr
2315 2320 2325

Glu Ile Asn Ser Leu Ile Arg Tyr His Cys Lys Asp Gly Phe Ile
2330 2335 2340

Gln Arg His Leu Pro Thr Ile Arg Cys Leu Gly Asn Gly Arg Trp
2345 2350 2355

Ala Ile Pro Lys Ile Thr Cys Met Asn Pro Ser Ala Tyr Gln Arg
2360 2365 2370

Thr Tyr Ser Met Lys Tyr Phe Lys Asn Ser Ser Ser Ala Lys Asp
2375 2380 2385

Asn Ser Ile Asn Thr Ser Lys His Asp His Arg Trp Ser Arg Arg
2390 2395 2400

Trp Gln Glu Ser Arg Arg
2405

<210> 128
<211> 1642
<212> PRT
<213> Homo sapiens

<400> 128

Met Phe Ile Asn Ile Lys Ser Ile Leu Trp Met Cys Ser Thr Leu Ile
1 5 10 15

Val Thr His Ala Leu His Lys Val Lys Val Gly Lys Ser Pro Pro Val
20 25 30

Arg Gly Ser Leu Ser Gly Lys Val Ser Leu Pro Cys His Phe Ser Thr
35 40 45

Met Pro Thr Leu Pro Pro Ser Tyr Asn Thr Ser Glu Phe Leu Arg Ile
50 55 60

Lys Trp Ser Lys Ile Glu Val Asp Lys Asn Gly Lys Asp Leu Lys Glu
65 70 75 80

Thr Thr Val Leu Val Ala Gln Asn Gly Asn Ile Lys Ile Gly Gln Asp
85 90 95

Tyr Lys Gly Arg Val Ser Val Pro Thr His Pro Glu Ala Val Gly Asp
100 105 110

```
Ala Ser Leu Thr Val Val Lys Leu Leu Ala Ser Asp Ala Gly Leu Tyr
        115                 120                 125

Arg Cys Asp Val Met Tyr Gly Ile Glu Asp Thr Gln Asp Thr Val Ser
    130                 135                 140

Leu Thr Val Asp Gly Val Val Phe His Tyr Arg Ala Ala Thr Ser Arg
145                 150                 155                 160

Tyr Thr Leu Asn Phe Glu Ala Ala Gln Lys Ala Cys Leu Asp Val Gly
                165                 170                 175

Ala Val Ile Ala Thr Pro Glu Gln Leu Phe Ala Ala Tyr Glu Asp Gly
            180                 185                 190

Phe Glu Gln Cys Asp Ala Gly Trp Leu Ala Asp Gln Thr Val Arg Tyr
        195                 200                 205

Pro Ile Arg Ala Pro Arg Val Gly Cys Tyr Gly Asp Lys Met Gly Lys
    210                 215                 220

Ala Gly Val Arg Thr Tyr Gly Phe Arg Ser Pro Gln Glu Thr Tyr Asp
225                 230                 235                 240

Val Tyr Cys Tyr Val Asp His Leu Asp Gly Asp Val Phe His Leu Thr
                245                 250                 255

Val Pro Ser Lys Phe Thr Phe Glu Glu Ala Ala Lys Glu Cys Glu Asn
            260                 265                 270

Gln Asp Ala Arg Leu Ala Thr Val Gly Glu Leu Gln Ala Ala Trp Arg
        275                 280                 285

Asn Gly Phe Asp Gln Cys Asp Tyr Gly Trp Leu Ser Asp Ala Ser Val
    290                 295                 300

Arg His Pro Val Thr Val Ala Arg Ala Gln Cys Gly Gly Gly Leu Leu
305                 310                 315                 320

Gly Val Arg Thr Leu Tyr Arg Phe Glu Asn Gln Thr Gly Phe Pro Pro
                325                 330                 335

Pro Asp Ser Arg Phe Asp Ala Tyr Cys Phe Lys Pro Lys Glu Ala Thr
            340                 345                 350

Thr Ile Asp Leu Ser Ile Leu Ala Glu Thr Ala Ser Pro Ser Leu Ser
```

355 360 365

Lys Glu Pro Gln Met Val Ser Asp Arg Thr Thr Pro Ile Ile Pro Leu
370 375 380

Val Asp Glu Leu Pro Val Ile Pro Thr Glu Phe Pro Pro Val Gly Asn
385 390 395 400

Ile Val Ser Phe Glu Gln Lys Ala Thr Val Gln Pro Gln Ala Ile Thr
405 410 415

Asp Ser Leu Ala Thr Lys Leu Pro Thr Pro Thr Gly Ser Thr Lys Lys
420 425 430

Pro Trp Asp Met Asp Asp Tyr Ser Pro Ser Ala Ser Gly Pro Leu Gly
435 440 445

Lys Leu Asp Ile Ser Glu Ile Lys Glu Glu Val Leu Gln Ser Thr Thr
450 455 460

Gly Val Ser His Tyr Ala Thr Asp Ser Trp Asp Gly Val Val Glu Asp
465 470 475 480

Lys Gln Thr Gln Glu Ser Val Thr Gln Ile Glu Gln Ile Glu Val Gly
485 490 495

Pro Leu Val Thr Ser Met Glu Ile Leu Lys His Ile Pro Ser Lys Glu
500 505 510

Phe Pro Val Thr Glu Thr Pro Leu Val Thr Ala Arg Met Ile Leu Glu
515 520 525

Ser Lys Thr Glu Lys Lys Met Val Ser Thr Val Ser Glu Leu Val Thr
530 535 540

Thr Gly His Tyr Gly Phe Thr Leu Gly Glu Glu Asp Asp Glu Asp Arg
545 550 555 560

Thr Leu Thr Val Gly Ser Asp Glu Ser Thr Leu Ile Phe Asp Gln Ile
565 570 575

Pro Glu Val Ile Thr Val Ser Lys Thr Ser Glu Asp Thr Ile His Thr
580 585 590

His Leu Glu Asp Leu Glu Ser Val Ser Ala Ser Thr Thr Val Ser Pro
595 600 605

```
Leu Ile Met Pro Asp Asn Asn Gly Ser Ser Met Asp Asp Trp Glu Glu
    610                 615                 620

Arg Gln Thr Ser Gly Arg Ile Thr Glu Glu Phe Leu Gly Lys Tyr Leu
625                 630                 635                 640

Ser Thr Thr Pro Phe Pro Ser Gln His Arg Thr Glu Ile Glu Leu Phe
                645                 650                 655

Pro Tyr Ser Gly Asp Lys Ile Leu Val Glu Gly Ile Ser Thr Val Ile
            660                 665                 670

Tyr Pro Ser Leu Gln Thr Glu Met Thr His Arg Arg Glu Arg Thr Glu
        675                 680                 685

Thr Leu Ile Pro Glu Met Arg Thr Asp Thr Tyr Thr Asp Glu Ile Gln
    690                 695                 700

Glu Glu Ile Thr Lys Ser Pro Phe Met Gly Lys Thr Glu Glu Glu Val
705                 710                 715                 720

Phe Ser Gly Met Lys Leu Ser Thr Ser Leu Ser Glu Pro Ile His Val
                725                 730                 735

Thr Glu Ser Ser Val Glu Met Thr Lys Ser Phe Asp Phe Pro Thr Leu
            740                 745                 750

Ile Thr Lys Leu Ser Ala Glu Pro Thr Glu Val Arg Asp Met Glu Glu
        755                 760                 765

Asp Phe Thr Ala Thr Pro Gly Thr Thr Lys Tyr Asp Glu Asn Ile Thr
    770                 775                 780

Thr Val Leu Leu Ala His Gly Thr Leu Ser Val Glu Ala Ala Thr Val
785                 790                 795                 800

Ser Lys Trp Ser Trp Asp Glu Asp Asn Thr Thr Ser Lys Pro Leu Glu
                805                 810                 815

Ser Thr Glu Pro Ser Ala Ser Ser Lys Leu Pro Pro Ala Leu Leu Thr
            820                 825                 830

Thr Val Gly Met Asn Gly Lys Asp Lys Asp Ile Pro Ser Phe Thr Glu
        835                 840                 845

Asp Gly Ala Asp Glu Phe Thr Leu Ile Pro Asp Ser Thr Gln Lys Gln
    850                 855                 860
```

```
Leu Glu Glu Val Thr Asp Glu Asp Ile Ala Ala His Gly Lys Phe Thr
865                 870                 875                 880

Ile Arg Phe Gln Pro Thr Thr Ser Thr Gly Ile Ala Glu Lys Ser Thr
                885                 890                 895

Leu Arg Asp Ser Thr Thr Glu Glu Lys Val Pro Pro Ile Thr Ser Thr
            900                 905                 910

Glu Gly Gln Val Tyr Ala Thr Met Glu Gly Ser Ala Leu Gly Glu Val
        915                 920                 925

Glu Asp Val Asp Leu Ser Lys Pro Val Ser Thr Val Pro Gln Phe Ala
    930                 935                 940

His Thr Ser Glu Val Glu Gly Leu Ala Phe Val Ser Tyr Ser Ser Thr
945                 950                 955                 960

Gln Glu Pro Thr Thr Tyr Val Asp Ser Ser His Thr Ile Pro Leu Ser
                965                 970                 975

Val Ile Pro Lys Thr Asp Trp Gly Val Leu Val Pro Ser Val Pro Ser
            980                 985                 990

Glu Asp Glu Val Leu Gly Glu Pro  Ser Gln Asp Ile Leu  Val Ile Asp
        995                 1000                 1005

Gln Thr  Arg Leu Glu Ala Thr  Ile Ser Pro Glu Thr  Met Arg Thr
    1010                 1015                 1020

Thr Lys  Ile Thr Glu Gly Thr  Thr Gln Glu Glu Phe  Pro Trp Lys
    1025                 1030                 1035

Glu Gln  Thr Ala Glu Lys Pro  Val Pro Ala Leu Ser  Ser Thr Ala
    1040                 1045                 1050

Trp Thr  Pro Lys Glu Ala Val  Thr Pro Leu Asp Glu  Gln Glu Gly
    1055                 1060                 1065

Asp Gly  Ser Ala Tyr Thr Val  Ser Glu Asp Glu Leu  Leu Thr Gly
    1070                 1075                 1080

Ser Glu  Arg Val Pro Val Leu  Glu Thr Thr Pro Val  Gly Lys Ile
    1085                 1090                 1095

Asp His  Ser Val Ser Tyr Pro  Pro Gly Ala Val Thr  Glu His Lys
    1100                 1105                 1110
```

```
Val Lys  Thr Asp Glu Val Val  Thr Leu Thr Pro Arg  Ile Gly Pro
    1115                 1120                 1125

Lys Val  Ser Leu Ser Pro Gly  Pro Glu Gln Lys Tyr  Glu Thr Glu
    1130                 1135                 1140

Gly Ser  Ser Thr Thr Gly Phe  Thr Ser Ser Leu Ser  Pro Phe Ser
    1145                 1150                 1155

Thr His  Ile Thr Gln Leu Met  Glu Glu Thr Thr Thr  Glu Lys Thr
    1160                 1165                 1170

Ser Leu  Glu Asp Ile Asp Leu  Gly Ser Gly Leu Phe  Glu Lys Pro
    1175                 1180                 1185

Lys Ala  Thr Glu Leu Ile Glu  Phe Ser Thr Ile Lys  Val Thr Val
    1190                 1195                 1200

Pro Ser  Asp Ile Thr Thr Ala  Phe Ser Ser Val Asp  Arg Leu His
    1205                 1210                 1215

Thr Thr  Ser Ala Phe Lys Pro  Ser Ser Ala Ile Thr  Lys Lys Pro
    1220                 1225                 1230

Pro Leu  Ile Asp Arg Glu Pro  Gly Glu Glu Thr Thr  Ser Asp Met
    1235                 1240                 1245

Val Ile  Ile Gly Glu Ser Thr  Ser His Val Pro Pro  Thr Thr Leu
    1250                 1255                 1260

Glu Asp  Ile Val Ala Lys Glu  Thr Glu Thr Asp Ile  Asp Arg Glu
    1265                 1270                 1275

Tyr Phe  Thr Thr Ser Ser Pro  Pro Ala Thr Gln Pro  Thr Arg Pro
    1280                 1285                 1290

Pro Thr  Val Glu Asp Lys Glu  Ala Phe Gly Pro Gln  Ala Leu Ser
    1295                 1300                 1305

Thr Pro  Gln Pro Pro Ala Ser  Thr Lys Phe His Pro  Asp Ile Asn
    1310                 1315                 1320

Val Tyr  Ile Ile Glu Val Arg  Glu Asn Lys Thr Gly  Pro Asp Arg
    1325                 1330                 1335

Cys Lys  Met Asn Pro Cys Leu  Asn Gly Gly Thr Cys  Tyr Pro Thr
```

1340 1345 1350

Glu Thr Ser Tyr Val Cys Thr Cys Val Pro Gly Tyr Ser Gly Asp
1355 1360 1365

Gln Cys Glu Leu Asp Phe Asp Glu Cys His Ser Asn Pro Cys Arg
1370 1375 1380

Asn Gly Ala Thr Cys Val Asp Gly Phe Asn Thr Phe Arg Cys Leu
1385 1390 1395

Cys Leu Pro Ser Tyr Val Gly Ala Leu Cys Glu Gln Asp Thr Glu
1400 1405 1410

Thr Cys Asp Tyr Gly Trp His Lys Phe Gln Gly Gln Cys Tyr Lys
1415 1420 1425

Tyr Phe Ala His Arg Arg Thr Trp Asp Ala Ala Glu Arg Glu Cys
1430 1435 1440

Arg Leu Gln Gly Ala His Leu Thr Ser Ile Leu Ser His Glu Glu
1445 1450 1455

Gln Met Phe Val Asn Arg Val Gly His Asp Tyr Gln Trp Ile Gly
1460 1465 1470

Leu Asn Asp Lys Met Phe Glu His Asp Phe Arg Trp Thr Asp Gly
1475 1480 1485

Ser Thr Leu Gln Tyr Glu Asn Trp Arg Pro Asn Gln Pro Asp Ser
1490 1495 1500

Phe Phe Ser Ala Gly Glu Asp Cys Val Val Ile Ile Trp His Glu
1505 1510 1515

Asn Gly Gln Trp Asn Asp Val Pro Cys Asn Tyr His Leu Thr Tyr
1520 1525 1530

Thr Cys Lys Lys Gly Thr Val Ala Cys Gly Gln Pro Pro Val Val
1535 1540 1545

Glu Asn Ala Lys Thr Phe Gly Lys Met Lys Pro Arg Tyr Glu Ile
1550 1555 1560

Asn Ser Leu Ile Arg Tyr His Cys Lys Asp Gly Phe Ile Gln Arg
1565 1570 1575

His Leu Pro Thr Ile Arg Cys Leu Gly Asn Gly Arg Trp Ala Ile
1580 1585 1590

Pro Lys Ile Thr Cys Met Asn Pro Ser Ala Tyr Gln Arg Thr Tyr
1595 1600 1605

Ser Met Lys Tyr Phe Lys Asn Ser Ser Ser Ala Lys Asp Asn Ser
1610 1615 1620

Ile Asn Thr Ser Lys His Asp His Arg Trp Ser Arg Arg Trp Gln
1625 1630 1635

Glu Ser Arg Arg
1640

<210> 129
<211> 3396
<212> PRT
<213> Homo sapiens

<400> 129

Met Phe Ile Asn Ile Lys Ser Ile Leu Trp Met Cys Ser Thr Leu Ile
1 5 10 15

Val Thr His Ala Leu His Lys Val Lys Val Gly Lys Ser Pro Pro Val
20 25 30

Arg Gly Ser Leu Ser Gly Lys Val Ser Leu Pro Cys His Phe Ser Thr
35 40 45

Met Pro Thr Leu Pro Pro Ser Tyr Asn Thr Ser Glu Phe Leu Arg Ile
50 55 60

Lys Trp Ser Lys Ile Glu Val Asp Lys Asn Gly Lys Asp Leu Lys Glu
65 70 75 80

Thr Thr Val Leu Val Ala Gln Asn Gly Asn Ile Lys Ile Gly Gln Asp
85 90 95

Tyr Lys Gly Arg Val Ser Val Pro Thr His Pro Glu Ala Val Gly Asp
100 105 110

Ala Ser Leu Thr Val Val Lys Leu Leu Ala Ser Asp Ala Gly Leu Tyr
115 120 125

Arg Cys Asp Val Met Tyr Gly Ile Glu Asp Thr Gln Asp Thr Val Ser
130 135 140

```
Leu Thr Val Asp Gly Val Val Phe His Tyr Arg Ala Ala Thr Ser Arg
145                 150                 155                 160

Tyr Thr Leu Asn Phe Glu Ala Ala Gln Lys Ala Cys Leu Asp Val Gly
                165                 170                 175

Ala Val Ile Ala Thr Pro Glu Gln Leu Phe Ala Ala Tyr Glu Asp Gly
            180                 185                 190

Phe Glu Gln Cys Asp Ala Gly Trp Leu Ala Asp Gln Thr Val Arg Tyr
        195                 200                 205

Pro Ile Arg Ala Pro Arg Val Gly Cys Tyr Gly Asp Lys Met Gly Lys
    210                 215                 220

Ala Gly Val Arg Thr Tyr Gly Phe Arg Ser Pro Gln Glu Thr Tyr Asp
225                 230                 235                 240

Val Tyr Cys Tyr Val Asp His Leu Asp Gly Asp Val Phe His Leu Thr
                245                 250                 255

Val Pro Ser Lys Phe Thr Phe Glu Glu Ala Ala Lys Glu Cys Glu Asn
            260                 265                 270

Gln Asp Ala Arg Leu Ala Thr Val Gly Glu Leu Gln Ala Ala Trp Arg
        275                 280                 285

Asn Gly Phe Asp Gln Cys Asp Tyr Gly Trp Leu Ser Asp Ala Ser Val
    290                 295                 300

Arg His Pro Val Thr Val Ala Arg Ala Gln Cys Gly Gly Gly Leu Leu
305                 310                 315                 320

Gly Val Arg Thr Leu Tyr Arg Phe Glu Asn Gln Thr Gly Phe Pro Pro
                325                 330                 335

Pro Asp Ser Arg Phe Asp Ala Tyr Cys Phe Lys Pro Lys Glu Ala Thr
            340                 345                 350

Thr Ile Asp Leu Ser Ile Leu Ala Glu Thr Ala Ser Pro Ser Leu Ser
        355                 360                 365

Lys Glu Pro Gln Met Val Ser Asp Arg Thr Thr Pro Ile Ile Pro Leu
    370                 375                 380

Val Asp Glu Leu Pro Val Ile Pro Thr Glu Phe Pro Pro Val Gly Asn
385                 390                 395                 400
```

```
Ile Val Ser Phe Glu Gln Lys Ala Thr Val Gln Pro Gln Ala Ile Thr
                405                 410                 415

Asp Ser Leu Ala Thr Lys Leu Pro Thr Pro Thr Gly Ser Thr Lys Lys
            420                 425                 430

Pro Trp Asp Met Asp Asp Tyr Ser Pro Ser Ala Ser Gly Pro Leu Gly
        435                 440                 445

Lys Leu Asp Ile Ser Glu Ile Lys Glu Glu Val Leu Gln Ser Thr Thr
    450                 455                 460

Gly Val Ser His Tyr Ala Thr Asp Ser Trp Asp Gly Val Val Glu Asp
465                 470                 475                 480

Lys Gln Thr Gln Glu Ser Val Thr Gln Ile Glu Gln Ile Glu Val Gly
                485                 490                 495

Pro Leu Val Thr Ser Met Glu Ile Leu Lys His Ile Pro Ser Lys Glu
            500                 505                 510

Phe Pro Val Thr Glu Thr Pro Leu Val Thr Ala Arg Met Ile Leu Glu
        515                 520                 525

Ser Lys Thr Glu Lys Lys Met Val Ser Thr Val Ser Glu Leu Val Thr
    530                 535                 540

Thr Gly His Tyr Gly Phe Thr Leu Gly Glu Glu Asp Asp Glu Asp Arg
545                 550                 555                 560

Thr Leu Thr Val Gly Ser Asp Glu Ser Thr Leu Ile Phe Asp Gln Ile
                565                 570                 575

Pro Glu Val Ile Thr Val Ser Lys Thr Ser Glu Asp Thr Ile His Thr
            580                 585                 590

His Leu Glu Asp Leu Glu Ser Val Ser Ala Ser Thr Thr Val Ser Pro
        595                 600                 605

Leu Ile Met Pro Asp Asn Asn Gly Ser Ser Met Asp Asp Trp Glu Glu
    610                 615                 620

Arg Gln Thr Ser Gly Arg Ile Thr Glu Glu Phe Leu Gly Lys Tyr Leu
625                 630                 635                 640

Ser Thr Thr Pro Phe Pro Ser Gln His Arg Thr Glu Ile Glu Leu Phe
                645                 650                 655
```

```
Pro Tyr Ser Gly Asp Lys Ile Leu Val Glu Gly Ile Ser Thr Val Ile
            660                 665                 670

Tyr Pro Ser Leu Gln Thr Glu Met Thr His Arg Arg Glu Arg Thr Glu
        675                 680                 685

Thr Leu Ile Pro Glu Met Arg Thr Asp Thr Tyr Thr Asp Glu Ile Gln
    690                 695                 700

Glu Glu Ile Thr Lys Ser Pro Phe Met Gly Lys Thr Glu Glu Glu Val
705                 710                 715                 720

Phe Ser Gly Met Lys Leu Ser Thr Ser Leu Ser Glu Pro Ile His Val
                725                 730                 735

Thr Glu Ser Ser Val Glu Met Thr Lys Ser Phe Asp Phe Pro Thr Leu
            740                 745                 750

Ile Thr Lys Leu Ser Ala Glu Pro Thr Glu Val Arg Asp Met Glu Glu
        755                 760                 765

Asp Phe Thr Ala Thr Pro Gly Thr Thr Lys Tyr Asp Glu Asn Ile Thr
    770                 775                 780

Thr Val Leu Leu Ala His Gly Thr Leu Ser Val Glu Ala Ala Thr Val
785                 790                 795                 800

Ser Lys Trp Ser Trp Asp Glu Asp Asn Thr Thr Ser Lys Pro Leu Glu
                805                 810                 815

Ser Thr Glu Pro Ser Ala Ser Ser Lys Leu Pro Pro Ala Leu Leu Thr
            820                 825                 830

Thr Val Gly Met Asn Gly Lys Asp Lys Asp Ile Pro Ser Phe Thr Glu
        835                 840                 845

Asp Gly Ala Asp Glu Phe Thr Leu Ile Pro Asp Ser Thr Gln Lys Gln
    850                 855                 860

Leu Glu Glu Val Thr Asp Glu Asp Ile Ala Ala His Gly Lys Phe Thr
865                 870                 875                 880

Ile Arg Phe Gln Pro Thr Thr Ser Thr Gly Ile Ala Glu Lys Ser Thr
                885                 890                 895

Leu Arg Asp Ser Thr Thr Glu Glu Lys Val Pro Pro Ile Thr Ser Thr
```

```
            900                 905                 910

Glu Gly Gln Val Tyr Ala Thr Met Glu Gly Ser Ala Leu Gly Glu Val
        915                 920                 925

Glu Asp Val Asp Leu Ser Lys Pro Val Ser Thr Val Pro Gln Phe Ala
    930                 935                 940

His Thr Ser Glu Val Glu Gly Leu Ala Phe Val Ser Tyr Ser Ser Thr
945                 950                 955                 960

Gln Glu Pro Thr Thr Tyr Val Asp Ser Ser His Thr Ile Pro Leu Ser
                965                 970                 975

Val Ile Pro Lys Thr Asp Trp Gly Val Leu Val Pro Ser Val Pro Ser
            980                 985                 990

Glu Asp Glu Val Leu Gly Glu Pro  Ser Gln Asp Ile Leu  Val Ile Asp
        995                 1000                 1005

Gln Thr  Arg Leu Glu Ala Thr  Ile Ser Pro Glu Thr  Met Arg Thr
    1010                 1015                 1020

Thr Lys  Ile Thr Glu Gly Thr  Thr Gln Glu Glu Phe  Pro Trp Lys
    1025                 1030                 1035

Glu Gln  Thr Ala Glu Lys Pro  Val Pro Ala Leu Ser  Ser Thr Ala
    1040                 1045                 1050

Trp Thr  Pro Lys Glu Ala Val  Thr Pro Leu Asp Glu  Gln Glu Gly
    1055                 1060                 1065

Asp Gly  Ser Ala Tyr Thr Val  Ser Glu Asp Glu Leu  Leu Thr Gly
    1070                 1075                 1080

Ser Glu  Arg Val Pro Val Leu  Glu Thr Thr Pro Val  Gly Lys Ile
    1085                 1090                 1095

Asp His  Ser Val Ser Tyr Pro  Pro Gly Ala Val Thr  Glu His Lys
    1100                 1105                 1110

Val Lys  Thr Asp Glu Val Val  Thr Leu Thr Pro Arg  Ile Gly Pro
    1115                 1120                 1125

Lys Val  Ser Leu Ser Pro Gly  Pro Glu Gln Lys Tyr  Glu Thr Glu
    1130                 1135                 1140
```

Gly Ser Ser Thr Thr Gly Phe Thr Ser Ser Leu Ser Pro Phe Ser
1145 1150 1155

Thr His Ile Thr Gln Leu Met Glu Glu Thr Thr Thr Glu Lys Thr
1160 1165 1170

Ser Leu Glu Asp Ile Asp Leu Gly Ser Gly Leu Phe Glu Lys Pro
1175 1180 1185

Lys Ala Thr Glu Leu Ile Glu Phe Ser Thr Ile Lys Val Thr Val
1190 1195 1200

Pro Ser Asp Ile Thr Thr Ala Phe Ser Ser Val Asp Arg Leu His
1205 1210 1215

Thr Thr Ser Ala Phe Lys Pro Ser Ser Ala Ile Thr Lys Lys Pro
1220 1225 1230

Pro Leu Ile Asp Arg Glu Pro Gly Glu Glu Thr Thr Ser Asp Met
1235 1240 1245

Val Ile Ile Gly Glu Ser Thr Ser His Val Pro Pro Thr Thr Leu
1250 1255 1260

Glu Asp Ile Val Ala Lys Glu Thr Glu Thr Asp Ile Asp Arg Glu
1265 1270 1275

Tyr Phe Thr Thr Ser Ser Pro Pro Ala Thr Gln Pro Thr Arg Pro
1280 1285 1290

Pro Thr Val Glu Asp Lys Glu Ala Phe Gly Pro Gln Ala Leu Ser
1295 1300 1305

Thr Pro Gln Pro Pro Ala Ser Thr Lys Phe His Pro Asp Ile Asn
1310 1315 1320

Val Tyr Ile Ile Glu Val Arg Glu Asn Lys Thr Gly Arg Met Ser
1325 1330 1335

Asp Leu Ser Val Ile Gly His Pro Ile Asp Ser Glu Ser Lys Glu
1340 1345 1350

Asp Glu Pro Cys Ser Glu Glu Thr Asp Pro Val His Asp Leu Met
1355 1360 1365

Ala Glu Ile Leu Pro Glu Phe Pro Asp Ile Ile Glu Ile Asp Leu
1370 1375 1380

```
Tyr His  Ser Glu Glu Asn Glu  Glu Glu Glu Glu Glu  Cys Ala Asn
    1385                 1390                 1395

Ala Thr  Asp Val Thr Thr Thr  Pro Ser Val Gln Tyr  Ile Asn Gly
    1400                 1405                 1410

Lys His  Leu Val Thr Thr Val  Pro Lys Asp Pro Glu  Ala Ala Glu
    1415                 1420                 1425

Ala Arg  Arg Gly Gln Phe Glu  Ser Val Ala Pro Ser  Gln Asn Phe
    1430                 1435                 1440

Ser Asp  Ser Ser Glu Ser Asp  Thr His Pro Phe Val  Ile Ala Lys
    1445                 1450                 1455

Thr Glu  Leu Ser Thr Ala Val  Gln Pro Asn Glu Ser  Thr Glu Thr
    1460                 1465                 1470

Thr Glu  Ser Leu Glu Val Thr  Trp Lys Pro Glu Thr  Tyr Pro Glu
    1475                 1480                 1485

Thr Ser  Glu His Phe Ser Gly  Gly Glu Pro Asp Val  Phe Pro Thr
    1490                 1495                 1500

Val Pro  Phe His Glu Glu Phe  Glu Ser Gly Thr Ala  Lys Lys Gly
    1505                 1510                 1515

Ala Glu  Ser Val Thr Glu Arg  Asp Thr Glu Val Gly  His Gln Ala
    1520                 1525                 1530

His Glu  His Thr Glu Pro Val  Ser Leu Phe Pro Glu  Glu Ser Ser
    1535                 1540                 1545

Gly Glu  Ile Ala Ile Asp Gln  Glu Ser Gln Lys Ile  Ala Phe Ala
    1550                 1555                 1560

Arg Ala  Thr Glu Val Thr Phe  Gly Glu Glu Val Glu  Lys Ser Thr
    1565                 1570                 1575

Ser Val  Thr Tyr Thr Pro Thr  Ile Val Pro Ser Ser  Ala Ser Ala
    1580                 1585                 1590

Tyr Val  Ser Glu Glu Glu Ala  Val Thr Leu Ile Gly  Asn Pro Trp
    1595                 1600                 1605

Pro Asp  Asp Leu Leu Ser Thr  Lys Glu Ser Trp Val  Glu Ala Thr
    1610                 1615                 1620
```

Pro Arg Gln Val Val Glu Leu Ser Gly Ser Ser Ser Ile Pro Ile
1625 1630 1635

Thr Glu Gly Ser Gly Glu Ala Glu Glu Asp Glu Asp Thr Met Phe
1640 1645 1650

Thr Met Val Thr Asp Leu Ser Gln Arg Asn Thr Thr Asp Thr Leu
1655 1660 1665

Ile Thr Leu Asp Thr Ser Arg Ile Ile Thr Glu Ser Phe Phe Glu
1670 1675 1680

Val Pro Ala Thr Thr Ile Tyr Pro Val Ser Glu Gln Pro Ser Ala
1685 1690 1695

Lys Val Val Pro Thr Lys Phe Val Ser Glu Thr Asp Thr Ser Glu
1700 1705 1710

Trp Ile Ser Ser Thr Thr Val Glu Glu Lys Lys Arg Lys Glu Glu
1715 1720 1725

Glu Gly Thr Thr Gly Thr Ala Ser Thr Phe Glu Val Tyr Ser Ser
1730 1735 1740

Thr Gln Arg Ser Asp Gln Leu Ile Leu Pro Phe Glu Leu Glu Ser
1745 1750 1755

Pro Asn Val Ala Thr Ser Ser Asp Ser Gly Thr Arg Lys Ser Phe
1760 1765 1770

Met Ser Leu Thr Thr Pro Thr Gln Ser Glu Arg Glu Met Thr Asp
1775 1780 1785

Ser Thr Pro Val Phe Thr Glu Thr Asn Thr Leu Glu Asn Leu Gly
1790 1795 1800

Ala Gln Thr Thr Glu His Ser Ser Ile His Gln Pro Gly Val Gln
1805 1810 1815

Glu Gly Leu Thr Thr Leu Pro Arg Ser Pro Ala Ser Val Phe Met
1820 1825 1830

Glu Gln Gly Ser Gly Glu Ala Ala Ala Asp Pro Glu Thr Thr Thr
1835 1840 1845

Val Ser Ser Phe Ser Leu Asn Val Glu Tyr Ala Ile Gln Ala Glu

1850 1855 1860

Lys Glu Val Ala Gly Thr Leu Ser Pro His Val Glu Thr Thr Phe
1865 1870 1875

Ser Thr Glu Pro Thr Gly Leu Val Leu Ser Thr Val Met Asp Arg
1880 1885 1890

Val Val Ala Glu Asn Ile Thr Gln Thr Ser Arg Glu Ile Val Ile
1895 1900 1905

Ser Glu Arg Leu Gly Glu Pro Asn Tyr Gly Ala Glu Ile Arg Gly
1910 1915 1920

Phe Ser Thr Gly Phe Pro Leu Glu Glu Asp Phe Ser Gly Asp Phe
1925 1930 1935

Arg Glu Tyr Ser Thr Val Ser His Pro Ile Ala Lys Glu Glu Thr
1940 1945 1950

Val Met Met Glu Gly Ser Gly Asp Ala Ala Phe Arg Asp Thr Gln
1955 1960 1965

Thr Ser Pro Ser Thr Val Pro Thr Ser Val His Ile Ser His Ile
1970 1975 1980

Ser Asp Ser Glu Gly Pro Ser Ser Thr Met Val Ser Thr Ser Ala
1985 1990 1995

Phe Pro Trp Glu Glu Phe Thr Ser Ser Ala Glu Gly Ser Gly Glu
2000 2005 2010

Gln Leu Val Thr Val Ser Ser Ser Val Val Pro Val Leu Pro Ser
2015 2020 2025

Ala Val Gln Lys Phe Ser Gly Thr Ala Ser Ser Ile Ile Asp Glu
2030 2035 2040

Gly Leu Gly Glu Val Gly Thr Val Asn Glu Ile Asp Arg Arg Ser
2045 2050 2055

Thr Ile Leu Pro Thr Ala Glu Val Glu Gly Thr Lys Ala Pro Val
2060 2065 2070

Glu Lys Glu Glu Val Lys Val Ser Gly Thr Val Ser Thr Asn Phe
2075 2080 2085

Pro Gln Thr Ile Glu Pro Ala Lys Leu Trp Ser Arg Gln Glu Val
2090 2095 2100

Asn Pro Val Arg Gln Glu Ile Glu Ser Glu Thr Thr Ser Glu Glu
2105 2110 2115

Gln Ile Gln Glu Glu Lys Ser Phe Glu Ser Pro Gln Asn Ser Pro
2120 2125 2130

Ala Thr Glu Gln Thr Ile Phe Asp Ser Gln Thr Phe Thr Glu Thr
2135 2140 2145

Glu Leu Lys Thr Thr Asp Tyr Ser Val Leu Thr Thr Lys Lys Thr
2150 2155 2160

Tyr Ser Asp Asp Lys Glu Met Lys Glu Glu Asp Thr Ser Leu Val
2165 2170 2175

Asn Met Ser Thr Pro Asp Pro Asp Ala Asn Gly Leu Glu Ser Tyr
2180 2185 2190

Thr Thr Leu Pro Glu Ala Thr Glu Lys Ser His Phe Phe Leu Ala
2195 2200 2205

Thr Ala Leu Val Thr Glu Ser Ile Pro Ala Glu His Val Val Thr
2210 2215 2220

Asp Ser Pro Ile Lys Lys Glu Glu Ser Thr Lys His Phe Pro Lys
2225 2230 2235

Gly Met Arg Pro Thr Ile Gln Glu Ser Asp Thr Glu Leu Leu Phe
2240 2245 2250

Ser Gly Leu Gly Ser Gly Glu Glu Val Leu Pro Thr Leu Pro Thr
2255 2260 2265

Glu Ser Val Asn Phe Thr Glu Val Glu Gln Ile Asn Asn Thr Leu
2270 2275 2280

Tyr Pro His Thr Ser Gln Val Glu Ser Thr Ser Ser Asp Lys Ile
2285 2290 2295

Glu Asp Phe Asn Arg Met Glu Asn Val Ala Lys Glu Val Gly Pro
2300 2305 2310

Leu Val Ser Gln Thr Asp Ile Phe Glu Gly Ser Gly Ser Val Thr
2315 2320 2325

Ser Thr Thr Leu Ile Glu Ile Leu Ser Asp Thr Gly Ala Glu Gly
2330 2335 2340

Pro Thr Val Ala Pro Leu Pro Phe Ser Thr Asp Ile Gly His Pro
2345 2350 2355

Gln Asn Gln Thr Val Arg Trp Ala Glu Glu Ile Gln Thr Ser Arg
2360 2365 2370

Pro Gln Thr Ile Thr Glu Gln Asp Ser Asn Lys Asn Ser Ser Thr
2375 2380 2385

Ala Glu Ile Asn Glu Thr Thr Thr Ser Ser Thr Asp Phe Leu Ala
2390 2395 2400

Arg Ala Tyr Gly Phe Glu Met Ala Lys Glu Phe Val Thr Ser Ala
2405 2410 2415

Pro Lys Pro Ser Asp Leu Tyr Tyr Glu Pro Ser Gly Glu Gly Ser
2420 2425 2430

Gly Glu Val Asp Ile Val Asp Ser Phe His Thr Ser Ala Thr Thr
2435 2440 2445

Gln Ala Thr Arg Gln Glu Ser Ser Thr Thr Phe Val Ser Asp Gly
2450 2455 2460

Ser Leu Glu Lys His Pro Glu Val Pro Ser Ala Lys Ala Val Thr
2465 2470 2475

Ala Asp Gly Phe Pro Thr Val Ser Val Met Leu Pro Leu His Ser
2480 2485 2490

Glu Gln Asn Lys Ser Ser Pro Asp Pro Thr Ser Thr Leu Ser Asn
2495 2500 2505

Thr Val Ser Tyr Glu Arg Ser Thr Asp Gly Ser Phe Gln Asp Arg
2510 2515 2520

Phe Arg Glu Phe Glu Asp Ser Thr Leu Lys Pro Asn Arg Lys Lys
2525 2530 2535

Pro Thr Glu Asn Ile Ile Ile Asp Leu Asp Lys Glu Asp Lys Asp
2540 2545 2550

Leu Ile Leu Thr Ile Thr Glu Ser Thr Ile Leu Glu Ile Leu Pro
2555 2560 2565

Glu Leu Thr Ser Asp Lys Asn Thr Ile Ile Asp Ile Asp His Thr
2570 2575 2580

Lys Pro Val Tyr Glu Asp Ile Leu Gly Met Gln Thr Asp Ile Asp
2585 2590 2595

Thr Glu Val Pro Ser Glu Pro His Asp Ser Asn Asp Glu Ser Asn
2600 2605 2610

Asp Asp Ser Thr Gln Val Gln Glu Ile Tyr Glu Ala Ala Val Asn
2615 2620 2625

Leu Ser Leu Thr Glu Glu Thr Phe Glu Gly Ser Ala Asp Val Leu
2630 2635 2640

Ala Ser Tyr Thr Gln Ala Thr His Asp Glu Ser Met Thr Tyr Glu
2645 2650 2655

Asp Arg Ser Gln Leu Asp His Met Gly Phe His Phe Thr Thr Gly
2660 2665 2670

Ile Pro Ala Pro Ser Thr Glu Thr Glu Leu Asp Val Leu Leu Pro
2675 2680 2685

Thr Ala Thr Ser Leu Pro Ile Pro Arg Lys Ser Ala Thr Val Ile
2690 2695 2700

Pro Glu Ile Glu Gly Ile Lys Ala Glu Ala Lys Ala Leu Asp Asp
2705 2710 2715

Met Phe Glu Ser Ser Thr Leu Ser Asp Gly Gln Ala Ile Ala Asp
2720 2725 2730

Gln Ser Glu Ile Ile Pro Thr Leu Gly Gln Phe Glu Arg Thr Gln
2735 2740 2745

Glu Glu Tyr Glu Asp Lys Lys His Ala Gly Pro Ser Phe Gln Pro
2750 2755 2760

Glu Phe Ser Ser Gly Ala Glu Glu Ala Leu Val Asp His Thr Pro
2765 2770 2775

Tyr Leu Ser Ile Ala Thr Thr His Leu Met Asp Gln Ser Val Thr
2780 2785 2790

Glu Val Pro Asp Val Met Glu Gly Ser Asn Pro Pro Tyr Tyr Thr

2795 2800 2805

Asp Thr Thr Leu Ala Val Ser Thr Phe Ala Lys Leu Ser Ser Gln
2810 2815 2820

Thr Pro Ser Ser Pro Leu Thr Ile Tyr Ser Gly Ser Glu Ala Ser
2825 2830 2835

Gly His Thr Glu Ile Pro Gln Pro Ser Ala Leu Pro Gly Ile Asp
2840 2845 2850

Val Gly Ser Ser Val Met Ser Pro Gln Asp Ser Phe Lys Glu Ile
2855 2860 2865

His Val Asn Ile Glu Ala Thr Phe Lys Pro Ser Ser Glu Glu Tyr
2870 2875 2880

Leu His Ile Thr Glu Pro Pro Ser Leu Ser Pro Asp Thr Lys Leu
2885 2890 2895

Glu Pro Ser Glu Asp Asp Gly Lys Pro Glu Leu Leu Glu Glu Met
2900 2905 2910

Glu Ala Ser Pro Thr Glu Leu Ile Ala Val Glu Gly Thr Glu Ile
2915 2920 2925

Leu Gln Asp Phe Gln Asn Lys Thr Asp Gly Gln Val Ser Gly Glu
2930 2935 2940

Ala Ile Lys Met Phe Pro Thr Ile Lys Thr Pro Glu Ala Gly Thr
2945 2950 2955

Val Ile Thr Thr Ala Asp Glu Ile Glu Leu Glu Gly Ala Thr Gln
2960 2965 2970

Trp Pro His Ser Thr Ser Ala Ser Ala Thr Tyr Gly Val Glu Ala
2975 2980 2985

Gly Val Val Pro Trp Leu Ser Pro Gln Thr Ser Glu Arg Pro Thr
2990 2995 3000

Leu Ser Ser Ser Pro Glu Ile Asn Pro Glu Thr Gln Ala Ala Leu
3005 3010 3015

Ile Arg Gly Gln Asp Ser Thr Ile Ala Ala Ser Glu Gln Gln Val
3020 3025 3030

```
Ala Ala  Arg Ile Leu Asp Ser  Asn Asp Gln Ala Thr  Val Asn Pro
    3035                 3040                 3045

Val Glu  Phe Asn Thr Glu Val  Ala Thr Pro Pro Phe  Ser Leu Leu
    3050                 3055                 3060

Glu Thr  Ser Asn Glu Thr Asp  Phe Leu Ile Gly Ile  Asn Glu Glu
    3065                 3070                 3075

Ser Val  Glu Gly Thr Ala Ile  Tyr Leu Pro Gly Pro  Asp Arg Cys
    3080                 3085                 3090

Lys Met  Asn Pro Cys Leu Asn  Gly Gly Thr Cys Tyr  Pro Thr Glu
    3095                 3100                 3105

Thr Ser  Tyr Val Cys Thr Cys  Val Pro Gly Tyr Ser  Gly Asp Gln
    3110                 3115                 3120

Cys Glu  Leu Asp Phe Asp Glu  Cys His Ser Asn Pro  Cys Arg Asn
    3125                 3130                 3135

Gly Ala  Thr Cys Val Asp Gly  Phe Asn Thr Phe Arg  Cys Leu Cys
    3140                 3145                 3150

Leu Pro  Ser Tyr Val Gly Ala  Leu Cys Glu Gln Asp  Thr Glu Thr
    3155                 3160                 3165

Cys Asp  Tyr Gly Trp His Lys  Phe Gln Gly Gln Cys  Tyr Lys Tyr
    3170                 3175                 3180

Phe Ala  His Arg Arg Thr Trp  Asp Ala Ala Glu Arg  Glu Cys Arg
    3185                 3190                 3195

Leu Gln  Gly Ala His Leu Thr  Ser Ile Leu Ser His  Glu Glu Gln
    3200                 3205                 3210

Met Phe  Val Asn Arg Val Gly  His Asp Tyr Gln Trp  Ile Gly Leu
    3215                 3220                 3225

Asn Asp  Lys Met Phe Glu His  Asp Phe Arg Trp Thr  Asp Gly Ser
    3230                 3235                 3240

Thr Leu  Gln Tyr Glu Asn Trp  Arg Pro Asn Gln Pro  Asp Ser Phe
    3245                 3250                 3255

Phe Ser  Ala Gly Glu Asp Cys  Val Val Ile Ile Trp  His Glu Asn
    3260                 3265                 3270
```

```
Gly Gln  Trp Asn Asp Val Pro  Cys Asn Tyr His Leu  Thr Tyr Thr
    3275                 3280                 3285

Cys Lys  Lys Gly Thr Val Ala  Cys Gly Gln Pro Pro  Val Val Glu
    3290                 3295                 3300

Asn Ala  Lys Thr Phe Gly Lys  Met Lys Pro Arg Tyr  Glu Ile Asn
    3305                 3310                 3315

Ser Leu  Ile Arg Tyr His Cys  Lys Asp Gly Phe Ile  Gln Arg His
    3320                 3325                 3330

Leu Pro  Thr Ile Arg Cys Leu  Gly Asn Gly Arg Trp  Ala Ile Pro
    3335                 3340                 3345

Lys Ile  Thr Cys Met Asn Pro  Ser Ala Tyr Gln Arg  Thr Tyr Ser
    3350                 3355                 3360

Met Lys  Tyr Phe Lys Asn Ser  Ser Ser Ala Lys Asp  Asn Ser Ile
    3365                 3370                 3375

Asn Thr  Ser Lys His Asp His  Arg Trp Ser Arg Arg  Trp Gln Glu
    3380                 3385                 3390

Ser Arg  Arg
    3395
```

<210> 130
<211> 20
<212> DNA
<213> Artificial Sequence

<220>
<223> VCAN_forward primer

<400> 130
tcctgtctca cgaagaacaa 20

<210> 131
<211> 20
<212> DNA
<213> Artificial Sequence

<220>
<223> VCAN_reverse primer

<400> 131
atcagtccaa cggaagtcat 20

<210> 132
<211> 26
<212> DNA

<213> Artificial Sequence

<220>
<223> VCAN probe

<400> 132
tcgtgtgggc catgattatc agtgga 26

<210> 133
<211> 2465
<212> DNA
<213> Homo sapiens

<400> 133

cctttcctcc ctccccgccc tctccccgct gtcccctccc cgtcggcccg cctgcccagc 60
ctttagcctc ccgcccgccg cctctgtctc cctctctcca caaactgccc aggagtgagt 120
agctgctttc ggtccgccgg acacaccgga cagatagacg tgcggacggc ccaccacccc 180
agcccgccaa ctagtcagcc tgcgcctggc gcctcccctc tccaggtcca tccgccatgt 240
ggcccctgtg gcgcctcgtg tctctgctgg ccctgagcca ggccctgccc tttgagcaga 300
gaggcttctg ggacttcacc ctggacgatg ggccattcat gatgaacgat gaggaagctt 360
cgggcgctga cacctcgggc gtcctggacc cggactctgt cacacccacc tacagcgcca 420
tgtgtccttt cggctgccac tgccacctgc gggtggttca gtgctccgac ctgggtctga 480
agtctgtgcc caaagagatc tcccctgaca ccacgctgct ggacctgcag aacaacgaca 540
tctccgagct ccgcaaggat gacttcaagg gtctccagca cctctacgcc ctcgtcctgg 600
tgaacaacaa gatctccaag atccatgaga aggccttcag cccactgcgg aagctgcaga 660
agctctacat ctccaagaac cacctggtgg agatcccgcc caacctaccc agctccctgg 720
tggagctccg catccacgac aaccgcatcc gcaaggtgcc caagggagtg ttcagcgggc 780
tccggaacat gaactgcatc gagatgggcg ggaacccact ggagaacagt ggctttgaac 840
ctggagcctt cgatggcctg aagctcaact acctgcgcat ctcagaggcc aagctgactg 900
gcatccccaa agacctccct gagaccctga atgaactcca cctagaccac aacaaaatcc 960
aggccatcga actggaggac ctgcttcgct actccaagct gtacaggctg ggcctaggcc 1020
acaaccagat caggatgatc gagaacggga gcctgagctt cctgcccacc ctccgggagc 1080
tccacttgga caacaacaag ttggccaggg tgccctcagg gctcccagac ctcaagctcc 1140
tccaggtggt ctatctgcac tccaacaaca tcaccaaagt gggtgtcaac gacttctgtc 1200
ccatgggctt cggggtgaag cgggcctact acaacggcat cagcctcttc aacaaccccg 1260
tgccctactg ggaggtgcag ccggccactt tccgctgcgt cactgaccgc ctggccatcc 1320
agtttggcaa ctacaaaaag tagaggcagc tgcagccacc gcggggcctc agtgggggtc 1380
tctggggaac acagccagac atcctgatgg ggaggcagag ccaggaagct aagccagggc 1440

```
ccagctgcgt ccaacccagc cccccacctc gggtccctga ccccagctcg atgccccatc     1500
accgcctctc cctggctccc aagggtgcag gtgggcgcaa ggcccggccc ccatcacatg     1560
ttcccttggc ctcagagctg cccctgctct cccaccacag ccacccagag gcaccccatg     1620
aagctttttt ctcgttcact cccaaaccca agtgtccaag gctccagtcc taggagaaca     1680
gtccctgggt cagcagccag gaggcggtcc ataagaatgg ggacagtggg ctctgccagg     1740
gctgccgcac ctgtccagac acacatgttc tgttcctcct cctcatgcat ttccagcctt     1800
tcaaccctcc ccgactctgc ggctcccctc agccccccttg caagttcatg gcctgtccct     1860
cccagacccc tgctccactg gcccttcgac cagtcctccc ttctgttctc tctttccccg     1920
tccttcctct ctctctctct ctctctctct ctctctttct gtgtgtgtgt gtgtgtgtgt     1980
gtgtgtgtgt gtgtgtgtgt gtgtcttgtg cttcctcaga cctttctcgc ttctgagctt     2040
ggtggcctgt tccctccatc tctccgaacc tggcttcgcc tgtccctttc actccacacc     2100
ctctggcctt ctgccttgag ctgggactgc tttctgtctg tccggcctgc acccagcccc     2160
tgcccacaaa accccaggga cagcagtctc cccagcctgc cctgctcagg ccttgccccc     2220
aaacctgtac tgtcccggag gaggttggga ggtggaggcc cagcatcccg cgcagatgac     2280
accatcaacc gccagagtcc cagacaccgg ttttcctaga agcccctcac ccccactggc     2340
ccactggtgg ctaggtctcc ccttatcctt ctggtccagc gcaaggaggg gctgcttctg     2400
aggtcggtgg ctgtctttcc attaaagaaa caccgtgcaa cgtgaaaaaa aaaaaaaaaa     2460
aaaaa                                                                 2465
```

<210> 134
<211> 368
<212> PRT
<213> Homo sapiens

<400> 134

```
Met Trp Pro Leu Trp Arg Leu Val Ser Leu Leu Ala Leu Ser Gln Ala
1               5                   10                  15

Leu Pro Phe Glu Gln Arg Gly Phe Trp Asp Phe Thr Leu Asp Asp Gly
            20                  25                  30

Pro Phe Met Met Asn Asp Glu Glu Ala Ser Gly Ala Asp Thr Ser Gly
        35                  40                  45

Val Leu Asp Pro Asp Ser Val Thr Pro Thr Tyr Ser Ala Met Cys Pro
    50                  55                  60

Phe Gly Cys His Cys His Leu Arg Val Val Gln Cys Ser Asp Leu Gly
65                  70                  75                  80
```

```
Leu Lys Ser Val Pro Lys Glu Ile Ser Pro Asp Thr Thr Leu Leu Asp
                85                  90                  95

Leu Gln Asn Asn Asp Ile Ser Glu Leu Arg Lys Asp Asp Phe Lys Gly
            100                 105                 110

Leu Gln His Leu Tyr Ala Leu Val Leu Val Asn Asn Lys Ile Ser Lys
        115                 120                 125

Ile His Glu Lys Ala Phe Ser Pro Leu Arg Lys Leu Gln Lys Leu Tyr
    130                 135                 140

Ile Ser Lys Asn His Leu Val Glu Ile Pro Pro Asn Leu Pro Ser Ser
145                 150                 155                 160

Leu Val Glu Leu Arg Ile His Asp Asn Arg Ile Arg Lys Val Pro Lys
                165                 170                 175

Gly Val Phe Ser Gly Leu Arg Asn Met Asn Cys Ile Glu Met Gly Gly
            180                 185                 190

Asn Pro Leu Glu Asn Ser Gly Phe Glu Pro Gly Ala Phe Asp Gly Leu
        195                 200                 205

Lys Leu Asn Tyr Leu Arg Ile Ser Glu Ala Lys Leu Thr Gly Ile Pro
    210                 215                 220

Lys Asp Leu Pro Glu Thr Leu Asn Glu Leu His Leu Asp His Asn Lys
225                 230                 235                 240

Ile Gln Ala Ile Glu Leu Glu Asp Leu Leu Arg Tyr Ser Lys Leu Tyr
                245                 250                 255

Arg Leu Gly Leu Gly His Asn Gln Ile Arg Met Ile Glu Asn Gly Ser
            260                 265                 270

Leu Ser Phe Leu Pro Thr Leu Arg Glu Leu His Leu Asp Asn Asn Lys
        275                 280                 285

Leu Ala Arg Val Pro Ser Gly Leu Pro Asp Leu Lys Leu Leu Gln Val
    290                 295                 300

Val Tyr Leu His Ser Asn Asn Ile Thr Lys Val Gly Val Asn Asp Phe
305                 310                 315                 320

Cys Pro Met Gly Phe Gly Val Lys Arg Ala Tyr Tyr Asn Gly Ile Ser
```

325 330 335

Leu Phe Asn Asn Pro Val Pro Tyr Trp Glu Val Gln Pro Ala Thr Phe
340 345 350

Arg Cys Val Thr Asp Arg Leu Ala Ile Gln Phe Gly Asn Tyr Lys Lys
355 360 365

<210> 135
<211> 17
<212> DNA
<213> Artificial Sequence

<220>
<223> BGN_forward primer

<400> 135
tgcccaaggg agtgttc 17

<210> 136
<211> 20
<212> DNA
<213> Artificial Sequence

<220>
<223> BGN_reverse primer

<400> 136
tagttgagct tcaggccatc 20

<210> 137
<211> 20
<212> DNA
<213> Artificial Sequence

<220>
<223> BGN probe

<400> 137
atgggcggga acccactgga 20

<210> 138
<211> 2537
<212> DNA
<213> Homo sapiens

<400> 138

cccagaaggc cgcgggggt ggaccgccta agagggcgtg cgctcccgac atgccccgcg 60

gcgcgccatt aaccgccaga tttgaatcgc gggacccgtt ggcagaggtg gcggcggcgg 120

catgggtgcc ccgacgttgc cccctgcctg gcagcccttt ctcaaggacc accgcatctc 180

tacattcaag aactggccct tcttggaggg ctgcgcctgc accccggagc ggatggccga 240

ggctggcttc atccactgcc ccactgagaa cgagccagac ttggcccagt gtttcttctg 300

cttcaaggag ctggaaggct gggagccaga tgacgacccc atgcaaagga aaccaacaat 360
aagaagaaag aatttgagga aactgcggag aaagtgcgcc gtgccatcga gcagctggct 420
gccatggatt gaggcctctg gccggagctg cctggtccca gagtggctgc accacttcca 480
gggtttattc cctggtgcca ccagccttcc tgtgggcccc ttagcaatgt cttaggaaag 540
gagatcaaca ttttcaaatt agatgtttca actgtgctct tgttttgtct tgaaagtggc 600
accagaggtg cttctgcctg tgcagcgggt gctgctggta acagtggctg cttctctctc 660
tctctctctt ttttgggggc tcatttttgc tgttttgatt cccgggctta ccaggtgaga 720
agtgagggag gaagaaggca gtgtcccttt tgctagagct gacagctttg ttcgcgtggg 780
cagagccttc cacagtgaat gtgtctggac ctcatgttgt tgaggctgtc acagtcctga 840
gtgtggactt ggcaggtgcc tgttgaatct gagctgcagg ttccttatct gtcacacctg 900
tgcctcctca gaggacagtt tttttgttgt tgtgtttttt tgtttttttt tttttggtag 960
atgcatgact tgtgtgtgat gagagaatgg agacagagtc cctggctcct ctactgttta 1020
acaacatggc tttcttattt tgtttgaatt gttaattcac agaatagcac aaactacaat 1080
taaaactaag cacaaagcca ttctaagtca ttggggaaac ggggtgaact tcaggtggat 1140
gaggagacag aatagagtga taggaagcgt ctggcagata ctccttttgc cactgctgtg 1200
tgattagaca ggcccagtga gccgcggggc acatgctggc cgctcctccc tcagaaaaag 1260
gcagtggcct aaatcctttt taaatgactt ggctcgatgc tgtgggggac tggctgggct 1320
gctgcaggcc gtgtgtctgt cagcccaacc ttcacatctg tcacgttctc cacacggggg 1380
agagacgcag tccgcccagg tccccgcttt ctttggaggc agcagctccc gcagggctga 1440
agtctggcgt aagatgatgg atttgattcg ccctcctccc tgtcatagag ctgcagggtg 1500
gattgttaca gcttcgctgg aaacctctgg aggtcatctc ggctgttcct gagaaataaa 1560
aagcctgtca tttcaaacac tgctgtggac cctactgggt ttttaaaata ttgtcagttt 1620
ttcatcgtcg tccctagcct gccaacagcc atctgcccag acagccgcag tgaggatgag 1680
cgtcctggca gagacgcagt tgtctctggg cgcttgccag agccacgaac cccagacctg 1740
tttgtatcat ccgggctcct tccgggcaga aacaactgaa aatgcacttc agacccactt 1800
atttctgcca catctgagtc ggcctgagat agacttttcc ctctaaactg ggagaatatc 1860
acagtggttt ttgttagcag aaaatgcact ccagcctctg tactcatcta agctgcttat 1920
ttttgatatt tgtgtcagtc tgtaaatgga tacttcactt taataactgt tgcttagtaa 1980
ttggctttgt agagaagctg gaaaaaaatg gttttgtctt caactccttt gcatgccagg 2040
cggtgatgtg gatctcggct tctgtgagcc tgtgctgtgg gcagggctga gctggagccg 2100
cccctctcag cccgcctgcc acggcctttc cttaaaggcc atccttaaaa ccagaccctc 2160
atggctacca gcacctgaaa gcttcctcga catctgttaa taaagccgta ggcccttgtc 2220

```
taagtgcaac cgcctagact ttctttcaga tacatgtcca catgtccatt tttcaggttc     2280
tctaagttgg agtggagtct gggaagggtt gtgaatgagg cttctgggct atgggtgagg     2340
ttccaatggc aggttagagc ccctcgggcc aactgccatc ctggaaagta gagacagcag     2400
tgcccgctgc ccagaagaga ccagcaagcc aaactggagc ccccattgca ggctgtcgcc     2460
atgtggaaag agtaactcac aattgccaat aaagtctcat gtggttttat ctaaaaaaaa     2520
aaaaaaaaaa aaaaaaa                                                    2537
```

<210> 139
<211> 2724
<212> DNA
<213> Homo sapiens

<400> 139

```
cccagaaggc cgcggggggt ggaccgccta agagggcgtg cgctcccgac atgccccgcg      60
gcgcgccatt aaccgccaga tttgaatcgc gggacccgtt ggcagaggtg gcggcggcgg     120
catgggtgcc ccgacgttgc cccctgcctg gcagcccttt ctcaaggacc accgcatctc     180
tacattcaag aactggccct tcttggaggg ctgcgcctgc accccggagc ggatggccga     240
ggctggcttc atccactgcc ccactgagaa cgagccagac ttggcccagt gtttcttctg     300
cttcaaggag ctggaaggct gggagccaga tgacgacccc attgggccgg gcacggtggc     360
ttacgcctgt aataccagca ctttgggagg ccgaggcggg cggatcacga gagaggaaca     420
taaaaagcat tcgtccggtt gcgctttcct ttctgtcaag aagcagtttg aagaattaac     480
ccttggtgaa tttttgaaac tggacagaga aagagccaag aacaaaattg caaaggaaac     540
caacaataag aagaaagaat ttgaggaaac tgcggagaaa gtgcgccgtg ccatcgagca     600
gctggctgcc atggattgag gcctctggcc ggagctgcct ggtcccagag tggctgcacc     660
acttccaggg tttattccct ggtgccacca gccttcctgt gggcccctta gcaatgtctt     720
aggaaaggag atcaacattt tcaaattaga tgtttcaact gtgctcttgt tttgtcttga     780
aagtggcacc agaggtgctt ctgcctgtgc agcgggtgct gctggtaaca gtggctgctt     840
ctctctctct ctctcttttt tgggggctca tttttgctgt tttgattccc gggcttacca     900
ggtgagaagt gagggaggaa gaaggcagtg tcccttttgc tagagctgac agctttgttc     960
gcgtgggcag agccttccac agtgaatgtg tctggacctc atgttgttga ggctgtcaca    1020
gtcctgagtg tggacttggc aggtgcctgt tgaatctgag ctgcaggttc cttatctgtc    1080
acacctgtgc ctcctcagag gacagttttt ttgttgttgt gttttttttgt tttttttttt    1140
ttggtagatg catgacttgt gtgtgatgag agaatggaga cagagtccct ggctcctcta    1200
ctgtttaaca acatggcttt cttattttgt ttgaattgtt aattcacaga atagcacaaa    1260
ctacaattaa aactaagcac aaagccattc taagtcattg gggaaacggg gtgaacttca    1320
```

ggtggatgag gagacagaat agagtgatag gaagcgtctg gcagatactc cttttgccac 1380

tgctgtgtga ttagacaggc ccagtgagcc gcggggcaca tgctggccgc tcctccctca 1440

gaaaaaggca gtggcctaaa tcctttttaa atgacttggc tcgatgctgt gggggactgg 1500

ctgggctgct gcaggccgtg tgtctgtcag cccaaccttc acatctgtca cgttctccac 1560

acgggggaga gacgcagtcc gcccaggtcc ccgctttctt tggaggcagc agctcccgca 1620

gggctgaagt ctggcgtaag atgatggatt tgattcgccc tcctccctgt catagagctg 1680

cagggtggat tgttacagct tcgctggaaa cctctggagg tcatctcggc tgttcctgag 1740

aaataaaaag cctgtcattt caaacactgc tgtggaccct actgggtttt taaaatattg 1800

tcagtttttc atcgtcgtcc ctagcctgcc aacagccatc tgcccagaca gccgcagtga 1860

ggatgagcgt cctggcagag acgcagttgt ctctgggcgc ttgccagagc cacgaacccc 1920

agacctgttt gtatcatccg ggctccttcc gggcagaaac aactgaaaat gcacttcaga 1980

cccacttatt tctgccacat ctgagtcggc ctgagataga cttttccctc taaactggga 2040

gaatatcaca gtggtttttg ttagcagaaa atgcactcca gcctctgtac tcatctaagc 2100

tgcttatttt tgatatttgt gtcagtctgt aaatggatac ttcactttaa taactgttgc 2160

ttagtaattg gctttgtaga gaagctggaa aaaaatggtt ttgtcttcaa ctcctttgca 2220

tgccaggcgg tgatgtggat ctcggcttct gtgagcctgt gctgtgggca gggctgagct 2280

ggagccgccc ctctcagccc gcctgccacg gcctttcctt aaaggccatc cttaaaacca 2340

gaccctcatg gctaccagca cctgaaagct tcctcgacat ctgttaataa agccgtaggc 2400

ccttgtctaa gtgcaaccgc ctagactttc tttcagatac atgtccacat gtccattttt 2460

caggttctct aagttggagt ggagtctggg aagggttgtg aatgaggctt ctgggctatg 2520

ggtgaggttc caatggcagg ttagagcccc tcgggccaac tgccatcctg gaaagtagag 2580

acagcagtgc ccgctgccca gaagagacca gcaagccaaa ctggagcccc cattgcaggc 2640

tgtcgccatg tggaaagagt aactcacaat tgccaataaa gtctcatgtg gttttatcta 2700

aaaaaaaaaa aaaaaaaaaa aaaa 2724

<210> 140
<211> 2655
<212> DNA
<213> Homo sapiens

<400> 140

```
cccagaaggc cgcgggggt ggaccgccta agagggcgtg cgctcccgac atgccccgcg      60
gcgcgccatt aaccgccaga tttgaatcgc gggacccgtt ggcagaggtg gcggcggcgg    120
catgggtgcc ccgacgttgc cccctgcctg gcagcccttt ctcaaggacc accgcatctc    180
tacattcaag aactggccct tcttggaggg ctgcgcctgc accccggagc ggatggccga    240
```

ggctggcttc atccactgcc ccactgagaa cgagccagac ttggcccagt gtttcttctg 300
cttcaaggag ctggaaggct gggagccaga tgacgacccc atagaggaac ataaaaagca 360
ttcgtccggt tgcgctttcc tttctgtcaa gaagcagttt gaagaattaa cccttggtga 420
atttttgaaa ctggacagag aaagagccaa gaacaaaatt gcaaaggaaa ccaacaataa 480
gaagaaagaa tttgaggaaa ctgcggagaa agtgcgccgt gccatcgagc agctggctgc 540
catggattga ggcctctggc cggagctgcc tggtcccaga gtggctgcac cacttccagg 600
gtttattccc tggtgccacc agccttcctg tgggcccctt agcaatgtct taggaaagga 660
gatcaacatt ttcaaattag atgtttcaac tgtgctcttg ttttgtcttg aaagtggcac 720
cagaggtgct tctgcctgtg cagcgggtgc tgctggtaac agtggctgct tctctctctc 780
tctctctttt ttgggggctc atttttgctg ttttgattcc cgggcttacc aggtgagaag 840
tgagggagga agaaggcagt gtcccttttg ctagagctga cagctttgtt cgcgtgggca 900
gagccttcca cagtgaatgt gtctggacct catgttgttg aggctgtcac agtcctgagt 960
gtggacttgg caggtgcctg ttgaatctga gctgcaggtt ccttatctgt cacacctgtg 1020
cctcctcaga ggacagtttt tttgttgttg tgtttttttg tttttttttt tttggtagat 1080
gcatgacttg tgtgtgatga gagaatggag acagagtccc tggctcctct actgtttaac 1140
aacatggctt tcttattttg tttgaattgt taattcacag aatagcacaa actacaatta 1200
aaactaagca caaagccatt ctaagtcatt ggggaaacgg ggtgaacttc aggtggatga 1260
ggagacagaa tagagtgata ggaagcgtct ggcagatact ccttttgcca ctgctgtgtg 1320
attagacagg cccagtgagc cgcggggcac atgctggccg ctcctccctc agaaaaaggc 1380
agtggcctaa atccttttta aatgacttgg ctcgatgctg tgggggactg gctgggctgc 1440
tgcaggccgt gtgtctgtca gcccaacctt cacatctgtc acgttctcca cacgggggag 1500
agacgcagtc cgcccaggtc cccgctttct ttggaggcag cagctcccgc agggctgaag 1560
tctggcgtaa gatgatggat ttgattcgcc ctcctccctg tcatagagct gcagggtgga 1620
ttgttacagc ttcgctggaa acctctggag gtcatctcgg ctgttcctga gaaataaaaa 1680
gcctgtcatt tcaaacactg ctgtggaccc tactgggttt ttaaaatatt gtcagttttt 1740
catcgtcgtc cctagcctgc caacagccat ctgcccagac agccgcagtg aggatgagcg 1800
tcctggcaga gacgcagttg tctctgggcg cttgccagag ccacgaaccc cagacctgtt 1860
tgtatcatcc gggctccttc cgggcagaaa caactgaaaa tgcacttcag acccacttat 1920
ttctgccaca tctgagtcgg cctgagatag acttttccct ctaaactggg agaatatcac 1980
agtggttttt gttagcagaa aatgcactcc agcctctgta ctcatctaag ctgcttattt 2040
ttgatatttg tgtcagtctg taaatggata cttcacttta ataactgttg cttagtaatt 2100

ggctttgtag agaagctgga aaaaaatggt tttgtcttca actcctttgc atgccaggcg 2160

gtgatgtgga tctcggcttc tgtgagcctg tgctgtgggc agggctgagc tggagccgcc 2220

cctctcagcc cgcctgccac ggcctttcct taaaggccat ccttaaaacc agaccctcat 2280

ggctaccagc acctgaaagc ttcctcgaca tctgttaata aagccgtagg cccttgtcta 2340

agtgcaaccg cctagacttt ctttcagata catgtccaca tgtccatttt tcaggttctc 2400

taagttggag tggagtctgg gaagggttgt gaatgaggct tctgggctat gggtgaggtt 2460

ccaatggcag gttagagccc ctcgggccaa ctgccatcct ggaaagtaga gacagcagtg 2520

cccgctgccc agaagagacc agcaagccaa actggagccc ccattgcagg ctgtcgccat 2580

gtggaaagag taactcacaa ttgccaataa agtctcatgt ggttttatct aaaaaaaaaa 2640

aaaaaaaaaa aaaaa 2655

<210> 141
<211> 137
<212> PRT
<213> Homo sapiens

<400> 141

Met Gly Ala Pro Thr Leu Pro Pro Ala Trp Gln Pro Phe Leu Lys Asp
1 5 10 15

His Arg Ile Ser Thr Phe Lys Asn Trp Pro Phe Leu Glu Gly Cys Ala
20 25 30

Cys Thr Pro Glu Arg Met Ala Glu Ala Gly Phe Ile His Cys Pro Thr
35 40 45

Glu Asn Glu Pro Asp Leu Ala Gln Cys Phe Phe Cys Phe Lys Glu Leu
50 55 60

Glu Gly Trp Glu Pro Asp Asp Asp Pro Met Gln Arg Lys Pro Thr Ile
65 70 75 80

Arg Arg Lys Asn Leu Arg Lys Leu Arg Arg Lys Cys Ala Val Pro Ser
85 90 95

Ser Ser Trp Leu Pro Trp Ile Glu Ala Ser Gly Arg Ser Cys Leu Val
100 105 110

Pro Glu Trp Leu His His Phe Gln Gly Leu Phe Pro Gly Ala Thr Ser
115 120 125

Leu Pro Val Gly Pro Leu Ala Met Ser
130 135

<210> 142
<211> 165
<212> PRT
<213> Homo sapiens

<400> 142

```
Met Gly Ala Pro Thr Leu Pro Pro Ala Trp Gln Pro Phe Leu Lys Asp
1               5                   10                  15

His Arg Ile Ser Thr Phe Lys Asn Trp Pro Phe Leu Glu Gly Cys Ala
            20                  25                  30

Cys Thr Pro Glu Arg Met Ala Glu Ala Gly Phe Ile His Cys Pro Thr
        35                  40                  45

Glu Asn Glu Pro Asp Leu Ala Gln Cys Phe Phe Cys Phe Lys Glu Leu
    50                  55                  60

Glu Gly Trp Glu Pro Asp Asp Asp Pro Ile Gly Pro Gly Thr Val Ala
65                  70                  75                  80

Tyr Ala Cys Asn Thr Ser Thr Leu Gly Gly Arg Gly Gly Arg Ile Thr
                85                  90                  95

Arg Glu Glu His Lys Lys His Ser Ser Gly Cys Ala Phe Leu Ser Val
            100                 105                 110

Lys Lys Gln Phe Glu Glu Leu Thr Leu Gly Glu Phe Leu Lys Leu Asp
        115                 120                 125

Arg Glu Arg Ala Lys Asn Lys Ile Ala Lys Glu Thr Asn Asn Lys Lys
    130                 135                 140

Lys Glu Phe Glu Glu Thr Ala Glu Lys Val Arg Arg Ala Ile Glu Gln
145                 150                 155                 160

Leu Ala Ala Met Asp
                165
```

<210> 143
<211> 142
<212> PRT
<213> Homo sapiens

<400> 143

```
Met Gly Ala Pro Thr Leu Pro Pro Ala Trp Gln Pro Phe Leu Lys Asp
1               5                   10                  15
```

His Arg Ile Ser Thr Phe Lys Asn Trp Pro Phe Leu Glu Gly Cys Ala
20 25 30

Cys Thr Pro Glu Arg Met Ala Glu Ala Gly Phe Ile His Cys Pro Thr
35 40 45

Glu Asn Glu Pro Asp Leu Ala Gln Cys Phe Phe Cys Phe Lys Glu Leu
50 55 60

Glu Gly Trp Glu Pro Asp Asp Asp Pro Ile Glu Glu His Lys Lys His
65 70 75 80

Ser Ser Gly Cys Ala Phe Leu Ser Val Lys Lys Gln Phe Glu Glu Leu
85 90 95

Thr Leu Gly Glu Phe Leu Lys Leu Asp Arg Glu Arg Ala Lys Asn Lys
100 105 110

Ile Ala Lys Glu Thr Asn Asn Lys Lys Lys Glu Phe Glu Glu Thr Ala
115 120 125

Glu Lys Val Arg Arg Ala Ile Glu Gln Leu Ala Ala Met Asp
130 135 140

<210> 144
<211> 18
<212> DNA
<213> Artificial Sequence

<220>
<223> BIRC5_forward primer

<400> 144
cttggcccag tgtttctt 18

<210> 145
<211> 21
<212> DNA
<213> Artificial Sequence

<220>
<223> BIRC5_reverse primer

<400> 145
gttcctctat ggggtcgtca t 21

<210> 146
<211> 20
<212> DNA
<213> Artificial Sequence

<220>

<223> BIRC5 probe

<400> 146
agctggaagg ctgggagcca 20

<210> 147
<211> 6010
<212> DNA
<213> Homo sapiens

<400> 147

cgcgggggcg cgcgcgcgcg ggcccgggag aggctcccga gccaggcggt cttcggtcct 60
cgcagcgctt ccagctcccc gcgcccctat gtgagggaga cggggaggcc cgcggcgcgc 120
aggggagggc gaggcatgtg cacgggccgg agggtgctgc agccgcccga ggaagaggag 180
gacggcggcg aggaggagag cggggggctc gcggcggcgg gccccggccg aggggatgca 240
gtggactgtg tgtgtctggc tgtagcagac gcgaggcggc gacgaggcgc cggggacccg 300
cgcgaggggc ggccgggagg cggcggcggc ggccgccaga agtagcagca ggaccggcgg 360
cggcgacggc agccctgaaa tgcattttcc tctccagcgg ccatgttaac caggaaacct 420
tcggccgccg ctcccgccgc ctacccgacc gattggcggc agtaagcaca caatgaatga 480
tcacctgcat gtcggcagcc acgctcacgg acagatccag gttcaacagt tgtttgagga 540
taacagtaac aagcggacag tgctcacgac acaaccaaat gggcttacaa cagtgggcaa 600
aacgggcttg ccagtggtgc cagagcggca gctggacagc attcatagac ggcaggggag 660
ctccacctct ctaaagtcca tggaaggcat ggggaaggtg aaagccaccc ccatgacacc 720
tgaacaagca atgaagcaat acatgcaaaa actcacagcc ttcgaacacc atgagatttt 780
cagctaccct gaaatatatt tcttgggtct aaatgctaag aagcgccagg gcatgacagg 840
tgggcccaac aatggtggct atgatgatga ccagggatca tatgtgcagg tgccccacga 900
tcacgtggct tacaggtatg aggtcctcaa ggtcattggg aaggggagct ttgggcaggt 960
ggtcaaggcc tacgatcaca aagtccacca gcacgtggcc ctaaagatgg tgcggaatga 1020
gaagcgcttc caccggcaag cagcggagga gatccgaatc ctggaacacc tgcggaagca 1080
ggacaaggat aacacaatga atgtcatcca tatgctggag aatttcacct tccgcaacca 1140
catctgcatg acgtttgagc tgctgagcat gaacctctat gagctcatca agaagaataa 1200
attccagggc ttcagtctgc ctttggttcg caagtttgcc cactcgattc tgcagtgctt 1260
ggatgctttg cacaaaaaca gaataattca ctgtgacctt aagcccgaga acattttgtt 1320
aaagcagcag ggtagaagcg gtattaaagt aattgatttt ggctccagtt gttacgagca 1380
tcagcgtgtc tacacgtaca tccagtcgcg tttttaccgg gctccagaag tgatccttgg 1440
ggccaggtat ggcatgccca ttgatatgtg gagcctgggc tgcattttag cagagctcct 1500
gacgggttac cccctcttgc ctggggaaga tgaaggggac cagctggcct gtatgattga 1560

actgttgggc atgccctcac agaaactgct ggatgcatcc aaacgagcca aaaattttgt 1620

gagctccaag ggttatcccc gttactgcac tgtcacgact ctctcagatg gctctgtggt 1680

cctaaacgga ggccgttccc ggagggggaa actgaggggc ccaccggaga gcagagagtg 1740

ggggaacgcg ctgaaggggt gtgatgatcc ccttttcctt gacttcttaa aacagtgttt 1800

agagtgggat cctgcagtgc gcatgacccc aggccaggct ttgcggcacc cctggctgag 1860

gaggcggttg ccaaagcctc ccaccgggga gaaaacgtca gtgaaaagga taactgagag 1920

caccggtgct atcacatcta tatccaagtt acctccacct tctagctcag cttccaaact 1980

gaggactaat ttggcgcaga tgacagatgc caatgggaat attcagcaga ggacagtgtt 2040

gccaaaactt gttagctgag ctcacgtccc ctgatgctgg taacctgaaa gatacgacat 2100

tgctgagcct tactgggttg aaaaggagta gctcagacct gtttttattt gctcaataac 2160

tctactcatt tgtatctttt cagcacttaa ttttaatgta agaaagttgt tcattttgtt 2220

tttataaaat acatgaggac aatgctttaa gtttttatac tttcagaaac tttttgtgtt 2280

ctaaaagtac aatgagcctt actgtattta gtgtggcaga ataataacat cagtggcagg 2340

ccactgatta cttcatgact gccacgcatt tacagattgg tgtcaaagac attcactatg 2400

tttttatggt tcatgttata tcctccccag ggtgacagcc ccttaaggcc ctccttttcc 2460

ctccatgctc caggtccatg cacaggtgta gcatgtcctg cttccgtttt tcataaatta 2520

atctgggtgt tgggggtagt gggaggagaa cggtcagaat caaagtgaca ttctaagaaa 2580

aactgtacct tagagatttt cctctagtgc tcaaacaaat acaaaataag atccccaagg 2640

tttaaactgc ccagttagca ttctgacatt ctaaaagccg gcaaagcagc ttttagtgga 2700

taaatgggaa tggaaacgtg tgtgttcctc caaattttct agtatgatcg gtgagctgtt 2760

ttgtaaagaa gcctcatatt acagagttgc ttttgcacct aaatttagaa ttgtattcca 2820

tgaactgttc ctccctttc tctgcttttc tcctctctgt tcctctttta ataccacacg 2880

tctgttgctt gcatttagtt tgtcttcttc cttcagctgt gtatcccaga ctgttaatac 2940

agaaaagaga catttcagct gtgattatga ccattgtttc atattccaat taaaaaaaga 3000

acagcagcct agctacttaa ggtggggatt tccatagttc caaagaagat ttagcagatt 3060

agagtgagtt cacacttttc aggtgccact gtaaggttct ctcagcctgg gaaactatca 3120

actctttctt taaaaagaaa gagggttgaa aatcctctgg acgaacagaa gtcactttgg 3180

ctgttcagta aggccaatgt taacaacacg tttagaggag gaaaagttca acctcaagtt 3240

aaatggtttg acttattctt cgtatcatta gaagaacccc agagatagca ttcctctatt 3300

ttattttact ttcttttgga ttgcactgat tgtttttgtg ggaatgacac tttatctggc 3360

aaagtaactg agagtttggt aaaagaatat tttcttctct gaataataat tattttcaca 3420

gtgaaaattt cagtatttta tcactaatgt atgagcaatg atctatatca atttcaaggc 3480

acgtgaaaaa aattttttag tatgtgcaat ttaatataga aagatttctg cctgtttgga 3540

caataggttt tgggtagtac agattaggat aagtaagctt atatatgcac agagattatt 3600

gtattacctg taaattgatt tacaagtact taaaagcgtg gtccccagtg aggccaagaa 3660

agtttccggt taagttcttt aataataatc ctacagttta tcttaagaaa aaaaaaaagg 3720

tttgaaaaaa acactttaat ttaggcttcg ttggttgatg gtggaaaaaa atgctcagga 3780

aatatttcag atatttgcca aaaaaccagt aataaggtta ccttattaaa atagtgatat 3840

ttgctttact atttaaggtg tcactgataa attaatttgt acttctgtgt ttagaaatta 3900

tagcttcttt tcccttagtc aaatttttta gcatattata cacatttctg tgtaatctgt 3960

ggaagtgcaa taatatgtta gtaagttaca ttttaaataa tgctcatgtg acaatactcc 4020

caatcaatgg cttatagaat ttaaagatct gtatattaga ttttggctta aaggcatgag 4080

aagtataaga cttggtttgg tggctttgta agaccaccag cctcttaatg atggttagct 4140

tctttaggtc attaaatcaa taaaaacata taatgctgtt ttgctcttct aatgctcctc 4200

ttccatttcc agttatcttc acatttacat ttaaatatac aaacctgagc ctgccattat 4260

taatttccct ataaaatgac gatacatgtg aacatttata aatggactaa tactgcttgt 4320

ctttccccca ccgcacaaaa ctggttctta agatgccagc aatgaatttg agactatctt 4380

tatttataaa tggaaaccgg aaacttttat accaaactat aataatgtgc agcactgtag 4440

ggcttttttt tttttccctc caaatacagt gaaatttttt tattcacaag agctgccaca 4500

tctcagcatt tagtaataga gctgctttaa taaaattcta gtttgattgt catgtcaaaa 4560

aaagaaaaat gttgcatctt tgtgatttta aaacataaat taatgaaggc tctgataggc 4620

tattaggagt tggcttggaa acagtttttg gtctcacagg ttaccattgt ttggggatgt 4680

ctgagctgtt ttcagatcta ggaatagcac agtgttgtct tgtctttggc agtctcattt 4740

ggctctgttt cttgcaccac cagcgtgttc attaccactt aaatatattg ctacagcagt 4800

ggaacaacag agtggtgcaa gacactgtag attaacggta gaggagaaat tgtgccctta 4860

gtgttaacaa tgtgcctttt gttctgaatg ccatgttgta gggcatgcat tttttggcct 4920

ctttaactct tcgaattcta gtcagtaaga atggaaccca tctctgcaaa gatacatctg 4980

tcttaaatat ctagttacag gccttaatag aaaccataag gcatgactca tcttcaggca 5040

ctgaaaaaag ataaccatca ggtagtgtta cacaaggact tcctatattt aaggggttaa 5100

agatggtctt tgttgtatct taacatcaga ctgattttta catttttttt ttgttatgct 5160

aacactagac aaaaatcaac tgtatttgta aaaatttacc tcaaaccatt taattttata 5220

gtgtgattaa tcccagggca tttggtatga accaaagtgc attcctttta tatgtgcctg 5280

gctctagtaa ggatggccag ggatttttac aatttgggtg caaggcactt aagccacttt 5340

taaacttaat gggtggtttg gggtcgtgtt aaatgactcc atcagaatgt tagaaaacac 5400

tttaggcatc agtagcattg ggccatattg gaatcctaaa gtgtgaatta ttttaaggag 5460

agcattcatt tttgtaattt ttttcatcaa aaatatttct ggtaagcaga agacttttta 5520

aaaaaactga tctggtctcg gtaaaggttt taatattgcc caacataatg ctgtaatagc 5580

attaaaaaaa gtatttgtga actctgtttc ttaggggctt gtacatctct ctgctatgga 5640

catacataaa attaattgta attatactca gctcaactgc tacagttctg tctaggcagt 5700

ggcttggggtt tttatcgagc aacaacttag acacgtgact gtaatatgct gcaactgtgt 5760

gtactgaaaa tatgtgaaaa tggttgaatg tggactgtgt atatatgtat gtaaaaattt 5820

ctgtgagatg ctgctgtcgc cacttaacat taaatatgtt ctagtggatt ttaatcctag 5880

tggccagttc tatgatactg tatgtattat acagctgatg acaggagtaa gactgtttag 5940

tgaatatctg ttaaatttta ttgttgtggc cagagataat ttcagaataa aattttaatg 6000

tcctacctta 6010

<210> 148
<211> 6159
<212> DNA
<213> Homo sapiens

<400> 148

```
cgcgggggcg cgcgcgcgcg ggcccgggag aggctcccga gccaggcggt cttcggtcct      60
cgcagcgctt ccagctcccc gcgcccctat gtgagggaga cggggaggcc cgcggcgcgc     120
aggggagggc gaggcatgtg cacgggccgg agggtgctgc agccgcccga ggaagaggag     180
gacggcggcg aggaggagag cggggggctc gcggcggcgg gccccggccg aggggatgca     240
gtggactgtg tgtgtctggc tgtagcagac gcgaggcggc gacgaggcgc cggggacccg     300
cgcgaggggc ggccgggagg cggcggcggc ggccgccaga agtagcagca ggaccggcgg     360
cggcgacggc agccctgaaa tgcattttcc tctccagcgg ccatgttaac caggaaacct     420
tcggccgccg ctcccgccgc ctacccgacc ggccgaggtg gggacagcgc cgttcgtcag     480
cttcaggctt ccccggggct cggtgcaggg gccacccgga gcggagtggg gactggcccg     540
ccctccccca tcgccctgcc gcctctccgg gccagcaacg ctgccgccgc agcccacacg     600
attggcggca gtaagcacac aatgaatgat cacctgcatg tcggcagcca cgctcacgga     660
cagatccagg ttcaacagtt gtttgaggat aacagtaaca agcggacagt gctcacgaca     720
caaccaaatg ggcttacaac agtgggcaaa acgggcttgc cagtggtgcc agagcggcag     780
ctggacagca ttcatagacg gcaggggagc tccacctctc taaagtccat ggaaggcatg     840
gggaaggtga aagccacccc catgacacct gaacaagcaa tgaagcaata catgcaaaaa     900
ctcacagcct tcgaacacca tgagattttc agctaccctg aaatatattt cttgggtcta     960
```

aatgctaaga agcgccaggg catgacaggt gggcccaaca atggtggcta tgatgatgac 1020
cagggatcat atgtgcaggt gccccacgat cacgtggctt acaggtatga ggtcctcaag 1080
gtcattggga aggggagctt tgggcaggtg gtcaaggcct acgatcacaa agtccaccag 1140
cacgtggccc taaagatggt gcggaatgag aagcgcttcc accggcaagc agcggaggag 1200
atccgaatcc tggaacacct gcggaagcag gacaaggata acacaatgaa tgtcatccat 1260
atgctggaga atttcacctt ccgcaaccac atctgcatga cgtttgagct gctgagcatg 1320
aacctctatg agctcatcaa gaagaataaa ttccagggct tcagtctgcc tttggttcgc 1380
aagtttgccc actcgattct gcagtgcttg gatgctttgc acaaaaacag aataattcac 1440
tgtgacctta agcccgagaa cattttgtta aagcagcagg gtagaagcgg tattaaagta 1500
attgattttg gctccagttg ttacgagcat cagcgtgtct acacgtacat ccagtcgcgt 1560
ttttaccggg ctccagaagt gatccttggg gccaggtatg gcatgcccat tgatatgtgg 1620
agcctgggct gcattttagc agagctcctg acgggttacc ccctcttgcc tggggaagat 1680
gaaggggacc agctggcctg tatgattgaa ctgttgggca tgccctcaca gaaactgctg 1740
gatgcatcca aacgagccaa aaattttgtg agctccaagg gttatccccg ttactgcact 1800
gtcacgactc tctcagatgg ctctgtggtc ctaaacggag gccgttcccg gagggggaaa 1860
ctgaggggcc caccggagag cagagagtgg gggaacgcgc tgaaggggtg tgatgatccc 1920
cttttccttg acttcttaaa acagtgttta gagtgggatc ctgcagtgcg catgacccca 1980
ggccaggctt tgcggcaccc ctggctgagg aggcggttgc caaagcctcc caccggggag 2040
aaaacgtcag tgaaaaggat aactgagagc accggtgcta tcacatctat atccaagtta 2100
cctccacctt ctagctcagc ttccaaactg aggactaatt tggcgcagat gacagatgcc 2160
aatgggaata ttcagcagag gacagtgttg ccaaaacttg ttagctgagc tcacgtcccc 2220
tgatgctggt aacctgaaag atacgacatt gctgagcctt actgggttga aaaggagtag 2280
ctcagacctg tttttatttg ctcaataact ctactcattt gtatcttttc agcacttaat 2340
tttaatgtaa gaaagttgtt cattttgttt ttataaaata catgaggaca atgctttaag 2400
tttttatact ttcagaaact tttgtgttc taaaagtaca atgagcctta ctgtatttag 2460
tgtggcagaa taataacatc agtggcaggc cactgattac ttcatgactg ccacgcattt 2520
acagattggt gtcaaagaca ttcactatgt ttttatggtt catgttatat cctccccagg 2580
gtgacagccc cttaaggccc tccttttccc tccatgctcc aggtccatgc acaggtgtag 2640
catgtcctgc ttccgttttt cataaattaa tctgggtgtt gggggtagtg ggaggagaac 2700
ggtcagaatc aaagtgacat tctaagaaaa actgtacctt agagattttc ctctagtgct 2760
caaacaaata caaaataaga tccccaaggt ttaaactgcc cagttagcat tctgacattc 2820
taaaagccgg caaagcagct tttagtggat aaatgggaat ggaaacgtgt gtgttcctcc 2880

aaattttcta gtatgatcgg tgagctgttt tgtaaagaag cctcatatta cagagttgct 2940
tttgcaccta aatttagaat tgtattccat gaactgttcc tccctttct ctgcttttct 3000
cctctctgtt cctcttttaa taccacacgt ctgttgcttg catttagttt gtcttcttcc 3060
ttcagctgtg tatcccagac tgttaataca gaaaagagac atttcagctg tgattatgac 3120
cattgtttca tattccaatt aaaaaaagaa cagcagccta gctacttaag gtggggattt 3180
ccatagttcc aaagaagatt tagcagatta gagtgagttc acacttttca ggtgccactg 3240
taaggttctc tcagcctggg aaactatcaa ctctttcttt aaaaagaaag agggttgaaa 3300
atcctctgga cgaacagaag tcactttggc tgttcagtaa ggccaatgtt aacaacacgt 3360
ttagaggagg aaaagttcaa cctcaagtta aatggtttga cttattcttc gtatcattag 3420
aagaacccca gagatagcat tcctctattt tattttactt tcttttggat tgcactgatt 3480
gtttttgtgg gaatgacact ttatctggca aagtaactga gagtttggta aaagaatatt 3540
ttcttctctg aataataatt attttcacag tgaaaatttc agtattttat cactaatgta 3600
tgagcaatga tctatatcaa tttcaaggca cgtgaaaaaa attttttagt atgtgcaatt 3660
taatatagaa agatttctgc ctgtttggac aataggtttt gggtagtaca gattaggata 3720
agtaagctta tatatgcaca gagattattg tattacctgt aaattgattt acaagtactt 3780
aaaagcgtgg tccccagtga ggccaagaaa gtttccggtt aagttcttta ataataatcc 3840
tacagtttat cttaagaaaa aaaaaaaggt ttgaaaaaaa cactttaatt taggcttcgt 3900
tggttgatgg tggaaaaaaa tgctcaggaa atatttcaga tatttgccaa aaaaccagta 3960
ataaggttac cttattaaaa tagtgatatt tgctttacta tttaaggtgt cactgataaa 4020
ttaatttgta cttctgtgtt tagaaattat agcttctttt cccttagtca aatttttag 4080
catattatac acatttctgt gtaatctgtg gaagtgcaat aatatgttag taagttacat 4140
tttaaataat gctcatgtga caatactccc aatcaatggc ttatagaatt taaagatctg 4200
tatattagat tttggcttaa aggcatgaga agtataagac ttggtttggt ggctttgtaa 4260
gaccaccagc ctcttaatga tggttagctt ctttaggtca ttaaatcaat aaaaacatat 4320
aatgctgttt tgctcttcta atgctcctct tccatttcca gttatcttca catttacatt 4380
taaatataca aacctgagcc tgccattatt aatttcccta taaaatgacg atacatgtga 4440
acatttataa atggactaat actgcttgtc tttcccccac cgcacaaaac tggttcttaa 4500
gatgccagca atgaatttga gactatcttt atttataaat ggaaaccgga aacttttata 4560
ccaaactata ataatgtgca gcactgtagg gctttttttt ttttccctcc aaatacagtg 4620
aaattttttt attcacaaga gctgccacat ctcagcattt agtaatagag ctgctttaat 4680
aaaattctag tttgattgtc atgtcaaaaa aagaaaaatg ttgcatcttt gtgattttaa 4740

aacataaatt aatgaaggct ctgataggct attaggagtt ggcttggaaa cagtttttgg 4800

tctcacaggt taccattgtt tggggatgtc tgagctgttt tcagatctag gaatagcaca 4860

gtgttgtctt gtctttggca gtctcatttg gctctgtttc ttgcaccacc agcgtgttca 4920

ttaccactta aatatattgc tacagcagtg gaacaacaga gtggtgcaag acactgtaga 4980

ttaacggtag aggagaaatt gtgcccttag tgttaacaat gtgccttttg ttctgaatgc 5040

catgttgtag ggcatgcatt ttttggcctc tttaactctt cgaattctag tcagtaagaa 5100

tggaacccat ctctgcaaag atacatctgt cttaaatatc tagttacagg ccttaataga 5160

aaccataagg catgactcat cttcaggcac tgaaaaaaga taaccatcag gtagtgttac 5220

acaaggactt cctatattta aggggttaaa gatggtcttt gttgtatctt aacatcagac 5280

tgatttttac attttttttt tgttatgcta acactagaca aaaatcaact gtatttgtaa 5340

aaatttacct caaaccattt aattttatag tgtgattaat cccagggcat ttggtatgaa 5400

ccaaagtgca ttccttttat atgtgcctgg ctctagtaag gatggccagg gatttttaca 5460

atttgggtgc aaggcactta agccactttt aaacttaatg ggtggtttgg ggtcgtgtta 5520

aatgactcca tcagaatgtt agaaaacact ttaggcatca gtagcattgg gccatattgg 5580

aatcctaaag tgtgaattat tttaaggaga gcattcattt ttgtaatttt tttcatcaaa 5640

aatatttctg gtaagcagaa gactttttaa aaaaactgat ctggtctcgg taaaggtttt 5700

aatattgccc aacataatgc tgtaatagca ttaaaaaaag tatttgtgaa ctctgtttct 5760

taggggcttg tacatctctc tgctatggac atacataaaa ttaattgtaa ttatactcag 5820

ctcaactgct acagttctgt ctaggcagtg gcttgggttt ttatcgagca acaacttaga 5880

cacgtgactg taatatgctg caactgtgtg tactgaaaat atgtgaaaat ggttgaatgt 5940

ggactgtgta tatatgtatg taaaaatttc tgtgagatgc tgctgtcgcc acttaacatt 6000

aaatatgttc tagtggattt taatcctagt ggccagttct atgatactgt atgtattata 6060

cagctgatga caggagtaag actgtttagt gaatatctgt taaattttat tgttgtggcc 6120

agagataatt tcagaataaa attttaatgt cctacctta 6159

<210> 149
<211> 528
<212> PRT
<213> Homo sapiens

<400> 149

```
Met Asn Asp His Leu His Val Gly Ser His Ala His Gly Gln Ile Gln
1               5                   10                  15

Val Gln Gln Leu Phe Glu Asp Asn Ser Asn Lys Arg Thr Val Leu Thr
            20                  25                  30
```

```
Thr Gln Pro Asn Gly Leu Thr Thr Val Gly Lys Thr Gly Leu Pro Val
        35                  40                  45

Val Pro Glu Arg Gln Leu Asp Ser Ile His Arg Arg Gln Gly Ser Ser
    50                  55                  60

Thr Ser Leu Lys Ser Met Glu Gly Met Gly Lys Val Lys Ala Thr Pro
65                  70                  75                  80

Met Thr Pro Glu Gln Ala Met Lys Gln Tyr Met Gln Lys Leu Thr Ala
                85                  90                  95

Phe Glu His His Glu Ile Phe Ser Tyr Pro Glu Ile Tyr Phe Leu Gly
            100                 105                 110

Leu Asn Ala Lys Lys Arg Gln Gly Met Thr Gly Gly Pro Asn Asn Gly
        115                 120                 125

Gly Tyr Asp Asp Asp Gln Gly Ser Tyr Val Gln Val Pro His Asp His
    130                 135                 140

Val Ala Tyr Arg Tyr Glu Val Leu Lys Val Ile Gly Lys Gly Ser Phe
145                 150                 155                 160

Gly Gln Val Val Lys Ala Tyr Asp His Lys Val His Gln His Val Ala
                165                 170                 175

Leu Lys Met Val Arg Asn Glu Lys Arg Phe His Arg Gln Ala Ala Glu
            180                 185                 190

Glu Ile Arg Ile Leu Glu His Leu Arg Lys Gln Asp Lys Asp Asn Thr
        195                 200                 205

Met Asn Val Ile His Met Leu Glu Asn Phe Thr Phe Arg Asn His Ile
    210                 215                 220

Cys Met Thr Phe Glu Leu Leu Ser Met Asn Leu Tyr Glu Leu Ile Lys
225                 230                 235                 240

Lys Asn Lys Phe Gln Gly Phe Ser Leu Pro Leu Val Arg Lys Phe Ala
                245                 250                 255

His Ser Ile Leu Gln Cys Leu Asp Ala Leu His Lys Asn Arg Ile Ile
            260                 265                 270

His Cys Asp Leu Lys Pro Glu Asn Ile Leu Leu Lys Gln Gln Gly Arg
        275                 280                 285
```

Ser Gly Ile Lys Val Ile Asp Phe Gly Ser Ser Cys Tyr Glu His Gln
290 295 300

Arg Val Tyr Thr Tyr Ile Gln Ser Arg Phe Tyr Arg Ala Pro Glu Val
305 310 315 320

Ile Leu Gly Ala Arg Tyr Gly Met Pro Ile Asp Met Trp Ser Leu Gly
325 330 335

Cys Ile Leu Ala Glu Leu Leu Thr Gly Tyr Pro Leu Leu Pro Gly Glu
340 345 350

Asp Glu Gly Asp Gln Leu Ala Cys Met Ile Glu Leu Leu Gly Met Pro
355 360 365

Ser Gln Lys Leu Leu Asp Ala Ser Lys Arg Ala Lys Asn Phe Val Ser
370 375 380

Ser Lys Gly Tyr Pro Arg Tyr Cys Thr Val Thr Thr Leu Ser Asp Gly
385 390 395 400

Ser Val Val Leu Asn Gly Gly Arg Ser Arg Arg Gly Lys Leu Arg Gly
405 410 415

Pro Pro Glu Ser Arg Glu Trp Gly Asn Ala Leu Lys Gly Cys Asp Asp
420 425 430

Pro Leu Phe Leu Asp Phe Leu Lys Gln Cys Leu Glu Trp Asp Pro Ala
435 440 445

Val Arg Met Thr Pro Gly Gln Ala Leu Arg His Pro Trp Leu Arg Arg
450 455 460

Arg Leu Pro Lys Pro Pro Thr Gly Glu Lys Thr Ser Val Lys Arg Ile
465 470 475 480

Thr Glu Ser Thr Gly Ala Ile Thr Ser Ile Ser Lys Leu Pro Pro Pro
485 490 495

Ser Ser Ser Ala Ser Lys Leu Arg Thr Asn Leu Ala Gln Met Thr Asp
500 505 510

Ala Asn Gly Asn Ile Gln Gln Arg Thr Val Leu Pro Lys Leu Val Ser
515 520 525

<210> 150
<211> 601

<212> PRT
<213> Homo sapiens

<400> 150

```
Met Leu Thr Arg Lys Pro Ser Ala Ala Ala Pro Ala Ala Tyr Pro Thr
1               5                   10                  15

Gly Arg Gly Gly Asp Ser Ala Val Arg Gln Leu Gln Ala Ser Pro Gly
            20                  25                  30

Leu Gly Ala Gly Ala Thr Arg Ser Gly Val Gly Thr Gly Pro Pro Ser
        35                  40                  45

Pro Ile Ala Leu Pro Pro Leu Arg Ala Ser Asn Ala Ala Ala Ala Ala
    50                  55                  60

His Thr Ile Gly Gly Ser Lys His Thr Met Asn Asp His Leu His Val
65                  70                  75                  80

Gly Ser His Ala His Gly Gln Ile Gln Val Gln Gln Leu Phe Glu Asp
                85                  90                  95

Asn Ser Asn Lys Arg Thr Val Leu Thr Thr Gln Pro Asn Gly Leu Thr
            100                 105                 110

Thr Val Gly Lys Thr Gly Leu Pro Val Val Pro Glu Arg Gln Leu Asp
        115                 120                 125

Ser Ile His Arg Arg Gln Gly Ser Ser Thr Ser Leu Lys Ser Met Glu
    130                 135                 140

Gly Met Gly Lys Val Lys Ala Thr Pro Met Thr Pro Glu Gln Ala Met
145                 150                 155                 160

Lys Gln Tyr Met Gln Lys Leu Thr Ala Phe Glu His His Glu Ile Phe
                165                 170                 175

Ser Tyr Pro Glu Ile Tyr Phe Leu Gly Leu Asn Ala Lys Lys Arg Gln
            180                 185                 190

Gly Met Thr Gly Gly Pro Asn Asn Gly Gly Tyr Asp Asp Asp Gln Gly
        195                 200                 205

Ser Tyr Val Gln Val Pro His Asp His Val Ala Tyr Arg Tyr Glu Val
    210                 215                 220

Leu Lys Val Ile Gly Lys Gly Ser Phe Gly Gln Val Val Lys Ala Tyr
```

```
225                 230                 235                 240

Asp His Lys Val His Gln His Val Ala Leu Lys Met Val Arg Asn Glu
                245                 250                 255

Lys Arg Phe His Arg Gln Ala Ala Glu Glu Ile Arg Ile Leu Glu His
            260                 265                 270

Leu Arg Lys Gln Asp Lys Asp Asn Thr Met Asn Val Ile His Met Leu
        275                 280                 285

Glu Asn Phe Thr Phe Arg Asn His Ile Cys Met Thr Phe Glu Leu Leu
    290                 295                 300

Ser Met Asn Leu Tyr Glu Leu Ile Lys Lys Asn Lys Phe Gln Gly Phe
305                 310                 315                 320

Ser Leu Pro Leu Val Arg Lys Phe Ala His Ser Ile Leu Gln Cys Leu
                325                 330                 335

Asp Ala Leu His Lys Asn Arg Ile Ile His Cys Asp Leu Lys Pro Glu
            340                 345                 350

Asn Ile Leu Leu Lys Gln Gln Gly Arg Ser Gly Ile Lys Val Ile Asp
        355                 360                 365

Phe Gly Ser Ser Cys Tyr Glu His Gln Arg Val Tyr Thr Tyr Ile Gln
    370                 375                 380

Ser Arg Phe Tyr Arg Ala Pro Glu Val Ile Leu Gly Ala Arg Tyr Gly
385                 390                 395                 400

Met Pro Ile Asp Met Trp Ser Leu Gly Cys Ile Leu Ala Glu Leu Leu
                405                 410                 415

Thr Gly Tyr Pro Leu Leu Pro Gly Glu Asp Glu Gly Asp Gln Leu Ala
            420                 425                 430

Cys Met Ile Glu Leu Leu Gly Met Pro Ser Gln Lys Leu Leu Asp Ala
        435                 440                 445

Ser Lys Arg Ala Lys Asn Phe Val Ser Ser Lys Gly Tyr Pro Arg Tyr
    450                 455                 460

Cys Thr Val Thr Thr Leu Ser Asp Gly Ser Val Val Leu Asn Gly Gly
465                 470                 475                 480
```

```
Arg Ser Arg Arg Gly Lys Leu Arg Gly Pro Pro Glu Ser Arg Glu Trp
                485                 490                 495

Gly Asn Ala Leu Lys Gly Cys Asp Asp Pro Leu Phe Leu Asp Phe Leu
            500                 505                 510

Lys Gln Cys Leu Glu Trp Asp Pro Ala Val Arg Met Thr Pro Gly Gln
        515                 520                 525

Ala Leu Arg His Pro Trp Leu Arg Arg Arg Leu Pro Lys Pro Pro Thr
    530                 535                 540

Gly Glu Lys Thr Ser Val Lys Arg Ile Thr Glu Ser Thr Gly Ala Ile
545                 550                 555                 560

Thr Ser Ile Ser Lys Leu Pro Pro Pro Ser Ser Ser Ala Ser Lys Leu
                565                 570                 575

Arg Thr Asn Leu Ala Gln Met Thr Asp Ala Asn Gly Asn Ile Gln Gln
            580                 585                 590

Arg Thr Val Leu Pro Lys Leu Val Ser
        595                 600
```

<210> 151
<211> 21
<212> DNA
<213> Artificial Sequence

<220>
<223> DYRK2_forward primer

<400> 151
cggccatgtt aaccaggaaa c 21

<210> 152
<211> 20
<212> DNA
<213> Artificial Sequence

<220>
<223> DYRK2_reverse primer

<400> 152
cgacatgcag gtgatcattc 20

<210> 153
<211> 24
<212> DNA
<213> Artificial Sequence

<220>

<223> DYRK2 probe

<400> 153
cgattggcgg cagtaagcac acaa 24

<210> 154
<211> 1921
<212> DNA
<213> Homo sapiens

<400> 154

ggcggaagtg acattatcaa cgcgcgccag gggttcagtg aggtcgggca ggttcgctgt 60
ggcgggcgcc tgggccgccg gctgtttaac ttcgcttccg ctggcccata gtgatctttg 120
cagtgaccca gcatcactgt ttcttggcgt gtgaagataa cccaaggaat tgaggaagtt 180
gctgagaaga gtgtgctgga gatgctctag gaaaaaattg aatagtgaga cgagttccag 240
cgcaagggtt tctggtttgc caagaagaaa gtgaacatca tggatcagaa caacagcctg 300
ccaccttacg ctcagggctt ggcctcccct cagggtgcca tgactcccgg aatccctatc 360
tttagtccaa tgatgcctta tggcactgga ctgaccccac agcctattca gaacaccaat 420
agtctgtcta ttttggaaga gcaacaaagg cagcagcagc aacaacaaca gcagcagcag 480
cagcagcagc agcaacagca acagcagcag cagcagcagc agcagcagca gcagcagcag 540
cagcagcagc agcagcagca acaggcagtg gcagctgcag ccgttcagca gtcaacgtcc 600
cagcaggcaa cacagggaac ctcaggccag gcaccacagc tcttccactc acagactctc 660
acaactgcac ccttgccggg caccactcca ctgtatccct cccccatgac tcccatgacc 720
cccatcactc ctgccacgcc agcttcggag agttctggga ttgtaccgca gctgcaaaat 780
attgtatcca cagtgaatct tggttgtaaa cttgacctaa agaccattgc acttcgtgcc 840
cgaaacgccg aatataatcc caagcggttt gctgcggtaa tcatgaggat aagagagcca 900
cgaaccacgg cactgatttt cagttctggg aaaatggtgt gcacaggagc caagagtgaa 960
gaacagtcca gactggcagc aagaaaatat gctagagttg tacagaagtt gggttttcca 1020
gctaagttct tggacttcaa gattcagaat atggtgggga gctgtgatgt gaagtttcct 1080
ataaggttag aaggccttgt gctcacccac caacaattta gtagttatga gccagagtta 1140
tttcctggtt taatctacag aatgatcaaa cccagaattg ttctccttat ttttgtttct 1200
ggaaaagttg tattaacagg tgctaaagtc agagcagaaa tttatgaagc atttgaaaac 1260
atctacccta ttctaaaggg attcaggaag acgacgtaat ggctctcatg taccctttgcc 1320
tcccccaccc ccttcttttt tttttttaa acaaatcagt ttgttttggt acctttaaat 1380
ggtggtgttg tgagaagatg gatgttgagt tgcagggtgt ggcaccaggt gatgcccttc 1440
tgtaagtgcc caccgcggga tgccgggaag gggcattatt tgtgcactga gaacaccgcg 1500
cagcgtgact gtgagttgct cataccgtgc tgctatctgg gcagcgctgc ccatttattt 1560

```
atatgtagat tttaaacact gctgttgaca agttggtttg agggagaaaa ctttaagtgt      1620
taaagccacc tctataattg attggacttt ttaattttaa tgtttttccc catgaaccac      1680
agtttttata tttctaccag aaaagtaaaa atcttttttta aaagtgttgt ttttctaatt      1740
tataactcct aggggttatt tctgtgccag acacattcca cctctccagt attgcaggac      1800
agaatatatg tgttaatgaa aatgaatggc tgtacatatt tttttctttc ttcagagtac      1860
tctgtacaat aaatgcagtt tataaaagtg ttagattgtt gttaaaaaaa aaaaaaaaaa      1920
a                                                                       1921
```

<210> 155
<211> 1435
<212> DNA
<213> Homo sapiens

<400> 155

ggcggggcct gcttctcctc agcttcaggc ggctgcgacg agccctcagg cgaacctctc 60
ggctttcccg cgcggcgccg cctcttgctg cgcctccgcc tcctcctctg ctccgccacc 120
ggcttcctcc tcctgagcag tcagcccgcg cgccggccgg ctccgttatg gcgacccgca 180
gccctggcgt cgtgattagt gatgatgaac caggttatga ccttgattta ttttgcatac 240
ctaatcatta tgctgaggat ttggaaaggg tgtttattcc tcatggacta attatggaca 300
ggactgaacg tcttgctcga gatgtgatga aggagatggg aggccatcac attgtagccc 360
tctgtgtgct caaggggggc tataaattct ttgctgacct gctggattac atcaaagcac 420
tgaatagaaa tagtgataga tccattccta tgactgtaga ttttatcaga ctgaagagct 480
attgtaatga ccagtcaaca ggggacataa aagtaattgg tggagatgat ctctcaactt 540
taactggaaa gaatgtcttg attgtggaag atataattga cactggcaaa acaatgcaga 600
ctttgctttc cttggtcagg cagtataatc caaagatggt caaggtcgca agcttgctgg 660
tgaaaaggac cccacgaagt gttggatata agccagactt tgttggattt gaaattccag 720
acaagtttgt tgtaggatat gcccttgact ataatgaata cttcagggat ttgaatcatg 780
tttgtgtcat tagtgaaact ggaaaagcaa aatacaaagc ctaagatgag agttcaagtt 840
gagtttggaa acatctggag tcctattgac atcgccagta aaattatcaa tgttctagtt 900
ctgtggccat ctgcttagta gagctttttg catgtatctt ctaagaattt tatctgtttt 960
gtactttaga aatgtcagtt gctgcattcc taaactgttt atttgcacta tgagcctata 1020
gactatcagt tccctttggg cggattgttg tttaacttgt aaatgaaaaa attctcttaa 1080
accacagcac tattgagtga aacattgaac tcatatctgt aagaaataaa gagaagatat 1140
attagttttt taattggtat tttaattttt atatatgcag gaaagaatag aagtgattga 1200
atattgttaa ttataccacc gtgtgttaga aaagtaagaa gcagtcaatt ttcacatcaa 1260
agacagcatc taagaagttt tgttctgtcc tggaattatt ttagtagtgt ttcagtaatg 1320
ttgactgtat tttccaactt gttcaaatta ttaccagtga atctttgtca gcagttccct 1380
tttaaatgca aatcaataaa ttcccaaaaa tttaaaaaaa aaaaaaaaaa aaaaa 1435

<210> 156
<211> 1852
<212> DNA
<213> Homo sapiens

<400> 156

accgccgaga ccgcgtccgc cccgcgagca cagagcctcg cctttgccga tccgccgccc 60
gtccacaccc gccgccagct caccatggat gatgatatcg ccgcgctcgt cgtcgacaac 120
ggctccggca tgtgcaaggc cggcttcgcg ggcgacgatg ccccccgggc cgtcttcccc 180
tccatcgtgg ggcgccccag gcaccagggc gtgatggtgg gcatgggtca gaaggattcc 240
tatgtgggcg acgaggccca gagcaagaga ggcatcctca ccctgaagta ccccatcgag 300
cacggcatcg tcaccaactg ggacgacatg gagaaaatct ggcaccacac cttctacaat 360
gagctgcgtg tggctcccga ggagcacccc gtgctgctga ccgaggcccc cctgaacccc 420
aaggccaacc gcgagaagat gacccagatc atgtttgaga ccttcaacac cccagccatg 480
tacgttgcta tccaggctgt gctatccctg tacgcctctg gccgtaccac tggcatcgtg 540
atggactccg gtgacggggt cacccacact gtgcccatct acgaggggta tgccctcccc 600
catgccatcc tgcgtctgga cctggctggc cgggacctga ctgactacct catgaagatc 660
ctcaccgagc gcggctacag cttcaccacc acggccgagc gggaaatcgt gcgtgacatt 720
aaggagaagc tgtgctacgt cgccctggac ttcgagcaag agatggccac ggctgcttcc 780
agctcctccc tggagaagag ctacgagctg cctgacggcc aggtcatcac cattggcaat 840
gagcggttcc gctgccctga ggcactcttc cagccttcct tcctgggcat ggagtcctgt 900
ggcatccacg aaactacctt caactccatc atgaagtgtg acgtggacat ccgcaaagac 960
ctgtacgcca acacagtgct gtctggcggc accaccatgt accctggcat tgccgacagg 1020
atgcagaagg agatcactgc cctggcaccc agcacaatga agatcaagat cattgctcct 1080
cctgagcgca agtactccgt gtggatcggc ggctccatcc tggcctcgct gtccaccttc 1140
cagcagatgt ggatcagcaa gcaggagtat gacgagtccg gcccctccat cgtccaccgc 1200
aaatgcttct aggcggacta tgacttagtt gcgttacacc ctttcttgac aaaacctaac 1260
ttgcgcagaa aacaagatga gattggcatg gctttatttg tttttttgt tttgttttgg 1320
tttttttttt ttttttggct tgactcagga tttaaaaact ggaacggtga aggtgacagc 1380
agtcggttgg agcgagcatc ccccaaagtt cacaatgtgg ccgaggactt tgattgcaca 1440
ttgttgtttt tttaatagtc attccaaata tgagatgcgt tgttacagga agtcccttgc 1500
catcctaaaa gccaccccac ttctctctaa ggagaatggc ccagtcctct cccaagtcca 1560
cacaggggag gtgatagcat tgctttcgtg taaattatgt aatgcaaaat ttttttaatc 1620
ttcgccttaa tactttttta ttttgtttta ttttgaatga tgagccttcg tgccccccct 1680
tccccctttt ttgtccccca acttgagatg tatgaaggct tttggtctcc ctgggagtgg 1740
gtggaggcag ccagggctta cctgtacact gacttgagac cagttgaata aaagtgcaca 1800
ccttaaaaat gaaaaaaaaa aaaaaaaaaa aaaaaaaaaa aaaaaaaaaa aa 1852

<210> 157
<211> 1229
<212> DNA
<213> Homo sapiens

<400> 157

```
gtctgacggg cgatggcgca gccaatagac aggagcgcta tccgcggttt ctgattggct      60
actttgttcg cattataaaa ggcacgcgcg ggcgcgaggc ccttctctcg ccaggcgtcc     120
tcgtggaagt gacatcgtct ttaaaccctg cgtggcaatc cctgacgcac cgccgtgatg     180
cccagggaag acagggcgac ctggaagtcc aactacttcc ttaagatcat ccaactattg     240
gatgattatc cgaaatgttt cattgtggga gcagacaatg tgggctccaa gcagatgcag     300
cagatccgca tgtcccttcg cgggaaggct gtggtgctga tgggcaagaa caccatgatg     360
cgcaaggcca tccgagggca cctggaaaac aacccagctc tggagaaact gctgcctcat     420
atccggggga atgtgggctt tgtgttcacc aaggaggacc tcactgagat cagggacatg     480
ttgctggcca ataaggtgcc agctgctgcc cgtgctggtg ccattgcccc atgtgaagtc     540
actgtgccag cccagaacac tggtctcggg cccgagaaga cctccttttt ccaggcttta     600
ggtatcacca ctaaaatctc caggggcacc attgaaatcc tgagtgatgt gcagctgatc     660
aagactggag acaaagtggg agccagcgaa gccacgctgc tgaacatgct caacatctcc     720
cccttctcct ttgggctggt catccagcag gtgttcgaca atggcagcat ctacaaccct     780
gaagtgcttg atatcacaga ggaaactctg cattctcgct tcctggaggg tgtccgcaat     840
gttgccagtg tctgtctgca gattggctac ccaactgttg catcagtacc ccattctatc     900
atcaacgggt acaaacgagt cctggccttg tctgtggaga cggattacac cttcccactt     960
gctgaaaagg tcaaggcctt cttggctgat ccatctgcct ttgtggctgc tgcccctgtg    1020
gctgctgcca ccacagctgc tcctgctgct gctgcagccc cagctaaggt tgaagccaag    1080
gaagagtcgg aggagtcgga cgaggatatg ggatttggtc tctttgacta atcaccaaaa    1140
agcaaccaac ttagccagtt ttatttgcaa aacaaggaaa taaaggctta cttctttaaa    1200
aagtaaaaaa aaaaaaaaaa aaaaaaaaa                                      1229
```

<210> 158
<211> 1229
<212> DNA
<213> Homo sapiens

<400> 158

gtctgacggg cgatggcgca gccaatagac aggagcgcta tccgcggttt ctgattggct 60
actttgttcg cattataaaa ggcacgcgcg ggcgcgaggc ccttctctcg ccaggcgtcc 120
tcgtggaagt gacatcgtct ttaaaccctg cgtggcaatc cctgacgcac cgccgtgatg 180
cccagggaag acagggcgac ctggaagtcc aactacttcc ttaagatcat ccaactattg 240
gatgattatc cgaaatgttt cattgtggga gcagacaatg tgggctccaa gcagatgcag 300
cagatccgca tgtcccttcg cgggaaggct gtggtgctga tgggcaagaa caccatgatg 360
cgcaaggcca tccgagggca cctggaaaac aacccagctc tggagaaact gctgcctcat 420
atccggggga atgtgggctt tgtgttcacc aaggaggacc tcactgagat cagggacatg 480
ttgctggcca ataaggtgcc agctgctgcc cgtgctggtg ccattgcccc atgtgaagtc 540
actgtgccag cccagaacac tggtctcggg cccgagaaga cctccttttt ccaggcttta 600
ggtatcacca ctaaaatctc caggggcacc attgaaatcc tgagtgatgt gcagctgatc 660
aagactggag acaaagtggg agccagcgaa gccacgctgc tgaacatgct caacatctcc 720
cccttctcct ttgggctggt catccagcag gtgttcgaca atggcagcat ctacaaccct 780
gaagtgcttg atatcacaga ggaaactctg cattctcgct tcctggaggg tgtccgcaat 840
gttgccagtg tctgtctgca gattggctac ccaactgttg catcagtacc ccattctatc 900
atcaacgggt acaaacgagt cctggccttg tctgtggaga cggattacac cttcccactt 960
gctgaaaagg tcaaggcctt cttggctgat ccatctgcct ttgtggctgc tgcccctgtg 1020
gctgctgcca ccacagctgc tcctgctgct gctgcagccc cagctaaggt tgaagccaag 1080
gaagagtcgg aggagtcgga cgaggatatg ggatttggtc tctttgacta atcaccaaaa 1140
agcaaccaac ttagccagtt ttatttgcaa aacaaggaaa taaaggctta cttctttaaa 1200
aagtaaaaaa aaaaaaaaaa aaaaaaaaa 1229

<210> 159
<211> 6738
<212> DNA
<213> Homo sapiens

<400> 159

gagagcgcgg ccgggacggt tggagaagaa ggcggctccc ggaaggggga gagacaaact 60
gccgtaacct ctgccgttca ggaacccggt tacttattta ttcgttaccc tttttcttct 120
tcctccccca aaaacctttt ccttttccct tcttttttttt tcctttttgg gagctgaaaa 180
atttccggta agggaaagaa gggctccttt cgctccttat ttccccgcct ccttccctcc 240
cccaccttcc cctcctccgg ctttttcctc ccaactcggg gaggtccttc ccggtggccg 300

```
ccctgacgag gtctgagcac ctaggcggag gcggcgcagg ctttttgtag tgaggtttgc      360
gcctgcgcag cgcgcctgcc tccgccatgc acgggggtgg ccccccctcg ggggacagcg      420
catgcccgct gcgcaccatc aagagagtcc agttcggagt cctgagtccg gatgaactga      480
agcgaatgtc tgtgacggag ggtggcatca aatacccaga gacgactgag ggaggccgcc      540
ccaagcttgg ggggctgatg gacccgaggc aggggggtgat tgagcggact ggccgctgcc      600
aaacatgtgc aggaaacatg acagagtgtc ctggccactt tggccacatt gaactggcca      660
agcctgtgtt tcacgtgggc ttcctggtga agacaatgaa agttttgcgc tgtgtctgct      720
tcttctgctc caaactgctt gtggactcta acaacccaaa gatcaaggat atcctggcta      780
agtccaaggg acagcccaag aagcggctca cacatgtcta cgacctttgc aagggcaaaa      840
acatatgcga gggtggggag gagatggaca acaagttcgg tgtggaacaa cctgagggtg      900
acgaggatct gaccaaagaa aagggccatg gtggctgtgg gcggtaccag cccaggatcc      960
ggcgttctgg cctagagctg tatgcggaat ggaagcacgt taatgaggac tctcaggaga     1020
agaagatcct gctgagtcca gagcgagtgc atgagatctt caaacgcatc tcagatgagg     1080
agtgttttgt gctgggcatg gagccccgct atgcacggcc agagtggatg attgtcacag     1140
tgctgcctgt gcccccgctc tccgtgcggc ctgctgttgt gatgcagggc tctgcccgta     1200
accaggatga cctgactcac aaactggctg acatcgtgaa gatcaacaat cagctgcggc     1260
gcaatgagca gaacggcgca gcggcccatg tcattgcaga ggatgtgaag ctcctccagt     1320
tccatgtggc caccatggtg gacaatgagc tgcctggctt gccccgtgcc atgcagaagt     1380
ctgggcgtcc cctcaagtcc ctgaagcagc ggttgaaggg caaggaaggc cgggtgcgag     1440
ggaacctgat gggcaaaaga gtggacttct cggcccgtac tgtcatcacc cccgacccca     1500
acctctccat tgaccaggtt ggcgtgcccc gctccattgc tgccaacatg acctttgcgg     1560
agattgtcac cccctcaac attgacagac ttcaagaact agtgcgcagg gggaacagcc      1620
agtacccagg cgccaagtac atcatccgag acaatggtga tcgcattgac ttgcgtttcc     1680
accccaagcc cagtgacctt cacctgcaga ccggctataa ggtggaacgg cacatgtgtg     1740
atggggacat tgttatcttc aaccggcagc caactctgca caaaatgtcc atgatggggc     1800
atcgggtccg cattctccca tggtctacct ttcgcttgaa tcttagtgtg acaactccgt     1860
acaatgcaga ctttgacggg gatgagatga acttgcacct gccacagtct ctggagacgc     1920
gagcagagat ccaggagctg gccatggttc ctcgcatgat tgtcaccccc cagagcaatc     1980
ggcctgtcat gggtattgtg caggacacac tcacagcagt gcgcaaattc accaagagag     2040
acgtcttcct ggagcggggt gaagtgatga acctcctgat gttcctgtcg acgtgggatg     2100
ggaaggtccc acagccggcc atcctaaagc ccggcccct gtggacaggc aagcaaatct      2160
```

tctccctcat catacctggt cacatcaatt gtatccgtac ccacagcacc catcccgatg 2220
atgaagacag tggcccttac aagcacatct ctcctgggga caccaaggtg gtggtggaga 2280
atggggagct gatcatgggc atcctgtgta agaagtctct gggcacgtca gctggctccc 2340
tggtccacat ctcctaccta gagatgggtc atgacatcac tcgcctcttc tactccaaca 2400
ttcagactgt cattaacaac tggctcctca tcgagggtca tactattggc attggggact 2460
ccattgctga ttctaagact taccaggaca ttcagaacac tattaagaag gccaagcagg 2520
acgtaataga ggtcatcgag aaggcacaca acaatgagct ggagcccacc ccagggaaca 2580
ctctgcggca gacgtttgag aatcaggtga accgcattct taacgatgcc cgagacaaga 2640
ctggctcctc tgctcagaaa tccctgtctg aatacaacaa cttcaagtct atggtcgtgt 2700
ccggagctaa aggttccaag attaacatct cccaggtcat tgctgtcgtt ggacagcaga 2760
acgtcgaggg caagcggatt ccatttggct tcaagcaccg gactctgcct cacttcatca 2820
aggatgacta cgggcctgag agccgtggct ttgtggagaa ctcctaccta gccggcctca 2880
cacccactga gttctttttc cacgccatgg ggggtcgtga ggggctcatt gacacggctg 2940
tcaagactgc tgagactgga tacatccagc ggcggctgat caagtccatg gagtcagtga 3000
tggtgaagta cgacgcgact gtgcggaact ccatcaacca ggtggtgcag ctgcgctacg 3060
gcgaagacgg cctggcaggc gagagcgttg agttccagaa cctggctacg cttaagcctt 3120
ccaacaaggc ttttgagaag aagttccgct ttgattatac caatgagagg gccctgcggc 3180
gcactctgca ggaggacctg gtgaaggacg tgctgagcaa cgcacacatc cagaacgagt 3240
tggagcggga atttgagcgg atgcgggagg atcgggaggt gctcagggtc atcttcccaa 3300
ctggagacag caaggtcgtc ctcccctgta acctgctgcg gatgatctgg aatgctcaga 3360
aaatcttcca catcaaccca cgccttccct ccgacctgca ccccatcaaa gtggtggagg 3420
gagtcaagga attgagcaag aagctggtga ttgtgaatgg ggatgaccca ctaagtcgac 3480
aggcccagga aaatgccacg ctgctcttca acatccacct gcggtccacg ttgtgttccc 3540
gccgcatggc agaggagttt cggctcagtg gggaggcctt cgactggctg cttggggaga 3600
ttgagtccaa gttcaaccaa gccattgcgc atcccgggga aatggtgggg gctctggctg 3660
cgcagtccct tggagaacct gccacccaga tgaccttgaa taccttccac tatgctggtg 3720
tgtctgccaa gaatgtgacg ctgggtgtgc cccgacttaa ggagctcatc aacatttcca 3780
agaagccaaa gactccttcg cttactgtct tcctgttggg ccagtccgct cgagatgctg 3840
agagagccaa ggatattctg tgccgtctgg agcatacaac gttgaggaag gtgactgcca 3900
acacagccat ctactatgac cccaaccccc agagcacggt ggtggcagag gatcaggaat 3960
gggtgaatgt ctactatgaa atgcctgact ttgatgtggc ccgaatctcc ccctggctgt 4020
tgcgggtgga gctggatcgg aagcacatga ctgaccggaa gctcaccatg gagcagattg 4080

ctgaaaagat caatgctggt tttggtgacg acttgaactg catctttaat gatgacaatg 4140

cagagaagct ggtgctccgt attcgcatca tgaacagcga tgagaacaag atgcaagagg 4200

aggaagaggt ggtggacaag atggatgatg atgtcttcct gcgctgcatc gagtccaaca 4260

tgctgacaga tatgaccctg cagggcatcg agcagatcag caaggtgtac atgcacttgc 4320

cacagacaga caacaagaag aagatcatca tcacggagga tggggaattc aaggccctgc 4380

aggagtggat cctggagacg gacggcgtga gcttgatgcg ggtgctgagt gagaaggacg 4440

tggaccccgt acgcaccacg tccaatgaca ttgtggagat cttcacggtg ctgggcattg 4500

aagccgtgcg gaaggccctg gagcgggagc tgtaccacgt catctccttt gatggctcct 4560

atgtcaatta ccgacacttg gctctcttgt gtgataccat gacctgtcgt ggccacttga 4620

tggccatcac ccgacacgga gtcaaccgcc aggacacagg accactcatg aagtgttcct 4680

ttgaggaaac ggtggacgtg cttatggaag cagccgcaca cggtgagagt gaccccatga 4740

aggggggtctc tgagaatatc atgctgggcc agctggctcc ggccggcact ggctgctttg 4800

acctcctgct tgatgcagag aagtgcaagt atggcatgga gatccccacc aatatccccg 4860

gcctgggggc tgctggaccc accggcatgt tctttggttc agcacccagt cccatgggtg 4920

gaatctctcc tgccatgaca ccttggaacc agggtgcaac ccctgcctat ggcgcctggt 4980

cccccagtgt tgggagtgga atgaccccag gggcagccgg cttctctccc agtgctgcgt 5040

cagatgccag cggcttcagc ccaggttact cccctgcctg gtctcccaca ccgggctccc 5100

cggggtcccc aggtccctca agcccctaca tcccttcacc aggtggtgcc atgtctccca 5160

gctactcgcc aacgtcacct gcctacgagc cccgctctcc tggggggctac acaccccaga 5220

gtccctctta ttcccccact tcaccctcct actcccctac ctctccatcc tattctccaa 5280

ccagtcccaa ctatagtccc acatcaccca gctattcgcc aacgtcaccc agctactcac 5340

cgacctctcc cagctactca cccacctctc ccagctactc gcccacctct cccagctact 5400

cgcccacctc tcccagctac tcacccactt cccctagcta ctcgcccact tcccctagct 5460

actcgccaac gtctcccagc tactcgccga catctcccag ctactcgcca acttcaccca 5520

gctattctcc cacttctccc agctactcac ctacctctcc aagctattca cccacctccc 5580

ccagctactc acccacttcc ccaagttact cacccaccag cccgaactat tctccaacca 5640

gtcccaatta caccccaaca tcacccagct acagcccgac atcacccagc tattcaccta 5700

ctagtcccaa ctacacacct accagcccta actacagccc aacctctcca agctactctc 5760

caacatcacc cagctattcc ccgacctcac caagttactc cccttccagc ccacgataca 5820

caccacagtc tccaacctat accccaagct cacccagcta cagccccagc tcgcccagct 5880

acagcccaac ctcacccaag tacaccccaa ccagtccttc ttacagtccc agctccccag 5940

```
agtatacccc aacctctccc aagtactcac ctaccagtcc caaatattca cccacctctc      6000
ccaagtactc gcctaccagt cccacctatt cacccaccac cccaaaatac tccccaacat      6060
ctcctactta ttccccaacc tctccagtct acaccccaac ctctcccaag tactcaccta      6120
ctagccccac ttactcgccc acttccccca agtactcgcc caccagcccc acctactcgc      6180
ccacctcccc caaaggctca acctactctc ccacttcccc tggttactcg cccaccagcc      6240
ccacctacag tctcacaagc ccggctatca gcccggatga cagtgacgag gagaactgag      6300
ggcacgtggg gtgcggcagc gggctagggc ccagggcagc ttgcccgtgc tgctgtgcag      6360
ttcttgcctc cctcacgggg cgtcaccccc agcccagctc cgttgtacat aaatgccttg      6420
tggcagagct cccggtgaac ttctggatcc cgtttctgat gcagactctt gtcttgttct      6480
ccacttgtgc tgttagaact cactggccca gtggtgttct cactcctacc ccacccaccc      6540
cctgcctgtc cccaaattga agatccttcc ttgcctgtgg cttgatgcgg ggcgggtaaa      6600
gggtatttta acttaggggt agttcctgct gtgagtggtt acagctgatc ctcgggaaga      6660
acaaagctaa agctgccttt tgtctgttat tttatttttt tgaagtttaa ataaagttta      6720
ctaattttga ccaaaagt                                                    6738
```

<210> 160
<211> 987
<212> DNA
<213> Homo sapiens

<400> 160

```
aatataagtg gaggcgtcgc gctggcgggc attcctgaag ctgacagcat tcgggccgag      60
atgtctcgct ccgtggcctt agctgtgctc gcgctactct ctctttctgg cctggaggct     120
atccagcgta ctccaaagat tcaggtttac tcacgtcatc cagcagagaa tggaaagtca     180
aatttcctga attgctatgt gtctgggttt catccatccg acattgaagt tgacttactg     240
aagaatggag agagaattga aaaagtggag cattcagact tgtctttcag caaggactgg     300
tctttctatc tcttgtacta cactgaattc acccccactg aaaaagatga gtatgcctgc     360
cgtgtgaacc atgtgacttt gtcacagccc aagatagtta agtgggatcg agacatgtaa     420
gcagcatcat ggaggtttga agatgccgca tttggattgg atgaattcca aattctgctt     480
gcttgctttt taatattgat atgcttatac acttacactt tatgcacaaa atgtagggtt     540
ataataatgt taacatggac atgatcttct ttataattct actttgagtg ctgtctccat     600
gtttgatgta tctgagcagg ttgctccaca ggtagctcta ggagggctgg caacttagag     660
gtggggagca gagaattctc ttatccaaca tcaacatctt ggtcagattt gaactcttca     720
atctcttgca ctcaaagctt gttaagatag ttaagcgtgc ataagttaac ttccaattta     780
catactctgc ttagaatttg ggggaaaatt tagaaatata attgacagga ttattggaaa     840
tttgttataa tgaatgaaac attttgtcat ataagattca tatttacttc ttatacattt     900
gataaagtaa ggcatggttg tggttaatct ggtttatttt tgttccacaa gttaaataaa     960
tcataaaact tgatgtgtta tctctta                                         987
```

<210> 161
<211> 5416
<212> DNA
<213> Homo sapiens

<400> 161

agtgggccgc catgttgtcg gagtgaaagg taagggggag cgagagcgcc agagagagaa 60
gatcgggggg ctgaaatcca tcttcatcct accgctccgc ccgtgttggt ggaatgagcg 120
ttgcatgtgt cttgaagaga aaagcagtgc tttggcagga ctctttcagc ccccacctga 180
aacatcaccc tcaagaacca gctaatccca acatgcctgt tgttttgaca tctggaacag 240
ggtcgcaagc gcagccacaa ccagctgcaa atcaggctct tgcagctggg actcactcca 300
gccctgtccc aggatctata ggagttgcag gccgttccca ggacgacgct atggtggact 360
acttctttca gaggcagcat ggtgagcagc ttgggggagg aggaagtgga ggaggcggct 420
ataataatag caaacatcga tggcctactg ggataacat tcatgcagaa catcaggtgc 480
gttccatgga tgaactgaat catgattttc aagcacttgc tctggaggga agagcgatgg 540
gagagcagct cttgccaggt aaaaagtttt gggaaacaga tgaatccagc aaagatggac 600
caaaaggaat attcctgggt gatcaatggc gagacagtgc ctggggaaca tcagatcatt 660
cagtttccca gccaatcatg gtgcagagaa gacctggtca gagtttccat gtgaacagtg 720
aggtcaattc tgtactgtcc ccacgatcgg agagtggggg actaggcgtt agcatggtgg 780
agtatgtgtt gagctcatcc ccgggcgatt cctgtctaag aaaaggagga tttggcccaa 840
gggatgcaga cagtgatgaa aacgacaaag gtgaaaagaa gaacaagggt acgtttgatg 900
gagataagct aggagatttg aaggaggagg gtgatgtgat ggacaagacc aatggtttac 960
cagtgcagaa tgggattgat gcagacgtca aagattttag ccgtacccct ggtaattgcc 1020
agaactctgc taatgaagtg gatcttctgg gtccaaacca gaatggttct gagggcttag 1080
cccagctgac cagcaccaat ggtgccaagc ctgtggagga tttctccaac atggagtccc 1140
agagtgtccc cttggacccc atggaacatg tgggcatgga gcctcttcag tttgattatt 1200
caggcacgca ggtacctgtg gactcagcag cagcaactgt gggacttttt gactacaatt 1260
ctcaacaaca gctgttccaa agacctaatg cgcttgctgt ccagcagttg acagctgctc 1320
agcagcagca gtatgcactg gcagctgctc atcagccgca catcggttta gctcccgctg 1380
cgtttgtccc caatccatac atcatcagcg ctgctccccc agggacggac ccctacacag 1440
ctggattggc tgcagcagcg acactaggcc cagctgtggt ccctcaccag tattatggag 1500

ttactccctg gggagtctac cctgccagtc ttttccagca gcaagctgcc gctgccgctg 1560

cagcaactaa ttcagctaat caacagacca ccccacaggc tcagcaagga cagcagcagg 1620

ttctccgtgg aggagccagc caacgtcctt tgaccccaaa ccagaaccag cagggacagc 1680

aaacggatcc ccttgtggca gctgcagcag tgaattctgc ccttgcattt ggacaaggtc 1740

tggcagcagg catgccaggt tatccggtgt tggctcctgc tgcttactat gaccaaactg 1800

gtgcccttgt agtgaatgca ggcgcgagaa atggtcttgg agctcctgtt cgacttgtag 1860

ctcctgcccc agtcatcatt agttcctcag ctgcacaagc agctgttgca gcagccgcag 1920

cttcagcaaa tggagcagct ggtggtcttg ctggaacaac aaatggacca tttcgccctt 1980

taggaacaca gcagcctcag ccccagcccc agcagcagcc caataacaac ctggcatcca 2040

gttctttcta cggcaacaac tctctgaaca gcaattcaca gagcagctcc ctcttctccc 2100

agggctctgc ccagcctgcc aacacatcct tgggattcgg aagtagcagt tctctcggcg 2160

ccaccctggg atccgcccctt ggagggtttg gaacagcagt tgcaaactcc aacactggca 2220

gtggctcccg ccgtgactcc ctgactggca gcagtgacct ttataagagg acatcgagca 2280

gcttgacccc cattggacac agtttttata acggccttag cttttcctcc tctcctggac 2340

ccgtgggcat gcctctccct agtcagggac caggacattc acagacacca cctccttccc 2400

tctcttcaca tggatcctct tcaagcttaa acctgggagg actcacgaat ggcagtggaa 2460

gatacatctc tgctgctcca ggcgctgaag ccaagtaccg cagtgcaagc agcgcctcca 2520

gcctcttcag cccgagcagc actcttttct cttcctctcg tttgcgatat ggaatgtctg 2580

atgtcatgcc ttctggcagg agcaggcttt tggaagattt tcgaaacaac cggtacccca 2640

atttacaact gcgggagatt gctggacata taatggaatt ttcccaagac cagcatgggt 2700

ccagattcat tcagctgaaa ctggagcgtg ccacaccagc tgagcgccag cttgtcttca 2760

atgaaatcct ccaggctgcc taccaactca tggtggatgt gtttggtaat tacgtcattc 2820

agaagttctt tgaatttggc agtcttgaac agaagctggc tttggcagaa cggattcgag 2880

gccacgtcct gtcattggca ctacagatgt atggctgccg tgttatccag aaagctcttg 2940

agtttattcc ttcagaccag caggtaatta atgagatggt tcgggaacta gatggccatg 3000

tcttgaagtg tgtgaaagat cagaatggca atcacgtggt tcagaaatgc attgaatgtg 3060

tacagcccca gtctttgcaa tttatcatcg atgcgtttaa gggacaggta tttgccttat 3120

ccacacatcc ttatggctgc cgagtgattc agagaatcct ggagcactgt ctccctgacc 3180

agacactccc tattttagag gagcttcacc agcacacaga gcagcttgta caggatcaat 3240

atggaaatta tgtaatccaa catgtactgg agcacggtcg tcctgaggat aaaagcaaaa 3300

ttgtagcaga aatccgaggc aatgtacttg tattgagtca gcacaaattt gcaagcaatg 3360

ttgtggagaa gtgtgttact cacgcctcac gtacggagcg cgctgtgctc atcgatgagg 3420

tgtgcaccat gaacgacggt ccccacagtg ccttatacac catgatgaag gaccagtatg 3480
ccaactacgt ggtccagaag atgattgacg tggcggagcc aggccagcgg aagatcgtca 3540
tgcataagat ccggccccac atcgcaactc ttcgtaagta cacctatggc aagcacattc 3600
tggccaagct ggagaagtac tacatgaaga acggtgttga cttagggccc atctgtggcc 3660
cccctaatgg tatcatctga ggcagtgtca cccgctgttc cctcattccc gctgacctca 3720
ctggcccact ggcaaatcca accagcaacc agaaatgttc tagtgtagag tctgagacgg 3780
gcaagtggtt gctccaggat tactccctcc tccaaaaaag gaatcaaatc cacgagtgga 3840
aaagcctttg taaatttaat tttattacac ataacatgta ctattttttt taattgacta 3900
attgccctgc tgttttactg gtgtatagga tacttgtaca taggtaacca atgtacatgg 3960
gaggccacat attttgttca ctgttgtatc tatatttcac atgtggaaac tttcagggtg 4020
gttggtttaa caaaaaaaaa aagctttaaa aaaaaaagaa aaaaaggaaa aggtttttag 4080
ctcatttgcc tggccggcaa gttttgcaaa tagctcttcc ccacctcctc attttagtaa 4140
aaaacaaaca aaaacaaaaa aacctgagaa gtttgaattg tagttaaatg accccaaact 4200
ggcatttaac actgtttata aaaaatatat atatatatat atatatatat aatgaaaaag 4260
gtttcagagt tgctaaagct tcagtttgtg acattaagtt tatgaaattc taaaaaatgc 4320
ctttttttgga gactatatta tgctgaagaa ggctgttcgt gaggaggaga tgcgagcacc 4380
cagaacgtct tttgaggctg ggcgggtgtg attgtttact gcctactgga tttttttcta 4440
ttaacattga aaggtaaaat ctgattattt agcatgagaa aaaaaaatcc aactctgctt 4500
ttggtcttgc ttctataaat atatagtgta tacttggtgt agactttgca tatatacaaa 4560
tttgtagtat tttcttgttt tgatgtctaa tctgtatcta taatgtaccc tagtagtcga 4620
acatactttt gattgtacaa ttgtacattt gtatacctgt aatgtaaatg tggagaagtt 4680
tgaatcaaca taaacacgtt ttttggtaag aaaagagaat tagccagccc tgtgcattca 4740
gtgtatattc tcacctttta tggtcgtagc atatagtgtt gtatattgta aattgtaatt 4800
tcaaccagaa gtaaattttt ttcttttgaa ggaataaatg ttctttatac agcctagtta 4860
atgtttaaaa agaaaaaaat agcttggttt tatttgtcat ctagtctcaa gtatagcgag 4920
attctttcta aatgttattc aagattgagt tctcactagt gtttttttaa tcctaaaaaa 4980
gtaatgtttt gattttgtga cagtcaaaag gacgtgcaaa agtctagcct tgcccgagct 5040
ttccttacaa tcagagcccc tctcaccttg taaagtgtga atcgcccttc ccttttgtac 5100
agaagatgaa ctgtattttg cattttgtct acttgtaagt gaatgtaaca tactgtcaat 5160
tttccttgtt tgaatataga attgtaacac tacacggtgt acatttccag agccttgtgt 5220
atatttccaa tgaacttttt tgcaagcaca cttgtaacca tatgtgtata attaacaaac 5280

ctgtgtatgc ttatgcctgg gcaactattt tttgtaactc ttgtgtagat tgtctctaaa 5340

caatgtgtga tctttatttt gaaaaataca gaactttgga atctgaaaaa aaaaaaaaaa 5400

aaaaaaaaaa aaaaaa 5416

<210> 162
<211> 5410
<212> DNA
<213> Homo sapiens

<400> 162

agtgggccgc catgttgtcg gagtgaaagg taagggggag cgagagcgcc agagagagaa 60
gatcgggggg ctgaaatcca tcttcatcct accgctccgc ccgtgttggt ggaatgagcg 120
ttgcatgtgt cttgaagaga aaagcagtgc tttggcagga ctctttcagc ccccacctga 180
aacatcaccc tcaagaacca gctaatccca acatgcctgt tgttttgaca tctggaacag 240
ggtcgcaagc gcagccacaa ccagctgcaa atcaggctct tgcagctggg actcactcca 300
gccctgtccc aggatctata ggagttgcag gccgttccca ggacgacgct atggtggact 360
acttctttca gaggcagcat ggtgagcagc ttgggggagg aggaagtgga ggaggcggct 420
ataataatag caaacatcga tggcctactg ggataacat tcatgcagaa catcaggtgc 480
gttccatgga tgaactgaat catgattttc aagcacttgc tctggaggga agagcgatgg 540
gagagcagct cttgccaggt aaaaagtttt gggaaacaga tgaatccagc aaagatggac 600
caaaaggaat attcctgggt gatcaatggc gagacagtgc ctggggaaca tcagatcatt 660
cagtttccca gccaatcatg gtgcagagaa gacctggtca gagtttccat gtgaacagtg 720
aggtcaattc tgtactgtcc ccacgatcgg agagtggggg actaggcgtt agcatggtgg 780
agtatgtgtt gagctcatcc ccgggcgatt cctgtctaag aaaaggagga tttggcccaa 840
gggatgcaga cagtgatgaa aacgacaaag gtgaaaagaa gaacaagggt acgtttgatg 900
gagataagct aggagatttg aaggaggagg gtgatgtgat ggacaagacc aatggtttac 960
cagtgcagaa tgggattgat gcagacgtca aagattttag ccgtacccct ggtaattgcc 1020
agaactctgc taatgaagtg gatcttctgg gtccaaacca gaatggttct gagggcttag 1080
cccagctgac cagcaccaat ggtgccaagc ctgtggagga tttctccaac atggagtccc 1140
agagtgtccc cttggacccc atggaacatg tgggcatgga gcctcttcag tttgattatt 1200
caggcacgca ggtacctgtg gactcagcag cagcaactgt gggacttttt gactacaatt 1260
ctcaacaaca gctgttccaa agacctaatg cgcttgctgt ccagcagttg acagctgctc 1320
agcagcagca gtatgcactg gcagctgctc atcagccgca catcggttta gctcccgctg 1380
cgtttgtccc caatccatac atcatcagcg ctgctccccc agggacggac ccctacacag 1440
ctggattggc tgcagcagcg acactaggcc cagctgtggt ccctcaccag tattatggag 1500

ttactccctg gggagtctac cctgccagtc ttttccagca gcaagctgcc gctgccgctg 1560

cagcaactaa ttcagctaat caacagacca ccccacaggc tcagcaagga cagcagcagg 1620

ttctccgtgg aggagccagc caacgtcctt tgaccccaaa ccagaaccag cagggacagc 1680

aaacggatcc ccttgtggca gctgcagcag tgaattctgc ccttgcattt ggacaaggtc 1740

tggcagcagg catgccaggt tatccggtgt tggctcctgc tgcttactat gaccaaactg 1800

gtgcccttgt agtgaatgca ggcgcgagaa atggtcttgg agctcctgtt cgacttgtag 1860

ctcctgcccc agtcatcatt agttcctcag ctgcacaagc agctgttgca gcagccgcag 1920

cttcagcaaa tggagcagct ggtggtcttg ctggaacaac aaatggacca tttcgccctt 1980

taggaacaca gcagcctcag ccccagcccc agcagcagcc caataacaac ctggcatcca 2040

gttctttcta cggcaacaac tctctgaaca gcaattcaca gagcagctcc ctcttctccc 2100

agggctctgc ccagcctgcc aacacatcct tgggattcgg aagtagcagt tctctcggcg 2160

ccaccctggg atccgcccctt ggagggtttg gaacagcagt tgcaaactcc aacactggca 2220

gtggctcccg ccgtgactcc ctgactggca gcagtgacct ttataagagg acatcgagca 2280

gcttgacccc cattggacac agtttttata acggccttag cttttcctcc tctcctggac 2340

ccgtgggcat gcctctccct agtcagggac caggacattc acagacacca cctccttccc 2400

tctcttcaca tggatcctct tcaagcttaa acctgggagg actcacgaat ggcagtggaa 2460

gatacatctc tgctgctcca ggcgctgaag ccaagtaccg cagtgcaagc agcgcctcca 2520

gcctcttcag cccgagcagc actcttttct cttcctctcg tttgcgatat ggaatgtctg 2580

atgtcatgcc ttctggcagg agcaggcttt tggaagattt tcgaaacaac cggtacccca 2640

atttacaact gcgggagatt gctggacata taatggaatt ttcccaagac cagcatgggt 2700

ccagattcat tcagctgaaa ctggagcgtg ccacaccagc tgagcgccag cttgtcttca 2760

atgaaatcct ccaggctgcc taccaactca tggtggatgt gtttggtaat tacgtcattc 2820

agaagttctt tgaatttggc agtcttgaac agaagctggc tttggcagaa cggattcgag 2880

gccacgtcct gtcattggca ctacagatgt atggctgccg tgttatccag aaagctcttg 2940

agtttattcc ttcagaccag cagaatgaga tggttcggga actagatggc catgtcttga 3000

agtgtgtgaa agatcagaat ggcaatcacg tggttcagaa atgcattgaa tgtgtacagc 3060

cccagtcttt gcaatttatc atcgatgcgt ttaagggaca ggtatttgcc ttatccacac 3120

atccttatgg ctgccgagtg attcagagaa tcctggagca ctgtctccct gaccagacac 3180

tccctatttt agaggagctt caccagcaca cagagcagct tgtacaggat caatatggaa 3240

attatgtaat ccaacatgta ctggagcacg gtcgtcctga ggataaaagc aaaattgtag 3300

cagaaatccg aggcaatgta cttgtattga gtcagcacaa atttgcaagc aatgttgtgg 3360

agaagtgtgt tactcacgcc tcacgtacgg agcgcgctgt gctcatcgat gaggtgtgca 3420

ccatgaacga cggtccccac agtgccttat acaccatgat gaaggaccag tatgccaact 3480
acgtggtcca gaagatgatt gacgtggcgg agccaggcca gcggaagatc gtcatgcata 3540
agatccggcc ccacatcgca actcttcgta agtacaccta tggcaagcac attctggcca 3600
agctggagaa gtactacatg aagaacggtg ttgacttagg gcccatctgt ggccccccta 3660
atggtatcat ctgaggcagt gtcacccgct gttccctcat tcccgctgac ctcactggcc 3720
cactggcaaa tccaaccagc aaccagaaat gttctagtgt agagtctgag acgggcaagt 3780
ggttgctcca ggattactcc ctcctccaaa aaaggaatca aatccacgag tggaaaagcc 3840
tttgtaaatt taattttatt acacataaca tgtactattt tttttaattg actaattgcc 3900
ctgctgtttt actggtgtat aggatacttg tacataggta accaatgtac atgggaggcc 3960
acatattttg ttcactgttg tatctatatt tcacatgtgg aaactttcag ggtggttggt 4020
ttaacaaaaa aaaaaagctt taaaaaaaaa agaaaaaaag gaaaaggttt ttagctcatt 4080
tgcctggccg gcaagttttg caaatagctc ttccccacct cctcatttta gtaaaaaaca 4140
aacaaaaaca aaaaaacctg agaagtttga attgtagtta aatgacccca aactggcatt 4200
taacactgtt tataaaaaat atatatatat atatatatat atataatgaa aaaggtttca 4260
gagttgctaa agcttcagtt tgtgacatta agtttatgaa attctaaaaa atgccttttt 4320
tggagactat attatgctga agaaggctgt tcgtgaggag gagatgcgag cacccagaac 4380
gtcttttgag gctgggcggg tgtgattgtt tactgcctac tggatttttt tctattaaca 4440
ttgaaaggta aaatctgatt atttagcatg agaaaaaaaa atccaactct gcttttggtc 4500
ttgcttctat aaatatatag tgtatacttg gtgtagactt tgcatatata caaatttgta 4560
gtattttctt gttttgatgt ctaatctgta tctataatgt accctagtag tcgaacatac 4620
ttttgattgt acaattgtac atttgtatac ctgtaatgta aatgtggaga agtttgaatc 4680
aacataaaca cgtttttttgg taagaaaaga gaattagcca gccctgtgca ttcagtgtat 4740
attctcacct tttatggtcg tagcatatag tgttgtatat tgtaaattgt aatttcaacc 4800
agaagtaaat ttttttcttt tgaaggaata aatgttcttt atacagccta gttaatgttt 4860
aaaaagaaaa aaatagcttg gttttatttg tcatctagtc tcaagtatag cgagattctt 4920
tctaaatgtt attcaagatt gagttctcac tagtgttttt ttaatcctaa aaaagtaatg 4980
ttttgatttt gtgacagtca aaaggacgtg caaaagtcta gccttgcccg agctttcctt 5040
acaatcagag cccctctcac cttgtaaagt gtgaatcgcc cttccctttt gtacagaaga 5100
tgaactgtat tttgcatttt gtctacttgt aagtgaatgt aacatactgt caattttcct 5160
tgtttgaata tagaattgta acactacacg gtgtacattt ccagagcctt gtgtatattt 5220
ccaatgaact tttttgcaag cacacttgta accatatgtg tataattaac aaacctgtgt 5280

atgcttatgc ctgggcaact attttttgta actcttgtgt agattgtctc taaacaatgt 5340

gtgatcttta ttttgaaaaa tacagaactt tggaatctga aaaaaaaaaa aaaaaaaaaa 5400

aaaaaaaaaa 5410

<210> 163
<211> 1771
<212> DNA
<213> Homo sapiens

<400> 163

```
ggcggccagg ccgggcgcgg agtgggcgcg cggggccgga ggaggggcca gcgaccgcgg      60
caccgcctgt gcccgcccgc ccctccgcag ccgctactta agaggctcca gcgccggccc     120
cgccctagtg cgttacttac ctcgactctt agcttgtcgg ggacggtaac cgggacccgg     180
tgtctgctcc tgtcgccttc gcctcctaat ccctagccac tatgcgtgag tgcatctcca     240
tccacgttgg ccaggctggt gtccagattg gcaatgcctg ctgggagctc tactgcctgg     300
aacacggcat ccagcccgat ggccagatgc caagtgacaa gaccattggg ggaggagatg     360
actccttcaa caccttcttc agtgagacgg gcgctggcaa gcacgtgccc cgggctgtgt     420
ttgtagactt ggaacccaca gtcattgatg aagttcgcac tggcacctac cgccagctct     480
tccaccctga gcagctcatc acaggcaagg aagatgctgc caataactat gcccgagggc     540
actacaccat tggcaaggag atcattgacc ttgtgttgga ccgaattcgc aagctggctg     600
accagtgcac cggtcttcag ggcttcttgg ttttccacag ctttggtggg ggaactggtt     660
ctgggttcac ctccctgctc atggaacgtc tctcagttga ttatggcaag aagtccaagc     720
tggagttctc catttaccca gcaccccagg tttccacagc tgtagttgag ccctacaact     780
ccatcctcac cacccacacc accctggagc actctgattg tgccttcatg gtagacaatg     840
aggccatcta tgacatctgt cgtagaaacc tcgatatcga gcgcccaacc tacactaacc     900
ttaaccgcct tattagccag attgtgtcct ccatcactgc ttccctgaga tttgatggag     960
ccctgaatgt tgacctgaca gaattccaga ccaacctggt gccctacccc cgcatccact    1020
tccctctggc cacatatgcc cctgtcatct ctgctgagaa agcctaccat gaacagcttt    1080
ctgtagcaga gatcaccaat gcttgctttg agccagccaa ccagatggtg aaatgtgacc    1140
ctcgccatgg taaatacatg gcttgctgcc tgttgtaccg tggtgacgtg gttcccaaag    1200
atgtcaatgc tgccattgcc accatcaaaa ccaagcgcag catccagttt gtggattggt    1260
gccccactgg cttcaaggtt ggcatcaact accagcctcc cactgtggtg cctggtggag    1320
acctggccaa ggtacagaga gctgtgtgca tgctgagcaa caccacagcc attgctgagg    1380
cctgggctcg cctggaccac aagtttgacc tgatgtatgc caagcgtgcc tttgttcact    1440
ggtacgtggg tgaggggatg gaggaaggcg agttttcaga ggcccgtgaa gatatggctg    1500
cccttgagaa ggattatgag gaggttggtg tggattctgt tgaaggagag ggtgaggaag    1560
aaggagagga atactaatta tccattcctt ttggccctgc agcatgtcat gctcccagaa    1620
tttcagcttc agcttaactg acagacgtta aagctttctg gttagattgt tttcacttgg    1680
tgatcatgtc ttttccatgt gtacctgtaa tatttttcca tcatatctca aagtaaagtc    1740
attaacatca aaaaaaaaaa aaaaaaaaaa a                                   1771
```

<210> 164

<211> 2458
<212> DNA
<213> Homo sapiens

<400> 164

```
cagaagaagg cagcgcccaa ggcgcatgcg cagcggtcac tcccgctgta tattaaggcg      60
ccggcgatcg cggcctgagg ctgctcccgg acaagggcaa cgagcgtttc gtttggactt     120
ctcgacttga gtgcccgcct ccttcgccgc cgcctctgca gtcctcagcg cagttatgcc     180
cagttcttcc cgctgtgggg acacgaccac ggaggaatcc ttgcttcagg gactcgggac     240
cctgctggac cccttcctcg ggtttagggg atgtggggac caggagaaag tcaggatccc     300
taagagtctt ccctgcctgg atggatgagt ggcttcttct ccacctagat tctttccaca     360
ggagccagca tacttcctga acatggagag tgttgttcgc cgctgcccat tcttatcccg     420
agtcccccag gcctttctgc agaaagcagg caaatctctg ttgttctatg cccaaaactg     480
ccccaagatg atggaagttg gggccaagcc agcccctcgg gcattgtcca ctgcagcagt     540
acactaccaa cagatcaaag aaaccctcc ggccagtgag aaagacaaaa ctgctaaggc     600
caaggtccaa cagactcctg atggatccca gcagagtcca gatggcacac agcttccgtc     660
tggacacccc ttgcctgcca caagccaggg cactgcaagc aaatgccctt tcctggcagc     720
acagatgaat cagagaggca gcagtgtctt ctgcaaagcc agtcttgagc ttcaggagga     780
tgtgcaggaa atgaatgccg tgaggaaaga ggttgctgaa acctcagcag gccccagtgt     840
ggttagtgtg aaaaccgatg gaggggatcc cagtggactg ctgaagaact tccaggacat     900
catgcaaaag caaagaccag aaagagtgtc tcatcttctt caagataact tgccaaaatc     960
tgtttccact tttcagtatg atcgtttctt tgagaaaaaa attgatgaga aaaagaatga    1020
ccacacctat cgagttttta aaactgtgaa ccggcgagca cacatcttcc ccatggcaga    1080
tgactattca gactccctca tcaccaaaaa gcaagtgtca gtctggtgca gtaatgacta    1140
cctaggaatg agtcgccacc cacgggtgtg tggggcagtt atggacactt tgaaacaaca    1200
tggtgctggg gcaggtggta ctagaaatat ttctggaact agtaaattcc atgtggactt    1260
agagcgggag ctggcagacc tccatgggaa agatgccgca ctcttgtttt cctcgtgctt    1320
tgtggccaat gactcaaccc tcttcaccct ggctaagatg atgccaggct gtgagattta    1380
```

ctctgattct gggaaccatg cctccatgat ccaagggatt cgaaacagcc gagtgccaaa 1440

gtacatcttc cgccacaatg atgtcagcca cctcagagaa ctgctgcaaa gatctgaccc 1500

ctcagtcccc aagattgtgg catttgaaac tgtccattca atggatgggg cggtgtgccc 1560

actggaagag ctgtgtgatg tggcccatga gtttggagca atcaccttcg tggatgaggt 1620

ccacgcagtg gggctttatg gggctcgagg cggagggatt ggggatcggg atggagtcat 1680

gccaaaaatg gacatcattt ctggaacact tggcaaagcc tttggttgtg ttggagggta 1740

catcgccagc acgagttctc tgattgacac cgtacggtcc tatgctgctg gcttcatctt 1800

caccacctct ctgccaccca tgctgctggc tggagccctg gagtctgtgc ggatcctgaa 1860

gagcgctgag ggacgggtgc ttcgccgcca gcaccagcgc aacgtcaaac tcatgagaca 1920

gatgctaatg gatgccggcc tccctgttgt ccactgcccc agccacatca tccctgtgcg 1980

ggttgcagat gctgctaaaa acacagaagt ctgtgatgaa ctaatgagca gacataacat 2040

ctacgtgcaa gcaatcaatt accctacggt gccccgggga gaagagctcc tacggattgc 2100

ccccacccct caccacacac cccagatgat gaactacttc cttgagaatc tgctagtcac 2160

atggaagcaa gtggggctgg aactgaagcc tcattcctca gctgagtgca acttctgcag 2220

gaggccactg cattttgaag tgatgagtga aagagagaag tcctatttct caggcttgag 2280

caagttggta tctgctcagg cctgagcatg acctcaatta tttcacttaa ccccaggcca 2340

ttatcatatc cagatggtct tcagagttgt ctttatatgt gaattaagtt atattaaatt 2400

ttaatctata gtaaaaacat agtcctggaa ataaattctt gcttaaatgg tgaaaaaa 2458

<210> 165
<211> 2281
<212> DNA
<213> Homo sapiens

<400> 165

```
cagaagaagg cagcgcccaa ggcgcatgcg cagcggtcac tcccgctgta tattaaggcg      60
ccggcgatcg cggcctgagg ctgctcccgg acaagggcaa cgagcgtttc gtttggactt     120
ctcgacttga gtgcccgcct ccttcgccgc cgcctctgca gtcctcagcg cagtctttcc     180
acaggagcca gcatacttcc tgaacatgga gagtgttgtt cgccgctgcc cattcttatc     240
ccgagtcccc caggcctttc tgcagaaagc aggcaaatct ctgttgttct atgcccaaaa     300
ctgccccaag atgatggaag ttggggccaa gccagcccct cgggcattgt ccactgcagc     360
agtacactac caacagatca aagaaacccc tccggccagt gagaaagaca aaactgctaa     420
ggccaaggtc caacagactc ctgatggatc ccagcagagt ccagatggca cacagcttcc     480
gtctggacac cccttgcctg ccacaagcca gggcactgca agcaaatgcc ctttcctggc     540
agcacagatg aatcagagag gcagcagtgt cttctgcaaa gccagtcttg agcttcagga     600
```

ggatgtgcag gaaatgaatg ccgtgaggaa agaggttgct gaaacctcag caggccccag 660
tgtggttagt gtgaaaaccg atggagggga tcccagtgga ctgctgaaga acttccagga 720
catcatgcaa aagcaaagac cagaaagagt gtctcatctt cttcaagata acttgccaaa 780
atctgtttcc acttttcagt atgatcgttt ctttgagaaa aaaattgatg agaaaaagaa 840
tgaccacacc tatcgagttt ttaaaactgt gaaccggcga gcacacatct tccccatggc 900
agatgactat tcagactccc tcatcaccaa aaagcaagtg tcagtctggt gcagtaatga 960
ctacctagga atgagtcgcc acccacgggt gtgtggggca gttatggaca ctttgaaaca 1020
acatggtgct ggggcaggtg gtactagaaa tatttctgga actagtaaat tccatgtgga 1080
cttagagcgg gagctggcag acctccatgg gaaagatgcc gcactcttgt tttcctcgtg 1140
ctttgtggcc aatgactcaa ccctcttcac cctggctaag atgatgccag gctgtgagat 1200
ttactctgat tctgggaacc atgcctccat gatccaaggg attcgaaaca gccgagtgcc 1260
aaagtacatc ttccgccaca atgatgtcag ccacctcaga gaactgctgc aaagatctga 1320
cccctcagtc cccaagattg tggcatttga aactgtccat tcaatggatg gggcggtgtg 1380
cccactggaa gagctgtgtg atgtggccca tgagtttgga gcaatcacct tcgtggatga 1440
ggtccacgca gtggggcttt atggggctcg aggcggaggg attggggatc gggatggagt 1500
catgccaaaa atggacatca tttctggaac acttggcaaa gcctttggtt gtgttggagg 1560
gtacatcgcc agcacgagtt ctctgattga caccgtacgg tcctatgctg ctggcttcat 1620
cttcaccacc tctctgccac ccatgctgct ggctggagcc ctggagtctg tgcggatcct 1680
gaagagcgct gagggacggg tgcttcgccg ccagcaccag cgcaacgtca aactcatgag 1740
acagatgcta atggatgccg gcctccctgt tgtccactgc cccagccaca tcatccctgt 1800
gcgggttgca gatgctgcta aaaacacaga agtctgtgat gaactaatga gcagacataa 1860
catctacgtg caagcaatca attaccctac ggtgccccgg ggagaagagc tcctacggat 1920
tgcccccacc cctcaccaca caccccagat gatgaactac ttccttgaga atctgctagt 1980
cacatggaag caagtggggc tggaactgaa gcctcattcc tcagctgagt gcaacttctg 2040
caggaggcca ctgcattttg aagtgatgag tgaaagagag aagtcctatt tctcaggctt 2100
gagcaagttg gtatctgctc aggcctgagc atgacctcaa ttatttcact taaccccagg 2160
ccattatcat atccagatgg tcttcagagt tgtctttata tgtgaattaa gttatattaa 2220
attttaatct atagtaaaaa catagtcctg gaaataaatt cttgcttaaa tggtgaaaaa 2280
a 2281

<210> 166
<211> 339
<212> PRT

<213> Homo sapiens

<400> 166

```
Met Asp Gln Asn Asn Ser Leu Pro Pro Tyr Ala Gln Gly Leu Ala Ser
1               5                   10                  15

Pro Gln Gly Ala Met Thr Pro Gly Ile Pro Ile Phe Ser Pro Met Met
            20                  25                  30

Pro Tyr Gly Thr Gly Leu Thr Pro Gln Pro Ile Gln Asn Thr Asn Ser
        35                  40                  45

Leu Ser Ile Leu Glu Glu Gln Gln Arg Gln Gln Gln Gln Gln Gln Gln
    50                  55                  60

Gln Gln Gln Gln Gln Gln Gln Gln Gln Gln Gln Gln Gln Gln Gln Gln
65                  70                  75                  80

Gln Gln Gln Gln Gln Gln Gln Gln Gln Gln Gln Gln Gln Gln Gln Ala
                85                  90                  95

Val Ala Ala Ala Ala Val Gln Gln Ser Thr Ser Gln Gln Ala Thr Gln
            100                 105                 110

Gly Thr Ser Gly Gln Ala Pro Gln Leu Phe His Ser Gln Thr Leu Thr
        115                 120                 125

Thr Ala Pro Leu Pro Gly Thr Thr Pro Leu Tyr Pro Ser Pro Met Thr
    130                 135                 140

Pro Met Thr Pro Ile Thr Pro Ala Thr Pro Ala Ser Glu Ser Ser Gly
145                 150                 155                 160

Ile Val Pro Gln Leu Gln Asn Ile Val Ser Thr Val Asn Leu Gly Cys
                165                 170                 175

Lys Leu Asp Leu Lys Thr Ile Ala Leu Arg Ala Arg Asn Ala Glu Tyr
            180                 185                 190

Asn Pro Lys Arg Phe Ala Ala Val Ile Met Arg Ile Arg Glu Pro Arg
        195                 200                 205

Thr Thr Ala Leu Ile Phe Ser Ser Gly Lys Met Val Cys Thr Gly Ala
    210                 215                 220

Lys Ser Glu Glu Gln Ser Arg Leu Ala Ala Arg Lys Tyr Ala Arg Val
225                 230                 235                 240
```

Val Gln Lys Leu Gly Phe Pro Ala Lys Phe Leu Asp Phe Lys Ile Gln
245 250 255

Asn Met Val Gly Ser Cys Asp Val Lys Phe Pro Ile Arg Leu Glu Gly
260 265 270

Leu Val Leu Thr His Gln Gln Phe Ser Ser Tyr Glu Pro Glu Leu Phe
275 280 285

Pro Gly Leu Ile Tyr Arg Met Ile Lys Pro Arg Ile Val Leu Leu Ile
290 295 300

Phe Val Ser Gly Lys Val Val Leu Thr Gly Ala Lys Val Arg Ala Glu
305 310 315 320

Ile Tyr Glu Ala Phe Glu Asn Ile Tyr Pro Ile Leu Lys Gly Phe Arg
325 330 335

Lys Thr Thr

<210> 167
<211> 218
<212> PRT
<213> Homo sapiens

<400> 167

Met Ala Thr Arg Ser Pro Gly Val Val Ile Ser Asp Asp Glu Pro Gly
1 5 10 15

Tyr Asp Leu Asp Leu Phe Cys Ile Pro Asn His Tyr Ala Glu Asp Leu
20 25 30

Glu Arg Val Phe Ile Pro His Gly Leu Ile Met Asp Arg Thr Glu Arg
35 40 45

Leu Ala Arg Asp Val Met Lys Glu Met Gly Gly His His Ile Val Ala
50 55 60

Leu Cys Val Leu Lys Gly Gly Tyr Lys Phe Phe Ala Asp Leu Leu Asp
65 70 75 80

Tyr Ile Lys Ala Leu Asn Arg Asn Ser Asp Arg Ser Ile Pro Met Thr
85 90 95

Val Asp Phe Ile Arg Leu Lys Ser Tyr Cys Asn Asp Gln Ser Thr Gly
100 105 110

Asp Ile Lys Val Ile Gly Gly Asp Asp Leu Ser Thr Leu Thr Gly Lys
115 120 125

Asn Val Leu Ile Val Glu Asp Ile Ile Asp Thr Gly Lys Thr Met Gln
130 135 140

Thr Leu Leu Ser Leu Val Arg Gln Tyr Asn Pro Lys Met Val Lys Val
145 150 155 160

Ala Ser Leu Leu Val Lys Arg Thr Pro Arg Ser Val Gly Tyr Lys Pro
165 170 175

Asp Phe Val Gly Phe Glu Ile Pro Asp Lys Phe Val Val Gly Tyr Ala
180 185 190

Leu Asp Tyr Asn Glu Tyr Phe Arg Asp Leu Asn His Val Cys Val Ile
195 200 205

Ser Glu Thr Gly Lys Ala Lys Tyr Lys Ala
210 215

<210> 168
<211> 375
<212> PRT
<213> Homo sapiens

<400> 168

Met Asp Asp Asp Ile Ala Ala Leu Val Val Asp Asn Gly Ser Gly Met
1 5 10 15

Cys Lys Ala Gly Phe Ala Gly Asp Asp Ala Pro Arg Ala Val Phe Pro
20 25 30

Ser Ile Val Gly Arg Pro Arg His Gln Gly Val Met Val Gly Met Gly
35 40 45

Gln Lys Asp Ser Tyr Val Gly Asp Glu Ala Gln Ser Lys Arg Gly Ile
50 55 60

Leu Thr Leu Lys Tyr Pro Ile Glu His Gly Ile Val Thr Asn Trp Asp
65 70 75 80

Asp Met Glu Lys Ile Trp His His Thr Phe Tyr Asn Glu Leu Arg Val
85 90 95

Ala Pro Glu Glu His Pro Val Leu Leu Thr Glu Ala Pro Leu Asn Pro
100 105 110

```
Lys Ala Asn Arg Glu Lys Met Thr Gln Ile Met Phe Glu Thr Phe Asn
        115                 120                 125

Thr Pro Ala Met Tyr Val Ala Ile Gln Ala Val Leu Ser Leu Tyr Ala
    130                 135                 140

Ser Gly Arg Thr Thr Gly Ile Val Met Asp Ser Gly Asp Gly Val Thr
145                 150                 155                 160

His Thr Val Pro Ile Tyr Glu Gly Tyr Ala Leu Pro His Ala Ile Leu
                165                 170                 175

Arg Leu Asp Leu Ala Gly Arg Asp Leu Thr Asp Tyr Leu Met Lys Ile
            180                 185                 190

Leu Thr Glu Arg Gly Tyr Ser Phe Thr Thr Thr Ala Glu Arg Glu Ile
        195                 200                 205

Val Arg Asp Ile Lys Glu Lys Leu Cys Tyr Val Ala Leu Asp Phe Glu
    210                 215                 220

Gln Glu Met Ala Thr Ala Ala Ser Ser Ser Ser Leu Glu Lys Ser Tyr
225                 230                 235                 240

Glu Leu Pro Asp Gly Gln Val Ile Thr Ile Gly Asn Glu Arg Phe Arg
                245                 250                 255

Cys Pro Glu Ala Leu Phe Gln Pro Ser Phe Leu Gly Met Glu Ser Cys
            260                 265                 270

Gly Ile His Glu Thr Thr Phe Asn Ser Ile Met Lys Cys Asp Val Asp
        275                 280                 285

Ile Arg Lys Asp Leu Tyr Ala Asn Thr Val Leu Ser Gly Gly Thr Thr
    290                 295                 300

Met Tyr Pro Gly Ile Ala Asp Arg Met Gln Lys Glu Ile Thr Ala Leu
305                 310                 315                 320

Ala Pro Ser Thr Met Lys Ile Lys Ile Ile Ala Pro Pro Glu Arg Lys
                325                 330                 335

Tyr Ser Val Trp Ile Gly Gly Ser Ile Leu Ala Ser Leu Ser Thr Phe
            340                 345                 350

Gln Gln Met Trp Ile Ser Lys Gln Glu Tyr Asp Glu Ser Gly Pro Ser
        355                 360                 365
```

Ile Val His Arg Lys Cys Phe
370 375

<210> 169
<211> 317
<212> PRT
<213> Homo sapiens

<400> 169

Met Pro Arg Glu Asp Arg Ala Thr Trp Lys Ser Asn Tyr Phe Leu Lys
1 5 10 15

Ile Ile Gln Leu Leu Asp Asp Tyr Pro Lys Cys Phe Ile Val Gly Ala
20 25 30

Asp Asn Val Gly Ser Lys Gln Met Gln Gln Ile Arg Met Ser Leu Arg
35 40 45

Gly Lys Ala Val Val Leu Met Gly Lys Asn Thr Met Met Arg Lys Ala
50 55 60

Ile Arg Gly His Leu Glu Asn Asn Pro Ala Leu Glu Lys Leu Leu Pro
65 70 75 80

His Ile Arg Gly Asn Val Gly Phe Val Phe Thr Lys Glu Asp Leu Thr
85 90 95

Glu Ile Arg Asp Met Leu Leu Ala Asn Lys Val Pro Ala Ala Ala Arg
100 105 110

Ala Gly Ala Ile Ala Pro Cys Glu Val Thr Val Pro Ala Gln Asn Thr
115 120 125

Gly Leu Gly Pro Glu Lys Thr Ser Phe Phe Gln Ala Leu Gly Ile Thr
130 135 140

Thr Lys Ile Ser Arg Gly Thr Ile Glu Ile Leu Ser Asp Val Gln Leu
145 150 155 160

Ile Lys Thr Gly Asp Lys Val Gly Ala Ser Glu Ala Thr Leu Leu Asn
165 170 175

Met Leu Asn Ile Ser Pro Phe Ser Phe Gly Leu Val Ile Gln Gln Val
180 185 190

Phe Asp Asn Gly Ser Ile Tyr Asn Pro Glu Val Leu Asp Ile Thr Glu
195 200 205

Glu Thr Leu His Ser Arg Phe Leu Glu Gly Val Arg Asn Val Ala Ser
210 215 220

Val Cys Leu Gln Ile Gly Tyr Pro Thr Val Ala Ser Val Pro His Ser
225 230 235 240

Ile Ile Asn Gly Tyr Lys Arg Val Leu Ala Leu Ser Val Glu Thr Asp
245 250 255

Tyr Thr Phe Pro Leu Ala Glu Lys Val Lys Ala Phe Leu Ala Asp Pro
260 265 270

Ser Ala Phe Val Ala Ala Ala Pro Val Ala Ala Ala Thr Thr Ala Ala
275 280 285

Pro Ala Ala Ala Ala Ala Pro Ala Lys Val Glu Ala Lys Glu Glu Ser
290 295 300

Glu Glu Ser Asp Glu Asp Met Gly Phe Gly Leu Phe Asp
305 310 315

<210> 170
<211> 317
<212> PRT
<213> Homo sapiens

<400> 170

Met Pro Arg Glu Asp Arg Ala Thr Trp Lys Ser Asn Tyr Phe Leu Lys
1 5 10 15

Ile Ile Gln Leu Leu Asp Asp Tyr Pro Lys Cys Phe Ile Val Gly Ala
20 25 30

Asp Asn Val Gly Ser Lys Gln Met Gln Gln Ile Arg Met Ser Leu Arg
35 40 45

Gly Lys Ala Val Val Leu Met Gly Lys Asn Thr Met Met Arg Lys Ala
50 55 60

Ile Arg Gly His Leu Glu Asn Asn Pro Ala Leu Glu Lys Leu Leu Pro
65 70 75 80

His Ile Arg Gly Asn Val Gly Phe Val Phe Thr Lys Glu Asp Leu Thr
85 90 95

Glu Ile Arg Asp Met Leu Leu Ala Asn Lys Val Pro Ala Ala Ala Arg
100 105 110

Ala Gly Ala Ile Ala Pro Cys Glu Val Thr Val Pro Ala Gln Asn Thr
115 120 125

Gly Leu Gly Pro Glu Lys Thr Ser Phe Phe Gln Ala Leu Gly Ile Thr
130 135 140

Thr Lys Ile Ser Arg Gly Thr Ile Glu Ile Leu Ser Asp Val Gln Leu
145 150 155 160

Ile Lys Thr Gly Asp Lys Val Gly Ala Ser Glu Ala Thr Leu Leu Asn
165 170 175

Met Leu Asn Ile Ser Pro Phe Ser Phe Gly Leu Val Ile Gln Gln Val
180 185 190

Phe Asp Asn Gly Ser Ile Tyr Asn Pro Glu Val Leu Asp Ile Thr Glu
195 200 205

Glu Thr Leu His Ser Arg Phe Leu Glu Gly Val Arg Asn Val Ala Ser
210 215 220

Val Cys Leu Gln Ile Gly Tyr Pro Thr Val Ala Ser Val Pro His Ser
225 230 235 240

Ile Ile Asn Gly Tyr Lys Arg Val Leu Ala Leu Ser Val Glu Thr Asp
245 250 255

Tyr Thr Phe Pro Leu Ala Glu Lys Val Lys Ala Phe Leu Ala Asp Pro
260 265 270

Ser Ala Phe Val Ala Ala Ala Pro Val Ala Ala Ala Thr Thr Ala Ala
275 280 285

Pro Ala Ala Ala Ala Ala Pro Ala Lys Val Glu Ala Lys Glu Glu Ser
290 295 300

Glu Glu Ser Asp Glu Asp Met Gly Phe Gly Leu Phe Asp
305 310 315

<210> 171
<211> 1970
<212> PRT
<213> Homo sapiens

<400> 171

Met His Gly Gly Gly Pro Pro Ser Gly Asp Ser Ala Cys Pro Leu Arg
1 5 10 15

```
Thr Ile Lys Arg Val Gln Phe Gly Val Leu Ser Pro Asp Glu Leu Lys
            20                  25                  30

Arg Met Ser Val Thr Glu Gly Gly Ile Lys Tyr Pro Glu Thr Thr Glu
        35                  40                  45

Gly Gly Arg Pro Lys Leu Gly Gly Leu Met Asp Pro Arg Gln Gly Val
    50                  55                  60

Ile Glu Arg Thr Gly Arg Cys Gln Thr Cys Ala Gly Asn Met Thr Glu
65                  70                  75                  80

Cys Pro Gly His Phe Gly His Ile Glu Leu Ala Lys Pro Val Phe His
                85                  90                  95

Val Gly Phe Leu Val Lys Thr Met Lys Val Leu Arg Cys Val Cys Phe
            100                 105                 110

Phe Cys Ser Lys Leu Leu Val Asp Ser Asn Asn Pro Lys Ile Lys Asp
        115                 120                 125

Ile Leu Ala Lys Ser Lys Gly Gln Pro Lys Lys Arg Leu Thr His Val
    130                 135                 140

Tyr Asp Leu Cys Lys Gly Lys Asn Ile Cys Glu Gly Gly Glu Glu Met
145                 150                 155                 160

Asp Asn Lys Phe Gly Val Glu Gln Pro Glu Gly Asp Glu Asp Leu Thr
                165                 170                 175

Lys Glu Lys Gly His Gly Gly Cys Gly Arg Tyr Gln Pro Arg Ile Arg
            180                 185                 190

Arg Ser Gly Leu Glu Leu Tyr Ala Glu Trp Lys His Val Asn Glu Asp
        195                 200                 205

Ser Gln Glu Lys Lys Ile Leu Leu Ser Pro Glu Arg Val His Glu Ile
    210                 215                 220

Phe Lys Arg Ile Ser Asp Glu Glu Cys Phe Val Leu Gly Met Glu Pro
225                 230                 235                 240

Arg Tyr Ala Arg Pro Glu Trp Met Ile Val Thr Val Leu Pro Val Pro
                245                 250                 255

Pro Leu Ser Val Arg Pro Ala Val Val Met Gln Gly Ser Ala Arg Asn
            260                 265                 270
```

Gln Asp Asp Leu Thr His Lys Leu Ala Asp Ile Val Lys Ile Asn Asn
275 280 285

Gln Leu Arg Arg Asn Glu Gln Asn Gly Ala Ala Ala His Val Ile Ala
290 295 300

Glu Asp Val Lys Leu Leu Gln Phe His Val Ala Thr Met Val Asp Asn
305 310 315 320

Glu Leu Pro Gly Leu Pro Arg Ala Met Gln Lys Ser Gly Arg Pro Leu
325 330 335

Lys Ser Leu Lys Gln Arg Leu Lys Gly Lys Glu Gly Arg Val Arg Gly
340 345 350

Asn Leu Met Gly Lys Arg Val Asp Phe Ser Ala Arg Thr Val Ile Thr
355 360 365

Pro Asp Pro Asn Leu Ser Ile Asp Gln Val Gly Val Pro Arg Ser Ile
370 375 380

Ala Ala Asn Met Thr Phe Ala Glu Ile Val Thr Pro Phe Asn Ile Asp
385 390 395 400

Arg Leu Gln Glu Leu Val Arg Arg Gly Asn Ser Gln Tyr Pro Gly Ala
405 410 415

Lys Tyr Ile Ile Arg Asp Asn Gly Asp Arg Ile Asp Leu Arg Phe His
420 425 430

Pro Lys Pro Ser Asp Leu His Leu Gln Thr Gly Tyr Lys Val Glu Arg
435 440 445

His Met Cys Asp Gly Asp Ile Val Ile Phe Asn Arg Gln Pro Thr Leu
450 455 460

His Lys Met Ser Met Met Gly His Arg Val Arg Ile Leu Pro Trp Ser
465 470 475 480

Thr Phe Arg Leu Asn Leu Ser Val Thr Thr Pro Tyr Asn Ala Asp Phe
485 490 495

Asp Gly Asp Glu Met Asn Leu His Leu Pro Gln Ser Leu Glu Thr Arg
500 505 510

Ala Glu Ile Gln Glu Leu Ala Met Val Pro Arg Met Ile Val Thr Pro

515 520 525

Gln Ser Asn Arg Pro Val Met Gly Ile Val Gln Asp Thr Leu Thr Ala
530 535 540

Val Arg Lys Phe Thr Lys Arg Asp Val Phe Leu Glu Arg Gly Glu Val
545 550 555 560

Met Asn Leu Leu Met Phe Leu Ser Thr Trp Asp Gly Lys Val Pro Gln
565 570 575

Pro Ala Ile Leu Lys Pro Arg Pro Leu Trp Thr Gly Lys Gln Ile Phe
580 585 590

Ser Leu Ile Ile Pro Gly His Ile Asn Cys Ile Arg Thr His Ser Thr
595 600 605

His Pro Asp Asp Glu Asp Ser Gly Pro Tyr Lys His Ile Ser Pro Gly
610 615 620

Asp Thr Lys Val Val Val Glu Asn Gly Glu Leu Ile Met Gly Ile Leu
625 630 635 640

Cys Lys Lys Ser Leu Gly Thr Ser Ala Gly Ser Leu Val His Ile Ser
645 650 655

Tyr Leu Glu Met Gly His Asp Ile Thr Arg Leu Phe Tyr Ser Asn Ile
660 665 670

Gln Thr Val Ile Asn Asn Trp Leu Leu Ile Glu Gly His Thr Ile Gly
675 680 685

Ile Gly Asp Ser Ile Ala Asp Ser Lys Thr Tyr Gln Asp Ile Gln Asn
690 695 700

Thr Ile Lys Lys Ala Lys Gln Asp Val Ile Glu Val Ile Glu Lys Ala
705 710 715 720

His Asn Asn Glu Leu Glu Pro Thr Pro Gly Asn Thr Leu Arg Gln Thr
725 730 735

Phe Glu Asn Gln Val Asn Arg Ile Leu Asn Asp Ala Arg Asp Lys Thr
740 745 750

Gly Ser Ser Ala Gln Lys Ser Leu Ser Glu Tyr Asn Asn Phe Lys Ser
755 760 765

```
Met Val Val Ser Gly Ala Lys Gly Ser Lys Ile Asn Ile Ser Gln Val
    770                 775                 780

Ile Ala Val Val Gly Gln Gln Asn Val Glu Gly Lys Arg Ile Pro Phe
785                 790                 795                 800

Gly Phe Lys His Arg Thr Leu Pro His Phe Ile Lys Asp Asp Tyr Gly
                805                 810                 815

Pro Glu Ser Arg Gly Phe Val Glu Asn Ser Tyr Leu Ala Gly Leu Thr
            820                 825                 830

Pro Thr Glu Phe Phe Phe His Ala Met Gly Gly Arg Glu Gly Leu Ile
        835                 840                 845

Asp Thr Ala Val Lys Thr Ala Glu Thr Gly Tyr Ile Gln Arg Arg Leu
    850                 855                 860

Ile Lys Ser Met Glu Ser Val Met Val Lys Tyr Asp Ala Thr Val Arg
865                 870                 875                 880

Asn Ser Ile Asn Gln Val Val Gln Leu Arg Tyr Gly Glu Asp Gly Leu
                885                 890                 895

Ala Gly Glu Ser Val Glu Phe Gln Asn Leu Ala Thr Leu Lys Pro Ser
            900                 905                 910

Asn Lys Ala Phe Glu Lys Lys Phe Arg Phe Asp Tyr Thr Asn Glu Arg
        915                 920                 925

Ala Leu Arg Arg Thr Leu Gln Glu Asp Leu Val Lys Asp Val Leu Ser
    930                 935                 940

Asn Ala His Ile Gln Asn Glu Leu Glu Arg Glu Phe Glu Arg Met Arg
945                 950                 955                 960

Glu Asp Arg Glu Val Leu Arg Val Ile Phe Pro Thr Gly Asp Ser Lys
                965                 970                 975

Val Val Leu Pro Cys Asn Leu Leu Arg Met Ile Trp Asn Ala Gln Lys
            980                 985                 990

Ile Phe His Ile Asn Pro Arg Leu  Pro Ser Asp Leu His  Pro Ile Lys
        995                 1000                 1005

Val Val  Glu Gly Val Lys Glu  Leu Ser Lys Lys Leu  Val Ile Val
    1010                 1015                 1020
```

```
Asn Gly  Asp Asp Pro Leu Ser  Arg Gln Ala Gln Glu  Asn Ala Thr
    1025                 1030                 1035

Leu Leu  Phe Asn Ile His Leu  Arg Ser Thr Leu Cys  Ser Arg Arg
    1040                 1045                 1050

Met Ala  Glu Glu Phe Arg Leu  Ser Gly Glu Ala Phe  Asp Trp Leu
    1055                 1060                 1065

Leu Gly  Glu Ile Glu Ser Lys  Phe Asn Gln Ala Ile  Ala His Pro
    1070                 1075                 1080

Gly Glu  Met Val Gly Ala Leu  Ala Ala Gln Ser Leu  Gly Glu Pro
    1085                 1090                 1095

Ala Thr  Gln Met Thr Leu Asn  Thr Phe His Tyr Ala  Gly Val Ser
    1100                 1105                 1110

Ala Lys  Asn Val Thr Leu Gly  Val Pro Arg Leu Lys  Glu Leu Ile
    1115                 1120                 1125

Asn Ile  Ser Lys Lys Pro Lys  Thr Pro Ser Leu Thr  Val Phe Leu
    1130                 1135                 1140

Leu Gly  Gln Ser Ala Arg Asp  Ala Glu Arg Ala Lys  Asp Ile Leu
    1145                 1150                 1155

Cys Arg  Leu Glu His Thr Thr  Leu Arg Lys Val Thr  Ala Asn Thr
    1160                 1165                 1170

Ala Ile  Tyr Tyr Asp Pro Asn  Pro Gln Ser Thr Val  Val Ala Glu
    1175                 1180                 1185

Asp Gln  Glu Trp Val Asn Val  Tyr Tyr Glu Met Pro  Asp Phe Asp
    1190                 1195                 1200

Val Ala  Arg Ile Ser Pro Trp  Leu Leu Arg Val Glu  Leu Asp Arg
    1205                 1210                 1215

Lys His  Met Thr Asp Arg Lys  Leu Thr Met Glu Gln  Ile Ala Glu
    1220                 1225                 1230

Lys Ile  Asn Ala Gly Phe Gly  Asp Asp Leu Asn Cys  Ile Phe Asn
    1235                 1240                 1245

Asp Asp  Asn Ala Glu Lys Leu  Val Leu Arg Ile Arg  Ile Met Asn
    1250                 1255                 1260
```

Ser Asp Glu Asn Lys Met Gln Glu Glu Glu Glu Val Val Asp Lys
    1265                1270                1275

Met Asp Asp Asp Val Phe Leu Arg Cys Ile Glu Ser Asn Met Leu
    1280                1285                1290

Thr Asp Met Thr Leu Gln Gly Ile Glu Gln Ile Ser Lys Val Tyr
    1295                1300                1305

Met His Leu Pro Gln Thr Asp Asn Lys Lys Lys Ile Ile Ile Thr
    1310                1315                1320

Glu Asp Gly Glu Phe Lys Ala Leu Gln Glu Trp Ile Leu Glu Thr
    1325                1330                1335

Asp Gly Val Ser Leu Met Arg Val Leu Ser Glu Lys Asp Val Asp
    1340                1345                1350

Pro Val Arg Thr Thr Ser Asn Asp Ile Val Glu Ile Phe Thr Val
    1355                1360                1365

Leu Gly Ile Glu Ala Val Arg Lys Ala Leu Glu Arg Glu Leu Tyr
    1370                1375                1380

His Val Ile Ser Phe Asp Gly Ser Tyr Val Asn Tyr Arg His Leu
    1385                1390                1395

Ala Leu Leu Cys Asp Thr Met Thr Cys Arg Gly His Leu Met Ala
    1400                1405                1410

Ile Thr Arg His Gly Val Asn Arg Gln Asp Thr Gly Pro Leu Met
    1415                1420                1425

Lys Cys Ser Phe Glu Glu Thr Val Asp Val Leu Met Glu Ala Ala
    1430                1435                1440

Ala His Gly Glu Ser Asp Pro Met Lys Gly Val Ser Glu Asn Ile
    1445                1450                1455

Met Leu Gly Gln Leu Ala Pro Ala Gly Thr Gly Cys Phe Asp Leu
    1460                1465                1470

Leu Leu Asp Ala Glu Lys Cys Lys Tyr Gly Met Glu Ile Pro Thr
    1475                1480                1485

Asn Ile Pro Gly Leu Gly Ala Ala Gly Pro Thr Gly Met Phe Phe

1490 1495 1500

Gly Ser Ala Pro Ser Pro Met Gly Gly Ile Ser Pro Ala Met Thr
1505 1510 1515

Pro Trp Asn Gln Gly Ala Thr Pro Ala Tyr Gly Ala Trp Ser Pro
1520 1525 1530

Ser Val Gly Ser Gly Met Thr Pro Gly Ala Ala Gly Phe Ser Pro
1535 1540 1545

Ser Ala Ala Ser Asp Ala Ser Gly Phe Ser Pro Gly Tyr Ser Pro
1550 1555 1560

Ala Trp Ser Pro Thr Pro Gly Ser Pro Gly Ser Pro Gly Pro Ser
1565 1570 1575

Ser Pro Tyr Ile Pro Ser Pro Gly Gly Ala Met Ser Pro Ser Tyr
1580 1585 1590

Ser Pro Thr Ser Pro Ala Tyr Glu Pro Arg Ser Pro Gly Gly Tyr
1595 1600 1605

Thr Pro Gln Ser Pro Ser Tyr Ser Pro Thr Ser Pro Ser Tyr Ser
1610 1615 1620

Pro Thr Ser Pro Ser Tyr Ser Pro Thr Ser Pro Asn Tyr Ser Pro
1625 1630 1635

Thr Ser Pro Ser Tyr Ser Pro Thr Ser Pro Ser Tyr Ser Pro Thr
1640 1645 1650

Ser Pro Ser Tyr Ser Pro Thr Ser Pro Ser Tyr Ser Pro Thr Ser
1655 1660 1665

Pro Ser Tyr Ser Pro Thr Ser Pro Ser Tyr Ser Pro Thr Ser Pro
1670 1675 1680

Ser Tyr Ser Pro Thr Ser Pro Ser Tyr Ser Pro Thr Ser Pro Ser
1685 1690 1695

Tyr Ser Pro Thr Ser Pro Ser Tyr Ser Pro Thr Ser Pro Ser Tyr
1700 1705 1710

Ser Pro Thr Ser Pro Ser Tyr Ser Pro Thr Ser Pro Ser Tyr Ser
1715 1720 1725

Pro Thr Ser Pro Ser Tyr Ser Pro Thr Ser Pro Ser Tyr Ser Pro
1730 1735 1740

Thr Ser Pro Asn Tyr Ser Pro Thr Ser Pro Asn Tyr Thr Pro Thr
1745 1750 1755

Ser Pro Ser Tyr Ser Pro Thr Ser Pro Ser Tyr Ser Pro Thr Ser
1760 1765 1770

Pro Asn Tyr Thr Pro Thr Ser Pro Asn Tyr Ser Pro Thr Ser Pro
1775 1780 1785

Ser Tyr Ser Pro Thr Ser Pro Ser Tyr Ser Pro Thr Ser Pro Ser
1790 1795 1800

Tyr Ser Pro Ser Ser Pro Arg Tyr Thr Pro Gln Ser Pro Thr Tyr
1805 1810 1815

Thr Pro Ser Ser Pro Ser Tyr Ser Pro Ser Ser Pro Ser Tyr Ser
1820 1825 1830

Pro Thr Ser Pro Lys Tyr Thr Pro Thr Ser Pro Ser Tyr Ser Pro
1835 1840 1845

Ser Ser Pro Glu Tyr Thr Pro Thr Ser Pro Lys Tyr Ser Pro Thr
1850 1855 1860

Ser Pro Lys Tyr Ser Pro Thr Ser Pro Lys Tyr Ser Pro Thr Ser
1865 1870 1875

Pro Thr Tyr Ser Pro Thr Thr Pro Lys Tyr Ser Pro Thr Ser Pro
1880 1885 1890

Thr Tyr Ser Pro Thr Ser Pro Val Tyr Thr Pro Thr Ser Pro Lys
1895 1900 1905

Tyr Ser Pro Thr Ser Pro Thr Tyr Ser Pro Thr Ser Pro Lys Tyr
1910 1915 1920

Ser Pro Thr Ser Pro Thr Tyr Ser Pro Thr Ser Pro Lys Gly Ser
1925 1930 1935

Thr Tyr Ser Pro Thr Ser Pro Gly Tyr Ser Pro Thr Ser Pro Thr
1940 1945 1950

Tyr Ser Leu Thr Ser Pro Ala Ile Ser Pro Asp Asp Ser Asp Glu
1955 1960 1965

```
Glu Asn
    1970
```

<210> 172
<211> 119
<212> PRT
<213> Homo sapiens

<400> 172

```
Met Ser Arg Ser Val Ala Leu Ala Val Leu Ala Leu Leu Ser Leu Ser
1               5                   10                  15

Gly Leu Glu Ala Ile Gln Arg Thr Pro Lys Ile Gln Val Tyr Ser Arg
            20                  25                  30

His Pro Ala Glu Asn Gly Lys Ser Asn Phe Leu Asn Cys Tyr Val Ser
        35                  40                  45

Gly Phe His Pro Ser Asp Ile Glu Val Asp Leu Leu Lys Asn Gly Glu
    50                  55                  60

Arg Ile Glu Lys Val Glu His Ser Asp Leu Ser Phe Ser Lys Asp Trp
65                  70                  75                  80

Ser Phe Tyr Leu Leu Tyr Tyr Thr Glu Phe Thr Pro Thr Glu Lys Asp
                85                  90                  95

Glu Tyr Ala Cys Arg Val Asn His Val Thr Leu Ser Gln Pro Lys Ile
            100                 105                 110

Val Lys Trp Asp Arg Asp Met
        115
```

<210> 173
<211> 1188
<212> PRT
<213> Homo sapiens

<400> 173

```
Met Ser Val Ala Cys Val Leu Lys Arg Lys Ala Val Leu Trp Gln Asp
1               5                   10                  15

Ser Phe Ser Pro His Leu Lys His His Pro Gln Glu Pro Ala Asn Pro
            20                  25                  30

Asn Met Pro Val Val Leu Thr Ser Gly Thr Gly Ser Gln Ala Gln Pro
        35                  40                  45
```

```
Gln Pro Ala Ala Asn Gln Ala Leu Ala Ala Gly Thr His Ser Ser Pro
    50                  55                  60

Val Pro Gly Ser Ile Gly Val Ala Gly Arg Ser Gln Asp Asp Ala Met
65                  70                  75                  80

Val Asp Tyr Phe Phe Gln Arg Gln His Gly Glu Gln Leu Gly Gly Gly
                85                  90                  95

Gly Ser Gly Gly Gly Gly Tyr Asn Asn Ser Lys His Arg Trp Pro Thr
            100                 105                 110

Gly Asp Asn Ile His Ala Glu His Gln Val Arg Ser Met Asp Glu Leu
        115                 120                 125

Asn His Asp Phe Gln Ala Leu Ala Leu Glu Gly Arg Ala Met Gly Glu
    130                 135                 140

Gln Leu Leu Pro Gly Lys Lys Phe Trp Glu Thr Asp Glu Ser Ser Lys
145                 150                 155                 160

Asp Gly Pro Lys Gly Ile Phe Leu Gly Asp Gln Trp Arg Asp Ser Ala
                165                 170                 175

Trp Gly Thr Ser Asp His Ser Val Ser Gln Pro Ile Met Val Gln Arg
            180                 185                 190

Arg Pro Gly Gln Ser Phe His Val Asn Ser Glu Val Asn Ser Val Leu
        195                 200                 205

Ser Pro Arg Ser Glu Ser Gly Gly Leu Gly Val Ser Met Val Glu Tyr
    210                 215                 220

Val Leu Ser Ser Ser Pro Gly Asp Ser Cys Leu Arg Lys Gly Gly Phe
225                 230                 235                 240

Gly Pro Arg Asp Ala Asp Ser Asp Glu Asn Asp Lys Gly Glu Lys Lys
                245                 250                 255

Asn Lys Gly Thr Phe Asp Gly Asp Lys Leu Gly Asp Leu Lys Glu Glu
            260                 265                 270

Gly Asp Val Met Asp Lys Thr Asn Gly Leu Pro Val Gln Asn Gly Ile
        275                 280                 285

Asp Ala Asp Val Lys Asp Phe Ser Arg Thr Pro Gly Asn Cys Gln Asn
    290                 295                 300
```

```
Ser Ala Asn Glu Val Asp Leu Leu Gly Pro Asn Gln Asn Gly Ser Glu
305                 310                 315                 320

Gly Leu Ala Gln Leu Thr Ser Thr Asn Gly Ala Lys Pro Val Glu Asp
                325                 330                 335

Phe Ser Asn Met Glu Ser Gln Ser Val Pro Leu Asp Pro Met Glu His
            340                 345                 350

Val Gly Met Glu Pro Leu Gln Phe Asp Tyr Ser Gly Thr Gln Val Pro
        355                 360                 365

Val Asp Ser Ala Ala Ala Thr Val Gly Leu Phe Asp Tyr Asn Ser Gln
    370                 375                 380

Gln Gln Leu Phe Gln Arg Pro Asn Ala Leu Ala Val Gln Gln Leu Thr
385                 390                 395                 400

Ala Ala Gln Gln Gln Gln Tyr Ala Leu Ala Ala Ala His Gln Pro His
                405                 410                 415

Ile Gly Leu Ala Pro Ala Ala Phe Val Pro Asn Pro Tyr Ile Ile Ser
            420                 425                 430

Ala Ala Pro Pro Gly Thr Asp Pro Tyr Thr Ala Gly Leu Ala Ala Ala
        435                 440                 445

Ala Thr Leu Gly Pro Ala Val Val Pro His Gln Tyr Tyr Gly Val Thr
    450                 455                 460

Pro Trp Gly Val Tyr Pro Ala Ser Leu Phe Gln Gln Gln Ala Ala Ala
465                 470                 475                 480

Ala Ala Ala Ala Thr Asn Ser Ala Asn Gln Gln Thr Thr Pro Gln Ala
                485                 490                 495

Gln Gln Gly Gln Gln Gln Val Leu Arg Gly Gly Ala Ser Gln Arg Pro
            500                 505                 510

Leu Thr Pro Asn Gln Asn Gln Gln Gly Gln Gln Thr Asp Pro Leu Val
        515                 520                 525

Ala Ala Ala Ala Val Asn Ser Ala Leu Ala Phe Gly Gln Gly Leu Ala
    530                 535                 540

Ala Gly Met Pro Gly Tyr Pro Val Leu Ala Pro Ala Ala Tyr Tyr Asp
545                 550                 555                 560
```

```
Gln Thr Gly Ala Leu Val Val Asn Ala Gly Ala Arg Asn Gly Leu Gly
                565                 570                 575

Ala Pro Val Arg Leu Val Ala Pro Ala Pro Val Ile Ile Ser Ser Ser
            580                 585                 590

Ala Ala Gln Ala Ala Val Ala Ala Ala Ala Ala Ser Ala Asn Gly Ala
        595                 600                 605

Ala Gly Gly Leu Ala Gly Thr Thr Asn Gly Pro Phe Arg Pro Leu Gly
    610                 615                 620

Thr Gln Gln Pro Gln Pro Gln Pro Gln Gln Gln Pro Asn Asn Asn Leu
625                 630                 635                 640

Ala Ser Ser Ser Phe Tyr Gly Asn Asn Ser Leu Asn Ser Asn Ser Gln
                645                 650                 655

Ser Ser Ser Leu Phe Ser Gln Gly Ser Ala Gln Pro Ala Asn Thr Ser
            660                 665                 670

Leu Gly Phe Gly Ser Ser Ser Ser Leu Gly Ala Thr Leu Gly Ser Ala
        675                 680                 685

Leu Gly Gly Phe Gly Thr Ala Val Ala Asn Ser Asn Thr Gly Ser Gly
    690                 695                 700

Ser Arg Arg Asp Ser Leu Thr Gly Ser Ser Asp Leu Tyr Lys Arg Thr
705                 710                 715                 720

Ser Ser Ser Leu Thr Pro Ile Gly His Ser Phe Tyr Asn Gly Leu Ser
                725                 730                 735

Phe Ser Ser Ser Pro Gly Pro Val Gly Met Pro Leu Pro Ser Gln Gly
            740                 745                 750

Pro Gly His Ser Gln Thr Pro Pro Pro Ser Leu Ser Ser His Gly Ser
        755                 760                 765

Ser Ser Ser Leu Asn Leu Gly Gly Leu Thr Asn Gly Ser Gly Arg Tyr
    770                 775                 780

Ile Ser Ala Ala Pro Gly Ala Glu Ala Lys Tyr Arg Ser Ala Ser Ser
785                 790                 795                 800

Ala Ser Ser Leu Phe Ser Pro Ser Ser Thr Leu Phe Ser Ser Ser Arg
```

805 810 815

Leu Arg Tyr Gly Met Ser Asp Val Met Pro Ser Gly Arg Ser Arg Leu
820 825 830

Leu Glu Asp Phe Arg Asn Asn Arg Tyr Pro Asn Leu Gln Leu Arg Glu
835 840 845

Ile Ala Gly His Ile Met Glu Phe Ser Gln Asp Gln His Gly Ser Arg
850 855 860

Phe Ile Gln Leu Lys Leu Glu Arg Ala Thr Pro Ala Glu Arg Gln Leu
865 870 875 880

Val Phe Asn Glu Ile Leu Gln Ala Ala Tyr Gln Leu Met Val Asp Val
885 890 895

Phe Gly Asn Tyr Val Ile Gln Lys Phe Phe Glu Phe Gly Ser Leu Glu
900 905 910

Gln Lys Leu Ala Leu Ala Glu Arg Ile Arg Gly His Val Leu Ser Leu
915 920 925

Ala Leu Gln Met Tyr Gly Cys Arg Val Ile Gln Lys Ala Leu Glu Phe
930 935 940

Ile Pro Ser Asp Gln Gln Val Ile Asn Glu Met Val Arg Glu Leu Asp
945 950 955 960

Gly His Val Leu Lys Cys Val Lys Asp Gln Asn Gly Asn His Val Val
965 970 975

Gln Lys Cys Ile Glu Cys Val Gln Pro Gln Ser Leu Gln Phe Ile Ile
980 985 990

Asp Ala Phe Lys Gly Gln Val Phe Ala Leu Ser Thr His Pro Tyr Gly
995 1000 1005

Cys Arg Val Ile Gln Arg Ile Leu Glu His Cys Leu Pro Asp Gln
1010 1015 1020

Thr Leu Pro Ile Leu Glu Glu Leu His Gln His Thr Glu Gln Leu
1025 1030 1035

Val Gln Asp Gln Tyr Gly Asn Tyr Val Ile Gln His Val Leu Glu
1040 1045 1050

His Gly Arg Pro Glu Asp Lys Ser Lys Ile Val Ala Glu Ile Arg
1055 1060 1065

Gly Asn Val Leu Val Leu Ser Gln His Lys Phe Ala Ser Asn Val
1070 1075 1080

Val Glu Lys Cys Val Thr His Ala Ser Arg Thr Glu Arg Ala Val
1085 1090 1095

Leu Ile Asp Glu Val Cys Thr Met Asn Asp Gly Pro His Ser Ala
1100 1105 1110

Leu Tyr Thr Met Met Lys Asp Gln Tyr Ala Asn Tyr Val Val Gln
1115 1120 1125

Lys Met Ile Asp Val Ala Glu Pro Gly Gln Arg Lys Ile Val Met
1130 1135 1140

His Lys Ile Arg Pro His Ile Ala Thr Leu Arg Lys Tyr Thr Tyr
1145 1150 1155

Gly Lys His Ile Leu Ala Lys Leu Glu Lys Tyr Tyr Met Lys Asn
1160 1165 1170

Gly Val Asp Leu Gly Pro Ile Cys Gly Pro Pro Asn Gly Ile Ile
1175 1180 1185

<210> 174
<211> 1186
<212> PRT
<213> Homo sapiens

<400> 174

Met Ser Val Ala Cys Val Leu Lys Arg Lys Ala Val Leu Trp Gln Asp
1 5 10 15

Ser Phe Ser Pro His Leu Lys His His Pro Gln Glu Pro Ala Asn Pro
20 25 30

Asn Met Pro Val Val Leu Thr Ser Gly Thr Gly Ser Gln Ala Gln Pro
35 40 45

Gln Pro Ala Ala Asn Gln Ala Leu Ala Ala Gly Thr His Ser Ser Pro
50 55 60

Val Pro Gly Ser Ile Gly Val Ala Gly Arg Ser Gln Asp Asp Ala Met
65 70 75 80

Val Asp Tyr Phe Phe Gln Arg Gln His Gly Glu Gln Leu Gly Gly Gly
85 90 95

Gly Ser Gly Gly Gly Gly Tyr Asn Asn Ser Lys His Arg Trp Pro Thr
100 105 110

Gly Asp Asn Ile His Ala Glu His Gln Val Arg Ser Met Asp Glu Leu
115 120 125

Asn His Asp Phe Gln Ala Leu Ala Leu Glu Gly Arg Ala Met Gly Glu
130 135 140

Gln Leu Leu Pro Gly Lys Lys Phe Trp Glu Thr Asp Glu Ser Ser Lys
145 150 155 160

Asp Gly Pro Lys Gly Ile Phe Leu Gly Asp Gln Trp Arg Asp Ser Ala
165 170 175

Trp Gly Thr Ser Asp His Ser Val Ser Gln Pro Ile Met Val Gln Arg
180 185 190

Arg Pro Gly Gln Ser Phe His Val Asn Ser Glu Val Asn Ser Val Leu
195 200 205

Ser Pro Arg Ser Glu Ser Gly Gly Leu Gly Val Ser Met Val Glu Tyr
210 215 220

Val Leu Ser Ser Ser Pro Gly Asp Ser Cys Leu Arg Lys Gly Gly Phe
225 230 235 240

Gly Pro Arg Asp Ala Asp Ser Asp Glu Asn Asp Lys Gly Glu Lys Lys
245 250 255

Asn Lys Gly Thr Phe Asp Gly Asp Lys Leu Gly Asp Leu Lys Glu Glu
260 265 270

Gly Asp Val Met Asp Lys Thr Asn Gly Leu Pro Val Gln Asn Gly Ile
275 280 285

Asp Ala Asp Val Lys Asp Phe Ser Arg Thr Pro Gly Asn Cys Gln Asn
290 295 300

Ser Ala Asn Glu Val Asp Leu Leu Gly Pro Asn Gln Asn Gly Ser Glu
305 310 315 320

Gly Leu Ala Gln Leu Thr Ser Thr Asn Gly Ala Lys Pro Val Glu Asp
325 330 335

```
Phe Ser Asn Met Glu Ser Gln Ser Val Pro Leu Asp Pro Met Glu His
            340                 345                 350

Val Gly Met Glu Pro Leu Gln Phe Asp Tyr Ser Gly Thr Gln Val Pro
        355                 360                 365

Val Asp Ser Ala Ala Ala Thr Val Gly Leu Phe Asp Tyr Asn Ser Gln
    370                 375                 380

Gln Gln Leu Phe Gln Arg Pro Asn Ala Leu Ala Val Gln Gln Leu Thr
385                 390                 395                 400

Ala Ala Gln Gln Gln Gln Tyr Ala Leu Ala Ala Ala His Gln Pro His
                405                 410                 415

Ile Gly Leu Ala Pro Ala Ala Phe Val Pro Asn Pro Tyr Ile Ile Ser
            420                 425                 430

Ala Ala Pro Pro Gly Thr Asp Pro Tyr Thr Ala Gly Leu Ala Ala Ala
        435                 440                 445

Ala Thr Leu Gly Pro Ala Val Val Pro His Gln Tyr Tyr Gly Val Thr
    450                 455                 460

Pro Trp Gly Val Tyr Pro Ala Ser Leu Phe Gln Gln Gln Ala Ala Ala
465                 470                 475                 480

Ala Ala Ala Ala Thr Asn Ser Ala Asn Gln Gln Thr Thr Pro Gln Ala
                485                 490                 495

Gln Gln Gly Gln Gln Gln Val Leu Arg Gly Gly Ala Ser Gln Arg Pro
            500                 505                 510

Leu Thr Pro Asn Gln Asn Gln Gln Gly Gln Gln Thr Asp Pro Leu Val
        515                 520                 525

Ala Ala Ala Ala Val Asn Ser Ala Leu Ala Phe Gly Gln Gly Leu Ala
    530                 535                 540

Ala Gly Met Pro Gly Tyr Pro Val Leu Ala Pro Ala Ala Tyr Tyr Asp
545                 550                 555                 560

Gln Thr Gly Ala Leu Val Val Asn Ala Gly Ala Arg Asn Gly Leu Gly
                565                 570                 575

Ala Pro Val Arg Leu Val Ala Pro Ala Pro Val Ile Ile Ser Ser Ser
            580                 585                 590
```

```
Ala Ala Gln Ala Ala Val Ala Ala Ala Ala Ala Ser Ala Asn Gly Ala
        595                 600                 605

Ala Gly Gly Leu Ala Gly Thr Thr Asn Gly Pro Phe Arg Pro Leu Gly
    610                 615                 620

Thr Gln Gln Pro Gln Pro Gln Pro Gln Gln Gln Pro Asn Asn Asn Leu
625                 630                 635                 640

Ala Ser Ser Ser Phe Tyr Gly Asn Asn Ser Leu Asn Ser Asn Ser Gln
                645                 650                 655

Ser Ser Ser Leu Phe Ser Gln Gly Ser Ala Gln Pro Ala Asn Thr Ser
            660                 665                 670

Leu Gly Phe Gly Ser Ser Ser Ser Leu Gly Ala Thr Leu Gly Ser Ala
        675                 680                 685

Leu Gly Gly Phe Gly Thr Ala Val Ala Asn Ser Asn Thr Gly Ser Gly
    690                 695                 700

Ser Arg Arg Asp Ser Leu Thr Gly Ser Ser Asp Leu Tyr Lys Arg Thr
705                 710                 715                 720

Ser Ser Ser Leu Thr Pro Ile Gly His Ser Phe Tyr Asn Gly Leu Ser
                725                 730                 735

Phe Ser Ser Ser Pro Gly Pro Val Gly Met Pro Leu Pro Ser Gln Gly
            740                 745                 750

Pro Gly His Ser Gln Thr Pro Pro Pro Ser Leu Ser Ser His Gly Ser
        755                 760                 765

Ser Ser Ser Leu Asn Leu Gly Gly Leu Thr Asn Gly Ser Gly Arg Tyr
    770                 775                 780

Ile Ser Ala Ala Pro Gly Ala Glu Ala Lys Tyr Arg Ser Ala Ser Ser
785                 790                 795                 800

Ala Ser Ser Leu Phe Ser Pro Ser Ser Thr Leu Phe Ser Ser Ser Arg
                805                 810                 815

Leu Arg Tyr Gly Met Ser Asp Val Met Pro Ser Gly Arg Ser Arg Leu
            820                 825                 830

Leu Glu Asp Phe Arg Asn Asn Arg Tyr Pro Asn Leu Gln Leu Arg Glu
```

835 840 845

Ile Ala Gly His Ile Met Glu Phe Ser Gln Asp Gln His Gly Ser Arg
850 855 860

Phe Ile Gln Leu Lys Leu Glu Arg Ala Thr Pro Ala Glu Arg Gln Leu
865 870 875 880

Val Phe Asn Glu Ile Leu Gln Ala Ala Tyr Gln Leu Met Val Asp Val
885 890 895

Phe Gly Asn Tyr Val Ile Gln Lys Phe Phe Glu Phe Gly Ser Leu Glu
900 905 910

Gln Lys Leu Ala Leu Ala Glu Arg Ile Arg Gly His Val Leu Ser Leu
915 920 925

Ala Leu Gln Met Tyr Gly Cys Arg Val Ile Gln Lys Ala Leu Glu Phe
930 935 940

Ile Pro Ser Asp Gln Gln Asn Glu Met Val Arg Glu Leu Asp Gly His
945 950 955 960

Val Leu Lys Cys Val Lys Asp Gln Asn Gly Asn His Val Val Gln Lys
965 970 975

Cys Ile Glu Cys Val Gln Pro Gln Ser Leu Gln Phe Ile Ile Asp Ala
980 985 990

Phe Lys Gly Gln Val Phe Ala Leu Ser Thr His Pro Tyr Gly Cys Arg
995 1000 1005

Val Ile Gln Arg Ile Leu Glu His Cys Leu Pro Asp Gln Thr Leu
1010 1015 1020

Pro Ile Leu Glu Glu Leu His Gln His Thr Glu Gln Leu Val Gln
1025 1030 1035

Asp Gln Tyr Gly Asn Tyr Val Ile Gln His Val Leu Glu His Gly
1040 1045 1050

Arg Pro Glu Asp Lys Ser Lys Ile Val Ala Glu Ile Arg Gly Asn
1055 1060 1065

Val Leu Val Leu Ser Gln His Lys Phe Ala Ser Asn Val Val Glu
1070 1075 1080

```
Lys Cys Val Thr His Ala Ser Arg Thr Glu Arg Ala Val Leu Ile
    1085                1090                1095

Asp Glu Val Cys Thr Met Asn Asp Gly Pro His Ser Ala Leu Tyr
    1100                1105                1110

Thr Met Met Lys Asp Gln Tyr Ala Asn Tyr Val Val Gln Lys Met
    1115                1120                1125

Ile Asp Val Ala Glu Pro Gly Gln Arg Lys Ile Val Met His Lys
    1130                1135                1140

Ile Arg Pro His Ile Ala Thr Leu Arg Lys Tyr Thr Tyr Gly Lys
    1145                1150                1155

His Ile Leu Ala Lys Leu Glu Lys Tyr Tyr Met Lys Asn Gly Val
    1160                1165                1170

Asp Leu Gly Pro Ile Cys Gly Pro Pro Asn Gly Ile Ile
    1175                1180                1185
```

<210> 175
<211> 451
<212> PRT
<213> Homo sapiens

<400> 175

```
Met Arg Glu Cys Ile Ser Ile His Val Gly Gln Ala Gly Val Gln Ile
1               5                   10                  15

Gly Asn Ala Cys Trp Glu Leu Tyr Cys Leu Glu His Gly Ile Gln Pro
            20                  25                  30

Asp Gly Gln Met Pro Ser Asp Lys Thr Ile Gly Gly Gly Asp Asp Ser
        35                  40                  45

Phe Asn Thr Phe Phe Ser Glu Thr Gly Ala Gly Lys His Val Pro Arg
    50                  55                  60

Ala Val Phe Val Asp Leu Glu Pro Thr Val Ile Asp Glu Val Arg Thr
65                  70                  75                  80

Gly Thr Tyr Arg Gln Leu Phe His Pro Glu Gln Leu Ile Thr Gly Lys
                85                  90                  95

Glu Asp Ala Ala Asn Asn Tyr Ala Arg Gly His Tyr Thr Ile Gly Lys
            100                 105                 110
```

```
Glu Ile Ile Asp Leu Val Leu Asp Arg Ile Arg Lys Leu Ala Asp Gln
        115                 120                 125

Cys Thr Gly Leu Gln Gly Phe Leu Val Phe His Ser Phe Gly Gly Gly
    130                 135                 140

Thr Gly Ser Gly Phe Thr Ser Leu Leu Met Glu Arg Leu Ser Val Asp
145                 150                 155                 160

Tyr Gly Lys Lys Ser Lys Leu Glu Phe Ser Ile Tyr Pro Ala Pro Gln
                165                 170                 175

Val Ser Thr Ala Val Val Glu Pro Tyr Asn Ser Ile Leu Thr Thr His
            180                 185                 190

Thr Thr Leu Glu His Ser Asp Cys Ala Phe Met Val Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Ser Gln Ile Val Ser Ser Ile Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Pro Leu Ala Thr Tyr
            260                 265                 270

Ala Pro Val Ile Ser Ala Glu Lys Ala Tyr His Glu Gln Leu Ser Val
        275                 280                 285

Ala Glu Ile Thr Asn Ala Cys Phe Glu Pro Ala Asn Gln Met Val Lys
    290                 295                 300

Cys Asp Pro Arg His Gly Lys Tyr Met Ala Cys Cys Leu Leu Tyr Arg
305                 310                 315                 320

Gly Asp Val Val Pro Lys Asp Val Asn Ala Ala Ile Ala Thr Ile Lys
                325                 330                 335

Thr Lys Arg Ser Ile Gln Phe Val Asp Trp Cys Pro Thr Gly Phe Lys
            340                 345                 350

Val Gly Ile Asn Tyr Gln Pro Pro Thr Val Val Pro Gly Gly Asp Leu
        355                 360                 365
```

Ala Lys Val Gln Arg Ala Val Cys Met Leu Ser Asn Thr Thr Ala Ile
370 375 380

Ala Glu Ala Trp Ala Arg Leu Asp His Lys Phe Asp Leu Met Tyr Ala
385 390 395 400

Lys Arg Ala Phe Val His Trp Tyr Val Gly Glu Gly Met Glu Glu Gly
405 410 415

Glu Phe Ser Glu Ala Arg Glu Asp Met Ala Ala Leu Glu Lys Asp Tyr
420 425 430

Glu Glu Val Gly Val Asp Ser Val Glu Gly Glu Gly Glu Glu Glu Gly
435 440 445

Glu Glu Tyr
450

<210> 176
<211> 640
<212> PRT
<213> Homo sapiens

<400> 176

Met Glu Ser Val Val Arg Arg Cys Pro Phe Leu Ser Arg Val Pro Gln
1 5 10 15

Ala Phe Leu Gln Lys Ala Gly Lys Ser Leu Leu Phe Tyr Ala Gln Asn
20 25 30

Cys Pro Lys Met Met Glu Val Gly Ala Lys Pro Ala Pro Arg Ala Leu
35 40 45

Ser Thr Ala Ala Val His Tyr Gln Gln Ile Lys Glu Thr Pro Pro Ala
50 55 60

Ser Glu Lys Asp Lys Thr Ala Lys Ala Lys Val Gln Gln Thr Pro Asp
65 70 75 80

Gly Ser Gln Gln Ser Pro Asp Gly Thr Gln Leu Pro Ser Gly His Pro
85 90 95

Leu Pro Ala Thr Ser Gln Gly Thr Ala Ser Lys Cys Pro Phe Leu Ala
100 105 110

Ala Gln Met Asn Gln Arg Gly Ser Ser Val Phe Cys Lys Ala Ser Leu
115 120 125

```
Glu Leu Gln Glu Asp Val Gln Glu Met Asn Ala Val Arg Lys Glu Val
    130                 135                 140

Ala Glu Thr Ser Ala Gly Pro Ser Val Val Ser Val Lys Thr Asp Gly
145                 150                 155                 160

Gly Asp Pro Ser Gly Leu Leu Lys Asn Phe Gln Asp Ile Met Gln Lys
                165                 170                 175

Gln Arg Pro Glu Arg Val Ser His Leu Leu Gln Asp Asn Leu Pro Lys
            180                 185                 190

Ser Val Ser Thr Phe Gln Tyr Asp Arg Phe Phe Glu Lys Lys Ile Asp
        195                 200                 205

Glu Lys Lys Asn Asp His Thr Tyr Arg Val Phe Lys Thr Val Asn Arg
    210                 215                 220

Arg Ala His Ile Phe Pro Met Ala Asp Asp Tyr Ser Asp Ser Leu Ile
225                 230                 235                 240

Thr Lys Lys Gln Val Ser Val Trp Cys Ser Asn Asp Tyr Leu Gly Met
                245                 250                 255

Ser Arg His Pro Arg Val Cys Gly Ala Val Met Asp Thr Leu Lys Gln
            260                 265                 270

His Gly Ala Gly Ala Gly Gly Thr Arg Asn Ile Ser Gly Thr Ser Lys
        275                 280                 285

Phe His Val Asp Leu Glu Arg Glu Leu Ala Asp Leu His Gly Lys Asp
    290                 295                 300

Ala Ala Leu Leu Phe Ser Ser Cys Phe Val Ala Asn Asp Ser Thr Leu
305                 310                 315                 320

Phe Thr Leu Ala Lys Met Met Pro Gly Cys Glu Ile Tyr Ser Asp Ser
                325                 330                 335

Gly Asn His Ala Ser Met Ile Gln Gly Ile Arg Asn Ser Arg Val Pro
            340                 345                 350

Lys Tyr Ile Phe Arg His Asn Asp Val Ser His Leu Arg Glu Leu Leu
        355                 360                 365

Gln Arg Ser Asp Pro Ser Val Pro Lys Ile Val Ala Phe Glu Thr Val
    370                 375                 380
```

His Ser Met Asp Gly Ala Val Cys Pro Leu Glu Glu Leu Cys Asp Val
385 390 395 400

Ala His Glu Phe Gly Ala Ile Thr Phe Val Asp Glu Val His Ala Val
405 410 415

Gly Leu Tyr Gly Ala Arg Gly Gly Gly Ile Gly Asp Arg Asp Gly Val
420 425 430

Met Pro Lys Met Asp Ile Ile Ser Gly Thr Leu Gly Lys Ala Phe Gly
435 440 445

Cys Val Gly Gly Tyr Ile Ala Ser Thr Ser Ser Leu Ile Asp Thr Val
450 455 460

Arg Ser Tyr Ala Ala Gly Phe Ile Phe Thr Thr Ser Leu Pro Pro Met
465 470 475 480

Leu Leu Ala Gly Ala Leu Glu Ser Val Arg Ile Leu Lys Ser Ala Glu
485 490 495

Gly Arg Val Leu Arg Arg Gln His Gln Arg Asn Val Lys Leu Met Arg
500 505 510

Gln Met Leu Met Asp Ala Gly Leu Pro Val Val His Cys Pro Ser His
515 520 525

Ile Ile Pro Val Arg Val Ala Asp Ala Ala Lys Asn Thr Glu Val Cys
530 535 540

Asp Glu Leu Met Ser Arg His Asn Ile Tyr Val Gln Ala Ile Asn Tyr
545 550 555 560

Pro Thr Val Pro Arg Gly Glu Glu Leu Leu Arg Ile Ala Pro Thr Pro
565 570 575

His His Thr Pro Gln Met Met Asn Tyr Phe Leu Glu Asn Leu Leu Val
580 585 590

Thr Trp Lys Gln Val Gly Leu Glu Leu Lys Pro His Ser Ser Ala Glu
595 600 605

Cys Asn Phe Cys Arg Arg Pro Leu His Phe Glu Val Met Ser Glu Arg
610 615 620

Glu Lys Ser Tyr Phe Ser Gly Leu Ser Lys Leu Val Ser Ala Gln Ala

625 630 635 640

<210> 177
<211> 640
<212> PRT
<213> Homo sapiens

<400> 177

Met Glu Ser Val Val Arg Arg Cys Pro Phe Leu Ser Arg Val Pro Gln
1               5                   10                  15

Ala Phe Leu Gln Lys Ala Gly Lys Ser Leu Leu Phe Tyr Ala Gln Asn
            20                  25                  30

Cys Pro Lys Met Met Glu Val Gly Ala Lys Pro Ala Pro Arg Ala Leu
        35                  40                  45

Ser Thr Ala Ala Val His Tyr Gln Gln Ile Lys Glu Thr Pro Pro Ala
    50                  55                  60

Ser Glu Lys Asp Lys Thr Ala Lys Ala Lys Val Gln Gln Thr Pro Asp
65                  70                  75                  80

Gly Ser Gln Gln Ser Pro Asp Gly Thr Gln Leu Pro Ser Gly His Pro
                85                  90                  95

Leu Pro Ala Thr Ser Gln Gly Thr Ala Ser Lys Cys Pro Phe Leu Ala
            100                 105                 110

Ala Gln Met Asn Gln Arg Gly Ser Ser Val Phe Cys Lys Ala Ser Leu
        115                 120                 125

Glu Leu Gln Glu Asp Val Gln Glu Met Asn Ala Val Arg Lys Glu Val
    130                 135                 140

Ala Glu Thr Ser Ala Gly Pro Ser Val Val Ser Val Lys Thr Asp Gly
145                 150                 155                 160

Gly Asp Pro Ser Gly Leu Leu Lys Asn Phe Gln Asp Ile Met Gln Lys
                165                 170                 175

Gln Arg Pro Glu Arg Val Ser His Leu Leu Gln Asp Asn Leu Pro Lys
            180                 185                 190

Ser Val Ser Thr Phe Gln Tyr Asp Arg Phe Phe Glu Lys Lys Ile Asp
        195                 200                 205

Glu Lys Lys Asn Asp His Thr Tyr Arg Val Phe Lys Thr Val Asn Arg

```
    210                 215                 220

Arg Ala His Ile Phe Pro Met Ala Asp Asp Tyr Ser Asp Ser Leu Ile
225                 230                 235                 240

Thr Lys Lys Gln Val Ser Val Trp Cys Ser Asn Asp Tyr Leu Gly Met
                245                 250                 255

Ser Arg His Pro Arg Val Cys Gly Ala Val Met Asp Thr Leu Lys Gln
            260                 265                 270

His Gly Ala Gly Ala Gly Gly Thr Arg Asn Ile Ser Gly Thr Ser Lys
        275                 280                 285

Phe His Val Asp Leu Glu Arg Glu Leu Ala Asp Leu His Gly Lys Asp
    290                 295                 300

Ala Ala Leu Leu Phe Ser Ser Cys Phe Val Ala Asn Asp Ser Thr Leu
305                 310                 315                 320

Phe Thr Leu Ala Lys Met Met Pro Gly Cys Glu Ile Tyr Ser Asp Ser
                325                 330                 335

Gly Asn His Ala Ser Met Ile Gln Gly Ile Arg Asn Ser Arg Val Pro
            340                 345                 350

Lys Tyr Ile Phe Arg His Asn Asp Val Ser His Leu Arg Glu Leu Leu
        355                 360                 365

Gln Arg Ser Asp Pro Ser Val Pro Lys Ile Val Ala Phe Glu Thr Val
    370                 375                 380

His Ser Met Asp Gly Ala Val Cys Pro Leu Glu Glu Leu Cys Asp Val
385                 390                 395                 400

Ala His Glu Phe Gly Ala Ile Thr Phe Val Asp Glu Val His Ala Val
                405                 410                 415

Gly Leu Tyr Gly Ala Arg Gly Gly Gly Ile Gly Asp Arg Asp Gly Val
            420                 425                 430

Met Pro Lys Met Asp Ile Ile Ser Gly Thr Leu Gly Lys Ala Phe Gly
        435                 440                 445

Cys Val Gly Gly Tyr Ile Ala Ser Thr Ser Ser Leu Ile Asp Thr Val
    450                 455                 460
```

```
Arg Ser Tyr Ala Ala Gly Phe Ile Phe Thr Thr Ser Leu Pro Pro Met
465                 470                 475                 480

Leu Leu Ala Gly Ala Leu Glu Ser Val Arg Ile Leu Lys Ser Ala Glu
                485                 490                 495

Gly Arg Val Leu Arg Arg Gln His Gln Arg Asn Val Lys Leu Met Arg
            500                 505                 510

Gln Met Leu Met Asp Ala Gly Leu Pro Val Val His Cys Pro Ser His
        515                 520                 525

Ile Ile Pro Val Arg Val Ala Asp Ala Ala Lys Asn Thr Glu Val Cys
    530                 535                 540

Asp Glu Leu Met Ser Arg His Asn Ile Tyr Val Gln Ala Ile Asn Tyr
545                 550                 555                 560

Pro Thr Val Pro Arg Gly Glu Glu Leu Leu Arg Ile Ala Pro Thr Pro
                565                 570                 575

His His Thr Pro Gln Met Met Asn Tyr Phe Leu Glu Asn Leu Leu Val
            580                 585                 590

Thr Trp Lys Gln Val Gly Leu Glu Leu Lys Pro His Ser Ser Ala Glu
        595                 600                 605

Cys Asn Phe Cys Arg Arg Pro Leu His Phe Glu Val Met Ser Glu Arg
    610                 615                 620

Glu Lys Ser Tyr Phe Ser Gly Leu Ser Lys Leu Val Ser Ala Gln Ala
625                 630                 635                 640
```

<210> 178
<211> 23
<212> DNA
<213> Artificial Sequence

<220>
<223> TBP_forward primer

<400> 178
gccaagaaga aagtgaacat cat 23

<210> 179
<211> 20
<212> DNA
<213> Artificial Sequence

<220>

<223> TBP_reverse primer

<400> 179
atagggattc cgggagtcat 20

<210> 180
<211> 24
<212> DNA
<213> Artificial Sequence

<220>
<223> TBP probe

<400> 180
tcagaacaac agcctgccac ctta 24

<210> 181
<211> 24
<212> DNA
<213> Artificial Sequence

<220>
<223> HPRT1_forward primer

<400> 181
gaggatttgg aaagggtgtt tatt 24

<210> 182
<211> 21
<212> DNA
<213> Artificial Sequence

<220>
<223> HPRT1_reverse primer

<400> 182
acagagggct acaatgtgat g 21

<210> 183
<211> 26
<212> DNA
<213> Artificial Sequence

<220>
<223> HPRT1 probe

<400> 183
acgtcttgct cgagatgtga tgaagg 26

<210> 184
<211> 18
<212> DNA
<213> Artificial Sequence

<220>
<223> ACTB_forward primer

<400> 184

ccaaccgcga gaagatga 18

<210> 185
<211> 20
<212> DNA
<213> Artificial Sequence

<220>
<223> ACTB_reverse primer

<400> 185
ccagaggcgt acagggatag 20

<210> 186
<211> 23
<212> DNA
<213> Artificial Sequence

<220>
<223> ACTB probe

<400> 186
ccatgtacgt tgctatccag gct 23

<210> 187
<211> 18
<212> DNA
<213> Artificial Sequence

<220>
<223> RPLP0_forward primer

<400> 187
taaaccctgc gtggcaat 18

<210> 188
<211> 27
<212> DNA
<213> Artificial Sequence

<220>
<223> RPLP0_reverse primer

<400> 188
acatttcgga taatcatcca atagttg 27

<210> 189
<211> 24
<212> DNA
<213> Artificial Sequence

<220>
<223> RPLP0 probe

<400> 189
aagtagttgg acttccaggt cgcc 24

<210> 190

<211> 19
<212> DNA
<213> Artificial Sequence

<220>
<223> PoLR2A_forward primer

<400> 190
agtcctgagt ccggatgaa 19

<210> 191
<211> 18
<212> DNA
<213> Artificial Sequence

<220>
<223> POLR2A_reverse primer

<400> 191
cctccctcag tcgtctct 18

<210> 192
<211> 24
<212> DNA
<213> Artificial Sequence

<220>
<223> POLR2A probe

<400> 192
tgacggaggg tggcatcaaa tacc 24

<210> 193
<211> 17
<212> DNA
<213> Artificial Sequence

<220>
<223> B2M_forward primer

<400> 193
ccgtggcctt agctgtg 17

<210> 194
<211> 21
<212> DNA
<213> Artificial Sequence

<220>
<223> B2M_reverse primer

<400> 194
ctgctggatg acgtgagtaa a 21

<210> 195
<211> 24
<212> DNA
<213> Artificial Sequence

<220>
<223> B2M probe

<400> 195
tctctctttc tggcctggag gcta 24

<210> 196
<211> 22
<212> DNA
<213> Artificial Sequence

<220>
<223> PUM1_forward primer

<400> 196
gccagcttgt cttcaatgaa at 22

<210> 197
<211> 21
<212> DNA
<213> Artificial Sequence

<220>
<223> PUM1_reverse primer

<400> 197
caaagccagc ttctgttcaa g 21

<210> 198
<211> 24
<212> DNA
<213> Artificial Sequence

<220>
<223> PUM1 probe

<400> 198
atccaccatg agttggtagg cagc 24

<210> 199
<211> 22
<212> DNA
<213> Artificial Sequence

<220>
<223> K-ALPHA-1_forward primer

<400> 199
tgactccttc aacaccttct tc 22

<210> 200
<211> 18
<212> DNA
<213> Artificial Sequence

<220>
<223> K-ALPHA-1_reverse primer

<400> 200
tgccagtgcg aacttcat 18

<210> 201
<211> 24
<212> DNA
<213> Artificial Sequence

<220>
<223> K-ALPHA-1 probe

<400> 201
ccgggctgtg tttgtagact tgga 24

<210> 202
<211> 17
<212> DNA
<213> Artificial Sequence

<220>
<223> ALAS-1_forward primer

<400> 202
agccacatca tccctgt 17

<210> 203
<211> 22
<212> DNA
<213> Artificial Sequence

<220>
<223> ALAS-1_reverse primer

<400> 203
cgtagatgtt atgtctgctc at 22

<210> 204
<211> 22
<212> DNA
<213> Artificial Sequence

<220>
<223> ALAS-1 probe

<400> 204
tttagcagca tctgcaaccc gc 22

## Description

FIELD OF THE INVENTION

The invention pertains to methods for determining gene expression signatures for diagnosing patients with prostate cancer, e.g., to establish a prognosis for such patients, for determining the predisposition of said patient to develop aggressive or indolent disease, for example after primary treatment, and/or for identification and stratification of patients for available therapies. The invention also relates to products used in such methods, e.g. kits, software, and the like.

BACKGROUND OF THE INVENTION

Cancer is a class of diseases in which a group of cells display uncontrolled growth, invasion and sometimes metastasis. These three malignant properties of cancers differentiate them from benign tumors, which are self-limited, do not invade or metastasize.

Prostate Cancer (PCa) is the most commonly occurring non-skin malignancy in men, with estimated 900,000 new cases diagnosed world-wide in 2008. Due to ageing populations, the incidence of PCa will dramatically increase in the coming years. Routine diagnosis by determination of blood levels of the prostate-specific antigen (PSA), digital rectal exam (DRE) and transrectal ultrasound analysis (TRUS) leads to significant first-line over-diagnosis of non-cancerous, benign prostate conditions: of the approx. 1 million prostate biopsies annually performed in the U.S. alone to find about 250,000 new cases, about 75% are done unnecessarily, incurring both substantial complications (urosepsis, bleedings, urinary retention) in patients and total cost of >US$ 2 billion (~US$ 2,100 per biopsy procedure). At least 4 out of 100 men with a negative biopsy are likely to be hospitalized due to side-effects and 9 out of 10,000 biopsied patients are at risk of dying from the currently used procedure.

Of the approximately 250,000 newly detected PCa cases in the U.S. per year, about 200,000 are initially characterized as localized disease, i.e. as cancer confined to the prostate organ. This condition is to a certain extent curable by primary treatment approaches, such as radiation therapy or, in particular, the partial or total removal of the prostate by surgery (prostatectomy). However, these interventions typically come with serious side effects, particularly urinary incontinence and/or erectile dysfunctions as very frequent consequences of prostatectomy. Up to 50% of men undergoing radical prostatectomy develop urinary incontinence. Studies have shown that, one year after surgery, between 15% and 50% of men still report such problems. Erection problems likewise are serious side effects of radical prostatectomy (RP). Only about half of the operated men are able to regain some of their ability to have erections. Furthermore, all routinely applied treatments for localized PCa are expensive (typically in the order of US$ 20-30,000) and incur total direct costs of US$ 5 billion in the U.S. each year.

Among the ~200,000 men in the United States with clinically localized disease at diagnosis, up to 50% have very-low- or low-risk cancer. Accordingly, the NCCN (National Comprehensive Cancer Network) recently revised their PCa treatment guidelines to expand active surveillance (AS) as a gentle and convenient treatment alternative for patients with such low risk disease. By referring appropriate patients to AS, the quality of life for such patients is significantly improved as compared with men having undergone primary treatment and the 5-year cost for AS is reported to be less than US$ 10,000 per patient.

Moreover, in case surgery (vs. AS) is selected as the treatment of choice for a given patient, it is of significant advantage to stratify for the extent of surgery according to the potential aggressiveness of the patient's tumor. For instance, nerve-sparing operation techniques could be more generally applied for men with predicted low-risk disease to minimize potency-related adverse effects of radical prostatectomy. Likewise, according to the European Association Of Urology (EAU)'s latest Prostate Cancer Guidelines, extended lymph node dissection is recommended in case of a predicted high-risk cancer despite the fact that the procedure is complex, time-consuming and associated with higher complication rates as compared with more limited procedures. Consequently, while less limited lymph node dissection has shown to miss about 50% of lymph node metastases, the management of men with localized prostate cancer requires highly accurate pre-surgical predictions of the aggressiveness potential of an individual tumor to provide most optimal care for each patient.

US 2013/196321 A1 describes molecular assays, which comprise measurement of expression levels of one or more genes in a sample from a prostate cancer patient to provide information concerning the likelihood of a clinical outcome, identify risk classification and facilitate treatment decision making. The reference discloses a number of genes to be analyzed in such assays, which are normalized to e.g. a housekeeping gene. Furthermore, kits comprising gene-specific probes and/or primers to perform the assays and computer-based systems, algorithms and computer program products to perform the analysis of the expression levels are also disclosed.

The article "DYRK2 controls the epithelial-mesenchymal transition in breast cancer by degrading Snail" by Mimoto Rei et al. in Cancer Letters (2013), Volume 339, Number 2, pages 214-225, discloses that DYRK2 is down regulated in human breast cancer tissue and that patients with low DYRK2-expressing tumors have a worse outcome than those with high SYRK2-expressing tumors. The authors conclude that DYRK2 is a potential prognostic marker for breast cancer and additionally mention, that it has been demonstrated that DYRK2 has also been found to be down-regulated in lung, colon and prostate cancers.

WO 2010/131194 A1 relates to PDE4D7 as a marker for malignant hormone sensitive prostate cancer and discloses a method for diagnosing, detecting, monitoring and prognosticating malignant, hormone sensitive prostate cancer comprising the step of determining the level of PDE4D7. In addition, methods for identifying an individual for eligibility for certain cancer therapy are described as well as a composition for carrying out the method, comprising oligonucleotides or probes specific for the PDE4D7 expression product.

WO 2014/153442 A2 discloses methods for diagnosis, prognosis and treatment of ovarian cancer which involve the determination of the expression levels of certain marker genes. The expression levels are converted into an index, wherein the genes are weighted equally with the expression levels of down-regulated genes subtracted from the ones of up-regulated genes.

## SUMMARY OF THE INVENTION

The present invention relates to the identification and use of gene expression profiles, signatures, or patterns of biomarker genes of interest (also referred to as marker genes) with clinical relevance to prostate cancer. In particular, the invention is based on the gene expression analysis of nucleic acids, preferably transcripts of biomarker genes, obtained from biological samples and the identification of biomarker genes that are correlated with aggressive and indolent disease, patient survival and prostate cancer recurrence. Expression analysis of these marker genes can be used in providing a Prostate Cancer Progression Index (PCPI) that can be used in the classification of patients into those with an aggressive and indolent disease and with corresponding poor or good prognosis.

Further, the PCPI can also be used to predict the progression of disease in previously clinically diagnosed and treated subjects and to stratify the patients according to their individual PCPI. Therefore, the PCPI provides a very helpful parameter for personalized medicine relating to the diagnosis, prognosis and treatment of prostate cancer patients. The PCPI may be used alone or in combination with other means and methods that provide information on the patients' personal disease status or disease stage.

Physicians and/or pathologists can advantageously use the PCPI to confirm results obtained in other methods for diagnosing, identifying, prognosticating, classifying, and/or stratifying patients, e.g. patients with a predisposition to develop aggressive versus indolent prostate cancer forms. The methods and means provided by the invention therefore help establish better diagnosis, prognosis, etc. to find the best treatment for a patient, and to avoid unnecessary surgery or other treatments that are dangerous due to side-effects, occasionally superfluous and result in enormous costs for the public health systems.

One aspect of the invention is directed to a method comprising:

determining a gene expression level for the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, to obtain a subject expression profile for a subject and further comprising:
classifying the subject as having a good prognosis or a poor prognosis of prostate cancer based on the subject expression profile, wherein the good prognosis predicts an increased likelihood of survival within a predetermined period after initial diagnosis and/or no progression of disease after primary treatment, and the poor prognosis predicts an aggressive disease, a decreased likelihood of survival, an increased likelihood of biochemical recurrence, clinical recurrence, and/or the presence of local or distant metastases, within a predetermined period after initial diagnosis; and/or
classifying the subject as having or not having a predisposition of prostate cancer that is susceptible to disease progression based on the subject expression profile, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In one embodiment, the method further comprises:

classifying the subject as having a good prognosis or a poor prognosis of prostate cancer based on the subject expression profile, wherein the good prognosis predicts an increased likelihood of survival within a predetermined period after initial diagnosis and/or no progression of disease after primary treatment, and the poor prognosis predicts an aggressive disease, a decreased likelihood of survival, an increased likelihood of biochemical recurrence, clinical recurrence, and/or the presence of local or distant metastases, within a predetermined period after initial diagnosis; or

classifying the subject as having or not having a predisposition of prostate cancer that is susceptible to disease progression based on the subject expression profile, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In one embodiment, the method further comprises:

classifying the subject as having a good prognosis or a poor prognosis of prostate cancer based on the subject expression profile, wherein the good prognosis predicts an increased likelihood of survival within a predetermined period after initial diagnosis and/or no progression of disease after primary treatment, and the poor prognosis predicts an aggressive disease, a decreased likelihood of survival, an increased likelihood of biochemical recurrence, clinical recurrence, and/or the presence of local or distant metastases, within a predetermined period after initial diagnosis; and

classifying the subject as having or not having a predisposition of prostate cancer that is susceptible to disease progression based on the subject expression profile, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In one embodiment, the method further comprises:

classifying the subject as having a good prognosis or a poor prognosis of prostate cancer based on the subject expression profile, wherein the good prognosis predicts an increased likelihood of survival within a predetermined period after initial diagnosis and/or no progression of disease after primary treatment, and the poor prognosis predicts an aggressive disease, a decreased likelihood of survival, an increased likelihood of biochemical recurrence, clinical recurrence, and/or the presence of local or distant metastases, within a predetermined period after initial diagnosis; or

classifying the subject as having or not having a predisposition of prostate cancer that is susceptible to disease progression based on the subject expression profile, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In one embodiment, the method further comprises:
classifying the subject as having a good prognosis or a poor prognosis of prostate cancer based on the subject expression profile, wherein the good prognosis predicts an increased likelihood of survival within a predetermined period after initial diagnosis and/or no progression of disease after primary treatment, and the poor prognosis predicts an aggressive disease, a decreased likelihood of survival, an increased likelihood of biochemical recurrence, clinical recurrence, and/or the presence of local or distant metastases, within a predetermined period after initial diagnosis; and
classifying the subject as having or not having a predisposition of prostate cancer that is susceptible to disease progression based on the subject expression profile, wherein the predisposition predicts an aggressive disease, a decreased likelihood of survival, an increased likelihood of biochemical recurrence, clinical recurrence, and/or the presence of local or distant metastases, within a predetermined period after initial diagnosis.

Another aspect of the invention is directed to a method comprising:

classifying a subject as having a good prognosis or a poor prognosis based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, and wherein a good prognosis predicts an increased likelihood of survival within a predetermined period after initial diagnosis and/or no progression of disease after primary treatment, and poor prognosis predicts an aggressive disease, a decreased likelihood of survival, an increased likelihood of biochemical recurrence, clinical recurrence, and/or the presence of local or distant metastases, within a predetermined period after initial diagnosis; and/or

classifying a subject as having or not having a predisposition of prostate cancer that is susceptible to disease progression based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In one embodiment, a method comprises:
classifying a subject as having a good prognosis or a poor prognosis based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, and wherein a good prognosis predicts an increased likelihood of survival within a predetermined period after initial diagnosis and/or no progression of disease after primary treatment, and poor prognosis predicts an aggressive disease, a decreased likelihood of survival, an increased likelihood of biochemical recurrence, clinical recurrence, and/or the presence of local or distant metastases, within a predetermined period after initial diagnosis; or
classifying a subject as having or not having a predisposition of prostate cancer that is susceptible to disease progression based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In one embodiment, a method comprises:

classifying a subject as having a good prognosis or a poor prognosis based on a subject expression profile of the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, and wherein a good prognosis predicts an increased likelihood of survival within a predetermined period after initial diagnosis and/or no progression of disease after primary treatment, and poor prognosis predicts an aggressive disease, a decreased likelihood of survival, an increased likelihood of biochemical recurrence, clinical recurrence, and/or the presence of local or distant metastases, within a predetermined period after initial diagnosis; and
classifying a subject as having or not having a predisposition of prostate cancer that is susceptible to disease progression based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In one embodiment, a method comprises:

classifying a subject as having a good prognosis or a poor prognosis based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, and wherein a good prognosis predicts an increased likelihood of survival within a predetermined period after initial diagnosis and/or no progression of disease after primary treatment, and poor prognosis predicts an aggressive disease, a decreased likelihood of survival, an increased likelihood of biochemical recurrence, clinical recurrence, and/or the presence of local or distant metastases, within a predetermined period after initial diagnosis; or
classifying a subject as having or not having a predisposition of prostate cancer that is susceptible to disease progression based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In one embodiment, a method comprises:

classifying a subject as having a good prognosis or a poor prognosis based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, and wherein a good prognosis predicts an increased likelihood of survival within a predetermined period after initial diagnosis and/or no progression of disease after primary treatment, and poor prognosis predicts an aggressive disease, a decreased likelihood of survival, an increased likelihood of biochemical recurrence, clinical recurrence, and/or the presence of local or distant metastases, within a predetermined period after initial diagnosis; and

classifying a subject as having or not having a predisposition of prostate cancer that is susceptible to disease progression based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, wherein the predisposition predicts an aggressive disease, a decreased likelihood of survival, an increased likelihood of biochemical recurrence, clinical recurrence, and/or the presence of local or distant metastases, within a predetermined period after initial diagnosis.

In some embodiments of various aspects of the invention, the subject is a patient diagnosed with prostate cancer.

In some embodiments of various aspects of the invention, the subject expression profile is obtained from a sample from a subject.

In a particular embodiment of various aspects of the invention, the sample is cell lines.

In some embodiments of various aspects of the invention, the patient is selected from the group comprising (a) those with newly diagnosed prostate cancer, (b) those that have been subjected to primary treatment for prostate cancer.

In an embodiment of various aspects of the invention, the patient was previously diagnosed with clinically localized prostate cancer.

In an embodiment of various aspects of the invention, the primary treatment is selected from the group consisting of prostate surgery, prostate removal, radiotherapy, chemotherapy, limited or extended lymph node removal.

In an embodiment of various aspects of the invention, the patient in group (a) are further selected from patients with newly diagnosed prostate cancer that have been subjected to at least one of the following methods: determination of the blood levels of prostate-specific antigen (PSA), digital rectal exam (DRE) and transrectal ultrasound analysis (TRUS), and biopsy of the prostate.

In some embodiments of various aspects of the invention, the predetermined period is at least 6 months, at least 1 year, at least 2 years, at least 3 years, at least 4 years, at least 5 years, at least 10 years, or at least 15 years.

In some embodiments of various aspects of the invention, the gene expression level is determined by detecting mRNA expression using one or more probes and/or one or more probe sets.

In some embodiments of various aspects of the invention, the gene expression level is determined by an amplification based method and/or microarray analysis.

In some embodiments of various aspects of the invention, the gene expression level is determined by RNA sequencing, PCR, qPCR or multiplex-PCR.

In some embodiments of various aspects of the invention, the patient expression profile is converted to a prostate cancer progression index (PCPI).

In an embodiment, the prostate cancer progression index is calculated according to the following equation:

$$\mathrm{PCPI} = (\mathrm{GEV_av_GOI_up}) - (\mathrm{GEV_av_GOI_down}),$$

wherein GEV_av_GOI_up is an average gene expression value of up-regulated genes, and wherein GEV_av_GOI_down is an average gene expression value of down-regulated genes, wherein the values are determined on the basis of normalized gene expression data per each subject sample. The PCPI calculation can render a good prediction of prognosis or a good performance in other clinical applications as described herein.

In some embodiments of various aspects of the invention, the expression level of the set of the signature genes is normalized to the expression of a reference gene, preferably wherein the reference gene is a housekeeping gene, and more preferably wherein the housekeeping gene is TBP, HPRT1, ACTB, RPLPO, PUM1, POLR2A or B2M.

Described herein is also a method wherein the subject is classified as prostate cancer-positive when expression of any one or more genes selected from the group consisting of COL1A1, COL3A1, COL5A2, INHBA, THBS2, VCAN, BGN, BIRC5, DYRK2 is up-regulated as compared with the corresponding gene expression levels in a control sample, and wherein the expression of any one or more genes selected from the group consisting of AZGP1, FBLN1, ILK, KRT15, MEIS2, MYBPC1, PAGE4, SRD5A2 and optionally of PDE4D5 and/or PDE4D7 is down-regulated as compared with the corresponding gene expression levels in a control sample; or the subject is classified as prostate cancer-negative when expression of any one or more genes selected from the group consisting of COL1A1, COL3A1, COL5A2, INHBA, THBS2, VCAN, BGN, BIRC5, DYRK2 is down-regulated as compared with the corresponding gene expression levels in a control sample, and wherein the expression of any one or more genes selected from the group consisting of AZGP1, FBLN1, ILK, KRT15, MEIS2, MYBPC1, PAGE4, SRD5A2 and optionally of PDE4D5 and/or PDE4D7 is up-regulated as compared with the corresponding gene expression levels in a control sample. The selected genes can render a good performance in the classification.

In some embodiments of various aspects of the invention, the subject is classified as having a good prognosis if the prostate cancer progression index is below a selected threshold or as having a poor prognosis if the prostate cancer progression index is above the selected threshold; and/or the subject is classified as having a predisposition of prostate cancer that is susceptible to disease progression if the prostate cancer progression index is above a selected threshold or as not having a predisposition of prostate cancer that is susceptible to disease progression if the prostate cancer progression index is below the selected threshold.

In some embodiments of various aspects of the invention, the method further comprises stratifying the subject for the risk of aggressive disease versus non-aggressive disease according to the prognosis determined or the predisposition determined.

In some embodiments of various aspects of the invention, the poor prognosis or the predisposition of prostate cancer that is susceptible to disease progression is indicative of eligibility of the subject to be treated with any one or more of the treatments selected from the group consisting of prostate surgery, prostate removal, chemotherapy, radiotherapy, limited or extended lymph node dissection.

In some embodiments of various aspects of the invention, a predisposition for aggressive prostate cancer is indicative of eligibility for a treatment selected from the group comprising re-biopsy, prostate surgery, prostate removal, chemotherapy, radiotherapy, hormone therapy limited or extended lymph node dissection or combinations thereof, preferably the combination of hormone therapy and radiation therapy.

In some embodiments of various aspects of the invention, the method further comprises displaying or outputting a result of one of the steps to a user interface device, a computer readable storage medium, a monitor, or a computer that is part of a network.

A further aspect relates to the use of a product comprising:

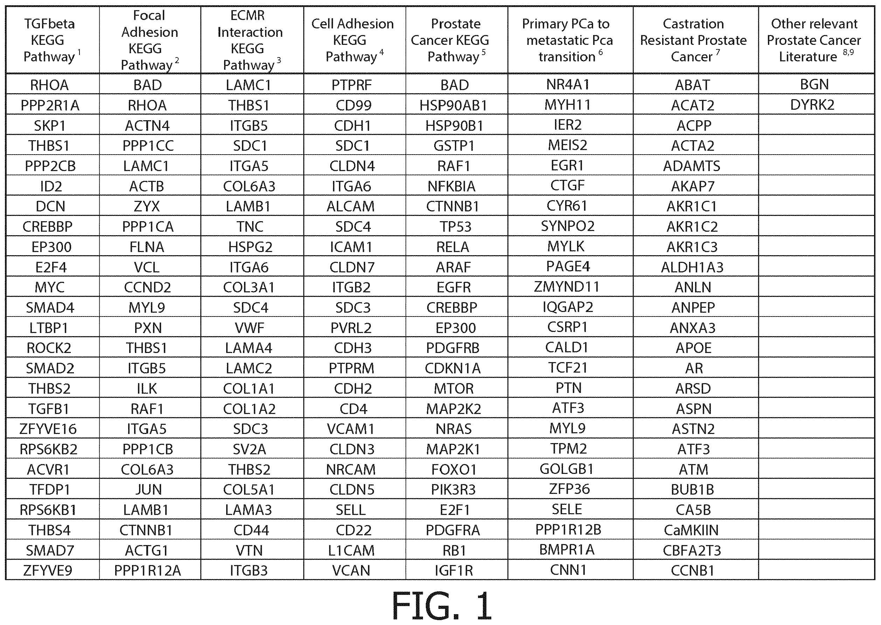

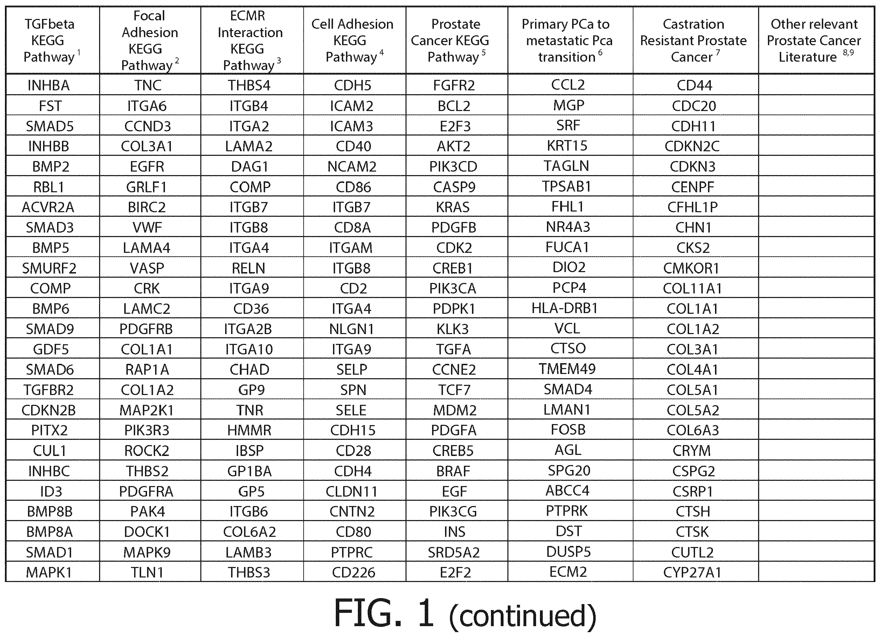

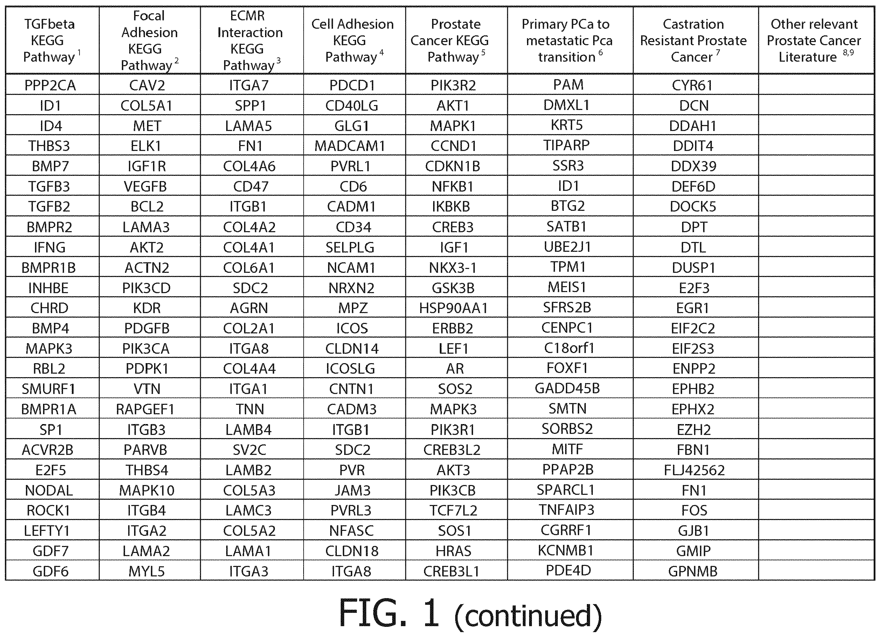

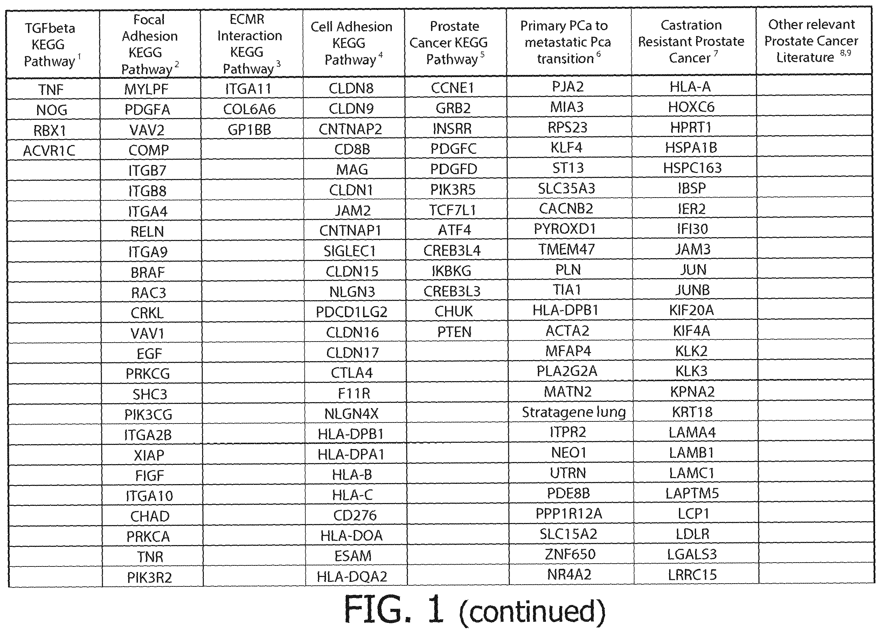

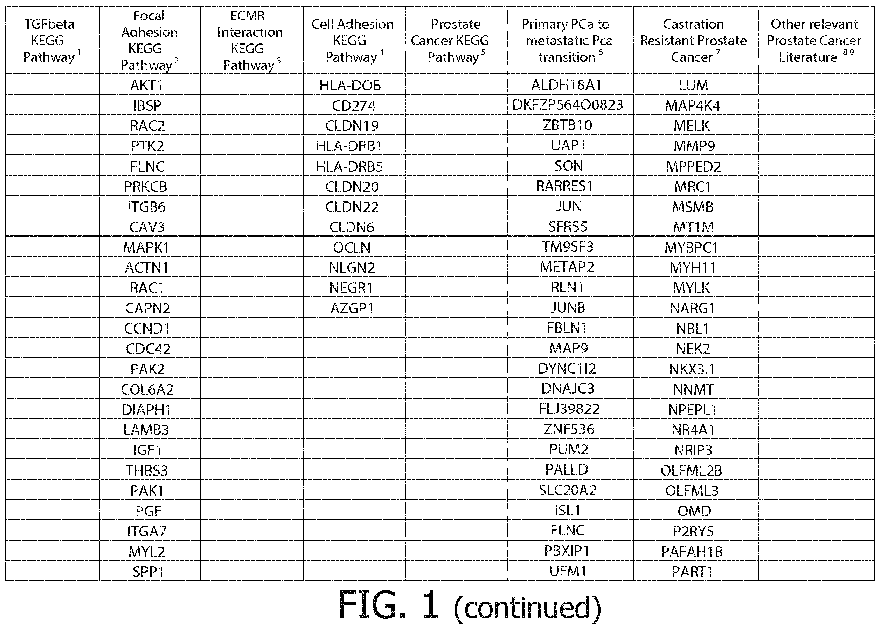

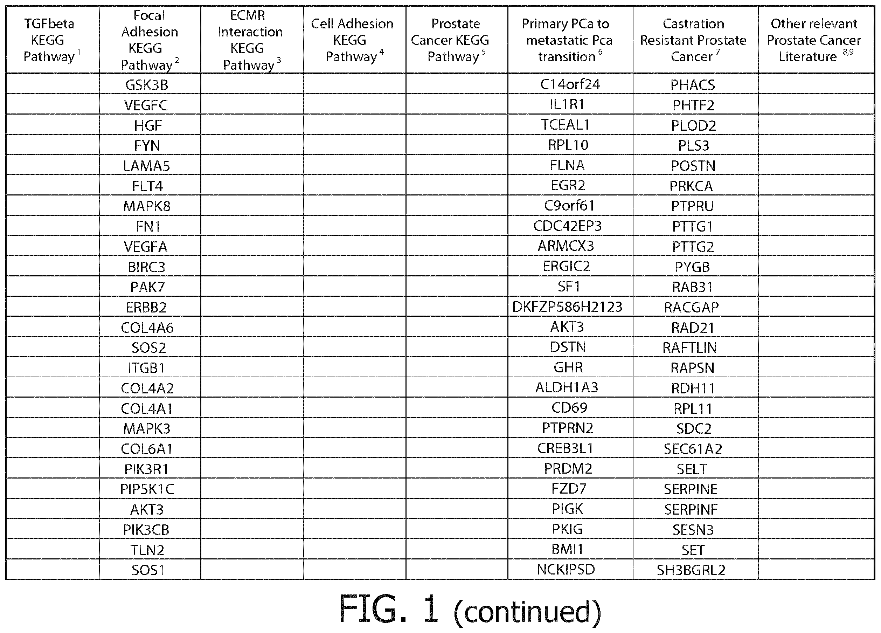

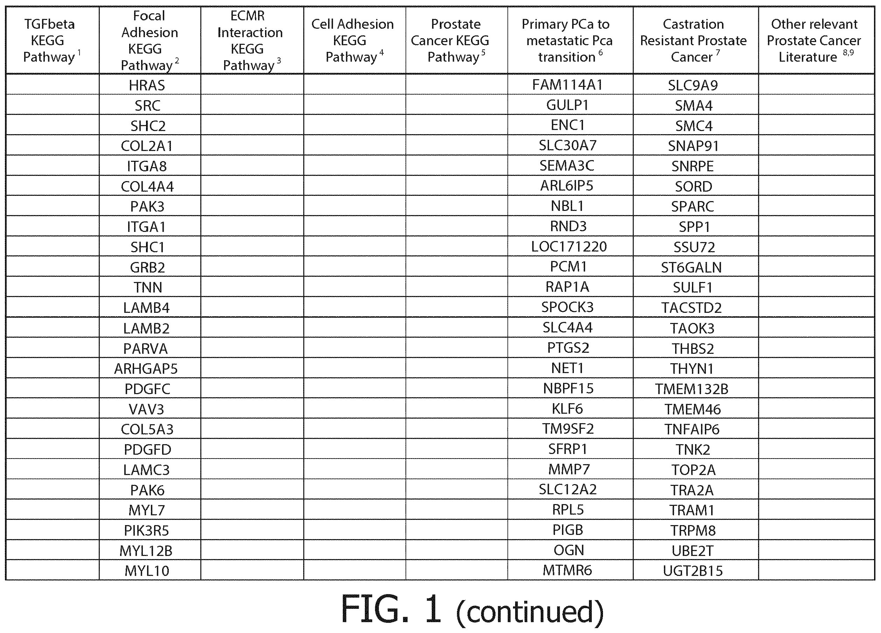

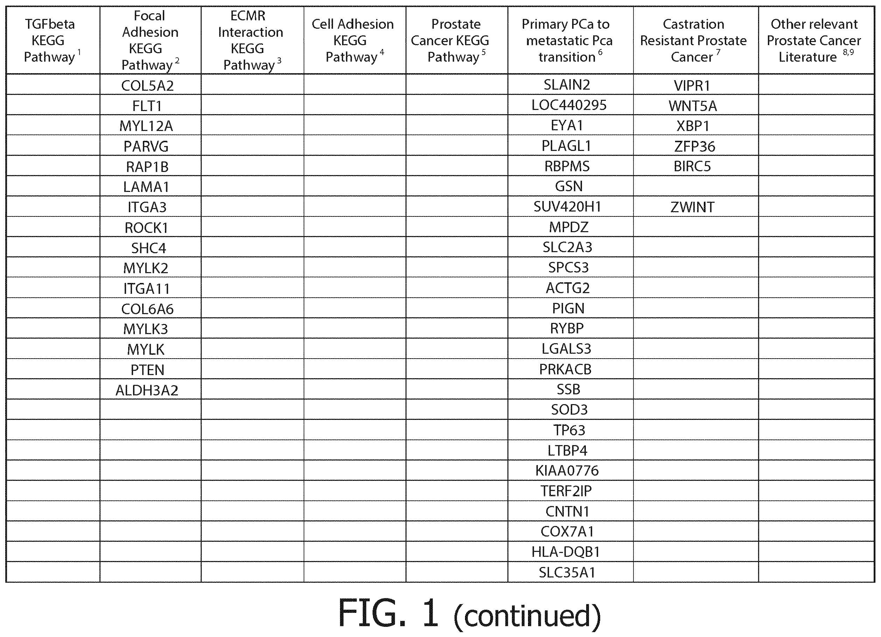

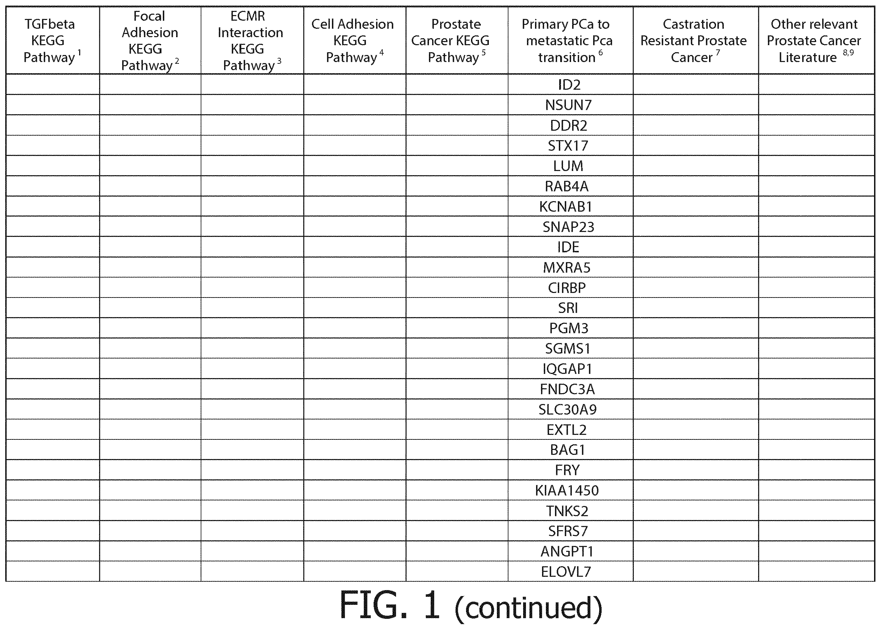

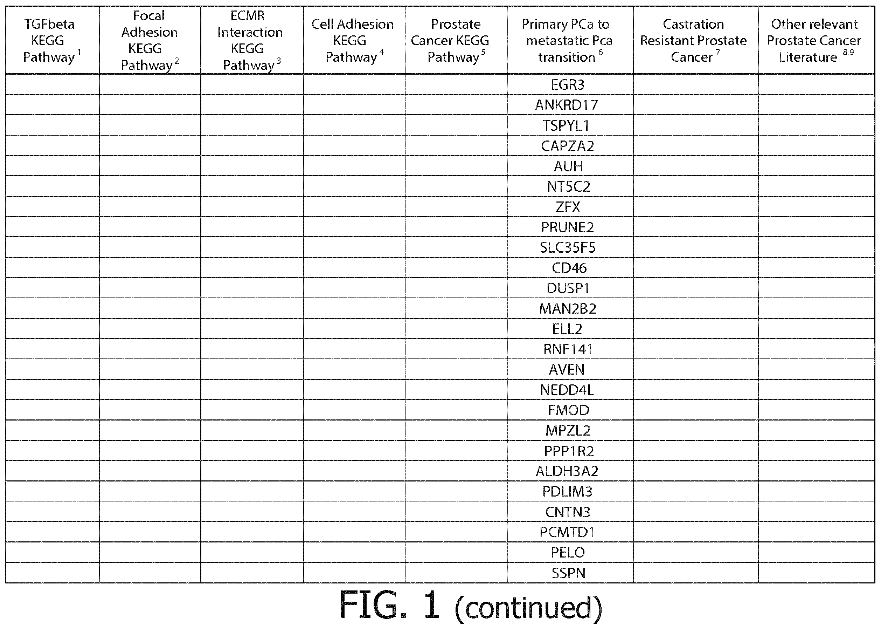

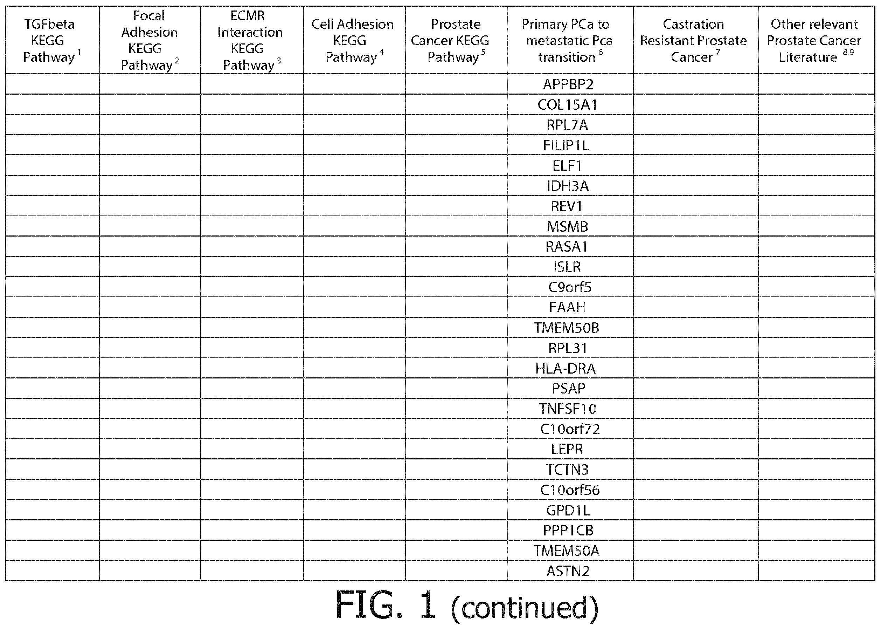

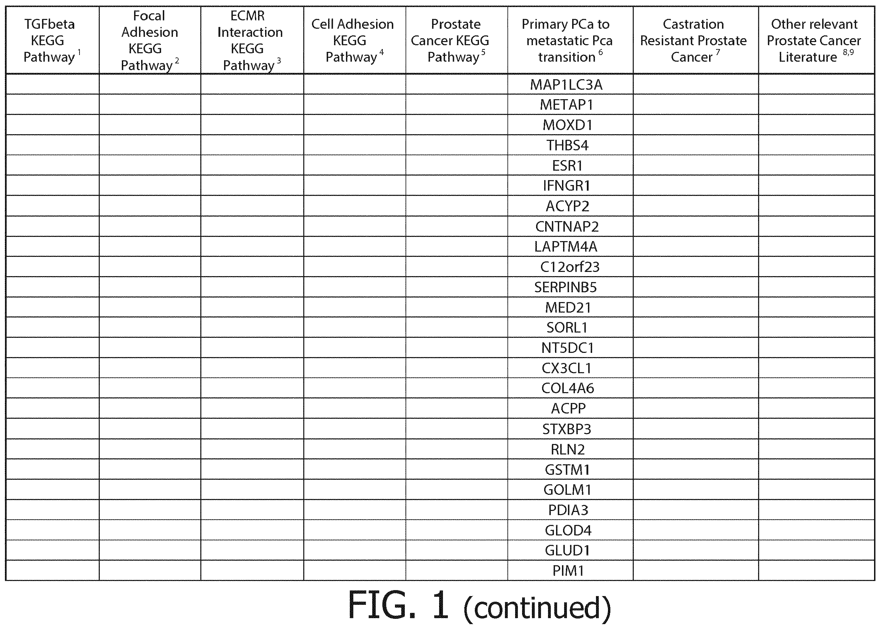

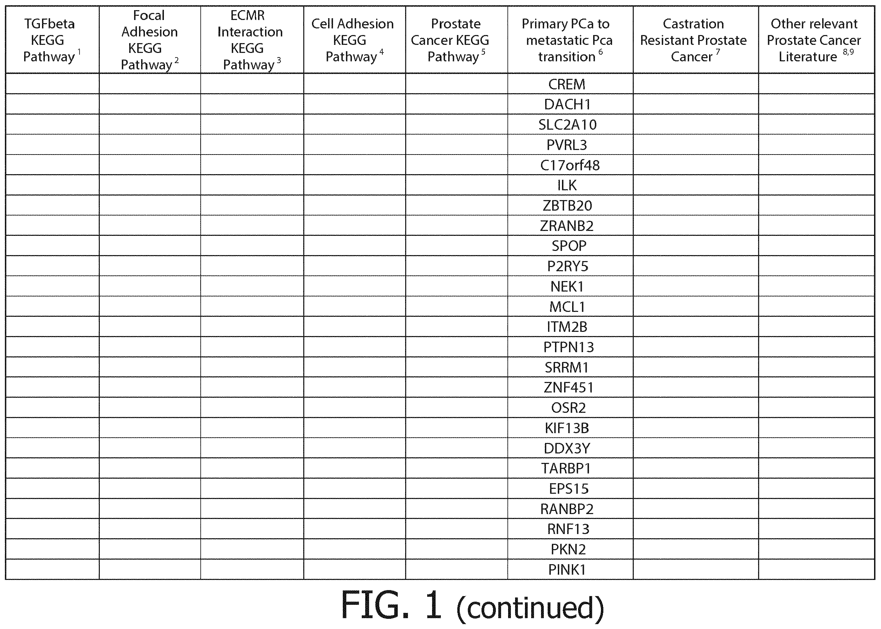

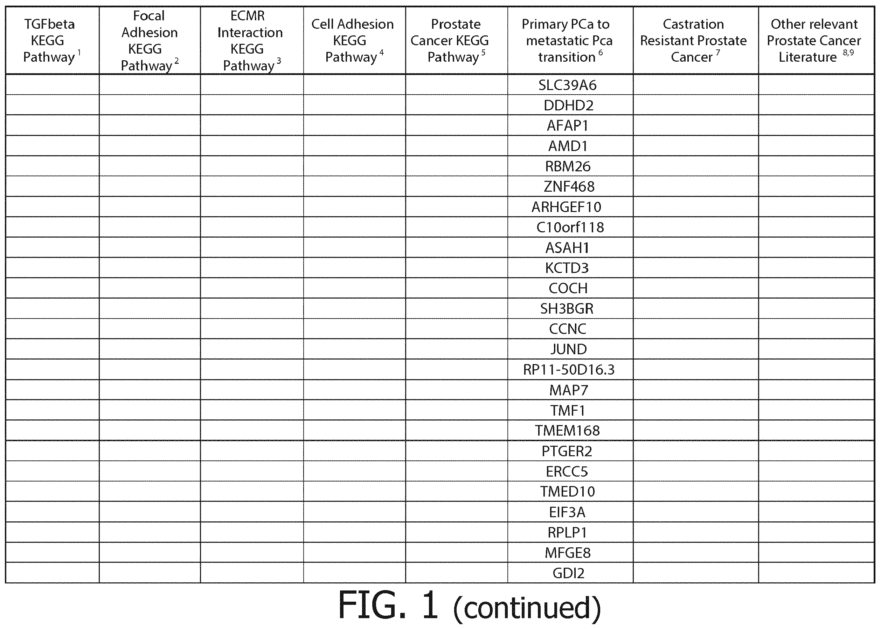

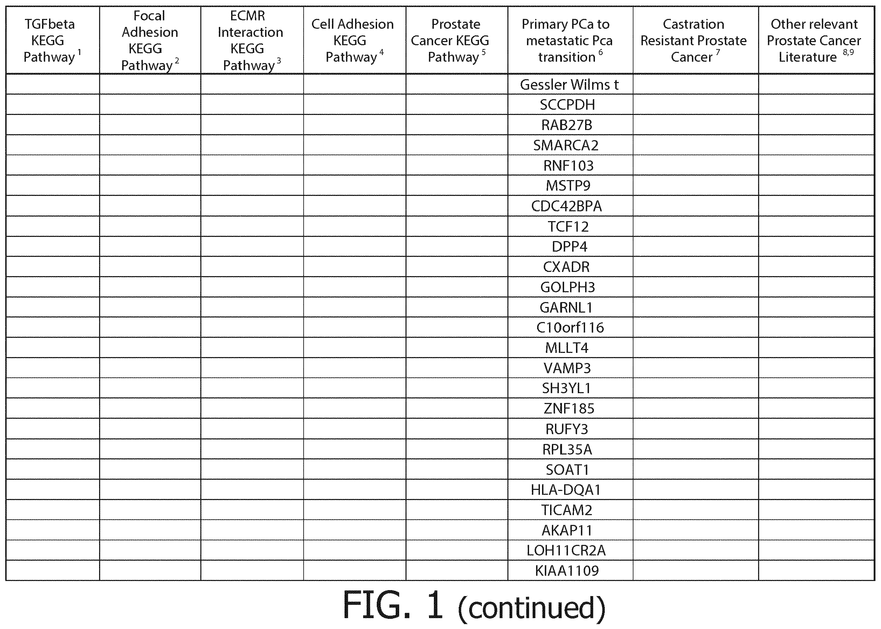

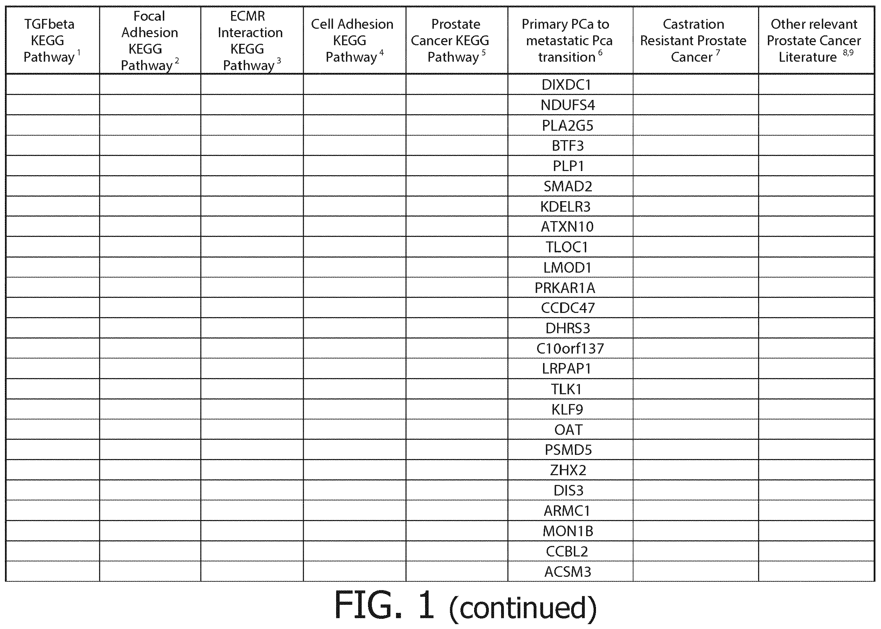

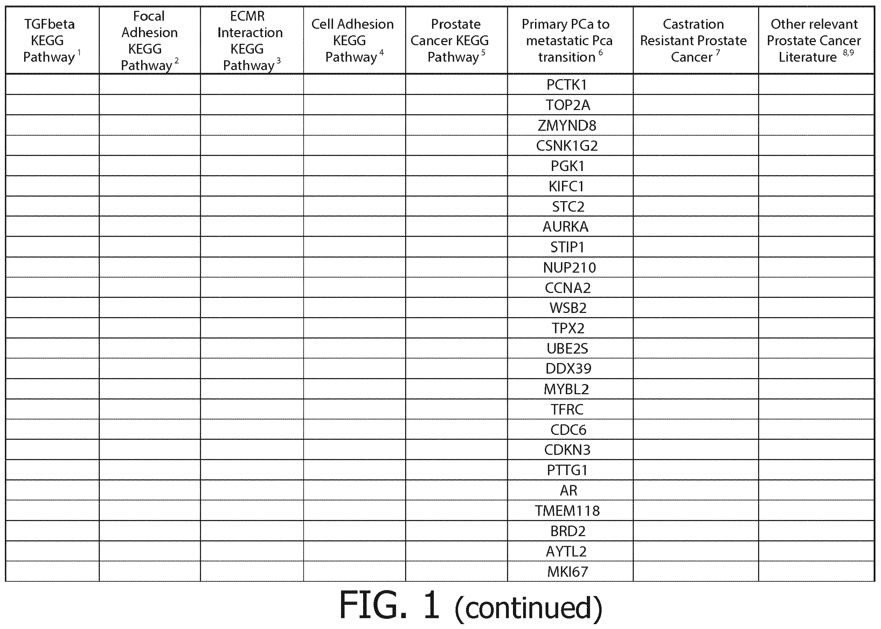

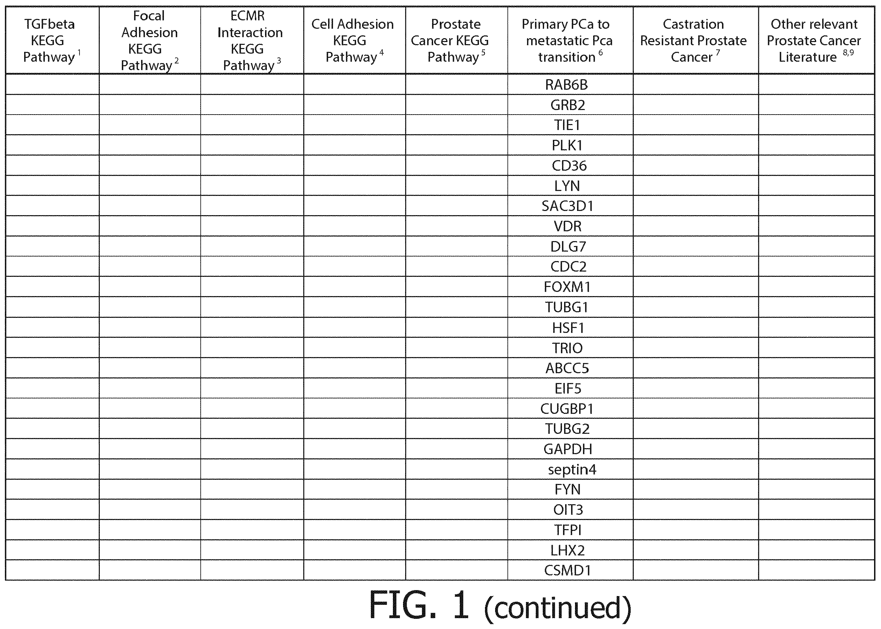

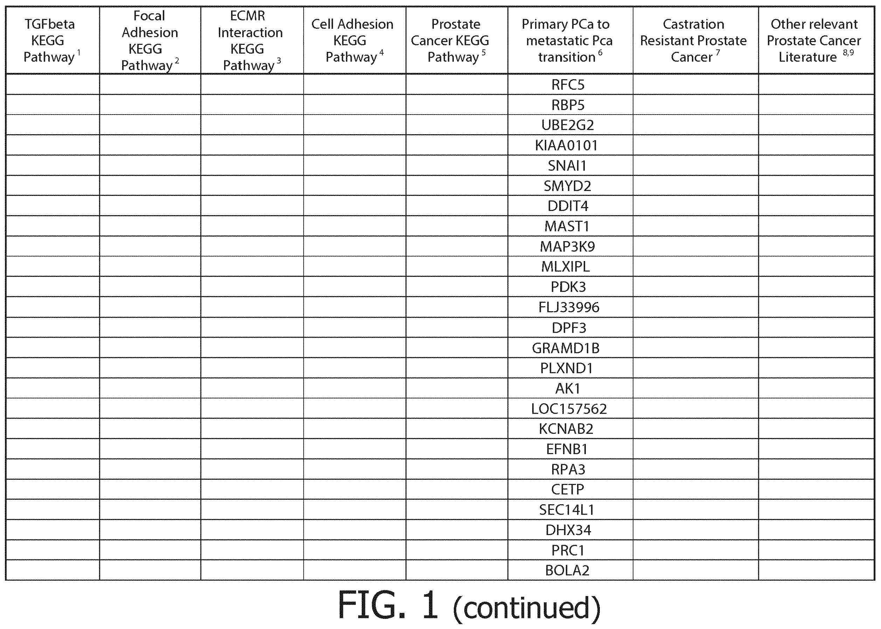

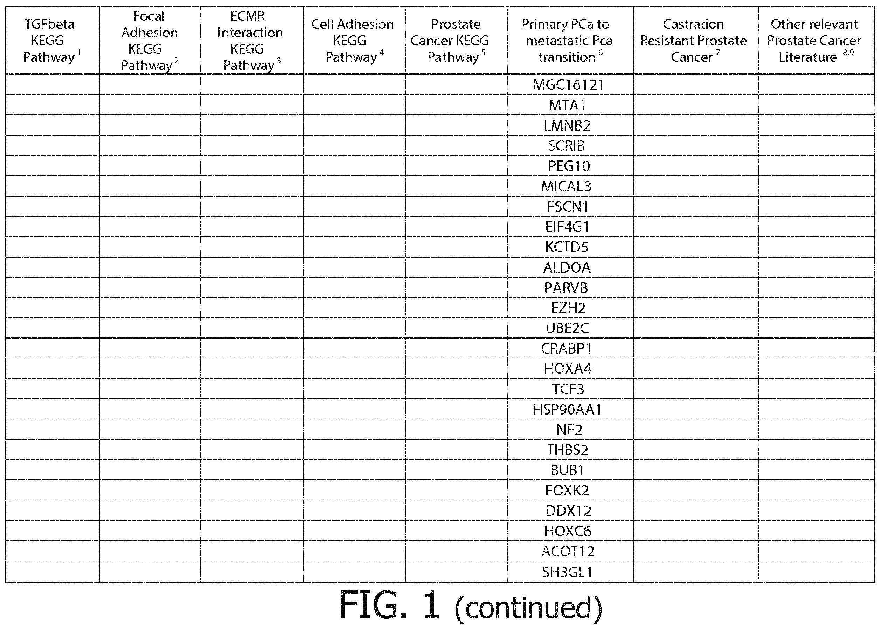

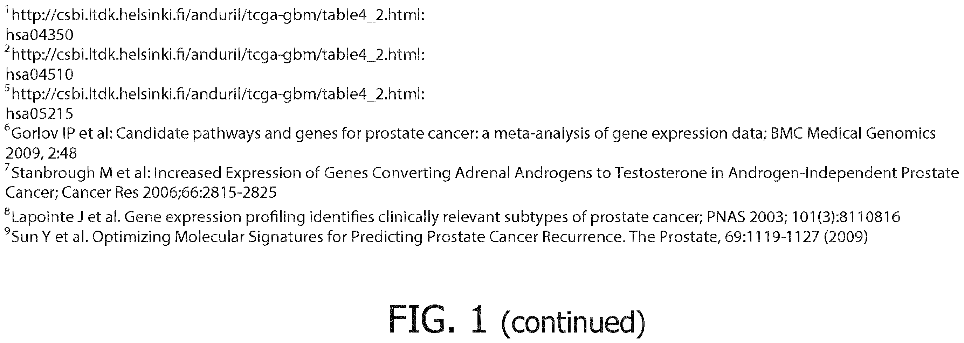

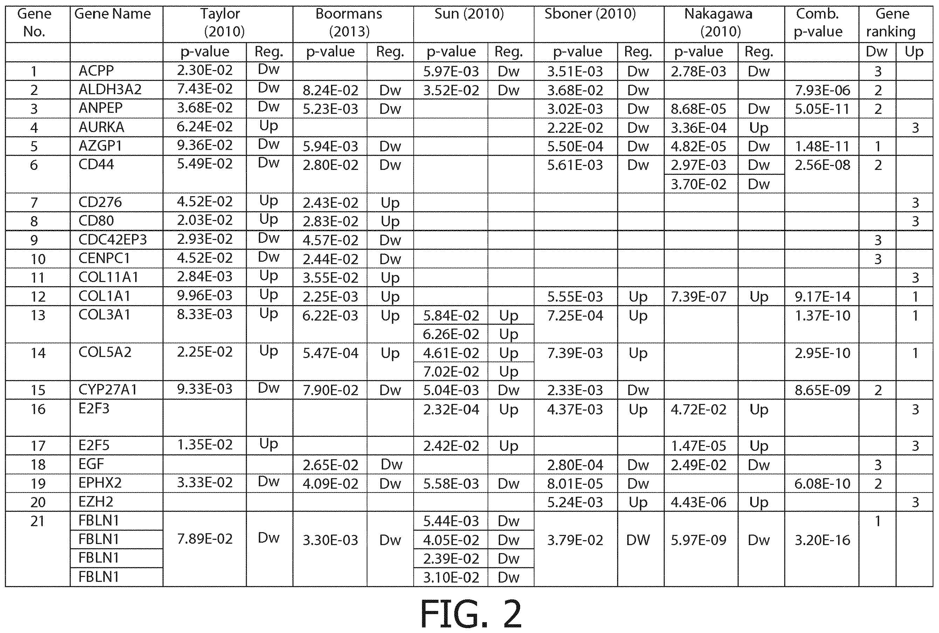

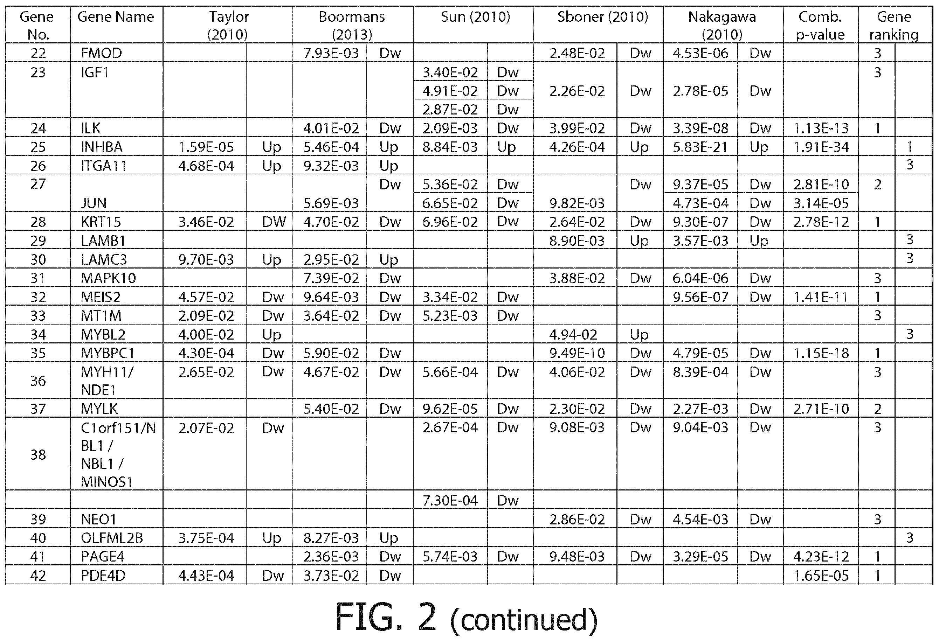

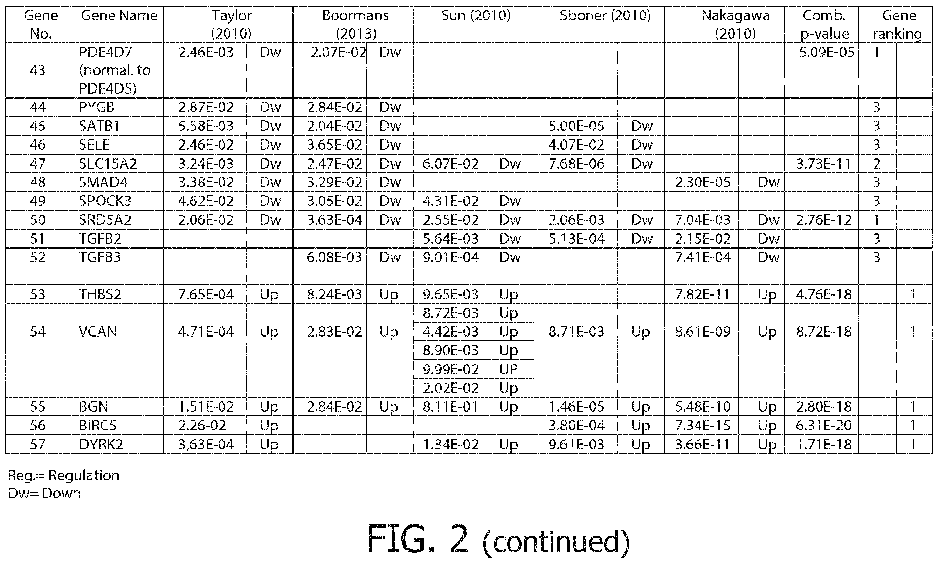

> primers and/or probes for determining the gene expression level for the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7;
> optionally further comprising primers and/or probes for determining the expression levels of a set of genes listed in either Figures 1 or 2 other than the above-mentioned genes; and/or
> optionally further comprising primers and/or probes for determining the gene expression level of a reference gene, preferably wherein the reference gene is a housekeeping gene, and more preferably wherein the housekeeping gene is TBP, HPRT1, ACTB, RPLPO, PUM1, POLR2A or B2M
> for establishing a prognosis for a patient diagnosed with prostate, for stratification of a patient diagnosed with prostate cancer, for the determination of predisposition for aggressive or indolent prostate cancer in a patient having a prostate cancer..

In some embodiments, the product is a kit including for example a PCR kit, a RNA-sequencing kit, or a microarray kit preferably a DNA microarray kit. In another embodiment, the product is a microarray. The product can provide an efficient way for determining the levels as described herein and thus can render a good performance in clinical applications as described herein.

In some embodiments, the product is a product for performing the method of the invention, or the product is a product for diagnosis of prostate cancer, for establishing a prognosis for a patient diagnosed with prostate, for stratification of a patient diagnosed with prostate cancer, for the determination of predisposition for aggressive or indolent prostate cancer in a patient having a prostate cancer.

In some embodiments, the product is a composition comprising a set of nucleic acid molecules each comprising at least one polynucleotide probe sequence in the analysis of the gene expression of the genes comprised by any one of Figures 1 or 2, or the genes as described above.

In some embodiments, the product is an nucleic acid array comprising for each gene in a set of genes, the set of genes comprising the genes listed in Figures 1 or 2, or the genes as described above, one or more polynucleotide probes complementary and hybridizable to a coding sequence in the gene, for determining a prostate expression profile and/or a PCPI as defined above.

In some embodiments, the product is a kit for establishing a prognosis for a patient diagnosed with prostate, for stratification of a patient diagnosed with prostate cancer, for the determination of predisposition for aggressive or indolent prostate cancer in a patient having a prostate cancer according any one of methods as described herein comprising: a) an array as defined herein, b) a kit control; and c) optionally instructions for use.

In some embodiments, the product is a nucleic acid array for the expression analysis of nucleic acids obtained from a biological sample, wherein said array comprises a substrate comprising probes suitable for the specific binding of nucleic acids or fragments thereof encoded by the genes referred to in Figures 1 or 2, or the genes as described above.

In some embodiments, the product is an article for PCR comprising reagents for specifically amplifying and detecting nucleic acids or fragments thereof encoded by the genes referred to of Figures 1 or 2, or the genes as described above.

A further aspect of the invention relates to a computer program product, comprising computer readable code stored on a computer readable medium or downloadable from a communications network, which, when run on a computer, implement one or more steps or all the steps of any one of the methods as described herein.

A further aspect of the invention relates to a non-transitory computer readable storage medium with an executable program stored thereon, wherein the program instructs a microprocessor to perform one or more of the steps of any of the methods of the invention.

A yet further aspect of the invention relates to a device for gene expression analysis of nucleic acids (or fragments thereof) encoded by genes referred to in Figures 1 or 2, or the genes described above, wherein the device preferably comprises:

a) a database including records comprising reference gene expression values associated with clinical outcomes, each reference profile comprising the expression levels of a set of genes listed in either Figures 1 or 2, or the genes as described above, and/or

b) a user interface capable of receiving and/or inputting a selection of gene expression values of a set of genes, the set comprising genes listed in Figures 1 or 2, or the genes as described above, for use in comparing to the gene reference expression profiles in the database;

c) an output that displays a prediction of clinical prognosis according to the expression levels of the set of genes.

A yet further aspect of the invention relates to a device for performing the method of the invention.

A still further aspect of the invention relates to a system comprising the product described above and the computer program product or the non-transitory computer readable storage medium of the invention or the device for gene expression analysis of nucleic acids of the invention or a device for performing the method of the invention.

BRIEF DESCRIPTION OF THE DRAWINGS

Fig. 1 shows a table comprising gene candidates analyzed which are derived from publically available study data.

Fig. 2 shows a table comprising 57 gene candidates analyzed to build the PCPI.

Fig. 3 shows an overview on the performance of PCPI_18 or PCPI_17 to predict the primary endpoint of development of distant metastases within 10-15 years after primary treatment (prostate surgery) for the data sets GSE21032, GSE41408, GSE25136, GSE16560, GSE10645.

Fig. 4 shows the performance of PCPI_18 to predict the primary endpoint of development of distant metastases within 10-15 years after primary treatment for the data set GSE46691.

Fig. 5 shows the performance of PCPI_18 or PCPI_17 to predict the primary endpoint of development of distant metastases within 10-15 years after primary treatment (prostate surgery in general) for the data sets GSE21032, GSE41408, GSE16560, GSE10645 in a multivariate COX regression analysis compared to standard clinical parameters like pretreatment PSA, biopsy or pathology Gleason score, or clinical/pathology disease stage. The Chi Square as well as the Hazard Ratio for the PCPI and the individual clinical parameters is shown for each individual data set.

Fig. 6 shows an overview on the performance of PCPI_18 to predict the primary endpoint of development of biochemical disease recurrence (BCR) within 10-15 years after primary treatment (prostate surgery in general) for the data sets GSE21032, GSE41408, GSE25136.

Fig. 7 shows the performance of PCPI_18 to predict the primary endpoint of development of biochemical disease recurrence (BCR) within 10-15 years after primary treatment (prostate surgery in general) for the data sets GSE21032, GSE41408, GSE25136 in a multivariate COX regression analysis compared to standard clinical parameters like pretreatment PSA, biopsy or pathology Gleason score, or clinical/pathology disease stage.

Fig. 8 shows a ROC curve cut-off analysis for PCPI_18 to support clinical decision making towards e.g. patient stratification for either active surveillance (AS) or active treatment (e.g., prostatectomy, radiotherapy and hormone therapy).

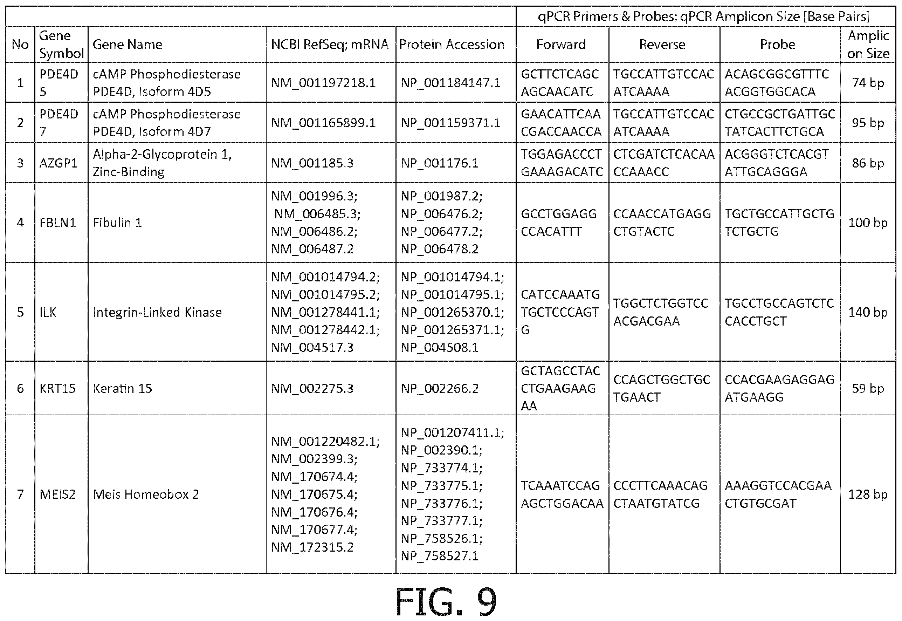

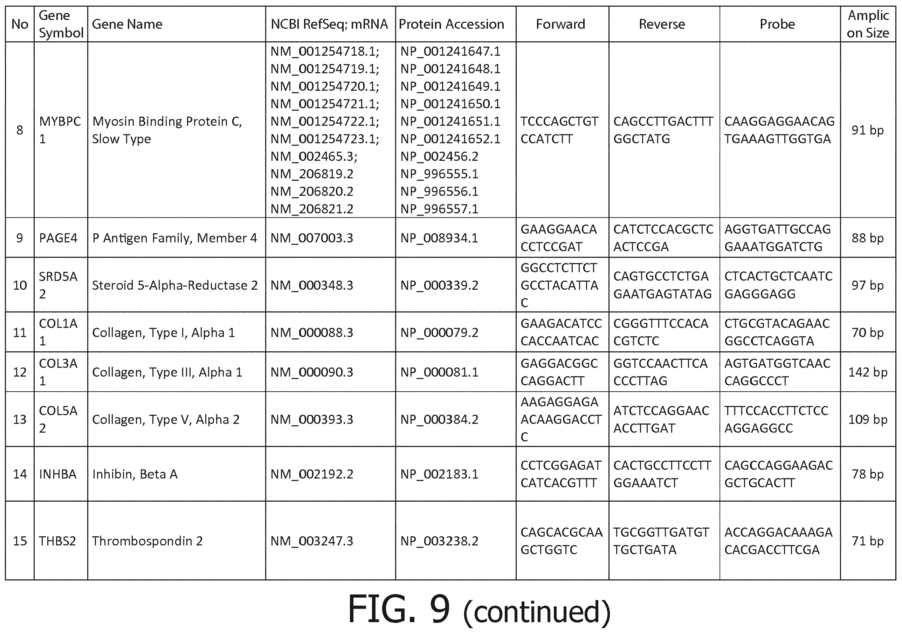

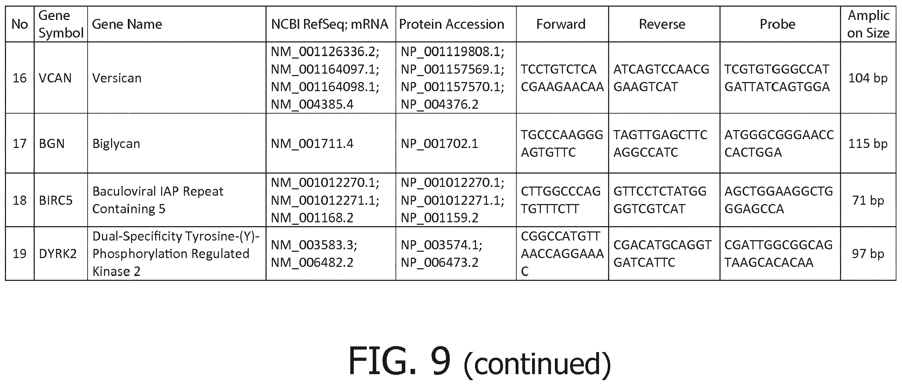

Fig. 9 lists genes of interest mentioned in this application with their Transcript ID, Protein ID as well as primer and probe sequences specific for the genes of interest.

Fig. 10 lists reference genes mentioned in this application with their Transcript ID, Protein ID as well as primer and probe sequences specific for the reference genes.

Fig. 11 shows an analysis of different recurrence risks over 5 to 10 years for 422 patients grouped according to the PCPI_19 and the NCCN clinical risk characteristics as can be inferred from the guidelines of the National Comprehensive Cancer Network's (NCCN) webpage (https://www.nccn.org/professionals/physician_gls/f_guidelines.asp).

Fig. 12 shows an analysis of different recurrence risks over 5 to 10 years for 407 patients grouped according to the GPSu score and the NCCN clinical risk characteristics as can be inferred from the guidelines of the National Comprehensive Cancer Network's (NCCN) webpage (https://www.nccn.org/professionals/physician_gls/f_guidelines.asp).

Fig. 13 shows an analysis of different recurrence risks over 5 to 10 years for 407 patients grouped according to the CCP score and the NCCN clinical risk characteristics as can be inferred from the guidelines of the National Comprehensive Cancer Network's (NCCN) webpage (https://www.nccn.org/professionals/physician_gls/f_guidelines.asp).

DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of' is considered to be a preferred embodiment of the term "comprising of'. If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention is not limited to the particular methodology, protocols, proteins, bacteria, vectors, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The term "signature gene(s)", marker gene(s), or "gene of interest (GOI)" in the context of the present invention can be used interchangeably and designates biomarkers whose expression level is determined in the methods of the present invention. In preferred embodiments of the present invention, these genes are depicted in Figure 2 of the present disclosure, and referred to explicitly below as well as in the claims. Therefore, the present invention concerns marker gene(s), the expression of which is either up-regulated or down-regulated when comparing the expression in prostate cancer tissue obtained after primary treatment or after initial prostate cancer diagnosis to the expression in prostate cancer tissue obtained before initial prostate cancer diagnosis and/or before initial prostate cancer-specific treatment. The marker gene expression in tissues obtained after primary treatment or after initial prostate cancer diagnosis may be correlated to patient outcome parameters, e.g. biochemical recurrence, development of metastasis, bone cancer and/or death.

When the expression values are determined in the context of the methods of the present invention, an individualized "gene expression profile" is obtained that reflects the gene expression values of the GOI's in a sample or in subject. In the latter case, the expression values are also referred to as "subject expression profile". It is noted that the sample expression profile and/or the subject expression profile may change as a function of time. For example, the gene expression profiles before or after therapeutic treatment of a patient with diagnosed prostate cancer may be different. Similarly, the genes expression profiles found in a sample containing prostate cancer cells will be different from those found in a sample that does not contain prostate cancer cells.

The term "marker gene(s)" or "gene(s) of interest" as used herein, thus relates to a set of gene(s), genetic unit(s) or sequence(s) (nucleotide sequence(s)) as defined herein above, whose expression level is either up-regulated or down-regulated in a prostate cancer cell or tissue or in any type of sample comprising such cells or tissues or portions or fragments thereof, when comparing to a control level, preferably when comparing to the expression in prostate tissue obtained at a different point in time, e.g. at initial diagnosis, directly after primary treatment, or later, i.e. several months or years after initial diagnosis or primary treatment. The term refers to any expression product of said genetic unit or sequence, in particular to an mRNA transcript. When the marker genes or genes of interest are examined for the first time, i.e. when a patient is for the first time suspected to have a prostate cancer, it is also contemplated to use additional control material that is derived from normal tissue or benign prostate tumor tissue.

The term "expression level" as used herein refers to the amount of marker gene transcript(s) derivable from a defined number of cells or a defined tissue portion, preferably to the amount of transcript obtainable in a standard nucleic acid (e.g. RNA) extraction procedure. Suitable extraction methods are known to the person skilled in the art.

The term "control level" (or "control state"), as used herein, relates to an expression level which may be determined at the same time and/or under similar or comparable conditions as the test sample by using (a) sample(s) previously collected and stored from a patient/patients whose condition or disease state, e.g. non-cancerous, normal or benign prostate tumor, advanced prostate cancer etc. is/are known. For the determination of a predisposition for aggressive cancer versus indolent cancer, the control level is preferably determined in cancer tissue obtained to establish the initial diagnosis, which is then compared to the expression of GOI's that is determined at a later stage, e.g. after primary treatment. The term "disease state" or "cancerous disease state" relates to any state or type of cellular or molecular condition between a non-cancerous cell state and (including) a terminal cancerous cell state. Preferably, the term includes different cancerous proliferation/developmental stages or levels of tumor development in the organism between (and excluding) a non-cancerous cell state and (including) a terminal cancerous cell state. Such developmental stages may include all stages of the TNM (Tumor, Node, Metastasis) classification system of malignant tumors as defined by the UICC, e.g. stages 0 and I to IV. The term also includes stages before TNM stage 0, e.g. developmental stages in which cancer biomarkers known to the person skilled in the art show a modified expression or expression pattern.

The expression level as mentioned above may preferably be the expression level of marker gene(s) or GOI's as defined herein.

The term "cancerous" relates in the context of the present invention to a cancerous disease state as defined herein above.

The term "non-cancerous" relates in the context of the present invention to a condition in which neither benign nor malign proliferation can be detected. Suitable means for said detection are known in the art.

The expression control level may be determined by a statistical method based on the results obtained by analyzing previously determined expression level(s) of the marker gene(s) of the present invention in samples from subjects whose disease state is known. Furthermore, the control level can be derived from a database of expression patterns or expression levels from previously tested patients, tissues or cells. The control level can be determined from a reference sample derived from a patient who has been diagnosed to suffer from prostate cancer, e.g. from hormone-independent or hormone-resistant prostate cancer. Moreover, the expression level of the marker genes of the present invention in a biological sample to be tested may be compared to multiple control levels, whose control levels are determined from multiple reference samples. It is contemplated to use a control level determined from a reference sample derived from a tissue type similar to that of the subject-derived biological sample. It is particularly preferred to use sample(s) derived from a patient whose disease state is cancerous as defined herein above.

In general diagnostic methods of prostate cancer using the herein described marker genes, it also possible to determine the control level in tissues of subjects that present a health condition in which neither benign nor malign proliferation can be detected. It is also contemplated to use sample(s) derived from a patient/patients having benign prostate tumor as defined herein above, i.e. which present a health condition in which benign proliferation can be detected.

The term "benign prostate tumor" as used herein refers to a prostate tumor which lacks all three of the malignant properties of a cancer, i.e. does not grow in an unlimited, aggressive manner, does not invade surrounding tissues, and does not metastasize. Typically, a benign prostate tumor implies a mild and non-progressive prostate neoplastic or swelling disease lacking the invasive properties of a cancer. Furthermore, benign prostate tumors are typically encapsulated, and thus inhibited in their ability to behave in a malignant manner. A benign tumor or a healthy condition may be determined by any suitable, independent molecular, histological or physiological method known to the person skilled in the art.

Alternatively, reference samples may comprise material derived from cell lines, e.g. immortalized cancer cell lines. Preferably, material derived from prostate cancer cell lines, may be comprised in a reference sample according to the present invention. Examples of preferred cancer cell lines comprise cells lines PC346P, PC346B, LNCaP, VCaP, DuCaP, PC346C, PC3, DU145, PC346CDD, PC346Flul, PC346Flu2.

In a further, preferred alternative, reference samples may be derived from subject/patient tissues, or tissue panels or tissue collections obtained in clinical environments. The samples may, for example, be obtained from male patients undergoing surgery. The samples may be derived from any suitable tissue type, e.g. from prostate tissue or lymph nodes. Preferred examples of patient tissue collections are derived from surgical procedures (e.g., prostatectomy).

Moreover, it is contemplated to use the standard value of the expression levels of the marker gene(s) of the present invention in a population with a known disease state, e.g. a population having benign prostate tumor or a healthy population. The standard value may be obtained by any method known in the art. For example, a range of mean ± 2 SD (standard deviation) or mean ± 3 SD may be used as standard value.

Furthermore, the control level may also be determined at the same time and/or under similar or comparable conditions as the test sample by using (a) sample(s) previously collected and stored from a patient/patients whose disease state is/are known to be cancerous, i.e. who have independently been diagnosed to suffer from prostate cancer, in particular from aggressive prostate cancer.

In the context of the present invention, a control level determined from a biological sample that is known not to be cancerous, e.g. is a healthy tissue sample or a benign prostate tumor sample, is called "normal control level".

If the control level is determined from a cancerous biological sample, in particular a sample from a patient for which prostate cancer was diagnosed independently, it may be designated as "cancerous control level".

The term "prostate cancer" relates to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 4.0. In another embodiment the term relates to cancer showing PSA levels above 2.0. The term "PSA level" refers to the concentration of PSA in the blood in ng/ml.

The term "prostate cancer" also comprises prostate cancer or prostate cancer cell lines that are sensitive on male sex hormone stimulation as far as their growth or proliferation is concerned. The term "sensitive" relates to situations in which the prostate cancer or prostate cancer cell line shows a biochemical or cellular reaction pattern in the presence of male sex hormones, but does need a male sex hormone for growth and/or proliferation. A hormone-sensitive prostate is accordingly understood as a malignant prostate tumor that has developed from pre-malignant forms like PIN (Prostate Intraepithelial Neoplasia) and is characterized by the fact that its growth is still dependent on the presence of the male sex hormones androgens. In contrast, once such tumors are treated by hormone depletion therapies those cancers typically develop into a hormone-resistant form that grows independent of the presence of androgens.

The term "hormone-resistant prostate cancer" means that the growth and proliferation of prostate cancer or prostate cancer cell lines is resistant to male sex hormone stimulation. The term also relates to a late prostate cancer developmental stage which is no longer amenable to an administration of anti-hormones, preferably anti-androgens as defined herein above.

The term "male sex hormone" as used herein refers to an androgen, preferably to testosterone, androstenedione, dihydrotestosterone, dehydroepiandrosterone, androstenediol or androsterone.

In a further aspect the present invention relates to the use of marker gene(s) or GOI's as a marker for diagnosing, detecting, monitoring or prognosticating malignant, hormone-sensitive prostate cancer or the progression towards prostate cancer.

The term "up-regulated" or "up-regulated expression level" or "increase of expression level" (which may be used synonymously) in the context of the present invention thus denotes a raise in the expression level between a situation to be analyzed, e.g. a situation derivable from a patient's sample, and a reference point, which could either be a normal control level or cancerous control level derivable from any suitable prostate tumor or cancer stage known to the person skilled in the art. Expression levels are deemed to be "up-regulated" when the gene expression, e.g. in a sample to be analyzed, differs by, i.e. is elevated by, for example, 5%, 6%, 7%, 8%, 9%, 10%, 15%, 20%, 25%, 30%, 40%, 50%, or more than 50% in comparison to a control level, or by at least 0.1 fold, at least 0.2 fold, at least 1 fold, at least 2 fold, at least 5 fold, or at least 10 fold or more in comparison to a control level. The control level may either be a normal control level or a cancerous control level as defined herein above. If a comparison with a cancerous control level is to be carried out, an additional comparison with a normal control level is preferred. Such an additional comparison allows for the determination of a tendency of the modification, e.g. the magnitude of an increase of the expression level may be observed and/or corresponding conclusions may be drawn. Preferred is a comparison to a benign prostate tumor, or to a healthy tissue or a sample derived from a healthy individual.

The term "down-regulated" or "down-regulated expression level" or "decrease of expression level" (which may be used synonymously) in the context of the present invention thus denotes a drop in the expression level between a situation to be analyzed, e.g. a situation derivable from a patient's sample, and a reference point, which could either be a normal control level or preferably cancerous control level derivable from any suitable prostate tumor or cancer stage known to the person skilled in the art. Expression levels are deemed to be "down-regulated" when the gene expression, e.g. in a sample to be analyzed, differs by, i.e. is decreased by, for example, 5%, 6%, 7%, 8%, 9%, 10%, 15%, 20%, 25%, 30%, 40%, 50%, or more than 50% in comparison to a control level, or by at least 0.1 fold, at least 0.2 fold, at least 1 fold, at least 2 fold, at least 5 fold, or at least 10 fold or more in comparison to a control level. The control level may either be a normal control level or a cancerous control level as defined herein above. If a comparison with a cancerous control level is to be carried out, an additional comparison with a normal control level is possible. Such an additional comparison allows for the determination of a tendency of the modification, e.g. the magnitude of decrease of the expression level maybe observed and/or corresponding conclusions may be drawn. Preferred is a comparison to a benign prostate tumor, or to a healthy tissue or a sample derived from a healthy individual.

The expression values of the gene(s) of interest referred to herein can be used to establish a "Prostate Cancer Progression Index (PCPI)" using the following equation:

$$PCPI = (GEV_av_GOI_up) - (GEV_av_GOI_down),$$

wherein GOI refers to selected Genes of Interest, e.g. (bio-)marker genes, which are grouped into those down-regulated between patient groups with and without progression after primary treatment compared to those up-regulated between patient groups with and without progression after primary treatment. An average gene expression value for the down-regulated genes is calculated (GEV_av_GOI_down) as well as an average gene expression value for the up-regulated genes (GEV_av_GOI_up).

A specific embodiment a subject sample specific prostate cancer progression index is calculated according to the following equation:

$$PCPI = (GEV_av_GOI_up) - (GEV_av_GOI_down),$$

wherein GEV_av_GOI_up is an average gene expression value of up-regulated genes, and wherein GEV_av_GOI_down is an average gene expression value of a down-regulated genes, wherein the values are determined on the basis of normalized gene expression data per each patient sample and wherein "up-regulated genes" refer to genes up-regulated between patient groups with and without progression after primary treatment and "down-regulated genes" refer to genes down-regulated between patient groups with and without progression after primary treatment.

A selection of genes potentially relevant to patient stratification in prostate cancer or to determine the predisposition to develop aggressive prostate cancer after primary treatment is provided e.g. in Figure 2. Figure 2 provides an overview of 57 selected genes that were found to be significantly ($p<0.05$) differentially expressed and were regulated between patient groups in the same direction (i.e., either up- or down-regulated). These 57 genes may be used as GOI's according to one aspect of the invention. It is also possible to use selected genes from the above 57 genes, e.g. at least 17 genes, at least 18 genes, at least 19 genes, at least 20 genes, at least 21 genes, at least 22 genes, at least 23 genes, at least 24 genes, at least 25 genes, at least 26 genes, at least 27 genes, at least 28 genes, at least 29 genes, at least 30 genes, at least 31 genes, at least 32 genes, at least 33 genes, at least 34 genes, at least 35 genes, at least 36 genes, at least 37 genes, at least 38 genes, at least 39 genes, at least 40 genes, at least 41 genes, at least 42 genes, at least 43 genes, at least 44 genes, at least 45 genes, at least 46 genes, at least 47 genes, at least 48 genes, at least 49 genes, at least 50 genes, or more.

In another aspect a gene expression prognostic signature comprised of a combination of individual genes is provided, wherein the expression level of these GOI's can be measured using, e.g. RNA extracted from human prostate cancer tissue samples in a quantitative manner. The combination of these features into a single data model (i.e. the PCPI) provides significantly improved classification power to predict the progression of prostate cancer.

In one preferred embodiment the PCPI signature comprises the following 19 genes: PDE4D5, PDE4D7, AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2.

The term "phosphodiesterase 4D5" or "PDE4D5" relates to the splice variant 5 of the human phosphodiesterase PDE4D, i.e. the human phosphodiesterase PDE4D5 gene, preferably to the sequence as defined in NCBI Reference Sequence: NM_001197218.1, more preferably to the nucleotide sequence as set forth in SEQ ID NO: 1, which corresponds to the sequence of the above indicated NCBI Reference Sequence of the PDE4D5 transcript, and also relates to the corresponding amino acid sequence as set forth in SEQ ID NO: 2, which corresponds to the protein sequence defined in NCBI Protein Accession Reference Sequence NP_001184147.1 encoding the PDE4D5 polypeptide. The term "phosphodiesterase 4D5" or "PDE4D5" also relates to the amplicon that can be generated by the primer pair PDE1D5_forward (SEQ ID NO: 3) and the PDE1D5_reverse (SEQ ID NO: 4) and can be detected by probe SEQ ID NO: 5.

The term "phosphodiesterase 4D7" or "PDE4D7" relates to the splice variant 7 of the human phosphodiesterase PDE4D, i.e. the human phosphodiesterase PDE4D7 gene, preferably to the sequence as defined in NCBI Reference Sequence: NM_001165899.1, more preferably to the nucleotide sequence as set forth in SEQ ID NO: 6, which corresponds to the sequence of the above indicated NCBI Reference Sequence of the PDE4D7 transcript, and also relates to the corresponding amino acid sequence as set forth in SEQ ID NO: 7, which corresponds to the protein sequence defined in NCBI Protein Accession Reference Sequence NP_001159371.1 encoding the PDE4D7 polypeptide. The term "phosphodiesterase 4D7" or "PDE4D7" also relates to the amplicon that can be generated by the primer pair PDE1D7_forward (SEQ ID NO: 8) and the PDE1D7_reverse (SEQ ID NO: 9) and can be detected by probe SEQ ID NO: 10.

The term "AZGP1" relates to the Alpha-2-Glycoprotein 1- Zinc-Binding gene, preferably to the sequence as defined in NCBI Reference Sequence: NM_001185.3, more preferably to the nucleotide sequence as set forth in SEQ ID NO: 11, which corresponds to the sequence of the above indicated NCBI Reference Sequence of the AZGP1 transcript, and also relates to the corresponding amino acid sequence as set forth in SEQ ID NO: 12, which corresponds to the protein sequence defined in NCBI Protein Accession Reference Sequence NP_001176.1 encoding the AZGP1 polypeptide. The term "AZGP1" also relates to the amplicon that can be generated by the primer pair AZGP1_forward (SEQ ID NO: 13) and the AZGP1_reverse (SEQ ID NO: 14) and can be detected by probe SEQ ID NO: 15.

The term "FBLN1" relates to the Fibulin1 gene, preferably to one or more of the sequences as defined in NCBI Reference Sequence: NM_001996.3, NM_006485.3, NM_006486.2 and NM_006487.2, more preferably to the nucleotide sequences as set forth in SEQ ID NO: 16, 17, 18 and 19, respectively, which correspond to the sequences of the above indicated NCBI Reference Sequences of the Fibulin1 transcripts, and also relate to the corresponding amino acid sequence as set forth in SEQ ID NO: 20, 21, 22 and 23 which correspond to the protein sequences defined in NCBI Protein Accession Reference Sequences NP_001987.2, NP_006476.2, NP_006477.2 and NP_006478.2, respectively, encoding Fibulin1 polypeptides. The term "FBLN1" also relates to the amplicon(s) that can be generated by the primer pair FBLN1_forward (SEQ ID NO: 24) and the FBLN1_reverse (SEQ ID NO: 25) and can be detected by probe SEQ ID NO: 26.

The term "ILK" relates to the Integrin-Linked Kinase gene, preferably to one or more of the sequences as defined in NCBI Reference Sequences: NM_001014794.2, NM_001014795.2, NM_001278441.1, NM_001278442.1 and NM_004517.3, more preferably to the nucleotide sequences as set forth in SEQ ID NO: 27, 28, 29, 30 and 31, respectively, which correspond to the sequences of the above indicated NCBI Reference Sequences of the Integrin-Linked Kinase transcripts, and also relate to the corresponding amino acid sequence as set forth in SEQ ID NO: 32, 33, 34, 35 and 36 which correspond to the protein sequences defined in NCBI Protein Accession Reference Sequences NP_001014794.1, NP_001014795.1, NP_001265370.1, NP_001265371.1 and NP_004508.1 respectively, encoding Integrin-Linked Kinase polypeptides. The term "ILK" also relates to the amplicon(s) that can be generated by the primer pair ILK_forward (SEQ ID NO: 37) and the ILK_reverse (SEQ ID NO: 38) and can be detected by probe SEQ ID NO: 39.

The term "KRT15" relates to the Keratin 15 gene, preferably to the sequence as defined in NCBI Reference Sequence: NM_002275.3, more preferably to the nucleotide sequence as set forth in SEQ ID NO: 40, which corresponds to the sequence of the above indicated NCBI Reference Sequence of the KRT15 transcript, and also relates to the corresponding amino acid sequence as set forth in SEQ ID NO: 41, which corresponds to the protein sequence defined in NCBI Protein Accession Reference Sequence NP_002266.2 encoding the KRT15 polypeptide. The term "KRT15" also relates to the amplicon that can be generated by the primer pair KRT15_forward (SEQ ID NO: 42) and the KRT15_reverse (SEQ ID NO: 43) and can be detected by probe SEQ ID NO: 44.

The term "MEIS2" relates to the Meis Homeobox 2 preferably to one or more of the sequences as defined in NCBI Reference Sequences: NM_001220482.1, NM_002399.3, NM_170674.4, NM_170675.4, NM_170676.4, NM_170677.4, NM_172315.2 and NM_020149.2 more preferably to the nucleotide sequences as set forth in SEQ ID NO: 45, 46, 47, 48, 49, 50, and/or 51, respectively, which correspond to the sequences of the above indicated NCBI Reference Sequences of the MEIS2 transcripts, and also relate to the corresponding amino acid sequence as set forth in SEQ ID NO: 53, 54, 55, 56, 57, 58, 59 and/or 60 which correspond to the protein sequences defined in NCBI Protein Accession Reference Sequences NP_001207411.1, NP_002390.1, NP_733774.1, NP_733775.1, NP_733776.1, NP_733777.1, NP_758526.1 and NP_758527.1 respectively, encoding MEIS2 polypeptides. The term "MEIS2" also relates to the amplicon(s) that can be generated by the primer pair MEIS2 _forward (SEQ ID NO: 61) and the MEIS2_reverse (SEQ ID NO: 62) and can be detected by probe SEQ ID NO: 63.

The term "MYBPC1" relates to the Myosin Binding Protein C, Slow Type preferably to one or more of the sequences as defined in NCBI Reference Sequences: NM_001254718.1, NM_001254719.1, NM_001254720.1, NM_001254721.1, NM_001254722.1, NM_001254723.1, NM_002465.3, NM_206819.2, NM_206820.2 and NM_206821.2 more preferably to the nucleotide sequences as set forth in SEQ ID NO: 64, 65, 66,67, 68, 69, 70, 71, 72 and/or 73, respectively, which correspond to the sequences of the above indicated NCBI Reference Sequences of the MYBPC1 transcripts, and also relate to the corresponding amino acid sequence as set forth in SEQ ID NO: 74, 75, 76, 77, 78, 79, 80, 81, 82 and/or 83 which correspond to the protein sequences defined in NCBI Protein Accession Reference Sequences NP_001241647.1, NP_001241648.1, NP_001241649.1, NP_001241650.1, NP_001241651.1, NP_001241652.1, NP_002456.2,NP_996555.1, NP_996556.1 and NP_996557.1 respectively, encoding MYBPC1 polypeptides. The term "MYBPC1" also relates to the amplicon(s) that can be generated by the primer pair MYBPC1_forward (SEQ ID NO: 84) and the MYBPC1_reverse (SEQ ID NO: 85) and can be detected by probe SEQ ID NO: 86.

The term "PAGE4" relates to the P Antigen Family, Member 4 gene, preferably to the sequence as defined in NCBI Reference Sequence: NM_007003.3, more preferably to the nucleotide sequence as set forth in SEQ ID NO: 87, which corresponds to the sequence of the above indicated NCBI Reference Sequence of the PAGE4 transcript, and also relates to the corresponding amino acid sequence as set forth in SEQ ID NO: 88, which corresponds to the protein sequence defined in NCBI Protein Accession Reference Sequence NP_008934.1 encoding the PAGE4 polypeptide. The term "PAGE4" also relates to the amplicon that can be generated by the primer pair PAGE4_forward (SEQ ID NO: 89) and the PAGE4_reverse (SEQ ID NO: 90) and can be detected by probe SEQ ID NO: 91.

The term "SRD5A2" relates to the Steroid 5-Alpha-Reductase 2 gene, preferably to the sequence as defined in NCBI Reference Sequence: NM_000348.3, more preferably to the nucleotide sequence as set forth in SEQ ID NO: 92, which corresponds to the sequence of the above indicated NCBI Reference Sequence of the SRD5A2 transcript, and also relates to the corresponding amino acid sequence as set forth in SEQ ID NO: 93, which corresponds to the protein sequence defined in NCBI Protein Accession Reference Sequence NP_000339.2 encoding the SRD5A2 polypeptide. The term "SRD5A2" also relates to the amplicon that can be generated by the primer pair SRD5A2_forward (SEQ ID NO: 94) and the SRD5A2_reverse (SEQ ID NO: 95) and can be detected by probe SEQ ID NO: 96.

The term "COL1A1" relates to the Collagen, Type I, Alpha 1 gene, preferably to the sequence as defined in NCBI Reference Sequence: NM_000088.3, more preferably to the nucleotide sequence as set forth in SEQ ID NO: 97, which corresponds to the sequence of the above indicated NCBI Reference Sequence of the COL1A1 transcript, and also relates to the corresponding amino acid sequence as set forth in SEQ ID NO: 98, which corresponds to the protein sequence defined in NCBI Protein Accession Reference Sequence NP_000079.2 encoding the COL1A1 polypeptide. The term "COL1A1" also relates to the amplicon that can be generated by the primer pair COL1A1_forward (SEQ ID NO: 99) and the COL1A1_reverse (SEQ ID NO: 100) and can be detected by probe SEQ ID NO: 101.

The term "COL3A1" relates to the Collagen, Type III, Alpha 1 gene, preferably to the sequence as defined in NCBI Reference Sequence: NM_000090.3, more preferably to the nucleotide sequence as set forth in SEQ ID NO: 102, which corresponds to the sequence of the above indicated NCBI Reference Sequence of the COL3A1 transcript, and also relates to the corresponding amino acid sequence as set forth in SEQ ID NO: 103, which corresponds to the protein sequence defined in NCBI Protein Accession Reference Sequence NP_000081.1 encoding the COL3A1 polypeptide. The term "COL3A1" also relates to the amplicon that can be generated by the primer pair COL3A1_forward (SEQ ID NO: 104) and the COL3A1_reverse (SEQ ID NO: 105) and can be detected by probe SEQ ID NO: 106.

The term "COL5A2" relates to the Collagen, Type V, Alpha 2 gene, preferably to the sequence as defined in NCBI Reference Sequence: NM_000393.3, more preferably to the nucleotide sequence as set forth in SEQ ID NO: 107, which corresponds to the sequence of the above indicated NCBI Reference Sequence of the COL5A2 transcript, and also relates to the corresponding amino acid sequence as set forth in SEQ ID NO: 108, which corresponds to the protein sequence defined in NCBI Protein Accession Reference Sequence NP_000384.2 encoding the SRD5A2 polypeptide. The term "COL5A2" also relates to the amplicon that can be generated by the primer pair COL5A2_forward (SEQ ID NO: 109) and the COL5A2_reverse (SEQ ID NO: 110) and can be detected by probe SEQ ID NO: 111.

The term "INHBA" relates to the Inhibin, Beta A gene, preferably to the sequence as defined in NCBI Reference Sequence: NM_002192.2, more preferably to the nucleotide sequence as set forth in SEQ ID NO: 112, which corresponds to the sequence of the above indicated NCBI Reference Sequence of the INHBA transcript, and also relates to the corresponding amino acid sequence as set forth in SEQ ID NO: 113, which corresponds to the protein sequence defined in NCBI Protein Accession Reference Sequence NP_002183.1 encoding the INHBA polypeptide. The term "INHBA" also relates to the amplicon that can be generated by the primer pair INHBA_forward (SEQ ID NO: 114) and the INHBA_reverse (SEQ ID NO: 115) and can be detected by probe SEQ ID NO: 116.

The term "THBS2" relates to the Thrombospondin 2 gene, preferably to the sequence as defined in NCBI Reference Sequence: NM_003247.3 more preferably to the nucleotide sequence as set forth in SEQ ID NO: 117, which corresponds to the sequence of the above indicated NCBI Reference Sequence of the THBS2 transcript, and also relates to the corresponding amino acid sequence as set forth in SEQ ID NO: 118, which corresponds to the protein sequence defined in NCBI Protein Accession Reference Sequence NP_003238.2 encoding the THBS2 polypeptide. The term "THBS2" also relates to the amplicon that can be generated by the primer pair THBS2_forward (SEQ ID NO: 119) and the THBS2_reverse (SEQ ID NO: 120) and can be detected by probe SEQ ID NO: 121.

The term "VCAN" relates to the Versican gene, preferably to one or more of the sequences as defined in NCBI Reference Sequences: NM_001126336.2, NM_001164097.1, NM_001164098.1 and NM_004385.4 more preferably to one or more nucleotide sequences as set forth in SEQ ID NO: 122, 123, 124 and 125, respectively, which correspond to the sequences of the above indicated NCBI Reference Sequences of the VCAN transcripts, and also relates to the corresponding amino acid sequences as set forth in SEQ ID NO: 126, 127, 128 and 129 which correspond to the protein sequences defined in NCBI Protein Accession Reference Sequences NP_001119808.1, NP_001157569.1, NP_001157570.1 and NP_004376.2 respectively, encoding VCAN polypeptides. The term "VCAN" also relates to the amplicon(s) that can be generated by the primer pair VCAN_forward (SEQ ID NO: 130) and the VCAN_reverse (SEQ ID NO: 131) and can be detected by probe SEQ ID NO: 132.

The term "BNG" relates to the Biglycan gene, preferably to the sequence as defined in NCBI Reference Sequence: NM_001711.4, more preferably to the nucleotide sequence as set forth in SEQ ID NO: 133, which corresponds to the sequence of the above indicated NCBI Reference Sequence of the BNG transcript, and also relates to the corresponding amino acid sequence as set forth in SEQ ID NO: 134, which corresponds to the protein sequence defined in NCBI Protein Accession Reference Sequence NP_001702.1 encoding the BNG polypeptide. The term "BNG" also relates to the amplicon that can be generated by the primer pair BNG_forward (SEQ ID NO: 135) and the BNG _reverse (SEQ ID NO: 136) and can be detected by probe SEQ ID NO: 137.

The term "BIRC5" relates to the Versican gene, preferably to one or more of the sequences as defined in NCBI Reference Sequences: NM_001012270.1, NM_001012271.1 and NM_001168.2 more preferably to one or more nucleotide sequences as set forth in SEQ ID NO: 138, 139 and 140, respectively, which correspond to the sequences of the above indicated NCBI Reference Sequences of the BIRC5 transcripts, and also relates to the corresponding amino acid sequences as set forth in SEQ ID NO: 141, 142, and 143 which correspond to the protein sequences defined in NCBI Protein Accession Reference Sequences NP_001012270.1, NP_001012271.1 and NP_001159.2 respectively, encoding BIRC5 polypeptides. The term "BIRC5" also relates to the amplicon(s) that can be generated by the primer pair BIRC5_forward (SEQ ID NO: 144) and the BIRC5_reverse (SEQ ID NO: 145) and can be detected by probe SEQ ID NO: 146.

The term "DYRK2" relates to the Versican gene, preferably to one or more of the sequences as defined in NCBI Reference Sequences: NM_003583.3 and NM_006482.2 more preferably to one or more nucleotide sequences as set forth in SEQ ID NO: 147 and 148, respectively, which correspond to the sequences of the above indicated NCBI Reference Sequences of the DYRK2 transcripts, and also relates to the corresponding amino acid sequences as set forth in SEQ ID NO: 149 and 150 which correspond to the protein sequences defined in NCBI Protein Accession Reference Sequences NP_003574.1 and NP_006473.2 respectively, encoding DYRK2 polypeptides. The term "DYRK2" also relates to the amplicon(s) that can be generated by the primer pair DYRK2_forward (SEQ ID NO: 151) and the DYRK2_reverse (SEQ ID NO: 152) and can be detected by probe SEQ ID NO: 153.

In other embodiments the PCPI signature can be an 18-gene list comprising PDE4D instead of PDE4D5 and PDE4D7, or it may comprise the remaining 17 genes. Therefore, in a preferred embodiment, the PCPI signature can be a 17-gene list which comprises the above-mentioned 17 genes and comprises neither PDE4D5 nor PDE4D7.

In another embodiment, the present invention provides a gene expression prognostic signature comprised of a combination of 19 individual genes which can be measured on extracted RNA from human prostate cancer tissue samples in a quantitative manner. The combination of these features into a single data model (=PCPI -> Prostate Cancer Progression Index) provides significantly improved classification power to predict the progression of prostate cancer. In embodiments using either these 19 genes, or 17, 18 or 57, the PCPI referred to in the present invention is used in methods of diagnosing prostate cancer in a sample, prognosticating the patient's individual disease status and expected development of disease, and it provides a parameter in methods for stratifying and/or identifying patients for an individually preferred treatments or therapy, if applicable. Based on the PCPI parameter, the predisposition or likelihood that, e.g., the tumor progresses to aggressive prostate cancer can be determined. For example, if the PCPI obtained in the analysis of a tumor is higher than a defined cut-off (or threshold) level, the probability that said tumor progresses to an aggressive form is also higher, and vice versa.

The classification step of tumor samples that are analysed according to the methods described herein thus comprises the comparison of PCPI values between samples that have been obtained at a specific time point in the past (e.g. before initial diagnosis of prostate cancer) and samples obtained at a later stage. When the PCPI obtained in the analysis of a tumor sample at said later stage is higher than a defined threshold or cut-off, this parameter can be used as an indication of a progression towards aggressive prostate cancer.

The cut-off may be defined based on a reference data set comprising patient groups with or without progression. The cut-off may be set for example at 70 %, 80 %, 85 %, 90 %, 93 %, 95 %, 96 %, 97 %, 98 % or 99 % sensitivity with regard to classification of patients without or with progression. In a preferred embodiment the cut-off may be set at 90 % sensitivity.

Of course, the PCPI may also be used in methods of establishing a patients' prognosis, in a method for the stratification of the patients into groups for an appropriate treatment, with the aid of which the physician comes to the conclusion that a specific treatment is not appropriate, that a therapy is not (yet) required, or that therapies are no longer needed in view of the patients overall physical condition.

Embodiments of the present invention thus relate to the identification and use of marker gene (or sequence) expression patterns (or "profiles" or "signatures") which are clinically relevant to prostate cancer. The marker gene expression profiles, whether embodied in nucleic acid expression, protein expression, or other expression formats, may be used to predict prostate cancer recurrence and/or survival of subjects afflicted with prostate cancer. Particularly, the marker genes are used in establishing the herein described Prostate Cancer Progression Profile (PCPI) that is be used in the identification /classification of patients with an aggressive or indolent disease.

In the context of the present application, the expression "initial diagnosis" refers to the first or a subsequent diagnosis preceding the date on which one of the methods according to the present invention was performed, and the PCPI was calculated. For example the initial diagnosis may be established for a patient whose prostate tissue material was analyzed for the first time, and preferably before diagnosis of prostate cancer.

In the context of the present application, the term "predetermined period after initial diagnosis" comprises a period of about 6 months, 1 year, 2 years, 3 years, 4 years, 5 years, 6 years, 7 years, 8 years, 9 years, 10 years, 15 years or 20 years or more. In the context of the present application, the expression "patient with newly diagnosed prostate cancer" refers to an individual who has been diagnosed as having biochemical values, clinical values or PCPI values that confirm or raise suspicion of the presence of prostate cancer cells in a sample taken from this patient for the first time.

In the context of the present application, the expression "primary treatment for prostate cancer" refers to any known method for the treatment of prostate cancer, i.e. surgery, chemotherapy, treatment with biologicals, radiation therapy, and the like.

In the context of the present application, the expression "treatment with biologicals" refers to the treatment of prostate cancers with monoclonal antibodies or derivatives thereof, or peptides or fragments thereof that specifically inhibit or block or neutralize biological molecules *in vivo* as well as *in vitro* that are implicated in growth, proliferation, maintenance, or in any other metabolic pathway that is used by prostate cancer cells. Monoclonal antibodies directed to established targets include those that are approved for solid tumors such as anti-human EGFR-2 monoclonal antibodies such as trastuzumab, anti-EGFR antibodies such as cituximab and panitumumab and the anti-vascular endothelial growth factor (VEGF) monoclonal antibody bevacizumab.

In the context of the present application, the expression "biochemical recurrence" refers, e.g., to recurrent biological values of increased PSA indicating the presence of prostate cancer cells in a sample. However, it is also possible to use other markers that can be used in the detection of the presence or that rise suspicion of such presence.

In the context of the present application, the term "clinical recurrence" refers to the presence of clinical signs or clinical values as measured, for example using the Gleason Score.

In the context of the present application, the expression "predisposition of prostate cancer that is susceptible to disease progression" refers to a measurable and significant parameter (for example the PCPI), which provides an indication that the likelihood that the patient from whom a sample was investigated will develop aggressive prostate cancer, i.e. after primary treatment of diagnosed prostate cancer, for example by prostatectomy, chemotherapy and/or radiation therapy, prostate cancer cells re-emerged which have a potential for aggressive growth, such as the formation of metastases in the lymph nodes and bones.

In the context of the present application, the expression "computer implemented method for determining a prognosis of a patient having a prostate cancer" refers to a method wherein software algorithms calculate a PCPI and based thereon provide a prognosis for the patient that is analyzed, wherein this method uses raw data obtained upon measurement of the gene expression level of the genes referred to herein and conversion thereof into a PCPI using the above-described equation.

In the context of the present application, the methods for diagnosis, prognosis, identification of a predisposition for development or progression of aggressive prostate cancer, the identification of patients suffering from prostate cancer or subject to recurrence of prostate cancer after primary treatment, may be treated with a suitable therapeutic regime on the basis of the results obtained in any of the methods according to the present invention. Thus, for example, if the prognosis is that a patient will develop aggressive prostate cancer based on results determining a predisposition for this type of cancer, the physician may decide whether or not to use any of the available therapies for the treatment of prostate cancer, such as surgery, chemotherapy, radiation therapy, hormone therapy or combinations thereof and the like.

The methods according to the present invention also provide information with the aid of the PCPI based on which it can be decided whether or not a patient should be subjected to surgery, chemotherapy, radiation therapy, or whether a further biopsy is to be performed. Thus, the PCPI represents an important parameter in establishing an individualized diagnosis, prognosis, allows to identify patients with a predisposition for aggressive prostate cancer, or to stratify the same for the therapy that is best suited for this patient. The PCPI can also be used in combination with other means and methods to establish a diagnosis or prognosis, and to identify or stratify patients as discussed herein. Described herein are also prostate cancer-specific therapies or treatments as pointed out above for use in the treatment of patients that have been identified as eligible for such therapies on the basis of the PCPI, optionally in combination with other diagnostic or prognostic methods, with a view to selecting the most suitable cancer treatment for said patient. For example, the complete removal of lymph nodes may be an option in the treatment of patients that have been identified or diagnosed or for whom a prognosis has been established using the methods according to the invention, when the PCPI is high compared with a control sample.

In embodiments of the present invention involving patients, who were treated by prostatectomy after initial clinical diagnosis, the invention includes methods to predict the likelihood or predisposition of prostate cancer recurrence, cancer metastasis, and/or recurrence of elevated PSA levels.

Further embodiments of the present invention relate to methods to identify, and then use, marker gene (or sequence) expression profiles in a sample which provide prognostic information related to prostate cancer. The expression profiles correlate with (and so are able to discriminate between) patients with good or poor cancer recurrence, cancer metastasis, and/or survival outcomes.

In embodiments of the present invention a method to identify marker gene expression profiles relevant to cancer recurrence and/or metastasis in prostate cancer afflicted patients is provided.

In further embodiments of the invention, methods comparing marker gene (or sequence) expression in a sample comprising gene expression analysis in prostate cancer cells from a patient to an identified expression profile, the PCPI, are provided to determine the likely outcome for the patient, for example after prostatectomy, chemotherapy, radiation therapy, etc. In further aspects of these embodiments of the invention, the obtained information may be advantageously used to predict whether a patient will likely benefit from, e.g., surgery to treat prostate cancer or whether a patient will be better off with another type of treatment or need adjuvant treatment in addition to surgery. Patients that will likely benefit from surgery, such as a radical prostatectomy, can be selected on the basis of the expression profile of the patient's prostate sample as disclosed herein, to be free of cancer recurrence and/or metastasis following the surgery. In a further embodiment, a patient that may be expected to not benefit from surgery is one predicted, by the expression profile of the patient's prostate cancer cells as disclosed herein, to develop cancer metastasis following the surgery. By way of example, the recurrence of prostate cancer may be at the same location while metastasis is frequently to a different location or tissue, such as to a lymph node or bone. Further embodiments of the present invention relate to a method to identify a patient, from a population of patients with prostate cancer cells, or following prostatectomy, as belonging to a subpopulation of patients with either a better cancer recurrence and/or survival outcome (such as lower risk of recurrence or metastasis), relative to another subpopulation, or as belonging to a subpopulation with a poorer cancer recurrence and/or survival outcome (such as elevated risk of recurrence or metastasis), relative to another subpopulation. The methods of the invention are thus allowing to stratify patients into subpopulations or subtypes.

Methods of the present invention relate to embodiments for the identification of patients with prostate cancer, optionally following prostatectomy, as likely to have a better or poorer cancer recurrence, cancer metastasis, and/or survival outcome by assaying for the PCPI disclosed herein. Where previously subjective interpretation of sample obtained from patients (such as that based upon immunohistochemical staining and subjective analysis) may have been used to determine the predisposition for aggressive versus indolent disease and/or treatment of prostate cancer patients, or pre- or post-prostatectomy patients, or prostate cancer patients that have been treated by a different therapy, the present invention introduces an objective index reflecting expression patterns, which may be used alone or in combination with other (subjective and/or objective) criteria to provide a more accurate assessment of patient outcomes, including survival and the recurrence or metastasis of cancer. The methods based on the analysis of the marker gene expression patterns of the invention thus include a means to detect/diagnose prostate cancer, or pre- or post-prostatectomy prognosis, or, more generally, to determine the predisposition to either aggressive or indolent disease pre- or post-primary treatment.

The PCPI is based on the analysis of more than one marker gene, or expressed sequence, capable of discriminating between prostate cancer stages, or pre- or post-prostatectomy prostate cancer stages with significant accuracy. The expression level(s) of the marker gene(s), or expressed sequence(s) of interest are identified as correlated with outcomes such that the expression level(s) is/are relevant to a determination of the preferred treatment protocols of a patient. Thus, the invention includes embodiments relating to a method to determine the outcome of a subject afflicted with prostate cancer, optionally pre- or post-prostatectomy, by assaying a prostate sample suspected to contain a cancer cell from said patient for expression levels that are correlated with patient outcomes according to the herein disclosed methods. In another embodiment of the invention method to determine the risk of prostate cancer recurrence and/or metastasis in a subject patient are disclosed.

In methods of the invention, a sample suspected to comprise prostate cancer cells from a patient is analyzed for the expression levels of marker genes disclosed herein. The aggregated expression levels of the marker genes can be compared with the mean or median expression level(s) thereof in a population of prostate cancer cells. This assessment may then be used to determine the risk of prostate cancer recurrence and/or metastasis in the subject from which the sample was obtained when the PCPI is used. The expression levels of the disclosed genes (or expressed sequences) are correlated with a low risk of cancer recurrence and/or metastasis, or a high risk of cancer recurrence and/or metastasis.

Put differently, the individual marker genes (or expressed sequences) disclosed herein are expressed at higher, or lower, levels (in comparison to normalized expression values in a prostate cancer cell population) such that the deviation is correlated with an higher or lower risk or predisposition for the development of aggressive versus indolent cancer in patients previously diagnosed with prostate cancer, optionally patients that have already been treated specifically for prostate cancer, or de novo diagnosed patients.

In some embodiments, assaying for expression levels may be performed by use of nucleic acid probes selected that are specifically recognizing the marker genes as disclosed herein. These probes hybridize, under appropriate conditions, and detect expressed sequences as disclosed herein. In embodiments of the present invention, probes can be used to detect a region of sequence amplified from an expressed marker (gene) sequence.

The detection or determination of expression levels of marker (gene) sequences may be referred to as detecting or determining an expression profile or signature. Expression patterns of the invention comprising disclosed marker gene sequences are identified and used as described herein. For their identification, a large sampling of the gene expression levels in a sample containing prostate cancer cells is obtained through quantifying the expression levels of mRNA. In one embodiment, the invention includes detecting marker gene (or sequence) expression levels by analyzing gene expression from single cells or homogenous cell populations which have been dissected away from, or otherwise isolated or purified from, contaminating cells beyond that possible by a simple biopsy. Since the expression of numerous genes fluctuates between cells from different patients as well as between cells from the same patient sample, multiple data from expression of individual gene sequences are used as reference data to generate models which in turn permit the identification of individual genes and sequences, the expression of which are correlated with particular prostate cancer, or pre- or post-prostatectomy, outcomes, or which allow classifying patients according to their predisposition for aggressive versus indolent cancer. In these methods, the gene expression levels are preferably converted into a PCPI as defined above, using the marker genes of interest referred to in the claims and throughout the present the present application.

According to the present invention, marker gene expression levels of various genes in these models were then analyzed to identify nucleic acid sequences, the expressions of which are positively, or negatively, correlated, with a prostate cancer, or post-prostate cancer treatment, outcome.

Preferred embodiments of the present invention is based on the identification of a subset of expressed marker gene sequences in a cell, identified as correlating to outcomes as described herein. An expression pattern or profile of the invention includes a combination of these identified marker sequences (or genes) that are converted into a PCPI. The use of multiple samples for identification of expressed sequences increases the confidence with which a gene is considered to correlate with a prostate cancer recurrence, metastasis, and/or survival outcome. Without sufficient confidence, it remains unpredictable whether a particular gene is actually correlated with an outcome and also unpredictable whether expression of a gene may be successfully used to identify the outcome for a prostate cancer, or pre- or post-prostatectomy, subject or patient. The identified, nucleic acid sequences corresponding to the disclosed marker genes (or expressed sequences) of interest may be selected for assessment as an expression profile to be detected or assessed for its predictive properties.

A profile of expression levels or PCPI that is highly correlated with one outcome relative to another may be used to assay a prostate cancer cell containing sample from a subject or patient to predict the outcome of the subject from whom the sample was obtained. Such an assay may be used as part of a method to determine the therapeutic treatment for said subject based upon the outcome identified. This correlation provides a molecular determination of prostate cancer recurrence, cancer metastasis, and/or survival outcomes as disclosed herein. Without being bound by theory, and offered to improve understanding of the instant invention, the disclosed molecular determination is more powerful than other molecular indicators related to prostate cancer, such as the determination of prostate serum antigen (PSA) levels. Additional uses of the correlated expression patterns and profiles are in the classification of cells and tissues; determination of diagnosis and/or prognosis; and determination and/or alteration of therapy.

The ability to discriminate is conferred by the identification of expression of the individual marker gene sequences as relevant and not by the form of the assay used to determine the actual level of expression. The methods of the invention reflect, quantitatively or qualitatively, expression of marker gene sequence(s) in the "transcriptome" (the transcribed fraction of genes in a genome). The nature of the prostate cancer cell containing sample is not limiting, as fresh tissue, freshly frozen tissue, and fixed tissue, such as formalin-fixed paraffin-embedded (FFPE) tissues, may be used in the disclosed methods. In some embodiments, the sample may be that of a needle (core) biopsy or other biopsy.

For detecting an identified expression pattern or PCPI signature, embodiments of the invention include detecting gene (or sequence) expression patterns by gathering global, or near global, gene expression data from single cells or homogenous cell populations which have been dissected away from, or otherwise isolated or purified from, contaminating cells beyond that possible by a simple biopsy. The expression levels of the marker genes (sequences) in the pattern or profile are then detected or otherwise measured. In other embodiments, a method may only detect or measure the expression levels of the genes (sequences) in the profile without assessment or other determination of the expression of other genes or sequences.

In an additional aspect, the analysis of expression levels may be performed in combination with, or in place of, other assessments or indicators of prostate cancer. In some embodiments, the analysis is made in combination with a method of determining the grade of prostate cancer in a sample comprising prostate cancer cells from a subject. In other embodiments, the combination is with a method of determining the stage of prostate cancer in the sample. A third possibility is combination with detecting or determining PSA levels in the subject, optionally before a procedure used to isolate the prostate cancer cells. Of course a combination with any one, two, or all three of these representative examples is possible. Whenever more than one type of assessment is used, the result is a multivariate analysis. The invention expressly includes all possible combinations of assessments described herein as multivariate embodiments.

Generally, any accepted method of assessing prostate cancer grade and/or stage as known to the skilled person may be used. In some cases, the method of determining prostate cancer grade comprises determination of a Gleason Score (or Gleason Grade). In other cases, the method of determining prostate cancer stage comprises a determination according to the American Joint Committee on Cancer (AJCC) tumor staging system for assessing prostate cancer stage. And as described herein, the analysis of gene (sequence) expression levels may be performed in place of or in combination with either the Gleason Score or the AJCC tumor stage determination. In cases of PSA levels, its assessment may be conducted before a prostatectomy which is used to provide a sample comprising prostate cancer cells for use in any method described herein.

In further embodiments, the invention includes physical and methodological means for detecting the expression of genes (or sequences) disclosed herein. These means may be directed to assaying one or more aspect of the DNA template(s) underlying the expression of the gene (or sequence), of the RNA used as an intermediate to express the gene (or sequence), or of the proteinaceous product expressed by the gene (or sequence).

While the present invention is described mainly in the context of human prostate cancer, it may be practiced in the context of prostate cancer of any animal known to be potentially afflicted by prostate cancer. Non-limiting examples of animals for the application of the present invention are mammals, particularly those important to agricultural applications (such as, but not limited to, cattle, sheep, horses, and other "farm animals"), animal models of prostate cancer, and animals for human companionship (such as, but not limited to, dogs and cats).

A gene expression "pattern" or "profile" or "signature" and the corresponding calculated PCPI refers to the relative expression of genes of interest (expressed sequences) between two or more prostate cancer, or pre- or post-prostatectomy, cancer recurrence, metastasis, and/or survival outcomes which expression is correlated with being able to distinguish between the outcomes.

A "gene" or "expressed sequence" is a polynucleotide that encodes, and expresses in a detectable manner, a discrete product, whether RNA or proteinaceous in nature. It is appreciated that more than one polynucleotide may be capable of encoding a discrete product. The term includes alleles and polymorphisms of a gene or expressed sequence that encodes the same product, or a functionally associated (including gain, loss, or modulation of function) analog thereof, based upon chromosomal location and ability to recombine during normal mitosis.

The terms "correlate" or "correlation" or equivalents thereof refer to an association between expression of two or more genes and a physiologic state of a prostate cell to the exclusion of one or more other states as identified by use of the methods as described herein. A gene may be expressed at higher or lower levels and still be correlated with one or more prostate cancer, or pre- or post-prostatectomy, state or outcome.

As used herein, a "marker gene" or "marker gene sequence" the expression of which is analyzed in the context of the present invention, may also be described as a gene of interest (GOI).

In embodiments of the invention, the strength of expression of a given marker gene (or expressed sequence) is normalized (such as relative to expression of a reference gene that is expressed at relatively constant levels, or the expression of the GOI is measured across all available samples and the expression value is normalized) to provide a normalized value or score for the correlation analysis of the marker gene expression signature (e.g. the PCPI) and other parameter, e.g. clinical data. In many cases, the expression data is normalized, median-centered, and log-transformed as known to the skilled person prior to further use, such as in clustering and discriminant analysis. Where more than one expression level is used (as in the case of a gene expression profile or index, e.g. the PCPI), the values or scores may be summed and then analyzed as an aggregate value for assessing the correlation or conducting classification based on the correlation.

A "polynucleotide" is a polymeric form of nucleotides of any length, either ribonucleotides or deoxyribonucleotides. This term refers only to the primary structure of the molecule. Thus, this term includes double- and single-stranded DNA and RNA. It also includes known types of modifications including labels known in the art, methylation, "caps", substitution of one or more of the naturally occurring nucleotides with an analog, and internucleotide modifications such as uncharged linkages (e.g., phosphorothioates, phosphorodithioates, etc.), as well as unmodified forms of the polynucleotide.

The term "amplify" is used in the broad sense to mean creating an amplification product can be made enzymatically with DNA or RNA polymerases. "Amplification," as used herein, generally refers to the process of producing multiple copies of a desired sequence, particularly those of a sample. "Multiple copies" mean at least 2 copies. A "copy" does not necessarily mean perfect sequence complementarity or identity to the template sequence. It is possible to further use any sequencing method known in the art to identify the sequences of GOI's.

The term "corresponding" may refer to, where appropriate, a nucleic acid molecule as sharing a substantial amount of sequence identity with another nucleic acid molecule. Substantial amount means at least 95%, usually at least 98% and more usually at least 99%, and sequence identity is determined using the BLAST algorithm, as described in Altschul et al. (1990), J. Mol. Biol. 215:403-410 (using the published default setting, i.e. parameters w=4, t=17). Methods for amplifying mRNA are generally known in the art, and include reverse transcription PCR (RT-PCR) and those described in U.S. patent application Ser. No. 10/062,857 (filed on Oct. 25, 2001), as well as U.S. Provisional Patent Applications 60/298,847 (filed Jun. 15, 2001) and 60/257,801 (filed Dec. 22, 2000), all of which are hereby incorporated by reference in their entireties as if fully set forth. Another method which may be used is quantitative PCR (or Q-PCR). Alternatively, RNA may be directly labeled as the corresponding cDNA by methods known in the art.

The term "Cq value" defines that PCR cycle in qRT-PCR (quantitative real-time PCR) where the measured fluorescence crosses for the first time the background fluorescence.

A "microarray" is a linear or two-dimensional array of discrete regions, each having a defined area, formed on the surface of a generally solid support such as, but not limited to, glass, plastic, or synthetic membrane. The density of the discrete regions on a microarray is determined by the total numbers of immobilized polynucleotides to be detected on the surface of a single solid phase support, such as at least about 50/cm2, at least about 100/cm2, at least about 500/cm2, but below about 1,000/cm2 in some embodiments. The arrays may contain less than about 500, about 1000, about 1500, about 2000, about 2500, or about 3000 immobilized polynucleotides in total. As used herein, a DNA microarray is an array of oligonucleotides or polynucleotides placed on a chip or other surfaces used to hybridize to amplified or cloned polynucleotides from a sample. Because the position of each particular group of polynucleotides in the array is known, the identities of a sample polynucleotides can be determined based on their binding to a particular position in the microarray.

Because the invention relies upon the identification of genes (or expressed sequences) that are over- or under-expressed, one embodiment of the invention involves determining expression by hybridization of mRNA, or an amplified or cloned version thereof (such as DNA or cDNA), of a sample cell to a polynucleotide that is unique to a particular gene sequence. Polynucleotides of this type may contain at least about 20, at least about 22, at least about 24, at least about 26, at least about 28, at least about 30, or at least about 32 consecutive basepairs of a gene sequence that is not found in other gene sequences. The term "about" as used in the previous sentence refers to an increase or decrease of 1 from the stated numerical value. Other embodiments may use polynucleotides of at least or about 50, at least or about 100, at least about or 150, at least or about 200, at least or about 250, at least or about 300, at least or about 350, or at least or about 400 basepairs of a gene sequence that is not found in other gene sequences. The term "about" as used in the preceding sentence refers to an increase or decrease of 10% from the stated numerical value. Such polynucleotides may also be referred to as polynucleotide probes that are capable of hybridizing to sequences of the genes, or unique portions thereof, described herein. In many cases, the hybridization conditions are stringent conditions of about 30% v/v to about 50% formamide and from about 0.01M to about 0.15M salt for hybridization and from about 0.01M to about 0.15M salt for wash conditions at about 55 to about 65° C. or higher, or conditions equivalent thereto.

In other embodiments, polynucleotide probes for use in the invention may have about or 95%, about or 96%, about or 97%, about or 98%, or about or 99% identity with the marker gene sequences the expression of which shall be determined. Identity is determined using the BLAST algorithm, as described above. These probes may also be described on the basis of the ability to hybridize to expressed marker genes used in methods of the invention under stringent conditions as described above or conditions equivalent thereto.

In many cases, the sequences are those of mRNA encoded by the marker genes, the corresponding cDNA to such mRNAs, and/or amplified versions of such sequences. In some embodiments of the invention, the polynucleotide probes are immobilized on an array, other devices, or in individual spots that localize the probes.

Suitable labels that can be used according to the invention, include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical or chemical means. The term "support" refers to conventional supports such as beads, particles, dipsticks, fibers, filters, membranes and silane or silicate supports such as glass slides.

As used herein, a "prostate tissue sample" or "prostate cancer cell sample" refers to a sample of prostate tissue isolated from an individual, such as one afflicted with prostate cancer. The sample may be from material removed via a prostatectomy, such as a radical prostatectomy. Alternatively, they are obtained by other means, such as needle (core) biopsy or other biopsy techniques, like laterally directed biopsies, the conventional sextant biopsy approach, different combinations of sextant and lateral biopsies as extended techniques, transrectal ultrasound guided prostate biopsy, and others as known to the skilled person. Such samples are primary isolates (in contrast to cultured cells) and may be collected by any suitable means recognized in the art. In some embodiments, the "sample" may be collected by an invasive method, including, but not limited to, surgical biopsy. A sample may contain prostate tumor cells which are isolated by known methods or other appropriate methods as deemed desirable by the skilled practitioner. Isolation methods include, but are not limited to, microdissection, laser capture microdissection (LCM), or laser microdissection (LMD) before use in accordance with the invention. "Expression" and "gene expression" include transcription and/or translation of nucleic acid material. As used herein, the term "comprising" and its cognates are used in their inclusive sense; that is, equivalent to the term "including" and its corresponding cognates.

Conditions that "allow" an event to occur or conditions that are "suitable" for an event to occur, such as hybridization, strand extension, and the like, or "suitable" conditions are conditions that do not prevent such events from occurring. Thus, these conditions permit, enhance, facilitate, and/or are conducive to the event. Such conditions, known in the art and described herein, depend upon, for example, the nature of the nucleotide sequence, temperature, and buffer conditions. These conditions also depend on what event is desired, such as hybridization, cleavage, strand extension or transcription.

"Detection" includes any means of detecting, including direct and indirect detection of gene expression and changes therein. For example, "detectably less" products may be observed directly or indirectly, and the term indicates any reduction (including the absence of detectable signal). Similarly, "detectably more" product means any increase, whether observed directly or indirectly.

"Prostatectomy" refers to the removal of prostate tissue by a skilled clinician, such as a surgeon. Non-limiting examples include radical prostatectomy; open (traditional) prostatectomy (involving an incision through the perineum); laparoscopic prostatectomy; and robotic (nerve sparing) prostatectomy.

"Gleason score" refers to the grading of a sample of prostate cancer by a trained pathologist according to the Gleason system, which assigns a Gleason score using numbers from 1 to 5 based upon similarities in the cells of a sample of prostate tissue to cancerous tissue or normal prostate tissue. Tissue that looks much like normal prostate tissue is given a score or grade of 1 while a tissue that lacks normal features and the cells seem to be spread haphazardly through the prostate is given a score or grade of 5. Scores, or grades of 2 through 4, inclusive, have features in between these possibilities. But because prostate cancers may have areas with different scores or grades, separate scores or grades are given to the two areas that make up most of the tissue. The two scores or grades are added to yield a Gleason score (or Gleason sum) between 2 and 10.

The expression levels of the marker genes referred to herein correlate clinical outcomes as described herein and therefore may be used as predictors as described herein. The identified genes have been reported to participate in various cellular functions as outlined in http://csbi.ltdk. Helsinki.fi/anduril/tcga-gbm/table4_2.html. In the methods underlying the embodiments of the present invention, these data were correlated with the putative prognostic gene marker PDE4D7 as described in WO2010131194 and WO2010131195, whose contents are herewith incorporated by reference. In the context of the present invention, those molecular pathways that showed significant correlation were selected for down-stream analysis (Fig. 2).

In embodiments of the invention, the marker gene expression pattern (or the PCPI) may be correlated to pre- or post-surgery PSA levels, Gleason score and pT stage. The profile also has the ability to stratify samples with Gleason scores of <=6 and 7 into low and high risk of PSA failure. In multivariate analysis with known prognostic factors, the profile was the only predictor that remains statistically significant ($p<0.05$). Therefore, detection of the profile at biopsy, or in tissue removed during prostatectomy, allows the distinguishing of indolent from aggressive cancers.

Embodiments of the invention thus relate to the identification and use of gene expression patterns (or profiles or "signatures") which discriminate between (or are correlated with) prostate cancer survival and recurrence outcomes in a subject. Such patterns may be determined by the methods of the invention by use of a number of reference cell or tissue samples, such as those reviewed by a pathologist of ordinary skill in the pathology of prostate cancer, which reflect prostate cancer cells as opposed to normal or other non-cancerous cells. The outcomes experienced by the subjects from whom the samples may be correlated with expression data to identify patterns that correlate with the outcomes. Because the overall gene expression profile differs from person to person, cancer to cancer, and cancer cell to cancer cell, correlations between certain cells and genes expressed or underexpressed may be made as disclosed herein to identify genes that are capable of discriminating between prostate cancer outcomes.

In embodiments of the present invention, genes with significant correlations to prostate cancer (or pre- or post-prostatectomy) survival, metastasis, or recurrence outcomes are used to discriminate between outcomes. The patterns or indices are capable of predicting the classification of an unknown sample based upon the expression of the genes used for discrimination in the models.

In embodiments of the present invention, marker genes (sequences) expressed in correlation with prostate cancer, or pre- or post-prostatectomy/primary treatment, outcomes provide the ability to focus gene expression analysis to only those genes that contribute to the ability to stratify a subject among different outcomes.

As will be appreciated by those skilled in the art, that the expression patterns (and the PCPI) are highly useful for discriminating between different outcomes in prostate cancer, or pre- or post-prostatectomy, and may be readily performed by the skilled artisan to permit the generation of models as described above to predict the status of an unknown sample based upon the expression levels of those genes.

To determine the (up-regulated or down-regulated) expression levels of genes (expressed sequences) in the practice of the invention, any method known in the art may be utilized. In some embodiments, expression based on detection of RNA which hybridizes to the genes (or probe sequences) identified and disclosed herein is used. This is readily performed by any RNA detection or amplification + detection method known or recognized as equivalent in the art such as, but not limited to, RNA sequencing, reverse transcription-PCR (RT-PCR), real-time PCR, real-time RT-PCR, the methods disclosed in U.S. patent application Ser. No. 10/062,857 (filed on Oct. 25, 2001) as well as U.S. Provisional Patent Applications 60/298,847 (filed Jun. 15, 2001) and 60/257,801 (filed Dec. 22, 2000), and methods to detect the presence, or absence, of RNA stabilizing or destabilizing sequences.

Alternatively, expression based on detection of DNA status may be used. Detection of the DNA of an identified gene as methylated or deleted may be used for genes that have decreased expression in correlation with a particular prostate cancer, or pre- or post-prostatectomy, outcome. This may be readily performed by PCR based methods known in the art, including, but not limited to, Q-PCR.

Conversely, detection of the DNA of an identified gene as amplified may be used for genes that have up-regulated expression in correlation with a particular prostate cancer, or pre- or post-prostatectomy, outcome. This may be readily performed by PCR based, fluorescent in situ hybridization (FISH) and chromosome in situ hybridization (CISH) methods known in the art.

Expression based on detection of a presence, increase, or decrease in protein levels or activity may also be used, e.g. in addition to calculating a PCPI for an individual sample/patient. Detection may be performed by any immunohistochemistry (IHC) based, blood based (especially for secreted proteins), antibody (including autoantibodies against the protein) based, exfoliate cell (from the cancer) based, mass spectroscopy based, and image (including used of labeled ligand) based method known in the art and recognized as appropriate for the detection of the protein. Antibody and image based methods are additionally useful for the localization of tumors after determination of cancer by use of cells obtained by a non-invasive procedure (such as lavage or needle aspiration), where the source of the cancerous cells is not known. A labeled antibody or ligand maybe used to localize the carcinoma(s) within a patient.

Embodiments of the present invention relating to the use of a nucleic acid based assay to determine expression are accomplished by immobilization of one or more sequences of the marker genes identified herein as a polynucleotide on a solid support, including, but not limited to, a solid substrate as an array or to beads or bead based technology as known in the art. In some embodiments, the assay is DASL (cDNA-mediated Annealing, Selection, extension and Ligation) assay available from Illumina. Alternatively, solution based expression assays known in the art may also be used. The immobilized polynucleotide probes may be unique or otherwise specific to the disclosed genes (or expressed sequences) such that the polynucleotides are capable of hybridizing to a DNA or RNA corresponding to the genes (or expressed sequences). These polynucleotides may be the full length of the genes (or expressed sequences) or be probes of shorter length (up to one nucleotide shorter than the full length sequence known in the art by deletion from the 5' or 3' end of the sequence) that are optionally minimally interrupted (such as by mismatches or inserted non-complementary basepairs) so that their hybridization with cognate DNA or RNA corresponding to the genes (or expressed sequences) is not affected. In many embodiments, a polynucleotide probe contains sequence from the 3' end of a disclosed gene or expressed sequence. Polynucleotide probes containing mutations relative to the sequences of the disclosed genes may also be used so long as the presence of the mutations still allows hybridization to produce a detectable signal. The immobilized polynucleotides may be used to determine the state of nucleic acid samples prepared from sample prostate cell(s) for which the outcome of the sample's subject (e.g. patient from whom the sample is obtained) is not known or for confirmation of an outcome that is already assigned to the sample's subject. Without limiting the invention, such a cell may be from a patient with prostate cancer, such as material removed by prostatectomy. The immobilized polynucleotide(s) need only be sufficient to specifically hybridize to the corresponding nucleic acid molecules derived from the sample under suitable conditions. While expression of even a single correlated gene may to able to provide adequate accuracy in discriminating between two prostate cancer outcomes, the invention includes use of expression levels from more than one gene or expressed sequence. In particular, the genes (expressed sequences) of the set of 17, 18, 19, or the set of 57 referred to in the examples section (infra), are used.

In some embodiments, the nucleic acid derived from the sample prostate cancer cell(s) may be preferentially amplified by use of appropriate primers (such as used in PCR) such that only the genes to be analyzed are amplified to reduce contaminating background signals from other expressed genes or sequences in the sample. The size of a PCR amplicon of the invention may be of any size, including at least or about 50, at least or about 100, at least about or 150, at least or about 200, at least or about 250, at least or about 300, at least or about 350, or at least or about 400 consecutive nucleotides, all with inclusion of the portion complementary to the PCR primers used. Of course the PCR may optionally be reverse-transcription coupled PCR (or RT-PCR in the case of RNA starting material) or quantitative PCR, such as real-time PCR, or combinations thereof. Of course RNA from the samples described herein may be prepared and used by means readily known to the skilled person. Alternatively, and where multiple genes are to be analyzed or where very few cells (or one cell) is used, the nucleic acid from the sample may be globally amplified before hybridization to immobilized polynucleotide probes, such as on an array or microarray. Of course RNA, or the cDNA counterpart thereof may be directly labeled and used, without amplification, by methods known in the art. A preferred embodiment of the methods for detection of the herein described marker genes is RNA sequencing.

The invention provides a more objective set of criteria, in the form of gene expression profiles of a discrete set of marker genes, to discriminate (or delineate) between prostate cancer, or pre- or post-prostatectomy, outcomes, and allows for the prediction of a predisposition for aggressive versus indolent disease. In some embodiments, the assays are used to discriminate between better and poorer outcomes within about 6 months, 1 year, 2 years, 3 years, 4 years, 5 years, 7 years, 10 years, 15 years or even later.

While better and poorer cancer recurrence, metastasis and/or survival outcomes may be defined relatively in comparison to each other, a "better" outcome or a "good prognosis" may be viewed as one that is better than a 50% chance of cancer recurrence and/or 50% chance of survival after about 60 months post surgical intervention to remove prostate cancer tumor(s). A "better" outcome or a "good prognosis" may also be a better than about 60%, about 70%, about 80% or about 90% cancer recurrence and/or chance of survival about 60 months post surgical intervention. A "poorer" outcome/prognosis may be viewed as a 50% or more chance of cancer recurrence and/or less than 50% chance of survival after about 60 months post surgical intervention to remove prostate cancer tumor(s). The PCPI and the methods according to the invention help classifying patients into those that have the above prognosis.

The disclosed methods may also be used with solid tissue material. For example, a solid biopsy may be collected and prepared for visualization followed by determination of expression of two or more genes or expressed sequences identified herein to determine the prostate cancer, or pre- or post-prostatectomy, outcome. One means is by use of in situ hybridization with polynucleotide identifying probe(s) for assaying expression of said gene(s).

In some embodiments, the detection of gene expression from the samples may be by use of a single microarray able to assay gene expression from some or all genes disclosed herein for convenience and accuracy.

Other uses of the invention include providing the ability to identify prostate cancer cell samples as correlated with particular prostate cancer survival or recurrence outcomes for further research or study. This provides a particular advantage in many contexts requiring the identification of cells based on objective genetic or molecular criteria.

The materials for use in the methods of the present invention are ideally suited for preparation of kits produced in accordance with well known procedures. The invention thus provides kits comprising agents for the detection of expression of the disclosed genes and sequences for identifying prostate cancer, or pre- or post-prostatectomy, outcomes. Such kits optionally comprise the agent with an identifying description or label or instructions relating to their use in the methods of the present invention, is provided. Such a kit may comprise containers, each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, RGTP and UTP), reverse transcriptase, DNA polymerase, RNA polymerase, and one or more primer complexes of the present invention (e.g., appropriate length poly(T) or random primers linked to a promoter reactive with the RNA polymerase). A set of instructions will also typically be included.

The methods provided by the invention may also be automated in whole or in part. All aspects of the invention may also be practiced such that they consist essentially of a subset of the disclosed genes to the exclusion of material irrelevant to the identification of prostate cancer recurrence, metastasis, and/or survival outcomes via a cell containing sample.

In the context of the methods of the present invention, the PCPI is used to diagnose prostate cancer in patients, identify prostate cancer in patients, and to establish a prognosis for prostate cancer patients, to determine the predisposition to develop aggressive versus indolent disease, and to stratify prostate cancer patients so that a decision can be made by the treating physician on how, if at all, a patient should be treated. The PCPI can be used as sole parameter for the above purposes or it can be used in combination with other known diagnostic, prognostic, or stratification methods to provide further support for the physician.

The term "diagnosing prostate cancer" as used herein means that a patient or individual may be considered to be suffering from a prostate cancer, when the expression level of the GOI'(s) of the present invention is either up-regulated or down-regulated, compared to a control level as defined herein above, preferably if compared to the normal control level as defined herein above so that the PCPI is significantly higher than/different from that found in a control tissue. The term "diagnosing" also refers to the conclusion reached through that comparison process.

In another embodiment of the present invention, the diagnosis may be combined with the elucidation of additional cancer biomarker expression levels, in particular prostate cancer biomarkers. Several particular prostate cancer biomarkers would be known to the person skilled in the art. For example, the expression of biomarkers like PSA maybe tested.

A prostate cancer may be considered as being diagnosed when the expression level of the GOI's of the present invention is up-regulated or down-regulated, compared to the normal control level as defined herein above and when the PCPI is (significantly higher than the one obtained with control material).

In a further preferred embodiment a prostate cancer may considered as being diagnosed or a predisposition to develop aggressive vs. indolent disease be determined, if the expression level of the GOI's referred to herein, is up-regulated or down-regulated by a value of between 20% to 80%, preferably by a value of 30%, 40%, 50%, 60% or 70% in a test sample in comparison to a control level, preferably a normalized control level. The control level may either be a normal control level or a cancerous control level, as defined herein above. In a particularly preferred embodiment a prostate cancer may be considered as being diagnosed or a predisposition to develop aggressive vs. indolent disease if the expression level, as defined herein above, is up-regulated or down-regulated by a factor of 1.5- to 10-fold, preferably by a factor of 2- to 5-fold in a test sample in comparison to a control level, in particular a healthy tissue or a benign prostate tumor or very preferably in comparison to prostate cancer tissue of the same patient at initial diagnosis.

The term "detecting prostate cancer" as used herein means that the presence of a prostate cancer disease or disorder in an organism may be determined or that such a disease or disorder may be identified in an organism. The determination or identification of a prostate cancer disease or disorder may be accomplished by a comparison of the expression level of the GOI's of the present invention and the normal control level as defined herein above, or by determining the PCPI. A prostate cancer may be detected when the expression level of the GOI's is up-regulated or down-regulated in comparison to the normal control level as defined herein above. In a preferred embodiment of the present invention a prostate cancer may be detected if the expression level of the GOI's is similar to an expression level of an established, e.g. independently established, prostate cancer cell or cell line, e.g. a prostate cancer cell line, or a cell line as mentioned herein above.

The terms "monitoring prostate cancer " and "identifying a subject with a predisposition of prostate cancer that is susceptible to disease progression" (i.e. identification of aggressive disease versus indolent disease), as used herein relates to the accompaniment of a diagnosed or detected prostate cancer disease or disorder, e.g. during or after a treatment procedure or during or after a certain period of time, typically during 2 months, 3 months, 4 months, 6 months, 1 year, 2 years, 3 years, 5 years, 10 years, or any other period of time during or after the start or end of the primary treatment. The term "accompaniment" means that states of disease or the PCPI as defined herein above and, in particular, changes of these states of disease or PCPI may be detected by comparing the expression levels of the GOI's of the present invention in a sample to a normal control level as defined herein above, preferably a control expression level derived from a benign tumor control or a healthy control or to the expression level of an established, e.g. independently established, prostate cancer cell or cell line, e.g. a prostate cancer cell line, or a cell line in any type of periodical time segment, e.g. every week, every 2 weeks, every month, every 2, 3, 4, 5, 6, 7, 8, 9, 10, 11 or 12 month, every 1.5 year, every 2, 3, 4, 5, 6, 7, 8,9 or 10 years, during any period of time, e.g. during 2 weeks, 3 weeks, 1, 2, 3, 4, 5, 6, 7, 8, 9, 10, 11, 12 months, 1.5, 2, 3, 4, 5, 6, 7, 8, 9, 10, 15 or 20 years, respectively. The established, e.g. independently established, prostate cancer cell or cell line giving rise to an additional control level may be derived from samples corresponding to different stages of cancer development, e.g. stages 0 and I to IV of the TNM classification system. In a preferred embodiment of the present invention the term relates to the accompaniment of a diagnosed prostate cancer, e.g. a malignant, hormone- sensitive prostate cancer. The monitoring may also include the detection of the expression of additional genes or genetic elements, e.g. housekeeping genes like GAPDH or PBGD. The present invention also relates to methods of monitoring patients with a view to identify changes in the herein disclosed signature genes using the PCPI as disclosed throughout the specification in order to identify those patients likely developing aggressive prostate cancer. These patients will then be treated as described throughout the specification.

The term "prognosticating (aggressive) prostate cancer" as used herein refers to the prediction of the course or outcome of a diagnosed or detected aggressive prostate cancer, e.g. during a certain period of time, during a treatment or after a treatment. The term also refers to a determination of chance of survival or recovery from the disease, as well as to a prediction of the expected survival time of a patient. A prognosis may, specifically, involve establishing the likelihood for survival of a patient during a period of time into the future, such as 6 months, 1 year, 2 years, 3 years, 5 years, 10 years or any other period of time.

The terms "progression towards prostate cancer" and "progression towards aggressive (versus indolent) prostate cancer" as used herein relates to a switch between different stages of cancer development, e.g. stages 0 and I to IV of the TNM classification, or any other stage or sub-stage, starting from a healthy condition up to malignant, hormone-sensitive or malignant hormone-resistant prostate cancer. Typically such switches are accompanied by a modification of the expression level of marker gene(s) or GOI's and a different PCPI as used herein, in a test sample in comparison to a previous test sample from the same individual, e.g. in comparison to a sample derived from a benign prostate tumor control or a healthy tissue control. A progression towards aggressive prostate cancer may be considered as being detected or diagnosed if the expression level of GOI's used for calculating the PCPI, as defined herein above, is different, e.g. by a value of between 3% to 50%, preferably by a value of 10%, 15%, 20% or 25% in a test sample in comparison to a previous PCPI calculated from the gene expression values in a test sample from the same individual. The modification may be detected over any period of time, preferably over 1, 2, 3, 4, 5, 6, 7, 8, 9, 10, 11, 12 months, 1.5, 2, 3, 4, 5, 6, 7, 8, 9, 10, 15 or 20 years, i.e. the value indicated above may be calculated by comparing the expression level of marker gene(s) or GOI's or the PCPI at a first point in time and at a second point in time after the above indicated period of time.

In a particularly preferred embodiment of the present invention the term "progression towards prostate cancer" relates to a switch from a healthy state or a benign prostate tumor state to a malignant prostate cancer state. A progression from a healthy state to a prostate cancer state may be considered as being detected or diagnosed if the PCPI, as defined herein above, is higher by a value of between 20% to 50%, preferably by a value of 20%, 25%, 30% or 35% in a test sample in comparison to a previous test sample from the same individual, which was diagnosed as being healthy. Alternatively, for the comparison test samples from other individuals may be used, e.g. test samples of healthy individuals. Also envisaged is the use of available database information on the expression or the employment of cancer cell collection samples etc.

A progression from a benign prostate tumor state to a malignant prostate cancer state may be considered as being detected or diagnosed if the PCPI, as defined herein above, is higher by a value of between 3% to 30%, preferably by a value of 5%, 10%, 15%, or 20% in a test sample in comparison to a previous test sample from the same individual, which has been diagnosed as suffering from a benign prostate tumor. Alternatively, for the comparison test samples from other individuals may be used, e.g. test samples of individuals diagnosed to suffer from benign prostate tumors. Also envisaged is the use of available database information on the expression or the employment of cancer cell collection samples etc.

The modification may be detected over any period of time, preferably over 1, 2, 3, 4, 5, 6, 7, 8, 9, 10, 11, 12 months, 1.5, 2, 3, 4, 5, 6, 7, 8, 9, 10, 15 or 20 years, i.e. the value indicated above may be calculated by comparing the expression level of marker gene(s) or GOI's at a first point in time and at a second point in time after the above indicated period of time.

In a further embodiment the present invention relates to the diagnosis and detection of a predisposition for developing prostate cancer. A "predisposition for developing (aggressive) prostate cancer" in the context of the present invention is a state of risk of developing (aggressive) prostate cancer. Preferably a predisposition for developing aggressive prostate cancer may be present in cases in which the GOI's expression level or the PCPI defined herein above is significantly different from a control level as defined herein, i.e. a reference expression level or reference PCPI derived from tissues or samples of a patient which are evidently healthy, or from a sample or tissues of a patient obtained before initial diagnosis of prostate cancer, so that also the PCPI calculated on the basis of the gene expression in the cancerous tissues is higher than the PCPI obtained for the control sample. Thus, a predisposition for aggressive prostate cancer may be considered as being diagnosed or detected if the above depicted situation is observed.

Generally, and also in the establishment of the PCPI, the difference between the expression levels of a test biological sample and a control level can be normalized to the expression level of further control nucleic acids, e.g. housekeeping genes whose expression levels are known not to differ depending on the cancerous or noncancerous state of the cell. Exemplary control genes include inter alia β-actin, glycerinaldehyde 3 -phosphate dehydrogenase (GAPDH), porphobilinogen deanimase (PBGD) and the like.

In the context of the present invention, the terms "diagnosing" and "prognosticating" are also intended to encompass predictions and likelihood analyses. The PCPI as a parameter may accordingly be used clinically in making decisions concerning treatment modalities, including therapeutic intervention or diagnostic criteria such as a surveillance for the disease. According to the present invention, an intermediate result for examining the condition of a patient may be provided. Such intermediate result may be combined with additional information to assist a doctor, nurse, or other practitioner to diagnose that a patient suffers from the disease. Alternatively, the present invention may be used to detect cancerous cells in a patient-derived tissue, and provide a doctor with useful information to diagnose that the patient suffers from the disease.

A patient or individual to be diagnosed, monitored or in which a prostate cancer, a progression towards aggressive versus indolent prostate cancer or a predisposition for aggressive versus indolent prostate cancer is to be detected or prognosticated according to the present invention is an animal, preferably a mammal, more preferably a human being. The use of molecular imaging tools as known to the person skilled in the art, e.g. magnetic resonance imaging (MRI) and/or magnetic photon resonance imaging (MPI) technology and/or multi-parametric MRI (mpMRI) technology in the in addition to the PCPI as a marker for diagnosing, detecting, monitoring or prognosticating prostate cancer of the progression towards aggressive versus indolent prostate cancer.

In a further aspect the present invention relates to a composition for diagnosing, detecting, monitoring or prognosticating prostate cancer or the progression towards prostate cancer or a predisposition for prostate cancer in an individual. The composition according to the present invention may comprise a nucleic acid affinity ligand for the GOI expression product(s).

The term "nucleic acid affinity ligand for the GOI expression product" as used herein refers to a nucleic acid molecule being able to specifically bind to a GOI transcript. The nucleic acid affinity ligand may also be able to specifically bind to a nucleic acid sequence being at least 90%, 91%, 92%, 93%, 94%, 95%, 96%, 97%, 98% or 99% identical to GOI sequences, or to any fragments of said sequences.

The term "expression product" as used herein refers to a transcript of a GOI or an mRNA molecule generated by the expression of the GOI. More preferably, the term relates to a processed transcript as defined herein above.

The composition of the present invention may, for example, comprise a set of oligonucleotides specific for the GOI expression products and/or a probe specific for the GOI expression products. The term "oligonucleotide specific for the GOI expression products" as used herein refers to a nucleotide sequence which is complementary to the sense- or antisense-strand of a GOI.

The oligonucleotide may have any suitable length and sequence known to the person skilled in the art, as derivable from the sequence of the GOI or its complement. Typically, the oligonucleotide may have a length of between 8 and 60 nucleotides, preferably of between 10 and 35 nucleotides, more preferably a length of 11, 12, 13, 14, 15, 16, 17, 18, 19, 20, 21, 22, 23, 24, 25, 26, 27, 28, 29, 30, 31, 32 or 33 nucleotides. Oligonucleotide sequences, for instance, oligonucleotides usable as a forward and reverse primers specific for the GOI expression products may be defined with the help of software tools known to the person skilled in the art.

The term "probe specific for the GOI expression product" as used herein means a nucleotide sequence which is complementary to the sense- or antisense-strand of the herein described GOI's.

The probe may have any suitable length and sequence known to the person skilled in the art, as derivable from the sequence of the herein described GOI's. Typically, the probe may have a length of between 6 and 300 nucleotides, preferably of between 15 and 60 nucleotides, more preferably a length of 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides. Probe sequences specific for the GOI's expression product may be defined with the help of software tools known to the person skilled in the art.

A nucleic affinity ligand, as described herein above, may be labeled with various markers or may be detected by a secondary affinity ligand, labeled with various markers, to allow detection, visualization and/or quantification. This can be accomplished by using any suitable labels, which can be conjugated to the affinity ligand capable of interaction with the expression product or to any secondary affinity ligand, using any suitable technique or methods known to the person skilled in the art. The term "secondary affinity ligand" refers to a molecule which is capable of binding to the affinity ligand as defined herein above (i.e. a "primary affinity ligand" if used in the context of a system with two interacting affinity ligands). The binding interaction is preferably a specific binding.

Examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bio luminescent proteins (e.g. luciferin, luciferase), haptens (e.g. biotin).

In embodiments of the present invention, nucleic affinity ligands to be used as probes, in particular a probe specific for the GOI expression products as defined herein above, maybe labeled with a fluorescent label like 6-FAM, HEX, TET, ROX, Cy3, Cy5, Texas Red or Rhodamine, and/or at the same time with a quenching label like TAMRA, Dabcyl, Black Hole Quencher, BHQ-I or BHQ-2. A variety of other useful fluorescents and chromophores are described in Stryer, 1968, Science, 162:526-533. Affinity ligands may also be labeled with enzymes (e.g. horseradish peroxidase, alkaline phosphatase, beta-lactamase), radioisotopes (e.g. 3H, 14C, 32P, 33P, 35S, 1251) or particles (e.g. gold). The different types of labels may be conjugated to an affinity ligand using various chemistries, e.g. the amine reaction or the thiol reaction. However, other reactive groups than amines and thiols can also be used, e.g. aldehydes, carboxylic acids and glutamine.

In a specific embodiment of the present invention a composition may additionally comprise accessory ingredients like PCR buffers, dNTPs, a polymerase, ions like bivalent cations or monovalent cations, hybridization solutions, detection dyes and any other suitable compound or liquid necessary for the performance of a detection as defined herein above, which is known to the person skilled in the art.

In another aspect the present invention relates to the use of a nucleic acid for the GOI expression product, as defined herein above, for the preparation of a composition for diagnosing, detecting, monitoring or prognosticating prostate cancer or the progression towards aggressive versus indolent prostate cancer or a predisposition for aggressive versus indolent disease in an individual, as described herein above.

In a preferred embodiment the present invention relates to the use of a set of oligonucleotides specific for the GOI expression products and/or a probe specific for the expression products, as defined herein above, for the preparation of a composition for diagnosing, detecting, monitoring or prognosticating prostate cancer or the progression towards aggressive versus indolent prostate cancer or a predisposition for aggressive versus indolent disease in an individual, as described herein above.

In a preferred embodiment of the present invention a composition as defined herein above is a diagnostic composition.

In another aspect the present invention relates to a diagnostic kit for detecting, diagnosing, monitoring or prognosticating prostate cancer or the progression towards prostate cancer or a predisposition for aggressive prostate cancer, comprising a set of oligonucleotides specific for the GOI expression products, a probe specific for the GOI expression products.

Typically, the diagnostic kit of the present invention contains one or more agents allowing the specific detection of marker gene(s) or GOI's as defined herein above. The agents or ingredients of a diagnostic kit may, according to the present invention, be comprised in one or more containers or separate entities. The nature of the agents is determined by the method of detection for which the kit is intended.

Furthermore, the kit may comprise an amount of a known nucleic acid molecule, which can be used for a calibration of the kit or as an internal control. Typically, a diagnostic kit for the detection of marker gene(s) or GOI's expression products may comprise accessory ingredients like a PCR buffers, dNTPs, a polymerase, ions like bivalent cations or monovalent cations, hybridization solutions etc. Such ingredients are known to the person skilled in the art and may vary depending on the detection method carried out. Additionally, the kit may comprise an instruction leaflet and/or may provide information as to the relevance of the obtained results.

In other aspects the present invention relates to methods for detecting, diagnosing, monitoring or prognosticating prostate cancer or the progression towards aggressive versus indolent prostate cancer, e.g. in an individual, comprising at least the step of determining the level of marker gene(s) or GOI's in a sample. The term "determining the level of marker gene(s) or GOI's" refers to the determination of the presence or amount of marker gene(s) or GOI's expression products. The term "level of marker gene(s) or GOI's" thus means the presence or amount of marker gene(s) or GOI's expression products, e.g. transcript(s), and/or the determination of the presence or amount of marker gene(s) or GOI's. The determination of the presence or amount of marker gene(s) or GOI's expression products, may be accomplished by any means known in the art.

In a preferred embodiment of the present invention the determination of the presence or amount of marker gene(s) or GOI's expression products is accomplished by the measurement of nucleic acid. Thus, the expression level(s) may be determined by a method involving the detection of an mRNA encoded by the gene.

For example, the measurement of the nucleic acid level of marker gene(s) or GOI's expression may be assessed by purification of nucleic acid molecules (e.g. RNA or cDNA) obtained from the sample, followed by hybridization with specific oligonucleotide probes as defined herein above. Comparison of expression levels may be accomplished visually or by means of an appropriate device. Methods for the detection of mRNA or expression products are known to the person skilled in the art.

Alternatively, the nucleic acid level of marker gene(s) or GOI's expression may be detected in a DNA array or microarray approach. Typically, sample nucleic acids derived from patients to be tested are processed and labeled, preferably with a fluorescent label. Subsequently, such nucleic acid molecules may be used in a hybridization approach with immobilized capture probes corresponding to the marker genes of the present invention. Suitable means for carrying out microarray analyses are known to the person skilled in the art.

In a standard setup a DNA array or microarray comprises immobilized high-density probes to detect a number of genes. The probes on the array are complementary to one or more parts of the sequence of the marker genes. Typically, cDNAs, PCR products, and oligonucleotides are useful as probes.

A DNA array- or microarray-based detection method typically comprises the following steps: (1) Isolating mRNA from a sample and optionally converting the mRNA to cDNA, and subsequently labeling this RNA or cDNA. Methods for isolating RNA, converting it into cDNA and for labeling nucleic acids are described in manuals for micro array technology. (2) Hybridizing the nucleic acids from step 1 with probes for the marker genes. The nucleic acids from a sample can be labeled with a dye, such as the fluorescent dyes Cy3 (red) or Cy5 (blue). Generally a control sample is labeled with a different dye. (3) Detecting the hybridization of the nucleic acids from the sample with the probes and determining at least qualitatively, and more particularly quantitatively, the amounts of mRNA in the sample for marker genes investigated. The difference in the expression level between sample and control can be estimated based on a difference in the signal intensity. These can be measured and analyzed by appropriate software such as, but not limited to the software provided for example by Affymetrix.

There is no limitation on the number of probes corresponding to the marker genes used, which are spotted on a DNA array. Also, a marker gene can be represented by two or more probes, the probes hybridizing to different parts of a gene. Probes are designed for each selected marker gene. Such a probe is typically an oligonucleotide comprising 5-50 nucleotide residues. Longer DNAs can be synthesized by PCR or chemically. Methods for synthesizing such oligonucleotides and applying them on a substrate are well known in the field of micro-arrays. Genes other than the marker genes may be also spotted on the DNA array. For example, a probe for a gene whose expression level is not significantly altered may be spotted on the DNA array to normalize assay results or to compare assay results of multiple arrays or different assays.

Alternatively, the nucleic acid level of marker gene(s) or GOI's expression may be detected in a quantitative RT-PCR approach, preferably in a real-time PCR approach following the reverse transcription transcripts of interest. Typically, as first step, a transcript is reverse transcribed into a cDNA molecule according to any suitable method known to the person skilled in the art. A quantitative or real-time PCR approach may subsequently be carried out based on a first DNA strand obtained as described above.

Preferably, Taqman or Molecular Beacon probes as principal FRET-based probes of this type may be used for quantitative PCR detection. In both cases, the probes, serve as internal probes which are used in conjunction with a pair of opposing primers that flank the target region of interest, preferably a set of marker gene(s) specific oligonucleotides as defined herein above. Upon amplification of a target segment, the probe may selectively bind to the products at an identifying sequence in between the primer sites, thereby causing increases in FRET signaling relative to increases in target frequency.

Preferably, a Taqman probe to be used for a quantitative PCR approach according to the present invention may comprises a specific oligonucleotide as defined above of about 22 to 30 bases that is labeled on both ends with a FRET pair. Typically, the 5' end will have a shorter wavelength fluorophore such as fluorescein (e.g. FAM) and the 3' end is commonly labeled with a longer wavelength fluorescent quencher (e.g. TAMRA) or a non-fluorescent quencher compound (e.g. Black Hole Quencher). It is preferred that the probes to be used for quantitative PCR, in particular probes as defined herein above, have no guanine (G) at the 5' end adjacent to the reporter dye in order to avoid quenching of the reporter fluorescence after the probe is degraded.

A Molecular Beacon probe to be used for a quantitative PCR approach according to the present invention preferably uses FRET interactions to detect and quantify a PCR product, with each probe having a 5' fluorescent-labeled end and a 3' quencher-labeled end. This hairpin or stem-loop configuration of the probe structure comprises preferably a stem with two short self-binding ends and a loop with a long internal target-specific region of about 20 to 30 bases.

Alternative detection mechanisms which may also be employed in the context of the present invention are directed to a probe fabricated with only a loop structure and without a short complementary stem region. An alternative FRET -based approach for quantitative PCR which may also be used in the context of the present invention is based on the use of two hybridization probes that bind to adjacent sites on the target wherein the first probe has a fluorescent donor label at the 3 ' end and the second probe has a fluorescent acceptor label at its 5' end.

In yet another embodiment as a further, additional step a decision on the presence or stage of cancer or the progression of cancer may be based on the results of a comparison step. A malignant, hormone- sensitive prostate cancer may be diagnosed or prognosticated or a progression towards (aggressive) prostate cancer may be diagnosed or prognosticated in said method according to the corresponding definitions provided herein above in the context of marker gene(s) or GOI's as marker for malignant prostate cancer.

In another embodiment the present invention relates to a method for detecting, diagnosing, monitoring or prognosticating (aggressive) prostate cancer or the progression towards (aggressive) prostate cancer comprising at least the steps of:

(a) testing in at least one sample obtained from at least one individual suspected to suffer from prostate cancer for expression level of the GOI expression products;
(b) testing in at least one control sample for the expression level of the GOI expression product;
(c) determining the difference in the expression of steps (a) and (b); and
(d) deciding on the presence or stage of prostate cancer or the progression towards aggressive prostate cancer based on the results obtained in step (c).

In one embodiment, steps a), b), c) and/or d) of this method of diagnosis may be performed outside the human or animal body, e.g. in samples obtained from a patient or individual.

In another aspect the present invention relates to a method for diagnosing, monitoring or prognosticating, e.g. aggressive, prostate cancer or the progression towards aggressive prostate cancer, wherein said method discriminates between a benign and aggressive prostate cancer, comprising the steps of

(a) determining the level of marker gene(s) or GOI's,
(b) determining the level of expression of a reference gene in a sample;
(c) normalizing the measured expression level of marker gene(s) or GOI's to the expression of the reference gene; and
(d) comparing the normalized expression level with a predetermined cutoff value chosen to exclude benign prostate tumor, wherein a normalized expression level above the cutoff value is indicative of a aggressive prostate cancer, wherein said cutoff value is between -2 and +2, preferably about 0.

Expression results may be normalized according to any suitable method known to the person skilled in the art. Typically, such tests or corresponding formula, which would be known to the person skilled in the art, would be used to standardize expression data to enable differentiation between real variations in gene expression levels and variations due to the measurement processes. For microarrays, the Robust Multi-array Average (RMA) may be used as normalization approach.

In a specific embodiment the normalized values are generated by applying the following:

$$N(Cq_{\textit{gene of interest}}) = \text{Mean}\ (Cq_{\textit{ref gene}}) - (Cq_{\textit{gene of interest}})$$

Where $N(Cq_{gene\ of\ interest})$ is normalized gene expression value for selected genes of interest; where $Mean(Cq_{ref\ gene})$ is the arithmetic mean of the PCR Cq values of the selected combination of reference genes; where $(Cq_{gene\ of\ interest})$ is the PCR Cq value of the gene of interest.

In a specific embodiment the normalized values are generated by applying the following:

$$N(Cq_{\textit{gene of interest}}) = \text{Mean}(Cq_{\textit{ref gene}}) - (Cq_{\textit{gene of interest}})$$

Where $N(Cq_{gene\ of\ interest})$ is normalized gene expression value for selected genes of interest; where $Mean(Cq_{ref\ gene})$ is the arithmetic mean of the PCR Cq values of the of at least two reference genes selected from TBP, HPRT1, ACTB, RPLPO, POLR2A, B2M, PUM1, K-ALPHA-1 and ALAS-1; where $(Cq_{gene\ of\ interest})$ is the PCR Cq value of the gene of interest.

In a specific embodiment the normalized values are generated by applying the following:

$$N(Cq_{\textit{gene of interest}}) = Mean(Cq_{\textit{ref gene}}) - (Cq_{\textit{gene of interest}})$$

Where $N(Cq_{gene\ of\ interest})$ is normalized gene expression value for selected genes of interest; where $Mean(Cq_{ref\ gene})$ is the arithmetic mean of the PCR Cq values of the reference genes TBP, HPRT1, ACTB and RPLP0; where $(Cq_{gene\ of\ interest})$ is the PCR Cq value of the gene of interest.

In a specific embodiment the normalized values are generated by applying the following:

$$N(Cq_{\textit{gene of interest}}) = Mean(Cq_{\textit{ref gene}}) - (Cq_{\textit{gene of interest}})$$

Where $N(Cq_{gene\ of\ interest})$ is normalized gene expression value for selected genes of interest; where $Mean(Cq_{ref\ gene})$ is the arithmetic mean of the PCR Cq values of the of the reference genes TBP, HPRT1, PUM1, K-ALPHA-1 and ALAS-1 where $(Cq_{gene\ of\ interest})$ is the PCR Cq value of the gene of interest.

Exemplary reference genes include inter alia Homo sapiens TATA box binding protein (TBP), Homo sapiens hypoxanthine phosphoribosyltransferase 1 (HPRT1), Homo sapiens actin, beta, mRNA (ACTB), Homo sapiens 60S acidic ribosomal phosphoprotein P0 mRNA (RPLP0), Homo sapiens pumilio RNA-Binding Family Member (PUM1), Polymerase (RNA) II (DNA Directed) Polypeptide A, 220kDa (POLR2A), Beta-2-Microglobulin (B2M), Tubulin-Alpha-lb (K-ALPHA-1), Aminolevulinate-Delta-Synthase (ALAS-1).

In a preferred embodiment a combination of reference genes is selected from TATA box binding protein (TBP), Homo sapiens hypoxanthine phosphoribosyltransferase 1 (HPRT1), Homo sapiens actin, beta, mRNA (ACTB), Homo sapiens 60S acidic ribosomal phosphoprotein P0 mRNA (RPLP0), Homo sapiens pumilio RNA-Binding Family Member (PUM1), Polymerase (RNA) II (DNA Directed) Polypeptide A, 220kDa (POLR2A), Beta-2-Microglobulin (B2M), Tubulin-Alpha-lb (K-ALPHA-1), Aminolevulinate-Delta-Synthase (ALAS-1).

In a specific embodiment the expression level of the signature gene is normalized to the expression of a reference gene, wherein the reference gene is preferably a housekeeping gene and wherein the housekeeping gene is preferably TBP, HPRT1, ACTB, RPLPO, PUM1, POLR2A or B2M.

In another preferred embodiment the combination of reference genes comprises TBP, HPRT1 and at least one, at least two or at least three additional reference genes selected from the group comprising ACTB, RPLPO, PUM1, POLR2A, B2M, K-ALPHA-1 (TUBAIB) or ALAS-1.

In another preferred embodiment the combination of reference genes comprises TBP, HPRT1 and at least two additional reference genes selected from the group comprising ACTB, RPLPO, PUM1, K-ALPHA-1 (TUBAIB) or ALAS-1.

A particularly preferred combination is TBP, HPRT1, ACTB, RPLP0. Another particularly preferred combination is TBP, HPRT1, PUM1, K-ALPHA-1, ALAS-1.

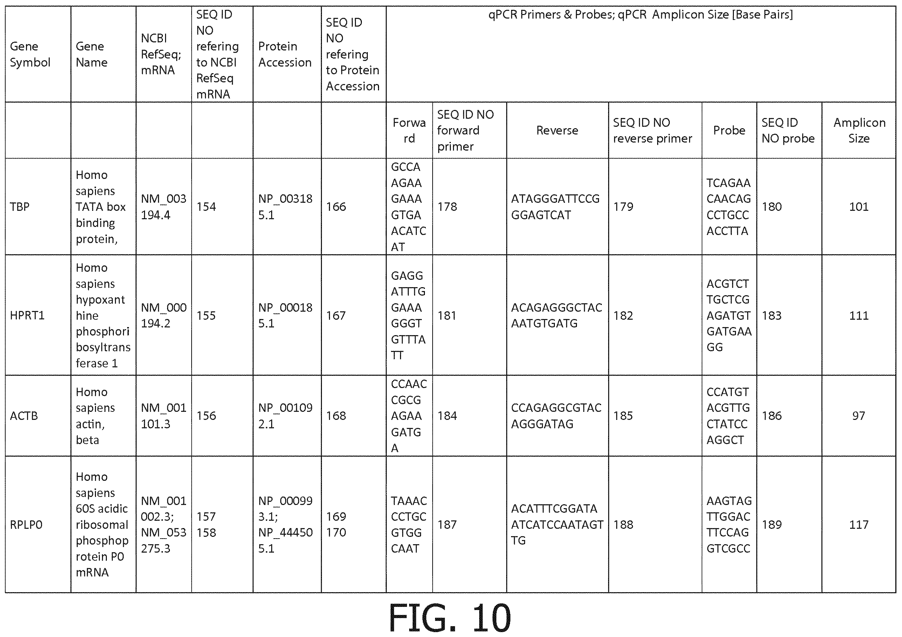

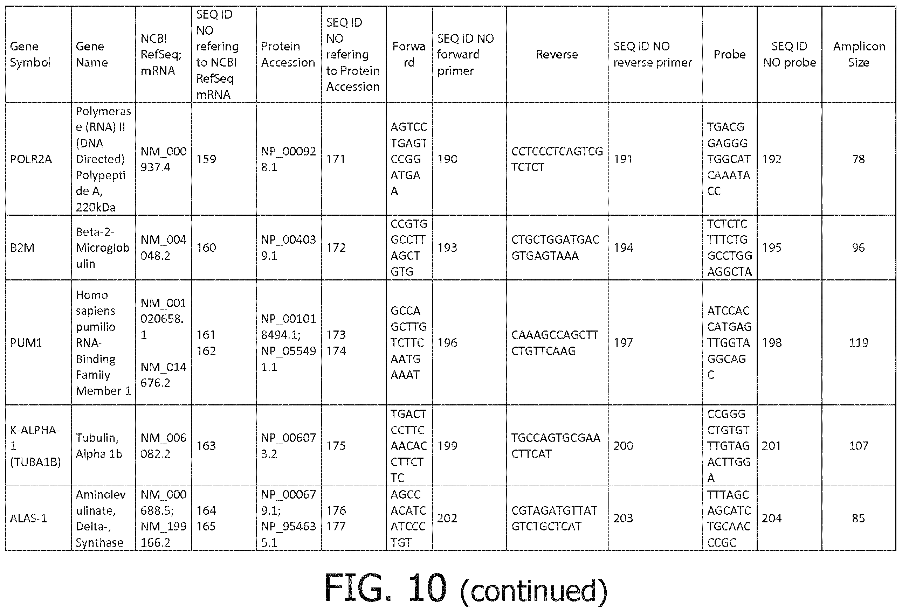

A detailed description of the reference genes including their Transcript ID (NCBI RefSeq) and the SEQ ID NO of the corresponding nucleotide sequence, the protein ID (protein accession) and the SEQ ID NO of the corresponding amino acid sequence is disclosed in Figure 10. Further Figure 10 discloses for each reference gene a forward primer, reverse primer for the generation of an amplicon which is specific for the reference gene and a probe sequence that specifically binds to the amplicon. The expression level of marker gene(s) or GOI's maybe determined on the nucleic acid, as described herein above. Preferred is the determination of the expression level of marker gene(s) or GOI's transcript(s). In addition the level of a house-keeping gene in sample may be determined.

The term "reference gene" as used herein refers to any suitable gene, e.g. to any steadily expressed and continuously detectable gene, gene product, expression product, protein or protein variant in the organism of choice.

The expression may be preferably be carried out in the same sample, i.e. the level of marker gene(s) or GOI's and of the reference gene is determined in the same sample. If the testing is carried out in the same sample, a single detection or a multiplex detection approach as described herein may be performed. For the performance of the multiplex detection the concentration of primers and/or probe oligonucleotides may be modified. Furthermore, the concentration and presence of further ingredients like buffers, ions etc. may be modified, e.g. up-regulated or down-regulated or decreased in comparison to manufacturers' indications.

Furthermore, expression results may be compared to already known results from reference cases or databases. The comparison may additionally include a normalization procedure in order to improve the statistical relevance of the results.

Expression results may be normalized according to any suitable method known to the person skilled in the art, e.g. according to normalization statistical methods like the standard score, Student's T-test, studentized residual test, standardized moment text, or coefficient variation test. Typically, such tests or corresponding formula, which would be known to the person skilled in the art, would be used to standardize expression data to enable differentiation between real variations in gene expression levels and variations due to the measurement processes.

Based on the expression results obtained in steps (a) and (b) and/or the normalized results obtained in step (c) a comparison with a cutoff value for GOI expression levels may be carried out. The cutoff value above which the expression level of marker gene(s) or GOI's is indicative of an aggressive prostate cancer, thereby excluding benign prostate tumor forms or healthy situations or healthy tissue forms, is between about -3 and + 3, -3 and + 2.75, -3 and + 2.5, -3 and + 2.25, -3 and + 2, -3 and + 1.75, -3 and +1.5, -3 and + 1.25, -3 and + 1, -3 and + 0.75, -3 and + 0.5, -3 and + 0.25, -3 and 0, -2.75 and + 3, - 2.5 and + 3, -2.25 and + 3, -2 and + 3, -1.75 and + 3, -1. 5 and + 3, -1.25 and + 3, -1 and + 3, -0.75 and + 3, -0. 5 and + 3, -0.25 and + 3, or 0 and + 3. More preferred is a cutoff value of about 0, e.g. 0.9, 0.8, 0.7, 0.6, 0.5, 0.4, 0.3, 0.2, 0.1 or -0.9, -0.8, -0.7, -0.6, -0.5, -0.4, -0.3, - 0.2, or -0.1.

If the measured and/or normalized expression levels is below the indicated cutoff value this may be seen as an indication that the individual is either healthy with respect to prostate tumors or suffers only from benign prostate tumors, but not from aggressive prostate cancer.

In another aspect the present invention relates to a method of data acquisition comprising at least the steps of:

(a) testing in an individual for expression of marker gene(s) or GOI's; and

(b) comparing the expression as determined in step (a) to a control level. The testing for expression of marker gene(s) or GOI's may be carried out according to steps as defined herein above. Preferably the testing may be carried out as measurement of nucleic acid of marker gene(s) or GOI's. The testing may be carried out in an individual, i.e. in vivo, or outside the individual, i.e. ex vivo or in vitro. The term "control level" as used in the context of the method of data acquisition refers to the expression of the marker genes referred to herein or other suitable markers in a normal control or a cancerous control, as defined herein above. The status, nature, amount and condition of the control level may be adjusted according to the necessities. Preferably a normal, healthy control level may be used. A comparison of the expression to a control level may be carried out according to any suitable method of assessing, calculating, evaluating or processing of data and particularly aims at the detection of differences between two data sets. A statistical evaluation of the significance of the difference may further be carried out. Suitable statistical methods are known to the person skilled in the art. Obtained data and information may be stored, accumulated or processed by suitable informatics or computer methods or tools known to the person skilled in the art and/or be presented in an appropriate manner in order to allow the practitioner to use the data for one or more subsequent deduction or conclusion steps.

In addition the level of a reference gene as defined herein above in a sample may be determined. Alternatively, the determination of the reference gene may be carried out with any other suitable agent or be combined with the detection of the presence or amount of nucleic acids as described herein.

In a further aspect the present invention relates to a method of identifying an individual for eligibility for prostate cancer therapy comprising:

(a) testing in a sample obtained from an individual for the expression of marker gene(s) or GOI's;

(b) testing in said sample for the expression of a reference gene and/or testing in a control sample for the expression of marker gene(s) or GOI's;

(c) classifying the levels of expression of step (a) relative to levels of step (b); and calculating respective PCPI's,

(d) identifying the individual as eligible to receive a prostate cancer therapy where the individual's sample is classified as having an up-regulated or down-regulated level of marker gene(s) or GOI's expression or an PCPI that is indicative of aggressive disease.

The term "classifying the levels of expression of step (a) relative to levels of step (b)" as used herein means that the expression in a test sample for GOI's and the expression in a control sample for GOI's are compared, e.g. after normalization against a suitable normalization references and/or after calculating respective PCPI's.

According to the calculation of the PCPI's an individual may be considered to be eligible for a prostate cancer therapy when the gene expression levels are significantly different as compared with a control sample.

The term "stratifying an individual or cohort of individuals to prostate cancer therapy" as used herein means that an individual is identified as belonging to a group of similar individuals, whose optimal therapy form is a prostate cancer therapy, e.g. a therapy in accordance with the outcome of the calculation of the PCPI as described herein above.

An individual being considered to be eligible for prostate cancer therapy or being stratified to a prostate cancer therapy as described herein above may receive any suitable therapeutic treatment for this prostate cancer form known to the person skilled the art. In particular, the term "prostate cancer therapy" as used herein refers to any suitable prostate cancer therapy known to the person skilled in the art, and preferably includes surgical castration by removal of the testes as the main organ of male sex hormone production, chemical castration by e.g., hormone therapy such as suppression of generation of androgens or by inhibition of the androgen receptor activity, cytotoxic, chemotherapy, radiation therapy (External Beam Radiation Therapy, Brachytherapy), cryotherapy, focal therapies like HIFU ablation (High Frequency Ultrasound ablation), or thermal ablation or combinations thereof, preferably the combination of hormone therapy and radiation therapy.

Typically, an individual considered to be eligible for prostate cancer therapy may be deemed to be suffering from (aggressive) prostate cancer or be prone to develop such in the future, e.g. within the next 1 to 24 months. A correspondingly identified or stratified individual may be treated with an inhibitory pharmaceutical composition. In a further embodiment a correspondingly identified individual may be treated with an inhibitory pharmaceutical composition in combination with an additional cancer therapy. The term "additional cancer therapy" refers to any types of cancer therapy known to the person skilled in the art. Preferred are cancer therapy forms known for prostate cancer. The term includes, for example, all suitable forms of chemotherapy, radiation therapy, surgery, antibody therapies etc.

Alternatively, a correspondingly identified or stratified individual may also be treated solely with one or more cancer therapies such as a chemotherapy, radiation therapy, surgery, antibody therapies etc. Preferred are cancer therapies typically used for prostate cancer, more preferred cancer therapies used for aggressive prostate cancer.

In a further embodiment of the present invention the classification method for eligibility or for stratification as described herein above may also be used for monitoring the treatment of an individual, e.g. an individual being classified as suffering from an aggressive prostate cancer. The monitoring process may be carried out as expression determination over a prolonged period of time, e.g. during or after treatment sessions, for 1, 2, 3, 4, 5, 6, 7, 8, 9, 10 weeks, 1, 2, 3, 4, 5, 6, 7, 8, 9, 10 months, or 1, 2, 3 or more years. The determination steps may be carried out in suitable intervals, e.g. every week, 2 weeks, 3 weeks, every month, 2 months, 3 months, 6 months, 12 months etc. In a further embodiment of the present invention any treatment scheme as mentioned herein above may be adjusted, e.g. enforced or attenuated, or altered in any suitable manner in correspondence with the results of the monitoring process.

In a further embodiment of the present invention the method of identifying an individual for eligibility for aggressive prostate cancer therapy based on the expression of marker gene(s) or GOI's and the corresponding PCPI as described herein above may further be combined with one or more similar identification methods, based on the expression of one or more different biomarkers. Preferred is the determination of the level of prostate specific antigen (PSA) in blood. Thus, if the level of PSA in blood is encountered to be of a range of about 2 to 5 or more ng/ml, preferably of about 2.2 to 4.8 ng/ml or more, 2.4 to 4.4 ng/ml or more, 2.6 to 4.2 ng/ml ore more or 2.8 to 4.0 ng/ml or more, more preferably of about 2.5 to 4 ng/ml or more, an individual may be considered to be suffering from malignant prostate cancer, or be likely to develop malignant prostate cancer in the near future, i.e. within the next 1, 2, 3, 4, 5, 6, 12, 14, 48 months. The testing for expression of marker gene(s) or GOI's and determination of the PCPI may be carried out according to steps as defined herein above.

In a preferred embodiment of the present invention the diagnosing, detecting, monitoring or prognosticating as mentioned above is to be carried out on a sample obtained from an individual. The term "sample obtained from an individual" as used herein relates to any biological material obtained via suitable methods known to the person skilled in the art from an individual. The sample used in the context of the present invention should preferably be collected in a clinically acceptable manner, more preferably in a way that nucleic acids (in particular RNA) or proteins are preserved.

The biological samples may include body tissues and fluids, such as blood, sweat, and urine. Furthermore, the biological sample may contain a cell extract derived from or a cell population including an epithelial cell, preferably a cancerous epithelial cell or an epithelial cell derived from tissue suspected to be cancerous. Even more preferably the biological sample may contain a cell population derived from a glandular tissue, e.g. the sample may be derived from the prostate of a male individual. Additionally, cells may be purified from obtained body tissues and fluids if necessary, and then used as the biological sample.

Samples, in particular after initial processing, may be pooled. However, also non-pooled samples may be used.

In a specific embodiment of the present invention the content of a biological sample may also be submitted to an enrichment step. For instance, a sample maybe contacted with ligands specific for the cell membrane or organelles of certain cell types, e.g. prostate cells, functionalized for example with magnetic particles. The material concentrated by the magnetic particles may subsequently be used for detection and analysis steps as described herein above or below.

In a specific embodiment of the invention, biopsy or resections samples may be obtained and/or used. Such samples may comprise cells or cell lysates.

Furthermore, cells, e.g. tumor cells, may be enriched via filtration processes of fluid or liquid samples, e.g. blood, urine, etc. Such filtration processes may also be combined with enrichment steps based on ligand specific interactions as described herein above.

In a particularly preferred embodiment of the present invention a sample may be a tissue sample, a biopsy sample, a urine sample, a urine sediment sample, a blood sample, a saliva sample, a semen sample, a sample comprising circulating tumor cells, or a sample containing prostate secreted exosomes. A blood sample, may, for example, be a serum sample or a plasma sample.

In the treatment of prostate cancer compounds or pharmaceutical compositions being active against cancer cells can be used. The pharmaceutical composition may comprise hormone-inhibitors, preferably anti-androgens or androgen antagonists like spironolactone, cyproterone acetate, flutamide, nilutamide, bicalutamide, ketoconazole, finasteride or dutasteride.

In a further specific embodiment the present invention envisages a method of monitoring the development of prostate cancer, which encompasses the determination of marker gene(s) or GOI’s, preferably in combination with the determination of a reference gene as described herein above, over a certain period of time, i.e. after repeated determination steps, e.g. every 4 weeks, 6 weeks, two months, 4 months, 6 months, 8 months, 12 months, 1.5 years, 2 years, 2.5 years, 3 years, 4 years or any other suitable period of time etc. The method may provide data showing an increase or decrease of the level of marker gene(s) or GOI’s in comparison to controls, e.g. non-cancerous controls, cancerous controls or to earlier data obtained from the same individual. With the help of suitable statistical methods known to the person skilled in the art the position within said curve may be determined. In dependence of the position within said curve, i.e. in an augmenting portion or a falling portion of said curve, the presence or future development prostate cancer may be diagnosed. Correspondingly, the use of pharmaceutical compositions is envisaged. Preferably, any such determination may be combined with the determination of secondary biomarkers, e.g. markers for prostate cancer, in particular PSA. In case of low PSA levels (up to 2.0 to 4.0 ng/ml) the GOI data may be analyses with respect to early prostate cancer, i.e. benign or hormone-dependent/hormone-sensitive prostate cancer. In case of higher PSA levels (higher than about 20 ng/ml, e.g. about 30, 40 or 50 ng/ml) the data may be analysed with respect to more advanced prostate cancer, e.g. hormone-resistant prostate cancer.

A "hormone-sensitive stage I to IV prostate cancer" as used herein denotes a prostate cancer which can be classified according to the TNM classification by the International Union Against Cancer (UICC) into stages I to IV.

A "hormone-sensitive recurrent prostate cancer" as used herein denotes a prostate cancer whose growth and progression is regulated and dependent on a male sex hormone. A preferred example of such a male sex hormone is an androgen.

A "hormone-sensitive metastatic prostate cancer" as used herein denotes prostate cancer whose growth and progression regulated and dependent on a male sex hormone. A preferred example of such a male sex hormone is androgen.

A "hormone-insensitive prostate cancer" as used herein denotes prostate cancer whose growth and progression is not regulated and independent on a male sex hormone. A preferred example of such a male sex hormone is androgen.

A further aspect of the invention relates to a computer program product, comprising computer readable code stored on a computer readable medium or downloadable from a communications network, which, when run on a computer, implement one or more steps or all the steps of any one of the methods as described herein.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of the present invention may be written in any combination of one or more programming languages, including an object oriented programming language such as Java, Smalltalk, C++ or the like and conventional procedural programming languages, such as the "C" programming language or similar programming languages. The program code may execute entirely on the user’s computer, partly on the user’s computer, as a stand-alone software package, partly on the user’s computer and partly on a remote computer or entirely on the remote computer or server. In the latter scenario, the remote computer may be connected to the user’s computer through any type of network, including a local area network (LAN) or a wide area network (WAN), or the connection may be made to an external computer (for example, through the Internet using an Internet Service Provider).

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified herein.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Unless defined otherwise all technical and scientific terms used herein have the same meaning as commonly understood to one of ordinary skill in the art to which this invention belongs.

The following examples and figures are provided for illustrative purposes. It is thus understood that the example and figures are not to be construed as limiting. The skilled person in the art will clearly be able to envisage further modifications of the principles laid out herein.

EXAMPLES

Example 1 - Gene selection to build a Prostate Cancer Prognostic Index (PCPI)

To select gene candidates to build the PCPI various molecular pathways (as outlined on http://csbi.ltdk.helsinki.fi/anduril/tcga-gbm/table4_2.html) were investigated for correlation of the putative prognostic gene marker PDE4D7 as described in WO2010131194 and WO2010131195. Those molecular pathways that showed significant correlation were selected for down-stream analysis (Figure 1).

Furthermore, gene candidates related to metastatic and/or hormone-refractory prostate cancer were selected from the following literature

- Gorlov IP et al. Candidate pathways and genes for prostate cancer: a meta-analysis of gene expression data; BMC Medical Genomics 2009, 2:48
- Stanbrough M et al: Increased Expression of Genes Converting Adrenal Androgens to Testosterone in Androgen-Independent Prostate Cancer; Cancer Res 2006;66:2815-2825
- Tamura K et al. Molecular Features of Hormone-Refractory Prostate Cancer Cells by Genome-Wide Gene Expression Profiles; Cancer Res 2007; 67(11):5117-25
- Lapointe J et al. Gene expression profiling identifies clinically relevant subtypes of prostate cancer; PNAS 2003; 101(3):8110816
- Sun Y et al. Optimizing Molecular Signatures for Predicting Prostate Cancer Recurrence. The Prostate, 69:1119-1127 (2009)

In total 967 genes (*cf*. Figure 1) reported to be potentially relevant to prostate cancer progression were selected for further analysis.

To build a 19-gene prognostic signature, i.e. a set of marker genes of interest, the following data sets were used:

- Taylor BS et al. Integrative Genomic Profiling of Human Prostate Cancer. Cancer Cell 18, 11-22, 2010 (GEO data set ID: GSE21032)
- Boormans JL et al. Identification of TDRD1 as a direct target gene of ERG in primary prostate cancer. Int J Cancer 2013 Jul 15; 133(2):335-45 (GEO data set ID: GSE41408)
- Sun Y et al. Optimizing Molecular Signatures for Predicting Prostate Cancer Recurrence. The Prostate, 69:1119-1127 (2009) (GEO data set ID: GSE25136)
- Sboner A et al. Molecular sampling of prostate cancer: a dilemma for predicting disease progression. BMC Medical Genomics 2010, 3:8 (GEO data set ID: GSE16560)
- Nagakawa T et al. A Tissue Biomarker Panel Predicting Systemic Progression after PSA Recurrence Post-Definitive Prostate Cancer Therapy. PLoS ONE 3(5), e2318 (2010) (GEO data set ID: GSE10645)

To further test and validate the built Prostate Cancer Prognostic Index (PCPI) the following data set was used: Erho N et al. Discovery and Validation of a Prostate Cancer Genomic Classifier that Predicts Early Metastasis Following Radical Prostatectomy. PLoS One 8(6), e66855 (2013) (GEO data set ID: GSE46691)

Processing of GSE Gene Expression Data

Respective CEL files were downloaded from GEO (Gene Expression Omnibus): http://www.ncbi.nlm.nih.gov/geo/. The CEL file data were uploaded into Expression Console (Affymetrix Inc; Build 1.3.1.187) and were pre-processed using the appropriate probe set annotation files provided by Affymetrix Inc. where appropriate, i.e., data sets run on an Affymetrix Inc platform: GSE21032, GSE41408, GSE25136. For data sets run on the DASL platform (i.e., GSE16560, GSE10645) the data series matrix files were used as provided by GEO for downstream data analysis.

Reference genes are supposed to have a stable expression independently of the sample being processed and therefore can be used as an internal standard to normalize the output Cq values of a PCR analysis so that the results are comparable independently of the amount of input sample used. Though such genes are supposed to be stable, there is always some variability in their expression, and this stability can also depend on the tissue being analyzed. For such reason it is recommended to use more than one reference gene in the normalization of PCR Cq values and to use a set of genes that presents low variability in the specific type of tissue/sample being analyzed (C. L. Andersen, J. L. Jensen, and T. F. Ørntoft, "Normalization of Real-Time Quantitative Reverse Transcription-PCR Data: A Model-Based Variance Estimation Approach to Identify Genes Suited for Normalization, Applied to Bladder and Colon Cancer Data Sets," Cancer Res., vol. 64, no. 15, pp. 5245-5250, Aug. 2004; J. Vandesompele, K. De Preter, F. Pattyn, B. Poppe, N. Van Roy, A. De Paepe, and F. Speleman, "Accurate normalization of real-time quantitative RT-PCR data by geometric averaging of multiple internal control genes," Genome Biol., vol. 3, no. 7, p. RESEARCH0034, Jun. 2002)..

An initial selection of 9 reference genes to be used in this study was performed. Final selection of reference gene set used for normalization was done by running the with the Biogazelle Software tool (qBase+ with GeNorm analysis J. Hellemans, G. Mortier, A. D. Paepe, F. Speleman, and J. Vandesompele, "qBase relative quantification framework and software for management and automated analysis of real-time quantitative PCR data," Genome Biol., vol. 8, no. 2, p. R19, Feb. 2007.) on all the analyzed clinical samples.

The output Cq values of a PCR assay are intrinsically logarithmic (base 2) and inversely proportional to the amount of mRNA present in the sample. We use the following formula to normalize the raw Cq values:

$$N(Cq_{\,gene\ of\ interest}) = Mean(Cq_{\,ref\ gene}) - (Cq_{\,gene\ of\ interest})$$

Where $N(Cq_{gene\ of\ interest})$ is normalized gene expression value for selected genes of interest; where $Mean(Cq_{ref}$ gene) is the arithmetic mean of the PCR Cq values of the selected combination of reference genes; where $(Cq_{gene\ of\ interest})$ is the PCR Cq value of the gene of interest.

In case other technologies than qRT-PCR are used to measure the expression of the reference genes or the genes of interest the PCR Cq value will be replaced by a normalized measurement of the respective technology (e.g., a RMA (Robust Multi-array Average) normalized gene expression value for DNA microarrays, or a FPKM (Fragements Per Kilobase of Exon Per Million Fragments Mapped) normalized gene expression value for RNA sequencing).

Calculation of Prostate Cancer Progression Index (PCPI)

Selected Genes of Interest (GOI) were grouped into those down-regulated between patient groups with and without progression after primary treatment compared to those up-regulated between patient groups with and without progression after primary treatment. An average gene expression value for the down-regulated genes was calculated (GEV_av_GOI_down) as well as an average gene expression value for the up-regulated genes (GEV_av_GOI_up) from the normalized gene expression data per each analyzed patient sample. The Prostate Cancer Progression Index PCPI per patient sample was determined as:

$$PCPI = (GEV_av_GOI_up) - (GEV_av_GOI_down)$$

The calculated PCPI was used for any further statistical data analysis.

**PCPI_17 computation (based on quantitative real-time PCR data)**

The PCPI was computed using the following formula:

$$PCPI = Mean(N(Cq_{\,PCPI\ genes\ up})) - Mean(N(Cq_{\,PCPI\ genes\ down}))$$

where

Mean($N(Cq_{PCPI\ genes\ up})$) is the arithmetic mean of the normalized gene expression values (see above) for the genes: BGN, BIRC5, COL1A1, COL3A1, COL5A2, DYRK2, INHBA, THBS2, and VCAN
and where
Mean($N(Cq_{PCPI\ genes\ down})$), is the arithmetic mean of the normalized gene expression values (see above) for the genes: AZGP1, FBLN1, ILK, KRT15, MEIS2, MYBPC1, PAGE4, and SRD5A2.

**PCPI_18 computation (based on quantitative real-time PCR data)**

The PCPI was computed using the following formula:

$$PCPI = Mean(N(Cq_{PCPI\ genes\ up})) - Mean(N(Cq_{PCPI\ genes\ down}))$$

where

Mean(N($Cq_{PCPI\ genes\ up}$)) is the arithmetic mean of the normalized gene expression values (see above) for the genes: BGN, BIRC5, COL1A1, COL3A1, COL5A2, DYRK2, INHBA, THBS2, and VCAN
and where
Mean(N($Cq_{PCPI\ genes\ down}$)), is the arithmetic mean of the normalized gene expression values (see above) for the genes: PDE4D, AZGP1, FBLN1, ILK, KRT15, MEIS2, MYBPC1, PAGE4, and SRD5A2

**PCPI_19 computation (based on quantitative real-time PCR data)**

The PCPI was computed using the following formula:

$$PCPI = Mean(N(Cq_{PCPI\ genes\ up})) - Mean(N(Cq_{PCPI\ genes\ down}))$$

where

Mean(N($Cq_{PCPI\ genes\ up}$)) is the arithmetic mean of the normalized gene expression values (see above) for the genes: BGN, BIRC5, COL1A1, COL3A1, COL5A2, DYRK2, INHBA, THBS2, and VCAN
and where
Mean(N($Cq_{PCPI\ genes\ down}$)), is the arithmetic mean of the normalized gene expression values (see above) for the genes: PDE4D5, PDE4D7, AZGP1, FBLN1, ILK, KRT15, MEIS2, MYBPC1, PAGE4, and SRD5A2.

Correlation Analysis of the Prostate Cancer Progression Index (PCPI) vs. Clinical Parameters to Patient Outcome

All statistical analysis (e.g., logistic regression analysis, ROC analysis, COX regression analysis, etc.) of the PCPI and its correlation to patient outcome in comparison to clinical parameters like PSA, pGleason, pT Stage was performed with XLSTAT Version 2014.1.03

Results

The 967 selected gene candidates were investigated for differential expression in the above gene expression data sets between prostate cancer patients that did not show progression of their disease after primary treatment vs. those patient groups that demonstrated disease progression to either of the following clinical endpoints: biochemical recurrence, clinical recurrence by detection of local or distant metastases, prostate cancer specific death.

Table 2 (*cf.* Figure 2) provides an overview of 57 selected genes that were found to be significantly ($p<0.05$) differentially expressed and were regulated between patient groups in the same direction (i.e., either up- or down-regulated) between above mentioned patient groups at least in three out of the five tested data sets. Subsequently, genes were ranked into three categories according to the following criteria:

Rank_3: all genes that were observed in max 3 data sets to be differentially expressed with a significant p-value ($p<0.05$) in the same direction or genes with non-unique annotation
Rank_2: all genes that were observed in 4 or 5 data sets to be differentially expressed with a significant p-value ($p<0.05$) in the same direction
Rank_1: all genes that were observed in 4 or 5 data sets to be differentially expressed with a significant p-value ($p<0.05$) in the same direction but limited to 9 genes up-regulated as well as 10 genes down-regulated

A preferred PCPI signature comprises or consists of the following 19 genes: PDE4D5, PDE4D7, AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2.

As not all data sets provide information on transcript level of PDE4D isoforms (i.e., PDE4D5 and PDE4D7) the demonstrated results are based on the measurement of the PDE4D gene as the expression of PDE4D is very well correlated to the expression of PDE4D7 (normalized to the expression of PDE4D5) in data sets GSE21032 and GSE41408); consequently, the determined PCPI_18 comprised the following 18 genes: PDE4D, AZGP1, FBLN1, ILK, KRT15, MEIS2, MYBPC1, PAGE4, SRD5A2, COL1A1, COL3A1, INHBA, THBS2, VCAN, BGN, BIRC5, DYRK2.

For such data sets where the PDE4D gene was not measured (i.e., GSE16560, GSE10645) the determined PCPI_17 comprised the following 17 genes: AZGP1, FBLN1, ILK, KRT15, MEIS2, MYBPC1, PAGE4, SRD5A2, COL1A1, COL3A1, INHBA, THBS2, VCAN, BGN, BIRC5, DYRK2.

Correlation of the PCPI to clinical outcome data of prostate cancer patients after primary treatment

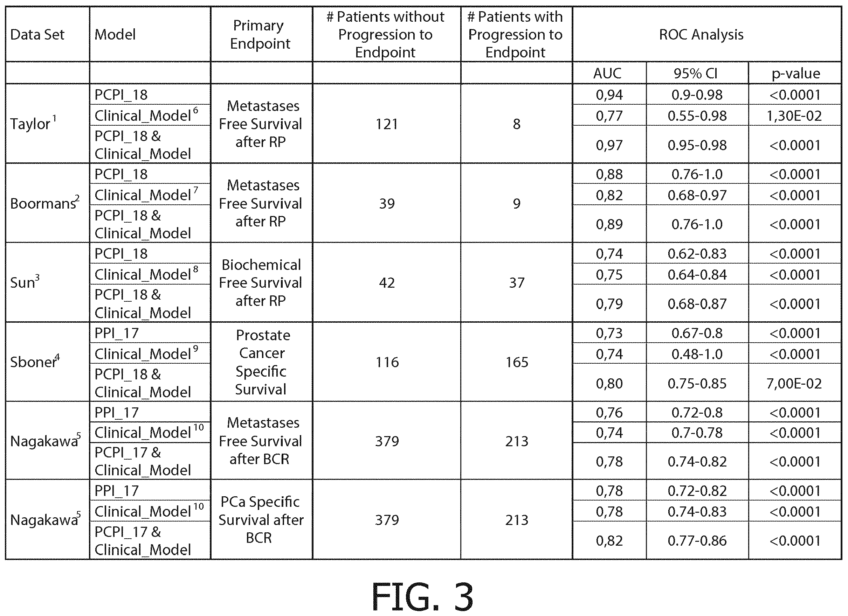

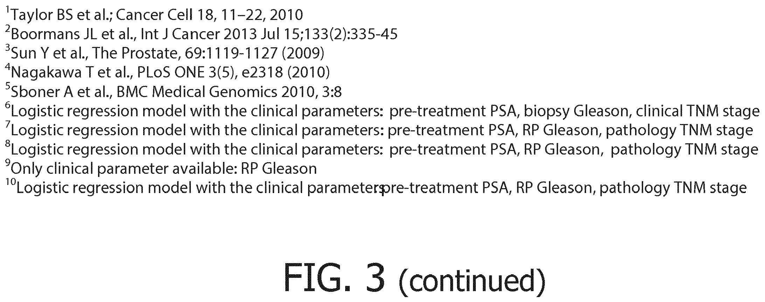

Table 3 (*cf.* Figure 3) shows an overview on the performance of PCPI_18 or PCPI_17 to predict the primary endpoint of development of distant metastases within 10-15 years after primary treatment (prostate surgery in general) for the data sets GSE21032, GSE41408, GSE25136, GSE16560, GSE10645. For patients investigated in data set GSE16560 the cancers of the prostate were detected during a TURP (transurethral resection) and patients were subsequently managed conservatively (i.e., without removal of the prostate). For patients investigated in data set GSE10645 the monitoring of patients for the endpoint did only start after biochemical recurrence). These data sets were used to build the PCPI signature (training data). The number of patients per group (no progression vs. progression to metastases) is shown as well as the AUC (Area Under the Curve) derived from a ROC analysis. The AUC indicates the relative power of the PCPI in the different data sets to predict the primary endpoint within the cohort).

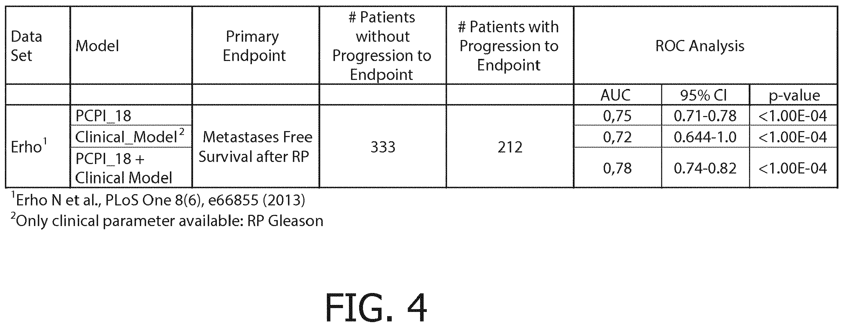

Table 4 (*cf.* Figure 4) shows the performance of PCPI_18 to predict the primary endpoint of development of distant metastases within 10-15 years after primary treatment for the data set GSE46691; this data set was not used to train the PCPI and is used as an independent verification data set (validation data). For patients investigated in data set GSE 10645 the monitoring of patients for the endpoint did only start after biochemical recurrence). The number of patients per group (no progression vs. progression to metastases) is shown as well as the AUC (Area Under the Curve) derived from a ROC analysis. The AUC indicates the relative power of the PCPI in the different data sets to predict the primary endpoint within the cohort.

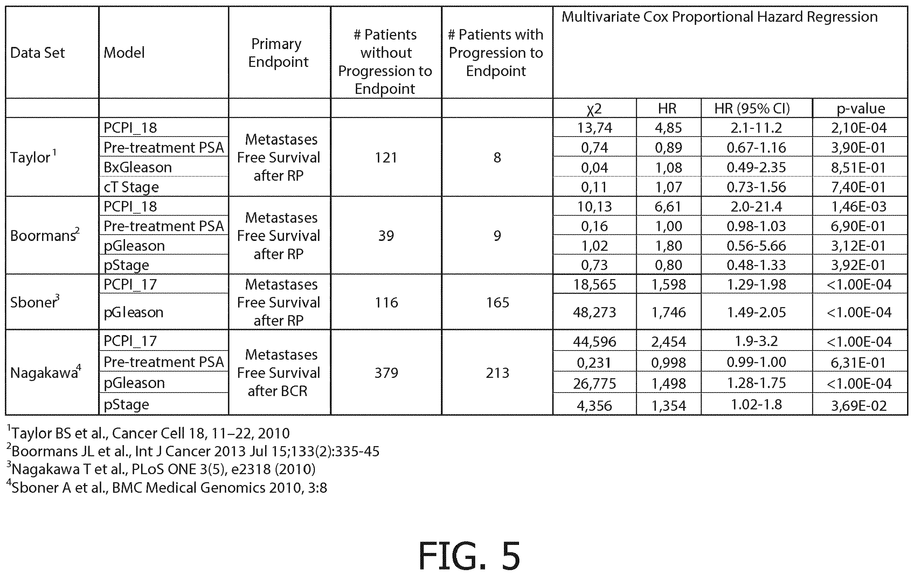

Table 5 (*cf.* Figure 5) shows the performance of PCPI_18 or PCPI_17 to predict the primary endpoint of development of distant metastases within 10-15 years after primary treatment (prostate surgery in general) for the data sets GSE21032, GSE41408, GSE16560, GSE10645 in a multivariate COX regression analysis compared to standard clinical parameters like pre-treatment PSA, biopsy or pathology Gleason score, or clinical/pathology disease stage. The Chi Square as well as the Hazard Ratio for the PCPI and the individual clinical parameters is shown for each individual data set.

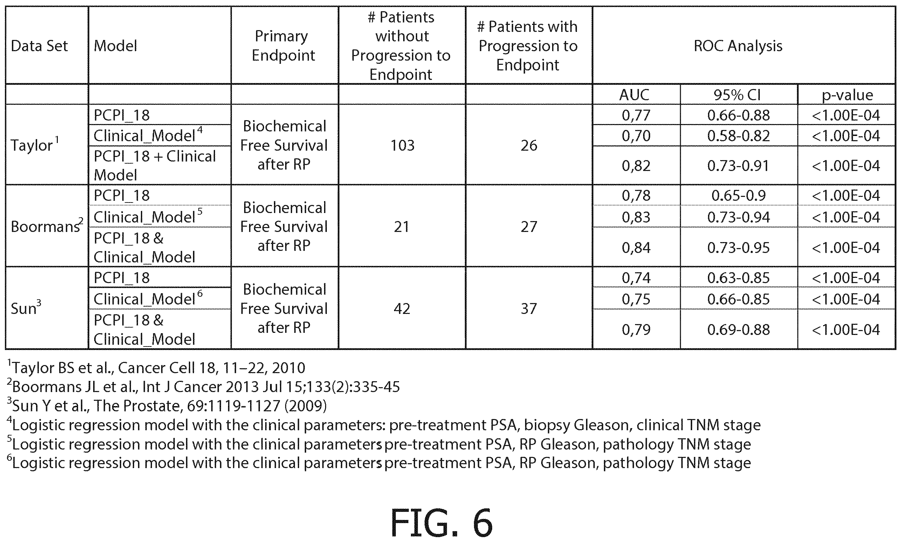

Table 6 (*cf.* Figure 6) shows an overview on the performance of PCPI_18 to predict the primary endpoint of development of biochemical disease recurrence (BCR) within 10-15 years after primary treatment (prostate surgery in general) for the data sets GSE21032, GSE41408, GSE25136. The number of patients per group (no progression vs. progression to BCR) is shown as well as the AUC (Area Under the Curve) derived from a ROC analysis. The AUC indicates the relative power of the PCPI in the different data sets to predict the primary endpoint within the cohort).

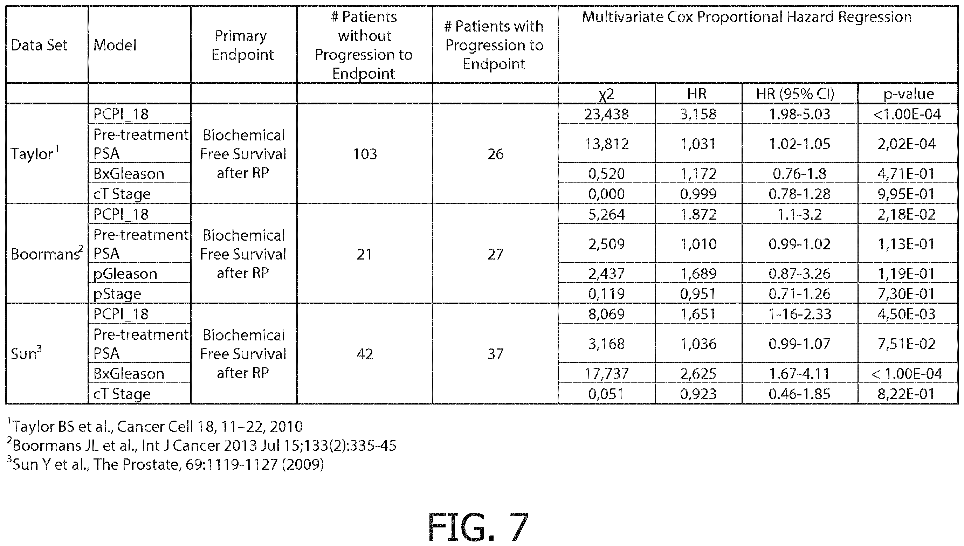

Table 7 (*cf.* Figure 7) shows the performance of PCPI_18 to predict the primary endpoint of development of biochemical disease recurrence (BCR) within 10-15 years after primary treatment (prostate surgery in general) for the data sets GSE21032, GSE41408, GSE25136 in a multivariate COX regression analysis compared to standard clinical parameters like pre-treatment PSA, biopsy or pathology Gleason score, or clinical/pathology disease stage. The Chi Square as well as the Hazard Ratio for the PCPI and the individual clinical parameters is shown for each individual data set.

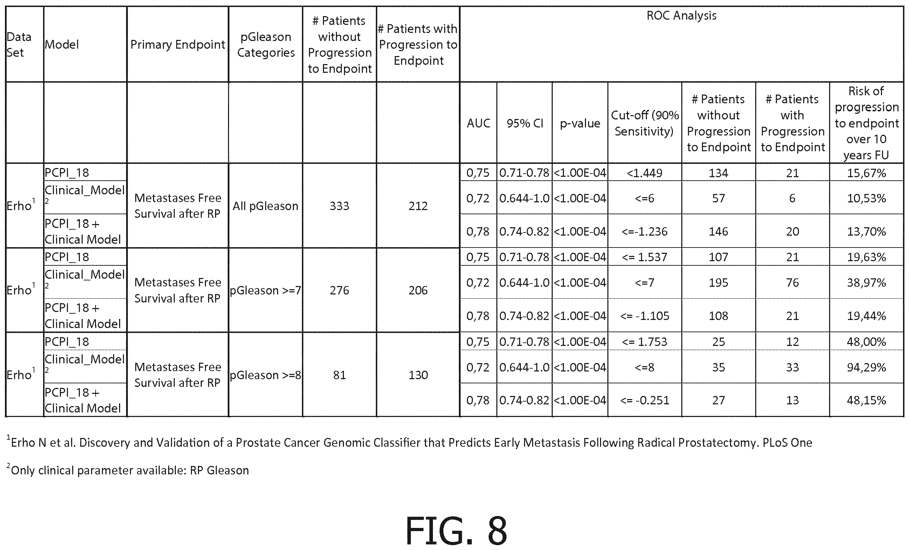

Table 8 (*cf.* Figure 8) shows a ROC curve cut-off analysis for PCPI_18 to support clinical decision making towards patient stratification for either active surveillance (AS) or active treatment (e.g., prostatectomy, radiotherapy, hormone therapy). The cut-off as shown in the table was selected from the ROC curve such that >=90% of men with aggressive disease were correctly classified (corresponding to 90% sensitivity). This cut-off allows the stratification of 134 with a very low-risk profile towards e.g. active surveillance instead of e.g. surgery. The progression-free survival in this cohort to the endpoint of metastatic disease over approx. 10 years follow-up is close to 85%. The same approach was applied to the clinical model (pathology Gleason score) with the result that a cut-off of a Gleason score <=6 would lead to the stratification of only 57 men towards e.g. active surveillance instead of e.g. surgery. The progression-free survival in this patient cohort would be close to 90% but on the cost that a lot more men with non-progressive disease would be stratified to e.g. surgery; the use of PCPI_18 stratifies more than twice as many men to AS compared to pathology Gleason score while the risk profile is very comparable (85% vs. 90% progression-free survival, respectively). The stratification power can be even further increased by a combination model of PCPI_18 and the pathology Gleason. Using that model 146 men (compared to 134) would be stratified to AS with a progression-free survival probability over 10 years of around 87%.

The cut-off analysis for the shown patient cohort was performed for the total cohort (545 patients), a sub-cohort with pathology Gleason scores >=7 (482 patients), as well as another sub-cohort with pathology Gleason scores >=8 (211 patients).

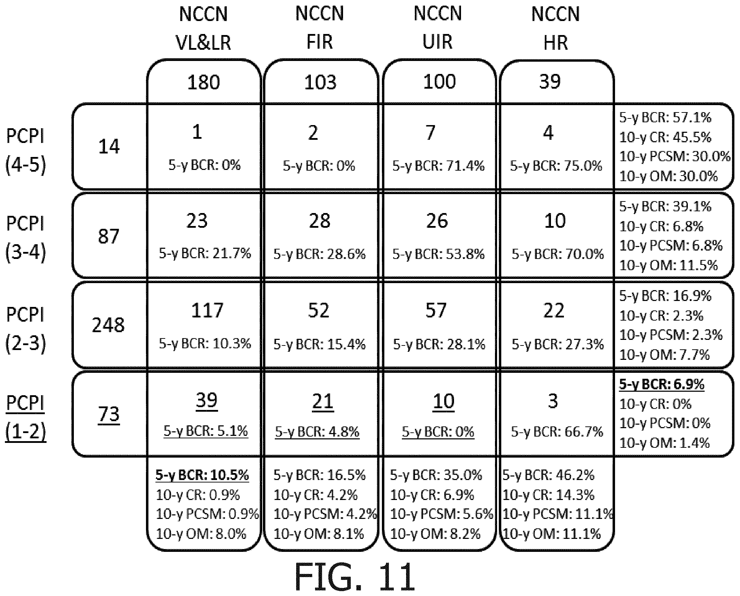

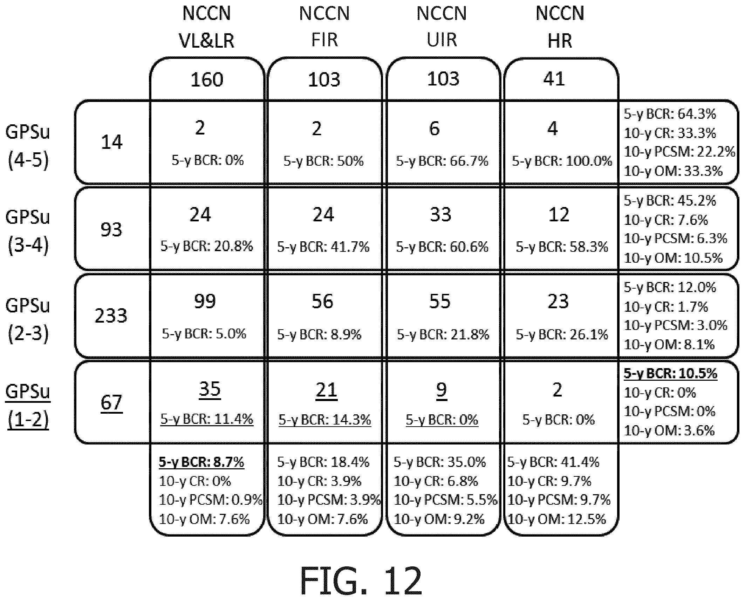

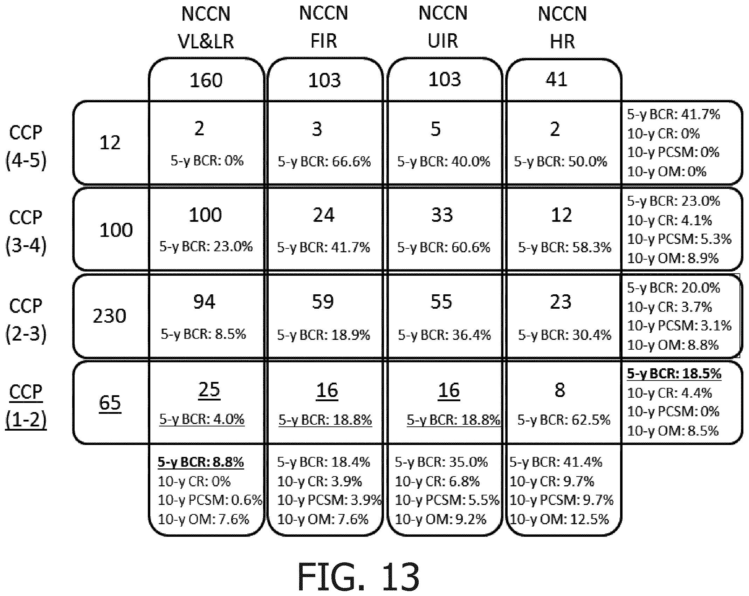

Figures 11, 12 and 13 refer to an analysis obtained from a study, which has been performed on approx. 500 patients with longitudinal follow up comparing the performance of the PCPI_19 (Figure 11), the GPSu score, which is derived from RNAseq data analyzing the expression of 12 cancer related genes and 5 reference genes and was calculated following the method as far as it can be inferred from the article "Analytical validation of the Oncotype DX prostate cancer assay - a clinical RT-PCR assay optimized for prostate needle biopsies" by Dejan Knezevic et al. in BMC Genomics (2013), 14: 690 (Figure 12) and the CCP score derived from the expression levels of 31 genes and calculated as far as can be inferred from the article "Prognostic value of an RNA expression signature derived from cell cycle proliferation genes for recurrence and death from prostate cancer: A retrospective study in two cohorts" by Jack Cuzick et al. in Lancet Oncol. (2011); 12(3): 245-255) (Figure 13).

For better comparison, the GPSu and CCP score have been scaled to a range between 1 to 5.

The Figures 11, 12 and 13 show different recurrence risks (see below for description) over 5 or 10 years according to different patient groups. On the x-axis, patient groups have been defined by the NCCN risk groups; VL&LR=very low and low risk; FIR=favorable intermediate risk; UIR=unfavorable intermediate risk; HR=high risk. The NCCN are commonly used US guidelines in oncology (https://www.nccn.org/professionals/physician_gls/f_guidelines.asp). On the y-axis the patient groups have been defined by 4 categories of the different scores: score 1-2; 2-3; 3-4, and 4-5. In the 4x4 grid, patients have been selected for any of the cells of the grid according to their NCCN clinical risk characteristics and according to the score they have for PCPI, GPSu or CCP. The different risk according to clinical risk group was calculated (the line at the bottom) or according to score category (the column on the right hand side of the table). Within the cells only the 5-year BCR recurrence risk is depicted for reasons of clarity.

The risk descriptions are:

5-y BCR - biochemical recurrence over 5 years
10-y CR - clinical recurrence to metastases over 10 years
10-y PCSM - prostate cancer specific mortality over 10 years
10-y OM - overall mortality (due to all reasons) over 10 years

The numbers underlined in Figures 11, 12 and 13 indicate areas of improved performance of PCPI over the other signatures. The patients that would fall into these categories are potential patients for active surveillance compared to active treatment clinical decisions and should therefore have preferably as little recurrence risk as possible. The PCPI outperforms the other signatures in these areas.

## Claims

**1.** A method comprising:

- determining a gene expression level for the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, to obtain a subject expression profile for a subject,

and further comprising:

- classifying the subject as having a good prognosis or a poor prognosis of prostate cancer based on the subject expression profile, wherein the good prognosis predicts an increased likelihood of survival within a predetermined period after initial diagnosis and/or no progression of disease after primary treatment, and the poor prognosis predicts an aggressive disease, a decreased likelihood of survival, and an increased likelihood of biochemical recurrence, clinical recurrence, and/or the presence of local or distant metastases, within a predetermined period after initial diagnosis; and/or
- classifying the subject as having or not having a predisposition of prostate cancer that is susceptible to disease progression based on the subject expression profile, wherein the predisposition predicts an aggressive disease, decreased likelihood of survival, and an increased likelihood of biochemical recurrence, clinical recurrence, and/or the presence of local or distant metastases, within a predetermined period after initial diagnosis.

**2.** A method comprising:

- classifying a subject as having a good prognosis or a poor prognosis based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, and wherein a good prognosis predicts an increased likelihood of survival within a predetermined period after initial diagnosis and/or no progression of disease after primary treatment, and poor prognosis predicts an aggressive disease, a decreased likelihood of survival, and an increased likelihood of biochemical recurrence, clinical recurrence, and/or the presence of local or distant metastases, within a predetermined period after initial diagnosis; and/or
- classifying a subject as having or not having a predisposition of prostate cancer that is susceptible to disease progression based on a subject expression profile for the subject, wherein the subject expression profile includes the gene expression level of the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7, wherein the predisposition predicts an aggressive disease, decreased likelihood of survival, an increased likelihood of biochemical recurrence, clinical recurrence, and/or the presence of local or distant metastases, within a predetermined period after initial diagnosis.

**3.** The method of any one of claims 1 or 2, wherein the subject expression profile is converted to a prostate cancer progression index.

**4.** The method of claim 3, wherein the prostate cancer progression index is calculated according to the following equation:

$$PCPI = (GEV_av_GOI_up) - (GEV_av_GOI_down),$$

wherein GEV_av_GOI_up is an average gene expression value of up-regulated genes, and
wherein GEV_av_GOI_down is an average gene expression value of down-regulated genes,
wherein the values are determined on the basis of normalized gene expression data per each subject sample.

**5.** The method of any one of claims 1-4, wherein the expression level of the set of the signature genes is normalized to the expression of a reference gene, preferably wherein the reference gene is a housekeeping gene, and more preferably wherein the housekeeping gene is TBP, HPRT1, ACTB, RPLPO, PUM1, POLR2A or B2M.

**6.** The method of claim 1 or 2,
wherein the subject is classified as having a good prognosis if the prostate cancer progression index is below a selected threshold or as having a poor prognosis if the prostate cancer progression index is above the selected threshold; and/or
wherein the subject is classified as having a predisposition of prostate cancer that is susceptible to disease progression if the prostate cancer progression index is above a selected threshold or as not having a predisposition of prostate cancer that is susceptible to disease progression if the prostate cancer progression index is below the selected threshold.

**7.** The method of any one of claims 1-6, further comprising
stratifying the subject for the risk of aggressive disease versus non-aggressive disease according to the prognosis determined or the predisposition determined.

8. The method of claim 1 or 2, wherein the poor prognosis or the predisposition of prostate cancer that is susceptible to disease progression is indicative of eligibility of the subject to be treated with any one or more of the treatments selected from the group consisting of prostate surgery, prostate removal, chemotherapy, radiotherapy, brachytherapy, limited or extended lymph node dissection, hormonal therapy.

9. Use of a product comprising:

primers and/or probes for determining the gene expression level for the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7;
optionally further comprising primers and/or probes for determining the expression levels of a set of genes listed in either Figures 1 or 2 other than the above-mentioned genes; and/or
optionally further comprising primers and/or probes for determining the gene expression level of a reference gene, preferably wherein the reference gene is a housekeeping gene, and more preferably wherein the housekeeping gene is TBP, HPRT1, ACTB, RPLPO, PUM1, POLR2A or B2M
for establishing a prognosis for a patient diagnosed with prostate cancer, for stratification of a patient diagnosed with prostate cancer, or for the determination of predisposition for aggressive or indolent prostate cancer in a patient having prostate cancer.

10. Use according to claim 9, wherein the product is a PCR kit, a RNA-sequencing kit, or a microarray or a microarray kit.

11. A computer program product, comprising computer readable code stored on a computer readable medium or downloadable from a communications network, which, when run on a computer, implement one or more steps of any one of claims 1-8.

12. A system comprising the computer program product of claim 11 and a product comprising:

primers and/or probes for determining the gene expression level for the signature genes: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, and DYRK2, and optionally PDE4 isoforms comprising PDE4D5 and/or PDE4D7;
optionally further comprising primers and/or probes for determining the expression levels of a set of genes listed in either Figures 1 or 2 other than the above-mentioned genes; and/or
optionally further comprising primers and/or probes for determining the gene expression level of a reference gene, preferably wherein the reference gene is a housekeeping gene, and more preferably wherein the housekeeping gene is TBP, HPRT1, ACTB, RPLPO, PUM1, POLR2A or B2M.

13. The system of claim 12, wherein the product is a PCR kit, a RNA-sequencing kit, or a microarray or a microarray kit.

## Patentansprüche

1. Verfahren, umfassend:

- Bestimmen einer Genexpressionsstufe für die Signaturgene: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5 und DYRK2, und optional PDE4-Isoforme, PDE4D5 und/oder PDE4D7 umfassen, um ein Subjektausdrucksprofil für ein Subjekt zu erhalten,

und weiter umfassend:

- Klassifizieren des Subjekts als eine gute Prognose oder eine schlechte Prognose für Prostatakrebs auf Basis des Subjektausdrucksprofils aufweisend, wobei die gute Prognose eine erhöhte Wahrscheinlichkeit des Überlebens innerhalb einer vorbestimmten Zeitspanne nach ursprünglicher Diagnose und/oder kein Fortschreiten der Krankheit nach Erstbehandlung voraussagt, und die schlechte Prognose eine aggressive Krankheit, eine verringerte Wahrscheinlichkeit des Überlebens und eine erhöhte Wahrscheinlichkeit von biochemischem Wiederauftreten, klinischem Wiederauftreten und/oder des Vorhandenseins von lokalen oder distanzierten Metastasen innerhalb einer vorbestimmten Zeitspanne nach ursprünglicher Diagnose voraussagt; und/oder
- Klassifizieren des Subjekts als eine Veranlagung für Prostatakrebs aufweisend oder nicht aufweisend, welche für ein Fortschreiten der Krankheit empfänglich ist, auf Basis des Subjektausdrucksprofils, wobei die Veranlagung eine aggressive Krankheit, verringerte Wahrscheinlichkeit des Überlebens und eine erhöhte Wahrscheinlichkeit von biochemischem Wiederauftreten, klinischem Wiederauftreten und/oder des Vorhandenseins von lokalen oder distanzierten Metastasen innerhalb einer vorbestimmten Zeitspanne nach ursprünglicher Diagnose voraussagt.

**2.** Verfahren, umfassend:

- Klassifizieren eines Subjekt als eine gute Prognose oder eine schlechte Prognose aufweisend, auf Basis eines Subjektausdrucksprofils für das Subjekt, wobei das Subjektausdrucksprofil die Genexpressionsstufe der Signaturgene: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5 und DYRK2 , und optional PDE4-Isoforme, welche PDE4D5 und/oder PDE4D7 umfassen, beinhaltet, und wobei eine gute Prognose eine erhöhte Wahrscheinlichkeit des Überlebens innerhalb einer vorbestimmten Zeitspanne nach ursprünglicher Diagnose und/oder kein Fortschreiten der Krankheit nach Erstbehandlung voraussagt, und schlechte Prognose eine aggressive Krankheit, eine verringerte Wahrscheinlichkeit des Überlebens und eine erhöhte Wahrscheinlichkeit von biochemischem Wiederauftreten, klinischem Wiederauftreten und/oder des Vorhandenseins von lokalen oder distanzierten Metastasen innerhalb einer vorbestimmten Zeitspanne nach ursprünglicher Diagnose voraussagt; und/oder
- Klassifizieren eines Subjekts als eine Veranlagung für Prostatakrebs aufweisend oder nicht aufweisend, welche für ein Fortschreiten der Krankheit empfänglich ist, auf Basis eines Subjektausdrucksprofils für das Subjekt, wobei das Subjektausdrucksprofil die Genexpressionsstufe der Signaturgene: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5 und DYRK2, und optional PDE4-Isoforme, welche PDE4D5 und/oder PDE4D7 umfassen, beinhaltet, wobei die Veranlagung eine aggressive Krankheit, verringerte Wahrscheinlichkeit des Überlebens, eine erhöhte Wahrscheinlichkeit von biochemischem Wiederauftreten, klinischem Wiederauftreten und/oder des Vorhandenseins von lokalen oder distanzierten Metastasen innerhalb einer vorbestimmten Zeitspanne nach ursprünglicher Diagnose voraussagt.

**3.** Verfahren nach einem der Ansprüche 1 oder 2, wobei das Subjektausdrucksprofil in einen Prostatakrebsfortschreitungsindex umgewandelt wird.

**4.** Verfahren nach Anspruch 3, wobei der Prostatakrebsfortschreitungsindex nach der folgenden Gleichung berechnet wird:

$$PCPI = (GEV_av_GOI_up)-(GEV_av_GOI_down),$$

wobei GEV_av_GOI_up ein durchschnittlicher Genexpressionswert von hochregulierten Genen ist, und
wobei GEV_av_GOI_down ein durchschnittlicher Genexpressionswert von herunterregulierten Genen ist,
wobei die Werte auf der Basis von normalisierten Genexpressionsdaten für jedes Subjektmuster bestimmt werden.

**5.** Verfahren nach einem der Ansprüche 1-4, wobei die Expressionsstufe des Satzes von Signaturgenen auf die Expression eines Referenzgens normalisiert wird, vorzugsweise wobei das Referenzgen ein Haushaltsgen ist, und bevorzugter, wobei das Haushaltsgen TBP, HPRT1, ACTB, RPLPO, PUM1, POLR2A oder B2M ist.

**6.** Verfahren nach Anspruch 1 oder 2,
wobei das Subjekt als eine gute Prognose aufweisend, wenn der Prostatakrebsfortschreitungsindex unterhalb einer ausgewählten Schwelle ist, oder als eine schlechte Prognose aufweisend, wenn der Prostatakrebsfortschreitungsindex über der ausgewählten Schwelle ist, klassifiziert ist; und/oder
wobei das Subjekt als ein Veranlagung für Prostatakrebs aufweisend, welche empfänglich für Fortschreiten der Krankheit ist, wenn der Prostatakrebsfortschreitungsindex über einer ausgewählten Schwelle ist, oder als nicht eine Veranlagung für Prostatakrebs aufweisend, welche empfänglich für Fortschreiten der Krankheit ist, wenn der Prostatakrebsfortschreitungsindex unter der ausgewählten Schwelle ist, klassifiziert ist.

**7.** Verfahren nach einem der Ansprüche 1-6, weiter umfassend

Stratifizieren des Subjekts für das Risiko aggressiver Krankheit gegenüber nicht aggressiver Krankheit nach der bestimmten Prognose oder der bestimmten Veranlagung.

**8.** Verfahren nach Anspruch 1 oder 2, wobei die schlechte Prognose oder die Veranlagung für Prostatakrebs, welche empfänglich für Fortschreiten der Krankheit ist, die Eignung des Subjekts angibt, mit einer oder mehreren der Behandlungen, ausgewählt aus der Gruppe, bestehend aus Prostataoperation, Prostataentfernung, Chemotherapie, Strahlentherapie, Brachytherapie, eingeschränkter oder erweiterter Lymphknotendissektion, Hormontherapie behandelt zu werden.

**9.** Verwendung eines Produkts, umfassend:

Primer und/oder Sonden zum Bestimmen der Genexpressionsstufe für die Signaturgene: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5 und DYRK2, und optional PDE4-Isoforme, welche PDE4D5 und/oder PDE4D7 umfassen;
optional weiter Primer und/oder Sonden zum Bestimmen der Expressionsstufen eines Satzes von entweder in Figur 1 oder 2 aufgelisteten Genen umfassend, welche sich von den oben erwähnten Genen unterscheiden; und/oder
optional weiter Primer und/oder Sonden zum Bestimmen der Genexpressionsstufe eines Referenzgens umfassend, vorzugsweise wobei das Referenzgen ein Haushaltsgen ist, und bevorzugter, wobei das Haushaltsgen TBP, HPRT1, ACTB, RPLPO, PUM1, POLR2A oder B2M ist,
zum Erstellen einer Prognose für einen Patienten, bei dem Prostatakrebs diagnostiziert wurde, zur Stratifikation eines Patienten, bei dem Prostatakrebs diagnostiziert wurde, oder für die Bestimmung einer Veranlagung für aggressiven oder indolenten Prostatakrebs bei einem Patienten mit Prostatakrebs.

**10.** Verwendung nach Anspruch 9, wobei das Produkt ein PCR-Kit, ein RNA-Sequenzierungskit, oder ein Microarray oder ein Microarray-Kit ist.

**11.** Computerprogrammprodukt, umfassend computerlesbaren Code, welcher auf einem computerlesbaren Medium gespeichert ist oder aus einem Kommunikationsnetzwerk herunterladbar ist, welcher bei Ausführung auf einem Computer einen oder mehrerer Schritte nach einem der Ansprüche 1-8 implementiert.

**12.** System, umfassend das Computerprogrammprodukt nach Anspruch 11 und ein Produkt, umfassend:

Primer und/oder Sonden zum Bestimmen der Genexpressionsstufe für die Signaturgene: AZGP1, FBLN1, ILK, KRT15, MEIS2, MYBPC1, PAGE4, SRD5A2, COL1A1, COL3A1, COL5A2, INHBA, THBS2, VCAN, BGN, BIRC5 und DYRK2, und optional PDE4-Isoforme, welche PDE4D5 und/oder PDE4D7 umfassen;
optional weiter Primer und/oder Sonden zum Bestimmen der Expressionsstufen eines Satzes von entweder in Figur 1 oder 2 aufgelisteten Genen umfassend, welche sich von den oben erwähnten Genen unterscheiden; und/oder
optional weiter Primer und/oder Sonden zum Bestimmen der Genexpressionsstufe eines Referenzgens umfassend, vorzugsweise wobei das Referenzgen ein Haushaltsgen ist, und bevorzugter, wobei das Haushaltsgen TBP, HPRT1, ACTB, RPLPO, PUM1, POLR2A oder B2M ist.

**13.** System nach Anspruch 12, wobei das Produkt ein PCR-Kit, ein RNA-Sequenzierungskit, oder ein Microarray oder ein Microarray-Kit ist.

## Revendications

**1.** Procédé comprenant :

- la détermination d’un niveau d’expression génique pour les gènes de signature : AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, et DYRK2, et facultativement des isoformes de PDE4 comprenant PDE4D5 et/ou PDE4D7, pour obtenir un profil d’expression de sujet pour un sujet, et comprenant en outre les étapes consistant à :

  - classer le sujet comme ayant un bon pronostic ou un mauvais pronostic quant à un cancer de la prostate sur la base du profil d’expression de sujet, dans lequel le bon pronostic prédit une probabilité accrue de survie dans une période prédéterminée après un diagnostic initial et/ou une absence de progression de la maladie après un traitement primaire, et le mauvais pronostic prédit une maladie agressive, une probabilité de survie réduite, et une probabilité accrue de récidive biochimique, de récidive clinique, et/ou de la présence de métastases locales ou distantes, dans une période prédéterminée après un diagnostic initial ; et/ou
- classer le sujet comme ayant ou non une prédisposition à un cancer de la prostate qui est susceptible à une progression de maladie sur la base du profil d'expression de sujet, dans lequel la prédisposition prédit une maladie agressive, une probabilité réduite de survie, et une probabilité accrue de récidive biochimique, de récidive clinique, et/ou de la présence de métastases locales ou distantes, dans une période prédéterminée après un diagnostic initial.

**2.** Procédé comprenant les étapes consistant à :

- classer un sujet comme ayant un bon pronostic ou un mauvais pronostic sur la base d'un profil d'expression de sujet pour le sujet, dans lequel le profil d'expression de sujet inclut le niveau d'expression génique des gènes de signature : AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, et DYRK2, et facultativement des isoformes de PDE4 comprenant PDE4D5 et/ou PDE4D7, dans lequel un bon pronostic prédit une probabilité accrue de survie dans une période prédéterminée après un diagnostic initial et/ou une absence de progression de la maladie après un traitement primaire, et un mauvais pronostic prédit une maladie agressive, une probabilité de survie réduite, et une probabilité accrue de récidive biochimique, de récidive clinique, et/ou de la présence de métastases locales ou distantes, dans une période prédéterminée après un diagnostic initial ; et/ou
classer un sujet comme ayant ou non une prédisposition à un cancer de la prostate qui est susceptible à une progression de maladie sur la base du profil d'expression pour le sujet, dans lequel le profil d'expression du sujet comprend le niveau d'expression génique des gènes de signature : AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, et DYRK2, et facultativement des isoformes de PDE4 comprenant PDE4D5 et/ou PDE4D7, dans lequel la prédisposition prédit une maladie agressive, une probabilité de survie réduite, une probabilité accrue de récidive biochimique, de récidive clinique, et/ou de la présence de métastases locales ou distantes, dans une période prédéterminée après un diagnostic initial.

**3.** Procédé selon l'une quelconque des revendications 1 ou 2, dans lequel le profil d'expression de sujet est converti en un indice de progression de cancer de la prostate.

**4.** Procédé selon la revendication 3, dans lequel l'indice de progression de cancer de la prostate est calculé conformément à l'équation suivante :

$$PCPI = (GEV_av_GOI_up) - (GEV_av_GOI_down),$$

où GEV_av_GOI_up est une valeur d'expression génique moyenne de gènes régulés à la hausse, et
où GEV_av_GOI_down est une valeur d'expression génique moyenne de gènes régulés à la baisse,
dans lequel les valeurs sont déterminées sur la base de données d'expression génique normalisées pour chaque échantillon de sujet.

**5.** Procédé selon l'une quelconque des revendications 1-4, dans lequel le niveau d'expression de l'ensemble des gènes de signature est normalisé à l'expression d'un gène de référence, de préférence dans lequel le gène de référence est un gène domestique, et de manière plus préférée dans lequel le gène domestique est TBP, HPRT1, ACTB, RPLPO, PUM1, POLR2A ou B2M.

**6.** Procédé selon la revendication 1 ou 2,
dans lequel le sujet est classé comme ayant un bon pronostic si l'indice de progression de cancer de la prostate est inférieur à un seuil sélectionné ou comme ayant un mauvais pronostic si l'indice de progression de cancer de la prostate est supérieur au seuil sélectionné ; et/ou
dans lequel le sujet est classé comme ayant une prédisposition à un cancer de la prostate qui est susceptible à une progression de la maladie si l'indice de progression de cancer de la prostate est supérieur à un seuil sélectionné ou comme n'ayant pas une prédisposition à un cancer de la prostate qui est susceptible à une progression de la maladie si l'indice de progression de cancer de la prostate est inférieur au seuil sélectionné.

**7.** Procédé selon l'une quelconque des revendications 1-6, comprenant en outre
une stratification du sujet quant au risque de maladie agressive par rapport à une maladie non agressive conformément au pronostic déterminé ou à la prédisposition déterminée.

**8.** Procédé selon la revendication 1 ou 2, dans lequel le mauvais pronostic ou la prédisposition à un cancer de la prostate qui est susceptible à une progression de la maladie est représentatif de l'éligibilité du sujet à être traité à l'aide d'un ou plusieurs des traitements choisis parmi le groupe comprenant une chirurgie de la prostate, une ablation de la prostate, une chimiothérapie, une radiothérapie, une curiethérapie, une lymphadénectomie limitée ou étendue, une hormonothérapie.

**9.** Utilisation d'un produit comprenant :

des amorces et/ou des sondes pour déterminer le niveau d'expression génique pour les gènes de signature : AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, et DYRK2, et facultativement des isoformes de PDE4 comprenant PDE4D5 et/ou PDE4D7 ;
comprenant facultativement en outre des amorces et/ou des sondes pour déterminer les niveaux d'expression d'un ensemble de gènes énumérés sur les Figures 1 ou 2 autres que les gènes mentionnés ci-dessus ; et/ou
comprenant facultativement en outre des amorces et/ou des sondes pour déterminer le niveau d'expression génique d'un gène de référence, de préférence dans lequel le gène de référence est un gène domestique, et de manière plus préférée dans lequel le gène domestique est TBP, HPRT1, ACTB, RPLPO, PUM1, POLR2A ou B2M
pour établir un pronostic pour un patient diagnostiqué atteint d'un cancer de la prostate, pour stratifier un patient diagnostiqué atteint d'un cancer de la prostate, ou pour déterminer un prédisposition à un cancer de la prostate agressif ou indolent chez un patient atteint d'un cancer de la prostate.

**10.** Utilisation selon la revendication 9, dans laquelle le produit est un kit de PCR, un kit de séquençage d'ARN, une biopuce ou un kit de biopuce.

**11.** Produit de programme d'ordinateur, comprenant un code lisible par ordinateur stocké sur un support lisible par ordinateur ou téléchargeable à partir d'un réseau de communication, qui, lorsqu'il est exécuté sur un ordinateur, met en oeuvre une ou plusieurs étapes selon l'une quelconque des revendications 1-8.

**12.** Système comprenant le produit de programme d'ordinateur selon la revendication 11 et un produit comprenant :

des amorces et/ou des sondes pour déterminer le niveau d'expression génique pour les gènes de signature : AZGP1, FBLN1, ILK, KRT15, MEIS2, MYBPC1, PAGE4, SRD5A2, COL1A1, COL3A1, COL5A2, INHBA, THBS2, VCAN, BGN, BIRC5, et DYRK2, et facultativement des isoformes de PDE4 comprenant PDE4D5 et/ou PDE4D7 ;
comprenant facultativement en outre des amorces et/ou des sondes pour déterminer les niveaux d'expression d'un ensemble de gènes énumérés sur les Figures 1 ou 2 autres que les gènes mentionnés ci-dessus ; et/ou
comprenant facultativement en outre des amorces et/ou des sondes pour déterminer le niveau d'expression génique d'un gène de référence, de préférence dans lequel le gène de référence est un gène domestique, et de manière plus préférée dans lequel le gène domestique est TBP, HPRT1, ACTB, RPLPO, PUM1, POLR2A ou B2M.

**13.** Système selon la revendication 12, dans lequel le produit est un kit de PCR, un kit de séquençage d'ARN, une biopuce ou un kit de biopuce.

| TGFbeta KEGG Pathway[1] | Focal Adhesion KEGG Pathway[2] | ECMR Interaction KEGG Pathway[3] | Cell Adhesion KEGG Pathway[4] | Prostate Cancer KEGG Pathway[5] | Primary PCa to metastatic Pca transition[6] | Castration Resistant Prostate Cancer[7] | Other relevant Prostate Cancer Literature[8,9] |
|---|---|---|---|---|---|---|---|
| RHOA | BAD | LAMC1 | PTPRF | BAD | NR4A1 | ABAT | BGN |
| PPP2R1A | RHOA | THBS1 | CD99 | HSP90AB1 | MYH11 | ACAT2 | DYRK2 |
| SKP1 | ACTN4 | ITGB5 | CDH1 | HSP90B1 | IER2 | ACPP | |
| THBS1 | PPP1CC | SDC1 | SDC1 | GSTP1 | MEIS2 | ACTA2 | |
| PPP2CB | LAMC1 | ITGA5 | CLDN4 | RAF1 | EGR1 | ADAMTS | |
| ID2 | ACTB | COL6A3 | ITGA6 | NFKBIA | CTGF | AKAP7 | |
| DCN | ZYX | LAMB1 | ALCAM | CTNNB1 | CYR61 | AKR1C1 | |
| CREBBP | PPP1CA | TNC | SDC4 | TP53 | SYNPO2 | AKR1C2 | |
| EP300 | FLNA | HSPG2 | ICAM1 | RELA | MYLK | AKR1C3 | |
| E2F4 | VCL | ITGA6 | CLDN7 | ARAF | PAGE4 | ALDH1A3 | |
| MYC | CCND2 | COL3A1 | ITGB2 | EGFR | ZMYND11 | ANLN | |
| SMAD4 | MYL9 | SDC4 | SDC3 | CREBBP | IQGAP2 | ANPEP | |
| LTBP1 | PXN | VWF | PVRL2 | EP300 | CSRP1 | ANXA3 | |
| ROCK2 | THBS1 | LAMA4 | CDH3 | PDGFRB | CALD1 | APOE | |
| SMAD2 | ITGB5 | LAMC2 | PTPRM | CDKN1A | TCF21 | AR | |
| THBS2 | ILK | COL1A1 | CDH2 | MTOR | PTN | ARSD | |
| TGFB1 | RAF1 | COL1A2 | CD4 | MAP2K2 | ATF3 | ASPN | |
| ZFYVE16 | ITGA5 | SDC3 | VCAM1 | NRAS | MYL9 | ASTN2 | |
| RPS6KB2 | PPP1CB | SV2A | CLDN3 | MAP2K1 | TPM2 | ATF3 | |
| ACVR1 | COL6A3 | THBS2 | NRCAM | FOXO1 | GOLGB1 | ATM | |
| TFDP1 | JUN | COL5A1 | CLDN5 | PIK3R3 | ZFP36 | BUB1B | |
| RPS6KB1 | LAMB1 | LAMA3 | SELL | E2F1 | SELE | CA5B | |
| THBS4 | CTNNB1 | CD44 | CD22 | PDGFRA | PPP1R12B | CaMKIIN | |
| SMAD7 | ACTG1 | VTN | L1CAM | RB1 | BMPR1A | CBFA2T3 | |
| ZFYVE9 | PPP1R12A | ITGB3 | VCAN | IGF1R | CNN1 | CCNB1 | |

FIG. 1

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| INHBA | TNC | THBS4 | CDH5 | FGFR2 | CCL2 | CD44 | |
| FST | ITGA6 | ITGB4 | ICAM2 | BCL2 | MGP | CDC20 | |
| SMAD5 | CCND3 | ITGA2 | ICAM3 | E2F3 | SRF | CDH11 | |
| INHBB | COL3A1 | LAMA2 | CD40 | AKT2 | KRT15 | CDKN2C | |
| BMP2 | EGFR | DAG1 | NCAM2 | PIK3CD | TAGLN | CDKN3 | |
| RBL1 | GRLF1 | COMP | CD86 | CASP9 | TPSAB1 | CENPF | |
| ACVR2A | BIRC2 | ITGB7 | ITGB7 | KRAS | FHL1 | CFHL1P | |
| SMAD3 | VWF | ITGB8 | CD8A | PDGFB | NR4A3 | CHN1 | |
| BMP5 | LAMA4 | ITGA4 | ITGAM | CDK2 | FUCA1 | CKS2 | |
| SMURF2 | VASP | RELN | ITGB8 | CREB1 | DIO2 | CMKOR1 | |
| COMP | CRK | ITGA9 | CD2 | PIK3CA | PCP4 | COL11A1 | |
| BMP6 | LAMC2 | CD36 | ITGA4 | PDPK1 | HLA-DRB1 | COL1A1 | |
| SMAD9 | PDGFRB | ITGA2B | NLGN1 | KLK3 | VCL | COL1A2 | |
| GDF5 | COL1A1 | ITGA10 | ITGA9 | TGFA | CTSO | COL3A1 | |
| SMAD6 | RAP1A | CHAD | SELP | CCNE2 | TMEM49 | COL4A1 | |
| TGFBR2 | COL1A2 | GP9 | SPN | TCF7 | SMAD4 | COL5A1 | |
| CDKN2B | MAP2K1 | TNR | SELE | MDM2 | LMAN1 | COL5A2 | |
| PITX2 | PIK3R3 | HMMR | CDH15 | PDGFA | FOSB | COL6A3 | |
| CUL1 | ROCK2 | IBSP | CD28 | CREB5 | AGL | CRYM | |
| INHBC | THBS2 | GP1BA | CDH4 | BRAF | SPG20 | CSPG2 | |
| ID3 | PDGFRA | GP5 | CLDN11 | EGF | ABCC4 | CSRP1 | |
| BMP8B | PAK4 | ITGB6 | CNTN2 | PIK3CG | PTPRK | CTSH | |
| BMP8A | DOCK1 | COL6A2 | CD80 | INS | DST | CTSK | |
| SMAD1 | MAPK9 | LAMB3 | PTPRC | SRD5A2 | DUSP5 | CUTL2 | |
| MAPK1 | TLN1 | THBS3 | CD226 | E2F2 | ECM2 | CYP27A1 | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| PPP2CA | CAV2 | ITGA7 | PDCD1 | PIK3R2 | PAM | CYR61 | |
| ID1 | COL5A1 | SPP1 | CD40LG | AKT1 | DMXL1 | DCN | |
| ID4 | MET | LAMA5 | GLG1 | MAPK1 | KRT5 | DDAH1 | |
| THBS3 | ELK1 | FN1 | MADCAM1 | CCND1 | TIPARP | DDIT4 | |
| BMP7 | IGF1R | COL4A6 | PVRL1 | CDKN1B | SSR3 | DDX39 | |
| TGFB3 | VEGFB | CD47 | CD6 | NFKB1 | ID1 | DEF6D | |
| TGFB2 | BCL2 | ITGB1 | CADM1 | IKBKB | BTG2 | DOCK5 | |
| BMPR2 | LAMA3 | COL4A2 | CD34 | CREB3 | SATB1 | DPT | |
| IFNG | AKT2 | COL4A1 | SELPLG | IGF1 | UBE2J1 | DTL | |
| BMPR1B | ACTN2 | COL6A1 | NCAM1 | NKX3-1 | TPM1 | DUSP1 | |
| INHBE | PIK3CD | SDC2 | NRXN2 | GSK3B | MEIS1 | E2F3 | |
| CHRD | KDR | AGRN | MPZ | HSP90AA1 | SFRS2B | EGR1 | |
| BMP4 | PDGFB | COL2A1 | ICOS | ERBB2 | CENPC1 | EIF2C2 | |
| MAPK3 | PIK3CA | ITGA8 | CLDN14 | LEF1 | C18orf1 | EIF2S3 | |
| RBL2 | PDPK1 | COL4A4 | ICOSLG | AR | FOXF1 | ENPP2 | |
| SMURF1 | VTN | ITGA1 | CNTN1 | SOS2 | GADD45B | EPHB2 | |
| BMPR1A | RAPGEF1 | TNN | CADM3 | MAPK3 | SMTN | EPHX2 | |
| SP1 | ITGB3 | LAMB4 | ITGB1 | PIK3R1 | SORBS2 | EZH2 | |
| ACVR2B | PARVB | SV2C | SDC2 | CREB3L2 | MITF | FBN1 | |
| E2F5 | THBS4 | LAMB2 | PVR | AKT3 | PPAP2B | FLJ42562 | |
| NODAL | MAPK10 | COL5A3 | JAM3 | PIK3CB | SPARCL1 | FN1 | |
| ROCK1 | ITGB4 | LAMC3 | PVRL3 | TCF7L2 | TNFAIP3 | FOS | |
| LEFTY1 | ITGA2 | COL5A2 | NFASC | SOS1 | CGRRF1 | GJB1 | |
| GDF7 | LAMA2 | LAMA1 | CLDN18 | HRAS | KCNMB1 | GMIP | |
| GDF6 | MYL5 | ITGA3 | ITGA8 | CREB3L1 | PDE4D | GPNMB | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| TNF | MYLPF | ITGA11 | CLDN8 | CCNE1 | PJA2 | HLA-A | |
| NOG | PDGFA | COL6A6 | CLDN9 | GRB2 | MIA3 | HOXC6 | |
| RBX1 | VAV2 | GP1BB | CNTNAP2 | INSRR | RPS23 | HPRT1 | |
| ACVR1C | COMP | | CD8B | PDGFC | KLF4 | HSPA1B | |
| | ITGB7 | | MAG | PDGFD | ST13 | HSPC163 | |
| | ITGB8 | | CLDN1 | PIK3R5 | SLC35A3 | IBSP | |
| | ITGA4 | | JAM2 | TCF7L1 | CACNB2 | IER2 | |
| | RELN | | CNTNAP1 | ATF4 | PYROXD1 | IFI30 | |
| | ITGA9 | | SIGLEC1 | CREB3L4 | TMEM47 | JAM3 | |
| | BRAF | | CLDN15 | IKBKG | PLN | JUN | |
| | RAC3 | | NLGN3 | CREB3L3 | TIA1 | JUNB | |
| | CRKL | | PDCD1LG2 | CHUK | HLA-DPB1 | KIF20A | |
| | VAV1 | | CLDN16 | PTEN | ACTA2 | KIF4A | |
| | EGF | | CLDN17 | | MFAP4 | KLK2 | |
| | PRKCG | | CTLA4 | | PLA2G2A | KLK3 | |
| | SHC3 | | F11R | | MATN2 | KPNA2 | |
| | PIK3CG | | NLGN4X | | Stratagene lung | KRT18 | |
| | ITGA2B | | HLA-DPB1 | | ITPR2 | LAMA4 | |
| | XIAP | | HLA-DPA1 | | NEO1 | LAMB1 | |
| | FIGF | | HLA-B | | UTRN | LAMC1 | |
| | ITGA10 | | HLA-C | | PDE8B | LAPTM5 | |
| | CHAD | | CD276 | | PPP1R12A | LCP1 | |
| | PRKCA | | HLA-DOA | | SLC15A2 | LDLR | |
| | TNR | | ESAM | | ZNF650 | LGALS3 | |
| | PIK3R2 | | HLA-DQA2 | | NR4A2 | LRRC15 | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | AKT1 | | HLA-DOB | | ALDH18A1 | LUM | |
| | IBSP | | CD274 | | DKFZP564O0823 | MAP4K4 | |
| | RAC2 | | CLDN19 | | ZBTB10 | MELK | |
| | PTK2 | | HLA-DRB1 | | UAP1 | MMP9 | |
| | FLNC | | HLA-DRB5 | | SON | MPPED2 | |
| | PRKCB | | CLDN20 | | RARRES1 | MRC1 | |
| | ITGB6 | | CLDN22 | | JUN | MSMB | |
| | CAV3 | | CLDN6 | | SFRS5 | MT1M | |
| | MAPK1 | | OCLN | | TM9SF3 | MYBPC1 | |
| | ACTN1 | | NLGN2 | | METAP2 | MYH11 | |
| | RAC1 | | NEGR1 | | RLN1 | MYLK | |
| | CAPN2 | | AZGP1 | | JUNB | NARG1 | |
| | CCND1 | | | | FBLN1 | NBL1 | |
| | CDC42 | | | | MAP9 | NEK2 | |
| | PAK2 | | | | DYNC1I2 | NKX3.1 | |
| | COL6A2 | | | | DNAJC3 | NNMT | |
| | DIAPH1 | | | | FLJ39822 | NPEPL1 | |
| | LAMB3 | | | | ZNF536 | NR4A1 | |
| | IGF1 | | | | PUM2 | NRIP3 | |
| | THBS3 | | | | PALLD | OLFML2B | |
| | PAK1 | | | | SLC20A2 | OLFML3 | |
| | PGF | | | | ISL1 | OMD | |
| | ITGA7 | | | | FLNC | P2RY5 | |
| | MYL2 | | | | PBXIP1 | PAFAH1B | |
| | SPP1 | | | | UFM1 | PART1 | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | GSK3B | | | | C14orf24 | PHACS | |
| | VEGFC | | | | IL1R1 | PHTF2 | |
| | HGF | | | | TCEAL1 | PLOD2 | |
| | FYN | | | | RPL10 | PLS3 | |
| | LAMA5 | | | | FLNA | POSTN | |
| | FLT4 | | | | EGR2 | PRKCA | |
| | MAPK8 | | | | C9orf61 | PTPRU | |
| | FN1 | | | | CDC42EP3 | PTTG1 | |
| | VEGFA | | | | ARMCX3 | PTTG2 | |
| | BIRC3 | | | | ERGIC2 | PYGB | |
| | PAK7 | | | | SF1 | RAB31 | |
| | ERBB2 | | | | DKFZP586H2123 | RACGAP | |
| | COL4A6 | | | | AKT3 | RAD21 | |
| | SOS2 | | | | DSTN | RAFTLIN | |
| | ITGB1 | | | | GHR | RAPSN | |
| | COL4A2 | | | | ALDH1A3 | RDH11 | |
| | COL4A1 | | | | CD69 | RPL11 | |
| | MAPK3 | | | | PTPRN2 | SDC2 | |
| | COL6A1 | | | | CREB3L1 | SEC61A2 | |
| | PIK3R1 | | | | PRDM2 | SELT | |
| | PIP5K1C | | | | FZD7 | SERPINE | |
| | AKT3 | | | | PIGK | SERPINF | |
| | PIK3CB | | | | PKIG | SESN3 | |
| | TLN2 | | | | BMI1 | SET | |
| | SOS1 | | | | NCKIPSD | SH3BGRL2 | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | HRAS | | | | FAM114A1 | SLC9A9 | |
| | SRC | | | | GULP1 | SMA4 | |
| | SHC2 | | | | ENC1 | SMC4 | |
| | COL2A1 | | | | SLC30A7 | SNAP91 | |
| | ITGA8 | | | | SEMA3C | SNRPE | |
| | COL4A4 | | | | ARL6IP5 | SORD | |
| | PAK3 | | | | NBL1 | SPARC | |
| | ITGA1 | | | | RND3 | SPP1 | |
| | SHC1 | | | | LOC171220 | SSU72 | |
| | GRB2 | | | | PCM1 | ST6GALN | |
| | TNN | | | | RAP1A | SULF1 | |
| | LAMB4 | | | | SPOCK3 | TACSTD2 | |
| | LAMB2 | | | | SLC4A4 | TAOK3 | |
| | PARVA | | | | PTGS2 | THBS2 | |
| | ARHGAP5 | | | | NET1 | THYN1 | |
| | PDGFC | | | | NBPF15 | TMEM132B | |
| | VAV3 | | | | KLF6 | TMEM46 | |
| | COL5A3 | | | | TM9SF2 | TNFAIP6 | |
| | PDGFD | | | | SFRP1 | TNK2 | |
| | LAMC3 | | | | MMP7 | TOP2A | |
| | PAK6 | | | | SLC12A2 | TRA2A | |
| | MYL7 | | | | RPL5 | TRAM1 | |
| | PIK3R5 | | | | PIGB | TRPM8 | |
| | MYL12B | | | | OGN | UBE2T | |
| | MYL10 | | | | MTMR6 | UGT2B15 | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | COL5A2 | | | | SLAIN2 | VIPR1 | |
| | FLT1 | | | | LOC440295 | WNT5A | |
| | MYL12A | | | | EYA1 | XBP1 | |
| | PARVG | | | | PLAGL1 | ZFP36 | |
| | RAP1B | | | | RBPMS | BIRC5 | |
| | LAMA1 | | | | GSN | | |
| | ITGA3 | | | | SUV420H1 | ZWINT | |
| | ROCK1 | | | | MPDZ | | |
| | SHC4 | | | | SLC2A3 | | |
| | MYLK2 | | | | SPCS3 | | |
| | ITGA11 | | | | ACTG2 | | |
| | COL6A6 | | | | PIGN | | |
| | MYLK3 | | | | RYBP | | |
| | MYLK | | | | LGALS3 | | |
| | PTEN | | | | PRKACB | | |
| | ALDH3A2 | | | | SSB | | |
| | | | | | SOD3 | | |
| | | | | | TP63 | | |
| | | | | | LTBP4 | | |
| | | | | | KIAA0776 | | |
| | | | | | TERF2IP | | |
| | | | | | CNTN1 | | |
| | | | | | COX7A1 | | |
| | | | | | HLA-DQB1 | | |
| | | | | | SLC35A1 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | ID2 | | |
| | | | | | NSUN7 | | |
| | | | | | DDR2 | | |
| | | | | | STX17 | | |
| | | | | | LUM | | |
| | | | | | RAB4A | | |
| | | | | | KCNAB1 | | |
| | | | | | SNAP23 | | |
| | | | | | IDE | | |
| | | | | | MXRA5 | | |
| | | | | | CIRBP | | |
| | | | | | SRI | | |
| | | | | | PGM3 | | |
| | | | | | SGMS1 | | |
| | | | | | IQGAP1 | | |
| | | | | | FNDC3A | | |
| | | | | | SLC30A9 | | |
| | | | | | EXTL2 | | |
| | | | | | BAG1 | | |
| | | | | | FRY | | |
| | | | | | KIAA1450 | | |
| | | | | | TNKS2 | | |
| | | | | | SFRS7 | | |
| | | | | | ANGPT1 | | |
| | | | | | ELOVL7 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | EGR3 | | |
| | | | | | ANKRD17 | | |
| | | | | | TSPYL1 | | |
| | | | | | CAPZA2 | | |
| | | | | | AUH | | |
| | | | | | NT5C2 | | |
| | | | | | ZFX | | |
| | | | | | PRUNE2 | | |
| | | | | | SLC35F5 | | |
| | | | | | CD46 | | |
| | | | | | DUSP1 | | |
| | | | | | MAN2B2 | | |
| | | | | | ELL2 | | |
| | | | | | RNF141 | | |
| | | | | | AVEN | | |
| | | | | | NEDD4L | | |
| | | | | | FMOD | | |
| | | | | | MPZL2 | | |
| | | | | | PPP1R2 | | |
| | | | | | ALDH3A2 | | |
| | | | | | PDLIM3 | | |
| | | | | | CNTN3 | | |
| | | | | | PCMTD1 | | |
| | | | | | PELO | | |
| | | | | | SSPN | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | APPBP2 | | |
| | | | | | COL15A1 | | |
| | | | | | RPL7A | | |
| | | | | | FILIP1L | | |
| | | | | | ELF1 | | |
| | | | | | IDH3A | | |
| | | | | | REV1 | | |
| | | | | | MSMB | | |
| | | | | | RASA1 | | |
| | | | | | ISLR | | |
| | | | | | C9orf5 | | |
| | | | | | FAAH | | |
| | | | | | TMEM50B | | |
| | | | | | RPL31 | | |
| | | | | | HLA-DRA | | |
| | | | | | PSAP | | |
| | | | | | TNFSF10 | | |
| | | | | | C10orf72 | | |
| | | | | | LEPR | | |
| | | | | | TCTN3 | | |
| | | | | | C10orf56 | | |
| | | | | | GPD1L | | |
| | | | | | PPP1CB | | |
| | | | | | TMEM50A | | |
| | | | | | ASTN2 | | |

FIG. 1 (continued)

| TGFbeta KEGG Pathway[1] | Focal Adhesion KEGG Pathway[2] | ECMR Interaction KEGG Pathway[3] | Cell Adhesion KEGG Pathway[4] | Prostate Cancer KEGG Pathway[5] | Primary PCa to metastatic Pca transition[6] | Castration Resistant Prostate Cancer[7] | Other relevant Prostate Cancer Literature[8,9] |
|---|---|---|---|---|---|---|---|
| | | | | | MAP1LC3A | | |
| | | | | | METAP1 | | |
| | | | | | MOXD1 | | |
| | | | | | THBS4 | | |
| | | | | | ESR1 | | |
| | | | | | IFNGR1 | | |
| | | | | | ACYP2 | | |
| | | | | | CNTNAP2 | | |
| | | | | | LAPTM4A | | |
| | | | | | C12orf23 | | |
| | | | | | SERPINB5 | | |
| | | | | | MED21 | | |
| | | | | | SORL1 | | |
| | | | | | NT5DC1 | | |
| | | | | | CX3CL1 | | |
| | | | | | COL4A6 | | |
| | | | | | ACPP | | |
| | | | | | STXBP3 | | |
| | | | | | RLN2 | | |
| | | | | | GSTM1 | | |
| | | | | | GOLM1 | | |
| | | | | | PDIA3 | | |
| | | | | | GLOD4 | | |
| | | | | | GLUD1 | | |
| | | | | | PIM1 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | CREM | | |
| | | | | | DACH1 | | |
| | | | | | SLC2A10 | | |
| | | | | | PVRL3 | | |
| | | | | | C17orf48 | | |
| | | | | | ILK | | |
| | | | | | ZBTB20 | | |
| | | | | | ZRANB2 | | |
| | | | | | SPOP | | |
| | | | | | P2RY5 | | |
| | | | | | NEK1 | | |
| | | | | | MCL1 | | |
| | | | | | ITM2B | | |
| | | | | | PTPN13 | | |
| | | | | | SRRM1 | | |
| | | | | | ZNF451 | | |
| | | | | | OSR2 | | |
| | | | | | KIF13B | | |
| | | | | | DDX3Y | | |
| | | | | | TARBP1 | | |
| | | | | | EPS15 | | |
| | | | | | RANBP2 | | |
| | | | | | RNF13 | | |
| | | | | | PKN2 | | |
| | | | | | PINK1 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | SLC39A6 | | |
| | | | | | DDHD2 | | |
| | | | | | AFAP1 | | |
| | | | | | AMD1 | | |
| | | | | | RBM26 | | |
| | | | | | ZNF468 | | |
| | | | | | ARHGEF10 | | |
| | | | | | C10orf118 | | |
| | | | | | ASAH1 | | |
| | | | | | KCTD3 | | |
| | | | | | COCH | | |
| | | | | | SH3BGR | | |
| | | | | | CCNC | | |
| | | | | | JUND | | |
| | | | | | RP11-50D16.3 | | |
| | | | | | MAP7 | | |
| | | | | | TMF1 | | |
| | | | | | TMEM168 | | |
| | | | | | PTGER2 | | |
| | | | | | ERCC5 | | |
| | | | | | TMED10 | | |
| | | | | | EIF3A | | |
| | | | | | RPLP1 | | |
| | | | | | MFGE8 | | |
| | | | | | GDI2 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | Gessler Wilms t | | |
| | | | | | SCCPDH | | |
| | | | | | RAB27B | | |
| | | | | | SMARCA2 | | |
| | | | | | RNF103 | | |
| | | | | | MSTP9 | | |
| | | | | | CDC42BPA | | |
| | | | | | TCF12 | | |
| | | | | | DPP4 | | |
| | | | | | CXADR | | |
| | | | | | GOLPH3 | | |
| | | | | | GARNL1 | | |
| | | | | | C10orf116 | | |
| | | | | | MLLT4 | | |
| | | | | | VAMP3 | | |
| | | | | | SH3YL1 | | |
| | | | | | ZNF185 | | |
| | | | | | RUFY3 | | |
| | | | | | RPL35A | | |
| | | | | | SOAT1 | | |
| | | | | | HLA-DQA1 | | |
| | | | | | TICAM2 | | |
| | | | | | AKAP11 | | |
| | | | | | LOH11CR2A | | |
| | | | | | KIAA1109 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | DIXDC1 | | |
| | | | | | NDUFS4 | | |
| | | | | | PLA2G5 | | |
| | | | | | BTF3 | | |
| | | | | | PLP1 | | |
| | | | | | SMAD2 | | |
| | | | | | KDELR3 | | |
| | | | | | ATXN10 | | |
| | | | | | TLOC1 | | |
| | | | | | LMOD1 | | |
| | | | | | PRKAR1A | | |
| | | | | | CCDC47 | | |
| | | | | | DHRS3 | | |
| | | | | | C10orf137 | | |
| | | | | | LRPAP1 | | |
| | | | | | TLK1 | | |
| | | | | | KLF9 | | |
| | | | | | OAT | | |
| | | | | | PSMD5 | | |
| | | | | | ZHX2 | | |
| | | | | | DIS3 | | |
| | | | | | ARMC1 | | |
| | | | | | MON1B | | |
| | | | | | CCBL2 | | |
| | | | | | ACSM3 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | PCTK1 | | |
| | | | | | TOP2A | | |
| | | | | | ZMYND8 | | |
| | | | | | CSNK1G2 | | |
| | | | | | PGK1 | | |
| | | | | | KIFC1 | | |
| | | | | | STC2 | | |
| | | | | | AURKA | | |
| | | | | | STIP1 | | |
| | | | | | NUP210 | | |
| | | | | | CCNA2 | | |
| | | | | | WSB2 | | |
| | | | | | TPX2 | | |
| | | | | | UBE2S | | |
| | | | | | DDX39 | | |
| | | | | | MYBL2 | | |
| | | | | | TFRC | | |
| | | | | | CDC6 | | |
| | | | | | CDKN3 | | |
| | | | | | PTTG1 | | |
| | | | | | AR | | |
| | | | | | TMEM118 | | |
| | | | | | BRD2 | | |
| | | | | | AYTL2 | | |
| | | | | | MKI67 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | RAB6B | | |
| | | | | | GRB2 | | |
| | | | | | TIE1 | | |
| | | | | | PLK1 | | |
| | | | | | CD36 | | |
| | | | | | LYN | | |
| | | | | | SAC3D1 | | |
| | | | | | VDR | | |
| | | | | | DLG7 | | |
| | | | | | CDC2 | | |
| | | | | | FOXM1 | | |
| | | | | | TUBG1 | | |
| | | | | | HSF1 | | |
| | | | | | TRIO | | |
| | | | | | ABCC5 | | |
| | | | | | EIF5 | | |
| | | | | | CUGBP1 | | |
| | | | | | TUBG2 | | |
| | | | | | GAPDH | | |
| | | | | | septin4 | | |
| | | | | | FYN | | |
| | | | | | OIT3 | | |
| | | | | | TFPI | | |
| | | | | | LHX2 | | |
| | | | | | CSMD1 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | RFC5 | | |
| | | | | | RBP5 | | |
| | | | | | UBE2G2 | | |
| | | | | | KIAA0101 | | |
| | | | | | SNAI1 | | |
| | | | | | SMYD2 | | |
| | | | | | DDIT4 | | |
| | | | | | MAST1 | | |
| | | | | | MAP3K9 | | |
| | | | | | MLXIPL | | |
| | | | | | PDK3 | | |
| | | | | | FLJ33996 | | |
| | | | | | DPF3 | | |
| | | | | | GRAMD1B | | |
| | | | | | PLXND1 | | |
| | | | | | AK1 | | |
| | | | | | LOC157562 | | |
| | | | | | KCNAB2 | | |
| | | | | | EFNB1 | | |
| | | | | | RPA3 | | |
| | | | | | CETP | | |
| | | | | | SEC14L1 | | |
| | | | | | DHX34 | | |
| | | | | | PRC1 | | |
| | | | | | BOLA2 | | |

FIG. 1 (continued)

| TGFbeta KEGG Pathway [1] | Focal Adhesion KEGG Pathway [2] | ECMR Interaction KEGG Pathway [3] | Cell Adhesion KEGG Pathway [4] | Prostate Cancer KEGG Pathway [5] | Primary PCa to metastatic Pca transition [6] | Castration Resistant Prostate Cancer [7] | Other relevant Prostate Cancer Literature [8,9] |
|---|---|---|---|---|---|---|---|
| | | | | | MGC16121 | | |
| | | | | | MTA1 | | |
| | | | | | LMNB2 | | |
| | | | | | SCRIB | | |
| | | | | | PEG10 | | |
| | | | | | MICAL3 | | |
| | | | | | FSCN1 | | |
| | | | | | EIF4G1 | | |
| | | | | | KCTD5 | | |
| | | | | | ALDOA | | |
| | | | | | PARVB | | |
| | | | | | EZH2 | | |
| | | | | | UBE2C | | |
| | | | | | CRABP1 | | |
| | | | | | HOXA4 | | |
| | | | | | TCF3 | | |
| | | | | | HSP90AA1 | | |
| | | | | | NF2 | | |
| | | | | | THBS2 | | |
| | | | | | BUB1 | | |
| | | | | | FOXK2 | | |
| | | | | | DDX12 | | |
| | | | | | HOXC6 | | |
| | | | | | ACOT12 | | |
| | | | | | SH3GL1 | | |

FIG. 1 (continued)

[1]http://csbi.ltdk.helsinki.fi/anduril/tcga-gbm/table4_2.html: hsa04350
[2]http://csbi.ltdk.helsinki.fi/anduril/tcga-gbm/table4_2.html: hsa04510
[5]http://csbi.ltdk.helsinki.fi/anduril/tcga-gbm/table4_2.html: hsa05215
[6]Gorlov IP et al: Candidate pathways and genes for prostate cancer: a meta-analysis of gene expression data; BMC Medical Genomics 2009, 2:48
[7]Stanbrough M et al: Increased Expression of Genes Converting Adrenal Androgens to Testosterone in Androgen-Independent Prostate Cancer; Cancer Res 2006;66:2815-2825
[8]Lapointe J et al. Gene expression profiling identifies clinically relevant subtypes of prostate cancer; PNAS 2003; 101(3):8110816
[9]Sun Y et al. Optimizing Molecular Signatures for Predicting Prostate Cancer Recurrence. The Prostate, 69:1119-1127 (2009)

FIG. 1 (continued)

| Gene No. | Gene Name | Taylor (2010) | | Boormans (2013) | | Sun (2010) | | Sboner (2010) | | Nakagawa (2010) | | Comb. p-value | Gene ranking | |
|---|---|---|---|---|---|---|---|---|---|---|---|---|---|---|
| | | p-value | Reg. | p-value | Reg. | p-value | Reg. | p-value | Reg. | p-value | Reg. | | Dw | Up |
| 1 | ACPP | 2.30E-02 | Dw | | | 5.97E-03 | Dw | 3.51E-03 | Dw | 2.78E-03 | Dw | | 3 | |
| 2 | ALDH3A2 | 7.43E-02 | Dw | 8.24E-02 | Dw | 3.52E-02 | Dw | 3.68E-02 | Dw | | | 7.93E-06 | 2 | |
| 3 | ANPEP | 3.68E-02 | Dw | 5.23E-03 | Dw | | | 3.02E-03 | Dw | 8.68E-05 | Dw | 5.05E-11 | 2 | |
| 4 | AURKA | 6.24E-02 | Up | | | | | 2.22E-02 | Dw | 3.36E-04 | Up | | | 3 |
| 5 | AZGP1 | 9.36E-02 | Dw | 5.94E-03 | Dw | | | 5.50E-04 | Dw | 4.82E-05 | Dw | 1.48E-11 | 1 | |
| 6 | CD44 | 5.49E-02 | Dw | 2.80E-02 | Dw | | | 5.61E-03 | Dw | 2.97E-03 | Dw | 2.56E-08 | 2 | |
| | | | | | | | | | | 3.70E-02 | Dw | | | |
| 7 | CD276 | 4.52E-02 | Up | 2.43E-02 | Up | | | | | | | | | 3 |
| 8 | CD80 | 2.03E-02 | Up | 2.83E-02 | Up | | | | | | | | | 3 |
| 9 | CDC42EP3 | 2.93E-02 | Dw | 4.57E-02 | Dw | | | | | | | | 3 | |
| 10 | CENPC1 | 4.52E-02 | Dw | 2.44E-02 | Dw | | | | | | | | 3 | |
| 11 | COL11A1 | 2.84E-03 | Up | 3.55E-02 | Up | | | | | | | | | 3 |
| 12 | COL1A1 | 9.96E-03 | Up | 2.25E-03 | Up | | | 5.55E-03 | Up | 7.39E-07 | Up | 9.17E-14 | | 1 |
| 13 | COL3A1 | 8.33E-03 | Up | 6.22E-03 | Up | 5.84E-02 | Up | 7.25E-04 | Up | | | 1.37E-10 | | 1 |
| | | | | | | 6.26E-02 | Up | | | | | | | |
| 14 | COL5A2 | 2.25E-02 | Up | 5.47E-04 | Up | 4.61E-02 | Up | 7.39E-03 | Up | | | 2.95E-10 | | 1 |
| | | | | | | 7.02E-02 | Up | | | | | | | |
| 15 | CYP27A1 | 9.33E-03 | Dw | 7.90E-02 | Dw | 5.04E-03 | Dw | 2.33E-03 | Dw | | | 8.65E-09 | 2 | |
| 16 | E2F3 | | | | | 2.32E-04 | Up | 4.37E-03 | Up | 4.72E-02 | Up | | | 3 |
| 17 | E2F5 | 1.35E-02 | Up | | | 2.42E-02 | Up | | | 1.47E-05 | Up | | | 3 |
| 18 | EGF | | | 2.65E-02 | Dw | | | 2.80E-04 | Dw | 2.49E-02 | Dw | | 3 | |
| 19 | EPHX2 | 3.33E-02 | Dw | 4.09E-02 | Dw | 5.58E-03 | Dw | 8.01E-05 | Dw | | | 6.08E-10 | 2 | |
| 20 | EZH2 | | | | | | | 5.24E-03 | Up | 4.43E-06 | Up | | | 3 |
| 21 | FBLN1 | 7.89E-02 | Dw | 3.30E-03 | Dw | 5.44E-03 | Dw | 3.79E-02 | DW | 5.97E-09 | Dw | 3.20E-16 | 1 | |
| | FBLN1 | | | | | 4.05E-02 | Dw | | | | | | | |
| | FBLN1 | | | | | 2.39E-02 | Dw | | | | | | | |
| | FBLN1 | | | | | 3.10E-02 | Dw | | | | | | | |

FIG. 2

| Gene No. | Gene Name | Taylor (2010) | | Boormans (2013) | | Sun (2010) | | Sboner (2010) | | Nakagawa (2010) | | Comb. p-value | Gene ranking | |
|---|---|---|---|---|---|---|---|---|---|---|---|---|---|---|
| 22 | FMOD | | | 7.93E-03 | Dw | | | 2.48E-02 | Dw | 4.53E-06 | Dw | | 3 | |
| 23 | IGF1 | | | | | 3.40E-02 | Dw | 2.26E-02 | Dw | 2.78E-05 | Dw | | 3 | |
| | | | | | | 4.91E-02 | Dw | | | | | | | |
| | | | | | | 2.87E-02 | Dw | | | | | | | |
| 24 | ILK | | | 4.01E-02 | Dw | 2.09E-03 | Dw | 3.99E-02 | Dw | 3.39E-08 | Dw | 1.13E-13 | 1 | |
| 25 | INHBA | 1.59E-05 | Up | 5.46E-04 | Up | 8.84E-03 | Up | 4.26E-04 | Up | 5.83E-21 | Up | 1.91E-34 | | 1 |
| 26 | ITGA11 | 4.68E-04 | Up | 9.32E-03 | Up | | | | | | | | | 3 |
| 27 | JUN | | | 5.69E-03 | Dw | 5.36E-02 | Dw | 9.82E-03 | Dw | 9.37E-05 | Dw | 2.81E-10 | 2 | |
| | | | | | | 6.65E-02 | Dw | | | 4.73E-04 | Dw | 3.14E-05 | | |
| 28 | KRT15 | 3.46E-02 | DW | 4.70E-02 | Dw | 6.96E-02 | Dw | 2.64E-02 | Dw | 9.30E-07 | Dw | 2.78E-12 | 1 | |
| 29 | LAMB1 | | | | | | | 8.90E-03 | Up | 3.57E-03 | Up | | | 3 |
| 30 | LAMC3 | 9.70E-03 | Up | 2.95E-02 | Up | | | | | | | | | 3 |
| 31 | MAPK10 | | | 7.39E-02 | Dw | | | 3.88E-02 | Dw | 6.04E-06 | Dw | | 3 | |
| 32 | MEIS2 | 4.57E-02 | Dw | 9.64E-03 | Dw | 3.34E-02 | Dw | | | 9.56E-07 | Dw | 1.41E-11 | 1 | |
| 33 | MT1M | 2.09E-02 | Dw | 3.64E-02 | Dw | 5.23E-03 | Dw | | | | | | 3 | |
| 34 | MYBL2 | 4.00E-02 | Up | | | | | 4.94-02 | Up | | | | | 3 |
| 35 | MYBPC1 | 4.30E-04 | Dw | 5.90E-02 | Dw | | | 9.49E-10 | Dw | 4.79E-05 | Dw | 1.15E-18 | 1 | |
| 36 | MYH11/ NDE1 | 2.65E-02 | Dw | 4.67E-02 | Dw | 5.66E-04 | Dw | 4.06E-02 | Dw | 8.39E-04 | Dw | | 3 | |
| 37 | MYLK | | | 5.40E-02 | Dw | 9.62E-05 | Dw | 2.30E-02 | Dw | 2.27E-03 | Dw | 2.71E-10 | 2 | |
| 38 | C1orf151/NBL1 / NBL1 / MINOS1 | 2.07E-02 | Dw | | | 2.67E-04 | Dw | 9.08E-03 | Dw | 9.04E-03 | Dw | | 3 | |
| | | | | | | 7.30E-04 | Dw | | | | | | | |
| 39 | NEO1 | | | | | | | 2.86E-02 | Dw | 4.54E-03 | Dw | | 3 | |
| 40 | OLFML2B | 3.75E-04 | Up | 8.27E-03 | Up | | | | | | | | | 3 |
| 41 | PAGE4 | | | 2.36E-03 | Dw | 5.74E-03 | Dw | 9.48E-03 | Dw | 3.29E-05 | Dw | 4.23E-12 | 1 | |
| 42 | PDE4D | 4.43E-04 | Dw | 3.73E-02 | Dw | | | | | | | 1.65E-05 | 1 | |

FIG. 2 (continued)

| Gene No. | Gene Name | Taylor (2010) | | Boormans (2013) | | Sun (2010) | | Sboner (2010) | | Nakagawa (2010) | | Comb. p-value | Gene ranking | |
|---|---|---|---|---|---|---|---|---|---|---|---|---|---|---|
| 43 | PDE4D7 (normal. to PDE4D5) | 2.46E-03 | Dw | 2.07E-02 | Dw | | | | | | | 5.09E-05 | 1 | |
| 44 | PYGB | 2.87E-02 | Dw | 2.84E-02 | Dw | | | | | | | | 3 | |
| 45 | SATB1 | 5.58E-03 | Dw | 2.04E-02 | Dw | | | 5.00E-05 | Dw | | | | 3 | |
| 46 | SELE | 2.46E-02 | Dw | 3.65E-02 | Dw | | | 4.07E-02 | Dw | | | | 3 | |
| 47 | SLC15A2 | 3.24E-03 | Dw | 2.47E-02 | Dw | 6.07E-02 | Dw | 7.68E-06 | Dw | | | 3.73E-11 | 2 | |
| 48 | SMAD4 | 3.38E-02 | Dw | 3.29E-02 | Dw | | | | | 2.30E-05 | Dw | | 3 | |
| 49 | SPOCK3 | 4.62E-02 | Dw | 3.05E-02 | Dw | 4.31E-02 | Dw | | | | | | 3 | |
| 50 | SRD5A2 | 2.06E-02 | Dw | 3.63E-04 | Dw | 2.55E-02 | Dw | 2.06E-03 | Dw | 7.04E-03 | Dw | 2.76E-12 | 1 | |
| 51 | TGFB2 | | | | | 5.64E-03 | Dw | 5.13E-04 | Dw | 2.15E-02 | Dw | | 3 | |
| 52 | TGFB3 | | | 6.08E-03 | Dw | 9.01E-04 | Dw | | | 7.41E-04 | Dw | | 3 | |
| 53 | THBS2 | 7.65E-04 | Up | 8.24E-03 | Up | 9.65E-03 | Up | | | 7.82E-11 | Up | 4.76E-18 | | 1 |
| 54 | VCAN | 4.71E-04 | Up | 2.83E-02 | Up | 8.72E-03 | Up | 8.71E-03 | Up | 8.61E-09 | Up | 8.72E-18 | | 1 |
| | | | | | | 4.42E-03 | Up | | | | | | | |
| | | | | | | 8.90E-03 | Up | | | | | | | |
| | | | | | | 9.99E-02 | UP | | | | | | | |
| | | | | | | 2.02E-02 | Up | | | | | | | |
| 55 | BGN | 1.51E-02 | Up | 2.84E-02 | Up | 8.11E-01 | Up | 1.46E-05 | Up | 5.48E-10 | Up | 2.80E-18 | | 1 |
| 56 | BIRC5 | 2.26-02 | Up | | | | | 3.80E-04 | Up | 7.34E-15 | Up | 6.31E-20 | | 1 |
| 57 | DYRK2 | 3,63E-04 | Up | | | 1.34E-02 | Up | 9.61E-03 | Up | 3.66E-11 | Up | 1.71E-18 | | 1 |

Reg.= Regulation
Dw= Down

FIG. 2 (continued)

| Data Set | Model | Primary Endpoint | # Patients without Progression to Endpoint | # Patients with Progression to Endpoint | ROC Analysis | | |
|---|---|---|---|---|---|---|---|
| | | | | | AUC | 95% CI | p-value |
| Taylor[1] | PCPI_18 | Metastases Free Survival after RP | 121 | 8 | 0,94 | 0.9-0.98 | <0.0001 |
| | Clinical_Model[6] | | | | 0,77 | 0.55-0.98 | 1,30E-02 |
| | PCPI_18 & Clinical_Model | | | | 0,97 | 0.95-0.98 | <0.0001 |
| Boormans[2] | PCPI_18 | Metastases Free Survival after RP | 39 | 9 | 0,88 | 0.76-1.0 | <0.0001 |
| | Clinical_Model[7] | | | | 0,82 | 0.68-0.97 | <0.0001 |
| | PCPI_18 & Clinical_Model | | | | 0,89 | 0.76-1.0 | <0.0001 |
| Sun[3] | PCPI_18 | Biochemical Free Survival after RP | 42 | 37 | 0,74 | 0.62-0.83 | <0.0001 |
| | Clinical_Model[8] | | | | 0,75 | 0.64-0.84 | <0.0001 |
| | PCPI_18 & Clinical_Model | | | | 0,79 | 0.68-0.87 | <0.0001 |
| Sboner[4] | PPI_17 | Prostate Cancer Specific Survival | 116 | 165 | 0,73 | 0.67-0.8 | <0.0001 |
| | Clinical_Model[9] | | | | 0,74 | 0.48-1.0 | <0.0001 |
| | PCPI_18 & Clinical_Model | | | | 0,80 | 0.75-0.85 | 7,00E-02 |
| Nagakawa[5] | PPI_17 | Metastases Free Survival after BCR | 379 | 213 | 0,76 | 0.72-0.8 | <0.0001 |
| | Clinical_Model[10] | | | | 0,74 | 0.7-0.78 | <0.0001 |
| | PCPI_17 & Clinical_Model | | | | 0,78 | 0.74-0.82 | <0.0001 |
| Nagakawa[5] | PPI_17 | PCa Specific Survival after BCR | 379 | 213 | 0,78 | 0.72-0.82 | <0.0001 |
| | Clinical_Model[10] | | | | 0,78 | 0.74-0.83 | <0.0001 |
| | PCPI_17 & Clinical_Model | | | | 0,82 | 0.77-0.86 | <0.0001 |

FIG. 3

[1]Taylor BS et al.; Cancer Cell 18, 11–22, 2010
[2]Boormans JL et al., Int J Cancer 2013 Jul 15;133(2):335-45
[3]Sun Y et al., The Prostate, 69:1119-1127 (2009)
[4]Nagakawa T et al., PLoS ONE 3(5), e2318 (2010)
[5]Sboner A et al., BMC Medical Genomics 2010, 3:8
[6]Logistic regression model with the clinical parameters: pre-treatment PSA, biopsy Gleason, clinical TNM stage
[7]Logistic regression model with the clinical parameters: pre-treatment PSA, RP Gleason, pathology TNM stage
[8]Logistic regression model with the clinical parameters: pre-treatment PSA, RP Gleason, pathology TNM stage
[9]Only clinical parameter available: RP Gleason
[10]Logistic regression model with the clinical parameters: pre-treatment PSA, RP Gleason, pathology TNM stage

FIG. 3 (continued)

| Data Set | Model | Primary Endpoint | # Patients without Progression to Endpoint | # Patients with Progression to Endpoint | ROC Analysis | | |
|---|---|---|---|---|---|---|---|
| | | | | | AUC | 95% CI | p-value |
| Erho[1] | PCPI_18 | Metastases Free Survival after RP | 333 | 212 | 0,75 | 0.71-0.78 | <1.00E-04 |
| | Clinical_Model[2] | | | | 0,72 | 0.644-1.0 | <1.00E-04 |
| | PCPI_18 + Clinical Model | | | | 0,78 | 0.74-0.82 | <1.00E-04 |

[1]Erho N et al., PLoS One 8(6), e66855 (2013)

[2]Only clinical parameter available: RP Gleason

FIG. 4

| Data Set | Model | Primary Endpoint | # Patients without Progression to Endpoint | # Patients with Progression to Endpoint | Multivariate Cox Proportional Hazard Regression | | | |
|---|---|---|---|---|---|---|---|---|
| | | | | | $\chi 2$ | HR | HR (95% CI) | p-value |
| Taylor[1] | PCPI_18 | Metastases Free Survival after RP | 121 | 8 | 13,74 | 4,85 | 2.1-11.2 | 2,10E-04 |
| | Pre-treatment PSA | | | | 0,74 | 0,89 | 0.67-1.16 | 3,90E-01 |
| | BxGleason | | | | 0,04 | 1,08 | 0.49-2.35 | 8,51E-01 |
| | cT Stage | | | | 0,11 | 1,07 | 0.73-1.56 | 7,40E-01 |
| Boormans[2] | PCPI_18 | Metastases Free Survival after RP | 39 | 9 | 10,13 | 6,61 | 2.0-21.4 | 1,46E-03 |
| | Pre-treatment PSA | | | | 0,16 | 1,00 | 0.98-1.03 | 6,90E-01 |
| | pGleason | | | | 1,02 | 1,80 | 0.56-5.66 | 3,12E-01 |
| | pStage | | | | 0,73 | 0,80 | 0.48-1.33 | 3,92E-01 |
| Sboner[3] | PCPI_17 | Metastases Free Survival after RP | 116 | 165 | 18,565 | 1,598 | 1.29-1.98 | <1.00E-04 |
| | pGleason | | | | 48,273 | 1,746 | 1.49-2.05 | <1.00E-04 |
| Nagakawa[4] | PCPI_17 | Metastases Free Survival after BCR | 379 | 213 | 44,596 | 2,454 | 1.9-3.2 | <1.00E-04 |
| | Pre-treatment PSA | | | | 0,231 | 0,998 | 0.99-1.00 | 6,31E-01 |
| | pGleason | | | | 26,775 | 1,498 | 1.28-1.75 | <1.00E-04 |
| | pStage | | | | 4,356 | 1,354 | 1.02-1.8 | 3,69E-02 |

[1]Taylor BS et al., Cancer Cell 18, 11–22, 2010
[2]Boormans JL et al., Int J Cancer 2013 Jul 15;133(2):335-45
[3]Nagakawa T et al., PLoS ONE 3(5), e2318 (2010)
[4]Sboner A et al., BMC Medical Genomics 2010, 3:8

FIG. 5

| Data Set | Model | Primary Endpoint | # Patients without Progression to Endpoint | # Patients with Progression to Endpoint | ROC Analysis | | |
|---|---|---|---|---|---|---|---|
| | | | | | AUC | 95% CI | p-value |
| Taylor[1] | PCPI_18 | Biochemical Free Survival after RP | 103 | 26 | 0,77 | 0.66-0.88 | <1.00E-04 |
| | Clinical_Model[4] | | | | 0,70 | 0.58-0.82 | <1.00E-04 |
| | PCPI_18 + Clinical Model | | | | 0,82 | 0.73-0.91 | <1.00E-04 |
| Boormans[2] | PCPI_18 | Biochemical Free Survival after RP | 21 | 27 | 0,78 | 0.65-0.9 | <1.00E-04 |
| | Clinical_Model[5] | | | | 0,83 | 0.73-0.94 | <1.00E-04 |
| | PCPI_18 & Clinical_Model | | | | 0,84 | 0.73-0.95 | <1.00E-04 |
| Sun[3] | PCPI_18 | Biochemical Free Survival after RP | 42 | 37 | 0,74 | 0.63-0.85 | <1.00E-04 |
| | Clinical_Model[6] | | | | 0,75 | 0.66-0.85 | <1.00E-04 |
| | PCPI_18 & Clinical_Model | | | | 0,79 | 0.69-0.88 | <1.00E-04 |

[1]Taylor BS et al., Cancer Cell 18, 11–22, 2010
[2]Boormans JL et al., Int J Cancer 2013 Jul 15;133(2):335-45
[3]Sun Y et al., The Prostate, 69:1119-1127 (2009)
[4]Logistic regression model with the clinical parameters: pre-treatment PSA, biopsy Gleason, clinical TNM stage
[5]Logistic regression model with the clinical parameters pre-treatment PSA, RP Gleason, pathology TNM stage
[6]Logistic regression model with the clinical parameters pre-treatment PSA, RP Gleason, pathology TNM stage

FIG. 6

| Data Set | Model | Primary Endpoint | # Patients without Progression to Endpoint | # Patients with Progression to Endpoint | Multivariate Cox Proportional Hazard Regression | | | |
|---|---|---|---|---|---|---|---|---|
| | | | | | $\chi 2$ | HR | HR (95% CI) | p-value |
| Taylor[1] | PCPI_18 | Biochemical Free Survival after RP | 103 | 26 | 23,438 | 3,158 | 1.98-5.03 | <1.00E-04 |
| | Pre-treatment PSA | | | | 13,812 | 1,031 | 1.02-1.05 | 2,02E-04 |
| | BxGleason | | | | 0,520 | 1,172 | 0.76-1.8 | 4,71E-01 |
| | cT Stage | | | | 0,000 | 0,999 | 0.78-1.28 | 9,95E-01 |
| Boormans[2] | PCPI_18 | Biochemical Free Survival after RP | 21 | 27 | 5,264 | 1,872 | 1.1-3.2 | 2,18E-02 |
| | Pre-treatment PSA | | | | 2,509 | 1,010 | 0.99-1.02 | 1,13E-01 |
| | pGleason | | | | 2,437 | 1,689 | 0.87-3.26 | 1,19E-01 |
| | pStage | | | | 0,119 | 0,951 | 0.71-1.26 | 7,30E-01 |
| Sun[3] | PCPI_18 | Biochemical Free Survival after RP | 42 | 37 | 8,069 | 1,651 | 1-16-2.33 | 4,50E-03 |
| | Pre-treatment PSA | | | | 3,168 | 1,036 | 0.99-1.07 | 7,51E-02 |
| | BxGleason | | | | 17,737 | 2,625 | 1.67-4.11 | < 1.00E-04 |
| | cT Stage | | | | 0,051 | 0,923 | 0.46-1.85 | 8,22E-01 |

[1]Taylor BS et al., Cancer Cell 18, 11–22, 2010
[2]Boormans JL et al., Int J Cancer 2013 Jul 15;133(2):335-45
[3]Sun Y et al., The Prostate, 69:1119-1127 (2009)

FIG. 7

| Data Set | Model | Primary Endpoint | pGleason Categories | # Patients without Progression to Endpoint | # Patients with Progression to Endpoint | ROC Analysis | | | | | | |
|---|---|---|---|---|---|---|---|---|---|---|---|---|
| | | | | | | AUC | 95% CI | p-value | Cut-off (90% Sensitivity) | # Patients without Progression to Endpoint | # Patients with Progression to Endpoint | Risk of progression to endpoint over 10 years FU |
| Erho[1] | PCPI_18 | Metastases Free Survival after RP | All pGleason | 333 | 212 | 0,75 | 0.71-0.78 | <1.00E-04 | <1.449 | 134 | 21 | 15,67% |
| | Clinical_Model[2] | | | | | 0,72 | 0.644-1.0 | <1.00E-04 | <=6 | 57 | 6 | 10,53% |
| | PCPI_18 + Clinical Model | | | | | 0,78 | 0.74-0.82 | <1.00E-04 | <=-1.236 | 146 | 20 | 13,70% |
| Erho[1] | PCPI_18 | Metastases Free Survival after RP | pGleason >=7 | 276 | 206 | 0,75 | 0.71-0.78 | <1.00E-04 | <= 1.537 | 107 | 21 | 19,63% |
| | Clinical_Model[2] | | | | | 0,72 | 0.644-1.0 | <1.00E-04 | <=7 | 195 | 76 | 38,97% |
| | PCPI_18 + Clinical Model | | | | | 0,78 | 0.74-0.82 | <1.00E-04 | <= -1.105 | 108 | 21 | 19,44% |
| Erho[1] | PCPI_18 | Metastases Free Survival after RP | pGleason >=8 | 81 | 130 | 0,75 | 0.71-0.78 | <1.00E-04 | <= 1.753 | 25 | 12 | 48,00% |
| | Clinical_Model[2] | | | | | 0,72 | 0.644-1.0 | <1.00E-04 | <=8 | 35 | 33 | 94,29% |
| | PCPI_18 + Clinical Model | | | | | 0,78 | 0.74-0.82 | <1.00E-04 | <= -0.251 | 27 | 13 | 48,15% |

[1]Erho N et al. Discovery and Validation of a Prostate Cancer Genomic Classifier that Predicts Early Metastasis Following Radical Prostatectomy. PLoS One

[2]Only clinical parameter available: RP Gleason

FIG. 8

| | | | | | qPCR Primers & Probes; qPCR Amplicon Size [Base Pairs] | | | |
|---|---|---|---|---|---|---|---|---|
| No | Gene Symbol | Gene Name | NCBI RefSeq; mRNA | Protein Accession | Forward | Reverse | Probe | Amplicon Size |
| 1 | PDE4D5 | cAMP Phosphodiesterase PDE4D, Isoform 4D5 | NM_001197218.1 | NP_001184147.1 | GCTTCTCAGCAGCAACATC | TGCCATTGTCCACATCAAAA | ACAGCGGCGTTTCACGGTGGCACA | 74 bp |
| 2 | PDE4D7 | cAMP Phosphodiesterase PDE4D, Isoform 4D7 | NM_001165899.1 | NP_001159371.1 | GAACATTCAACGACCAACCA | TGCCATTGTCCACATCAAAA | CTGCCGCTGATTGCTATCACTTCTGCA | 95 bp |
| 3 | AZGP1 | Alpha-2-Glycoprotein 1, Zinc-Binding | NM_001185.3 | NP_001176.1 | TGGAGACCCTGAAAGACATC | CTCGATCTCACAACCAAACC | ACGGGTCTCACGTATTGCAGGGA | 86 bp |
| 4 | FBLN1 | Fibulin 1 | NM_001996.3; NM_006485.3; NM_006486.2; NM_006487.2 | NP_001987.2; NP_006476.2; NP_006477.2; NP_006478.2 | GCCTGGAGGCCACATTT | CCAACCATGAGGCTGTACTC | TGCTGCCATTGCTGTCTGCTG | 100 bp |
| 5 | ILK | Integrin-Linked Kinase | NM_001014794.2; NM_001014795.2; NM_001278441.1; NM_001278442.1; NM_004517.3 | NP_001014794.1; NP_001014795.1; NP_001265370.1; NP_001265371.1; NP_004508.1 | CATCCAAATGTGCTCCCAGTG | TGGCTCTGGTCCACGACGAA | TGCCTGCCAGTCTCCACCTGCT | 140 bp |
| 6 | KRT15 | Keratin 15 | NM_002275.3 | NP_002266.2 | GCTAGCCTACCTGAAGAAGAA | CCAGCTGGCTGCTGAACT | CCACGAAGAGGAGATGAAGG | 59 bp |
| 7 | MEIS2 | Meis Homeobox 2 | NM_001220482.1; NM_002399.3; NM_170674.4; NM_170675.4; NM_170676.4; NM_170677.4; NM_172315.2 | NP_001207411.1; NP_002390.1; NP_733774.1; NP_733775.1; NP_733776.1; NP_733777.1; NP_758526.1; NP_758527.1 | TCAAATCCAGAGCTGGACAA | CCCTTCAAACAGCTAATGTATCG | AAAGGTCCACGAACTGTGCGAT | 128 bp |

FIG. 9

| No | Gene Symbol | Gene Name | NCBI RefSeq; mRNA | Protein Accession | Forward | Reverse | Probe | Amplicon Size |
|---|---|---|---|---|---|---|---|---|
| 8 | MYBPC1 | Myosin Binding Protein C, Slow Type | NM_001254718.1; NM_001254719.1; NM_001254720.1; NM_001254721.1; NM_001254722.1; NM_001254723.1; NM_002465.3; NM_206819.2 NM_206820.2 NM_206821.2 | NP_001241647.1 NP_001241648.1 NP_001241649.1 NP_001241650.1 NP_001241651.1 NP_001241652.1 NP_002456.2 NP_996555.1 NP_996556.1 NP_996557.1 | TCCCAGCTGTCCATCTT | CAGCCTTGACTTTGGCTATG | CAAGGAGGAACAGTGAAAGTTGGTGA | 91 bp |
| 9 | PAGE4 | P Antigen Family, Member 4 | NM_007003.3 | NP_008934.1 | GAAGGAACACCTCCGAT | CATCTCCACGCTCACTCCGA | AGGTGATTGCCAGGAAATGGATCTG | 88 bp |
| 10 | SRD5A2 | Steroid 5-Alpha-Reductase 2 | NM_000348.3 | NP_000339.2 | GGCCTCTTCTGCCTACATTAC | CAGTGCCTCTGAGAATGAGTATAG | CTCACTGCTCAATCGAGGGAGG | 97 bp |
| 11 | COL1A1 | Collagen, Type I, Alpha 1 | NM_000088.3 | NP_000079.2 | GAAGACATCCCACCAATCAC | CGGGTTTCCACACGTCTC | CTGCGTACAGAACGGCCTCAGGTA | 70 bp |
| 12 | COL3A1 | Collagen, Type III, Alpha 1 | NM_000090.3 | NP_000081.1 | GAGGACGGCCAGGACTT | GGTCCAACTTCACCCTTAG | AGTGATGGTCAACCAGGCCCT | 142 bp |
| 13 | COL5A2 | Collagen, Type V, Alpha 2 | NM_000393.3 | NP_000384.2 | AAGAGGAGAACAAGGACCTC | ATCTCCAGGAACACCTTGAT | TTTCCACCTTCTCCAGGAGGCC | 109 bp |
| 14 | INHBA | Inhibin, Beta A | NM_002192.2 | NP_002183.1 | CCTCGGAGATCATCACGTTT | CACTGCCTTCCTTGGAAATCT | CAGCCAGGAAGACGCTGCACTT | 78 bp |
| 15 | THBS2 | Thrombospondin 2 | NM_003247.3 | NP_003238.2 | CAGCACGCAAGCTGGTC | TGCGGTTGATGTTGCTGATA | ACCAGGACAAAGACACGACCTTCGA | 71 bp |

FIG. 9 (continued)

| No | Gene Symbol | Gene Name | NCBI RefSeq; mRNA | Protein Accession | Forward | Reverse | Probe | Amplicon Size |
|---|---|---|---|---|---|---|---|---|
| 16 | VCAN | Versican | NM_001126336.2; NM_001164097.1; NM_001164098.1; NM_004385.4 | NP_001119808.1; NP_001157569.1; NP_001157570.1; NP_004376.2 | TCCTGTCTCA CGAAGAACAA | ATCAGTCCAACG GAAGTCAT | TCGTGTGGGCCAT GATTATCAGTGGA | 104 bp |
| 17 | BGN | Biglycan | NM_001711.4 | NP_001702.1 | TGCCCAAGGG AGTGTTC | TAGTTGAGCTTC AGGCCATC | ATGGGCGGGAACC CACTGGA | 115 bp |
| 18 | BIRC5 | Baculoviral IAP Repeat Containing 5 | NM_001012270.1; NM_001012271.1; NM_001168.2 | NP_001012270.1; NP_001012271.1; NP_001159.2 | CTTGGCCCAG TGTTTCTT | GTTCCTCTATGG GGTCGTCAT | AGCTGGAAGGCTG GGAGCCA | 71 bp |
| 19 | DYRK2 | Dual-Specificity Tyrosine-(Y)-Phosphorylation Regulated Kinase 2 | NM_003583.3; NM_006482.2 | NP_003574.1; NP_006473.2 | CGGCCATGTT AACCAGGAAA C | CGACATGCAGGT GATCATTC | CGATTGGCGGCAG TAAGCACACAA | 97 bp |

FIG. 9 (continued)

| Gene Symbol | Gene Name | NCBI RefSeq; mRNA | SEQ ID NO refering to NCBI RefSeq mRNA | Protein Accession | SEQ ID NO refering to Protein Accession | qPCR Primers & Probes; qPCR Amplicon Size [Base Pairs] | | | | | | | |
|---|---|---|---|---|---|---|---|---|---|---|---|---|---|
| | | | | | | Forward | SEQ ID NO forward primer | Reverse | SEQ ID NO reverse primer | Probe | SEQ ID NO probe | Amplicon Size |
| TBP | Homo sapiens TATA box binding protein, | NM_003194.4 | 154 | NP_003185.1 | 166 | GCCAAGAAGAAAGTGAACATCAT | 178 | ATAGGGATTCCGGGAGTCAT | 179 | TCAGAACAACAGCCTGCCACCTTA | 180 | 101 |
| HPRT1 | Homo sapiens hypoxanthine phosphoribosyltransferase 1 | NM_000194.2 | 155 | NP_000185.1 | 167 | GAGGATTTGGAAAGGGTGTTTATT | 181 | ACAGAGGGCTACAATGTGATG | 182 | ACGTCTTGCTCGAGATGTGATGAAGG | 183 | 111 |
| ACTB | Homo sapiens actin, beta | NM_001101.3 | 156 | NP_001092.1 | 168 | CCAACCGCGAGAAGATGA | 184 | CCAGAGGCGTACAGGGATAG | 185 | CCATGTACGTTGCTATCCAGGCT | 186 | 97 |
| RPLP0 | Homo sapiens 60S acidic ribosomal phosphoprotein P0 mRNA | NM_001002.3; NM_053275.3 | 157 158 | NP_000993.1; NP_444505.1 | 169 170 | TAAACCCTGCGTGGCAAT | 187 | ACATTTCGGATAATCATCCAATAGTTG | 188 | AAGTAGTTGGACTTCCAGGTCGCC | 189 | 117 |

FIG. 10

| Gene Symbol | Gene Name | NCBI RefSeq; mRNA | SEQ ID NO refering to NCBI RefSeq mRNA | Protein Accession | SEQ ID NO refering to Protein Accession | Forward | SEQ ID NO forward primer | Reverse | SEQ ID NO reverse primer | Probe | SEQ ID NO probe | Amplicon Size |
|---|---|---|---|---|---|---|---|---|---|---|---|---|
| POLR2A | Polymerase (RNA) II (DNA Directed) Polypeptide A, 220kDa | NM_000937.4 | 159 | NP_000928.1 | 171 | AGTCCTGAGTCCGGATGAA | 190 | CCTCCCTCAGTCGTCTCT | 191 | TGACGGAGGGTGGCATCAAATACC | 192 | 78 |
| B2M | Beta-2-Microglobulin | NM_004048.2 | 160 | NP_004039.1 | 172 | CCGTGGCCTTAGCTGTG | 193 | CTGCTGGATGACGTGAGTAAA | 194 | TCTCTCTTTCTGGCCTGGAGGCTA | 195 | 96 |
| PUM1 | Homo sapiens pumilio RNA-Binding Family Member 1 | NM_001020658.1<br>NM_014676.2 | 161<br>162 | NP_001018494.1; NP_055491.1 | 173<br>174 | GCCAGCTTGTCTTCAATGAAAT | 196 | CAAAGCCAGCTTCTGTTCAAG | 197 | ATCCACCATGAGTTGGTAGGCAGC | 198 | 119 |
| K-ALPHA-1 (TUBA1B) | Tubulin, Alpha 1b | NM_006082.2 | 163 | NP_006073.2 | 175 | TGACTCCTTCAACACCTTCTTC | 199 | TGCCAGTGCGAACTTCAT | 200 | CCGGGCTGTGTTTGTAGACTTGGA | 201 | 107 |
| ALAS-1 | Aminolevulinate, Delta-, Synthase | NM_000688.5; NM_199166.2 | 164<br>165 | NP_000679.1; NP_954635.1 | 176<br>177 | AGCCACATCATCCCTGT | 202 | CGTAGATGTTATGTCTGCTCAT | 203 | TTTAGCAGCATCTGCAACCCGC | 204 | 85 |

FIG. 10 (continued)

| | | NCCN VL&LR | NCCN FIR | NCCN UIR | NCCN HR | |
|---|---|---|---|---|---|---|
| | | 180 | 103 | 100 | 39 | |
| PCPI (4-5) | 14 | 1<br>5-y BCR: 0% | 2<br>5-y BCR: 0% | 7<br>5-y BCR: 71.4% | 4<br>5-y BCR: 75.0% | 5-y BCR: 57.1%<br>10-y CR: 45.5%<br>10-y PCSM: 30.0%<br>10-y OM: 30.0% |
| PCPI (3-4) | 87 | 23<br>5-y BCR: 21.7% | 28<br>5-y BCR: 28.6% | 26<br>5-y BCR: 53.8% | 10<br>5-y BCR: 70.0% | 5-y BCR: 39.1%<br>10-y CR: 6.8%<br>10-y PCSM: 6.8%<br>10-y OM: 11.5% |
| PCPI (2-3) | 248 | 117<br>5-y BCR: 10.3% | 52<br>5-y BCR: 15.4% | 57<br>5-y BCR: 28.1% | 22<br>5-y BCR: 27.3% | 5-y BCR: 16.9%<br>10-y CR: 2.3%<br>10-y PCSM: 2.3%<br>10-y OM: 7.7% |
| PCPI (1-2) | 73 | 39<br>5-y BCR: 5.1% | 21<br>5-y BCR: 4.8% | 10<br>5-y BCR: 0% | 3<br>5-y BCR: 66.7% | **5-y BCR: 6.9%**<br>10-y CR: 0%<br>10-y PCSM: 0%<br>10-y OM: 1.4% |
| | | **5-y BCR: 10.5%**<br>10-y CR: 0.9%<br>10-y PCSM: 0.9%<br>10-y OM: 8.0% | 5-y BCR: 16.5%<br>10-y CR: 4.2%<br>10-y PCSM: 4.2%<br>10-y OM: 8.1% | 5-y BCR: 35.0%<br>10-y CR: 6.9%<br>10-y PCSM: 5.6%<br>10-y OM: 8.2% | 5-y BCR: 46.2%<br>10-y CR: 14.3%<br>10-y PCSM: 11.1%<br>10-y OM: 11.1% | |

FIG. 11

| | | NCCN VL&LR | NCCN FIR | NCCN UIR | NCCN HR | |
|---|---|---|---|---|---|---|
| | | 160 | 103 | 103 | 41 | |
| GPSu (4-5) | 14 | 2<br>5-y BCR: 0% | 2<br>5-y BCR: 50% | 6<br>5-y BCR: 66.7% | 4<br>5-y BCR: 100.0% | 5-y BCR: 64.3%<br>10-y CR: 33.3%<br>10-y PCSM: 22.2%<br>10-y OM: 33.3% |
| GPSu (3-4) | 93 | 24<br>5-y BCR: 20.8% | 24<br>5-y BCR: 41.7% | 33<br>5-y BCR: 60.6% | 12<br>5-y BCR: 58.3% | 5-y BCR: 45.2%<br>10-y CR: 7.6%<br>10-y PCSM: 6.3%<br>10-y OM: 10.5% |
| GPSu (2-3) | 233 | 99<br>5-y BCR: 5.0% | 56<br>5-y BCR: 8.9% | 55<br>5-y BCR: 21.8% | 23<br>5-y BCR: 26.1% | 5-y BCR: 12.0%<br>10-y CR: 1.7%<br>10-y PCSM: 3.0%<br>10-y OM: 8.1% |
| GPSu (1-2) | 67 | 35<br>5-y BCR: 11.4% | 21<br>5-y BCR: 14.3% | 9<br>5-y BCR: 0% | 2<br>5-y BCR: 0% | **5-y BCR: 10.5%**<br>10-y CR: 0%<br>10-y PCSM: 0%<br>10-y OM: 3.6% |
| | | **5-y BCR: 8.7%**<br>10-y CR: 0%<br>10-y PCSM: 0.9%<br>10-y OM: 7.6% | 5-y BCR: 18.4%<br>10-y CR: 3.9%<br>10-y PCSM: 3.9%<br>10-y OM: 7.6% | 5-y BCR: 35.0%<br>10-y CR: 6.8%<br>10-y PCSM: 5.5%<br>10-y OM: 9.2% | 5-y BCR: 41.4%<br>10-y CR: 9.7%<br>10-y PCSM: 9.7%<br>10-y OM: 12.5% | |

FIG. 12

| | | NCCN VL&LR | NCCN FIR | NCCN UIR | NCCN HR | |
|---|---|---|---|---|---|---|
| | | 160 | 103 | 103 | 41 | |
| CCP (4-5) | 12 | 2<br>5-y BCR: 0% | 3<br>5-y BCR: 66.6% | 5<br>5-y BCR: 40.0% | 2<br>5-y BCR: 50.0% | 5-y BCR: 41.7%<br>10-y CR: 0%<br>10-y PCSM: 0%<br>10-y OM: 0% |
| CCP (3-4) | 100 | 100<br>5-y BCR: 23.0% | 24<br>5-y BCR: 41.7% | 33<br>5-y BCR: 60.6% | 12<br>5-y BCR: 58.3% | 5-y BCR: 23.0%<br>10-y CR: 4.1%<br>10-y PCSM: 5.3%<br>10-y OM: 8.9% |
| CCP (2-3) | 230 | 94<br>5-y BCR: 8.5% | 59<br>5-y BCR: 18.9% | 55<br>5-y BCR: 36.4% | 23<br>5-y BCR: 30.4% | 5-y BCR: 20.0%<br>10-y CR: 3.7%<br>10-y PCSM: 3.1%<br>10-y OM: 8.8% |
| CCP (1-2) | 65 | 25<br>5-y BCR: 4.0% | 16<br>5-y BCR: 18.8% | 16<br>5-y BCR: 18.8% | 8<br>5-y BCR: 62.5% | **5-y BCR: 18.5%**<br>10-y CR: 4.4%<br>10-y PCSM: 0%<br>10-y OM: 8.5% |
| | | **5-y BCR: 8.8%**<br>10-y CR: 0%<br>10-y PCSM: 0.6%<br>10-y OM: 7.6% | 5-y BCR: 18.4%<br>10-y CR: 3.9%<br>10-y PCSM: 3.9%<br>10-y OM: 7.6% | 5-y BCR: 35.0%<br>10-y CR: 6.8%<br>10-y PCSM: 5.5%<br>10-y OM: 9.2% | 5-y BCR: 41.4%<br>10-y CR: 9.7%<br>10-y PCSM: 9.7%<br>10-y OM: 12.5% | |

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2013196321 A1
- WO 2010131194 A1
- WO 2014153442 A2
- US 06285701 A
- US 29884701 P
- US 60257801 B
- WO 2010131194 A
- WO 2010131195 A


### Non-patent literature cited in the description


- **MIMOTO REI et al.** DYRK2 controls the epithelial-mesenchymal transition in breast cancer by degrading Snail. *Cancer Letters,* 2013, vol. 339 (2), 214-225
- **ALTSCHUL et al.** *J. Mol. Biol.,* 1990, vol. 215, 403-410
- **STRYER.** *Science,* 1968, vol. 162, 526-533
- **GORLOV IP et al.** Candidate pathways and genes for prostate cancer: a meta-analysis of gene expression data. *BMC Medical Genomics,* 2009, vol. 2, 48
- **STANBROUGH M et al.** Increased Expression of Genes Converting Adrenal Androgens to Testosterone in Androgen-Independent Prostate Cancer. *Cancer Res,* 2006, vol. 66, 2815-2825
- **TAMURA K et al.** Molecular Features of Hormone-Refractory Prostate Cancer Cells by Genome-Wide Gene Expression Profiles. *Cancer Res,* 2007, vol. 67 (11), 5117-25
- **LAPOINTE J et al.** Gene expression profiling identifies clinically relevant subtypes of prostate cancer. *PNAS,* 2003, vol. 101 (3), 8110816
- **SUN Y et al.** Optimizing Molecular Signatures for Predicting Prostate Cancer Recurrence. *The Prostate,* 2009, vol. 69, 1119-1127
- **TAYLOR BS et al.** Integrative Genomic Profiling of Human Prostate Cancer. *Cancer Cell,* 2010, vol. 18, 11-22
- **BOORMANS JL et al.** Identification of TDRD1 as a direct target gene of ERG in primary prostate cancer. *Int J Cancer,* 15 July 2013, vol. 133 (2), 335-45
- **SBONER A et al.** Molecular sampling of prostate cancer: a dilemma for predicting disease progression. *BMC Medical Genomics,* 2010, vol. 3, 8
- **NAGAKAWA T et al.** A Tissue Biomarker Panel Predicting Systemic Progression after PSA Recurrence Post-Definitive Prostate Cancer Therapy. *PLoS ONE,* 2010, vol. 3 (5), e2318
- **ERHO N et al.** Discovery and Validation of a Prostate Cancer Genomic Classifier that Predicts Early Metastasis Following Radical Prostatectomy. *PLoS One,* 2013, vol. 8 (6), e66855
- **C. L. ANDERSEN ; J. L. JENSEN ; T. F. ØRNTOFT.** Normalization of Real-Time Quantitative Reverse Transcription-PCR Data: A Model-Based Variance Estimation Approach to Identify Genes Suited for Normalization, Applied to Bladder and Colon Cancer Data Sets. *Cancer Res.,* August 2004, vol. 64 (15), 5245-5250
- **J. VANDESOMPELE ; K. DE PRETER ; F. PATTYN ; B. POPPE ; N. VAN ROY ; A. DE PAEPE ; F. SPELEMAN.** Accurate normalization of real-time quantitative RT-PCR data by geometric averaging of multiple internal control genes. *Genome Biol.,* June 2002, vol. 3 (7
- **J. HELLEMANS ; G. MORTIER ; A. D. PAEPE ; F. SPELEMAN ; J. VANDESOMPELE.** qBase relative quantification framework and software for management and automated analysis of real-time quantitative PCR data. *Genome Biol.,* February 2007, vol. 8 (2), 19
- **DEJAN KNEZEVIC et al.** Analytical validation of the Oncotype DX prostate cancer assay - a clinical RT-PCR assay optimized for prostate needle biopsies. *BMC Genomics,* 2013, vol. 14, 690
- **JACK CUZICK et al.** Prognostic value of an RNA expression signature derived from cell cycle proliferation genes for recurrence and death from prostate cancer: A retrospective study in two cohorts. *Lancet Oncol.,* 2011, vol. 12 (3), 245-255